# EUROPEAN PATENT SPECIFICATION

(11) **EP 3 971 187 B1**
(45) Date of publication and mention of the grant of the patent: **07.05.2025**
(21) Application number: 20806520.1
(22) Date of filing: 14.05.2020
(51) Int. Cl.: C07D 471/04, A61K 31/437, A61P 35/00, C07D 401/14, C07D 471/08, C07D 519/00, C07F 9/53, C07F 9/6561

(54) **INHIBITOR CONTAINING BICYCLIC DERIVATIVE, PREPARATION METHOD THEREFOR AND USE THEREOF**
INHIBITOR MIT EINEM BICYCLISCHEN DERIVAT, HERSTELLUNGSVERFAHREN DAFÜR UND VERWENDUNG DAVON
INHIBITEUR CONTENANT UN DÉRIVÉ BICYCLIQUE, SON PROCÉDÉ DE PRÉPARATION ET SON UTILISATION

(30) Priority: 14.05.2019 CN 201910400013; 09.07.2019 CN 201910615987; 30.08.2019 CN 201910816375; 20.09.2019 CN 201910895078; 13.03.2020 CN 202010177893
(43) Date of publication of application: 23.03.2022
(73) Proprietor: Shanghai Hansoh Biomedical Co., Ltd., Shanghai 201203 (CN); Jiangsu Hansoh Pharmaceutical Group Co., Ltd., Lianyungang, Jiangsu 222069 (CN)
(72) Inventor: SU, Yidong, Shanghai 201203 (CN); WANG, Jun, Shanghai 201203 (CN); BAO, Rudi, Shanghai 201203 (CN)
(74) Representative: Hoffmann Eitle
(86) International application number: PCT/CN2020/090142
(87) International publication number: WO 2020/228756

(56) References cited:
- EP-A1- 3 936 504
- EP-A1- 3 950 685
- EP-A1- 3 998 265
- EP-A1- 4 074 710
- WO-A1-2017/011776
- WO-A1-2019/075108
- WO-A1-2020/063751
- CN-A- 108 349 969
- US-A1- 2018 133 207
- US-B2- 10 137 124
- DATABASE PubChem Compound [online] NCBI; 15 December 2010 (2010-12-15), .: "5-[6-(4-Methylpiperazin-1-yl)pyridin-3-yl]imidazo[1,2-a]pyridine", XP093004872, retrieved from https://pubchem.ncbi.nlm.nih.gov/compound/49790026 Database accession no. CID 49790026

## Description

This application claims the priorities of the Chinese patent application CN201910400013.0 filed on May 14, 2019, the Chinese patent application CN201910615987.0 filed on July 9, 2019, the Chinese patent application CN201910816375.8 filed on August 30, 2019, the Chinese patent application CN 201910895078.7 filed on September 20, 2019, the Chinese patent application CN202010177893.2 filed on March 13, 2020. The present disclosure refers to the full text of the above Chinese patent disclosures.

### Technical field

The present disclosure belongs to the field of drug synthesis, specifically related to an inhibitor containing a bicyclic derivative, a preparation method therefor and a use thereof.

### Prior Art

RET (rearranged during transfection) protein is encoded by the proto-oncogene RET located on chromosome 10, and is a receptor tyrosine kinase that consists of an extracellular domain, a transmembrane domain, and an intracellular kinase domain. RET ligands are glial-cell-line derived neurotrophic factor (GDNF) family ligands (GFLs) such as GDNF, neuroturin (NRTN), artemin (ARTN), persephin (PSPN), and the activation of the receptor also requires the combined effect of the co-receptor GFRa family, GFLs and GFRa form a dimer binding to RET and recruiting it to the cholesterol-rich membrane region, the RET protein undergoes dimerization and autophosphorylation, thereby activating downstream RAS-MAPK and PI3K-AKT, PKC and other signal pathways. RET plays an important role in the development of the kidney and enteric nervous system during embryonic development; it is also important for the homeostasis of neuroendocrine, hematopoietic and male germ cells and other tissues.

The disorder of RET protein function has led to the occurrence of many diseases. The lack of RET protein function during developmental processes can lead to a series of congenital diseases such as Hirschsprung disease (HSCR), congenital kidney and urinary tract malformations (CAKUT), etc. The activating mutations of RET protein, including point mutations and RET protein fusion caused by chromosome rearrangement, are also related to the occurrence of many diseases. RET fusion mainly occurs in 1 to 2% of non-small cell lung cancer (NSCLC) patients and 5 to 10% of papillary thyroid carcinoma, while RET mutations mainly occur in 60% of medullary thyroid carcinoma, and the activating mutations of RET protein are found in many other tumors such as breast cancer, gastric cancer, bowel cancer, and chronic bone marrow myelomonocytic leukemia.

Although there is a large clinical need, the current treatment for RET targets is still extremely limited, unlike ALK, EGFR and other targeted drugs that have achieved excellent efficacy in the clinic, there are still no approved targeted drugs for RET targets. At present, multi-kinase inhibitors (MKI) such as vandetinib and cabozantinib are mostly used in clinical drugs, these multi-kinase inhibitors have the disadvantages of high side effects and poor efficacy due to poor selectivity, and they cannot overcome the drug resistance problems that occur during treatment.

The demand for RET targeted drugs has attracted a large number of domestic and foreign pharmaceutical companies to develop RET specific targeted drugs, wherein the more prominent ones are Loxo Oncology's LOXO-292, which has entered clinical phase I/II, and Blueprint's BLU-667, which has also entered clinical phase I. Both of these targeted drugs have shown very good efficacy and safety in preclinical trials for patients with RET activating mutations, as well as overcoming possible drug resistance mutations in preclinical activity screening, which is expected to bring more treatment options for cancers with RET activating mutations in the future.

WO 2017/011776 discloses heteroaromatic substituted fused ring RET kinase inhibitor compounds and methods of their use.

US 10137124A and US 20180133207A disclose fused ring RET kinase inhibitor compounds substituted with alkoxyl groups and other substituents and methods of their use.

WO 2019/075108 discloses crystalline form of RET kinase inhibitor compounds and methods of their use.

There are currently no specific targeted drugs for RET targets, and there is a large clinical demand. RET inhibitors with higher selectivity, better activity, better safety, and the ability to overcome drug-resistant mutations have the potential to treat a variety of cancers and have broad market prospects.

### Content of the present invention

The invention is as defined in the appended claims. The references to methods of treatment throughout the description are to be interpreted as references to the compounds, pharmaceutical compositions and medicaments of the present invention for use in a method for treatment of the human (or animal) body by therapy (or for diagnosis).

The object of the present disclosure is to provide a compound represented by general formula (I), a stereoisomer thereof or a pharmaceutically acceptable salt thereof, wherein the structure of the compound represented by general formula (I) is as follows: 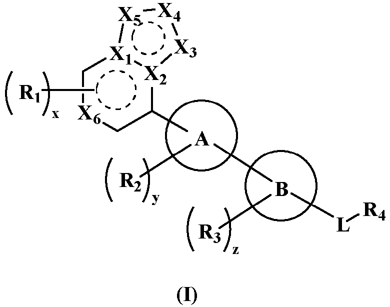 wherein:
X₁-X₆ are each independently selected from C, N, CR₅, CRₐₐR_{bb} or NRₐₐ;
L is selected from bond, -(CH₂)ₙ₁CRₐₐR_{bb}-, -(CH₂)ₙ₁NRₐₐ(O)(CH₂)ₙ₂-, - (CH₂)ₙ₁C(O)(CH₂)ₙ₂(CRₐₐR_{bb})ₘ-, -(CH₂)ₙ₁C(O)(CRₐₐR_{bb})ₘ(CH₂)ₙ₂-, -(CH₂)ₙ₁C(O)NR_{cc}(CRₐₐR_{bb})ₙ₂-, - (CH₂)ₙ₁(O)(CH₂)ₙ₂- or -(CH₂)ₙ₁NRₐₐ(CH₂)ₙ₂-;
ring A is selected from heterocyclyl, aryl or heteroaryl;
ring B is selected from cycloalkyl, heterocyclyl, aryl or heteroaryl;
R₁ is selected from hydrogen, deuterium, alkyl, deuterated alkyl, haloalkyl, alkoxy, haloalkoxy, halogen, amino, nitro, hydroxyl, cyano, oxo, alkenyl, alkynyl, cycloalkyl, heterocyclyl, aryl, heteroaryl, - (CH₂)ₙ₁Rₐₐ, -(C≡C)ₙ₁(CRₐₐR_{bb})ₘR_{cc}, -(C=C)ₙ₁(CRₐₐR_{bb})ₘR_{cc}, -(CH₂)ₙ₁O(CH₂)ₙ₂(CRₐₐR_{bb})ₘR_{cc}, - (CH₂)ₙ₁O(CH₂)ₙ₂Rₐₐ, -(CH₂)ₙ₁S(CH₂)ₙ₂(CRₐₐR_{bb})ₘR_{cc}, -(CH₂)ₙ₁O(CH₂)ₙ₂S(O)ₘRₐₐ, - (CH₂)ₙ₁O(CH₂)ₙ₂S(O)(=NRₐₐ)(CH₂)ₘR_{bb}, -(CH₂)ₙ₁C(O)Rₐₐ, -(CH₂)ₙ₁C(O)ORₐₐ, -(CH₂)ₙ₁S(O)ₘRₐₐ, - (CH₂)ₙ₁S(O)(=NRₐₐ)(CH₂)ₙ₂R_{bb}, -(CH₂)ₙ₁NRₐₐR_{bb}, -(CH₂)ₙ₁P(O)RₐₐR_{bb}, -(CH₂)ₙ₁C(O)NRₐₐR_{bb}, - (CH₂)ₙ₁NRₐₐ(CH₂)ₙ₂R_{bb}, -(CH₂)ₙ₁NRₐₐC(O)R_{bb} or -(CH₂)ₙ₁NRₐₐS(O)ₘR_{bb}, wherein the alkyl, deuterated alkyl, haloalkyl, alkoxy, haloalkoxy, alkenyl, alkynyl, cycloalkyl, heterocyclyl, aryl and heteroaryl are optionally further substituted by one or more substituents selected from hydrogen, deuterium, substituted or unsubstituted alkyl, substituted or unsubstituted deuterated alkyl, substituted or unsubstituted haloalkyl, substituted or unsubstituted hydroxyalkyl, substituted or unsubstituted cyanoalkyl, substituted or unsubstituted alkoxy, substituted or unsubstituted haloalkoxy, halogen, substituted or unsubstituted amino, nitro, hydroxyl, cyano, oxo, thio, imino, substituted or unsubstituted alkenyl, substituted or unsubstituted alkynyl, substituted or unsubstituted cycloalkyl, substituted or unsubstituted heterocyclyl, substituted or unsubstituted aryl, substituted or unsubstituted heteroaryl, -(CH₂)ₙ₁R_{dd}, -(CH₂)ₙ₁OR_{dd}, -(CH₂)ₙ₁S(CH₂)ₙ₂R_{dd}, -(CH₂)ₙ₁C(O)R_{dd}, - (CH₂)ₙ₁C(O)OR_{dd}, -(CH₂)ₙ₁S(O)ₘR_{dd}, -(CH₂)ₙ₁S(O)(=NR_{dd})(CH₂)ₙ₂Rₑₑ, -(CH₂)ₙ₁NR_{dd}Rₑₑ, -(CH₂)ₙ₁P(O)R_{dd}Rₑₑ, -(CH₂)ₙ₁C(O)NR_{dd}Rₑₑ, -(CH₂)ₙ₁NR_{dd}C(O)Rₑₑ or -(CH₂)ₙ₁NR_{dd}S(O)ₘRₑₑ;
R₂ is selected from hydrogen, deuterium, alkyl, deuterated alkyl, haloalkyl, alkoxy, haloalkoxy, halogen, amino, nitro, hydroxyl, cyano, oxo, alkenyl, alkynyl, cycloalkyl, heterocyclyl, aryl or heteroaryl, wherein the cycloalkyl, heterocyclyl, aryl and heteroaryl are optionally further substituted by one or more substituents selected from hydrogen, deuterium, substituted or unsubstituted alkyl, substituted or unsubstituted deuterated alkyl, substituted or unsubstituted haloalkyl, halogen, amino, oxo, thio, nitro, cyano, hydroxyl, substituted or unsubstituted alkenyl, substituted or unsubstituted alkynyl, substituted or unsubstituted alkoxy, substituted or unsubstituted haloalkoxy, substituted or unsubstituted hydroxyalkyl, substituted or unsubstituted cycloalkyl, substituted or unsubstituted heterocyclyl, substituted or unsubstituted aryl or substituted or unsubstituted heteroaryl;
R₃ is selected from hydrogen, deuterium, alkyl, deuterated alkyl, haloalkyl, alkoxy, haloalkoxy, halogen, amino, nitro, hydroxyl, cyano, oxo, alkenyl, alkynyl, cycloalkyl, heterocyclyl, aryl or heteroaryl, wherein the cycloalkyl, heterocyclyl, aryl and heteroaryl are optionally further substituted by one or more substituents selected from hydrogen, deuterium, substituted or unsubstituted alkyl, substituted or unsubstituted deuterated alkyl, substituted or unsubstituted haloalkyl, halogen, amino, oxo, thio, nitro, cyano, hydroxyl, substituted or unsubstituted alkenyl, substituted or unsubstituted alkynyl, substituted or unsubstituted alkoxy, substituted or unsubstituted haloalkoxy, substituted or unsubstituted hydroxyalkyl, substituted or unsubstituted cycloalkyl, substituted or unsubstituted heterocyclyl, substituted or unsubstituted aryl or substituted or unsubstituted heteroaryl;
or, any two adjacent or non-adjacent R₃ are connected to form a cycloalkyl, heterocyclyl, aryl or heteroaryl, wherein the cycloalkyl, heterocyclyl, aryl and heteroaryl are optionally further substituted by one or more substituents selected from hydrogen, deuterium, substituted or unsubstituted alkyl, substituted or unsubstituted deuterated alkyl, substituted or unsubstituted haloalkyl, substituted or unsubstituted alkoxy, substituted or unsubstituted haloalkoxy, halogen, amino, nitro, hydroxyl, cyano, oxo, substituted or unsubstituted alkenyl, substituted or unsubstituted alkynyl, substituted or unsubstituted cycloalkyl, substituted or unsubstituted heterocyclyl, substituted or unsubstituted aryl, or substituted or unsubstituted heteroaryl;
R₄ is selected from hydrogen, deuterium, alkyl, deuterated alkyl, haloalkyl, alkoxy, haloalkoxy, halogen, amino, nitro, hydroxyl, cyano, alkenyl, alkynyl, cycloalkyl, heterocyclyl, aryl, heteroaryl, -(CH₂)ₙ₁Rₐₐ, -(CH₂)ₙ₁ORₐₐ, -(CH₂)ₙ₁S(CH₂)ₙ₂Rₐₐ, -(CH₂)ₙ₁C(O)Rₐₐ, -(CH₂)ₙ₁C(O)ORₐₐ, -(CH₂)ₙ₁S(O)ₘRₐₐ, - (CH₂)ₙ₁S(O)(=NRₐₐ)(CH₂)ₙ₂R_{bb}, -(CH₂)ₙ₁NRₐₐR_{bb}, -(CH₂)ₙ₁P(O)RₐₐR_{bb}, -(CH₂)ₙ₁C(O)NRₐₐR_{bb}, - (CH₂)ₙ₁NRₐₐC(O)R_{bb} or -(CH₂)ₙ₁NRₐₐS(O)ₘR_{bb}, wherein the alkyl, deuterated alkyl, haloalkyl, alkoxy, haloalkoxy, alkenyl, alkynyl, cycloalkyl, heterocyclyl, aryl and heteroaryl are optionally further substituted by one or more substituents selected from hydrogen, deuterium, substituted or unsubstituted alkyl, substituted or unsubstituted deuterated alkyl, substituted or unsubstituted haloalkyl, substituted or unsubstituted hydroxyalkyl, substituted or unsubstituted cyanoalkyl, substituted or unsubstituted alkoxy, substituted or unsubstituted haloalkoxy, halogen, amino, nitro, hydroxyl, cyano, substituted or unsubstituted alkenyl, substituted or unsubstituted alkynyl, substituted or unsubstituted cycloalkyl, substituted or unsubstituted heterocyclyl, substituted or unsubstituted aryl, substituted or unsubstituted heteroaryl, -(CH₂)ₙ₁R_{cc}, - (CH₂)ₙ₁OR_{cc}, -(CH₂)ₙ₁S(CH₂)ₙ₂R_{cc}, -(CH₂)ₙ₁C(O)R_{cc}, -(CH₂)ₙ₁C(O)OR_{cc}, -(CH₂)ₙ₁S(O)ₘR_{cc}, - (CH₂)ₙ₁S(O)(=NR_{cc})(CH₂)ₙ₂R_{dd}, -(CH₂)ₙ₁NR_{cc}R_{dd}, -(CH₂)ₙ₁P(O)R_{cc}R_{dd}, -(CH₂)ₙ₁C(O)NR_{cc}R_{dd}, - (CH₂)ₙ₁NR_{cc}C(O)R_{dd} or -(CH₂)ₙ₁NR_{cc}S(O)ₘR_{dd};
R₅ is selected from hydrogen, deuterium, alkyl, deuterated alkyl, haloalkyl, alkoxy, haloalkoxy, halogen, amino, nitro, hydroxyl, cyano, alkenyl, alkynyl, cycloalkyl, heterocyclyl, aryl, heteroaryl, -(CH₂)ₙ₁Rₐₐ, -(CH₂)ₙ₁ORₐₐ, -(CH₂)ₙ₁S(CH₂)ₙ₂Rₐₐ, -(CH₂)ₙ₁C(O)Rₐₐ, -(CH₂)ₙ₁C(O)ORₐₐ, -(CH₂)ₙ₁S(O)ₘRₐₐ, - (CH₂)ₙ₁S(O)(=NRₐₐ)(CH₂)ₙ₂R_{bb}, -(CH₂)ₙ₁NRₐₐR_{bb}, -(CH₂)ₙ₁P(O)RₐₐR_{bb}, -(CH₂)ₙ₁C(O)NRₐₐR_{bb}, - (CH₂)ₙ₁NRₐₐC(O)R_{bb} or -(CH₂)ₙ₁NRₐₐS(O)ₘR_{bb};
Rₐₐ, R_{bb}, R_{cc}, R_{dd} and Rₑₑ are each independently selected from hydrogen, deuterium, alkyl, deuterated alkyl, haloalkyl, alkoxy, haloalkoxy, halogen, cyano, nitro, hydroxyl, amino, alkenyl, alkynyl, cycloalkyl, heterocyclyl, aryl or heteroaryl, wherein the alkyl, deuterated alkyl, haloalkyl, alkoxy, haloalkoxy, alkenyl, alkynyl, cycloalkyl, heterocyclyl, aryl and heteroaryl are optionally further substituted by one or more substituents selected from hydrogen, deuterium, substituted or unsubstituted alkyl, substituted or unsubstituted deuterated alkyl, substituted or unsubstituted haloalkyl, substituted or unsubstituted alkoxy, substituted or unsubstituted haloalkoxy, halogen, cyano, nitro, hydroxyl, amino, oxo, imino, substituted or unsubstituted alkenyl, substituted or unsubstituted alkynyl, substituted or unsubstituted cycloalkyl, substituted or unsubstituted heterocyclyl, substituted or unsubstituted aryl, or substituted or unsubstituted heteroaryl;
or, any two of Rₐₐ, R_{bb}, R_{cc}, R_{dd} and Rₑₑ are connected to form a cycloalkyl, heterocyclyl, aryl or heteroaryl, wherein the cycloalkyl, heterocyclyl, aryl and heteroaryl are optionally further substituted by one or more substituents selected from hydrogen, deuterium, substituted or unsubstituted alkyl, substituted or unsubstituted deuterated alkyl, substituted or unsubstituted haloalkyl, substituted or unsubstituted alkoxy, substituted or unsubstituted haloalkoxy, halogen, cyano, nitro, hydroxyl, amino, oxo, imine, substituted or unsubstituted alkenyl, substituted or unsubstituted alkynyl, substituted or unsubstituted cycloalkyl, substituted or unsubstituted heterocyclyl, substituted or unsubstituted aryl, or substituted or unsubstituted heteroaryl;
x is 0, 1, 2, 3, 4 or 5;
y is 0, 1, 2, 3, 4 or 5;
z is 0, 1, 2, 3, 4, 5 or 6;
m is 0, 1 or 2;
n1 is 0, 1, 2 or 3; and
n2 is 0, 1, 2 or 3.

The present disclosure further provides a preferred embodiment, the compound represented by general formula (I), the stereoisomer thereof or the pharmaceutically acceptable salt thereof, and the general formula (I) is further represented by general formula (II): 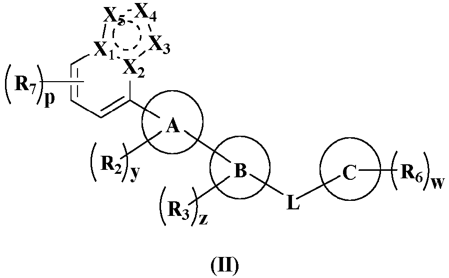 wherein:
ring C is selected from cycloalkyl, heterocyclyl, aryl or heteroaryl;
R₆ is selected from hydrogen, deuterium, alkyl, deuterated alkyl, haloalkyl, alkoxy, haloalkoxy, halogen, amino, nitro, hydroxyl, cyano, alkenyl, alkynyl, cycloalkyl, heterocyclyl, aryl, heteroaryl, -(CH₂)ₙ₁Rₐₐ, -(CH₂)ₙ₁ORₐₐ, -(CH₂)ₙ₁S(CH₂)ₙ₂Rₐₐ, -(CH₂)ₙ₁C(O)Rₐₐ, -(CH₂)ₙ₁C(O)ORₐₐ, -(CH₂)ₙ₁S(O)ₘRₐₐ, - (CH₂)ₙ₁S(O)(=NRₐₐ)(CH₂)ₙ₂R_{bb}, -(CH₂)ₙ₁NRₐₐR_{bb}, -(CH₂)ₙ₁P(O)RₐₐR_{bb}, -(CH₂)ₙ₁C(O)NRₐₐR_{bb}, - (CH₂)ₙ₁NRₐₐC(O)R_{bb} or -(CH₂)ₙ₁NRₐₐS(O)ₘR_{bb};
R₇ is selected from hydrogen, deuterium, alkyl, deuterated alkyl, haloalkyl, alkoxy, haloalkoxy, halogen, amino, nitro, hydroxyl, cyano, alkenyl, alkynyl, cycloalkyl, heterocyclyl, aryl, heteroaryl, -(CH₂)ₙ₁Rₐₐ, -(C≡C)ₙ₁(CRₐₐR_{bb})ₘR_{cc}, -(C=C)ₙ₁(CRₐₐR_{bb})ₘR_{cc}, -(CH₂)ₙ₁O(CH₂)ₙ₂(CRₐₐR_{bb})ₘR_{cc}, -(CH₂)ₙ₁O(CH₂)ₙ₂Rₐₐ, - (CH₂)ₙ₁S(CH₂)ₙ₂Rₐₐ, -(CH₂)ₙ₁O(CH₂)ₙ₂S(O)ₘRₐₐ, -(CH₂)ₙ₁O(CH₂)ₙ₂S(O)(=NRₐₐ)(CH₂)ₘR_{bb}, -(CH₂)ₙ₁C(O)Rₐₐ, -(CH₂)ₙ₁C(O)ORₐₐ, -(CH₂)ₙ₁S(O)ₘRₐₐ, =(CH₂)ₙ₁S(O)(=NRₐₐ)(CH₂)ₙ₂R_{bb}, -(CH₂)ₙ₁NRₐₐR_{bb}, - (CH₂)ₙ₁P(O)RₐₐR_{bb}, -(CH₂)ₙ₁C(O)NRₐₐR_{bb}, -(CH₂)ₙ₁NRₐₐC(O)R_{bb}, -(CH₂)ₙ₁NRₐₐ(CH₂)ₙ₂R_{bb} or - (CH₂)ₙ₁NRₐₐS(O)ₘR_{bb}, wherein the alkyl, deuterated alkyl, haloalkyl, alkoxy, haloalkoxy, alkenyl, alkynyl, cycloalkyl, heterocyclyl, aryl and heteroaryl are optionally further substituted by one or more substituents selected from hydrogen, deuterium, substituted or unsubstituted alkyl, substituted or unsubstituted deuterated alkyl, substituted or unsubstituted haloalkyl, substituted or unsubstituted hydroxyalkyl, substituted or unsubstituted cyanoalkyl, substituted or unsubstituted alkoxy, substituted or unsubstituted haloalkoxy, halogen, substituted or unsubstituted amino, nitro, hydroxyl, cyano, oxo, thio, imino, substituted or unsubstituted alkenyl, substituted or unsubstituted alkynyl, substituted or unsubstituted cycloalkyl, substituted or unsubstituted heterocyclyl, substituted or unsubstituted aryl, substituted or unsubstituted heteroaryl, - (CH₂)ₙ₁R_{dd}, -(CH₂)ₙ₁OR_{dd}, -(CH₂)ₙ₁S(CH₂)ₙ₂R_{dd}, -(CH₂)ₙ₁C(O)R_{dd}, -(CH₂)ₙ₁C(O)OR_{dd}, -(CH₂)ₙ₁S(O)ₘR_{dd}, - (CH₂)ₙ₁S(O)(=NR_{dd})(CH₂)ₙ₂Rₑₑ, -(CH₂)ₙ₁NR_{dd}Rₑₑ, -(CH₂)ₙ₁P(O)R_{dd}Rₑₑ, -(CH₂)ₙ₁C(O)NR_{dd}Rₑₑ, - (CH₂)ₙ₁NR_{dd}C(O)Rₑₑ, or -(CH₂)ₙ₁NR_{dd}S(O)ₘRₑₑ;
p is 0, 1, 2 or 3;
w is 0, 1, 2, 3, 4, 5 or 6;
ring A, ring B, X₁-X₅, L, R₂-R₃, Rₐₐ-Rₑₑ, y, z, n1, n2 and m are as defined in general formula (I).

The present disclosure further provides a preferred embodiment, the compound represented by general formula (II), the stereoisomer thereof or the pharmaceutically acceptable salt thereof, and the general formula (II) is further represented by general formula (III): 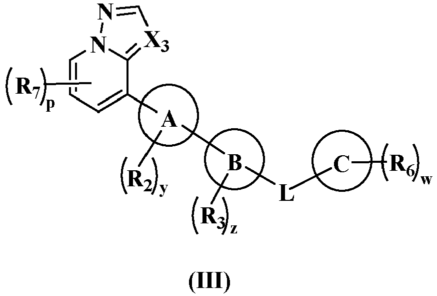 wherein:
X₃ is selected from N or CRs;
R₅ is selected from hydrogen, deuterium, alkyl, deuterated alkyl, haloalkyl, alkoxy, haloalkoxy, halogen, amino, nitro, hydroxyl, cyano, alkenyl, alkynyl, cycloalkyl, heterocyclyl, aryl, heteroaryl, -(CH₂)ₙ₁Rₐₐ, -(CH₂)ₙ₁ORₐₐ, -(CH₂)ₙ₁S(CH₂)ₙ₂Rₐₐ, -(CH₂)ₙ₁C(O)Rₐₐ, -(CH₂)ₙ₁C(O)ORₐₐ, -(CH₂)ₙ₁S(O)ₘRₐₐ, - (CH₂)ₙ₁S(O)=NRₐₐ)(CH₂)ₙ₂R_{bb}, -(CH₂)ₙ₁NRₐₐR_{bb}, -(CH₂)ₙ₁P(O)RₐₐR_{bb}, -(CH₂)ₙ₁C(O)NRₐₐR_{bb}, - (CH₂)ₙ₁NRₐₐC(O)R_{bb} or -(CH₂)ₙ₁NRₐₐS(O)ₘR_{bb};
ring A, ring B, ring C, X₃, L, R₂, R₃, R₆-R₇, Rₐₐ-R_{bb}, p, y, z, w, n1, n2 and m are as defined in general formula (II).

The present disclosure further provides a preferred embodiment, the compound represented by general formula (II), the stereoisomer thereof or the pharmaceutically acceptable salt thereof, and the general formula (II) is further represented by general formula (IV): 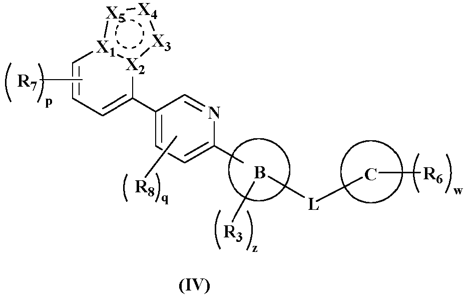 wherein:
R₈ is selected from hydrogen, deuterium, alkyl, deuterated alkyl, haloalkyl, alkoxy, haloalkoxy, halogen, amino, nitro, hydroxyl, cyano, alkenyl, alkynyl, cycloalkyl, heterocyclyl, aryl or heteroaryl;
q is 0, 1, 2, 3 or 4;
ring B, ring C, X₁-X₅, L, R₃, R₆-R₇, x, z and w are as defined in general formula (II).

The present disclosure further provides a preferred embodiment, the compound represented by general formula (II), the stereoisomer thereof or the pharmaceutically acceptable salt thereof, and the general formula (II) is further represented by general formula (V): 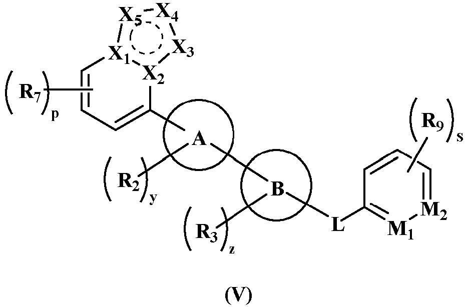 wherein:
M₁ and M₂ are each independently selected from CRₐₐ or N;
R₉ is selected from hydrogen, deuterium, alkyl, deuterated alkyl, haloalkyl, alkoxy, haloalkoxy, halogen, amino, nitro, hydroxyl, cyano, alkenyl, alkynyl, cycloalkyl, heterocyclyl, aryl, heteroaryl, -(CH₂)ₙ₁Rₐₐ, -(CH₂)ₙ₁ORₐₐ, -(CH₂)ₙ₁S(CH₂)ₙ₂Rₐₐ, -(CH₂)ₙ₁C(O)Rₐₐ, -(CH₂)ₙ₁C(O)ORₐₐ, -(CH₂)ₙ₁S(O)ₘRₐₐ, - (CH₂)ₙ₁S(O)(=NRₐₐ)(CH₂)ₙ₂R_{bb}, -(CH₂)ₙ₁NRₐₐR_{bb}, -(CH₂)ₙ₁P(O)RₐₐR_{bb}, -(CH₂)ₙ₁C(O)NRₐₐR_{bb}, - (CH₂)ₙ₁NRₐₐC(O)R_{bb} or -(CH₂)ₙ₁NRₐₐS(O)ₘR_{bb};
s is 0, 1, 2, 3, 4 or 5;
ring A, ring B, X₁-X₅, L, R₂, R₃, R₇, Pₐₐ, R_{bb}, p, y, z, n1, n2 and m are as defined in general formula (II).

The present disclosure further provides a preferred embodiment, the compound represented by general formula (II), the stereoisomer thereof or the pharmaceutically acceptable salt thereof, and the general formula (II) is further represented by general formula (VI): 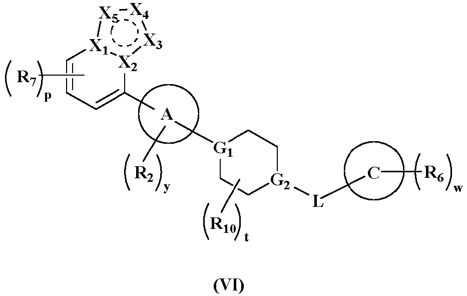 wherein:
G₁ and G₂ are each independently selected from CRₐₐ or N;
R₁₀ is selected from hydrogen, deuterium, alkyl, deuterated alkyl, haloalkyl, alkoxy, haloalkoxy, halogen, amino, nitro, hydroxyl, cyano, oxo, alkenyl, alkynyl, cycloalkyl, heterocyclyl, aryl or heteroaryl;
or, any two adjacent or non-adjacent R₁₀ are connected to form a cycloalkyl, heterocyclyl, aryl or heteroaryl, wherein the cycloalkyl, heterocyclyl, aryl and heteroaryl are optionally further substituted by one or more substituents selected from hydrogen, deuterium, substituted or unsubstituted alkyl, substituted or unsubstituted deuterated alkyl, substituted or unsubstituted haloalkyl, substituted or unsubstituted alkoxy, substituted or unsubstituted haloalkoxy, halogen, amino, nitro, hydroxyl, cyano, oxo, substituted or unsubstituted alkenyl, substituted or unsubstituted alkynyl, substituted or unsubstituted cycloalkyl, substituted or unsubstituted heterocyclyl, substituted or unsubstituted aryl, or substituted or unsubstituted heteroaryl;
t is 0, 1, 2, 3 or 4;
ring A, ring C, X₁-X₅, L, R₁, R₂, R₆-R₇, Pₐₐ, p, y and w are as defined in general formula (II).

The present disclosure further provides a preferred embodiment, the compound represented by general formula (II), the stereoisomer thereof or the pharmaceutically acceptable salt thereof, and the general formula (II) is further represented by general formula (VII): 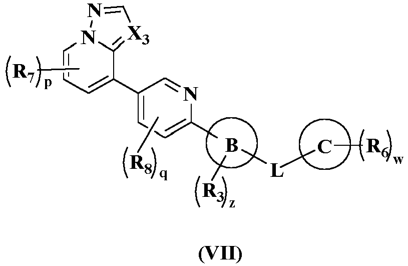 wherein:
ring B, ring C, X₃, L, R₆, R₇, p and w are as defined in general formula (III);
E, R₃, R₈, z and q are as described in general formula (IV).

The present disclosure further provides a preferred embodiment, the compound represented by general formula (II), the stereoisomer thereof or the pharmaceutically acceptable salt thereof, and the general formula (II) is further represented by general formula (VIII): 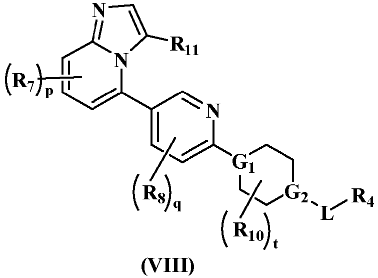 wherein:
L is selected from bond, -(CH₂)ₙ₁CRₐₐR_{bb}-, -(CH₂)ₙ₁NRₐₐC(O)(CH₂)ₙ₂-, - (CH₂)ₙ₁C(O)(CH₂)ₙ₂(CRₐₐR_{bb})ₘ-, -(CH₂)ₙ₁C(O)(CRₐₐR_{bb})ₘ(CH₂)ₙ₂-, -(CH₂)ₙ₁C(O)NR_{cc}(CRₐₐR_{bb})ₙ₂-, - (CH₂)ₙ₁(O)(CH₂)ₙ₂- or -(CH₂)ₙ₁NRₐₐ(CH₂)ₙ₂-;
G₁ and G₂ are each independently selected from CRₐₐ or N;
R₄ is selected from hydrogen, deuterium, alkyl, deuterated alkyl, haloalkyl, alkoxy, haloalkoxy, halogen, amino, nitro, hydroxyl, cyano, alkenyl, alkynyl, cycloalkyl, heterocyclyl, aryl, heteroaryl, -(CH₂)ₙ₁Rₐₐ, -(CH₂)ₙ₁ORₐₐ, -(CH₂)ₙ₁S(CH₂)ₙ₂Rₐₐ, -(CH₂)ₙ₁C(O)Rₐₐ, -(CH₂)ₙ₁C(O)ORₐₐ, -(CH₂)ₙ₁S(O)ₘRₐₐ, - (CH₂)ₙ₁S(O)(=NRₐₐ)(CH₂)ₙ₂R_{bb}, -(CH₂)ₙ₁NRₐₐR_{bb}, -(CH₂)ₙ₁P(O)RₐₐR_{bb}, -(CH₂)ₙ₁C(O)NRₐₐR_{bb}, - (CH₂)ₙ₁NRₐₐC(O)R_{bb} or -(CH₂)ₙ₁NRₐₐS(O)ₘR_{bb}, wherein the alkyl, deuterated alkyl, haloalkyl, alkoxy, haloalkoxy, alkenyl, alkynyl, cycloalkyl, heterocyclyl, aryl and heteroaryl are optionally further substituted by one or more substituents selected from hydrogen, deuterium, substituted or unsubstituted alkyl, substituted or unsubstituted deuterated alkyl, substituted or unsubstituted haloalkyl, substituted or unsubstituted hydroxyalkyl, substituted or unsubstituted cyanoalkyl, substituted or unsubstituted alkoxy, substituted or unsubstituted haloalkoxy, halogen, amino, nitro, hydroxyl, cyano, substituted or unsubstituted alkenyl, substituted or unsubstituted alkynyl, substituted or unsubstituted cycloalkyl, substituted or unsubstituted heterocyclyl, substituted or unsubstituted aryl, substituted or unsubstituted heteroaryl, -(CH₂)ₙ₁R_{cc}, - (CH₂)ₙ₁OR_{cc}, -(CH₂)ₙ₁S(CH₂)ₙ₂R_{cc}, -(CH₂)ₙ₁C(O)R_{cc}, -(CH₂)ₙ₁C(O)OR_{cc}, -(CH₂)ₙ₁S(O)ₘR_{cc}, - (CH₂)ₙ₁S(O)(=NR_{cc})(CH₂)ₙ₂R_{dd}, -(CH₂)ₙ₁NR_{cc}R_{dd}, -(CH₂)ₙ₁P(O)R_{cc}R_{dd}, -(CH₂)ₙ₁C(O)NR_{cc}R_{dd}, - (CH₂)ₙ₁NR_{cc}C(O)R_{dd} or -(CH₂)ₙ₁NR_{cc}S(O)ₘR_{dd};
R₇ is selected from hydrogen, deuterium, alkyl, deuterated alkyl, haloalkyl, alkoxy, haloalkoxy, halogen, amino, nitro, hydroxyl, cyano, alkenyl, alkynyl, cycloalkyl, heterocyclyl, aryl, heteroaryl, -(CH₂)ₙ₁Rₐₐ, -(C≡C)ₙ₁(CRₐₐR_{bb})ₘR_{cc}, -(C=C)ₙ₁(CRₐₐR_{bb})ₘR_{cc}, -(CH₂)ₙ₁O(CH₂)ₙ₂(CRₐₐR_{bb})ₘR_{cc}, -(CH₂)ₙ₁O(CH₂)ₙ₂Rₐₐ, - (CH₂)ₙ₁S(CH₂)ₙ₂Rₐₐ, -(CH₂)ₙ₁O(CH₂)ₙ₂S(O)ₘRₐₐ, -(CH₂)ₙ₁O(CH₂)ₙ₂S(O)(=NRₐₐ)(CH₂)ₘR_{bb}, -(CH₂)ₙ₁C(O)Rₐₐ, -(CH₂)ₙ₁C(O)ORₐₐ, -(CH₂)ₙ₁S(O)ₘRₐₐ, =(CH₂)ₙ₁S(O)(=NRₐₐ)(CH₂)ₙ₂R_{bb}, -(CH₂)ₙ₁NRₐₐR_{bb}, - (CH₂)ₙ₁P(O)RₐₐR_{bb}, -(CH₂)ₙ₁C(O)NRₐₐR_{bb}, -(CH₂)ₙ₁NRₐₐC(O)R_{bb}, -(CH₂)ₙ₁NRₐₐ(CH₂)ₙ₂R_{bb} or - (CH₂)ₙ₁NRₐₐS(O)ₘR_{bb}, wherein the alkyl, deuterated alkyl, haloalkyl, alkoxy, haloalkoxy, alkenyl, alkynyl, cycloalkyl, heterocyclyl, aryl and heteroaryl are optionally further substituted by one or more substituents selected from hydrogen, deuterium, substituted or unsubstituted alkyl, substituted or unsubstituted deuterated alkyl, substituted or unsubstituted haloalkyl, substituted or unsubstituted hydroxyalkyl, substituted or unsubstituted cyanoalkyl, substituted or unsubstituted alkoxy, substituted or unsubstituted haloalkoxy, halogen, substituted or unsubstituted amino, nitro, hydroxyl, cyano, oxo, thio, imino, substituted or unsubstituted alkenyl, substituted or unsubstituted alkynyl, substituted or unsubstituted cycloalkyl, substituted or unsubstituted heterocyclyl, substituted or unsubstituted aryl, substituted or unsubstituted heteroaryl, - (CH₂)ₙ₁R_{dd}, -(CH₂)ₙ₁OR_{dd}, (CH₂)ₙ₁S(CH₂)ₙ₂R_{dd}, -(CH₂)ₙ₁C(O)R_{dd}, -(CH₂)ₙ₁C(O)OR_{dd}, -(CH₂)ₙ₁S(O)ₘR_{dd}, - (CH₂)ₙ₁S(O)=NR_{dd})(CH₂)ₙ₂Rₑₑ, -(CH₂)ₙ₁NR_{dd}Rₑₑ, -(CH₂)ₙ₁P(O)R_{dd}Rₑₑ, -(CH₂)ₙ₁C(O)NR_{dd}Rₑₑ, - (CH₂)ₙ₁NR_{dd}C(O)Rₑₑ or -(CH₂)ₙ₁NR_{dd}S(O)ₘRₑₑ;
R₈ is selected from hydrogen, deuterium, alkyl, deuterated alkyl, haloalkyl, alkoxy, haloalkoxy, halogen, amino, nitro, hydroxyl, cyano, alkenyl, alkynyl, cycloalkyl, heterocyclyl, aryl or heteroaryl;
R₉ is selected from hydrogen, deuterium, alkyl, deuterated alkyl, haloalkyl, alkoxy, haloalkoxy, halogen, amino, nitro, hydroxyl, cyano, alkenyl, alkynyl, cycloalkyl, heterocyclyl, aryl, heteroaryl,-(CH₂)ₙ₁Rₐₐ, -(CH₂)ₙ₁ORₐₐ, -(CH₂)ₙ₁S(CH₂)ₙ₂Rₐₐ, -(CH₂)ₙ₁C(O)Rₐₐ, -(CH₂)ₙ₁C(O)ORₐₐ, -(CH₂)ₙ₁S(O)ₘRₐₐ, - (CH₂)ₙ₁S(O)(=NRₐₐ)(CH₂)ₙ₂R_{bb}, -(CH₂)ₙ₁NRₐₐR_{bb}, -(CH₂)ₙ₁P(O)RₐₐR_{bb}, -(CH₂)ₙ₁C(O)NRₐₐR_{bb}, - (CH₂)ₙ₁NRₐₐC(O)_{bb} or -(CH₂)ₙ₁NRₐₐS(O)ₘR_{bb};
R₁₀ is selected from hydrogen, deuterium, alkyl, deuterated alkyl, haloalkyl, alkoxy, haloalkoxy, halogen, amino, nitro, hydroxyl, cyano, oxo, alkenyl, alkynyl, cycloalkyl, heterocyclyl, aryl or heteroaryl;
or, any two adjacent or non-adjacent R₁₀ are connected to form a cycloalkyl, heterocyclyl, aryl or heteroaryl, wherein the cycloalkyl, heterocyclyl, aryl and heteroaryl are optionally further substituted by one or more substituents selected from hydrogen, deuterium, substituted or unsubstituted alkyl, substituted or unsubstituted deuterated alkyl, substituted or unsubstituted haloalkyl, substituted or unsubstituted alkoxy, substituted or unsubstituted haloalkoxy, halogen, amino, nitro, hydroxyl, cyano, oxo, substituted or unsubstituted alkenyl, substituted or unsubstituted alkynyl, substituted or unsubstituted cycloalkyl, substituted or unsubstituted heterocyclyl, substituted or unsubstituted aryl, and substituted or unsubstituted heteroaryl;
R₁₁ is selected from hydrogen, deuterium, alkyl, deuterated alkyl, haloalkyl, alkoxy, haloalkoxy, halogen, amino, nitro, hydroxyl, cyano, alkenyl, alkynyl, cycloalkyl, heterocyclyl, aryl, heteroaryl, -(CH₂)ₙ₁Rₐₐ, -(CH₂)ₙ₁ORₐₐ, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘRₐₐ, -(CH₂)ₙ₁NRₐₐR_{bb}, - (CH₂)ₙ₁P(O)RₐₐR_{bb}, -(CH₂)ₙ₁C(O)NRₐₐR_{bb}, -(CH₂)ₙ₁NRₐₐC(O)R_{bb} or -(CH₂)ₙ₁NRₐₐS(O)ₘR_{bb};
t is 0, 1, 2 or 3;
z is 0, 1, 2, 3, 4, 5 or 6;
p is 0, 1, 2 or 3;
q is 0, 1, 2, 3 or 4; and
s is 0, 1, 2, 3, 4 or 5.

The present disclosure further provides a preferred embodiment, the compound represented by general formula (II), the stereoisomer thereof or the pharmaceutically acceptable salt thereof, and the general formula (II) is further represented by general formula (IX): 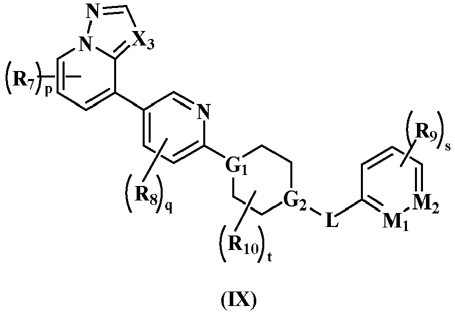 wherein:
X₃, L, R₇ and p are as defined in general formula (III);
R₈ and q are as described in general formula (IV);
M₁, M₂, R₉ and s are as defined in general formula (V);
G₁, G₂, R₁₀ and t are as defined in general formula (VI).

The present disclosure further provides a preferred embodiment, the compound represented by general formula (IX), the stereoisomer thereof or the pharmaceutically acceptable salt thereof, and the general formula (IX) is further represented by general formula (X): 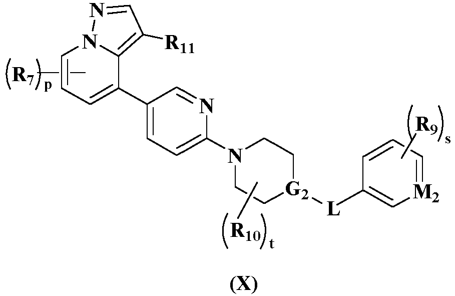 wherein:
G₂ is selected from CRₐₐ or N;
M₂ is selected from CRₐₐ or N;
L is selected from bond, -(CH₂)ₙ₁CRₐₐR_{bb}-, -(CH₂)ₙ₁NRₐₐC(O)(CH₂)ₙ₂-, - (CH₂)ₙ₁C(O)(CH₂)ₙ₂(CRₐₐR_{bb})ₘ-, -(CH₂)ₙ₁C(O)(CRₐₐR_{bb})ₘ(CH₂)ₙ₂-, -(CH₂)ₙ₁C(O)NR_{cc}(CRₐₐR_{bb})ₙ₂-, - (CH₂)ₙ₁(O)(CH₂)ₙ₂- or -(CH₂)ₙ₁NRₐₐ(CH₂)ₙ₂-;
R₇ is selected from hydrogen, deuterium, alkyl, deuterated alkyl, haloalkyl, alkoxy, haloalkoxy, halogen, amino, nitro, hydroxyl, cyano, alkenyl, alkynyl, cycloalkyl, heterocyclyl, aryl, heteroaryl, - (CH₂)ₙ₁(CRₐₐR_{bb})ₘR_{cc}, -(C=C)ₙ₁(CRₐₐR_{bb})ₘR_{cc}, -(C=C)ₙ₁(CRₐₐR_{bb})ₘR_{cc}, -(CH₂)ₙ₁O(CH₂)ₙ₂(CRₐₐR_{bb})ₘR_{cc}, - (CH₂)ₙ₁O(CH₂)ₙ₂Rₐₐ, -(CH₂)ₙ₁S(CH₂)ₙ₂(CRₐₐR_{bb})ₘR_{cc}, -(CH₂)ₙ₁O(CH₂)ₙ₂S(O)ₘRₐₐ, - (CH₂)ₙ₁O(CH₂)ₙ₂S(O)(=NRₐₐ)(CH₂)ₘR_{bb}, -(CH₂)ₙ₁C(O)Rₐₐ, -(CH₂)ₙ₁C(O)ORₐₐ, -(CH₂)ₙ₁S(O)ₘRₐₐ, - (CH₂)ₙ₁S(O)(=NRₐₐ)(CH₂)ₙ₂R_{bb}, -(CH₂)ₙ₁NRₐₐR_{bb}, -(CH₂)ₙ₁P(O)RₐₐR_{bb}, -(CH₂)ₙ₁C(O)NRₐₐR_{bb}, - (CH₂)ₙ₁NRₐₐC(O)R_{bb}, -(CH₂)ₙ₁NRₐₐ(CH₂)ₙ₂R_{bb} or -(CH₂)ₙ₁NRₐₐS(O)ₘR_{bb}, wherein the alkyl, deuterated alkyl, haloalkyl, alkoxy, haloalkoxy, alkenyl, alkynyl, cycloalkyl, heterocyclyl, aryl and heteroaryl are optionally further substituted by one or more substituents selected from hydrogen, deuterium, substituted or unsubstituted alkyl, substituted or unsubstituted deuterated alkyl, substituted or unsubstituted haloalkyl, substituted or unsubstituted hydroxyalkyl, substituted or unsubstituted cyanoalkyl, substituted or unsubstituted alkoxy, substituted or unsubstituted haloalkoxy, halogen, substituted or unsubstituted amino, nitro, hydroxyl, cyano, oxo, thio, imino, substituted or unsubstituted alkenyl, substituted or unsubstituted alkynyl, substituted or unsubstituted cycloalkyl, substituted or unsubstituted heterocyclyl, substituted or unsubstituted aryl, substituted or unsubstituted heteroaryl, -(CH₂)ₙ₁R_{dd}, -(CH₂)ₙ₁OR_{dd}, -(CH₂)ₙ₁S(CH₂)ₙ₂R_{dd}, -(CH₂)ₙ₁C(O)R_{dd}, - (CH₂)ₙ₁C(O)OR_{dd}, -(CH₂)ₙ₁S(O)ₘR_{dd}, -(CH₂)ₙ₁S(O)(=NR_{dd})(CH₂)ₙ₂Rₑₑ, -(CH₂)ₙ₁NR_{dd}Rₑₑ, -(CH₂)ₙ₁P(O)R_{dd}Rₑₑ, -(CH₂)ₙ₁C(O)NR_{dd}Rₑₑ, -(CH₂)ₙ₁NR_{dd}C(O)Rₑₑ or -(CH₂)ₙ₁NR_{dd}S(O)ₘRₑₑ;
R₉ is selected from hydrogen, deuterium, alkyl, deuterated alkyl, haloalkyl, alkoxy, haloalkoxy, halogen, amino, nitro, hydroxyl, cyano, alkenyl, alkynyl, cycloalkyl, heterocyclyl, aryl, heteroaryl, -(CH₂)ₙ₁Rₐₐ, -(CH₂)ₙ₁ORₐₐ, -(CH₂)ₙ₁S(CH₂)ₙ₂Rₐₐ, -(CH₂)ₙ₁C(O)Rₐₐ, -(CH₂)ₙ₁C(O)ORₐₐ, -(CH₂)ₙ₁S(O)ₘRₐₐ, - (CH₂)ₙ₁S(O)=NRₐₐ)(CH₂)ₙ₂R_{bb}, -(CH₂)ₙ₁NRₐₐR_{bb}, -(CH₂)ₙ₁P(O)RₐₐR_{bb}, -(CH₂)ₙ₁C(O)NRₐₐR_{bb}, - (CH₂)ₙ₁NRₐₐC(O)R_{bb} or -(CH₂)ₙ₁NRₐₐS(O)ₘR_{bb};
R₁₀ is selected from hydrogen, deuterium, alkyl, deuterated alkyl, haloalkyl, alkoxy, haloalkoxy, halogen, amino, nitro, hydroxyl, cyano, oxo, alkenyl, alkynyl, cycloalkyl, heterocyclyl, aryl or heteroaryl;
or, any two adjacent or non-adjacent R₁₀ are connected to form a cycloalkyl, heterocyclyl, aryl or heteroaryl, wherein the cycloalkyl, heterocyclyl, aryl and heteroaryl are optionally further substituted by one or more substituents selected from hydrogen, deuterium, substituted or unsubstituted alkyl, substituted or unsubstituted deuterated alkyl, substituted or unsubstituted haloalkyl, substituted or unsubstituted alkoxy, substituted or unsubstituted haloalkoxy, halogen, amino, nitro, hydroxyl, cyano, oxo, substituted or unsubstituted alkenyl, substituted or unsubstituted alkynyl, substituted or unsubstituted cycloalkyl, substituted or unsubstituted heterocyclyl, substituted or unsubstituted aryl, or substituted or unsubstituted heteroaryl;
R₁₁ is selected from hydrogen, deuterium, alkyl, deuterated alkyl, haloalkyl, alkoxy, haloalkoxy, halogen, amino, nitro, hydroxyl, cyano, alkenyl, alkynyl, cycloalkyl, heterocyclyl, aryl, heteroaryl, -(CH₂)ₙ₁Rₐₐ, -(CH₂)ₙ₁ORₐₐ, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘRₐₐ, -(CH₂)ₙ₁NRₐₐR_{bb}, - (CH₂)ₙ₁P(O)RₐₐR_{bb}, -(CH₂)ₙ₁C(O)NRₐₐR_{bb}, -(CH₂)ₙ₁NRₐₐC(O)R_{bb} or -(CH₂)ₙ₁NRₐₐS(O)ₘR_{bb};
Pₐₐ, R_{bb}, R_{cc}, R_{dd} and Rₑₑ are each independently selected from hydrogen, deuterium, alkyl, deuterated alkyl, haloalkyl, alkoxy, haloalkoxy, halogen, cyano, nitro, hydroxyl, amino, alkenyl, alkynyl, cycloalkyl, heterocyclyl, aryl and heteroaryl, wherein the alkyl, deuterated alkyl, haloalkyl, alkoxy, haloalkoxy, alkenyl, alkynyl, cycloalkyl, heterocyclyl, aryl and heteroaryl are optionally further substituted by one or more substituents selected from hydrogen, deuterium, substituted or unsubstituted alkyl, substituted or unsubstituted deuterated alkyl, substituted or unsubstituted haloalkyl substituted or unsubstituted, substituted or unsubstituted alkoxy, substituted or unsubstituted haloalkoxy, halogen, cyano, nitro, hydroxyl, amino, oxo, imino, substituted or unsubstituted alkenyl, substituted or unsubstituted alkynyl, substituted or unsubstituted cycloalkyl, substituted or unsubstituted heterocyclyl, substituted or unsubstituted aryl, or substituted or unsubstituted heteroaryl;
or, any two of Pₐₐ, R_{bb}, R_{cc}, R_{dd} and Rₑₑ are connected to form a cycloalkyl, heterocyclyl, aryl or heteroaryl, wherein the cycloalkyl, heterocyclyl, aryl and heteroaryl are optionally further substituted by one or more substituents selected from hydrogen, deuterium, substituted or unsubstituted alkyl, substituted or unsubstituted deuterated alkyl, substituted or unsubstituted haloalkyl substituted or unsubstituted, substituted or unsubstituted alkoxy, substituted or unsubstituted haloalkoxy, halogen, cyano, nitro, hydroxyl, amino, oxo, imine, substituted or unsubstituted alkenyl, substituted or unsubstituted alkynyl, substituted or unsubstituted cycloalkyl, substituted or unsubstituted heterocyclyl, substituted or unsubstituted aryl, or substituted or unsubstituted heteroaryl;
s is 0, 1, 2, 3, 4 or 5;
t is 0, 1, 2, 3 or 4; and
p is 0, 1, 2 , or 4.

The present disclosure further provides a preferred embodiment, the compound represented by general formula (X), the stereoisomer thereof or the pharmaceutically acceptable salt thereof, and the general formula (X) is further represented by general formula (XI) and (XI-A): 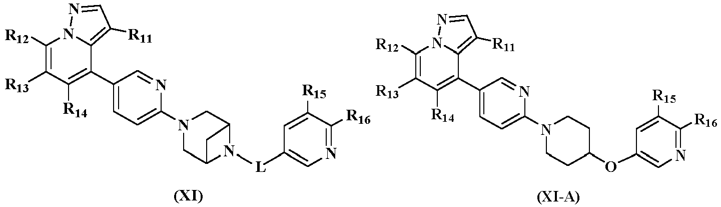 wherein:
R₁₂ is selected from hydrogen, deuterium, alkyl, deuterated alkyl, haloalkyl, alkoxy, haloalkoxy, halogen, amino, nitro, hydroxyl, cyano, alkenyl, alkynyl, cycloalkyl, heterocyclyl, aryl or heteroaryl;
R₁₃ is selected from hydrogen, deuterium, alkyl, deuterated alkyl, haloalkyl, alkoxy, haloalkoxy, halogen, amino, nitro, hydroxyl, cyano, alkenyl, alkynyl, cycloalkyl, heterocyclyl, aryl, heteroaryl, - (CH₂)ₙ₁(CRₐₐR_{bb})ₘR_{cc}, -(C≡C)ₙ₁(CRₐₐR_{bb})ₘR_{cc}, -(C=C)ₙ₁(CRₐₐR_{bb})ₘR_{cc}, -(CH₂)ₙ₁O(CH₂)ₙ₂(CRₐₐR_{bb})ₘR_{cc}, - (CH₂)ₙ₁ORₐₐ, -(CH₂)ₙ₁O(CH₂)ₙ₂Rₐₐ, -(CH₂)ₙ₁S(CH₂)ₙ₂(CRₐₐR_{bb})ₘR_{cc}, -(CH₂)ₙ₁O(CH₂)ₙ₂S(O)ₘRₐₐ, - (CH₂)ₙ₁O(CH₂)ₙ₂S(O)(=NRₐₐ)(CH₂)ₘR_{bb}, -(CH₂)ₙ₁C(O)Rₐₐ, -(CH₂)ₙ₁C(O)ORₐₐ, -(CH₂)ₙ₁S(O)ₘRₐₐ, - (CH₂)ₙ₁S(O)(=NRₐₐ)(CH₂)ₙ₂R_{bb}, -(CH₂)ₙ₁NRₐₐR_{bb}, -(CH₂)ₙ₁P(O)RₐₐR_{bb}, -(CH₂)ₙ₁C(O)NRₐₐR_{bb}, - (CH₂)ₙ₁NRₐₐC(O)R_{bb}, -(CH₂)ₙ₁NRₐₐ(CH₂)ₙ₂R_{bb} or -(CH₂)ₙ₁NRₐₐS(O)ₘR_{bb}, wherein the alkyl, deuterated alkyl, haloalkyl, alkoxy, haloalkoxy, alkenyl, alkynyl, cycloalkyl, heterocyclyl, aryl and heteroaryl are optionally further substituted by one or more substituents selected from hydrogen, deuterium, substituted or unsubstituted alkyl, substituted or unsubstituted deuterated alkyl, substituted or unsubstituted haloalkyl, substituted or unsubstituted hydroxyalkyl, substituted or unsubstituted cyanoalkyl, substituted or unsubstituted alkoxy, substituted or unsubstituted haloalkoxy, halogen, substituted or unsubstituted amino, nitro, hydroxyl, cyano, oxo, thio, imino, substituted or unsubstituted alkenyl, substituted or unsubstituted alkynyl, substituted or unsubstituted cycloalkyl, substituted or unsubstituted heterocyclyl, substituted or unsubstituted aryl, substituted or unsubstituted heteroaryl, -(CH₂)ₙ₁R_{dd}, -(CH₂)ₙ₁OR_{dd}, -(CH₂)ₙ₁S(CH₂)ₙ₂R_{dd}, -(CH₂)ₙ₁C(O)R_{dd}, - (CH₂)ₙ₁C(O)OR_{dd}, -(CH₂)ₙ₁S(O)ₘR_{dd}, -(CH₂)ₙ₁S(O)(=NR_{dd})(CH₂)ₙ₂Rₑₑ, -(CH₂)ₙ₁NR_{dd}Rₑₑ, -(CH₂)ₙ₁P(O)R_{dd}Rₑₑ, -(CH₂)ₙ₁C(O)NR_{dd}Rₑₑ, -(CH₂)ₙ₁NR_{dd}C(O)Rₑₑ or -(CH₂)ₙ₁NR_{dd}S(O)ₘRₑₑ;
R₁₂ is selected from hydrogen, deuterium, alkyl, deuterated alkyl, haloalkyl, alkoxy, haloalkoxy, halogen, amino, nitro, hydroxyl, cyano, alkenyl, alkynyl, cycloalkyl, heterocyclyl, aryl or heteroaryl;
R₁₅ and R₁₆ are each independently selected from hydrogen, deuterium, alkyl, deuterated alkyl, haloalkyl, alkoxy, haloalkoxy, halogen, amino, nitro, hydroxyl, cyano, alkenyl, alkynyl, cycloalkyl, heterocyclyl, aryl, heteroaryl or -(CH₂)ₙ₁ORₐₐ;
L, R₁₁, Rₐₐ-Rₑₑ, n1, n2 and m are as defined in general formula (X).

The present disclosure further provides a preferred embodiment, the compound represented by general formula (II), the stereoisomer thereof or the pharmaceutically acceptable salt thereof, and the general formula (II) is further represented by general formula (XII): 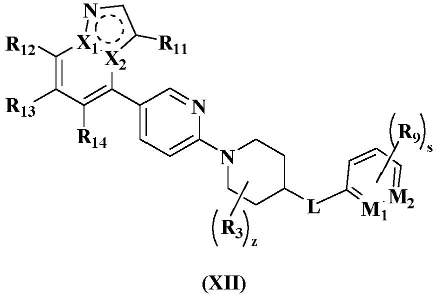 wherein:
L is selected from bond, -(CH₂)ₙ₁CRₐₐR_{bb}-, -(CH₂)ₙ₁NRₐₐC(O)(CH₂)ₙ₂-, - (CH₂)ₙ₁C(O)(CH₂)ₙ₂(CRₐₐR_{bb})ₘ-, -(CH₂)ₙ₁C(O)(CRₐₐR_{bb})ₘ(CH₂)ₙ₂-, -(CH₂)ₙ₁C(O)NR_{cc}(CRₐₐR_{bb})ₙ₂-, - (CH₂)ₙ₁(O)(CH₂)ₙ₂- or -(CH₂)ₙ₁NRₐₐ(CH₂)ₙ₂-;
M₁ and M₂ are each independently selected from CRₐₐ or N;
X₁ and X₂ are each independently selected from C, CRₐₐ or N;
R₃ is selected from hydrogen, deuterium, alkyl, deuterated alkyl, haloalkyl, alkoxy, haloalkoxy, halogen, amino, nitro, hydroxyl, cyano, oxo, alkenyl, alkynyl, cycloalkyl, heterocyclyl, aryl or heteroaryl;
R₉ is selected from hydrogen, deuterium, alkyl, deuterated alkyl, haloalkyl, alkoxy, haloalkoxy, halogen, amino, nitro, hydroxyl, cyano, alkenyl, alkynyl, cycloalkyl, heterocyclyl, aryl, heteroaryl, -(CH₂)ₙ₁Rₐₐ, -(CH₂)ₙ₁ORₐₐ, -(CH₂)ₙ₁S(CH₂)ₙ₂Rₐₐ, -(CH₂)ₙ₁C(O)Rₐₐ, -(CH₂)ₙ₁C(O)ORₐₐ, -(CH₂)ₙ₁S(O)ₘRₐₐ, - (CH₂)ₙ₁S(O)(=NRₐₐ)(CH₂)ₙ₂R_{bb}, -(CH₂)ₙ₁NRₐₐR_{bb}, -(CH₂)ₙ₁P(O)RₐₐR_{bb}, -(CH₂)ₙ₁C(O)NRₐₐR_{bb}, - (CH₂)ₙ₁NRₐₐC(O)R_{bb} or -(CH₂)ₙ₁NRₐₐS(O)ₘR_{bb};
R₁₁ is selected from hydrogen, deuterium, alkyl, deuterated alkyl, haloalkyl, alkoxy, haloalkoxy, halogen, amino, nitro, hydroxyl, cyano, alkenyl, alkynyl, cycloalkyl, heterocyclyl, aryl, heteroaryl, -(CH₂)ₙ₁Rₐₐ, -(CH₂)ₙ₁ORₐₐ, -(CH₂)ₙ₁SRₐₐ, -(CH₂)ₙ₁C(O)Rₐₐ, -(CH₂)ₙ₁C(O)ORₐₐ, -(CH₂)ₙ₁S(O)ₘRₐₐ, -(CH₂)ₙ₁NRₐₐR_{bb}, - (CH₂)ₙ₁P(O)RₐₐR_{bb}, (CH₂)ₙ₁C(O)NRₐₐR_{bb}, -(CH₂)ₙ₁NRₐₐC(O)R_{bb} or -(CH₂)ₙ₁NRₐₐS(O)ₘR_{bb};
z is 0, 1, 2, 3, 4 or 5;
s is 0, 1, 2, 3, 4 or 5;
R₁₂-R₁₄, Rₐₐ-Rₑₑ, n1, n2 and m are as defined in general formula (XI).

The present disclosure further provides a preferred embodiment, the compound represented by general formula (I), the stereoisomer thereof or the pharmaceutically acceptable salt thereof, and the general formula (I) is further represented by general formula (IX-A): 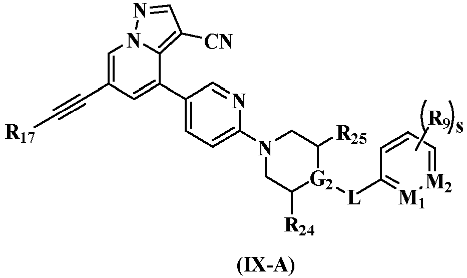 wherein:
L is selected from bond, -(CH₂)ₙ₁CRₐₐR_{bb}-, -(CH₂)ₙ₁NRₐₐC(O)(CH₂)ₙ₂-, - (CH₂)ₙ₁C(O)(CH)ₙ₂(CRₐₐR_{bb})ₘ-, -(CH₂)ₙ₁C(O)(CRₐₐR_{bb})ₘ(CH₂)ₙ₂-, -(CH₂)ₙ₁C(O)NR_{cc}(CRₐₐR_{bb})ₙ₂-, - (CH₂)ₙ₁(O)(CH₂)ₙ₂- or -(CH₂)ₙ₁NRₐₐ(CH₂)ₙ₂-,
preferably, -CH₂-, -CD₂-, -O- or -NHC(O)-;
G₂ is selected from N or CRₐₐ, preferably N, CH or CCH₃;
M₁ is selected from N or CRₐₐ, preferably N, CH or CCH₃;
M₂ is selected from N or CRₐₐ, preferably N or CH;
R₉ is selected from hydrogen, deuterium, C₁₋₆ alkyl, C₁₋₆ deuterated alkyl, C₁₋₆ haloalkyl, C₁₋₆ alkoxy,
C₁₋₆ deuterated alkoxy, C₁₋₆ haloalkoxy, halogen, amino, nitro, hydroxyl, cyano, C₂₋₆ alkenyl, C₂₋₆ alkynyl, C₃₋₈ cycloalkyl, 3-12 membered heterocyclyl, C₆₋₁₀ aryl, 5-12 membered heteroaryl or -(CH₂)ₙ₁ORₐₐ;
preferably hydrogen, halogen, C₁₋₆ alkoxy, C₁₋₆ deuterated alkoxy or -ORₐₐ;
most preferably hydrogen, fluorine, chlorine, methyl, methoxy, deuterated methoxy or cyclopropoxy;
R₁₇ is selected from C₁₋₆ alkyl, C₁₋₆ haloalkyl, C₁₋₆ alkoxy, C₂₋₆ alkynyl, 3-12 membered heterocyclyl, 5-12 membered heteroaryl or -(CH₂)ₙ₁(CRₐₐR_{bb}) R_{cc}, wherein the C₁₋₆ alkyl, C₁₋₆ haloalkyl, C₁₋₆ alkoxy, C₂₋₆ alkynyl, 3-12 membered heterocyclyl and 5-12 membered heteroaryl are optionally further substituted by one or more substituents selected from hydrogen, hydroxyl, cyano, oxo, thio, amino, imino, C₁₋₆ alkoxy, C₁₋₆ hydroxyalkyl, C₁₋₆ cyanoalkyl, C₃₋₈ cycloalkyl or 3-12 membered heterocyclyl;
R₂₄ and R₂₅ are each independently selected from hydrogen, deuterium, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, halogen, amino, nitro, hydroxyl, cyano, C₂₋₆ alkenyl, C₂₋₆ alkynyl, C₃₋₈ cycloalkyl, 3-12 membered heterocyclyl, C₆₋₁₀ aryl, 5-12 membered heteroaryl or -(CH₂)ₙ₁ORₐₐ, preferably hydrogen or methyl;
or, R₂₄ and R₂₅ together with the carbon atoms they are attached to and G2 form a C₃₋₈ cycloalkyl or 3-12 membered heterocyclyl, preferably azetidinyl;
Pₐₐ, R_{bb} and R_{cc} are each independently selected from hydrogen, deuterium, cyano, amino, C₁₋₆ alkyl, C₁₋₆ alkoxy, C₁₋₆ hydroxyalkyl, hydroxyl, C₃₋₈ cycloalkyl, 3- 12 membered heterocyclyl or C₆₋₁₄ aryl, wherein the C₁₋₆ alkyl, C₁₋₆ alkoxy, C₁₋₆ hydroxyalkyl, hydroxyl, C₃₋₈ cycloalkyl, 3-12 membered heterocylcyl and C₆₋₁₄ aryl are optionally further substituted by one or more substituents selected from hydrogen, halogen, cyano, hydroxyl, oxo, imino, C₁₋₆ alkyl or C₁₋₆ hydroxyalkyl;
or, any two of Rₐₐ, R_{bb} and R_{cc} are optionally connected to form a C₃₋₈ cycloalkyl or a 3-12 membered heterocyclyl, wherein the C₃₋₈ cycloalkyl and 3-12 membered heterocyclyl are optionally further substituted by one or more substituents selected from hydrogen, amino, halogen, cyano, hydroxyl, oxo, imino, C₁₋₆ alkyl or C₁₋₆ hydroxyalkyl;
n1 is 0, 1 or 2;
n2 is 0, 1 or 2;
m is 0, 1 or 2; and
s is 0, 1 , 2 or 3.

The present disclosure further provides a preferred embodiment, the compound represented by general formula (I), the stereoisomer thereof or the pharmaceutically acceptable salt thereof, and the general formula (I) is further represented by general formula (IX-B): 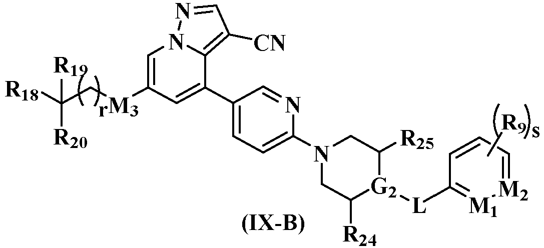 wherein:
M₃ is selected from bond, -O-, -S-, -NH- or -NCH₃-;
R₁₈ and R₁₉ are each independently selected from hydrogen, deuterium, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, halogen, amino, nitro, hydroxyl, cyano, C₂₋₆ alkenyl, C₂₋₆ alkynyl, C₃₋₈ cycloalkyl, 3-12 membered heterocyclyl, C₆₋₁₀ aryl or 5-12 membered heteroaryl, preferably hydrogen or methyl;
or, R₁₈ and R₁₉ together with the carbon atoms they are attached to form a C₃₋₈ cycloalkyl or a 3-12 membered heterocyclyl, preferably C₃₋₆ cycloalkyl or 3-7 membered heterocyclyl containing 1-2 oxygen atoms, nitrogen atoms or sulfur atoms, more preferably cyclopropyl, cyclobutyl, cyclopentyl, oxetanyl, azetidinyl, bicyclo[1,1,1]pentane or 1-imino-1-oxothiopyran, wherein the C₃₋₈ cycloalkyl or 3-12 membered heterocyclyl is optionally further substituted by one or more substituents selected from hydrogen, C₁₋₆ alkyl, hydroxyl, cyano or C₁₋₆ hydroxyalkyl;
R₂₀ is selected from hydrogen, deuterium, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, halogen, amino, nitro, hydroxyl, cyano, C₂₋₆ alkenyl, C₂₋₆ alkynyl, C₃₋₈ cycloalkyl, 3-12 membered heterocyclyl, C₆₋₁₀ aryl or 5-12 membered heteroaryl;
preferably hydrogen, cyano or amino;
R₂₄ and R₂₅ are each independently selected from hydrogen, deuterium, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, halogen, amino, nitro, hydroxyl, cyano, C₂₋₆ alkenyl, C₂₋₆ alkynyl, C₃₋₈ cycloalkyl, 3-12 membered heterocyclyl, C₆₋₁₀ aryl, 5-12 membered heteroaryl or -(CH₂)ₙ₁ORₐₐ, preferably hydrogen or methyl;
or, R₂₄ and R₂₅ together with the carbon atoms they are attached to and G2 form a C₃₋₈ cycloalkyl or 3-12 membered heterocyclyl, preferably azetidinyl;
r is 0, 1 or 2;
L, G₂, M₁, M₂, R₉, and s are as defined in general formula (IX-A).

The present disclosure further provides a preferred embodiment, the compound represented by general formula (I), the stereoisomer thereof or the pharmaceutically acceptable salt thereof, R₁₈ and R₁₉ in general formula (IX-B) are each independently selected from hydrogen, deuterium, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, halogen, amino, nitro, hydroxyl, cyano, C₂₋₆ alkenyl, C₂₋₆ alkynyl, C₃₋₈ cycloalkyl, 3-12 membered heterocyclyl, C₆₋₁₀ aryl or 5-12 membered heteroaryl, preferably hydrogen, methyl, ethynyl, amino, cyano or hydroxyl;
or, R₁₈ and R₁₉ together with the carbon atoms they are attached to form a C₃₋₈ cycloalkyl or a 3-12 membered heterocyclyl, preferably C₃₋₆ cycloalkyl or 3-7 membered heterocyclyl comprising 1-2 oxygen atoms, nitrogen atoms or sulfur atoms, more preferably cyclopropyl, cyclobutyl, cyclopentyl, oxetanyl, azetidinyl, tetrahydropyran, bicyclo[1,1,1]pentane or 1- imino-1-oxothiopyran, wherein the C₃₋₈ cycloalkyl or 3-12 membered heterocyclyl is optionally further substituted by one or more substituents selected from hydrogen, C₁₋₆ alkyl, halogen, hydroxyl, cyano, C₁₋₆ hydroxyalkyl and -(CH₂)ₙ₁C(O)NRₐₐR_{bb};
R₂₀ is selected from hydrogen, deuterium, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, halogen, amino, nitro, hydroxyl, cyano, C₂₋₆ alkenyl, C₂₋₆ alkynyl, C₃₋₈ cycloalkyl, 3-12 membered heterocyclyl, C₆₋₁₀ aryl or 5-12 membered heteroaryl, preferably hydrogen, methyl, ethynyl, amino, cyano or hydroxyl.

The present disclosure further provides a preferred embodiment, the compound, the stereoisomer thereof or the pharmaceutically acceptable salt thereof, the general formula (X) is further represented by general formula (XIII): 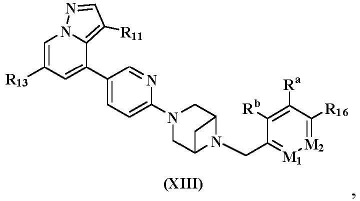 wherein:
R₁₁ is selected from hydrogen, deuterium, halogen, amino, nitro, hydroxyl, cyano, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₂₋₆ alkenyl or C₂₋₆ alkynyl;
R₁₃ is selected from hydrogen, deuterium, halogen, amino, nitro, hydroxyl, cyano, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₂₋₆ alkenyl, C₂₋₆ alkynyl, -O(CH₂)ₙ₁(CRₐₐR_{bb})ₘR_{cc} or 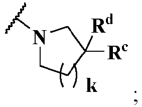
Rₐₐ, R_{bb} and R_{cc} are each independently selected from hydrogen, deuterium, halogen, amino, nitro, hydroxyl, cyano, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₂₋₆ alkenyl or C₂₋₆ alkynyl;
R^{c} and R^{d} are each independently selected from hydrogen, deuterium, halogen, amino, nitro, hydroxyl, cyano, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₂₋₆ alkenyl or C₂₋₆ alkynyl;
or, R^{c} and R^{d} together with the adjacent carbon atom form a C₃₋₈ cycloalkyl optionally substituted by one or more substituents selected from deuterium, halogen, amino, nitro, hydroxyl, cyano, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₂₋₆ alkenyl or C₂₋₆ alkynyl;
M₁ and M₂ are each independently selected from -N- or -CH-;
R₁₆ is selected from hydrogen, deuterium, halogen, amino, nitro, hydroxyl, cyano, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₂₋₆ alkenyl or C₂₋₆ alkynyl;
R^{a} and R^{b} are each independently selected from hydrogen, deuterium, halogen, amino, nitro, hydroxyl, cyano, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₂₋₆ alkenyl or C₂₋₆ alkynyl;
k is an integer of 0, 1 or 2;
n1 is an integer of 1, 2 or 3;
m is an integer of 1, 2 or 3.

The present disclosure further provides a preferred embodiment, the compound represented by general formula (XIII), the stereoisomer thereof or the pharmaceutically acceptable salt thereof, wherein:
R₁₁ is selected from hydrogen, deuterium, halogen, amino, nitro, hydroxyl, cyano or C₁₋₃ alkyl;
R₁₃ is selected from hydrogen, deuterium, halogen, amino, nitro, hydroxyl, cyano, C₁₋₃ alkyl, C₁₋₃ deuterated alkyl, C₁₋₃ haloalkyl, C₁₋₃ alkoxy, C₁₋₃ deuterium alkoxy, C₁₋₃ haloalkoxy, C₂₋₄ alkenyl, C₂₋₄ alkynyl, -OCH₂CRₐₐR_{bb}R_{cc} or 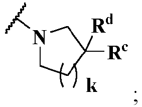
Pₐₐ, R_{bb} and R_{cc} are each independently selected from hydrogen, deuterium, halogen, amino, nitro, hydroxyl, cyano, C₁₋₃ alkyl, C₁₋₃ deuterated alkyl, C₁₋₃ haloalkyl, C₁₋₃ alkoxy, C₁₋3 deuterated alkoxy, C₁₋₃ haloalkoxy, C₂₋₄ alkenyl or C₂₋₄ alkynyl;
R^{c} and R^{d} are each independently selected from hydrogen, deuterium, halogen, amino, nitro, hydroxyl, cyano, C₁₋₃ alkyl, C₁₋₃ deuterated alkyl, C₁₋₃ haloalkyl, C₁₋₃ alkoxy, C₁₋₃ deuterated alkoxy or C₁₋₃ haloalkoxy;
or, R^{c} and R^{d} together with the adjacent carbon atom form a C₃₋₆ cycloalkyl optionally substituted by one or more substituents selected from deuterium, halogen, amino, nitro, hydroxyl, cyano, C₁₋₃ alkyl, C₁₋₃ deuterated alkyl, C₁₋₃ haloalkyl, C₁₋₃ alkoxy, C₁₋₃ deuterated alkoxy, C₁₋₃ haloalkoxy;
M₁ is -N-, M₂ is -CH-, or M₁ is -CH-, M₂ is -N-;
R₁₆ is selected from hydrogen, deuterium, halogen, amino, nitro, hydroxyl, cyano, C₁₋₃ alkyl, C₁₋₃ deuterated alkyl, C₁₋₃ haloalkyl, C₁₋₃ alkoxy, C₁₋₃ deuterated alkoxy or C₁₋₃ haloalkoxy;
R^{a} and R^{b} are each independently selected from hydrogen, deuterium or halogen.

The present disclosure further provides a preferred embodiment, the compound represented by general formula (I), the stereoisomer thereof or the pharmaceutically acceptable salt thereof, and the general formula (I) is further represented by general formula (X):

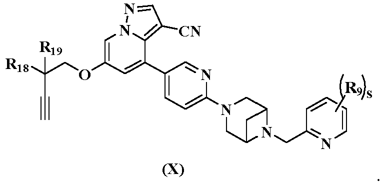

In a preferred embodiment of the present disclosure, R₁₈ and R₁₉ are each independently selected from hydrogen, deuterium, C₁₋₃ alkyl, C₁₋₃ deuterated alkyl, C₁₋₃ haloalkyl, C₁₋₃ alkoxy, C₁₋₃ deuterated alkoxy, C₁₋₃ haloalkoxy, halogen, amino, nitro, hydroxyl, cyano, C₂₋₄ alkenyl, C₂₋₄ alkynyl, C₃₋₆ cycloalkyl, 3-6 membered heterocyclyl, C₆₋₁₀ aryl or 5-6 membered heteroaryl, preferably hydroxyl or methyl;
R₉ is selected from hydrogen, deuterium, C₁₋₃ alkyl, C₁₋₃ deuterated alkyl, C₁₋₃ haloalkyl, C₁₋₃ alkoxy, C₁₋₃ deuterated alkoxy, C₁₋₃ haloalkoxy, halogen, amino, nitro, hydroxyl, cyano, C₂₋₄ alkenyl or C₂₋₄ alkynyl;
s is 0, 1 , 2 or 3.

The present disclosure further provides a preferred embodiment, the compound represented by general formula (I), the stereoisomer thereof or the pharmaceutically acceptable salt thereof, and 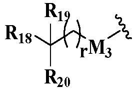 in general formula (IX-B) is selected from

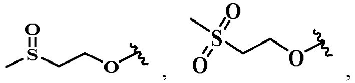

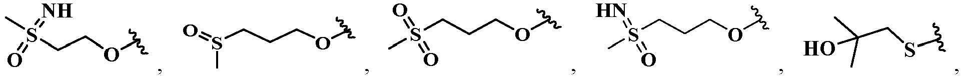

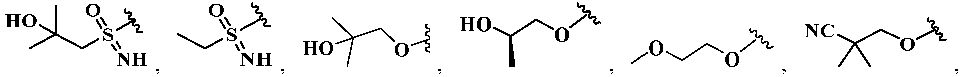

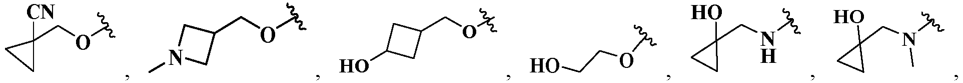

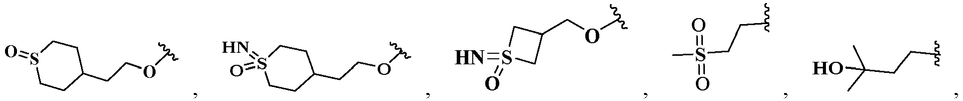

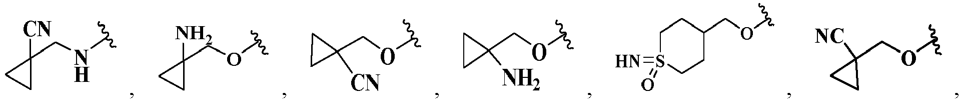

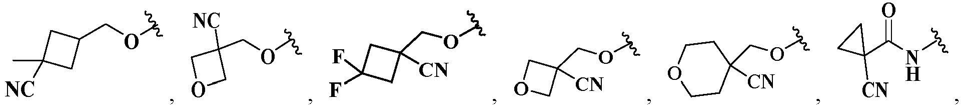

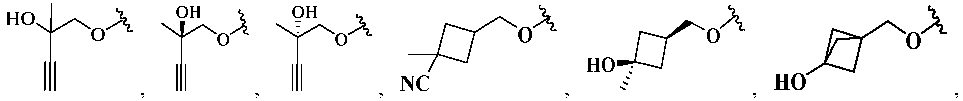

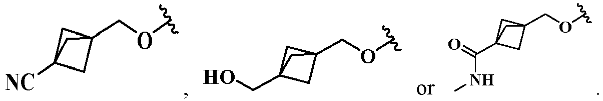

The present disclosure further provides a preferred embodiment, the compound represented by general formula (I), the stereoisomer thereof or the pharmaceutically acceptable salt thereof is further represented by general formula (IX-C): 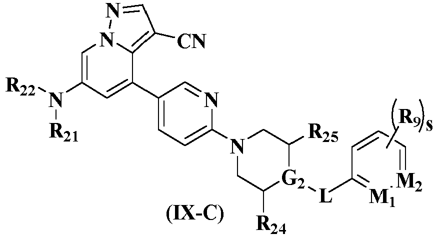 wherein:
R₂₁ and R₂₂ are each independently selected from hydrogen, deuterium, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, halogen, amino, nitro, hydroxyl, cyano, C₂₋₆ alkenyl, C₂₋₆ alkynyl, C₃₋₈ cycloalkyl, 3-12 membered heterocyclyl, C₆₋₁₀ aryl, 5-12 membered heteroaryl, -(CH₂)ₙ₁C(O)Rₐₐ or -(CH₂)ₙ₁Rₐₐ;
or, R₂₁ and R₂₂ together with the carbon atoms they are attached to form a 3-12 membered heterocyclyl, wherein the 3-12 membered heterocyclyl is optionally further substituted by one or more substituents selected from hydrogen, amino, halogen, cyano, hydroxyl, oxo, C₁₋₆ alkyl or C₁₋₆ hydroxyalkyl;
preferably azetidinyl, pyrrolidinyl, 2-azaspiro[3.3]heptane or piperidinyl, wherein the azetidinyl, pyrrolidinyl, 2-azaspiro[3.3]heptane or piperidinyl is optionally further substituted by one or more substituents selected from of hydrogen, C₁₋₆ alkyl, hydroxyl or hydroxyalkyl;
R₂₄ and R₂₅ are each independently selected from hydrogen, deuterium, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, halogen, amino, nitro, hydroxyl, cyano, C₂₋₆ alkenyl, C₂₋₆ alkynyl, C₃₋₈ cycloalkyl, 3-12 membered heterocyclyl, C₆₋₁₀ aryl, 5-12 membered heteroaryl or -(CH₂)ₙ₁ORₐₐ, preferably hydrogen or methyl;
or, R₂₄ and R₂₅ together with the carbon atoms they are attached to and G2 form a C₃₋₈ cycloalkyl or 3-12 membered heterocyclyl, preferably azetidinyl;
L, G₂, M₁, M₂, R₉, Pₐₐ, s and n1 are as defined in general formula (IX-A).

The present disclosure further provides a preferred embodiment, the compound represented by general formula (I), the stereoisomer thereof or the pharmaceutically acceptable salt thereof, R₂₁ and R₂₂ in general formula (IX-C) are each independently selected from hydrogen, deuterium, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, halogen, amino, nitro, hydroxyl, cyano, C₂₋₆ alkenyl, C₂₋₆ alkynyl, C₃₋₈ cycloalkyl, 3-12 membered heterocyclyl, C₆₋₁₀ aryl, 5-12 membered heteroaryl, -(CH₂)ₙ₁C(O)Rₐₐ or -(CH₂)ₙ₁Rₐₐ;
or, R₂₁ and R₂₂ together with the carbon atoms they are attached to form a 3-12 membered heterocyclyl, wherein the 3-12 membered heterocyclyl is optionally further substituted by one or more substituents selected from hydrogen, amino, halogen, cyano, hydroxyl, oxo, C₁₋₆ alkyl and C₁₋₆ hydroxyalkyl;
preferably azetidinyl, pyrrolidinyl, 2-azaspiro[3.3]heptane or piperidinyl, wherein the azetidinyl, pyrrolidinyl, 2-azaspiro[3.3]heptane or piperidinyl is optionally further substituted by one or more substituents selected from of hydrogen, C₁₋₃ alkyl, cyano, hydroxyl or hydroxyalkyl.

The present disclosure further provides a preferred embodiment, the compound represented by general formula (I), the stereoisomer thereof or the pharmaceutically acceptable salt thereof, 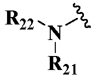 in general formula (IX-C) is selected from 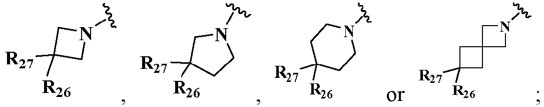
wherein, R₂₆ and R₂₇ are each independently selected from hydrogen, deuterium, halogen, amino, nitro, hydroxyl, cyano, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₁₋₆ hydroxyalkyl, C₂₋₆ alkenyl, C₂₋₆ alkynyl, C₃₋₈ cycloalkyl, 3-12 membered heterocyclyl, C₆₋₁₀ aryl or 5-12 membered heteroaryl;
preferably hydrogen, hydroxyl, cyano, C₁₋₃ alkyl or C₁₋₃ hydroxyalkyl;
more preferably hydrogen, hydroxyl, cyano, methyl or hydroxyisopropyl.

In a preferred embodiment of the present disclosure, L is selected from -CH₂-, -CD₂-, -O-, -S-, - C(O)NH- or -NHC(O)-;
G₂ is selected from -N-, -CH- or -CCH₃-;
M₁ is selected from -N-, -CH- or -CCH₃-;
M₂ is selected from - N or - CH-.

The present disclosure further provides a preferred embodiment, the compound represented by general formula (I), the stereoisomer thereof or the pharmaceutically acceptable salt thereof is further represented by general formula (IX-D): 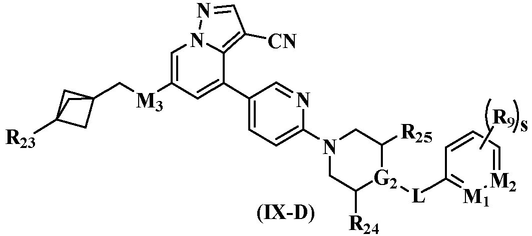 wherein:
R₂₃ is selected from hydrogen, deuterium, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₁₋₆ hydroxyalkyl, halogen, amino, nitro, hydroxyl, cyano, C₂₋₆ alkenyl, C₂₋₆ alkynyl, C₃₋₈ cycloalkyl, 3-12 membered heterocyclyl, C₆₋₁₀ aryl, 5-12 membered heteroaryl, - (CH₂)ₙ₁C(O)Rₐₐ or -(CH₂)ₙ₁Rₐₐ,
preferably hydroxyl, cyano or C₁₋₆ hydroxyalkyl;
L, G₂, M₁, M₂, M₃, R₉, Pₐₐ, s and n1 are as defined in general formula (IX-A).

The present disclosure further provides a preferred embodiment, the compound represented by general formula (I), the stereoisomer thereof or the pharmaceutically acceptable salt thereof, R₂₃ in general formula (IX-D) is selected from hydroxyl, cyano, C₁₋₆ hydroxyalkyl or -(CH₂)ₙ₁C(O)NRₐₐR_{bb}.

The present disclosure further provides a preferred embodiment, each of the compound represented by general formula, the stereoisomer thereof or the pharmaceutically acceptable salt thereof:
ring A is selected from the following groups: 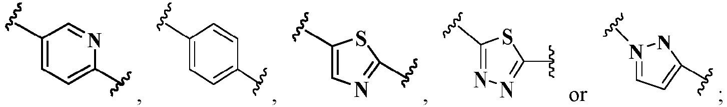
ring B is selected from the following groups: 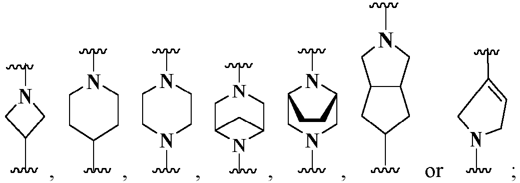
ring C is selected from the following groups:

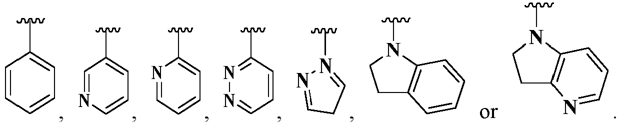

The present disclosure further provides a preferred embodiment, each of the compound represented by general formula, the stereoisomer thereof or the pharmaceutically acceptable salt thereof:
L is selected from bond, -(CH₂)ₙ₁CRₐₐR_{bb}-, -(CH₂)ₙ₁NRₐₐC(O)(CH₂)ₙ₂-, - (CH₂)ₙ₁C(O)(CRₐₐR_{bb})ₘ(CH₂)ₙ₂-, -(CH₂)ₙ₁C(O)(CH₂)ₘ(CRₐₐR_{bb})ₙ₂-, -(CH₂)ₙ₁C(O)NR_{cc}(CRₐₐR_{bb})ₙ₂-, - (CH₂)ₙ₁(O)(CH₂)ₙ₂- or -(CH₂)ₙ₁NRₐₐ(CH₂)ₙ₂-;
preferably -(CH₂)₂-, -(CD₂)₂-, -O-, -C(O)NH-, -C(O)N(CH₃)-, -NHC(O)- or -O(CH₂)₂-;
R₁ is selected from hydrogen, halogen, C₁₋₆ alkyl, C₁₋₆ alkoxy, C₁₋₆ hydroxyalkyl, oxo, C₂₋₆ alkynyl, 3-12 membered heterocyclyl, 5-12 membered heteroaryl, -(C≡C)ₙ₁(CRₐₐR_{bb})ₘR_{cc}, -(C=C)ₙ₁(CRₐₐR_{bb})ₘR_{cc}, - (CH₂)ₙ₁O(CH₂)ₙ₂(CRₐₐR_{bb})ₘR_{cc}, -(CH₂)ₙ₁O(CH₂)ₙ₂(CRₐₐR_{bb})ₘC(O)NHR_{cc}, -(CH₂)ₙ₁S(CH₂)ₙ₂(CRₐₐR_{bb})ₘR_{cc}, - (CH₂)ₙ₁O(CH₂)ₙ₂S(O)ₘRₐₐ, -(CH₂)ₙ₁O(CH₂)ₙ₂S(O)(=NRₐₐ)(CH₂)ₘR_{bb}, -(CH₂)ₙ₁O(CH₂)ₙ₂Rₐₐ, - (CH₂)ₙ₁NRₐₐ(CH₂)ₙ₂CR_{bb}, -(CH₂)ₙ₁NRₐₐC(O)R_{bb} or -(CH₂)ₙ₁S(O)(=NRₐₐ)(CH₂)ₙ₂R_{bb}, wherein the C₁₋₆ alkyl, C₁₋₆ alkoxy, C₂₋₆ alkynyl, 3-12 membered heterocyclyl and 5-12 membered heteroaryl are optionally further substituted by one or more substituents selected from hydrogen, deuterium, C₁₋₆ alkyl, C₁₋₆ haloalkyl, C₁₋₆ hydroxyalkyl, C₁₋₆ cyanoalkyl, C₁₋₆ alkoxy, halogen, hydroxyl, amino, cyano, oxo, thio, imino, C₃₋₈ cycloalkyl and 3-12 membered heterocyclyl;
R₂ is selected from hydrogen or halogen;
R₃ is selected from hydrogen, C₁₋₆ alkyl or amino; or, any two adjacent or non-adjacent R₃ are connected to form a C₃₋₈ cycloalkyl or 3-12 membered heterocyclyl;
R₄ is selected from C₁₋₆ alkyl, C₆₋₁₀ aryl or 5-12 membered heteroaryl, wherein the C₁₋₆ alkyl, C₆₋₁₀ aryl and 5-12 membered heteroaryl are optionally further substituted by one or more substituents selected from hydrogen, amino, C₁₋₆ alkyl, C₁₋₆ alkoxy, halogen, -(CH₂)ₙ₁ORₐₐ, -(CH₂)ₙ₁C(O)NRₐₐR_{bb} or - (CH₂)ₙ₁NRₐₐC(O)R_{bb};
R₅ is selected form cyano, -C(O)NRₐₐR_{bb} or -(CH₂)ₙ₁P(O)RₐₐR_{bb};
R₆ is selected from hydrogen, C₁₋₆ alkyl, C₁₋₆ alkoxy, halogen, amino, -(CH₂)ₙ₁ORₐₐ, - (CH₂)ₙ₁C(O)NRₐₐR_{bb} or -(CH₂)ₙ₁NRₐₐC(O)R_{bb};
R₇ is selected from hydrogen, C₁₋₆ alkyl, C₁₋₆ alkoxy, oxo, C₂₋₆ alkynyl, 3-12 membered heterocyclyl, 5-12 membered heteroaryl, -(C≡C)ₙ₁(CRₐₐR_{bb})ₘR_{cc}, -(C=C)ₙ₁(CRₐₐR_{bb})ₘR_{cc}, -(CH₂)ₙ₁O(CH₂)ₙ₂(CRₐₐR_{bb})ₘR_{cc}, -(CH₂)ₙ₁O(CH₂)ₙ₂(CRₐₐR_{bb})ₘC(O)NHR_{cc}, -(CH₂)ₙ₁S(CH₂)ₙ₂(CRₐₐR_{bb})ₘR_{cc}, -(CH₂)ₙ₁O(CH₂)ₙ₂S(O)ₘRₐₐ, - (CH₂)ₙ₁O(CH₂)ₙ₂S(O)(=NRₐₐ)(CH₂)ₘR_{bbb}, -(CH₂)ₙ₁O(CH₂)ₙ₂Rₐₐ, -(CH₂)ₙ₁NRₐₐ(CH₂)ₙ₂CR_{bb}, - (CH₂)ₙ₁NRₐₐC(O)R_{bb} or -(CH₂)ₙ₁S(O)(=NRₐₐ)(CH₂)ₙ₂R_{bb}, wherein the C₁₋₆ alkyl, C₁₋₆ alkoxy, C₂₋₆ alkynyl, 3-12 membered heterocyclyl and 5-12 membered heteroaryl are optionally further substituted by one or more substituents selected from hydrogen, deuterium, C₁₋₆ alkyl, C₁₋₆ haloalkyl, C₁₋₆ hydroxyalkyl, C₁₋₆ cyanoalkyl, C₁₋₆ alkoxy, halogen, hydroxyl, amino, cyano, oxo, thio, imino, C₃₋₈ cycloalkyl or 3-12 membered heterocyclyl;
R₈ is selected from hydrogen or halogen;
R₉ is selected from hydrogen, C₁₋₆ alkyl, C₁₋₆ alkoxy, halogen or -ORₐₐ;
R₁₀ is selected from hydrogen, C₁₋₆ alkyl or amino;
or, any two adjacent or non-adjacent R₁₀ are connected to form a C₃₋₈ cycloalkyl or 3-12 membered heterocyclyl;
R₁₁ is selected form cyano, -(CH₂)ₙ₁P(O)RₐₐR_{bb} or -(CH₂)ₙ₁C(O)NRₐₐR_{bb};
R₁₂ is selected from hydrogen, C₁₋₆ alkyl or halogen;
R₁₃ is selected from C₁₋₆ alkyl, C₁₋₆ alkoxy, C₂₋₆ alkynyl, 3-12 membered heterocyclyl, 5-12 membered heteroaryl, -(C≡C)ₙ₁(CRₐₐR_{bb})ₘR_{cc}, -(C=C)ₙ₁(CRₐₐR_{bb})ₘR_{cc}, -(CH₂)ₙ₁O(CH₂)ₙ₂(CRₐₐR_{bb})ₘR_{cc}, - (CH₂)ₙ₁O(CH₂)ₙ₂(CRₐₐR_{bb})ₘC(O)NHR_{cc}, -(CH₂)ₙ₁S(CH₂)ₙ₂(CRₐₐR_{bb})ₘR_{cc}, -(CH₂)ₙ₁O(CH₂)ₙ₂S(O)ₘRₐₐ, - (CH₂)ₙ₁O(CH₂)ₙ₂S(O)(=NRₐₐ)(CH₂)ₘR_{bb}, -(CH₂)ₙ₁O(CH₂)ₙ₂Rₐₐ, -(CH₂)ₙ₁NRₐₐ(CH₂)ₙ₂CR_{bb}, - (CH₂)ₙ₁NRₐₐC(O)R_{bb} or -(CH₂)ₙ₁S(O)(=NRₐₐ)(CH₂)ₙ₂R_{bb}, wherein the C₁₋₆ alkyl, C₁₋₆ alkoxy, C₂₋₆ alkynyl, 3-12 membered heterocyclyl and 5-12 membered heteroaryl are optionally further substituted by one or more substituents selected from hydrogen, C₁₋₆ alkyl, hydroxyl, thio, imino, C₁₋₆ alkoxy, C₁₋₆ hydroxyalkyl, C₁₋₆ cyanoalkyl, C₃₋₈ cycloalkyl or 3-12 membered heterocyclyl;
R₁₄ is selected from hydrogen or halogen;
R₁₅ is selected from hydrogen or halogen;
R₁₆ is selected from hydrogen, alkoxy or -ORₐₐ;
Rₐₐ, R_{bb} and R_{cc} are each independently selected from hydrogen, deuterium, cyano, amino, C₁₋₆ alkyl, C₁₋₆ alkoxy, C₁₋₆ hydroxyalkyl, hydroxyl, C₃₋₈ cycloalkyl, 3- 12 membered heterocyclyl or C₆₋₁₄ aryl, wherein the C₁₋₆ alkyl, C₁₋₆ alkoxy, C₁₋₆ hydroxyalkyl, hydroxyl, C₃₋₈ cycloalkyl, 3-12 membered heterocyclyl and C₆₋₁₄ aryl are optionally further substituted by one or more substituents selected from hydrogen, halogen, cyano, hydroxyl, oxo, imino, C₁₋₆ alkyl or C₁₋₆ hydroxyalkyl;
or, any two of Rₐₐ, R_{bb} and R_{cc} are optionally connected to form a C₃₋₈ cycloalkyl or a 3-12 membered heterocyclyl, wherein the C₃₋₈ cycloalkyl and 3-12 membered heterocyclyl are optionally further substituted by one or more substituents selected from hydrogen, halogen, cyano, hydroxyl, oxo, imino, C₁₋₆ alkyl or C₁₋₆ hydroxyalkyl;

The present disclosure also relates to a method for preparing the compound represented by general formula (IX-A), the stereoisomer thereof or the pharmaceutically acceptable salt thereof, comprising the following steps, 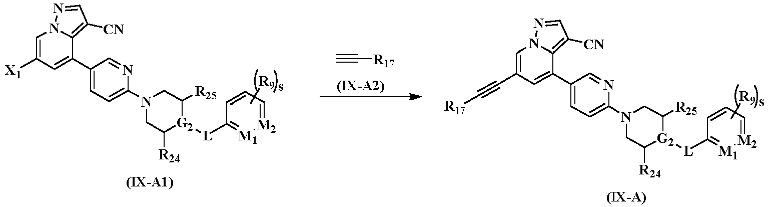
a coupling reaction is carried out with general formula (IX-A1) and general formula (IX-A2) to obtain the compound represented by general formula (IX-A) or the stereoisomer thereof and the pharmaceutically acceptable salt thereof;
wherein:
X₁ is selected from halogen; preferably fluorine, chlorine, bromine or iodine; more preferably bromine.

The present disclosure also relates to a method for preparing the compound represented by general formula (IX-B), the stereoisomer thereof or the pharmaceutically acceptable salt thereof, comprising the following steps, 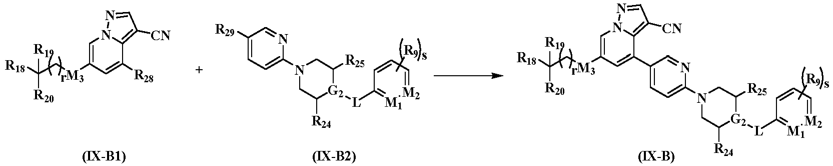
a reaction is carried out with general formula (IX-B1) and general formula (IX-B2) to obtain the compound represented by general formula (IX-B) or the stereoisomer thereof and the pharmaceutically acceptable salt thereof;
wherein:
R₂₈ is selected from halogen, -B(OH)₂ or borate ester; preferably fluorine, chlorine, bromine, iodine, -B(OH)₂ or 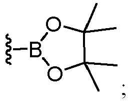
R₂₉ is selected from halogen, boric acid or borate ester; preferably fluorine, chlorine, bromine, iodine, -B(OH)₂ or 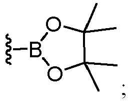
when R₂₈ is halogen, R₂₉ is selected from boric acid or borate ester;
when R₂₈ is selected from boric acid or borate ester, R₂₉ is halogen.

The present disclosure also relates to a method for preparing the compound represented by general formula (IX-B), the stereoisomer thereof or the pharmaceutically acceptable salt thereof, comprising the following steps, 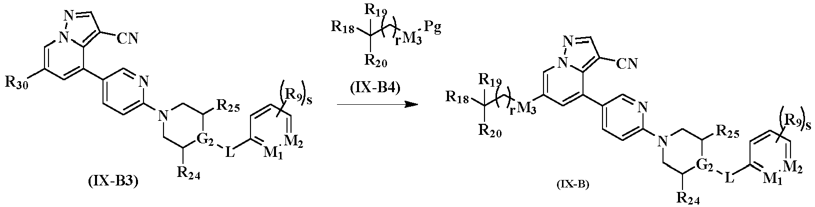
a reaction is carried out with general formula (IX-B3) and general formula (IX-B4) to obtain the compound represented by general formula (IX-B) or the stereoisomer thereof and the pharmaceutically acceptable salt thereof;
wherein:
M₃ is selected from bond, -O-, -S-, -NH- or -NCH₃-;
R₃₀ is selected from halogen, hydroxyl; preferably fluorine, chlorine, bromine, iodine, or hydroxyl; more preferably bromine or hydroxyl;
Pg is selected from hydrogen, halogen or hydroxyl protecting group, and the halogen is preferably fluorine, chlorine, bromine or iodine;
when Pg is a hydroxyl protecting group, it is selected from methyl, *tert-*butyl, triphenyl, methyl sulfide methyl ether, 2-methoxyethoxymethyl ether, methoxymethyl ether, *p*-methoxybenzyl ether, pivaloyl, benzyl ether, methoxymethyl, trimethylsilyl, tetrahydrofuranyl, *tert*-butyldisilyl, acetyl, benzoyl or *p-*toluenesulfonyl; preferably*p*-toluenesulfonyl;
when M₃ is -O-, Pg is selected from hydrogen or hydroxyl protecting group.

The present disclosure also relates to a method for preparing the compound represented by general formula (IX-C), the stereoisomer thereof or the pharmaceutically acceptable salt thereof, comprising the following steps, 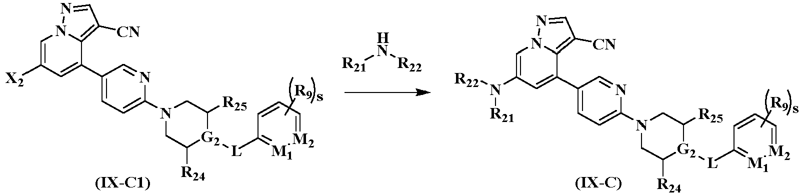
a reaction is carried out with general formula (IX-C1) and general formula (IX-C2) to obtain the compound represented by general formula (IX-B) or the stereoisomer thereof and the pharmaceutically acceptable salt thereof;
wherein:
X₂ is selected from halogen; preferably fluorine, chlorine, bromine or iodine; more preferably bromine.

The present disclosure also relates to a method for preparing the compound represented by general formula (X), the stereoisomer thereof or the pharmaceutically acceptable salt thereof, wherein the method comprises the following steps, 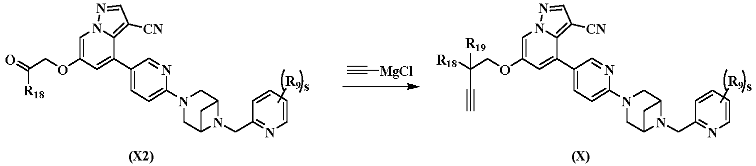 or 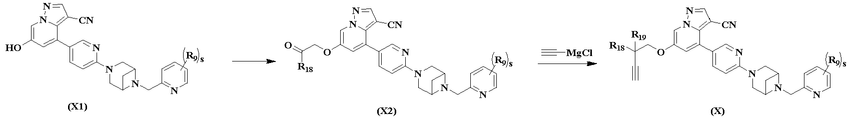 wherein:
R₁₈ and R₁₉ are each independently selected from hydrogen, deuterium, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, halogen, amino, nitro, hydroxyl, cyano, C₂₋₆ alkenyl, C₂₋₆ alkynyl, C₃₋₈ cycloalkyl, 3-12 membered heterocyclyl, C₆₋₁₀ aryl or 5-12 membered heteroaryl;
or, R₁₈ and R₁₉ together with the carbon atoms they are attached to form a C₃₋₈ cycloalkyl or a 3-12 membered heterocyclyl, preferably C₃₋₆ cycloalkyl or 3-7 membered heterocyclyl comprising 1-2 oxygen atoms, nitrogen atoms or sulfur atoms, more preferably cyclopropyl, cyclobutyl, cyclopentyl, oxetanyl, azetidinyl, bicyclo[1,1,1]pentane or 1- imino-1-oxothiopyran, wherein the C₃₋₈ cycloalkyl or 3-12 membered heterocyclyl is optionally further substituted by one or more substituents selected from hydrogen, C₁₋₆ alkyl, hydroxyl, cyano and C₁₋₆ hydroxyalkyl;
preferably, R₁₈ and R₁₉ are each independently selected from hydrogen, methyl or hydroxyl.

The present disclosure also provides a pharmaceutical composition comprising a therapeutically effective amount of the compound represented by each of the general formula and the stereoisomer thereof or the pharmaceutically acceptable salt thereof, and one or more pharmaceutically acceptable carriers, diluents or excipients.

The present disclosure further provides a preferred embodiment, related to a use of the compound represented by each of the general formula, the stereoisomer thereof or the pharmaceutically acceptable salt thereof, or the pharmaceutical composition in the preparation of medicaments related to RET inhibitor.

The present disclosure also provides a preferred embodiment, related to a use of the compound represented by general formula (I) and the stereoisomer thereof or the pharmaceutically acceptable salt thereof, or the pharmaceutical composition for the preparation of medicaments for the treatment and/or prevention of non-small cell lung cancer, fibrosarcoma, pancreatic tumor, medullary thyroid carcinoma, thyroid papillary tumor, soft tissue sarcoma, highly solid tumor, breast tumor and colon tumor and other related diseases.

The present disclosure further relates to a method of using the compound represented by general formula (I), the stereoisomer thereof or the pharmaceutically acceptable salt thereof, or the pharmaceutical composition for the preparation of medicaments for the treatment and/or prevention of non-small cell lung cancer, fibrosarcoma, pancreatic tumor, medullary thyroid carcinoma, thyroid papillary tumor, soft tissue sarcoma, highly solid tumor, breast tumor and colon tumor and other related diseases.

The present disclosure also relates to a method for the treatment and/or prevention of non-small cell lung cancer, fibrosarcoma, pancreatic tumor, medullary thyroid carcinoma, papillary thyroid tumor, soft tissue sarcoma, highly solid tumors, breast tumors, colon tumors and other related diseases, comprising administering a therapeutically effective amount of the compound of the present disclosure or the pharmaceutically acceptable salt, solvate or hydrate thereof to a mammal.

In some embodiments of the present disclosure, the method relates to, such as, the treatment and/or prevention of non-small cell lung cancer, fibrosarcoma, pancreatic tumor, medullary thyroid carcinoma, thyroid papillary tumor, soft tissue sarcoma, highly solid tumor, breast tumor and the treatment of colon tumor and other related diseases.

The methods for the treatment provided herein include administering a therapeutically effective amount of the compound of the disclosure to a subject. In an embodiment, the present disclosure provides a method for the treatment of diseases including menopausal hot flush related diseases in mammals. The method comprises administering a therapeutically effective amount of the compound of the present disclosure or the pharmaceutically acceptable salt, solvate or hydrate thereof to a mammal.

### Detailed description of the invention

Unless otherwise stated, the terms used in the description and claims have the following meanings.

The term "alkyl" refers to a saturated aliphatic hydrocarbon group, which is a straight or branched chain group comprising 1 to 20 carbon atoms, preferably alkyl comprising 1 to 8 carbon atoms, more preferably alkyl comprising 1 to 6 carbon atoms, the most preferably alkyl contianing 1 to 3 carbon atoms. Non-limiting examples include methyl, ethyl, *n*-propyl, isopropyl, *n*-butyl, isobutyl, *tert-*butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n-*hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n-*nonyl, 2-methyl-2-ethylhexyl, 2-methyl-3-ethylhexyl, 2,2-diethylpentyl, *n*-decyl, 3,3-diethylhexyl, 2,2-diethylhexyl, and various branched isomers. More preferrably lower alkyl comprising 1 to 6 carbon atoms, non-limiting examples include methyl, ethyl, *n*-propyl, isopropyl, *n*-butyl, isobutyl, *tert-*butyl, *sec*-butyl, *n-*pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. The alkyl may be substituted or unsubstituted, when substituted, the substituents may be substituted at any available attachment point, the substituents are preferably one or more of the following groups, which are independently selected from alkyl, alkenyl, alkynyl, alkoxy, alkylthio, alkylamino, halogen, sulfhydryl, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl, or carboxylate; alkyl substituted by methyl, ethyl, isopropyl, *tert-butyl,* haloalkyl, deuterated alkyl, alkoxy-substituted alkyl and alkyl substituted by hydroxyl are preferred in the present disclosure.

The term "alkylidene" refers to that one hydrogen atom of an alkyl is further substituted, for example: "methylene" refers to -CH₂-, "ethylene" refers to -(CH₂)₂-, and "propylene" refers to -(CH₂)₃-, "butylene" refers to -(CH₂)₄-, etc. The term "alkenyl" refers to an alkyl as defined above comprising at least two carbon atoms and at least one carbon-carbon double bond, such as vinyl, 1-propenyl, 2-propenyl, 1-, 2-, or 3-butenyl etc. The alkenyl may be substituted or unsubstituted, when substituted, the substituents are preferably one or more of the following groups, which are independently selected from alkyl, alkenyl, alkynyl, alkoxy, alkylthio, alkylamino, halogen, sulfhydryl, hydroxyl, nitro, cyano, cycloalkyl, heterocycloalkyl, aryl, heteroaryl, cycloalkoxy, heterocycloalkoxy, cycloalkylthio, heterocycloalkylthio.

The term "cycloalkyl" refers to a saturated or partially unsaturated monocyclic or polycyclic cyclic hydrocarbon substituent, the cycloalkyl ring contains 3 to 20 carbon atoms, preferably 3 to 12 carbon atoms, more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctanyl, etc.; polycyclic cycloalkyl includes spiro, fused and bridged cycloalkyls, preferably cyclopropyl, cyclobutyl, cyclohexyl, cyclopentyl and cycloheptyl.

The term "spirocycloalkyl" refers to polycyclic group that shares one carbon atom (called a spiro atom) between 5- to 20-membered monocyclic rings, which may contain one or more double bonds, but none of the rings have complete conjugate π electron system. Preferably 6-14 membered, more preferably 7-10 membered. According to the number of shared spiro atoms between the rings, the spirocycloalkyl is classified into single spirocycloalkyl, bispirocycloalkyl or polyspirocycloalkyl, preferably single spirocycloalkyl and bispirocycloalkyl. More preferably, 4-membered/4-membered, 4-membered/5-membered, 4-membered/6-membered, 5-membered/5-membered, or 5-membered/6-membered monospirocycloalkyl. Non-limiting examples of spirocycloalkyls include: 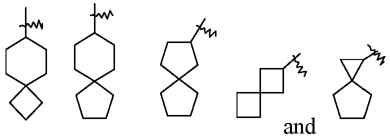 also include spirocycloalkyl in which single spirocycloalkyl and heterocycloalkyl share a spiro atom, non-limiting examples include:

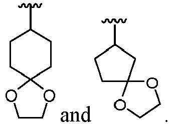

The term "fused cycloalkyl" refers to a 5-20 membered all-carbon polycyclic group in which each ring in the system shares an adjacent pair of carbon atoms with other rings in the system, wherein one or more of the rings may comprise one or multiple double bonds, but none of the ring has a fully conjugated x-electron system. Preferably 6-14 membered, more preferably 7-10 membered. According to the number of constituent rings, it can be classified into bicyclic, tricyclic, tetracyclic or polycyclic fused cycloalkyl, preferably bicyclic or tricyclic, and more preferably 5-membered/5-membered or 5-membered/6-membered bicyclic alkyl. Non-limiting examples of fused cycloalkyls include:

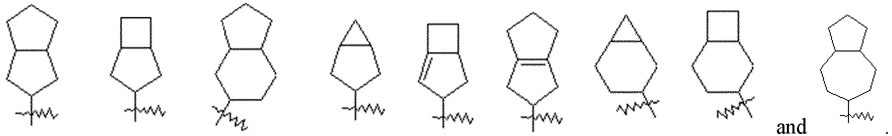

The term "bridged cycloalkyl" refers to 5-20 membered all-carbon polycyclic group, in which any two rings share two carbon atoms that are not directly connected, it may contain one or more double bonds, but none of the ring has a complete conjugated π electron system. Preferably 6-14 membered, more preferably 7-10 membered. According to the number of constituent rings, it can be classified into bicyclic, tricyclic, tetracyclic or polycyclic bridged cycloalkyl, preferably bicyclic, tricyclic,or tetracyclic, and more preferably bicyclic or tricyclic. Non-limiting examples of bridge ring alkyl include:

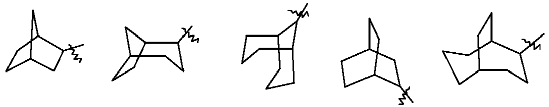

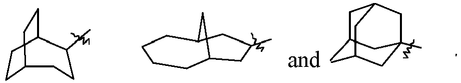

The cycloalkyl ring may be fused to an aryl, heteroaryl or heterocycloalkyl ring, wherein the ring connected to the parent structure is cycloalkyl, non-limiting examples include indanyl, tetrahydronaphthyl, benzocycloheptanyl, etc. The cycloalkyl may be substituted or unsubstituted, when substituted, the substituents are preferably one or more of the following groups, which are independently selected from alkyl, alkenyl, alkynyl, alkoxy, alkylthio, alkylamino, halogen, sulfhydryl, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl or carboylate.

The term "heterocyclyl" refers to saturated or partially unsaturated monocyclic or polycyclic hydrocarbon substituent comprising 3 to 20 ring atoms, wherein one or more of the ring atoms are heteroatoms selected from nitrogen, oxygen, C(O), S(O)(=NH) or S(O)ₘ (wherein m is an integer of 0 to 2), but not including the ring part of -O-O-, -O-S- or -S-S-, and the remaining ring atoms are carbon. It preferably contains 3 to 12 ring atoms, wherein 1 to 4 ring atoms are heteroatoms; more preferably contains 3 to 8 ring atoms; most preferably contains 3 to 8 ring atoms. Non-limiting examples of monocyclic heterocyclic include oxetane, trimethylene sulfide, azetidine, tetrahydropyranyl, azepanyl, pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl, etc., preferably oxetane, trimethylene sulfide, azetidine, tetrahydrofuranyl, tetrahydropyranyl, 1-imino-1-oxothiopyran, azepanyl, piperidinyl and piperazinyl. Polycyclic heterocyclyl includes spiro, fused and bridged heterocyclyl; the spiro, fused and bridged heterocyclyl are optionally connected to other groups through a single bond, or connect to other cycloalkyl, heterocyclyl, aryl and heteroaryl through any two or more of ring atoms. The heterocyclyl may be substituted or unsubstituted, when substituted, the substituent is preferably one or more of the following groups independently selected from hydrogen, alkyl, hydroxyalkyl, amino, imino, cyano, oxo, cycloalkyl, heterocycloalkyl, aryl, heteroaryl.

The term "spiroheterocyclyl" refers to polycyclic heterocyclic group sharing one atom (called a spiro atom) between 5-20 membered monocyclic ring, wherein one or more ring atoms are selected from nitrogen, oxygen, S(O) (=NH) or S(O)ₘ (wherein m is an integer of 0 to 2) heteroatoms, and the remaining ring atoms are carbon. It may contain one or more double bonds, but none of the ring have complete conjugate π electron system. Preferably 6-14 membered, more preferably 7-10 membered. According to the number of spiro atoms shared between the rings, the spiro heterocyclyl is classified into single spiro heterocyclyl, dispiro heterocyclyl or polyspiro heterocyclyl, preferably single spiro heterocyclyl and dispiro heterocyclyl. More preferably, 4-membered/4-membered, 4-membered/5-membered, 4-membered/6-membered, 5-membered/5-membered, or 5-membered/6-membered monospirocyclyl. Non-limiting examples of spiroheterocyclyl include:

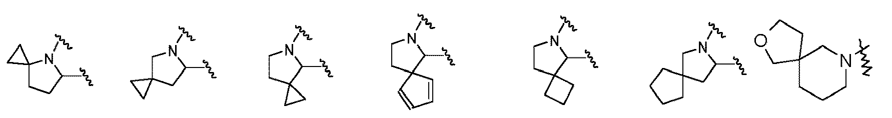

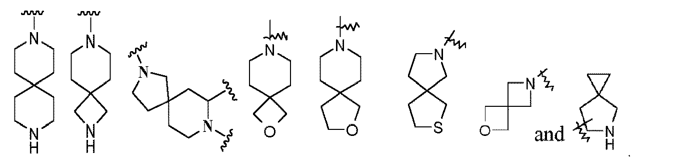

The term "fused heterocyclyl" refers to a 5-20 membered polycyclic heterocyclic group in which each ring in the system shares an adjacent pair of atoms with other rings in the system, one or more of the rings may comprise one or multiple double bonds, but none of the ring has a fully conjugated n-electron system, wherein one or more of the ring atoms are heteroatoms selected from nitrogen, oxygen or S(O)ₘ (wherein m is an integer of 0 to 2), the rest of the ring atoms are carbon. Preferably 6-14 membered, more preferably 7-10 membered. According to the number of constituent rings, it can be classified into bicyclic, tricyclic, tetracyclic or polycyclic fused heterocyclyl, preferably bicyclic or tricyclic, and more preferably 5-membered/5-membered or 5-membered/6-membered bicyclic fused heterocylyl. Non-limiting examples of fused heterocylyl include:

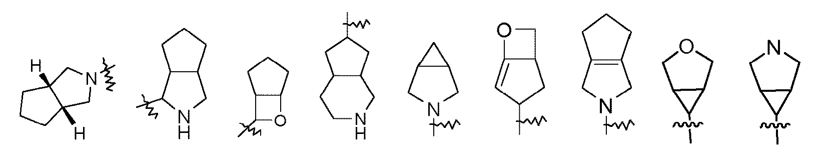

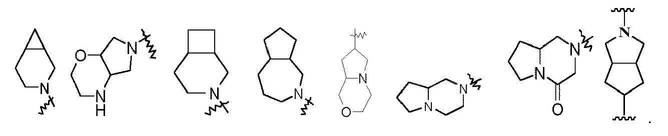

The term "bridged heterocyclyl" refers to a polycyclic heterocyclic group in which any two rings share two atoms that are not directly connected, it may contain one or multiple double bonds, but none of the ring has a fully conjugated π-electron system, wherein one or more of the ring atoms are heteroatoms selected from nitrogen, oxygen or S(O)ₘ (wherein m is an integer of 0 to 2), the rest of the ring atoms are carbon. Preferably 6-14 membered, more preferably 7-10 membered. According to the number of constituent rings, it can be classified into bicyclic, tricyclic, tetracyclic or polycyclic bridged heterocyclyl, preferably bicyclic, tricyclic, or tetracyclic, and more preferably bicyclic or tricyclic. Non-limiting examples of bridged heterocyclyl include: 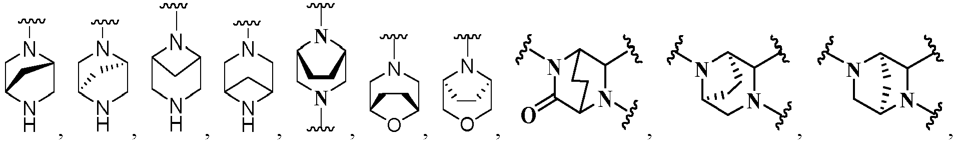 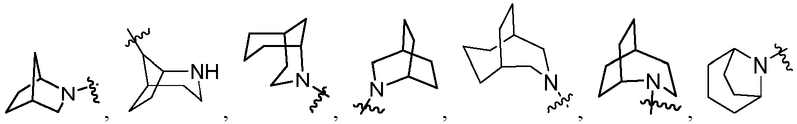 and

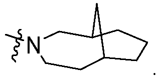

The heterocyclic ring may be fused to an aryl, heteroaryl or cycloalkyl ring, wherein the ring connected to the parent structure is heterocyclyl, non-limiting examples include:

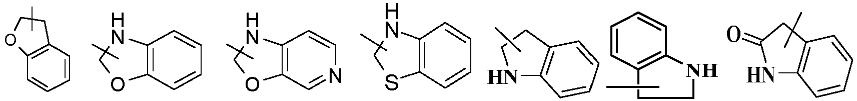

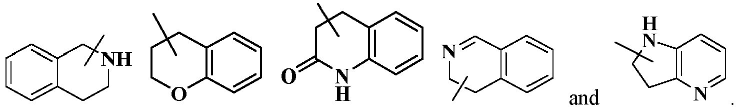

The heterocyclyl may be substituted or unsubstituted, when substituted, the substituents are preferably one or more of the following groups, which are independently selected from alkyl, alkenyl, alkynyl, alkoxy, alkylthio, alkylamino, halogen, sulfhydryl, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl or carboylate.

The term "aryl" refers to a 6-14 membered all-carbon monocyclic or fused polycyclic (that is, rings sharing adjacent pairs of carbon atoms) with conjugated π-electron system, preferably 6-10 membered, such as phenyl and naphthyl. More preferably phenyl. The aryl ring may be fused to heteroaryl, heteroaryl or cycloalkyl ring, wherein the ring connected to the parent structure is aryl ring, non-limiting examples include:

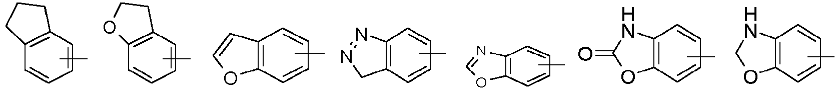

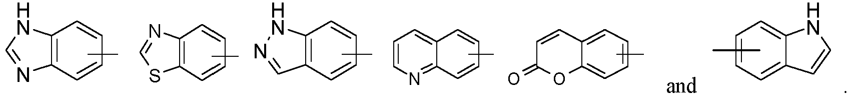

The aryl may be substituted or unsubstituted, when substituted, the substituents are preferably one or more of the following groups, which are independently selected from alkyl, alkenyl, alkynyl, alkoxy, alkylthio, alkylamino, halogen, sulfhydryl, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or carboylate.

The term "heteroaryl" refers to heteroaromatic system comprising 1 to 4 heteroatoms and 5 to 14 ring atoms, wherein the heteroatoms are selected from oxygen, sulfur, and nitrogen. The heteroaryl is preferably 5-10 membered, more preferably 5- or 6- membered, such as imidazole, furanyl, thiophenyl, thiazolyl, pyrazolyl, oxazolyl, pyrrolyl, triazolyl, tetrazolyl, pyridyl, pyrimidinyl, thiadiazole, pyrazinyl, pyridazinyl and oxadiazole, preferably triazolyl, thiophenyl, imidazolyl, pyrazolyl, pyridazinyl and pyrimidinyl, thiazolyl; more preferably, triazolyl, pyrrolyl, thiophenyl, thiazolyl, pyrimidinyl, pyrazolyl, oxazolyl, thiazolyl, thiadiazole, pyridyl, pyridazinyl and oxadiazole. The heteroaryl ring may be fused to an aryl, heteroaryl or cycloalkyl ring, wherein the ring connected to the parent structure is heteroaryl ring, non-limiting examples include:

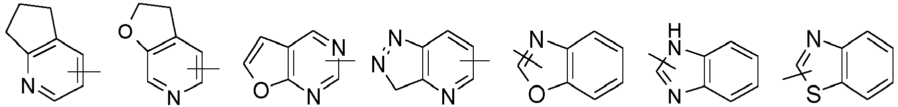

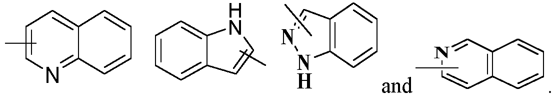

The heteroaryl may be optionally substituted or unsubstituted, when substituted, the substituents are preferably one or more of the following groups, which are independently selected from alkyl, alkenyl, alkynyl, alkoxy, alkylthio, alkylamino, halogen, sulfhydryl, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or carboylate.

The term "alkoxy" refers to -O-(alkyl) and -O-(unsubstituted cycloalkyl), wherein the definition of alkyl is as described above. Non-limiting examples of alkoxy include: methoxy, ethoxy, propoxy, butoxy, cyclopropoxy, cyclobutoxy, cyclopentyloxy, cyclohexyloxy. The alkoxy may be optionally substituted or unsubstituted, when substituted, the substituents are preferably one or more of the following groups, which are independently selected from alkyl, alkenyl, alkynyl, alkoxy, alkylthio, alkylamino, halogen, sulfhydryl, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or carboylate.

"Haloalkyl" refers to alkyl substituted by one or more halogens, wherein the alkyl is as defined above.

"Haloalkoxy" refers to alkoxy substituted by one or more halogens, wherein the alkoxy is as defined above.

"Hydroxyalkyl" refers to alkyl substituted by one or more hydroxyl, wherein the alkyl is as defined above.

"Alkenyl" refers to chain alkenyl, also known as olefinic group, wherein the alkenyl may be further substituted with other related groups, for example: alkyl, alkenyl, alkynyl, alkoxy, alkylthio, alkylamino, halogen, sulfhydryl, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or carboxylate.

"Alknyl" refers to (CH=C- or -C=C-), wherein the alknyl may be further substituted by other related groups, for example: alkyl, alkenyl, alkynyl, alkoxy, alkylthio, alkylamino, halogen, sulfhydryl, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or carboylate.

"Hydroxyl" refers to the -OH group.

"Halogen" refers to fluorine, chlorine, bromine or iodine.

"Amino" refers to -NH₂.

"Cyano" refers to -CN.

"Nitro" refers to -NO₂.

"Carboxyl" refers to -C(O)OH.

"THF" refers to tetrahydrofuran.

"EtOAc" refers to ethyl acetate.

"MeOH" refers to methanol.

"DMF" refers to *N*, *N*-dimethylformamide.

"DIPEA" refers to diisopropylethylamine.

"TFA" refers to trifluoroacetic acid.

"MeCN" refers to acetonitrile.

"DMA" refers to*N*,*N*-dimethylacetamide.

"Et₂O" refers to diethyl ether.

"DCE" refers to 1,2 dichloroethane.

"DIPEA" refers to *N*,*N*-diisopropylethylamine.

"NBS" refers to *N*-bromosuccinimide.

"NIS" refers to *N*-iodosuccinimide.

"Cbz-Cl" refers to benzyl chloroformate.

"Pd₂(dba)₃" refers to tris(dibenzylideneacetone)dipalladium.

"Dppf" refers to 1,1'-bis(diphenylphosphino)ferrocene.

"HATU" refers to 2-(7-aza-1*H*-benzotriazole-1-yl)-1,1,3,3-tetramethyluronium hexafluorophosphate.

"KHMDS" refers to potassium hexamethyldisilazide.

"LiHMDS" refers to lithium bistrimethylsilylamide.

"MeLi" refers to methyl lithium.

"n-BuLi" refers to n-butyl lithium.

"NaBH(OAc)₃" refers to sodium triacetoxyborohydride.

"X is selected from A, B, or C", "X is selected from A, B and C", "X is A, B or C", "X is A, B and C" and other terms all express the same meaning, which means that X can be any one or more of A, B, and C.

The hydrogen described in the present disclosure can be replaced by its isotope deuterium, and any hydrogen in the embodiment compounds of the present disclosure can also be replaced by a deuterium atom.

"Optional" or "optionally" refers to that the event or environment described later can but does not have to occur, and the description includes occasions where the event or environment occurs or does not occur. For example, "heterocyclic group optionally substituted by alkyl" refers to that alkyl may but does not have to be present, and the description includes the case where the heterocyclic group is substituted by alkyl and the case where the heterocyclic group is not substituted by alkyl.

"Substituted" refers to one or more hydrogen atoms in the group, preferably up to 5, more preferably 1 to 3 hydrogen atoms, independently substituted by a corresponding number of substituents. It goes without saying that the substituents are only in their possible chemical positions, and those skilled in the art can determine (by experiment or theory) possible or impossible substitutions without too much effort. For example, amino or hydroxyl having free hydrogen may be unstable when combined with a carbon atom having an unsaturated (e.g., olefinic) bond.

"Pharmaceutical composition" refers to a mixture comprising one or more of the compounds described herein or the physiologically/pharmaceutically acceptable salt thereof and other chemical components, and the other component is, for example, physiological/pharmaceutically acceptable carrier and excipient. The purpose of the pharmaceutical composition is to promote the administration to the organism, facilitate the absorption of the active ingredient and then exert the biological activity.

"Pharmaceutically acceptable salt" refers to a salt of the compound of the present disclosure, which is safe and effective when used in mammals, and has due biological activity.

### Detailed description of the preferred embodiment

The following embodiments will further describe the present disclosure, but these embodiments do not limit the scope of the present disclosure.

### Embodiment

The structures of the compounds of the present disclosure were determined by nuclear magnetic resonance (NMR) or/and liquid chromatography-mass spectrometry (LC-MS). NMR chemical shift (δ) was given in units of parts per million (ppm). NMR was determined using a Bruker AVANCE-400 NMR instrument with deuterated dimethyl sulfoxide (DMSO-*d₆*), deuterated methanol (CD₃OD) and deuterated chloroform (CDCl₃) as solvents and tetramethylsilane (TMS) as internal standard.

Liquid chromatography-mass spectrometry LC-MS was determined with an Agilent 1200 Infinity Series mass spectrometer. HPLC determinations were performed using an Agilent 1200DAD high pressure liquid chromatograph (Sunfire C18 150 × 4.6 mm column) and a Waters 2695-2996 high pressure liquid chromatograph (Gimini C18 150 × 4.6 mm column).

Yantai Huanghai HSGF254 or Qingdao GF254 silica gel plate was used as thin layer chromatography silica gel plate, the specification of TLC was 0.15 mm-0.20 mm, and the specification of thin layer chromatography separation and purification products was 0.4 mm-0.5 mm. Generally, Yantai Huanghai silica gel 200-300 mesh silica gel was used as carrier for column chromatography.

The starting materials in the embodiments of the present disclosure are known and commercially available, or can be synthesized by using or following methods known in the art.

Unless otherwise specified, all reactions of the present disclosure were carried out under continuous magnetic stirring under dry nitrogen or argon atmosphere, the solvent is a dry solvent, and the unit of the reaction temperature was degrees Celsius.

### [Not according to the invention] Embodiment 1

### 4-(6-(6-((6-Methoxypyridin-3-yl)methyl)-3,6-diazabicyclo[3.1.1]heptan-3-yl)pyridin-3-yl)-6-(2-(methylsulfinyl<sulfinyl>)ethoxy)pyrazolo[1,5-a]pyridine-3-carbonitrile

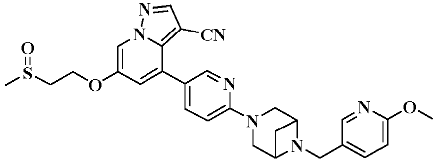

### Step 1: 2-(methylthio)ethan-1-ol

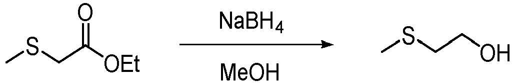

Ethyl 2-(methylthio)acetate (500 mg, 3.7 mmol) was dissolved in 20 mL of MeOH, and NaBH₄ (562 mg, 14.8 mmol) was added at 0 °C, and the mixture was stirred at room temperature for 0.5 hours. 10 mL of NH₄Cl solution was added thereto, and the mixture was extracted with ethyl acetate (20mL*3). The organic phase was washed with saturated saline, dried over anhydrous sodium sulfate. The residue was filtered and evaporated to dryness to obtain 2-(methylthio)ethan-1-ol (240 mg, colorless liquid, the yield was 70%).

### Step 2: 4-bromo-6-(2-(methylthio)ethoxy)pyrazolo[1,5-a]pyridine-3-carbonitrile

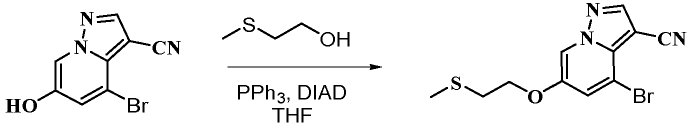

4-Bromo-6-hydroxypyrazolo[1,5-a]pyridine-3-carbonitrile (100 mg, 0.42 mmol) was dissolved in 10 mL of THF; 2-(methylthio)ethan-1-ol (16 mg, 0.5 mmol), triphenylphosphine (165 mg, 0.63 mmol) and DIAD (127 mg, 0.63 mmol) were added and stirred at room temperature overnight. 10 mL of water was added thereto, and the mixture was extracted with ethyl acetate (20mL*3). The organic phase was washed with saturated saline, dried over anhydrous sodium sulfate. The residue was filtered and evaporated to dryness, and the crude product was separated by column chromatography (eluted with dichloromethane/methanol = 10/1) to obtain 4-bromo-6-(2-(methylthio)ethoxy)pyrazolo[1,5-*a*]pyridine-3-carbonitrile (69 mg, white solid, the yield was 53%).

MS m/z (ESI): 311.9 [M+H]⁺.

### Step 3: 4-bromo-6-(2-(methylsulfinyl<sulfinyl>)ethoxy)pyrazolo[1,5-a]pyridine-3-carbonitrile

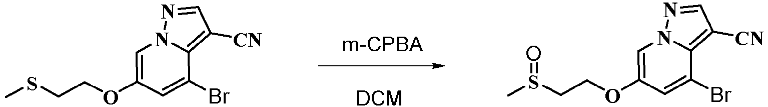

4-Bromo-6-(2-(methylthio)ethoxy)pyrazolo[1,5-a]pyridine-3-carbonitrile (200 mg, 0.64 mmol) was dissolved in 20 mL of DCM; *m*-chloroperoxybenzoic acid (110 mg, 0.64 mmol) was added thereto, and the mixture was stirred at room temperature for 4 hours, 10 mL of water was added thereto and the mixture was extracted with ethyl acetate (20 mL*3). The organic phase was washed with saturated saline, dried over anhydrous sodium sulfate. The residue was filtered and evaporated to dryness, and the crude product was separated by column chromatography (eluted with dichloromethane/methanol = 10/1) to obtain 4-bromo-6-(2-(methylsulfinyl<sulfinyl>)ethoxy)pyrazolo[1,5-*a*]pyridine-3-carbonitrile (189 mg, white solid, the yield was 90%).

MS m/z (ESI): 327.9 [M+H]⁺.

### Step 4: tert-butyl 3-(5-bromopyridin-2-yl)-3,6-diazabicyclo[3.1.1]heptane-6-carboxylate

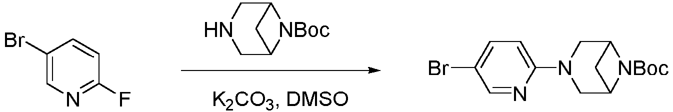

5-Bromo-2-fluoropyridine (500 mg, 2.5 mmol) was dissolved in 20 mL of DMSO; and *tert-*butyl 3,6-diazabicyclo[3.1.1]heptane-6-carboxylate (489 mg, 2.8 mmol), potassium carbonate (1.7 g, 12.5 mmol) were added thereto, and the mixture was stirred at 90 °C overnight. 10 mL of water was added thereto, and the mixture was extracted with ethyl acetate (20mL*3). The organic phase was washed with water and saturated saline, dried over anhydrous sodium sulfate. The residue was filtered, evaporated to dryness; and the crude product was separated by column chromatography (eluted with petroleum ether/ethyl acetate = 1/1) to obtain *tert-*butyl 3-(5-bromopyridin-2-yl)-3,6-diazabicyclo[3.1.1]heptane-6-carboxylate (650 mg, white solid, the yield was 3%).

MS m/z (ESI): 354.0 [M+H]⁺.

### Step 5: 3-(5-bromopyridin-2-yl)-3,6-diazabicyclo[3.1.1]heptane

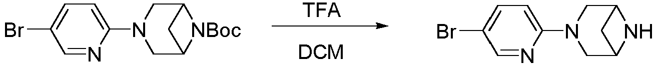

*Tert-butyl* 3-(5-bromopyridin-2-yl)-3,6-diazabicyclo[3.1.1]heptane-6-carboxylate (100 mg, 0.28 mmol) was dissolved in 3 mL of DCM; and 1 mL of TFA was added thereto, and the mixture was stirred at room temperature for 2 hours. The mixture was then evaporated to dryness, sodium bicarbonate aqueous solution was added to adjust the pH value to basic, and extracted with ethyl acetate (20 mL*3). The organic phase was washed with saturated saline, dried over anhydrous sodium sulfate. The residue was filtered, evaporated to dryness to obtain 3-(5-bromopyridin-2-yl)-3,6-diazabicyclo[3.1.1]heptane (70 mg, white solid, the yield was 99%).

MS m/z (ESI): 254.0 [M+H]⁺.

### Step 6: 3-(5-bromopyridin-2-yl)-6-((6-methoxypyridin-3-yl)methyl)-3,6-diazabicyclo[3.1.1]heptane

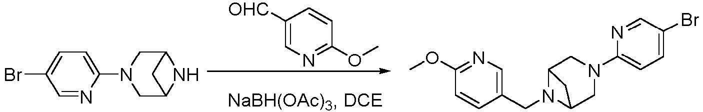

3-(5-Bromopyridin-2-yl)-3,6-diazabicyclo[3.1.1]heptane (70 mg, 0.28 mmol), 6-methoxynicotinaldehyde (113 mg, 0.83 mmol) were dissolved in 10 mL of DCE, NaBH(OAc)₃ (176 mg, 0.83 mmol) was added thereto, and the mixture was stirred at room temperature overnight. 10 mL of water was added, and the mixture was extracted with ethyl acetate (20 mL*3). The organic phase was washed with saturated saline, dried over anhydrous sodium sulfate. The residue was filtered and evaporated to dryness, and the crude product was separated by column chromatography (eluted with dichloromethane/methanol = 10/1) to obtain 3-(5-bromopyridin-2-yl)-6-((6-methoxypyridin-3-yl)methyl)-3,6-diazabicyclo[3.1.1]heptane (60 mg, white solid, the yield was 57%).

MS m/z (ESI): 375.0 [M+H]⁺.

### Step 7: 6-(2-(methylsulfinyl<sulfinyl>)ethoxy)-4-(4,4,5,5-tetramethyl-1,3,2-dioxaborolan-2-yl) pyrazolo[1,5-a]pyridine-3-carbonitrile

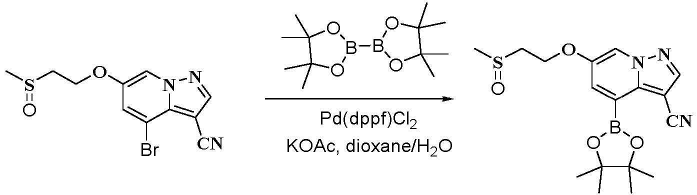

4-Bromo-6-(2-(methylsulfinyl<sulfinyl>)ethoxy)pyrazolo[1,5-a]pyridine-3-carbonitrile (200 mg, 0.6 mmol), bis(pinacolato)diboron (232 mg, 0.91 mmol), Pd(dppf)Cl₂ (44 mg, 0.06 mmol) and KOAc (176 mg, 1.8 mmol) were dissolved in dioxane/H₂O (20 mL, v/v = 10 : 1), and the mixture was stirred at 90 °C overnight under the protection of nitrogen. 10 mL of water was added thereto, and the mixture was extracted with ethyl acetate (20mL*3). The organic phase was washed with saturated saline, dried over anhydrous sodium sulfate. The residue was filtered and evaporated to dryness, and the crude product was separated by column chromatography (eluted with dichloromethane/methanol = 10/1) to obtain 6-(2-(methylsulfinyl<sulfinyl>)ethoxy)-4-(4,4,5,5-tetramethyl-1,3,2-dioxaborolan-2-yl)pyrazolo[1,5-*a*]pyridine-3-carbonitrile (117 mg, white solid, the yield was 52%).

MS m/z (ESI): 376.1 [M+H]⁺.

### Step 8: 4-(6-(6-((6-methoxypyridin-3-yl)methyl)-3,6-diazabicyclo[3.1.1]heptan-3-yl)pyridin-3-yl)-6-(2-(methylsulfinyl<sulfinyl>)ethoxy)pyrazolo[1,5-a]pyridine-3-carbonitrile

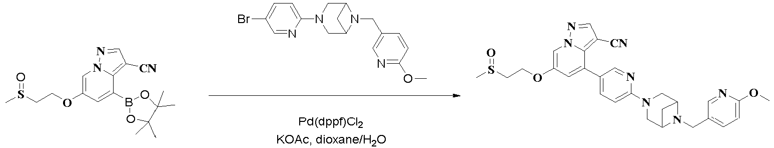

6-(2-(methylsulfinyl<sulfinyl>)ethoxy)-4-(4,4,5,5-tetramethyl-1,3,2-dioxaborolan-2-yl)pyrazolo[1,5-a]pyridine-3-carbonitrile (60 mg, 0.16 mmol), 3-(5-bromopyridin-2-yl)-6-((6-methoxypyridin-3-yl)methyl)-3,6-diazabicyclo[3.1.1]heptane (72 mg, 0.19 mmol), Pd(dppf)Cl₂ (15 mg, 0.02 mmol) and KOAc (44 mg, 0.5 mmol) were dissolved in dioxane/H₂O (20 mL, v/v = 10 : 1), and the mixture was stirred at 90 °C overnight under the protection of nitrogen. 10 mL of water was added thereto, and the mixture was extracted with ethyl acetate (20mL*3). The organic phase was washed with saturated saline, dried over anhydrous sodium sulfate. The residue was filtered and evaporated to dryness, the crude product was prepared by prep-HPLC to obtain 4-(6-(6-((6-methoxypyridin-3-yl)methyl)-3,6-diazabicyclo[3.1.1]heptan-3-yl)pyridin-3-yl)-6-(2-(methylsulfinyl<sulfinyl>)ethoxy)pyrazolo[1,5-a]pyridine-3-carbonitrile (41 mg, white solid, the yield was 48%).

MS m/z (ESI): 544.2 [M+H]⁺.

¹H NMR (400 MHz, DMSO) δ 8.82 (d, J = 1.9 Hz, 1H), 8.62 (s, 1H), 8.41 (d, J = 2.3 Hz, 1H), 8.07 (d, J = 1.9 Hz, 1H), 7.85 (dd, J = 8.8, 2.4 Hz, 1H), 7.68 (dd, J = 8.5, 2.2 Hz, 1H), 7.32 (d, J = 2.0 Hz, 1H), 6.78 (t, J = 9.4 Hz, 2H), 4.62 - 4.44 (m, 2H), 3.82 (s, 3H), 3.73 (d, J = 11.6 Hz, 2H), 3.67 (d, J = 5.7 Hz, 2H), 3.58 - 3.52 (m, 2H), 3.50 (s, 2H), 3.13 (dt, J = 13.6, 4.4 Hz, 1H), 2.67 (s, 3H), 2.59 - 2.52 (m, 2H), 1.59 (d, J = 8.5 Hz, 1H).

### [Not according to the invention] Embodiment 2

### 4-(6-(6-((6-Methoxypyridin-3-yl)methyl)-3,6-diazabicyclo[3.1.1]heptan-3-yl)pyridin-3-yl)-6-(2-(methylsulfonyl)ethoxy)pyrazolo[1,5-a]pyridine-3-carbonitrile

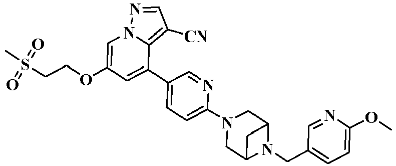

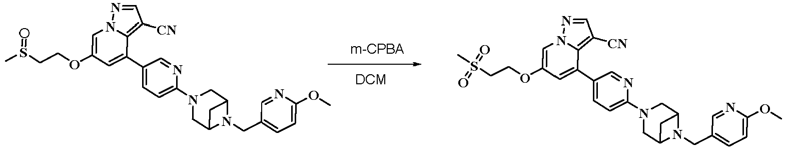

4-(6-(6-((6-Methoxypyridin-3-yl)methyl)-3,6-diazabicyclo[3.1.1]heptan-3-yl)pyridin-3-yl)-6-(2-(methylsulfinyl<sulfinyl>)ethoxy)pyrazolo[1,5-*a*]pyridine-3-carbonitrile was used as raw material, 4-(6-(6-((6-methoxypyridin-3-yl)methyl)-3,6-diazabicyclo[3.1.1]heptan-3-yl)pyridin-3-yl)-6-(2-(methylsulfonyl)ethoxy)pyrazolo[1,5-*a*]pyridine-3-carbonitrile (45mg, white solid, the yield was 70%) was obtained with reference to step 3 of embodiment 1.

MS m/z (ESI): 560.2 [M+H]⁺.

### [Not according to the invention] Embodiment 3

### 4-(6-(6-((6-Methoxypyridin-3-yl)methyl)-3,6-diazabicyclo[3.1.1]heptan-3-yl)pyridin-3-yl)-6-(2-(S-methylsulfonimidoyl)ethoxy)pyrazolo[1,5-a]pyridine-3-carbonitrile

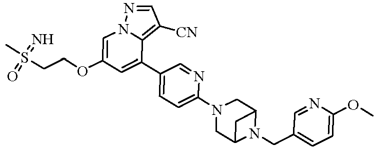

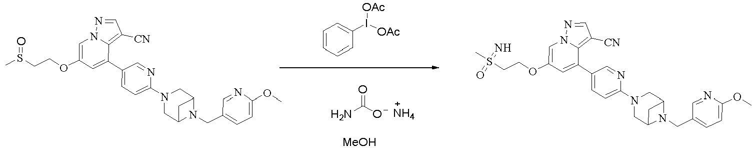

4-(6-(6-((6-Methoxypyridin-3-yl)methyl)-3,6-diazabicyclo[3.1.1]heptan-3-yl)pyridin-3-yl)-6-(2-(methylsulfonyl)ethoxy)pyrazolo[1,5-*a*]pyridine-3-carbonitrile (60 mg, 0.11 mmol) was dissolved in 5 mL of methanol; and ammonium carbamate (17 mg, 0.22 mmol), iodobenzene diacetate(70 mg, 0.22 mmol) were added thereto, and the reaction was carried out at room temperature for 2 hours. 10 mL of water was added thereto, and the mixture was extracted with ethyl acetate (10 mL*3). The organic phase was washed with saturated saline, dried over anhydrous sodium sulfate. The residue was filtered and evaporated to dryness, the crude product was prepared by prep-HPLC to obtain 4-(6-(6-((6-methoxypyridin-3-yl)methyl)-3,6-diazabicyclo[3.1.1]heptan-3-yl)pyridin-3-yl)-6-(2-(S-methylsulfonimidoyl)ethoxy)pyrazolo[1,5-*a*]pyridine-3-carbonitrile (25 mg, white solid, the yield was 45%).

MS m/z (ESI): 559.2 [M+H]⁺.

### [Not according to the invention] Embodiment 4

### 4-(6-(6-((6-Methoxypyridin-3-yl)methyl)-3,6-diazabicyclo[3.1.1]heptan-3-yl)pyridin-3-yl)-6-(3-(methylsulfinyl<sulfinyl>)propoxy)pyrazolo[1,5-a]pyridine-3-carbonitrile

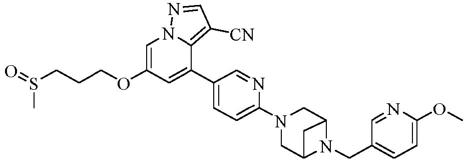

The title compound (35 mg, white solid, 40%) was obtained by using ethyl 3-(methylthio)propionate as raw material with reference to embodiment 1.

MS m/z (ESI): 558.2 [M+H]⁺.

### [Not according to the invention] Embodiment 5

### 4-(6-(6-((6-Methoxypyridin-3-yl)methyl)-3,6-diazabicyclo[3.1.1]heptan-3-yl)pyridin-3-yl)-6-(3-(methylsulfonyl)propoxy)pyrazolo[1,5-a]pyridine-3-carbonitrile

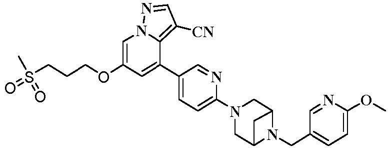

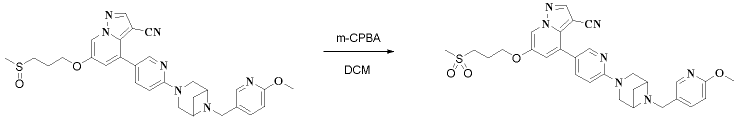

4-(6-(6-((6-Methoxypyridin-3-yl)methyl)-3,6-diazabicyclo[3.1.1]heptan-3-yl)pyridin-3-yl)-6-(3-(methylsulfinyl<sulfinyl>)propoxy)pyrazolo[1,5-*a*]pyridine-3-carbonitrile was used as raw material, 4-(6-(6-((6-methoxypyridin-3-yl)methyl)-3,6-diazabicyclo[3.1.1]heptan-3-yl)pyridin-3-yl)-6-(3-(methylsulfonyl)propoxy)pyrazolo[1,5-*a*]pyridine-3-carbonitrile was obtained with reference to embodiment 2 (32mg, white solid, the yield was 56%).

MS m/z (ESI): 574.2 [M+H]⁺.

### [Not according to the invention] Embodiment 6

### 4-(6-(6-((6-Methoxypyridin-3-yl)methyl)-3,6-diazabicyclo[3.1.1]heptan-3-yl)pyridin-3-yl)-6-(3-(S-methylsulfonimidoyl)propoxy)pyrazolo[1,5-a]pyridine-3-carbonitrile

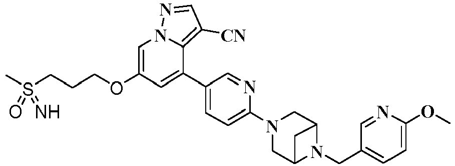

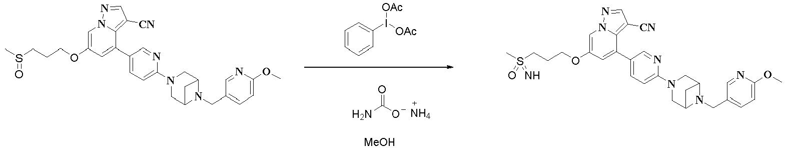

4-(6-(6-((6-Methoxypyridin-3-yl)methyl)-3,6-diazabicyclo[3.1.1]heptan-3-yl)pyridin-3-yl)-6-(3-(methylsulfinyl<sulfinyl>)propoxy)pyrazolo[1,5-*a*]pyridine-3-carbonitrile was used as raw material, 4-(6-(6-((6-methoxypyridin-3-yl)methyl)-3,6-diazabicyclo[3.1.1]heptan-3-yl)pyridin-3-yl)-6-(3-(S-methylsulfonimidoyl)propoxy)pyrazolo[1,5-a]pyridine-3-carbonitrile was obtained with reference to embodiment 3 (28mg, white solid, the yield was 44%).

MS m/z (ESI): 573.2 [M+H]⁺.

### [Not according to the invention] Embodiment 7

### 6-((2-Hydroxy-2-methylpropyl)thio)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

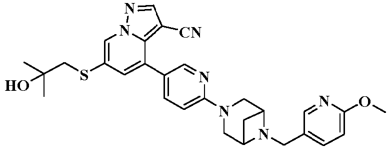

### Step 1: 4-bromo-3-cyanopyrazolo[1,5-a]pyridin-6-yl trifluoromethanesulfonate

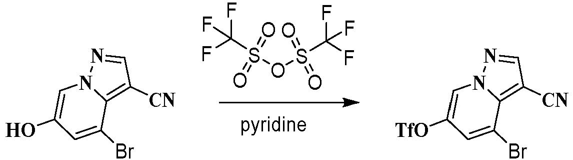

4-Bromo-6-hydroxypyrazolo[1,5-*a*]pyridine-3-carbonitrile (200 mg, 0.84 mmol), dichloromethane (5 mL) and pyridine (1 mL) were added to a 25 mL single-neck flask sequentially, after the reaction mixture was stirred for 2 minutes, trifluoromethanesulfonic anhydride (355 mg, 1.26 mmol) was slowly added thereto dropwise. The reaction mixture was stirred at room temperature for 12 hours, the reaction mixture was concentrated, dissolved in ethyl acetate (10 mL) and washed with saturated saline (5 mLx3), and the organic phase was dried over anhydrous sodium sulfate, filtered and evaporated to dryness. The crude product was purified by column chromatography (petroleum ether/ethyl acetate: 1/1) to obtain 4-bromo-3-cyanopyrazolo[1,5-*a*]pyridin-6-yl trifluoromethanesulfonate (280 mg, light yellow solid, yield: 90.0%).

MS m/z (ESI): 370.0[M+H]⁺, 372.0[M+H+2]⁺.

### Step 2: 4-bromo-6-((2-hydroxy-2-methylpropyl)thio)pyrazolo[1,5-a]pyridine-3-carbonitrile

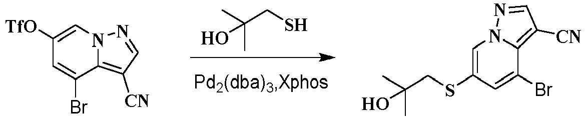

4-Bromo-3-cyanopyrazolo[1,5-*a*]pyridin-6-yl trifluoromethanesulfonate (280 mg, 0.75 mmol), 1-mercapto-2-methylpropan-2-ol (80 mg, 0.75 mmol), tris(dibenzylideneacetone)dipalladium (68 mg, 0.075 mmol), 2-dicyclohexylphosphino-2',4',6'-triisopropylbiphenyl (71 mg, 0.15 mmol), diisopropylethylamine (193 mg, 1.5 mmol) and dioxane (10 mL) were added to a 25 mL three-necked flask sequentially, and the reaction mixture was replaced with nitrogen 5 times. The reaction mixture was heated to 85 °C under the protection of nitrogen, and the mixture was stirred for 5 hours and then cooled to room temperature; the reaction mixture was concentrated, dissolved in ethyl acetate (10 mL) and washed with saturated saline (5 mLx3), and the organic phase was dried over anhydrous sodium sulfate, filtered and evaporated to dryness. The crude product was separated by column chromatography (dichloromethane/methanol: 30/1) and purified to obtain the product of 4-bromo-6-((2-hydroxy-2-methylpropyl)thio)pyrazolo[1,5-*a*]pyridine-3-carbonitrile (140 mg, white solid, the yield: 56.6%).

MS m/z (ESI): 326.0[M+H]⁺, 328.0[M+H+2]⁺.

### Step 3: 6-((2-hydroxy-2-methylpropyl)thio)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

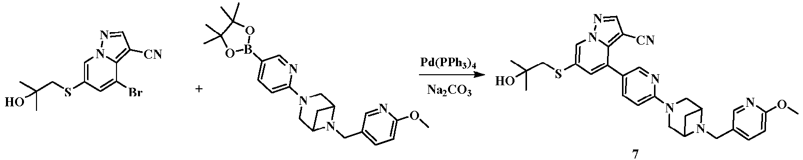

4-Bromo-6-((2-hydroxy-2-methylpropyl)thio)pyrazolo[1,5-*a*]pyridine-3-carbonitrile (50 mg, 0.15 mmol), 6-((6-methoxypyridin-3-yl)methyl)-3-(5-(4,4,5,5-tetramethyl-1,3,2-dioxaborolan-2-yl)pyridin-2-yl)-3,6-diazabicyclo[3.1.1]heptane (64 mg, 0.15 mmol), tetrakis(triphenylphosphine)palladium (17 mg, 0.015 mmol), sodium carbonate (48 mg, 0.45 mmol), and dioxane (5 mL) and water (1 mL) were added to a 25 mL three-necked flask sequentially, and the reaction mixture was replaced with nitrogen 5 times. The reaction mixture was heated to 85 °C under the protection of nitrogen, and the mixture was stirred for 5 hours and then cooled to room temperature; the reaction mixture was concentrated, dissolved in ethyl acetate (10 mL) and washed with saturated saline (5 mLx3), and the organic phase was dried over anhydrous sodium sulfate, filtered and evaporated to dryness. The crude product was purified by prep-HPLC to obtain 6-((2-hydroxy-2-methylpropyl)thio)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (25 mg, white solid, yield: 30.1%).

MS m/z (ESI): 542.2[M+H]⁺.

### [Not according to the invention] Embodiment 8

### 6-((2-Hydroxy-2-methylpropyl)thio)-4-(6-(4-((6-methoxypyridin-3-yl)oxo)piperidin-1-yl)pyridin-3-yl)pyrazolo[1,5-a]pyridine-3-carbonitrile

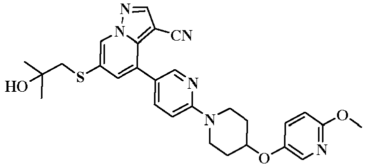

### Step 1: 4-(6-fluoropyridin-3-yl)-6-((2-hydroxy-2-methylpropyl)thio)pyrazolo[1,5-a]pyridine-3-carbonitrile

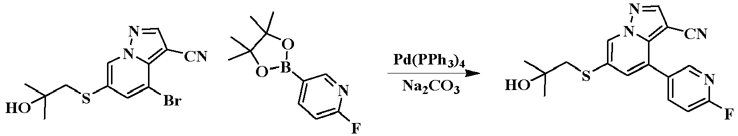

4-Bromo-6-((2-hydroxy-2-methylpropyl)thio)pyrazolo[1,5-*a*]pyridine-3-carbonitrile (100 mg, 0.30 mmol), 2-fluoro-5-(4,4,5,5-tetramethyl-1,3,2-dioxaborolan-2-yl)pyridine (68 mg, 0.30 mmol), tetrakis(triphenylphosphine)palladium (27 mg, 0.03 mmol), sodium carbonate (97 mg, 0.09 mmol), and dioxane (5 mL) and water (1 mL) were added to a 25 mL three-necked flask sequentially, and the reaction mixture was replaced with nitrogen 5 times. The reaction mixture was heated to 85 °C under the protection of nitrogen, and the mixture was stirred for 5 hours and then cooled to room temperature; the reaction mixture was concentrated, dissolved in ethyl acetate (10 mL) and washed with saturated saline (5 mLx3), and the organic phase was dried over anhydrous sodium sulfate, filtered and evaporated to dryness. The crude product was separated by column chromatography (dichloromethane/methanol: 30/1) and purified to obtain 4-(6-fluoropyridin-3-yl)-6-((2-hydroxy-2-methylpropyl)thio)pyrazolo[1,5-*a*]pyridine-3-carbonitrile (85 mg, light yellow solid, yield: 80.9%).

MS m/z (ESI): 343.1[M+H]⁺.

### Step 2: 6-((2-hydroxy-2-methylpropyl)thio)-4-(6-(4-((6-methoxypyridin-3-yl)oxo)piperidin-1-yl)pyridin-3-yl)pyrazolo[1,5-a]pyridine-3-carbonitrile

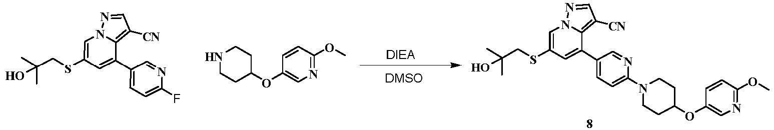

4-(6-Fluoropyridin-3-yl)-6-((2-hydroxy-2-methylpropyl)thio)pyrazolo[1,5-*a*]pyridine-3-carbonitrile (85 mg, 0.25 mmol), 2-methoxy-5-(piperidin-4-oxy)pyridine (52 mg, 0.25 mmol), diisopropylethylamine (65 mg, 0.50 mmol) and dimethyl sulfoxide (2 mL) were added to a 25 mL three-necked flask sequentially. The reaction mixture was heated to 90 °C under the protection of nitrogen, and the mixture was stirred for 48 hours and then cooled to room temperature; the reaction mixture was concentrated, dissolved in ethyl acetate (10 mL) and washed with saturated saline (5 mLx3), and the organic phase was dried over anhydrous sodium sulfate, filtered and evaporated to dryness. The crude product was purified by prep-HPLC to obtain product 6-((2-hydroxy-2-methylpropyl)thio)-4-(6-(4-((6-methoxypyridin-3-yl)oxo)piperidin-1-yl)pyridin-3-yl)pyrazolo[1,5-*a*]pyridine-3-carbonitrile (60 mg, white solid, yield: 45.5%).

MS m/z (ESI): 531.2[M+H]⁺.

### [Not according to the invention] Embodiment 9

### 6-(2-Hydroxy-2-methylpropylsulfonimidoyl)-4-(6-(4-(pyridin-2-oxy)piperidin-1-yl)pyridin-3-yl)pyrazolo[1,5-a]pyridine-3-carbonitrile

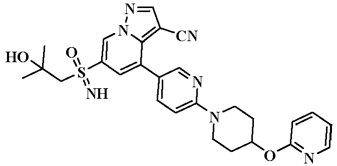

### Step 1: 4-bromo-6-((2-hydroxy-2-methylpropyl)sulfinl)pyrazolo[1,5-a]pyridine-3-carbonitrile

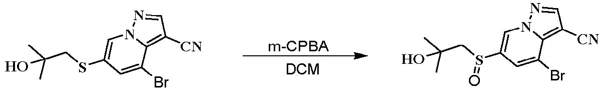

4-Bromo-6-((2-hydroxy-2-methylpropyl)thio)pyrazolo[1,5-*a*]pyridine-3-carbonitrile (100 mg, 0.30 mmol) was dissolved in dichloromethane (5 mL), cooled to -20 °C in a dry ice/ethyl acetate bath, and *m-*chloroperoxybenzoic acid (51 mg, 0.3 mmol) was added thereto in batches; after the addition was completed, the dry ice bath was removed, the mixture was warmed up to room temperature naturally and stirred for 30 minutes. Saturated sodium carbonate solution (5 mL) was added to the reaction mixture, the mixture was then extracted with ethyl acetate (5 mLx2); and the organic phases were mixed, washed with saturated sodium chloride solution (5 mL x2), dried over anhydrous sodium sulfate, and concentrated under reduced pressure to obtain 4-bromo-6-((2-hydroxy-2-methylpropyl)sulfinyl)pyrazolo[1,5-*a*]pyridine-3-carbonitrile (95 mg, 90.5%).

MS m/z (ESI): 342.0 [M+H]⁺, 344.0[M+2+H]⁺.

### Step 2: 4-bromo-6-(2-hydroxy-2-methylpropylsulfonimidoyl)pyrazolo[1,5-a]pyridine-3-carbonitrile

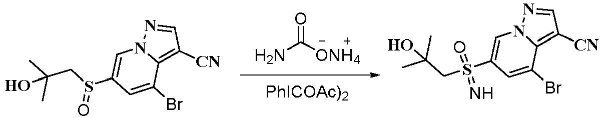

4-Bromo-6-((2-hydroxy-2-methylpropyl)thionyl)pyrazolo[1,5-*a*]pyridine-3-carbonitrile (95 mg, 0.28 mmol) was dissolved in methanol (5 mL); then ammonium carbamate(108 mg, 1.39 mmol) and iodobenzene diacetate (268 mg, 0.83 mmol) were added thereto sequentially, and the mixture was stirred at room temperature for 1 hour. The reaction was stopped, the reaction was quenched with water (5 mL), extracted with ethyl acetate (5 mLx2), and the organic phases were combined. The organic phase was washed with saturated sodium chloride (5 mL), dried over anhydrous sodium sulfate, filtered and concentrated under reduced pressure to obtain 4-bromo-6-(2-hydroxy-2-methylpropylsulfonimidoyl)pyrazolo[1,5-*a*]pyridine-3-carbonitrile (90 mg, 90.7%).

MS m/z (ESI): 357.0 [M+H]⁺, 359.0[M+2+H]⁺.

### Step 3: 4-(6-fluoropyridin-3-yl)-6-(2-hydroxy-2-methylpropylsulfonimidoyl)pyrazolo[1,5-a]pyridine-3-carbonitrile

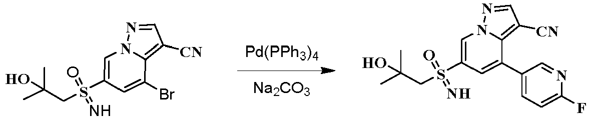

The product 4-(6-fluoropyridin-3-yl)-6-(2-hydroxy-2-methylpropylsulfonimidoyl)pyrazolo[1,5-a]pyridine-3-carbonitrile (95 mg, white solid, the yield was 95.6%) was obtained by using 4-bromo-6-(2-hydroxy-2-methylpropylsulfonimidoyl)pyrazolo[1,5-*a*]pyridine-3-carbonitrile as raw material with reference to step 1 of embodiment 8.

MS m/z (ESI): 374.1[M+H]⁺.

### Step 4: 6-(2-hydroxy-2-methylpropylsulfonimidoyl)-4-(6-(4-(pyridin-2-oxy)piperidin-1-yl)pyridin-3-yl)pyrazolo[1,5-a]pyridine-3-carbonitrile

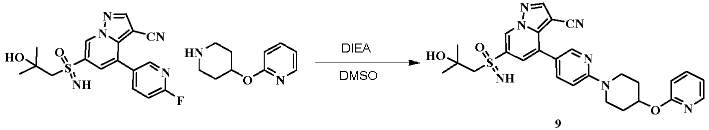

The product 6-(2-hydroxy-2-methylpropylsulfonimidyl)-4-(6-(4-(pyridin-2-oxy)piperidin-1-yl)pyridin-3-yl)pyrazolo[1,5-*a*]pyridine-3-carbonitrile (30 mg, white solid, yield was 22.0%) was obtained by using 4-(6-fluoropyridin-3-yl)-6-(2-hydroxy-2-methylpropylsulfonimidyl)pyrazolo[1,5-*a*]pyridine-3-carbonitrile as raw material with reference to step 2 of embodiment 8.

MS m/z (ESI): 532.2 [M+H]⁺.

### [Not according to the invention] Embodiment 10

### 6-(Ethylsulfonimidoyl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

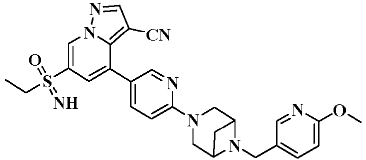

### Step 1: 4-bromo-6-(ethylthio)pyrazolo[1,5-a]pyridine-3-carbonitrile

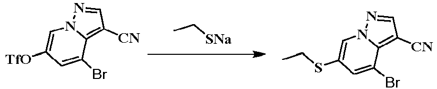

4-Bromo-3-cyanopyrazolo[1,5-*a*]pyridin-6-yl trifluoromethanesulfonate (200 mg, 0.54 mmol), sodium ethanethiolate (90 mg, 1.08 mmol) and dioxane (5 mL) were added to a 25 mL three-necked flask sequentially. The reaction mixture was heated to 85 °C under the protection of nitrogen, and the mixture was stirred for 5 hours and then cooled to room temperature; the reaction mixture was concentrated, dissolved in ethyl acetate (10 mL) and washed with saturated saline (5 mLx3), and the organic phase was dried over anhydrous sodium sulfate, filtered and evaporated to dryness. The crude product was separated by column chromatography (dichloromethane/methanol: 30/1) and purified to obtain 4-bromo-6-(ethylthio)pyrazolo[1,5-a]pyridine-3-carbonitrile (120 mg, white solid, yield: 78.7%).

MS m/z (ESI): 282.0[M+H]⁺, 284.0[M+H+2]⁺.

### Step 2: 4-bromo-6-(ethylsulfinyl<sulfinyl>)pyrazolo[1,5-a]pyridine-3-carbonitrile

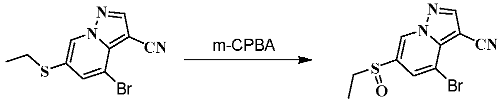

The product 4-bromo-6-(ethylsulfinyl<sulfinyl>)pyrazolo[1,5-*a*]pyridine-3-carbonitrile (120 mg, white solid, the yield was 96.6%) was obtained by using 4-bromo-6-(ethylthio)pyrazolo[1,5-*a*]pyridine-3-carbonitrile as raw material with reference to step 1 of embodiment 9.

MS m/z (ESI): 298.0[M+H]⁺, 300.0[M+H+2]⁺.

### Step 3: 4-bromo-6-(ethylsulfonimidoyl)pyrazolo[1,5-a]pyridine-3-carbonitrile

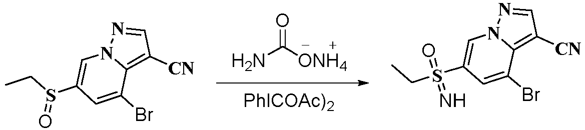

The product 4-bromo-6-(ethylsulfonimidoyl)pyrazolo[1,5-*a*]pyridine-3-carbonitrile (120 mg, white solid, the yield was 95.9%) was obtained by using 4-bromo-6-(ethylsulfinyl<sulfinyl>)pyrazolo[1,5-a]pyridine-3-carbonitrile as raw material with reference to step 2 of embodiment 9.

MS m/z (ESI): 313.0[M+H]⁺, 315.0[M+H+2]⁺.

### Step 4: 6-(ethylsulfonimidoyl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

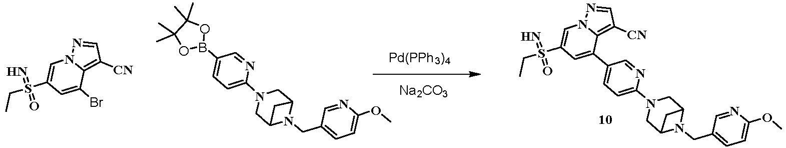

The product 6-(ethylsulfonimidoyl)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (45 mg, white solid, yield was 22.2%) was obtained by using 4-bromo-6-(ethylsulfonimidoyl)pyrazolo[1,5-*a*]pyridine-3-carbonitrile as raw material with reference to step 3 of embodiment 7.

MS m/z (ESI): 529.2[M+H]⁺.

### [Not according to the invention] Embodiment 11

### 6-(2-Hydroxy-2-methylpropoxy)-4-(6-(6-((6-methoxypyridin-3-yl)methyl)-3,6-diazabicyclo[3.1.1]heptan-3-yl)pyridin-3-yl)-7-methylpyrazolo[1,5-a]pyridine-3-carbonitrile

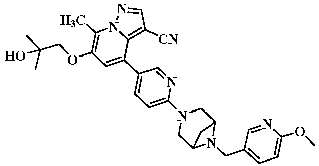

### Step 1: 5-bromo-3-methoxy-2-methylpyridine

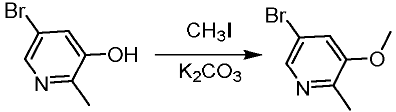

5-Bromo-2-methylpyridin-3-ol (10 g, 53.2 mmol) was dissolved in acetonitrile (50 mL), then potassium carbonate (14.7 g, 106.4 mmol) was added thereto and iodomethane (22.66 g, 160 mmol) was added dropwise, then the reaction mixture was stirred at 80°C for 16 hours; the reaction mixture was cooled to room temperature, concentrated under reduced pressure to dryness, and purified by layer-by-layer separation (petroleum ether/ethyl acetate = 15:1) to obtain light yellow solid 5-bromo-3-methoxy-2-methylpyridine (5 g, yield: 46.7%).

¹H NMR (400 MHz, CDCl₃) δ 8.14 (d, J = 1.6 Hz, 1H), 7.22 (d, J = 1.4 Hz, 1H), 3.84 (s, 3H), 2.42 (s, 3H).

MS m/z (ESI): 202.0 [M+H]⁺.

### Step 2: 2,4,6-trimethylbenzenesulfonic acid 1-amino-5-bromo-3-methoxy-2-methylpyridin-1-ium

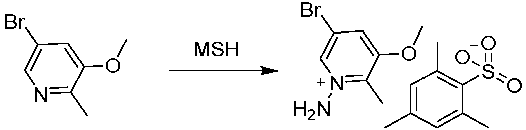

5-Bromo-3-methoxy-2-methylpyridine (5 g, 24.75 mmol) was added to a solution of 2-[(aminooxy)sulfonyl]-1,3,5-trimethylbenzene (5.32 g, 24.75 mmol) in dichloromethane (50 mL) at 0 °C in batches, then the mixture was stirred at 0 °C for 1.5 hours; methyl *tert-*butyl ether (30 mL) was added to the reaction mixture, and the mixture was slurried for 15 min, then filtered and the filter cake was dried to obtain white solid 2,4,6-trimethylbenzenesulfonic acid 1-amino-5-bromo-3-methoxy-2-methylpyridine-1-ium (9 g, yield: 87.3%).

### Step 3: 4-bromo-6-methoxy-7-methylpyrazolo[1,5-a]pyridine-3-carbonitrile

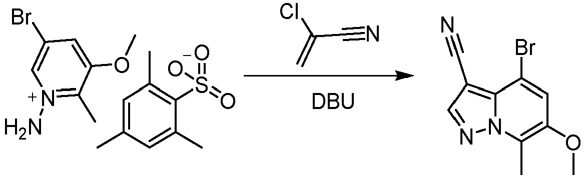

1.8-Diazabicyclo[5.4.0]undec-7-ene (6.6 g, 43.2 mmol) was added to a solution of 2,4,6-trimethylbenzenesulfonic acid 1-amino-5-bromo-3-methoxy-2-methylpyridine-1-ium (9 g, 21.6 mmol) and 2-chloroacrylonitrile (2.8 g, 32.37 mmol) in dichloromethane (100 mL) at 0 °C in batches, then the mixture was warmed up to room temperature and stirred for 24 hours, methyl *tert-*butyl ether (50 mL) was added to the reaction mixture, and the mixture was slurried at room temperature for 15 min, then filtered and the filter cake was dried to obtain light yellow solid 4-bromo-6-methoxy-7-methylpyrazolo[1,5-*a*]pyridine-3-carbonitrile (2 g, yield: 35%).

MS m/z (ESI): 266.0 [M+H]⁺.

### Step 4: 4-bromo-6-hydroxy-7-methylpyrazolo[1,5-a]pyridine-3-carbonitrile

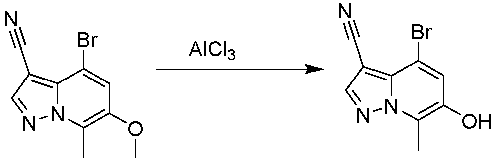

Aluminum trichloride (2 g, 15.1 mmol) was added to a solution of 4-bromo-6-methoxy-7-methylpyrazolo[1,5-*a*]pyridine-3-carbonitrile (2 g, 7.55 mmol) in 1.2-dichloroethane (20 mL) in batches, then the mixture was stirred at 80 °C for 2 hours, cooled to room temperature, quenched with sodium sulfate decahydrate, filtered, and the filter cake was washed with dichloromethane, the filtrate was washed with saturated saline; the organic phase was dried over anhydrous sodium sulfate, filtered, and the filtrate was concentrated under reduced pressure to dryness to obtain black solid 4-bromo-6-hydroxy-7-methylpyrazolo[1,5-*a*]pyridine-3-carbonitrile (1.5 g, yield: 79%).

MS m/z (ESI): 249.7 [M-H]⁺.

### Step 5: 4-bromo-6-(2-hydroxy-2-methylpropoxy)-7-methylpyrazolo[1,5-a]pyridine-3-carbonitrile

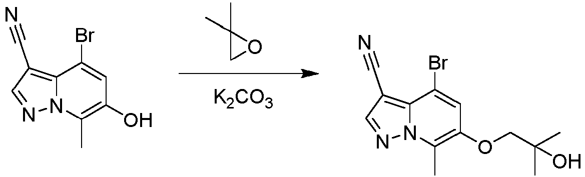

2,2-Dimethyloxirane (857 mg, 11.9 mmol) was added to a solution of 4-bromo-6-hydroxy-7-methylpyrazolo[1,5-*a*]pyridine-3-carbonitrile (1.5 g, 5.95 mmol), potassium carbonate (1.6 g, 11.9 mmol) and acetonitrile (10 mL), and then the reaction mixture was stirred at 80 °C for 16 hours; the reaction mixture was concentrated under reduced pressure to dryness, separated by column chromatography (dichloromethane/methanol = 10:1) to obtain colorless oil 4-bromo-6-(2-hydroxy-2-methylpropoxy)-7-methylpyrazolo[1,5-*a*]pyridine-3-carbonitrile (1.4 g, yield: 74%).

MS m/z (ESI): 324.0 [M+H]⁺.

### Step 6: 4-(6-fluoropyridin-3-yl)-6-(2-hydroxy-2-methylpropoxy)-7-methylpyrazolo[1,5-a]pyridine-3-carbonitrile

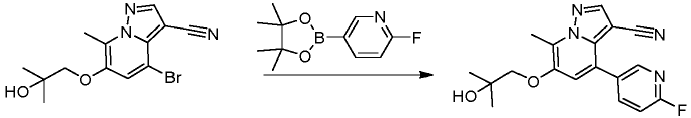

1,1'-Bis(diphenylphosphino)ferrocene-palladium(II)dichloride dichloromethane complex (178 mg, 0.22 mmol) was added to a mixed solution of 4-bromo-6-(2-hydroxy-2-methylpropoxy)-7-methylpyrazolo[1,5-*a*]pyridine-3-carbonitrile (1.4 g, 4.32 mmol), 2-fluoro-5-(4,4,5,5-tetramethyl-1,3,2-dioxaborolan-2-yl)pyridine (1.15 g, 5.18 mmol), potassium acetate (847 mg, 8.64 mmol) and dioxane (15 mL), the mixture was replaced with nitrogen three times and then stirred at 100 °C for 16 hours under the protection of the protection of nitrogen, after the reaction was complete, the mixture was cooled and filtered, the filtrate was concentrated under reduced pressure to dryness and separated by column chromatography (dichloromethane/methanol = 10:1) to obtain colorless oil 4-(6-fluoropyridin-3-yl)-6-(2-hydroxy-2-methylpropoxy)-7-methylpyrazolo[1,5-*a*]pyridine-3-carbonitrile (1.2 g, yield: 82%).

MS m/z (ESI): 341.1 [M+H]⁺.

### Step 7: tert-butyl 3-(5-(3-cyano-6-(2-hydroxy-2-methylpropoxy)-7-methylpyrazolo[1,5-a]pyridin-4-yl)pyridin-2-yl)-3,6-diazabicyclo[3.1.1]heptane-6-carboxylate

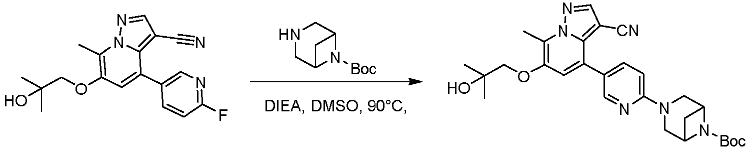

A mixed solution of 4-(6-fluoropyridin-3-yl)-6-(2-hydroxy-2-methylpropoxy)-7-methylpyrazolo[1,5-*a*]pyridine-3-carbonitrile (1.2 g, 3.52 mmol), *tert*-butyl 3,6-diazabicyclo[3.1.1]heptane-6-carboxylate (1.4 g, 7.04 mmol), *N,N*-diisopropylethylamine (1.36 g, 10.56 mmol) and dimethyl sulfoxide (10 mL) was stirred at 100 °C for 24 hours; the reaction was quenched with water and extracted with ethyl acetate (50 mL*3), the combined organic phase was washed with saturated saline, dried over anhydrous sodium sulfate, filtered, and the filtrate was concentrated under reduced pressure to dryness and separated by column chromatography (dichloromethane/methanol = 10:1) to obtain colorless oil *tert-*butyl 3-(5-(3-cyano-6-(2-hydroxy-2-methylpropoxy)-7-methylpyrazolo[1,5-*a*]pyridin-4-yl)pyridin-2-yl)-3,6-diazabicyclo[3.1.1]heptane-6-carboxylate (800 mg, yield: 44%).

MS m/z (ESI): 519.2 [M+H]⁺.

### Step 8: 4-(6-(3,6-diazabicyclo[3.1.1]heptan-3-yl)pyridin-3-yl)-6-(2-hydroxy-2-methylpropoxy)-7-methylpyrazolo[1,5-a]pyridine-3-carbonitrile

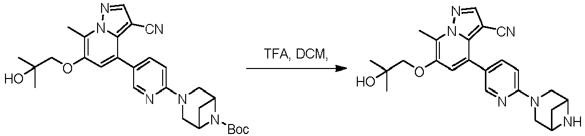

*Tert-*butyl 3-(5-(3-cyano-6-(2-hydroxy-2-methylpropoxy)-7-methylpyrazolo[1,5-*a*]pyridin-4-yl)pyridin-2-yl)-3,6-diazabicyclo[3.1.1]heptane-6-carboxylate (800 mg, 1.54 mmol) was dissolved in dichloromethane (9 mL), then trifluoroacetic acid (3 mL) was added, the mixture was stirred at room temperature for 1 hour; after the reaction was completed, the mixture was concentrated under reduced pressure to dryness and used directly in the next step without purification to obtain light yellow oil 4-(6-(3,6-diazabicyclo[3.1.1]heptan-3-yl)pyridin-3-yl)-6-(2-hydroxy-2-methylpropoxy)-7-methylpyrazolo[1,5-a]pyridine-3-carbonitrile (1 g, crude product).

MS m/z (ESI): 419.2 [M+H]⁺.

### Step 9: 6-(2-hydroxy-2-methylpropoxy)-4-(6-(6-((6-methoxypyridin-3-yl)methyl)-3,6-diazabicyclo[3.1.1]heptan-3-yl)pyridin-3-yl)-7-methylpyrazolo[1,5-a]pyridine-3-carbonitrile

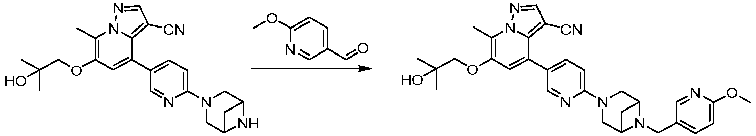

Sodium cyanoborohydride (45 mg, 0.72 mmol) was added to a solution of 4-(6-(3,6-diazabicyclo[3.1.1]heptan-3-yl)pyridin-3-yl)-6-(2-hydroxy-2-methylpropoxy)-7-methylpyrazolo[1,5-*a*]pyridine-3-carbonitrile (100 mg, 0.24 mmol), 6-methoxynicotinaldehyde (66 mg, 0.48 mmol) and 1,2-dichloroethane (3 mL), and then the mixture was stirred at room temperature for 24 hours, after the reaction was completed, the mixture was quenched with water and extracted with ethyl acetate (20 mL*3), and the combined organic phase was washed with saturated saline, dried over anhydrous sodium sulfate, filtered, and the filtrate was concentrated under reduced pressure to dryness and separated by preparative chromatography to obtain white solid 6-(2-hydroxy-2-methylpropoxy)-4-(6-(6-((6-methoxypyridin-3-yl)methyl)-3,6-diazabicyclo[3.1.1]heptan-3-yl)pyridin-3-yl)-7-methylpyrazolo[1,5-*a*]pyridine-3-carbonitrile (10 mg, yield: 8%).

MS m/z (ESI): 540.2 [M+H]⁺.

### [Not according to the invention] Embodiment 12

### 3-(5-(3-Cyano-6-(2-hydroxy-2-methylpropoxy)-7-methylpyrazolo[1,5-a]pyridin-4-yl)pyridin-2-yl)-N-phenyl-3-diazabicyclo[3.1.1]heptane-6-carboxamide

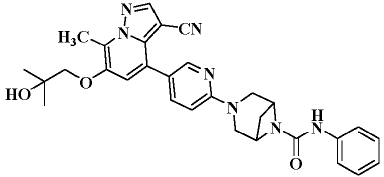

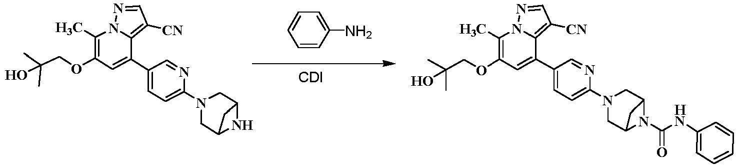

Carbonyldiimidazole (58 mg, 0.36 mmol) was added to a solution of 4-(6-(3,6-diazabicyclo[3.1.1]heptan-3-yl)pyridin-3-yl)-6-(2-hydroxy-2-methylpropoxy)-7-methylpyrazolo[1,5-*a*]pyridine-3-carbonitrile (100 mg, 0.24 mmol), triethylamine (48 mg, 0.48 mmol) and dichloromethane (3 mL), and the mixture was stirred at room temperature for 1 hour, then aniline was added (33 mg, 0.36 mmol) thereto, and the mixture was stirred at room temperature for 16 hours; after the reaction was completed, the mixture was quenched with water and extracted with ethyl acetate (20 ml * 3); the combined organic phase was washed with saturated saline, dried over anhydrous sodium sulfate, filtered, concentrated and dried under reduced pressure to dryness, the residue was separated by preparative chromatography to obtain white solid 3-(5-(3-cyano-6-(2-hydroxyl-2-methylpropoxy)-7-methylpyrazolo[1,5*-a*]pyridin-4-yl)pyridin-2-yl)-*N*-phenyl-3-diazabicyclo[3.1.1]heptane-6-carboxamide (15 mg, yield: 12%).

MS m/z (ESI): 538.2 [M+H]⁺.

### [Not according to the invention] Embodiment 13

### 6-(2-Hydroxy-2-methylpropoxy)-7-methyl-4-(6-(4-(pyridin-3-yloxy)piperidin-1-yl)pyridin-3-yl)pyrazolo[1,5-a]pyridino-3-carbonitrile

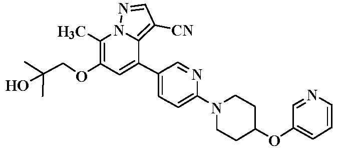

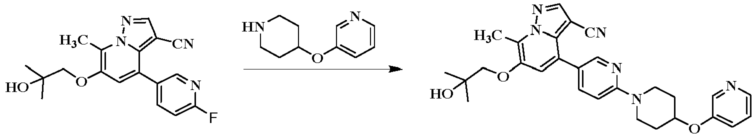

A mixture of 4-(6-fluoropyridin-3-yl)-6-(2-hydroxy-2-methylpropoxy)-7-methylpyrazolo[1,5-*a*]pyridine-3-carbonitrile (100 mg, 0.24 mmol), 3-(piperidin-4-yloxy)pyridine (85 mg, 0.48 mmol), potassium carbonate (99 mg, 0.72 mmol) and acetonitrile (3 mL) was stirred at 100 °C for 16 hours; after the reaction was completed, the mixture was quenched with water and extracted with ethyl acetate (20 mL*3); the combined organic phase was washed with saturated saline, dried over anhydrous sodium sulfate, filtered, and the filtrate was concentrated under reduced pressure to dryness and separated by preparative chromatography to obtain white solid 6-(2-hydroxy-2-methylpropoxy)-7-methyl-4-(6-(4-(pyridin-3-oxy)piperidin-1-yl)pyridin-3-yl)pyrazolo[1,5-*a*]pyridine-3-carbonitrile (15 mg, yield: 13%).

MS m/z (ESI): 499.2 [M+H]⁺.

### [Not according to the invention] Embodiment 14

### 4-(6-(6-((6-Ethoxy-5-fluoropyridin-3-yl)methyl)-3,6-diazabicyclo[3.1.1]heptan-3-yl)pyridin-3-yl)-7-fluoro-6-(2-hydroxy-2-methylpropoxy)pyrazolo[1,5-a]pyridine-3-carbonitrile

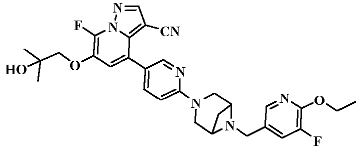

### Step 1: 5-bromo-2-fluoro-3-methoxypyridine

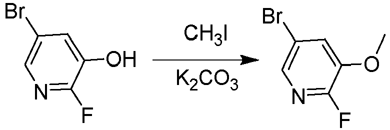

5-Bromo-2-fluoropyridin-3-ol (5 g, 26.18 mmol) was dissolved in acetonitrile (50 mL), then potassium carbonate (7.2 g, 52.36 mmol) was added and iodomethane (11.2 g, 78.54 mmol) was added dropwise, then the reaction mixture was stirred at 80°C for 16 hours; the reaction mixture was cooled to room temperature, concentrated under reduced pressure to dryness, and separated and purified by column chromatography (petroleum ether/ethyl acetate = 1:1) to obtain light yellow solid 5-bromo-2-fluoro-3-methoxypyridine (4.5 g, yield: 83%).

¹H NMR (400 MHz, CDCl₃) δ 7.80 (d, J = 1.8 Hz, 1H), 7.38 (dd, J = 8.8, 1.9 Hz, 1H), 3.91 (s, 3H).

MS m/z (ESI): 206.0 [M+H]⁺.

### Step 2: 2,4,6-trimethylbenzenesulfonic acid 1-amino-5-bromo-2-fluoro-3-methoxypyridine-1-ium

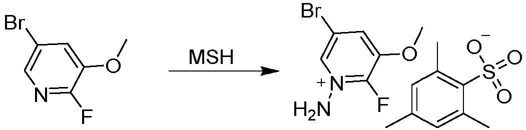

5-Bromo-2-fluoro-3-methoxypyridine (5 g, 21.84 mmol) was added to a solution of 2-[(aminooxy)sulfonyl]-1,3,5-trimethylbenzene (4.7 g, 21.84 mmol) in dichloromethane (50 mL) at 0 °Cin batches, then the mixture was stirred at 0 °C for 1.5 hours; methyl *tert-*butyl ether (100 mL) was added to the reaction mixture, and the mixture was slurried for 15 min, then filtered and the filter cake was dried to obtain light yellow solid 2,4,6-trimethylbenzenesulfonic acid 1-amino-5-bromo-2-fluoro-3-methoxypyridine-1-ium (8 g, yield: 87%).

### Step 3: 4-bromo-7-fluoro-6-methoxypyrazolo[1,5-a]pyridine-3-carbonitrile

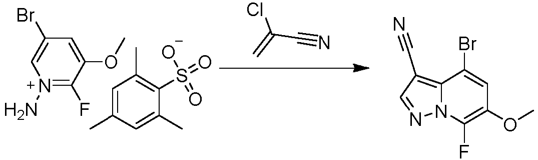

1.8-Diazabicyclo[5.4.0]undec-7-ene (5.8 g, 38 mmol) was added to a solution of 2,4,6-trimethylbenzenesulfonic acid 1-amino-5-bromo-2-fluoro-3-methoxypyridine-1-ium (8 g, 19 mmol) and 2-chloroacrylonitrile (2.5 g, 28.5 mmol) in dichloromethane (80 mL) at 0 °C, then the mixture was warmed up to room temperature and stirred for 24 hours; methyl *tert-*butyl ether (100 mL) was added to the reaction mixture, and the mixture was slurried at room temperature for 15 min, then filtered and the filter cake was dried to obtain light yellow solid 4-bromo-7-fluoro-6-methoxypyrazolo[1,5-*a*]pyridine-3-carbonitrile (3 g, yield: 58%).

MS m/z (ESI): 270.0 [M+H]⁺.

### Step 4: 4-bromo-7-fluoro-6-hydroxypyrazolo[1,5-a]pyridine-3-carbonitrile

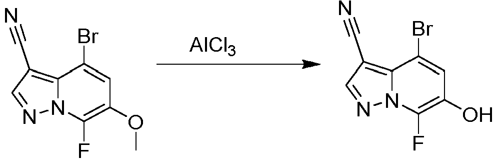

Aluminum trichloride (3 g, 22.22 mmol) was added to a solution of 4-bromo-7-fluoro-6-methoxypyrazolo[1,5-*a*]pyridine-3-carbonitrile (3 g, 11.11 mmol) in 1.2-dichloroethane (30 mL) in batches, then the mixture was stirred at 80 °C for 2 hours, cooled to room temperature, quenched with sodium sulfate decahydrate and filtered, the filter cake was washed with dichloromethane, the filtrate was washed with saturated saline; the organic phase was dried over anhydrous sodium sulfate, filtered, and the filtrate was concentrated under reduced pressure to dryness to obtain white solid 4-bromo-7-fluoro-6-hydroxypyrazolo[1,5-*a*]pyridine-3-carbonitrile (2.2 g, yield: 77%).

MS m/z (ESI): 255.0 [M-H]⁺.

### Step 5: 4-bromo-7-fluoro-6-(2-hydroxy-2-methylpropoxy)pyrazolo[1,5-a]pyridine-3-carbonitrile

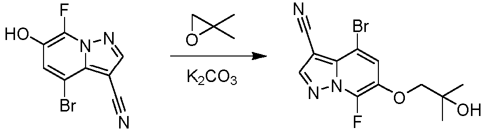

2,2-Dimethyloxirane (1.24 g, 17.2 mmol) was added to a solution of 4-bromo-7-fluoro-6-hydroxypyrazolo[1,5-*a*]pyridine-3-carbonitrile (2.2 g, 8.6 mmol), potassium carbonate (2.37 g, 17.2 mmol) and acetonitrile (25 mL) , and then the reaction mixture was stirred at 80 °C for 16 hours; the reaction mixture was concentrated under reduced pressure to dryness, separated by column chromatography (dichloromethane/methanol = 10:1) to obtain colorless oil 4-bromo-7-fluoro-6-(2-hydroxy-2-methylpropoxy)pyrazolo[1,5-*a*]pyridine-3-carbonitrile (2 g, yield: 71%).

MS m/z (ESI): 328.0 [M+H]⁺.

### Step 6: 7-fluoro-4-(6-fluoropyridin-3-yl)-6-(2-hydroxy-2-methylpropoxy)pyrazolo[1,5-a]pyridine-3-carbonitrile

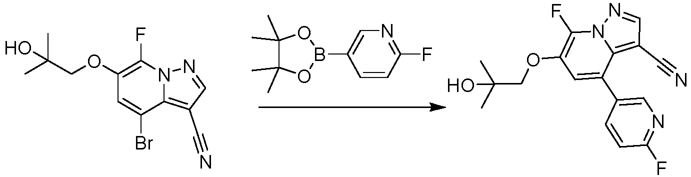

1,1'-Bis(diphenylphosphino)ferrocene-palladium(II)dichloride dichloromethane complex (50 mg, 0.061 mmol) was added to a mixed solution of 4-bromo-7-fluoro-6-(2-hydroxy-2-methylpropoxy)pyrazolo[1,5-*a*]pyridine-3-carbonitrile (2 g, 6.1 mmol), 2-fluoro-5-(4,4,5,5-tetramethyl-1,3,2-dioxaborolan-2-yl)pyridine (1.63 g, 7.32 mmol), potassium acetate (1.2 g, 12.2 mmol) and dioxane (30 mL), the mixture was replaced with nitrogen three times and then stirred at 100 °C under the protection of the protection of nitrogen for 16 hours; after the reaction was complete, the mixture was cooled and filtered, the filtrate was concentrated under reduced pressure to dryness and separated by column chromatography (dichloromethane/methanol = 10:1) to obtain colorless oil 7-fluoro-4-(6-fluoropyridin-3-yl)-6-(2-hydroxy-2-methylpropoxy)pyrazolo[1,5-*a*]pyridine-3-carbonitrile (1.4 g, yield: 67%).

MS m/z (ESI): 345.1 [M+H]⁺.

### Step 7: tert-butyl 3-(5-(3-cyano-7-fluoro-6-(2-hydroxy-2-methylpropoxy)pyrazolo[1,5-a]pyridin-4-yl)pyridin-2-yl)-3,6-diazabicyclo[3.1.1]heptane-6-carboxylate

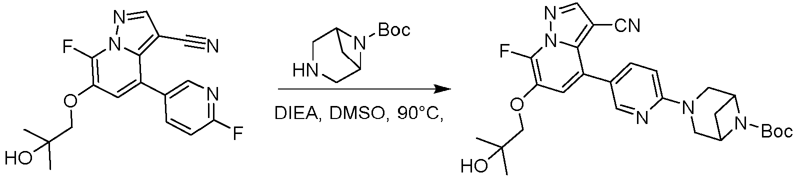

A mixed solution of 7-fluoro-4-(6-fluoropyridin-3-yl)-6-(2-hydroxy-2-methylpropoxy)pyrazolo[1,5-*a*]pyridine-3-carbonitrile (1.4 g, 4.06 mmol), *tert-*butyl 3,6-diazabicyclo[3.1.1]heptane-6-carboxylate (1.61 g, 8.12 mmol), *N*,*N*-diisopropylethylamine (1.57 g, 12.18 mmol) and dimethyl sulfoxide (15 mL) was stirred at 100 °C for 24 hours; after the reaction was complete, the mixture was quenched with water and extracted with ethyl acetate (50 mL*3), the combined organic phase was washed with saturated saline, the organic phase was dried over anhydrous sodium sulfate, filtered, and the filtrate was concentrated under reduced pressure to dryness and separated by column chromatography (dichloromethane/methanol = 10:1) to obtain colorless oil *tert*-butyl 3-(5-(3-cyano-7-fluoro-6-(2-hydroxy-2-methylpropoxy)pyrazolo[1,5-*a*]pyridin-4-yl)pyridin-2-yl)-3,6-diazabicyclo[3.1.1]heptane-6-carboxylate (1.05 g, yield: 50%).

MS m/z (ESI): 523.2 [M+H]⁺.

### Step 8: 4-(6-(3,6-diazabicyclo[3.1.1]heptan-3-yl)pyridin-3-yl)-7-fluoro-6-(2-hydroxy-2-methylpropoxy)pyrazolo[1,5-a]pyridine-3-carbonitrile

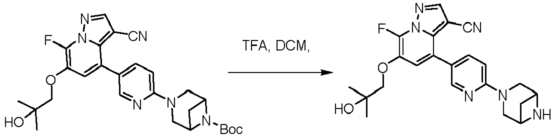

*Tert-*butyl 3-(5-(3-cyano-7-fluoro-6-(2-hydroxy-2-methylpropoxy)pyrazolo[1,5-*a*]pyridin-4-yl)pyridin-2-yl)-3,6-diazabicyclo[3.1.1]heptane-6-carboxylate (1.05 g, 2.01 mmol) was dissolved in dichloromethane (9 mL), then trifluoroacetic acid (3 mL) was added at room temperature for 1 hour; after the reaction was completed, the mixture was concentrated under reduced pressure to dryness and used directly in the next step without purification to obtain light yellow oil 4-(6-(3,6-diazabicyclo[3.1.1]heptan-3-yl)pyridin-3-yl)-7-fluoro-6-(2-hydroxy-2-methylpropoxy)pyrazolo[1,5-*a*]pyridine-3-carbonitrile (1.2 g, crude product).

MS m/z (ESI): 423.2 [M+H]⁺.

### Step 9: 4-(6-(6-((6-ethoxy-5-fluoropyridin-3-yl)methyl)-3,6-diazabicyclo[3.1.1]heptan-3-yl)pyridin-3-yl)-7-fluoro-6-(2-hydroxy-2-methylpropoxy)pyrazolo[1,5-a]pyridine-3-carbonitrile

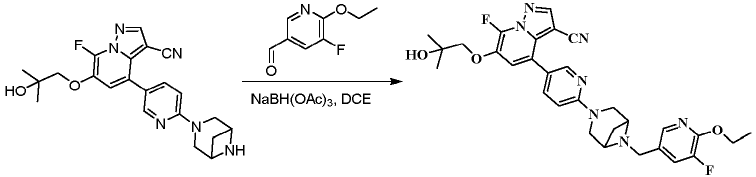

Sodium cyanoborohydride (54 mg, 0.85 mmol) was added to a solution of 4-(6-(3,6-diazabicyclo[3.1.1]heptan-3-yl)pyridin-3-yl)-7-fluoro-6-(2-hydroxy-2-methylpropoxy)-pyrazolo[1,5-*a*]pyridine-3-carbonitrile (120 mg, 0.28 mmol), 6-ethoxy-5-fluoronicotinaldehyde (73 g, 0.43 mmol) and 1,2-dichloroethane (5 mL) , and then the mixture was stirred at room temperature for 24 hours; after the reaction was completed, the mixture was quenched with water and extracted with ethyl acetate (50 mL*3), and the combined organic phase was washed with saturated saline, dried over anhydrous sodium sulfate, filtered, and the filtrate was concentrated under reduced pressure to dryness and separated by preparative chromatography to obtain white solid 4-(6-(6-((6-ethoxy-5-fluoropyridin-3-yl)methyl)-3,6-diazabicyclo[3.1.1]heptan-3-yl)pyridin-3-yl)-7-fluoro-6-(2-hydroxy-2-methylpropoxy)pyrazolo[1,5-*a*]pyridine-3-carbonitrile (15 mg, yield: 9%).

MS m/z (ESI): 576.2 [M+H]⁺.

### [Not according to the invention] Embodiment 15

### 7-Fluoro-6-(2-hydroxy-2-methylpropoxy)-4-(6-(4-(pyridin-2-oxy)piperidin-1-yl)pyridin-3-yl)pyrazolo[1,5-a]pyridine-3-carbonitrile

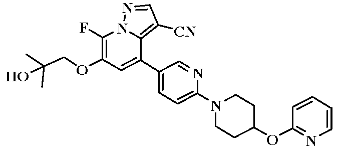

7-Fluoro-4-(6-fluoropyridin-3-yl)-6-(2-hydroxy-2-methylpropoxy)pyrazolo[1,5-*a*]pyridine-3-carbonitrile was used as raw material, the title compound was obtained by the synthetic method with reference to embodiment 13.

MS m/z (ESI): 503.2 [M+H]⁺.

### [Not according to the invention] Embodiment 16

### 7-Fluoro-6-(2-hydroxy-2-methylpropoxy)-4-(6-(4-((6-methylpyridazin-3-yl)oxo)piperidin-1-yl)pyridin-3-yl)pyrazolo[1,5-a]pyridine-3-carbonitrile

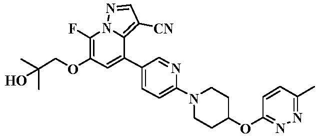

### Step 1: tert-butyl 4-((6-methylpyridazin-3-yl)oxo)piperidine-1-carboxylate

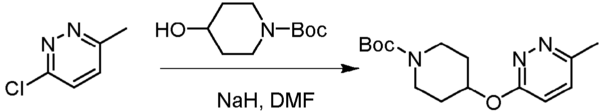

*Tert-*butyl 4-hydroxypiperidine-1-carboxylate (2 g, 9.95 mmol) was added to anhydrous *N,N-*dimethylformamide (20 mL) at 0 °C, then sodium hydrogen (796 mg.19.9 mmol) was added at 0 °C and the mixture was stirred at room temperature for 30 min, then 3-chloro-6-methylpyridazine (1.9 g, 14.93 mmol) was added; after the reaction was complete, the mixture was quenched with water, extracted with ethyl acetate (50 mL*3), and the combined organic phase was washed with saturated saline, dried over anhydrous sodium sulfate, filtered and the filtrate was concentrated under reduced pressure to dryness and separated by column chromatography to obtain white solid *tert*-butyl 4-((6-methylpyridazin-3-yl)oxo)piperidine-1-carboxylate (1.2 g, yield: 41%).

MS m/z (ESI): 294.1 [M+H]⁺.

### Step 2: 3-methyl-6-(piperidin-4-oxy)pyridazine

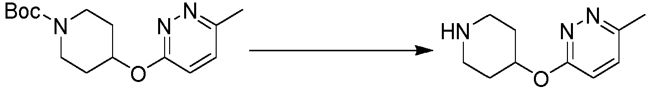

Trifluoroacetic acid (3 mL) was added dropwise to a solution of *tert*-butyl 4-((6-methylpyridazin-3-yl)oxo)piperidine-1-carboxylate (1.2 g, 4.1 mmol) in dichloromethane (9 mL), and then the mixture was stirred at room temperature for 1 hour; after the reaction was completed, the mixture was concentrated under reduced pressure to dryness to obtain light yellow solid 3-methyl-6-(piperidine-4-oxy)pyridazine (1.5 g, crude product).

MS m/z (ESI): 194.1 [M+H]⁺.

### Step 3: 7-fluoro-6-(2-hydroxy-2-methylpropoxy)-4-(6-(4-((6-methylpyridazin-3-yl)oxo)piperidin-1-yl)pyridin-3-yl)pyrazolo[1,5-a]pyridine-3-carbonitrile

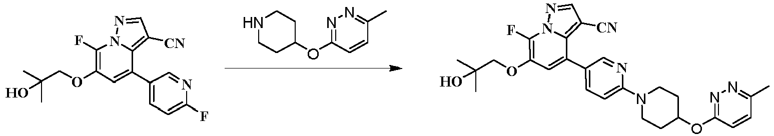

The title compound was obtained by using 4-(6-methylpyridin-3-yl)-6-(2-hydroxy-2-methylpropoxy)-7-methylpyrazolo[1,5-*a*]pyridine-3-carbonitrile and 3-methyl-6-(piperidin-4-oxy)pyridazine as raw material with reference to the method of embodiment 13.

MS m/z (ESI): 518.2 [M+H]⁺.

### [Not according to the invention] Embodiment 17

### 3-(5-(3-Cyano-7-fluoro-6-(2-hydroxy-2-methylpropoxy)pyrazolo[1,5-a]pyridin-4-yl)pyridin-2-yl)-N-phenyl-3,6-diazabicyclo[3.1.1]heptane-6-carboxamide

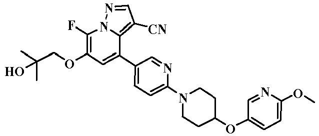

The title compound was obtained by using 7-fluoro-4-(6-fluoropyridin-3-yl)-6-(2-hydroxy-2-methylpropoxy)pyrazolo[1,5-*a*]pyridine-3-carbonitrile as raw material with reference to embodiment 13.

MS m/z (ESI): 533.2 [M+H]⁺.

### [Not according to the invention] Embodiment 18

### 3-(5-(3-Cyano-7-fluoro-6-(2-hydroxy-2-methylpropoxy)pyrazolo[1,5-a]pyridin-4-yl)pyridin-2-yl)-N-phenyl-3,6-diazabicyclo[3.1.1]heptane-6-carboxamide

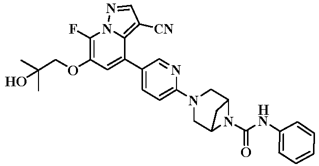

The title compound was obtained by using 7-fluoro-4-(6-fluoropyridin-3-yl)-6-(2-hydroxy-2-methylpropoxy)pyrazolo[1,5-*a*]pyridine-3-carbonitrile as raw material with reference to embodiment 12.

MS m/z (ESI): 542.2 [M+H]⁺.

### [Not according to the invention] Embodiment 19

### 7-Chloro-6-((R)-2-hydroxypropoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

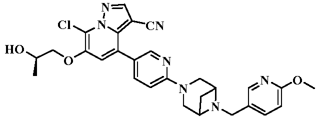

The title compound was obtained with reference to embodiment 14, while 5-bromo-2-chloropyridine-3-ol was replaced with 5-bromo-2-fluoropyridine-3-ol in step 1 and (*R*)-2-methyloxirane was replaced with 2,2-dimethyloxirane in step 5.

MS m/z (ESI): 546.2 [M+H]⁺.

### [Not according to the invention] Embodiment 20

### 5-Chloro-6-(2-hydroxy-2-methylpropoxy)-4-(6-(4-(pyridin-2-oxy)piperidin-1-yl)pyridin-3-yl)pyrazolo[1,5-a]pyridino-3-carbonitrile

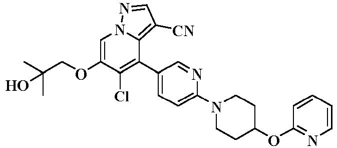

### Step 1: 2,4,6-trimethylbenzenesulfonic acid 1-amino-3-bromo-4-chloro-5-methoxypyridine-1-ium

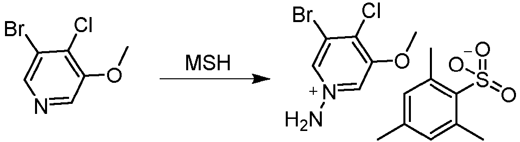

3-Bromo-4-chloro-5-methoxypyridine (5 g, 22.52 mmol) was added to a solution of 2-[(aminooxy)sulfonyl]-1,3,5-trimethylbenzene (4.8 g, 22.52 mmol) in dichloromethane (100 mL) at 0 °C in batches, then the mixture was stirred at 0 °C for 1.5 hours; methyl *tert-*butyl ether (30 mL) was added to the reaction mixture, and the mixture was slurried for 15 min, then filtered and the filter cake was dried to obtain light yellow solid 2,4,6-trimethylbenzenesulfonic acid 1-amino-3-bromo-4-chloro-5-methoxypyridine-1-ium (9 g, crude product).

### Step 2: 4-bromo-5-chloro-6-methoxypyrazolo[1,5-a]pyridine-3-carbonitrile

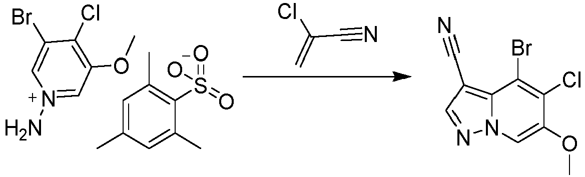

1.8-Diazabicyclo[5.4.0]undec-7-ene (6.3 g, 41.2 mmol) was added to a solution of 2,4,6-trimethylbenzenesulfonic acid 1-amino-3-bromo-4-chloro-5-methoxypyridine-1-ium (9 g, 20.6 mmol) and 2-chloroacrylonitrile (2.7 g, 30.9 mmol) in dichloromethane (100 mL) at 0 °C, then the mixture was warmed up to room temperature and stirred for 24 hours; methyl *tert-*butyl ether (50 mL) was added to the reaction mixture, and the mixture was slurried at room temperature for 15 min, then filtered and the filter cake was dried to obtain light yellow solid 4-bromo-5-chloro-6-methoxypyrazolo[1,5-*a*]pyridine-3-carbonitrile (3 g, yield: 51%).

MS m/z (ESI): 286.0 [M+H]⁺.

### Step 3: 4-bromo-5-chloro-6-hydroxypyrazolo[1,5-a]pyridine-3-carbonitrile

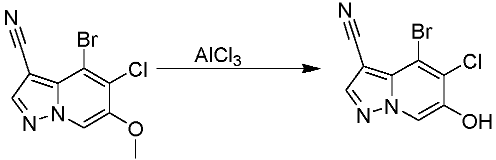

Aluminum trichloride (7 g, 52.63 mmol) was added to a solution of 4-bromo-5-chloro-6-methoxypyrazolo[1,5-*a*]pyridine-3-carbonitrile (3 g, 10.53 mmol) in 1.2-dichloroethane (30 mL) in batches, then the mixture was stirred at 80 °C for 2 hours, cooled to room temperature, quenched with sodium sulfate decahydrate and filtered, the filter cake was washed with dichloromethane, the filtrate was washed with saturated saline; the organic phase was dried over anhydrous sodium sulfate, filtered, and the filtrate was concentrated under reduced pressure to dryness to obtain white solid 4-bromo-5-chloro-6-hydroxypyrazolo[1,5-*a*]pyridine-3-carbonitrile (2.1 g, yield: 74%).

MS m/z (ESI): 269.9 [M+H]⁺.

### Step 4: 4-bromo-5-chloro-6-(2-hydroxy-2-methylpropoxy)pyrazolo[1,5-a]pyridine-3-carbonitrile

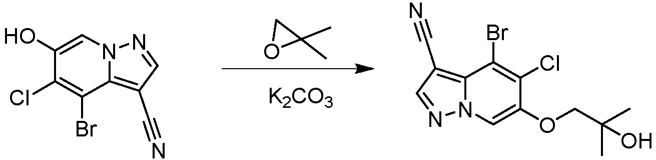

2,2-Dimethyloxirane (1.1 g, 15.5 mmol) was added to a solution of 4-bromo-5-chloro-6-hydroxypyrazolo[1,5-*a*]pyridine-3-carbonitrile (2.1 g, 7.75 mmol), potassium carbonate (2.14 g, 15.5 mmol) and acetonitrile (25 mL), and then the reaction mixture was stirred at 80 °C for 16 hours; the reaction mixture was concentrated under reduced pressure to dryness, separated by column chromatography (dichloromethane/methanol = 10:1) to obtain colorless oil 4-bromo-5-chloro-6-(2-hydroxy-2-methylpropoxy)pyrazolo[1,5-*a*]pyridine-3-carbonitrile (1.4 g, yield: 53%).

MS m/z (ESI): 344.0 [M+H]⁺.

### Step 5: 5-chloro-4-(6-fluoropyridin-3-yl)-6-(2-hydroxy-2-methylpropoxy)pyrazolo[1,5-a]pyridine-3-carbonitrile

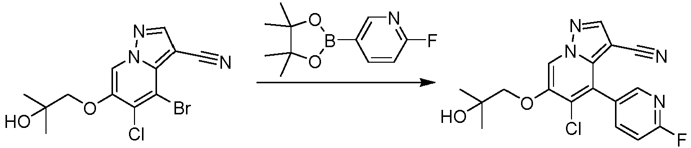

1,1'-Bis(diphenylphosphino)ferrocene-palladium(II)dichloride dichloromethane complex (330 mg, 0.4 mmol) was added to a mixed solution of 4-bromo-5-chloro-6-(2-hydroxy-2-methylpropoxy)pyrazolo[1,5-a]pyridine-3-carbonitrile (1.4 g, 4.08 mmol), 2-fluoro-5-(4,4,5,5-tetramethyl-1,3,2-dioxaborolan-2-yl)pyridine (1.1 g, 4.9 mmol), potassium acetate (800 mg, 8.16 mmol) and dioxane (20 mL), the mixture was replaced with nitrogen three times and then stirred at 100 °C under the protection of nitrogen for 16 hours; after the reaction was complete, the mixture was cooled and filtered, the filtrate was concentrated under reduced pressure to dryness and separated by column chromatography (dichloromethane/methanol = 10:1) to obtain colorless oil 5-chloro-4-(6-fluoropyridin-3-yl)-6-(2-hydroxy-2-methylpropoxy)pyrazolo[1,5-a]pyridine-3-carbonitrile (1 g, yield: 68%).

MS m/z (ESI): 361.1 [M+H]⁺.

### Step 6: 5-chloro-6-(2-hydroxy-2-methylpropoxy)-4-(6-(4-(pyridin-2-yloxy)piperidin-1-yl)pyridin-3-yl)pyrazolo[1,5-a]pyridine-3-carbonitrile

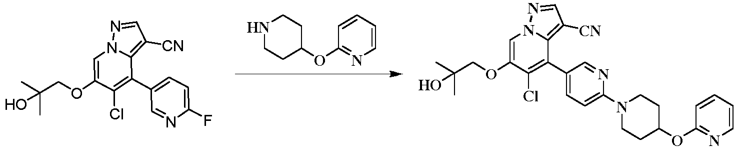

The synthesis of embodiment 22 was carried out with reference to embodiment 13.

MS m/z (ESI): 519.2 [M+H]⁺.

### [Not according to the invention] Embodiment 21

### 5-Fluoro-6-(2-hydroxy-2-methylpropoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

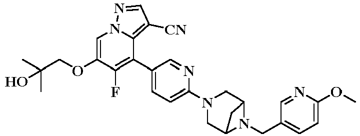

The title compound was obtained with reference to steps 1 to 5 of embodiment 20 by replacing 3-bromo-4-chloro-5-methoxypyridine with 3-bromo-4-fluoro-5-methoxypyridine, and then with reference to steps 7 to 9 of embodiment 14.

MS m/z (ESI): 544.2 [M+H]⁺.

### [Not according to the invention] Embodiment 22

### 5-Fluoro-6-(2-hydroxy-2-methylpropoxy)-4-(6-(4-(pyridin-2-yloxy)piperidin-1-yl)pyridin-3-yl)pyrazolo[1,5-a]pyridine-3-carbonitrile

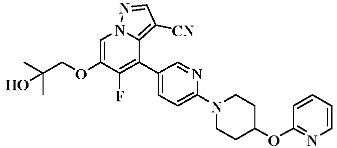

The title compound was obtained with reference to embodiment 20 by replacing 3-bromo-4-chloro-5-methoxypyridine with 3-bromo-4-fluoro-5-methoxypyridine.

MS m/z (ESI): 503.2 [M+H]⁺.

### Embodiment 23

### 6-(3-(2-Hydroxypropan-2-yl)azetidin-1-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

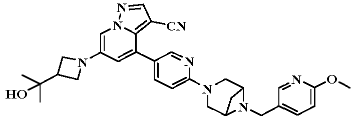

### Step 1: 6-bromo-3-cyanopyrazolo[1,5-a]pyridin-4-yl trifluoromethanesulfonate

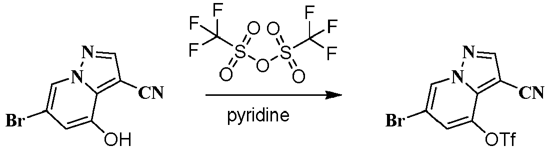

6-Bromo-4-hydroxypyrazolo[1,5-*a*]pyridine-3-carbonitrile was used as raw material to obtain product 6-bromo-3-cyanopyrazolo[1,5-*a*]pyridine-4-yl trifluoromethanesulfonate with reference to step 1 of embodiment 7.

MS m/z (ESI): 370.0[M+H]⁺, 372.0[M+H+2]⁺.

### Step 2: 6-bromo-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

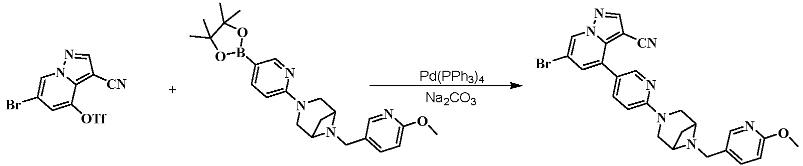

The product 6-bromo-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 6-bromo-3-cyanopyrazolo[1,5-a]pyridine-4-yl trifluoromethanesulfonate as raw material with reference to step 3 of embodiment 7.

MS m/z (ESI): 516.1[M+H]⁺, 518.1[M+H+2]⁺.

### Step 3: 6-(3-(2-hydroxypropan-2-yl)azetidin-1-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

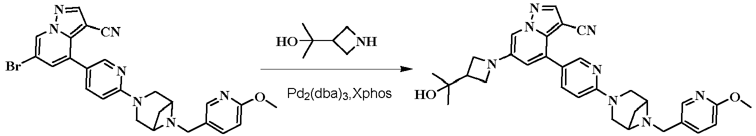

A mixture of 6-bromo-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (80 mg, 0.15 mmol), 2-(azetidin-3-yl)propan-2-ol (36 mg, 0.30 mmol), tris(dibenzylideneacetone)dipalladium (7 mg, 0.0075 mmol), 2-dicyclohexylphosphino-2',4',6'-triisopropylbiphenyl (4 mg, 0.0075 mmol), cesium carbonate (146 mg, 0.45 mmol) and toluene (4 mL) was replaced with nitrogen, then stirred at 130 °C for 2 hours under microwave conditions. After the reaction was complete, the mixture was cooled to room temperature, concentrated, dissolved in ethyl acetate (20 mL) and washed with saturated saline (15 mL); the organic phase was dried over anhydrous sodium sulfate, filtered, evaporated to dryness, and then purified by preparative chromatography (18 mg, white solid, yield: 21%).

MS m/z (ESI): 551.2[M+H]⁺.

¹H NMR (400 MHz, MeOD) δ 8.32 (d, *J* = 2.1 Hz, 1H), 8.24 (s, 1H), 8.08 (s, 1H), 7.85 - 7.79 (m, 2H), 7.71 (dd, *J* = 8.5, 2.3 Hz, 1H), 6.92 (d, *J* = 1.7 Hz, 1H), 6.86 (d, *J* = 8.8 Hz, 1H), 6.78 (d, *J* = 8.5 Hz, 1H), 3.97 (t, *J* = 7.8 Hz, 2H), 3.92 - 3.84 (m, 7H), 3.78 (d, *J* = 5.6 Hz, 2H), 3.65 (s, 1H), 3.62 (s, 3H), 2.95 - 2.81 (m, 1H), 2.70 (d, *J* = 7.0 Hz, 1H), 1.70 (d, *J* = 8.8 Hz, 1H), 1.21 (s, 6H).

### Embodiment 24

### 6-(3-(2-Hydroxypropan-2-yl)azetidin-1-yl)-4-(6-(4-((6-methoxypyridazin-3-yl)oxo)piperidin-1-yl)pyridin-3-yl)pyrazolo[1,5-a]pyridine-3-carbonitrile

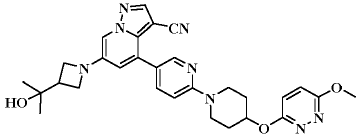

### Step 1: 6-bromo-4-(6-fluoropyridin-3-yl)pyrazolo[1,5-a]pyridine-3-carbonitrile

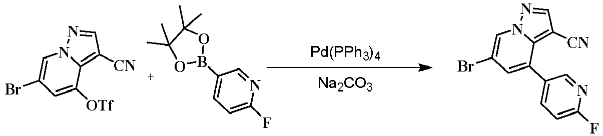

The product 6-bromo-4-(6-fluoropyridin-3-yl)pyrazolo[1,5-*a*]pyridine-3-carbonitrile was obtained by using 6-bromo-3-cyanopyrazolo[1,5-*a*]pyridin-4-yl trifluoromethanesulfonate as raw material with reference to step 3 of embodiment 7.

MS m/z (ESI):317.0[M+H]⁺, 319.0[M+H+2]⁺.

### Step 2: 6-bromo-4-(6-(4-((6-methoxypyridazin-3-yl)oxo)piperidin-1-yl)pyridin-3-yl)pyrazolo[1,5-a]pyridine-3-carbonitrile

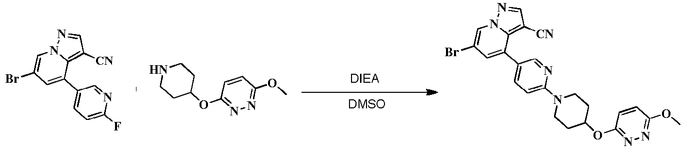

The product 6-bromo-4-(6-(4-((6-methoxypyridazin-3-yl)oxo)piperidin-1-yl)pyridin-3-yl)pyrazolo[1,5-*a*]pyridine-3-carbonitrile was obtained by using 6-bromo-4-(6-fluoropyridin-3-yl)pyrazolo[1,5-*a*]pyridine-3-carbonitrile as raw material with reference to step 2 of embodiment 8.

MS m/z (ESI):506.1[M+H]⁺, 508.1[M+H+2]⁺.

### Step 3: 6-(3-(2-hydroxypropan-2-yl)azetidin-1-yl)-4-(6-(4-((6-methoxypyridazin-3-yl)oxo)piperidin-1-yl)pyridin-3-yl)pyrazolo[1,5-a]pyridine-3-carbonitrile

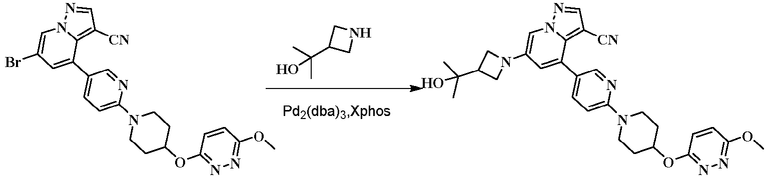

The product 6-(3-(2-hydroxypropan-2-yl)azetidin-1-yl)-4-(6-(4-((6-methoxypyridazin-3-yl)oxo)piperidin-1-yl)pyridin-3-yl)pyrazolo[1,5-*a*]pyridine-3-carbonitrile was obtained by using 6-bromo-4-(6-(4-((6-methoxypyridazin-3-yl)oxo)piperidin-1-yl)pyridine-3-yl)pyrazolo[1,5-*a*]pyridine-3-carbonitrile as raw material with reference to step 2 of embodiment 7.

MS m/z (ESI): 541.2[M+H]⁺.

### [Not according to the invention] Embodiment 25

### (6-(2-Hydroxy-2-methylpropoxy)-4-(6-(6-((6-methoxypyridin-3-yl)methyl)-3,6-diazabicyclo[3.1.1]heptan-3-yl)pyridin-3-yl)pyrazolo[1,5-a]pyridin-3-yl)dimethylphosphine oxide

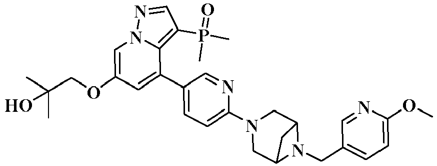

### Step 1: 4-bromo-3-iodopyrazolo[1,5-a]pyridin-6-ol

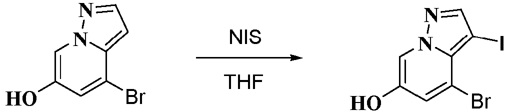

4-Bromopyrazolo[1,5-*a*]pyridin-6-ol (500 mg, 2.3 mmol) was dissolved in 20 mL of THF, and *N-*iodosuccinimide(792 mg, 3.5 mmol) was added, then the reaction was carried out at room temperature for 6 hours. 10 mL of ammonium chloride aqueous solution was added thereto, and the mixture was extracted with ethyl acetate (20mL*3). The organic phase was washed with saturated saline, dried over anhydrous sodium sulfate. The residue was filtered and evaporated to dryness, and the crude product was separated by column chromatography (eluted with dichloromethane/methanol = 10/1) to obtain 4-bromo-3-iodopyrazolo[1,5-*a*]pyridine-6-phenol (404 mg, white solid, the yield was 52%).

MS m/z (ESI): 338.8 [M+H]⁺.

### Step 2: 1-((4-bromo-3-iodopyrazolo[1,5-a]pyridin-6-yl)oxo)-2-methylpropan-2-ol

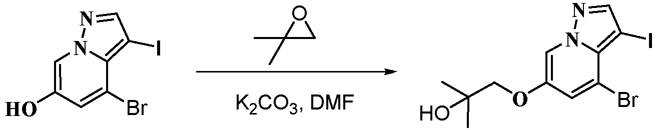

4-Bromo-3-iodopyrazolo[1,5-*a*]pyridin-6-ol (300 mg, 0.89 mmol) was dissolved in 20 mL of DMF, 2,2-dimethyloxirane (641 mg, 8.9 mmol) and K₂CO₃ (368 mg, 2.7 mmol) were added thereto, the reaction was carried out at 85 °C overnight. 10 mL of ammonium chloride aqueous solution was added thereto, and the mixture was extracted with ethyl acetate (20mL*3). The organic phase was washed with saturated saline and dried over anhydrous sodium sulfate. The residue was filtered and evaporated to dryness, and the crude product was separated by column chromatography (eluted with dichloromethane/methanol = 10/1) to obtain 1-((4-bromo-3-iodopyrazolo[1,5-*a*]pyridin-6-yl)oxo)-2-methylpropan-2-ol (262 mg, white solid, the yield was 72%).

MS m/z (ESI): 410.9 [M+H]⁺.

### Step 3: (4-bromo-6-(2-hydroxy-2-methylpropoxy)pyrazolo[1,5-a]pyridin-3-yl)dimethylphosphine oxide

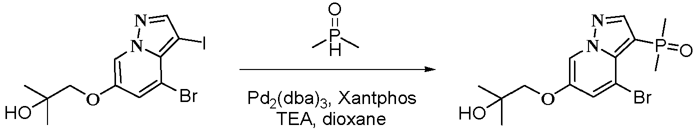

1-((4-Bromo-3-iodopyrazolo[1,5-*a*]pyridin-6-yl)oxo)-2-methylpropan-2-ol (300 mg, 0.89 mmol) was dissolved in 20 mL of dioxane, and dimethylphosphine oxide (104 mg, 1.3 mmol), Pd₂(dba)₃ (82 mg, 0.09 mmol), Xantphos (103 mg, 0.18 mmol) and TEA (270 mg, 2.7 mmol) were added, and the reaction was carried out at 85 °C overnight. 10 mL of ammonium chloride aqueous solution was added thereto, and the mixture was extracted with ethyl acetate (20mL*3). The organic phase was washed with saturated saline, dried over anhydrous sodium sulfate. The residue was filtered and evaporated to dryness, and the crude product was separated by column chromatography (eluted with dichloromethane/methanol = 10/1) to obtain (4-bromo-6-(2-hydroxy-2-methylpropoxy)pyrazolo[1,5-*a*]pyridin-3-yl)dimethylphosphine oxide (180 mg, white solid, the yield was 56%).

MS m/z (ESI): 361.0 [M+H]⁺.

### Step 4: (6-(2-hydroxy-2-methylpropoxy)-4-(4,4,5,5-tetramethyl-1,3,2-dioxaborolan-2-yl)pyrazolo[1,5-a]pyridin-3-yl)dimethylphosphine oxide

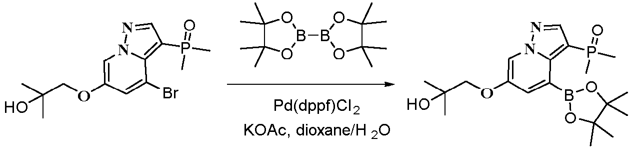

(6-(2-Hydroxy-2-methylpropoxy)-4-(4,4,5,5-tetramethyl-1,3,2-dioxaborolan-2-yl)pyrazolo[1,5-a]pyridin-3-yl)dimethylphosphine oxide (120 mg, white solid, yield was 65%) was obtained by using (4-bromo-6-(2-hydroxy-2-methylpropoxy)pyrazolo[1,5-*a*]pyridin-3-yl)dimethylphosphine oxide as raw material with reference to step 7 of embodiment 1.

MS m/z (ESI): 409.2 [M+H]⁺.

### Step 5: (6-(2-hydroxy-2-methylpropoxy)-4-(6-(6-((6-methoxypyridin-3-yl)methyl)-3,6-diazabicyclo[3.1.1]heptan-3-yl)pyridin-3-yl)pyrazolo[1,5-a]pyridin-3-yl)dimethylphosphine oxide

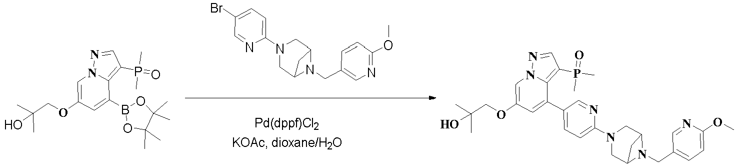

(6-(2-Hydroxy-2-methylpropoxy)-4-(4,4,5,5-tetramethyl-1,3,2-dioxaborolan-2-yl)pyrazolo[1,5-*a*]pyridin-3-yl)dimethylphosphine oxide and 3-(5-bromopyridin-2-yl)-6-((6-methoxypyridin-3-yl)methyl)-3,6-diazabicyclo[3.1.1]heptane were used as raw materials with reference to the step 8 of embodiment **1** to obtain (6-(2-hydroxy-2-methylpropoxy)-4-(6-(6-((6-methoxypyridin-3-yl)methyl)-3,6-diazabicyclo[3.1.1]heptan-3-yl)pyridin-3-yl)pyrazolo[1,5-*a*]pyridin-3-yl)dimethylphosphine oxide (35 mg, white solid, 53%).

MS m/z (ESI): 577.2 [M+H]⁺.

### [Not according to the invention] Embodiment 26

### (4-(6-(6-((5-Fluoro-6-methoxypyridin-3-yl)methyl)-3,6-diazabicyclo[3.1.1]heptan-3-yl)pyridin-3-yl)-6-(2-hydroxy-2-methylpropoxy)pyrazolo[1,5-a]pyridin-3-yl)dimethylphosphine oxide

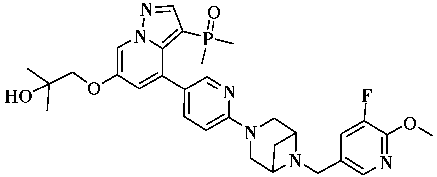

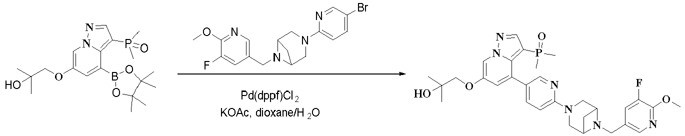

(6-(2-Hydroxy-2-methylpropoxy)-4-(4,4,5,5-tetramethyl-1,3,2-dioxaborolan-2-yl)pyrazolo[1,5-*a*]pyridin-3-yl)dimethylphosphine oxide and 3-(5-bromopyridin-2-yl)-6-((5-fluoro-6-methoxypyridin-3-yl)methyl)-3,6-diazabicyclo[3.1.1]heptane were used as raw materials with reference to the step 8 of embodiment 1 to obtain (4-(6-(6-((5-fluoro-6-methoxypyridin-3-yl)methyl)-3,6-diazabicyclo[3.1.1]heptan-3-yl)pyridin-3-yl)-6-(2-hydroxy-2-methylpropoxy)pyrazolo[1,5-*a*]pyridin-3-yl)dimethylphosphine oxide (28 mg, white solid, 49%).

MS m/z (ESI): 595.2 [M+H]⁺.

### [Not according to the invention] Embodiment 27

### 3-(5-(3-(Dimethylphosphoryl)-6-(2-hydroxy-2-methylpropoxy)pyrazolo[1,5-a]pyridin-4-yl)pyridin-2-yl)-N-phenyl-3,6-diazabicyclo[3.1.1]heptane-6-carboxamide

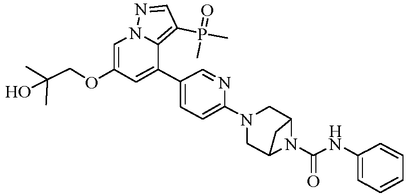

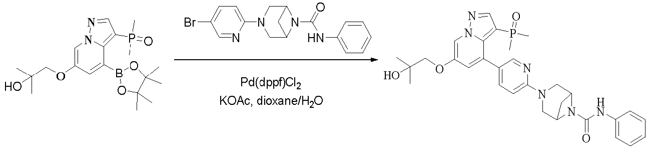

(6-(2-Hydroxy-2-methylpropoxy)-4-(4,4,5,5-tetramethyl-1,3,2-dioxaborolan-2-yl)pyrazolo[1,5-*a*]pyridin-3-yl)dimethylphosphine oxide and 3-(5-bromopyridin-2-yl)-*N*-phenyl-3,6-diazabicyclo[3.1.1]heptane-6-carboxamide were used as raw materials with reference to the step 8 of embodiment 1 to obtain 3-(5-(3-(dimethylphosphoryl)-6-(2-hydroxy-2-methylpropoxy)pyrazolo[1,5-*a*]pyridin-4-yl)pyridin-2-yl)-*N*-phenyl-3,6-diazabicyclo[3,1,1]heptane-6-carboxamide (38 mg, white solid, 56%).

MS m/z (ESI): 575.2 [M+H]⁺.

### [Not according to the invention] Embodiment 28

### 4-(6-(6-((5-Fluoro-6-methoxypyridin-3-yl)methyl)-3,6-diazabicyclo[3.1.1]heptan-3-yl)pyridin-3-yl)-6-(2-hydroxy-2-methylpropoxy)-N,N-dimethylpyrazolo[1,5-a]pyridine-3-carboxamide

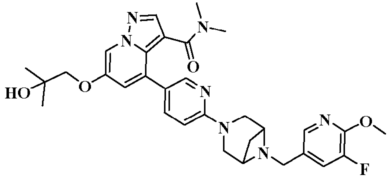

### Step 1: 2,4,6-trimethylbenzenesulfonic acid 1-amino-5-bromo-3-methoxy-2-methylpyridin-1-ium

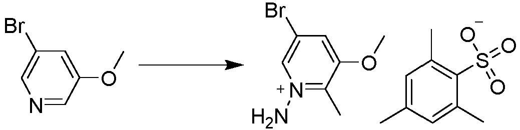

3-Bromo-5-methoxypyridine (10 g, 57.8 mmol) was added to a solution of 2-[(aminooxy)sulfonyl]-1,3,5-trimethylbenzene (12.4 g, 57.8 mmol) in dichloromethane (100 mL) at 0 °C in batches, then the mixture was stirred at 0 °C for 1.5 hours; methyl *tert-*butyl ether (150 mL) was added to the reaction mixture, and the mixture was slurried for 15 min, then filtered and the filter cake was dried to obtain crude white solid 2,4,6-trimethylbenzenesulfonic acid 1-amino-5-bromo-3-methoxy-2-methylpyridine-1-ium (15 g, yield: 62%).

### Step 2: ethyl 4-bromo-6-methoxypyrazolo[1,5-a]pyridine-3-carboxylate

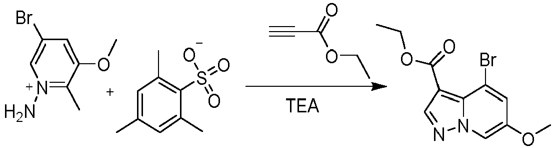

Ethyl propiolate (7.05 g, 71.94 mmol) was added to a solution of 2,4,6-trimethylbenzenesulfonic acid 1-amino-5-bromo-3-methoxy-2-methylpyridin-1-ium (15 g, 35.97 mmol), triethylamine (10.9 g, 107.91 mmol) and anhydrous *N*,*N*-dimethylformamide (100 mL) dropwise at 0 °C; then the reaction mixture was stirred at room temperature for 16 hours, added to ice water and slurried for 15 min, then filtered, the filter cake was dried, and then separated by column chromatography to obtain light yellow solid ethyl 4-bromo-6-methoxypyrazolo[1,5-*a*]pyridine-3-carboxylate (1 g, yield: 9%).

MS m/z (ESI): 299.0 [M+H]⁺.

### Step 3: 4-bromo-6-methoxypyrazolo[1,5-a]pyridine-3-carboxylic acid

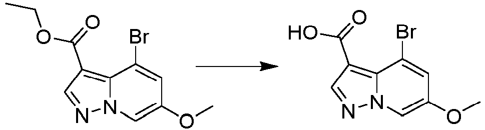

Lithium hydroxide monohydrate (281 mg, 6.68 mmol) was added to a solution of ethyl 4-bromo-6-methoxypyrazolo[1,5-*a*]pyridine-3-carboxylate (1 g, 3.34 mmol) in methanol (10 mL) and water (10 mL), then the mixture was stirred at room temperature for 16 hours; after the reaction was completed, the pH value was adjusted to 2 with dilute hydrochloric acid, and a white solid was precipitated; then the mixture was filtered, and the filter cake was washed with water and dried to obtain white solid 4-bromo-6-methoxypyrazolo[1,5-*a*]pyridine-3-carboxylic acid (800 mg, crude product).

MS m/z (ESI): 271.0 [M+H]⁺.

### Step 4: 4-bromo-6-methoxy-N,N-dimethylpyrazolo[1,5-a]pyridine-3-carboxamide

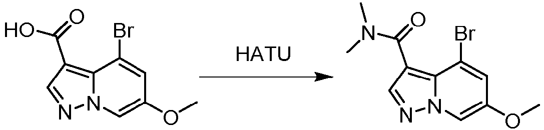

A mixture of 4-bromo-6-methoxypyrazolo[1,5-*a*]pyridine-3-carboxylic acid (800 mg, 2.96 mmol), 2-(7-azabenzotriazol-1-yl)-*N*,*N*,*N*',*N*'-tetramethyluronium hexafluorophosphate (1.35 g, 3.55 mmol), triethylamine (897 mg, 8.88 mmol), *N,N-*dimethylamine hydrochloride (485 mg, 5.92 mmol) and dichloromethane (15 mL) was stirred at room temperature for 16 hours; after the reaction was completed, the mixture was quenched with water, extracted with ethyl acetate, and the organic phase was washed with saturated saline, dried over anhydrous sodium sulfate, filtered, and the filtrate was concentrated under reduced pressure to dryness and separated by column chromatography (dichloromethane/methanol=10:1) to obtain colorless oil 4-bromo-6-methoxy-*N*,*N-*dimethylpyrazolo[1,5-*a*]pyridine-3-carboxamide (700 mg, yield: 80%).

MS m/z (ESI): 298.0 [M+H]⁺.

### Step 5: 4-bromo-6-hydroxy-N,N-dimethylpyrazolo[1,5-a]pyridine-3-carboxamide

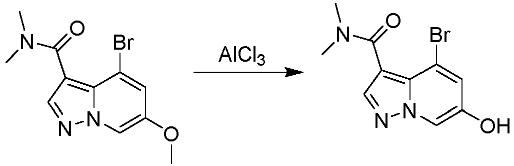

Aluminum trichloride (1.57 g, 11.8 mmol) was added to a solution of 4-bromo-6-methoxy-*N*,*N-*dimethylpyrazolo[1,5-*a*]pyridine-3-carbonitrile (700 mg, 2.36 mmol) in 1.2-dichloroethane (10 mL) in batches, then the mixture was stirred at 80 °C for 2 hours, cooled to room temperature, quenched with sodium sulfate decahydrate, filtered, the filter cake was washed with dichloromethane, the filtrate was washed with saturated saline; the organic phase was dried over anhydrous sodium sulfate, filtered, and the filtrate was concentrated under reduced pressure to dryness to obtain brown solid 4-bromo-6-hydroxy-*N*,*N*-dimethylpyrazolo[1,5-a]pyridine-3-carboxamide (620 mg, crude product).

MS m/z (ESI): 282.0 [M-H]⁺.

### Step 6: 4-bromo-6-(2-hydroxy-2-methylpropoxy)-N,N-dimethylpyrazolo[1,5-a]pyridine-3-carboxamide

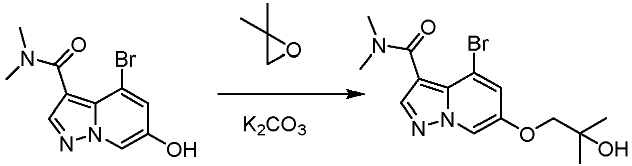

2,2-Dimethyloxirane (317 mg, 4.4 mmol) was added to solution of 4-bromo-6-methoxy-*N*,*N-*dimethylpyrazolo[1,5-*a*]pyridine-3-carboxamide (620 mg, 2.2 mmol), potassium carbonate (605 mg, 4.4 mmol) and acetonitrile (10 mL), and then the reaction mixture was stirred at 80 °C for 16 hours; the reaction mixture was concentrated under reduced pressure to dryness, separated by column chromatography (dichloromethane/methanol = 10:1) to obtain colorless oil 4-bromo-6-(2-hydroxy-2-methylpropoxy)-*N*,*N-*dimethylpyrazolo[1,5-*a*]pyridine-3-carboxamide (520 mg, yield: 67%).

MS m/z (ESI): 356.2 [M+H]⁺.

### Step 7: 4-(6-fluoropyridin-3-yl)-6-(2-hydroxy-2-methylpropoxy)-N,N-dimethylpyrazolo[1,5-a]pyridine-3-carboxamide

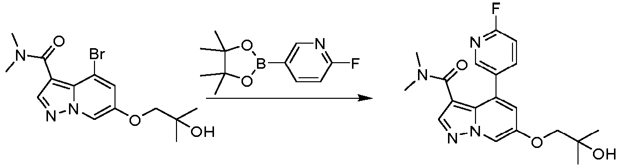

1,1'-Bis(diphenylphosphino)ferrocene-palladium(II)dichloride dichloromethane complex (60 mg, 0.07 mmol) was added to a mixed solution of 4-bromo-6-(2-hydroxy-2-methylpropoxy)-*N*,*N-*dimethylpyrazolo[1,5-*a*]pyridine-3-carboxamide (520 mg, 1.46 mmol), 2-fluoro-5-(4,4,5,5-tetramethyl-1,3,2-dioxaborolan-2-yl)pyridine (391 mg, 1.75 mmol), potassium acetate (286 mg, 2.92 mmol) and dioxane (10 mL), the mixture was replaced with nitrogen three times and then stirred at 100 °C for 16 hours under the protection of the protection of nitrogen; after the reaction was cooled and filtered, the filtrate was concentrated under reduced pressure to dryness and separated by column chromatography (dichloromethane/methanol = 10:1) to obtain colorless oil 4-(6-fluoropyridin-3-yl)-6-(2-hydroxy-2-methylpropoxy)-*N*,*N-*dimethylpyrazolo[1,5-*a*]pyridine-3-carboxamide (405 mg, yield: 75%).

MS m/z (ESI): 373.2 [M+H]⁺.

### Step 8: tert-butyl 3-(5-(3-(dimethylcarbamoyl)-6-(2-hydroxy-2-methylpropoxy)pyrazolo[1,5-a]pyridin-4-yl)pyridin-2-yl)-3,6-diazabicyclo[3.1.1]heptane-6-carboxylate

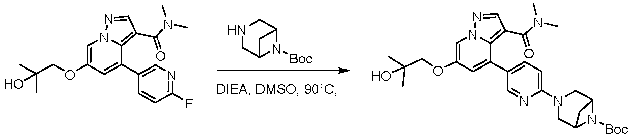

A mixed solution of 4-(6-fluoropyridin-3-yl)-6-(2-hydroxy-2-methylpropoxy)-*N*,*N-*dimethylpyrazolo[1,5-*a*]pyridine-3-carboxamide (405 mg, 1.09 mmol), *tert-*butyl 3,6-diazabicyclo[3.1.1]heptane-6-carboxylate (432 mg, 2.18 mmol), *N*,*N*-diisopropylethylamine (422 mg, 3.27 mmol) and dimethyl sulfoxide (8 mL) was stirred at 100 °C for 24 hours; the reaction mixture was quenched with water and extracted with ethyl acetate (50 mL*3), the combined organic phase was washed with saturated saline, the organic phase was dried over anhydrous sodium sulfate, filtered, and the filtrate was concentrated under reduced pressure to dryness and separated by column chromatography (dichloromethane/methanol = 10:1) to obtain colorless oil *tert-*butyl 3-(5-(3-(dimethylcarbamoyl)-6-(2-hydroxy-2-methylpropoxy)pyrazolo[1,5-*a*]pyridin-4-yl)pyridin-2-yl)-3,6-diazabicyclo[3.1.1]heptane-6-carboxylate (280 mg, yield: 47%).

MS m/z (ESI): 551.2 [M+H]⁺.

### Step 9: 4-(6-fluoropyridin-3-yl)-6-(2-hydroxy-2-methylpropoxy)-N,N-dimethylpyrazolo[1,5-a]pyridine-3-carboxamide

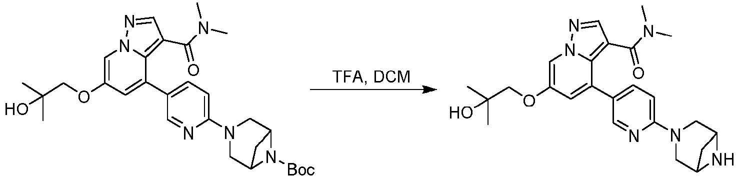

*Tert-*butyl 3-(5-(3-(dimethylcarbamoyl)-6-(2-hydroxy-2-methylpropoxy)pyrazolo[1,5-*a*]pyridin-4-yl)pyridin-2-yl)-3,6-diazabicyclo[3.1.1]heptane-6-carboxylate (280 mg, 0.51 mmol) was dissolved in dichloromethane (9 mL), then trifluoroacetic acid (3 mL) was added thereto at room temperature for 1 hour; after the reaction was completed, concentrated under reduced pressure to dryness and used directly in the next step without purification to obtain light yellow oil 4-(6-fluoropyridin-3-yl)-6-(2-hydroxy-2-methylpropoxy)-*N*,*N*-dimethylpyrazolo[1,5-*a*]pyridine-3-carboxamide (300 mg, crude product).

MS m/z (ESI): 451.2 [M+H]⁺.

### Step 10: 4-(6-(6-((5-fluoro-6-methoxypyridin-3-yl)methyl)-3,6-diazabicyclo[3.1.1]heptan-3-yl)pyridin-3-yl)-6-(2-hydroxy-2-methylpropoxy)-N,N-dimethylpyrazolo[1,5-a]pyridine-3-carboxamide

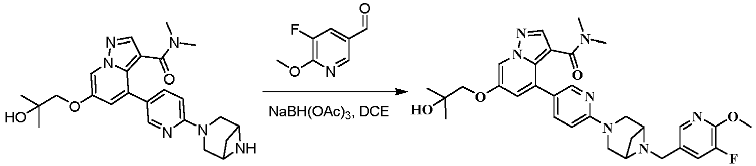

The title compound was obtained with reference to step 9 of embodiment 14 by using 4-(6-fluoropyridin-3-yl)-6-(2-hydroxy-2-methylpropoxy)-*N*,*N*-dimethylpyrazolo[1,5-*a*]pyridine-3-carboxamide as raw material.

MS m/z (ESI): 590.3 [M+H]⁺.

### [Not according to the invention] Embodiment 29

### 6-(2-Methoxyethoxy)-4-(6-(6-((6-methoxypyridin-3-yl)methyl)-3,6-diazabicyclo[3.1.1]heptan-3-yl)pyridin-3-yl)-N,N-dimethylpyrazolo[1,5-a]pyridine-3-carboxamide

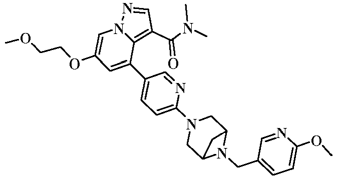

### Step 1: 8-bromo-6-(2-methoxyethoxy)-N,N-dimethylindolizine-1-carboxamide

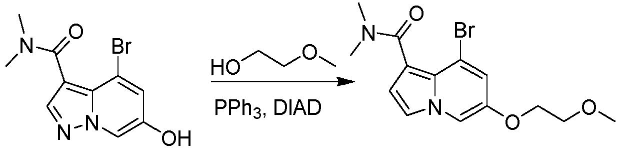

Diisopropyl azodicarboxylate (1.28 g, 6.36 mmol) was added dropwise to a mixed solution of 4-bromo-6-hydroxy-*N*,*N*-dimethylpyrazolo[1,5-*a*]pyridine-3-carboxamide (1.2 g, 4.24 mmol), 2-methoxyethan-1-ol (322 mg.5.09 mmol), triphenylphosphine (1.67 g, 6.36 mmol) and tetrahydrofuran (15 mL), then the mixture was stirred at room temperature under the protection of nitrogen for 16 hours; after the reaction was completed, the mixture was cooled, concentrated under reduced pressure to dryness and separated by column chromatography to obtain colorless oil 8-bromo-6-(2-methoxyethoxy)-*N*,*N*-dimethylindolizine-1-carboxamide (1.3 g, yield: 90%).

MS m/z (ESI): 341.0 [M+H]⁺

6-(2-Methoxyethoxy)-4-(6-(6-((6-methoxypyridine-3-yl)methyl)-3,6-diazabicyclo[3.1.1]heptan-3-yl)pyridin-3-yl)-*N,N*-dimethylpyrazolo[1,5-*a*]pyridine-3-carboxamide was obtained by the reaction of step 2 to step 5 with reference to the synthesis of step 7 to step 10 of embodiment 28.

MS m/z (ESI): 558.3 [M+H]⁺.

### [Not according to the invention] Embodiment 30

### N-Cyclopropyl-6-(2-hydroxy-2-methylpropoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carboxamide

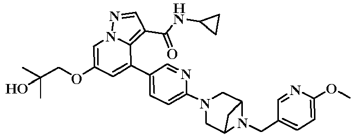

With reference to the synthesis of embodiment 28, the title compound was obtained by replacing *N*, *N*-dimethylamine hydrochloride with cyclopropylamine hydrochloride in step 4.

MS m/z (ESI): 584.3 [M+H]⁺.

### Embodiment 31

### 6-(3-Hydroxy-3-methylbut-1-yn-1-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

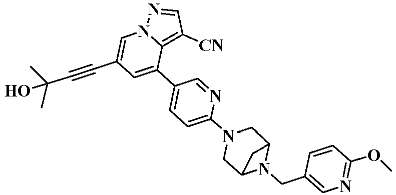

### Step 1: 6-bromo-4-(4,4,5,5-tetramethyl-1,3,2-dioxaborolan-2-yl)pyrazolo[1,5-a]pyridine-3-carbonitrile

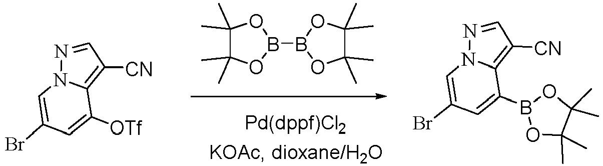

6-Bromo-4-(4,4,5,5-tetramethyl-1,3,2-dioxaborolan-2-yl)pyrazolo[1,5-*a*]pyridine-3-carbonitrile (620 mg, white solid, 56%) was obtained by using 6-bromo-3-cyanopyrazolo[1,5-*a*]pyridin-4-yl trifluoromethanesulfonate as raw material with reference to the step 7 of embodiment 1.

MS m/z (ESI): 348.0 [M+H]⁺.

### Step 2: 6-(3-hydroxy-3-methylbut-1-yn-1-yl)-4-(4,4,5,5-tetramethyl-1,3,2-dioxaborolan-2-yl)pyrazolo[1,5-a]pyridine-3-carbonitrile

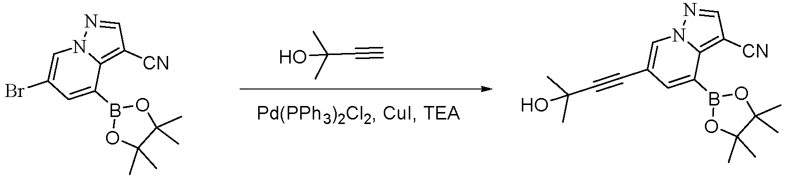

6-Bromo-4-(4,4,5,5-tetramethyl-1,3,2-dioxaborolan-2-yl)pyrazolo[1,5-*a*]pyridine-3-carbonitrile (300 mg, 0.86 mmol) was dissolved in 20 mL of triethylamine, and 2-methylbut-3-yn-2-ol (108 mg, 1.3 mmol), Pd₂(PPh₃)₂Cl₂ (120 mg, 0.17 mmol), CuI (17 mg, 0.09 mmol) were added thereto, and the reaction was carried out overnight at 65°C under the protection of nitrogen. 10 mL of ammonium chloride aqueous solution was added, and the mixture was extracted with ethyl acetate (20mL*3). The organic phase was washed with saturated saline, dried over anhydrous sodium sulfate. The residue was filtered and evaporated to dryness, and the crude product was separated by column chromatography (eluted with dichloromethane/methanol = 10/1) to obtain 6-(3-hydroxy-3-methylbut-1-yn-1-yl)-4-(4,4,5,5-tetramethyl-1,3,2-dioxaborolan-2-yl)pyrazolo[1,5-*a*]pyridine-3-carbonitrile (197 mg, white solid, yield was 65%).

MS m/z (ESI): 352.1 [M+H]⁺.

### Step 3: 6-(3-hydroxy-3-methylbut-1-yn-1-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

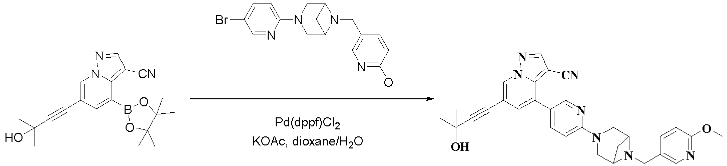

6-(3-Hydroxy-3-methylbut-1-yn-1-yl)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (25 mg, white solid, 43%) was obtained by using 6-(3-hydroxy-3-methylbut-1-yn-1-yl)-4-(4,4,5,5-tetramethyl-1,3,2-dioxaborolan-2-yl)pyrazolo[1,5-*a*]pyridine-3-carbonitrile and 3-(5-bromopyridin-2-yl)-6-((6-methoxypyridin-3-yl)methyl)-3,6-diazabicyclo[3.1.1]heptane as raw materials with reference to the step 8 of embodiment **1.**

MS m/z (ESI): 520.2 [M+H]⁺.

¹H NMR (400 MHz, MeOD) δ 8.83 (s, 1H), 8.47 (s, 1H), 8.35 (d, J = 2.1 Hz, 1H), 8.09 (s, 1H), 7.84 (dd, J = 8.8, 2.3 Hz, 1H), 7.72 (dd, J = 8.4, 2.1 Hz, 1H), 7.41 (s, 1H), 6.88 (d, J = 8.9 Hz, 1H), 6.78 (d, J = 8.5 Hz, 1H), 3.91 (s, 1H), 3.88 (s, 4H), 3.79 (d, J = 5.7 Hz, 2H), 3.66 (s, 1H), 3.63 (s, 3H), 2.74 - 2.62 (m, 1H), 1.70 (d, J = 8.9 Hz, 1H), 1.59 (s, 6H).

### Embodiment 32

### 6-(3-Hydroxy-3-methylbut-1-yn-1-yl)-4-(6-(4-((6-methoxypyridin-3-yl)oxo)piperidin-1-yl)pyridin-3-yl)pyrazolo[1,5-a]pyridine-3-carbonitrile

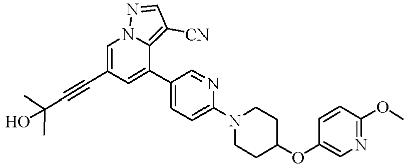

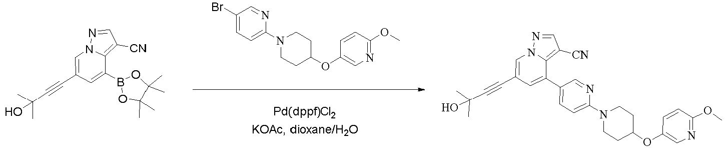

6-(3-Hydroxy-3-methylbut-1-yn-1-yl)-4-(6-(4-((6-methoxypyridin-3-yl)oxo)piperidin-1-yl)pyridin-3-yl)pyrazolo[1,5-*a*]pyridine-3-carbonitrile (33 mg, white solid, 49%) was obtained by using 6-(3-hydroxy-3-methylbut-1-yn-1-yl)-4-(4,4,5,5-tetramethyl-1,3,2-dioxaborolan-2-yl)pyrazolo[1,5-*a*]pyridine-3-carbonitrile and 5-bromo-2-(4-((6-methoxypyridin-3-yl)oxo)piperidin-1-yl)pyridine as raw materials with reference to the step 8 of embodiment **1.**

MS m/z (ESI): 509.2 [M+H]⁺.

¹H NMR (400 MHz, MeOD) δ 8.82 (s, 1H), 8.45 (s, 1H), 8.29 (d, *J* = 2.2 Hz, 1H), 7.84 (d, *J* = 2.9 Hz, 1H), 7.76 (dd, *J* = 8.8, 2.4 Hz, 1H), 7.43 (dd, *J* = 8.9, 3.0 Hz, 1H), 7.39 (s, 1H), 6.99 (d, *J* = 9.0 Hz, 1H), 6.76 (d, *J* = 8.9 Hz, 1H), 4.67 - 4.47 (m, 2H), 4.10 - 3.98 (m, 2H), 3.86 (s, 3H), 3.60 - 3.49 (m, 2H), 2.11 - 2.04 (m, 2H), 1.84 - 1.76 (m, 2H), 1.58 (s, 6H).

### Embodiment 33

### N-(1-(5-(3-cyano-6-(3-hydroxy-3-methylbut-1-yn-1-yl)pyrazolo[1,5-a]pyridin-4-yl)pyridin-2-yl)-4-methylpiperidin-4-yl)-5-fluoro-2-methylbenzamide

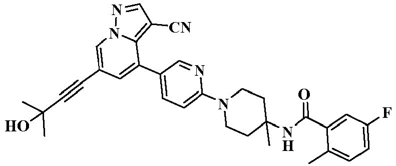

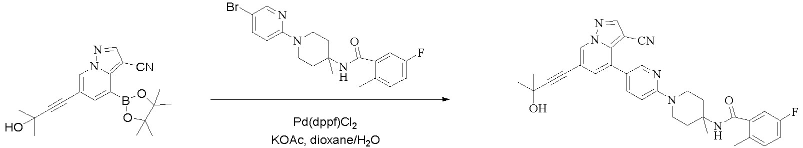

N-(1-(5-(3-cyano-6-(3-hydroxy-3-methylbut-1-yn-1-yl)pyrazolo[1,5-*a*]pyridin-4-yl)pyridin-2-yl)-4-methylpiperidin-4-yl)-5-fluoro-2-methylbenzamide (30 mg, white solid, 45%) was obtained by using 6-(3-hydroxy-3-methylbut-1-yn-1-yl)-4-(4,4,5,5-tetramethyl-1,3,2-dioxaborolan-2-yl)pyrazolo[1,5-a]pyridine-3-carbonitrile and *N*-(1-(5-bromopyridin-2-yl)-4-methylpiperidin-4-yl)-5-fluoro-2-methylbenzamide as raw materials with reference to step 8 of embodiment **1**.

MS m/z (ESI): 551.2 [M+H]⁺.

¹H NMR (400 MHz, Methanol-d₄) δ 8.82 (d, J = 1.3 Hz, 1H), 8.46 (s, 1H), 8.29 (d, J = 2.5 Hz, 1H), 7.76 (dd, J = 9.0, 2.5 Hz, 1H), 7.41 - 7.36 (m, 1H), 7.28 - 7.21 (m, 1H), 7.09 - 7.02 (m, 2H), 6.98 (d, J = 8.9 Hz, 1H), 4.10 - 4.00 (m, 2H), 3.44 - 3.39 (m, 2H), 2.44 - 2.38 (m, 2H), 2.38 (s, 3H), 1.77 - 1.69 (m, 2H), 1.58 (s, 6H), 1.54 (s, 3H).

### Embodiment 34

### 6-(2-Cyano-2-methylpropoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

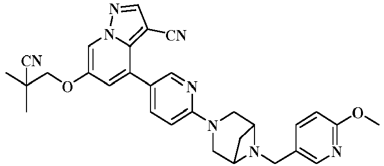

### Step 1: 4-bromo-6-(2-cyano-2-methylpropoxy)pyrazolo[1,5-a]pyridine-3-carbonitrile

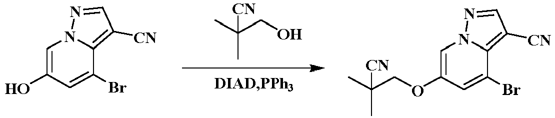

4-Bromo-6-hydroxypyrazolo[1,5-*a*]pyridine-3-carbonitrile (200 mg, 0.84 mmol) and 3-hydroxy-2,2-dimethylpropionitrile (83 mg, 0.84 mmol) were dissolved in 5 mL of anhydrous tetrahydrofuran solution, and then triphenylphosphine (330 mg, 1.26 mmol) and diisopropyl azodicarboxylate (202 mg, 1 mmol) were added thereto, and the reaction mixture was stirred at 0 °C for 12 hours under the protection of nitrogen. The reaction mixture was concentrated, dissolved in ethyl acetate (10 mL), washed three times with water (5 mL*3), and the organic phase was concentrated and separated by column chromatography (dichloromethane/methanol: 30/1) and purified to obtain product 4-bromo-6-(2-cyano-2-methylpropoxy)pyrazolo[1,5-*a*]pyridine-3-carbonitrile (180 mg, yellow solid, the yield was 67.1%).

MS m/z (ESI):319.0 [M+H]⁺. 321.0 [M+H+2]⁺.

### Step 2: 6-(2-cyano-2-methylpropoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

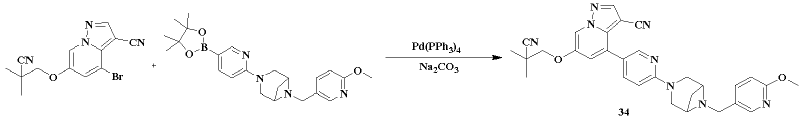

The product 6-(2-cyano-2-methylpropoxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 4-bromo-6-(2-cyano-2-methylpropoxy)pyrazolo[1,5-a]pyridine-3-carbonitrile as raw material with reference to step 3 of embodiment 7.

MS m/z (ESI):535.2[M+H]⁺.

¹H NMR (400 MHz, Chloroform-d) δ 8.43 (d, J = 2.5 Hz, 1H), 8.23 (s, 1H), 8.16 (d, J = 2.1 Hz, 1H), 8.13 (d, J = 2.4 Hz, 1H), 7.81-7.75 (m, 2H), 7.19 (d, J = 2.1 Hz, 1H), 6.75 (d, J = 8.5 Hz, 1H), 6.70 (d, J = 8.8 Hz, 1H), 3.97 (s, 3H), 3.96 - 3.86 (m, 6H), 3.78 - 3.64 (m, 4H), 2.94 - 2.80 (m, 1H), 1.78 - 1.72 (m, 1H), 1.55 (s, 6H).

### Embodiment 35

### 6-(2-Cyano-2-methylpropoxy)-4-(6-(4-((6-methylpyridazin-3-yl)oxo)piperidin-1-yl)pyridin-3-yl)pyrazolo[1,5-a]pyridino-3-carbonitrile

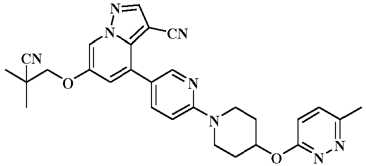

### Step 1: 6-(2-cyano-2-methylpropoxy)-4-(6-fluoropyridin-3-yl)pyrazolo[1,5-a]pyridine-3-carbonitrile

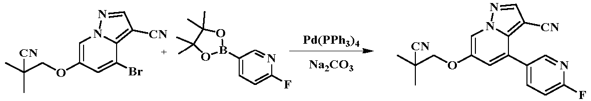

The product 6-(2-cyano-2-methylpropoxy)-4-(6-fluoropyridin-3-yl)pyrazolo[1,5-*a*]pyridine-3-carbonitrile was obtained by using 4-bromo-6-(2-cyano-2-methylpropoxy)pyrazolo[1,5*-a*]pyridine-3-carbonitrile as raw material with reference to step 3 of embodiment 7.

MS m/z (ESI):336.1[M+H]⁺ .

### Step 2: 6-(2-cyano-2-methylpropoxy)-4-(6-(4-((6-methylpyridazin-3-yl)oxo)piperidin-1-yl)pyridin-3-yl)pyrazolo[1,5-a]pyridine-3-carbonitrile

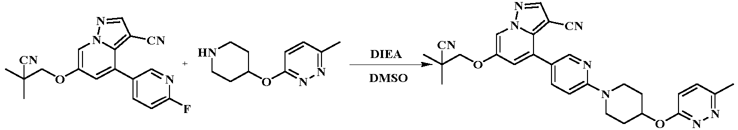

The product 6-(2-cyano-2-methylpropoxy)-4-(6-(4-((6-methylpyridazin-3-yl)oxo)piperidin-1-yl)pyridin-3-yl)pyrazolo[1,5-*a*]pyridine-3-carbonitrile was obtained by using 6-(2-cyano-2-methylpropoxy)-4-(6-fluoropyridin-3-yl)pyrazolo[1,5-*a*]pyridine-3-carbonitrile as raw material with reference to step 2 of embodiment 8.

MS m/z (ESI):509.2[M+H]⁺.

### Embodiment 36

### 6-((1-Cyanocyclopropyl)methoxy)-4-(6-(4-(pyridin-2-oxy)piperidin-1-yl)pyridin-3-yl)pyrazolo[1,5-a]pyridino-3-carbonitrile

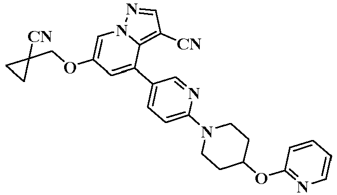

### Step 1: 4-bromo-6-((1-cyanocyclopropyl)methoxy)pyrazolo[1,5-a]pyridine-3-carbonitrile

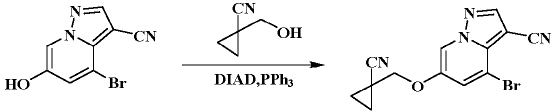

The product 4-bromo-6-((1-cyanocyclopropyl)methoxy)pyrazolo[1,5-*a*]pyridine-3-carbonitrile was obtained by using 4-bromo-6-hydroxypyrazolo[1,5-*a*]pyridine-3-carbonitrile as raw material with reference to step 1 of embodiment 34. MS m/z (ESI):317.0[M+H]⁺ , 319.0[M+H+2]⁺.

### Step 2: 6-((1-cyanocyclopropyl)methoxy)-4-(6-fluoropyridin-3-yl)pyrazolo[1,5-a]pyridine-3-carbonitrile

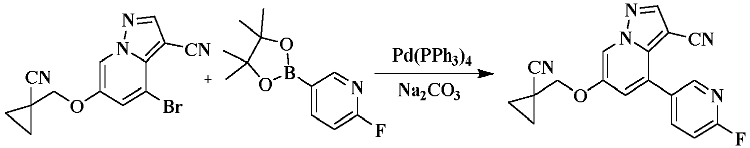

The product 6-((1-cyanocyclopropyl)methoxy)-4-(6-fluoropyridin-3-yl)pyrazolo[1,5-*a*]pyridine-3-carbonitrile was obtained by using 4-bromo-6-((1-cyanocyclopropyl)methoxy)pyrazolo[1,5-*a*]pyridine-3-carbonitrile as raw material with reference to step 3 of embodiment 7.

MS m/z (ESI):334.1[M+H]⁺ .

### Step 3: 6-((1-cyanocyclopropyl)methoxy)-4-(6-(4-(pyridin-2-oxy)piperidin-1-yl)pyridin-3-yl)pyrazolo[1,5-a]pyridine-3-carbonitrile

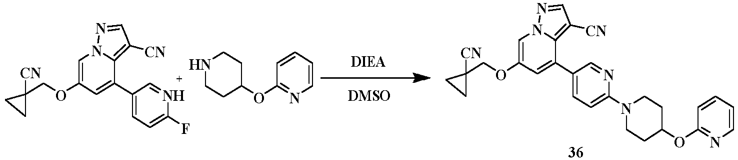

The product 6-((1-cyanocyclopropyl)methoxy)-4-(6-(4-(pyridin-2-oxy)piperidin-1-yl)pyridin-3-yl)pyrazolo[1,5-*a*]pyridine-3-carbonitrile was obtained by using 6-((1-cyanocyclopropyl)methoxy)-4-(6-fluoropyridin-3-yl)pyrazolo[1,5-a]pyridine-3-carbonitrile as raw material with reference to step 2 of embodiment 8.

MS m/z (ESI):492.2[M+H]⁺.

### [Not according to the invention] Embodiment 37

### 6-((6-Methoxypyridin-3-yl)methyl)-3-(5-(6-((1-methylazetidin-3-yl)methoxy)-[1,2,4]triazolo[1,5-a]pyridin-8-yl)pyridin-2-yl)-3,6-diazabicyclo[3.1.1]heptane

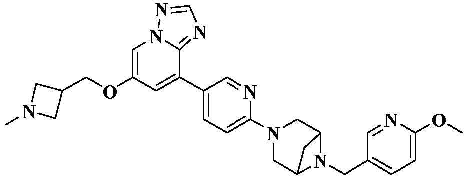

### Step 1: preparation of N-(3,5-dibromopyridin-2-yl)-N'-hydroxyformimidamide

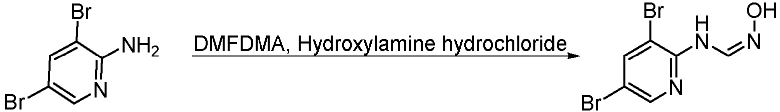

3,5-Dibromopyridin-2-amine (10 g, 39.7 mmol) was dissolved in isopropanol (100 mL), and DMFDMA (6.15 g, 51.6 mmol) was added thereto. The reaction mixture was stirred at 100 °C for 2 hours. The reaction mixture was cooled to 50 °C, hydroxylamine hydrochloride (3.59 g, 51.6 mmol) was added thereto, and then stirred overnight. The reaction mixture was evaporated to dryness directly. The crude product was purified by column chromatography to obtain the target molecule *N*-(3,5- dibromopyridine-2-yl)-*N'*-hydroxyformamidine (11 g, yield: 94%).

MS m/z (ESI): 293.8[M+H]⁺.

### Step 2: preparation of 6,8-dibromo-[1,2,4]triazolo[1,5-a]pyridine

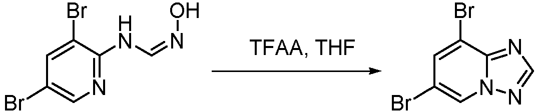

*N*-(3,5*-*dibromopyridin-2-yl)-*N'*-hydroxyformamidine (11 g, 37.3 mmol) was dissolved in anhydrous tetrahydrofuran (100 mL), and TFAA(8.62 g, 41.0 mmol) was slowly added thereto dropwise at 0°C. After the addition was completed, the reaction mixture was slowly raised to room temperature, and stirring was continued for 3 hours. NaHCO₃ aqueous solution was slowly added to the reaction mixture to quench the reaction, and then the mixture was extracted with methyl *tert-butyl* ether. The organic phase was dried and evaporated to dryness. The crude product was purified by column chromatography to obtain 6,8-dibromo-[1,2,4]triazolo[1,5-a]pyridine (8.2 g, yield: 79%).

MS m/z (ESI): 275.8[M+H]⁺.

### Step 3: preparation of 3-(5-(6-bromo-[1,2,4]triazolo[1,5-a]pyridin-8-yl)pyridin-2-yl)-6-((6-methoxypyridin-3-yl)methyl)-3,6-diazabicyclo[3.1.1]heptane

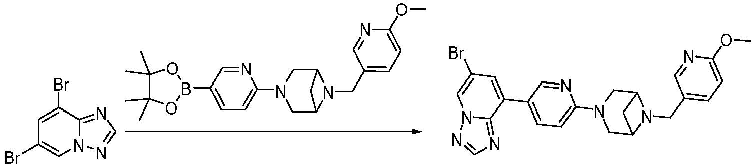

6,8-Dibromo-[1,2,4]triazolo[1,5-a]pyridine (4 g, 14.4 mmol) was dissolved in DMF (50 mL), and 6-((6-methoxypyridin-3-yl)methyl)-3-(5-(4,4,5,5-tetramethyl-1,3,2-dioxaborolan-2-yl)pyridin-2-yl)-3,6-diazabicyclo[3.1.1]heptane (7.3 g, 17.3 mmol) and saturated sodium carbonate aqueous solution (15 mL) were added thereto. [1,1'-Bis(diphenylphosphino)ferrocene]palladium dichloride (1.05 g, 1.44 mmol) was added to the reaction mixture under the protection of nitrogen. The reaction mixture was stirred at 90 °C for 3 hours. Water (50 mL) was added thereto, and then the mixture was extracted with ethyl acetate (100 mL). The organic phase was dried and evaporated to dryness. The crude product was purified by column chromatography to obtain the target molecule 3-(5-(6-bromo-[1,2,4]triazolo[1,5-*a*]pyridin-8-yl)pyridin-2-yl)-6-((6-methoxypyridin-3-yl)methyl)-3,6-diazabicyclo[3.1.1]heptane (3.5 g, yield: 49%).

MS m/z (ESI): 492.1[M+H]⁺.

### Step 4: preparation of 8-(6-(6-((6-methoxypyridin-3-yl)methyl)-3,6-diazabicyclo[3.1.1]heptan-3-yl)pyridin-3-yl)-[1,2,4]triazolo[1,5-a]pyridin-6-ol

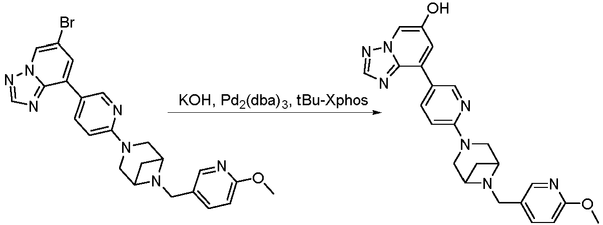

3-(5-(6-Bromo-[1,2,4]triazolo[1,5-*a*]pyridin-8-yl)pyridin-2-yl)-6-((6-methoxypyridin-3-yl)methyl)-3,6-diazabicyclo[3.1.1]heptane (3.5 g, 7.1 mmol) was dissolved in dioxane (40 mL), KOH (0.6 g, 10.7 mmol) and water (20 mL) were added thereto. Under the protection of nitrogen, tris (dibenzylideneacetone) dipalladium (0.65 g, 0.71 mmol) and tBu-Xphos(0.6 g, 1.42 mmol) were added thereto. The reaction mixture was stirred at 90 °C overnight. Water (50 mL) was added thereto, and then the reaction mixture was extracted with ethyl acetate (100 mL). The organic phase was dried and evaporated to dryness. The crude product was purified by column chromatography to obtain 8-(6-(6-((6-methoxypyridin-3-yl)methyl)-3,6-diazabicyclo[3.1.1]heptan-3-yl)pyridin-3-yl)-[1,2,4]triazolo[1,5-*a*]pyridin-6-ol) (2 g, yield: 65%).

MS m/z (ESI): 430.2[M+H]⁺.

### Step 5: preparation of 6-((6-methoxypyridin-3-yl)methyl)-3-(5-(6-((1-methylazetidin-3-yl)methoxy)-[1,2,4]triazolo[1,5-a]pyridin-8-yl)pyridin-2-yl)-3,6-diazabicyclo[3.1.1]heptane

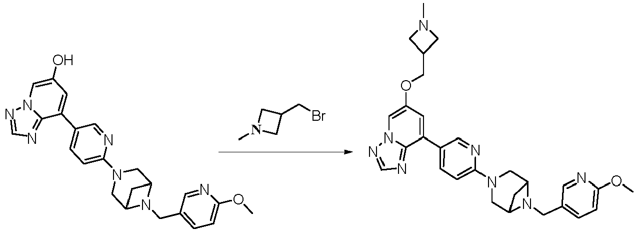

8-(6-(6-((6-Methoxypyridin-3-yl)methyl)-3,6-diazabicyclo[3.1.1]heptan-3-yl)pyridin-3-yl)-[1,2,4]triazolo[1,5-*a*]pyridin-6-ol (300 mg, 0.699 mmol) was dissolved in DMAc (10 mL), and 3-(bromomethyl)-1-methylazetidine (172 mg, 1.05 mmol) and cesium carbonate (569 mg, 1.75 mmol) were added thereto. The reaction mixture was stirred at 100 °C overnight. Water (20 mL) was added to the reaction mixture, and then ethyl acetate (50 mL) was added thereto for extraction. The organic phase was dried and evaporated to dryness. The crude product was purified by prep-HPLC to obtain 6-((6-methoxypyridin-3-yl)methyl)-3-(5-(6-((1-methylazetidin-3-yl)methoxy)-[1,2,4]triazolo[1,5-*a*]pyridin-8-yl)pyridin-2-yl)-3,6-diazabicyclo[3.1.1]heptane (50 mg, yield: 14%).

MS m/z (ESI): 513.2[M+H]⁺.

### [Not according to the invention] Embodiment 38

### 3-(((8-(6-(6-((6-Methoxypyridin-3-yl)methyl)-3,6-diazabicyclo[3.1.1]heptan-3-yl)pyridin-3-yl)-[1,2,4]triazolo[1,5-a]pyridin-6-yl)oxo)methyl)cyclobutan-1-ol

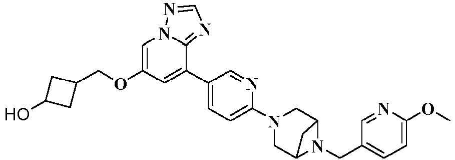

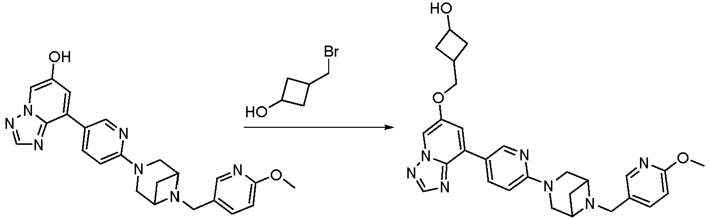

3-(((8-(6-(6-((6-Methoxypyridin-3-yl)methyl)-3,6-diazabicyclo[3.1.1]heptan-3-yl)pyridin-3-yl)-[1,2,4]triazolo[1,5-a]pyridin-6-yl)oxo)methyl)cyclobutan-1-ol (40 mg, yield: 11%) was obtained by using 8-(6-(6-((6-methoxypyridin-3-yl)methyl)-3,6-diazabicyclo[3.1.1]heptan-3-yl)pyridin-3-yl)-[1,2,4]triazolo[1,5-a]pyridin-6-ol and 3-(bromomethyl)cyclobutan-1-ol as raw materials with reference to step 5 of embodiment 37.

MS m/z (ESI): 514.2[M+H]⁺.

### [Not according to the invention] Embodiment 39

### (R)-1-((8-(6-(4-((6-methoxypyridin-3-yl)methyl)piperazin-1-yl)pyridin-3-yl)-[1,2,4]triazolo[1,5-a]pyridin-6-yl)oxo)propan-2-ol

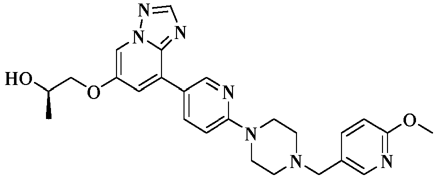

### Step 1: preparation of 6-bromo-8-(6-(4-((6-methoxypyridin-3-yl)methyl)piperazin-1-yl)pyridin-3-yl)-[1,2,4]triazolo[1,5-a]pyridine

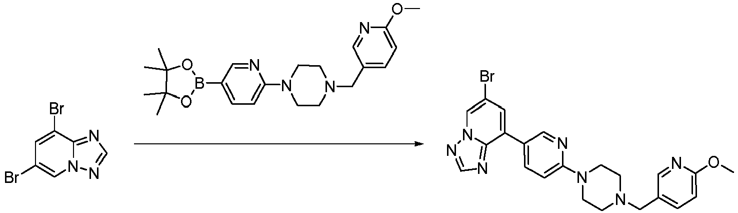

6-Bromo-8-(6-(4-((6-methoxypyridin-3-yl)methyl)piperazin-1-yl)pyridin-3-yl)-[1,2,4]triazolo[1,5-a]pyridine (1 g, yield: 70%) was obtained by using 6,8-dibromo-[1,2,4]triazolo[1,5-*a*]pyridine and 1-((6-methoxypyridin-3-yl)methyl)-4-(5-(4,4,5,5-tetramethyl-1,3,2-dioxaborolan-2-yl)pyridin-2-yl)piperazine with reference to step 3 of embodiment 37.

MS m/z (ESI): 480.2[M+H]⁺.

### Step 2: preparation of 8-(6-(4-((6-methoxypyridin-3-yl)methyl)piperazin-1-yl)pyridin-3-yl)-[1,2,4]triazolo[1,5-a]pyridin-6-ol

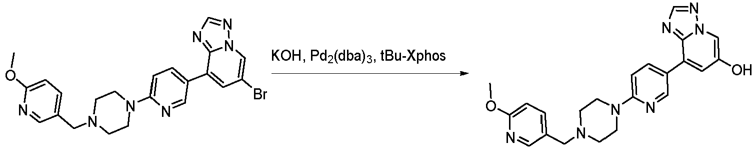

8-(6-(4-((6-Methoxypyridin-3-yl)methyl)piperazin-1-yl)pyridin-3-yl)-[1,2,4]triazolo[1,5-a]pyridin-6-ol (400 mg, yield: 57%) was obtained by using 6-bromo-8-(6-(4-((6-methoxypyridin-3-yl)methyl)piperazin-1-yl)pyridin-3-yl)-[1,2,4]triazolo[1,5-*a*]pyridine as raw material with reference to step 4 of embodiment 37.

MS m/z (ESI): 418.2[M+H]⁺.

### Step 3: preparation of (R)-1-((8-(6-(4-((6-methoxypyridin-3-yl)methyl)piperazin-1-yl)pyridin-3-yl)-[1,2,4]triazolo[1,5-a]pyridin-6-yl)oxo)propan-2-ol

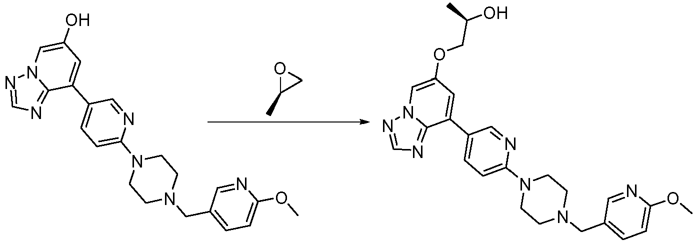

8-(6-(4-((6-Methoxypyridin-3-yl)methyl)piperazin-1-yl)pyridin-3-yl)-[1,2,4]triazolo[1,5-*a*]pyridin-6-ol (300 mg, 0.719 mmol) was dissolved in DMF (5 mL), (*R*)-2-methyloxirane (835 mg, 14.4 mmol) and potassium carbonate (497 mg, 3.59 mmol) were added thereto. The reaction mixture was stirred at 50 °C for 3 days. Water (10 mL) was added thereto, and the reaction mixture was then extracted with ethyl acetate (20 mL). The organic phase was dried and evaporated to dryness. The crude product was purified by prep-HPLC to obtain (*R*)-1-((8-(6-(4-((6-methoxypyridin-3-yl)methyl)piperazin-1-yl)pyridin-3-yl)-[1,2,4]triazolo[1,5-*a*]pyridin-6-yl)oxo)propan-2-ol (100 mg, yield: 29%).

MS m/z (ESI): 476.2[M+H]⁺.

### [Not according to the invention] Embodiment 40

### 7-(2-Hydroxy-2-methylpropoxy)-5-(6-(6-((6-methoxypyridin-3-yl)methyl)-3,6-diazabicyclo[3.1.1]heptan-3-y)pyridin-3-yl)imidazo[1,2-a]pyridine-3-carbonitrile

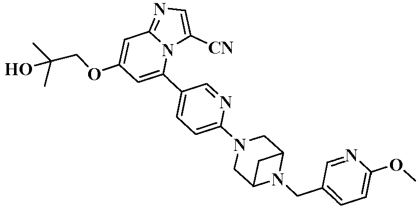

### Step 1: preparation of ethyl 5-bromo-7-methoxyimidazo[1,2-a]pyridine-3-carboxylate

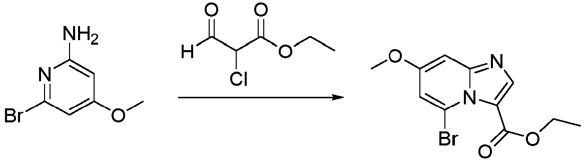

6-Bromo-4-methoxypyridin-2-amine (10 g, 49.2 mmol) was dissolved in ethanol (100 mL), and ethyl 2-chloro-3-carbonyl propionate (7.42 g, 49.2 mmol) was added thereto. The reaction was refluxed overnight. The reaction mixture was evaporated to dryness and directly purified by column chromatography to obtain target molecule ethyl 5-bromo-7-methoxyimidazo[1,2-*a*]pyridine-3-carboxylate (3.0 g, yield: 20%).

MS m/z (ESI): 299.2[M+H]⁺.

### Step 2: preparation of 5-bromo-7-methoxyimidazo[1,2-a]pyridine-3-carboxylic acid

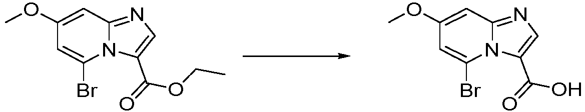

Ethyl 5-bromo-7-methoxyimidazo[1,2-*a*]pyridine-3-carboxylate (3.0 g, 10.0 mmol) was dissolved in THF (20 mL), and 2N LiOH (10 mL) was added thereto. The reaction mixture was stirred at room temperature overnight. Ethyl acetate (20 mL) was added for extraction. The aqueous phase was collected, and the pH value was adjusted to 3-4 by 1N HCl, then extracted with ethyl acetate (50 mL). The organic phase was collected, dried, and then evaporated to dryness to obtain 5-bromo-7-methoxyimidazo[1,2-*a*]pyridine-3-carboxylic acid (2.5 g, yield: 92%).

MS m/z (ESI): 270.8[M+H]⁺.

### Step 3: preparation of 5-bromo-7-methoxyimidazo[1,2-a]pyridine-3-carboxamide

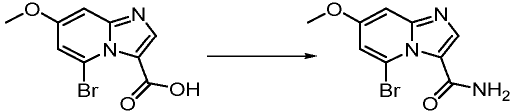

5-Bromo-7-methoxyimidazo[1,2-*a*]pyridine-3-carboxylic acid (2.5 g, 9.2 mmol) was dissolved in DMF (30 mL), and NH₄Cl (0.99 g, 18.5 mmol), HATU (5.3 g, 13.8 mmol) and DIEA (3.6 g, 27.7 mmol) were added thereto. The reaction mixture was stirred at room temperature overnight. Water (50 mL) was added thereto, and then the reaction mixture was extracted with ethyl acetate (100 mL). The organic phase was washed with saturated sodium chloride and dried, then evaporated to dryness. The crude product was purified by column chromatography to obtain 5-bromo-7-methoxyimidazo[1,2-*a*]pyridine-3-carboxamide (2 g, yield: 80%).

MS m/z (ESI): 269.8[M+H]⁺.

### Step 4: preparation of 5-bromo-7-methoxyimidazo[1,2-a]pyridine-3-carbonitrile

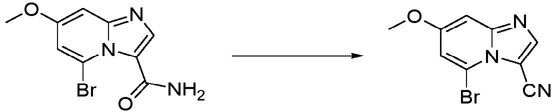

5-Bromo-7-methoxyimidazo[1,2-*a*]pyridine-3-carboxamide (2 g, 7.4 mmol) was dissolved in anhydrous tetrahydrofuran (30 mL), and pyridine (1.46 g, 18.5 mmol) was added thereto. TFAA(3.9 g, 18.5 mmol) was slowly added dropwise. After the addition was completed, the reaction was stirred at room temperature for 3 hours. Water (50 mL) was added thereto, and then the mixture was extracted with ethyl acetate (100 mL). The organic phase was dried and evaporated to dryness. The crude product was purified by column chromatography to obtain 5-bromo-7-methoxyimidazo[1,2-*a*]pyridine-3-carbonitrile (1.5 g, yield: 80%).

MS m/z (ESI): 251.8[M+H]⁺.

### Step 5: preparation of 5-bromo-7-hydroxyimidazo[1,2-a]pyridine-3-carbonitrile

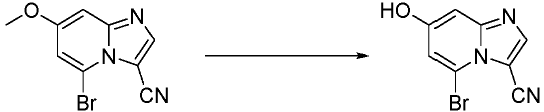

5-Bromo-7-methoxyimidazo[1,2-*a*]pyridine-3-carbonitrile (1.5 g, 6.0 mmol) was dissolved in DCE (20 mL). AlCl₃ (2.4 g, 17.9 mmol) was slowly added to the reaction mixture. After the addition was completed, the reaction was refluxed overnight under the protection of nitrogen. Tetrahydrofuran (100 mL) was added to the reaction mixture, and then an excess of sodium sulfate decahydrate (20 g) was added, and the mixture was stirred at room temperature overnight. The mixture was filtered, and the filtrate was evaporated to dryness. The crude product was subjected to column chromatography to obtain 5-bromo-7-hydroxyimidazo[1,2-*a*]pyridine-3-carbonitrile (1.2 g, yield: 85%).

MS m/z (ESI): 237.8[M+H]⁺.

### Step 6: preparation of 5-bromo-7-(2-hydroxy-2-methylpropoxy)imidazo[1,2-a]pyridine-3-carbonitrile

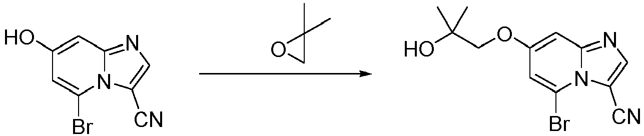

5-Bromo-7-hydroxyimidazo[1,2-*a*]pyridine-3-carbonitrile (1.2 g, 5.0 mmol) was dissolved in DMF (20 mL), and 2,2-dimethyloxirane (3.6 g, 50.4 mmol) and potassium carbonate (2.1 g, 15.1 mmol) were added thereto. The reaction mixture was stirred at 85 °C overnight. Water (30 mL) was added thereto, and then the reaction mixture was extracted with ethyl acetate (50 mL). The organic phase was washed with saturated sodium chloride aqueous solution, then dried and evaporated to dryness. The crude product was purified by column chromatography to obtain 5-bromo-7-(2-hydroxy-2-methylpropoxy)imidazo[1,2-*a*]pyridine-3-carbonitrile (1.3 g, yield: 83%).

MS m/z (ESI): 310.2[M+H]⁺.

### Step 7: preparation of 7-(2-hydroxy-2-methylpropoxy)-5-(6-(6-((6-methoxypyridin-3-yl)methyl)-3,6-diazabicyclo[3.1.1]heptan-3-yl)pyridin-3-yl)imidazo[1,2-a]pyridine-3-carbonitrile

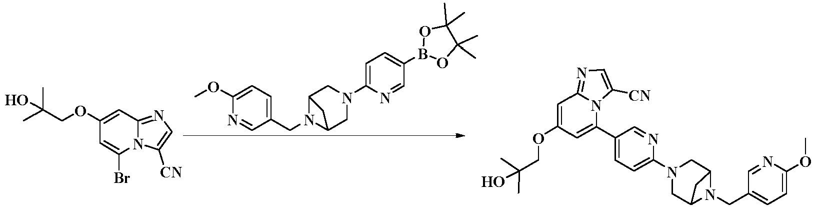

7-(2-Hydroxy-2-methylpropoxy)-5-(6-(6-((6-methoxypyridin-3-yl)methyl)-3,6-diazabicyclo[3.1.1]heptan-3-yl)pyridin-3-yl)imidazo[1,2-*a*]pyridine-3-carbonitrile (100 g, yield: 55%) was obtained by using 5-bromo-7-(2-hydroxy-2-methylpropoxy)imidazo[1,2-a]pyridine-3-carbonitrile and 6-((6-methoxypyridin-3-yl)methyl)-3-(5-(4,4,5,5-tetramethyl-1,3,2-dioxaborolan-2-yl)pyridin-2-yl)-3,6-diazabicyclo[3.1.1]heptane as raw materials with reference to step 3 of embodiment 37.

MS m/z (ESI): 526.2[M+H]⁺.

### [Not according to the invention] Embodiment 41

### 7-(2-Hydroxy-2-methylpropoxy)-5-(6-(4-(pyridin-2-oxy)piperidin-1-yl)pyridin-3-yl)imidazo[1,2-a]pyridine-3-carbonitrile

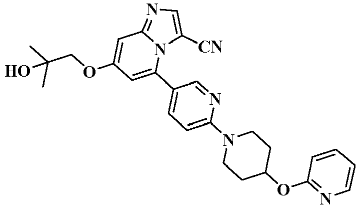

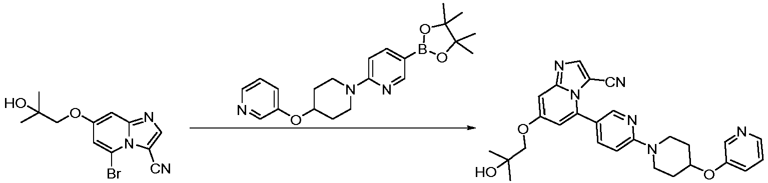

7-(2-Hydroxy-2-methylpropoxy)-5-(6-(4-(pyridin-2-oxy)piperidin-1-yl)pyridin-3-yl)imidazo[1,2-a]pyridine-3-carbonitrile (85 g, yield: 65%) was obtained by using 5-bromo-7-(2-hydroxy-2-methylpropoxy)imidazo[1,2-*a*]pyridine-3-carbonitrile and 2-(4-(pyridin-3-oxy)piperidin-1-yl)-5-(4,4,5,5-tetramethyl-1,3,2-dioxaborolan-2-yl)pyridine as raw material with reference to step 3 of embodiment 37.

MS m/z (ESI): 485.2[M+H]⁺.

### [Not according to the invention] Embodiment 42

### N-(1-(5-(3-cyano-7-(2-hydroxy-2-methylpropoxy)imidazo[1,2-a]pyridin-5-yl)pyridin-2-yl)-4-methylpiperidin-4-yl)-2,6-difluorobenzamide

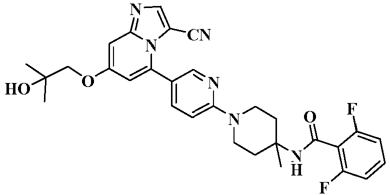

### Step 1: preparation of benzyl 4-((tert-butylsulfinyl<sulfinyl>)amino)-4-methylpiperidine-1-carboxylate

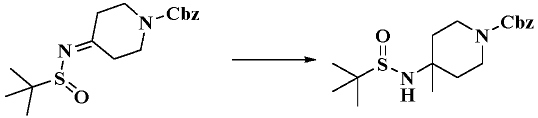

Benzyl 4-((*tert*-butylsulfinyl<sulfinyl>)imino)piperidine-1-carboxylate (5 g, 14.9 mmol) was dissolved in anhydrous tetrahydrofuran (50 mL), and the mixture was cooled to 0 °C. Methylmagnesium bromide (17.9 mL, 17.9 mmol) was added slowly dropwise to the reaction mixture. After the addition was completed, the reaction was slowly warmed to room temperature, and stirring was continued for 3 hours. Ammonium chloride aqueous solution (10 mL) was slowly added dropwise to quench the reaction, and then the mixture was extracted with ethyl acetate (100 mL). The organic phase was dried and evaporated to dryness. The crude product was purified by column chromatography to obtain benzyl 4-((*tert*-butyl sulfinyl<sulfinyl>)amino)-4-methylpiperidine-1-carboxylate (4.2 g, yield: 80%).

MS m/z (ESI): 353.2[M+H]⁺.

### Step 2: preparation of benzyl 4-amino-4-methylpiperidine-1-carboxylate

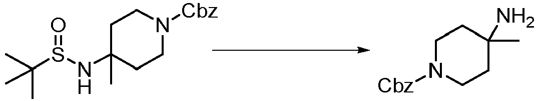

Benzyl 4-((*tert*-butylsulfinyl<sulfinyl>)amino)-4-methylpiperidine-1-carboxylate (4.2 g, 11.9 mmol) was dissolved in 25% trifluoroacetic acid/dichloromethane solution (50 mL). The reaction mixture was stirred at room temperature for 2 hours. The reaction mixture was evaporated to dryness, and dichloromethane (100 mL) was added thereto. NaHCO₃ aqueous solution was added slowly to adjust the pH value to 7-8. The organic phase was dried and then evaporated to dryness to obtain benzyl 4-amino-4-methylpiperidine-1-carboxylate (2.8 g, yield: 95%).

MS m/z (ESI): 249.2[M+H]⁺.

### Step 3: preparation of benzyl 4-(2,6-difluorobenzamido)-4-methylpiperidine-1-carboxylate

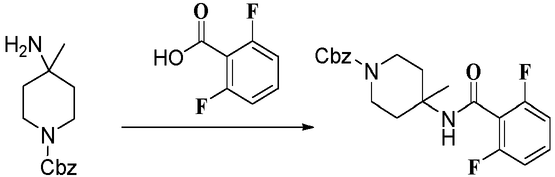

Benzyl 4-(2,6-difluorobenzamido)-4-methylpiperidine-1-carboxylate (1.5 g, yield: 84%) was obtained by using benzyl 4-amino-4-methylpiperidine-1-carboxylate and 2,6-difluorobenzoic acid as raw materials with reference to step 3 of example 40.

MS m/z (ESI): 389.2[M+H]⁺.

### Step 4: preparation of 2,6-difluoro-N-(4-methylpiperidin-4-yl)benzamide

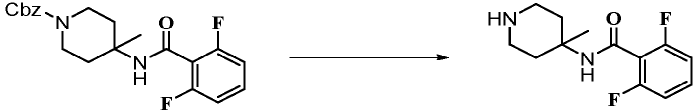

Methyl 4-(2,6-difluorobenzamido)-4-methylpiperidine-1-carboxylate (1.5 g, 3.9 mmol) was dissolved in methanol (50 mL), and palladium/carbon (200 mg) was added thereto. The reaction was stirred for 6 hours under hydrogen conditions. The reaction mixture was filtered. The filtrate was evaporated to dryness to obtain 2,6-difluoro-*N*-(4-methylpiperidin-4-yl)benzenamide (900 mg, yield: 92%).

MS m/z (ESI): 255.2[M+H]⁺.

### Step 5: preparation of 5-(6-fluoropyridin-3-yl)-7-(2-hydroxy-2-methylpropoxy)imidazo[1,2-a]pyridine-3-carbonitrile

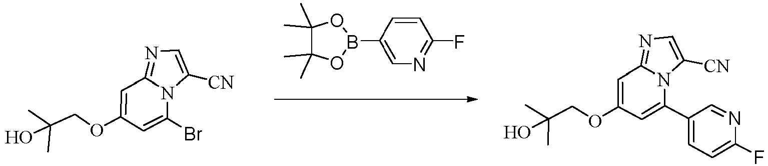

5-(6-Fluoropyridin-3-yl)-7-(2-hydroxy-2-methylpropoxy)imidazo[1,2-*a*]pyridine-3-carbonitrile (200 mg, yield was 72%) was obtained by using 5-bromo-7-(2-hydroxy-2-methylpropoxy)imidazo[1,2-*a*]pyridine-3-carbonitrile and 2-fluoro-5-(4,4,5,5-tetramethyl-1,3,2-dioxaborolan-2-yl)pyridine as raw materials with reference to step 3 of embodiment 37.

MS m/z (ESI): 327.2[M+H]⁺.

### Step 6: preparation of N-(1-(5-(3-cyano-7-(2-hydroxy-2-methylpropoxy)imidazo[1,2-a]pyridin-5-yl)pyridin-2-yl)-4-methylpiperidin-4-yl)-2,6-difluorobenzamide

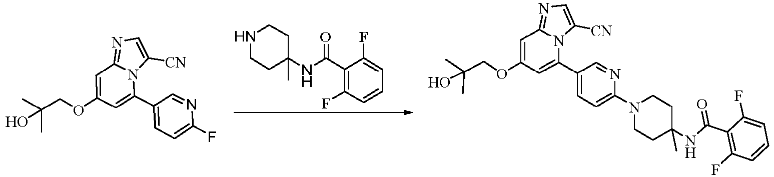

5-(6-Fluoropyridin-3-yl)-7-(2-hydroxy-2-methylpropoxy)imidazo[1,2-*a*]pyridine-3-carbonitrile (100 mg, 0.306 mmol) was dissolved in DMSO (10 mL), and 2,6-difluoro-*N*-(4-methylpiperidin-4-yl)benzamide (78 mg, 0.306 mmol) and DIEA (119 mg , 0.92 mmol) were added thereto. The reaction mixture was stirred at 90 °C for 2 days. Water (20 mL) was added thereto, and then the reaction mixture was extracted with ethyl acetate (30 mL). The organic phase was washed with saturated sodium chloride aqueous solution, then dried and evaporated to dryness. The crude product was purified by prep-HPLC to obtain *N-*(1-(5-(3-cyano-7-(2-hydroxy-2-methylpropoxy)imidazo[1,2-*a*]pyridin-5-yl)pyridin-2-yl)-4-methylpiperidin-4-yl)-2,6-difluorobenzamide (70 mg, yield: 41%).

MS m/z (ESI): 561.2[M+H]⁺.

### [Not according to the invention] Embodiment 43

### (R)-5-(6-(4-(3-hydroxy-3-phenylpropanoyl)piperazin-1-yl)pyridin-3-yl)-7-(1-methyl-1H-pyrazol-4-yl)imidazo[1,2-a]pyridine-3-carbonitrile

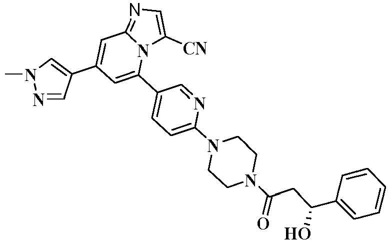

### Step 1: preparation of tert-butyl 4-(5-(3-cyano-7-hydroxyimidazo[1,2-a]pyridin-5-yl)pyridin-2-yl)piperazine-1-carboxylate

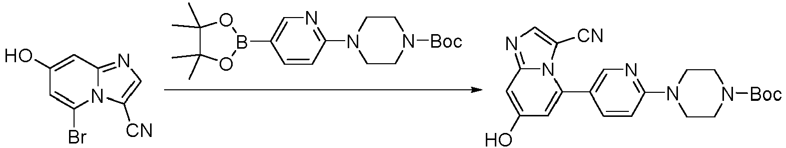

*Tert-butyl* 4-(5-(3-cyano-7-hydroxyimidazo[1,2-*a*]pyridin-5-yl)pyridin-2-yl)piperazine-1-carboxylate (1 g, yield: 72%) was obtained by using 5-bromo-7-hydroxyimidazo[1,2-*a*]pyridine-3-carbonitrile and *tert*-butyl 4-(5-(4,4,5,5-tetramethyl-1,3,2-dioxaborolan-2-yl)pyridin-2-yl)piperazine-1-carboxylate as raw materials with reference to step 3 of embodiment 37.

MS m/z (ESI): 421.2[M+H]⁺.

### Step 2: preparation of tert-butyl 4-(5-(3-cyano-7-(((trifluoromethyl)sulfonyl)oxy)imidazo[1,2-a]pyridin-5-yl)pyridin-2-yl)piperazine-1-carboxylate

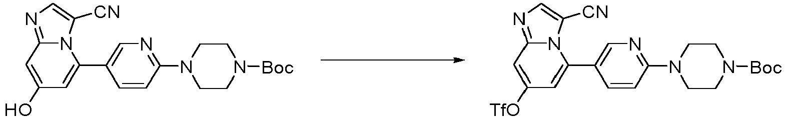

*Tert-butyl* 4-(5-(3-cyano-7-hydroxyimidazo[1,2-*a*]pyridin-5-yl)pyridin-2-yl)piperazine-1-carboxylate (1 g, 2.4 mmol) was dissolved in anhydrous dichloromethane (20 mL), and the mixture was cooled to 0 °C. DIEA (0.46 g, 3.6 mmol) was added to the reaction mixture. Trifluoromethanesulfonic anhydride (0.74 g, 2.6 mmol) was slowly added thereto. After the addition was completed, the reaction mixture was slowly warmed to room temperature, and stirring was continued for 3 hours. Water (20 mL) was added thereto, and then the reaction mixture was extracted with dichloromethane (20 mL). The organic phase was dried and evaporated to dryness to obtain *tert-butyl* 4-(5-(3-cyano-7-(((trifluoromethyl)sulfonyl)oxo)imidazo[1,2-*a*]pyridin-5-yl)pyridin-2-yl)piperazine-1-carboxylate (1.2 g, yield: 91%).

MS m/z (ESI): 553.2[M+H]⁺.

### Step 3: preparation of tert-butyl 4-(5-(3-cyano-7-(1-methyl-1H-pyrazol-4-yl)imidazo[1,2-a]pyridin-5-yl)pyridin-2-yl)piperazine-1-carboxylate

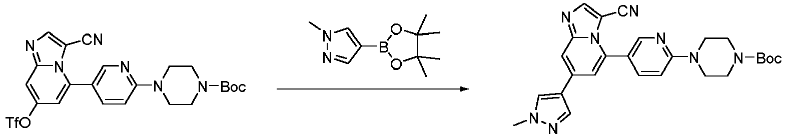

*Tert-butyl* 4-(5-(3-cyano-7-(1-methyl-1*H*-pyrazol-4-yl)imidazo[1,2-*a*]pyridin-5-yl)pyridin-2-yl)piperazine-1-carboxylate (0.8 g, yield: 76%) was obtained by using *tert-butyl* 4-(5-(3-cyano-7-(((trifluoromethyl)sulfonyl)oxo)imidazo[1,2-*a*]pyridin-5-yl)pyridin-2-yl)piperazine-1-carboxylate and 1-methyl-4-(4,4,5,5-tetramethyl-1,3,2-dioxaborolan-2-yl)-1*H*-pyrazole as raw material with reference to step 3 of embodiment 37.

MS m/z (ESI): 485.2[M+H]⁺.

### Step 4: preparation of 7-(1-methyl-1H-pyrazol-4-yl)-5-(6-(piperazin-1-yl)pyridin-3-yl)imidazo[1,2-a]pyridine-3-carbonitrile

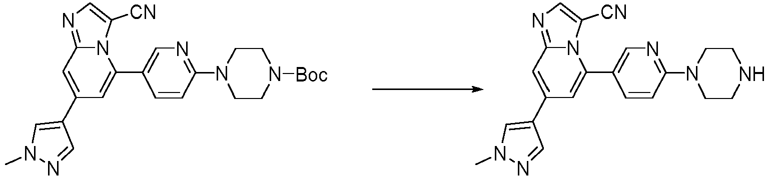

*Tert-butyl* 4-(5-(3-cyano-7-(1-methyl-1*H*-pyrazol-4-yl)imidazo[1,2-*a*]pyridin-5-yl)pyridin-2-yl)piperazine-1-carboxylate (0.8 g, 1.7 mmol) was dissolved in 25% trifluoroacetic acid/dichloromethane solution (20 mL). The reaction mixture was stirred at room temperature for 2 hours. The reaction mixture was evaporated to dryness and dichloromethane (30 mL) was added thereto. NaHCO₃ aqueous solution was added slowly to adjust the pH value to 7-8. The organic phase was dried and evaporated to dryness to obtain 7-(1-methyl-1*H*-pyrazol-4-yl)-5-(6-(piperazin-1-yl)pyridin-3-yl)imidazo[1,2-*a*]pyridine-3-carbonitrile (0.6 g, yield: 95%).

MS m/z (ESI): 385.2[M+H]⁺.

### Step 5: preparation of (R)-5-(6-(4-(3-hydroxy-3-phenylpropanoyl)piperazin-1-yl)pyridin-3-yl)-7-(1-methyl-1H-pyrazol-4-yl)imidazo[1,2-a]pyridine-3-carbonitrile

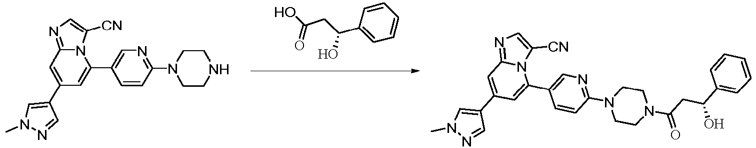

(*R*)-5-(6-(4-(3-hydroxy-3-phenylpropanoyl)piperazin-1-yl)pyridin-3-yl)-7-(1-methyl-1H-pyrazol-4-yl)imidazo[1,2-*a*]pyridine-3-carbonitrile (70 mg, yield: 62%) was obtained by using 7-(1-methyl-1*H-*pyrazol-4-yl)-5-(6-(piperazin-1-yl)pyridin-3-yl)imidazo[1,2-*a*]pyridine-3-carbonitrile and (*R*)-3-hydroxy-3-phenylpropanoic acid as raw materials with reference to step 3 of embodiment 40.

MS m/z (ESI): 533.2[M+H]⁺.

### [Not according to the invention] Embodiment 44

### (R)-5-(6-(4-(2-hydroxy-3-phenylpropanoyl)piperazin-1-yl)pyridin-3-yl)-7-(1-methyl-1H-pyrazol-4-yl)imidazo[1,2-a]pyridine-3-carbonitrile

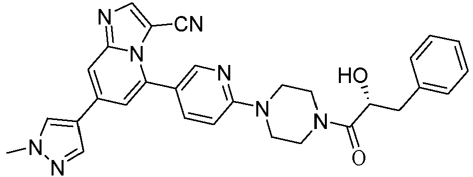

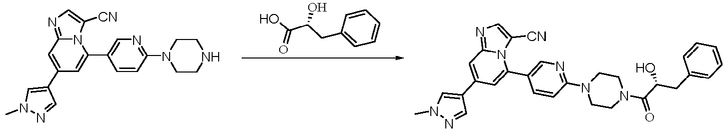

(*R*)-5-(6-(4-(2-hydroxy-3-phenylpropanoyl)piperazin-1-yl)pyridin-3-yl)-7-(1-methyl-1*H*-pyrazol-4-yl)imidazo[1,2-*a*]pyridine-3-carbonitrile (65 mg, yield: 66%) was obtained by using 7-(1-methyl-1*H-*pyrazol-4-yl)-5-(6-(piperazin-1-yl)pyridin-3-yl)imidazo[1,2-*a*]pyridine-3-carbonitrile and (*R*)-2-hydroxy-3-phenylpropanoic acid as raw materials with reference to step 3 of embodiment 40.

MS m/z (ESI): 533.2[M+H]⁺.

### [Not according to the invention] Embodiment 45

### 4-(5-(3-Cyano-7-(1-methyl-1H-pyrazol-4-yl)imidazo[1,2-a]pyridin-5-yl)pyridin-2-yl)-N-isobutylpiperazine-1-carboxamide

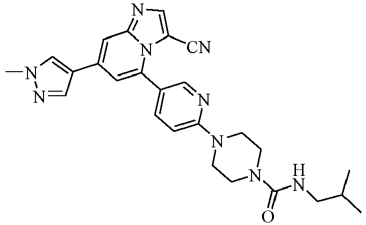

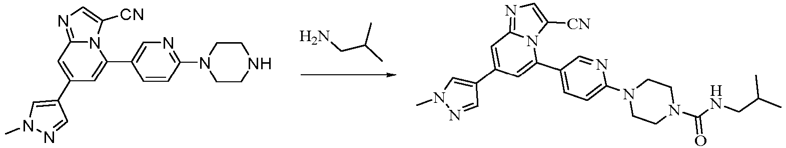

7-(1-Methyl-1*H*-pyrazol-4-yl)-5-(6-(piperazin-1-yl)pyridin-3-yl)imidazo[1,2-*a*]pyridine-3-carbonitrile (0.1 g, 0.26 mmol) was dissolved in dichloromethane (5 mL), and DIEA (168 mg, 1.3 mmol) and CDI (84 mg, 0.52 mmol) were added thereto. The reaction mixture was stirred at room temperature for 2 hours, and then 2-methylpropan-1-amine (38 mg, 0.52 mmol) was added thereto. The reaction mixture was stirred for 2 days. Water (10 mL) was added thereto, and then the reaction mixture was extracted with dichloromethane (20 mL). The organic phase was dried and evaporated to dryness. The crude product was purified by prep-HPLC to obtain 4-(5-(3-cyano-7-(1-methyl-1*H*-pyrazol-4-yl)imidazo[1,2-*a*]pyridin-5-yl)pyridin-2-yl)-*N*-isobutylpiperazine-1-carboxamide (30 mg, yield: 24%).

MS m/z (ESI): 484.2[M+H]⁺.

### [Not according to the invention] Embodiment 46

### 6-((R)-2-hydroxypropoxy)-4-(6-(6-((6-methoxypyridin-3-yl)methyl)-3,6-diazabicyclo[3.1.1]heptan-3-yl)pyridin-3-yl)-1-methyl-1H-indazole-3-carbonitrile

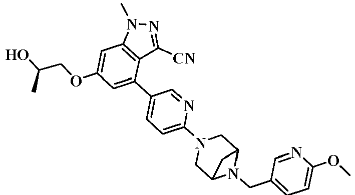

### Step 1: 2-bromo-6-fluoro-4-methoxybenzaldehyde

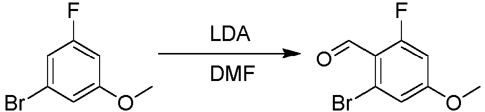

Lithium diisopropylamine (73.17 mmol, 36.6 mL, 2 M tetrahydrofuran) was added dropwise to a solution of 1-bromo-3-fluoro-5-methoxybenzene (10 g, 48.78 mmol) in tetrahydrofuran (100 mL) at -78 °C, then the mixture was stirred at -78 °C for 1 hour, then anhydrous *N,N*-dimethylformamide (7.12 g, 97.56 mmol ) was added dropwise, the mixture was stirred at -78 °C for 2 hours, quenched with saturated ammonium chloride solution, extracted with dichloromethane; the organic phase was washed with saturated saline, dried over anhydrous sodium sulfate, filtered, and the filtrate was concentrated under reduced pressure to dryness to obtain crude product 2-bromo-6-fluoro-4-methoxybenz(methyl)aldehyde (6.2 g, yield: 55%).

MS m/z (ESI): 232.9 [M+H]⁺.

### Step 2: 4-bromo-6-methoxy-1H-indazole

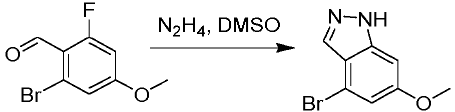

A mixed solution of 2-bromo-6-fluoro-4-methoxybenzene(methyl)aldehyde (6.2 g, 26.84 mmol), hydrazine hydrate (2.7 g, 53.68 mmol, 98%) and dimethyl sulfoxide (50 mL) was stirred at 130 °C for 2 hours, the mixture was cooled to room temperature and slurried with water, a solid precipitated and was filtered; the filter cake was washed with water, and dried to obtain light yellow solid 4-bromo-6-methoxy-1H-indazole (5.3 g, yield: 87%).

MS m/z (ESI): 227.0 [M+H]⁺.

### Step 3: 4-bromo-6-methoxy-1-methyl-1H-indazole

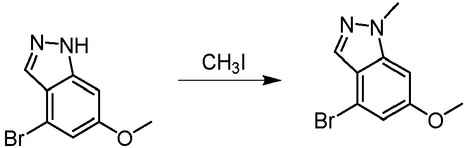

Sodium hydrogen (1.8 g, 46.9 mmol) was added to a solution of 4-bromo-6-methoxy-1*H*-indazole (5.3 g, 23.45 mmol) in tetrahydrofuran (50 mL) at 0 °C, then the mixture was stirred for 30 min, and iodomethane (5 g, 35.17 mmol) was added dropwise, then stirred at room temperature for 2 hours, quenched with water and extracted with dichloromethane; the organic phase was washed with saturated saline, dried over anhydrous sodium sulfate, filtered, and the filtrate was concentrated under reduced pressure to dryness and separated by column chromatography (dichloromethane/methanol=10:1) to obtain colorless oil 4-bromo-6-methoxy-1-methyl-1*H*-indazole (2.8 g, yield: 50%).

MS m/z (ESI): 241.0 [M+H]⁺.

### Step 4: 4-bromo-3-iodo-6-methoxy-1-methyl-1H-indazole

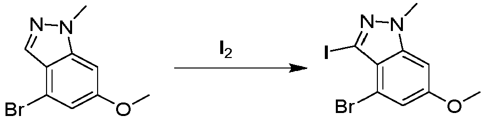

Iodine monomer (4.4 g, 17.5 mmol) was added to a mixed solution of 4-bromo-6-methoxy-1-methyl-1*H*-indazole (2.8 g, 11.67 mmol), potassium hydroxide (1.3 g, 23.34 mmol) and *N,N*-dimethylformamide (30 mL) , then the mixture was stirred at room temperature for 3 hours; after the reaction was completed, saturated sodium bisulfite solution was added thereto, a solid precipitated and was filtered, the filter cake was washed with water and dried to obtain light yellow solid 4-bromo-3-iodo-6-methoxy-1-methyl-1*H*-indazole (3.1 g, yield: 73%).

MS m/z (ESI): 366.8 [M+H]⁺.

### Step 5: 4-bromo-6-methoxy-1-methyl-1H-indazole-3-carbonitrile

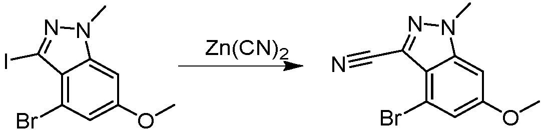

Tris(dibenzylideneacetone)dipalladium (388 mg, 0.42 mmol) was added to a mixture of 4-bromo-3-iodo-6-methoxy-1-methyl-1*H-*indazole (3.1 g, 8.47 mmol), zinc cyanide (2 g, 16.94 mmol) and anhydrous N,N-dimethylformamide (50 mL), then the mixture was replaced with nitrogen three times, and stirred at 100°C for 16 hours under the protection of nitrogen, after the reaction was completed, the mixture was cooled to room temperature, filtered, water and dichloromethane was added to the filtrate, the mixture was then extracted with dichloromethane; the organic phase was washed with saturated saline, dried over anhydrous sodium sulfate, filtered, the filtrate was concentrated under reduced pressure to dryness to obtain black solid 4-bromo-6-methoxy-1-methyl-1*H*-indazole-3-carbonitrile (3.5 g, crude product), the crude product was directly for the next step.

MS m/z (ESI): 266.0 [M+H]⁺.

### Step 5: 4-bromo-6-hydroxy-1-methyl-1H-indazole-3-carbonitrile

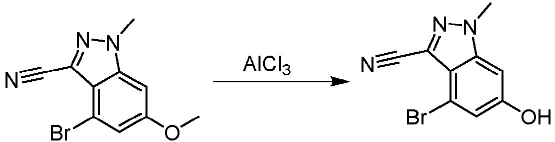

Aluminum trichloride (8.8 g, 66.05 mmol) was added to a solution of 4-bromo-6-methoxy-1-methyl-1*H*-indazole-3-carbonitrile (3.5 g, 13.21 mmol) in 1.2-dichloroethane (40 mL) in batches, then the mixture was stirred at 80 °C for 2 hours, cooled to room temperature, quenched with sodium sulfate decahydrate and filtered, the filter cake was washed with dichloromethane, the filtrate was washed with saturated saline; the organic phase was dried over anhydrous sodium sulfate, filtered, and the filtrate was concentrated under reduced pressure to dryness to obtain brown solid 4-bromo-6-hydroxy-1-methyl-1*H*-indazole-3-carbonitrile (3.2 g, crude product).

MS m/z (ESI): 250.9 [M-H]⁻.

### Step 7: 4-bromo-6-(2-hydroxypropoxy)-1-methyl-1H-indazole-3-carbonitrile

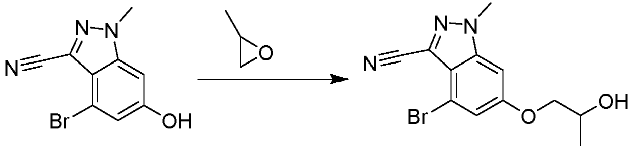

2-Methyloxirane (835 mg, 14.16 mmol) was added to a solution of 4-bromo-6-hydroxy-1-methyl-1*H*-indazole-3-carbonitrile (1.5 g, 7.08 mmol), potassium carbonate (2.93 g, 21.23 mmol) and acetonitrile (15 mL), and then the reaction mixture was stirred at 80 °C for 16 hours; the reaction mixture was concentrated under reduced pressure to dryness, separated by column chromatography (dichloromethane/methanol = 10:1) to obtain colorless oil 4-bromo-6-(2-hydroxypropoxy)-1-methyl-1*H*-indazole-3-carbonitrile (900 mg, yield: 41%).

MS m/z (ESI): 310.0 [M+H]⁺.

### Step 8: (R)-4-(6-fluoropyridin-3-yl)-6-(2-hydroxypropoxy)-1-methyl-1H-indazole-3-carbonitrile

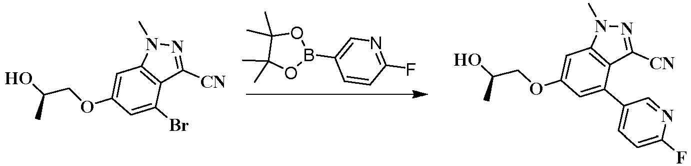

1,1'-Bis(diphenylphosphino)ferrocene-palladium(II)dichloride dichloromethane complex (120 mg, 0.15 mmol) was added to a mixed solution of 4-bromo-6-(2-hydroxypropoxy)-1-methyl-1*H*-indazole-3-carbonitrile (900 mg, 2.91 mmol), 2-fluoro-5-(4,4,5,5-tetramethyl-1,3,2-dioxaborolan-2-yl)pyridine (778 mg, 3.49 mmol), potassium acetate (570 mg, 5.82 mmol) and dioxane (10 mL), the mixture was replaced with nitrogen three times and then stirred at 100 °C under the protection of nitrogen for 16 hours; the reaction was cooled and filtered, the filtrate was concentrated under reduced pressure to dryness and separated by column chromatography (dichloromethane/methanol = 10:1) to obtain colorless oil (*R*)-4-(6-fluoropyridin-3-yl)-6-(2-hydroxypropoxy)-1-methyl-1*H*-indazole-3-carbonitrile (650 mg, yield: 69%).

MS m/z (ESI): 327.1 [M+H]⁺.

### Step 9: ethyl 3-(5-(3-cyano-6-((R)-2-hydroxypropoxy)-1-methyl-1H-indazol-4-yl)pyridin-2-yl)-3,6-diazabicyclo[3.1.1]heptane-6-carboxylate

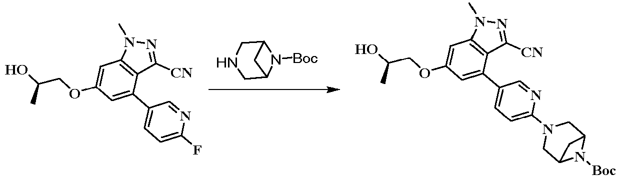

A mixed solution of (*R*)-4-(6-fluoropyridin-3-yl)-6-(2-hydroxypropoxy)-1-methyl-1*H*-indazole-3-carbonitrile (650 mg, 1.99 mmol), *tert-butyl* 3,6-diazabicyclo[3.1.1]heptane-6-carboxylate (788 mg, 3.98 mmol), *N,N*-diisopropylethylamine (770 mg, 5.97 mmol) and dimethyl sulfoxide (8 mL) was stirred at 100 °C for 24 hours; the reaction was quenched with water and extracted with ethyl acetate (50 mL*3), the combined organic phase was washed with saturated saline, the organic phase was dried over anhydrous sodium sulfate, filtered, and the filtrate was concentrated under reduced pressure to dryness and separated by column chromatography (dichloromethane/methanol = 10:1) to obtain colorless oil ethyl 3-(5-(3-cyano-6-((*R*)-2-hydroxypropoxy)-1-methyl-1*H*-indazol-4-yl)pyridin-2-yl)-3,6-diazabicyclo[3.1.1]heptane-6-carboxylate (410 mg, yield: 41%).

MS m/z (ESI): 505.2 [M+H]⁺.

### Step 10: 4-(6-(3,6-diazabicyclo[3.1.1]heptan-3-yl)pyridin-3-yl)-6-((R)-2-hydroxypropoxy)-1-methyl-1H-indazole-3-carbonitrile

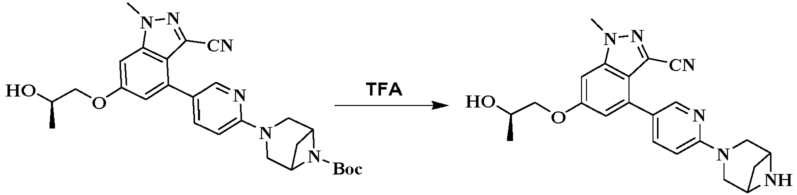

Ethyl 3-(5-(3-cyano-6-((*R*)-2-hydroxypropoxy)-1-methyl-1*H*-indazol-4-yl)pyridin-2-yl)-3,6-diazabicyclo[3.1.1]heptane-6-carboxylate (410 mg, 0.81 mmol) was dissolved in dichloromethane (6 mL), then trifluoroacetic acid (2 mL) was added at room temperature for 1 hour; after the reaction was completed, the mixture was concentrated under reduced pressure to dryness and used directly in the next step without purification to obtain light yellow oil 4-(6-(3,6-diazabicyclo[3.1.1]heptan-3-yl)pyridin-3-yl)-6-((*R*)-2-hydroxypropoxy)-1-methyl-1*H*-indazole-3-carbonitrile (520 mg, crude product).

MS m/z (ESI): 405.2 [M+H]⁺.

### Step 11: 6-((R)-2-hydroxypropoxy)-4-(6-(6-((6-methoxypyridin-3-yl)methyl)-3,6-diazabicyclo[3.1.1]heptan-3-yl)pyridin-3-yl)-1-methyl-1H-indazole-3-carbonitrile

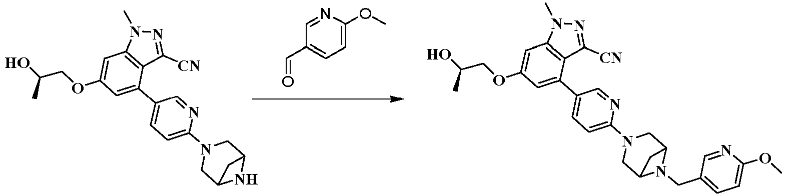

Sodium cyanoborohydride (47 mg, 0.75 mmol) was added to a solution of 4-(6-(3,6-diazabicyclo[3.1.1]heptan-3-yl)pyridin-3-yl)-6-((*R*)-2-hydroxypropoxy)-1-methyl-1H-indazole-3-carbonitrile (100 mg, 0.25 mmol), 6-methoxynicotinaldehyde (51 mg, 0.37 mmol) and 1,2-dichloroethane (3 mL), and then the mixture was stirred at room temperature for 24 hours; after the reaction was completed, the mixture was quenched with water and extracted with ethyl acetate (20 mL*3), and the combined organic phase was washed with saturated saline, dried over anhydrous sodium sulfate, filtered, and the filtrate was concentrated under reduced pressure to dryness and separated by preparative chromatography to obtain white solid 6-((*R*)-2-hydroxypropoxy)-4-(6-(6-((6-methoxypyridin-3-yl)methyl)-3,6-diazabicyclo[3.1.1]heptan-3-yl)pyridin-3-yl)-1-methyl-1*H*-indazole-3-carbonitrile (12 mg, yield: 9%).

MS m/z (ESI): 526.2 [M+H]⁺.

### [Not according to the invention] Embodiment 47

### 4-(6-(6-((5-Fluoro-6-methoxypyridin-3-yl)methyl)-3,6-diazabicyclo[3.1.1]heptan-3-yl)pyridin-3-yl)-6-(2-hydroxyethoxy)-1-methyl-1H-indazole-3-carbonitrile

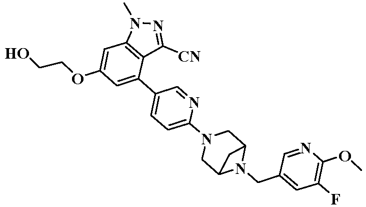

The title compound was obtained with reference to steps 1 to 8 of embodiment 46, wherein 2-methyloxirane in step 7 was replaced with ethylene oxide.

MS m/z (ESI): 530.2 [M+H]⁺.

### [Not according to the invention] Embodiment 48

### 6-(2-Hydroxy-2-methylpropoxy)-4-(6-(4-((6-methoxypyridin-3-yl)oxy)piperidin-1-yl)pyridin-3-yl)-1-methyl-1H-indazole-3-carbonitrile

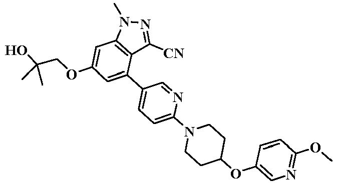

The title compound was obtained with reference to embodiment 46, wherein 2-methyloxirane in step 7 was replaced with 2,2-dimethyloxirane, and then with reference to embodiment 13.

MS m/z (ESI): 529.2 [M+H]⁺.

### [Not according to the invention] Embodiment 49

### 6-(2-Hydroxy-2-methylpropoxy)-4-(2-(6-((6-methoxypyridin-3-yl)methyl)-3,6-diazabicyclo[3.1.1]heptan-3-yl)thiazol-5-yl)pyrazolo[1,5-a]pyridine-3-carbonitrile

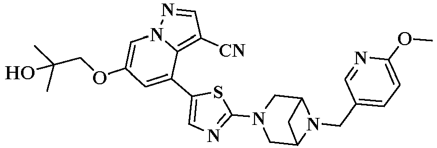

### Step 1: tert-butyl 3-(5-bromothiazol-2-yl)-3,6-diazabicyclo[3.1.1]heptane-6-carboxylate

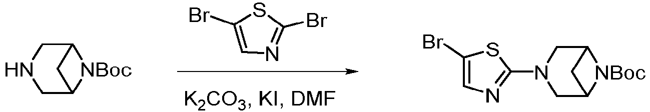

*Tert*-butyl 3,6-diazabicyclo[3.1.1]heptane-6-carboxylate (300 mg, 1.5 mmol) was dissolved in 20 mL of DMF; and 2,5- dibromothiazole (547 mg, 2.2 mmol), K₂CO₃ (621 mg, 4.5 mmol), KI (25 mg, 0.15 mmol) were added, and the reaction was carried out overnight at 90 °C under the protection of nitrogen. 10 mL of ammonium chloride aqueous solution was added, and the mixture was extracted with ethyl acetate (20mL*3). The organic phase was washed with saturated saline, dried over anhydrous sodium sulfate. The residue was filtered, evaporated to dryness; and the crude product was separated by column chromatography (eluted with dichloromethane/methanol = 10/1) to obtain *tert-butyl* 3-(5-bromothiazol-2-yl)-3,6-diazabicyclo[3.1.1]heptane-6-carboxylate (230 mg, white solid, the yield was 42%).

MS m/z (ESI): 360.0 [M+H]⁺.

### Step 2: 2-(3,6-diazabicyclo[3.1.1]heptan-3-yl)-5-bromothiazole

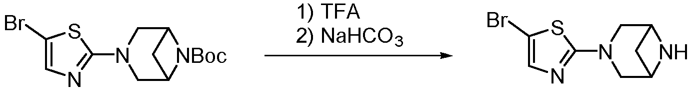

2-(3,6-diazabicyclo[3.1.1]heptan-3-yl)-5-bromothiazole (210 mg, white solid, 99%) was obtained by using *tert-butyl* 3-(5-bromothiazol-2-yl)-3,6-diazabicyclo[3.1.1]heptane-6-carboxylate as raw material with reference to step 5 of embodiment **1.**

MS m/z (ESI): 259.9 [M+H]⁺.

### Step 3: 5-bromo-2-(6-((6-methoxypyridin-3-yl)methyl)-3,6-diazabicyclo[3.1.1]heptan-3-yl)thiazole

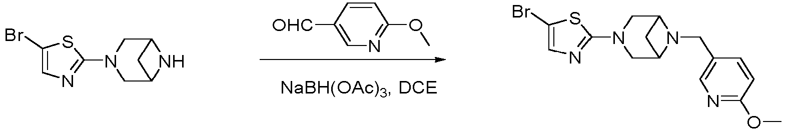

5-Bromo-2-(6-((6-methoxypyridin-3-yl)methyl)-3,6-diazabicyclo[3.1.1]heptan-3-yl)thiazole (100 mg, white solid, 52%) was obtained by using 2-(3,6-diazabicyclo[3.1.1]heptan-3-yl)-5-bromothiazole as raw material with reference to step 6 of embodiment 1.

MS m/z (ESI): 381.0 [M+H]⁺.

### Step 4: 6-(2-hydroxy-2-methylpropoxy)-4-(4,4,5,5-tetramethyl-1,3,2-dioxaborolan-2-yl)pyrazolo[1,5-a]pyridine-3-carbonitrile

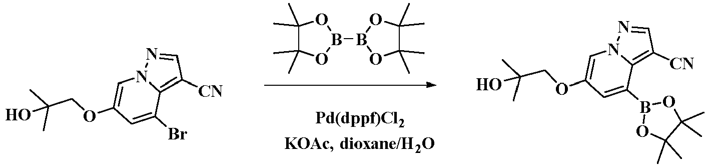

6-(2-Hydroxy-2-methylpropoxy)-4-(4,4,5,5-tetramethyl-1,3,2-dioxaborolan-2-yl)pyrazolo[1,5-a]pyridine-3-carbonitrile (130 mg, white solid, yield was 61%) was obtained by using 4-bromo-6-(2-hydroxy-2-methylpropoxy)pyrazolo[1,5-a]pyridine-3-carbonitrile as raw material with reference to step 7 of embodiment **1.**

MS m/z (ESI): 358.1 [M+H]⁺.

### Step 5: 6-(2-hydroxy-2-methylpropoxy)-4-(2-(6-((6-methoxypyridin-3-yl)methyl)-3,6-diazabicyclo[3.1.1]heptan-3-yl)thiazol-5-yl)pyrazolo[1,5-a]pyridine-3-carbonitrile

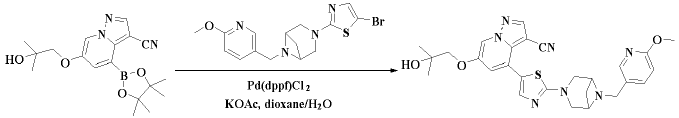

6-(2-Hydroxy-2-methylpropoxy)-4-(2-(6-((6-methoxypyridin-3-yl)methyl)-3,6-diazabicyclo[3.1.1]heptan-3-yl)thiazol-5-yl)pyrazolo[1,5-*a*]pyridine-3-carbonitrile (33 mg, white solid, 47%) was obtained by using 6-(2-hydroxy-2-methylpropoxy)-4-(4,4,5,5-tetramethyl-1,3,2-dioxaborolan-2-yl)pyrazolo[1,5-*a*]pyridine-3-carbonitrile and 5-bromo-2-(6-((6-methoxypyridin-3-yl)methyl)-3,6-diazabicyclo[3.1.1]heptan-3-yl)thiazole as raw materials with reference to step 8 of embodiment 1.

MS m/z (ESI): 532.2 [M+H]⁺.

### [Not according to the invention] Embodiment 50

### 4-(2-(6-((5-Fluoro-6-methoxypyridin-3-yl)methyl)-3,6-diazabicyclo[3.1.1]heptan-3-yl)thiazol-5-yl)-6-(2-hydroxy-2-methylpropoxy)pyrazolo[1,5-a]pyridine-3-carbonitrile

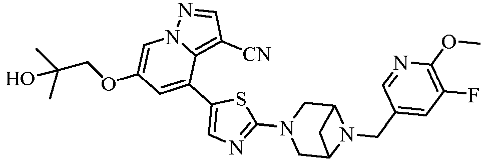

### Step 1: 5-bromo-2-(6-((5-fluoro-6-methoxypyridin-3-yl)methyl)-3,6-diazabicyclo[3.1.1]heptan-3-yl)thiazole

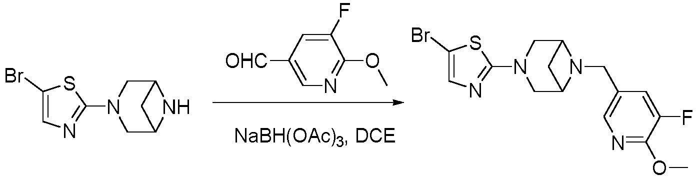

5-Bromo-2-(6-((5-fluoro-6-methoxypyridin-3-yl)methyl)-3,6-diazabicyclo[3.1.1]heptan-3-yl)thiazole (220 mg, white solid, 62%) was obtained by using 2-(3,6-diazabicyclo[3.1.1]heptan-3-yl)-5-bromothiazole and 5-fluoro-6-methoxynicotinaldehyde as raw material with reference to step 6 of embodiment 1.

MS m/z (ESI): 399.0 [M+H]⁺.

### Step 2: 4-(2-(6-((5-fluoro-6-methoxypyridin-3-yl)methyl)-3,6-diazabicyclo[3.1.1]heptan-3-yl)thiazol-5-yl)-6-(2-hydroxy-2-methylpropoxy)pyrazolo[1,5-a]pyridine-3-carbonitrile

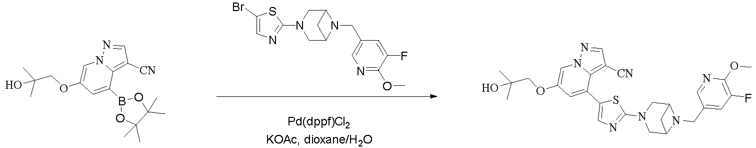

4-(2-(6-((5-Fluoro-6-methoxypyridin-3-yl)methyl)-3,6-diazabicyclo[3.1.1]heptan-3-yl)thiazol-5-yl)-6-(2-hydroxy-2-methylpropoxy)pyrazolo[1,5-*a*]pyridine-3-carbonitrile (35 mg, white solid, 48%) was obtained by using 6-(2-hydroxy-2-methylpropoxy)-4-(4,4,5,5-tetramethyl-1,3,2-dioxaborolan-2-yl)pyrazolo[1,5-*a*]pyridine-3-carbonitrile and 5-bromo-2-(6-((5-fluoro-6-methoxypyridin-3-yl)methyl)-3,6-diazabicyclo[3.1.1]heptan-3-yl)thiazole as raw materials with reference to step 8 of embodiment 1.

MS m/z (ESI): 550.2 [M+H]⁺.

### [Not according to the invention] Embodiment 51

### 3-(5-(3-Cyano-6-(2-hydroxy-2-methylpropoxy)pyrazolo[1,5-a]pyridin-4-yl)thiazol-2-yl)-N-phenyl-3,6-diazabicyclo[3.1.1]heptane-6-carboxamide

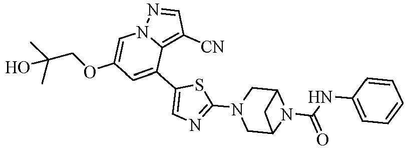

### Step 1: 3-(5-bromothiazol-2-yl)-N-phenyl-3,6-diazabicyclo[3.1.1]heptane-6-carboxamide

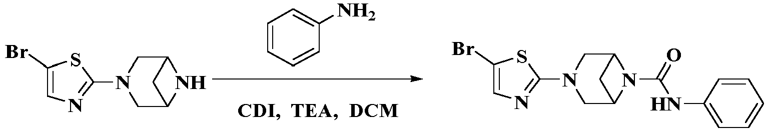

2-(3,6-Diazabicyclo[3.1.1]heptan-3-yl)-5-bromothiazole (300 mg, 1.2 mmol) was dissolved in 20 mL of DCM, and CDI (280 mg, 1.7 mmol) and TEA (245 mg, 2.4 mmol) were added, the reaction was carried out at room temperature for 2 hours. Aniline (223 mg, 2.4 mmol) was added thereto, and the reaction was carried out overnight at room temperature, 10 mL of ammonium chloride aqueous solution was added, and the mixture was extracted with ethyl acetate (20 mL*3). The organic phase was washed with saturated saline, dried over anhydrous sodium sulfate. The residue was filtered, evaporated to dryness; and the crude product was separated by column chromatography (eluted with dichloromethane/methanol = 10/1) to obtain 3-(5-bromothiazol-2-yl)-*N*-phenyl-3,6-diazabicyclo[3.1.1]heptane-6-carboxamide (223 mg, white solid, the yield was 49%).

MS m/z (ESI): 379.0 [M+H]⁺.

### Step 2: 3-(5-(3-cyano-6-(2-hydroxy-2-methylpropoxy)pyrazolo[1,5-a]pyridin-4-yl)thiazol-2-yl)-N-phenyl-3,6-diazabicyclo[3.1.1]heptane-6-carboxamide

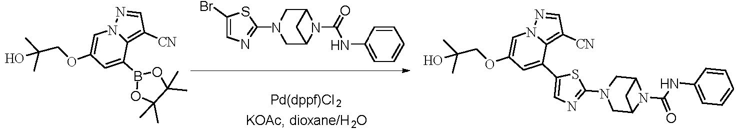

3-(5-(3-Cyano-6-(2-hydroxy-2-methylpropoxy)pyrazolo[1,5-*a*]pyridin-4-yl)thiazol-2-yl)-*N-*phenyl-3,6-diazabicyclo[3.1.1]heptane-6-carboxamide (29 mg, white solid, 39%) was obtained by using 6-(2-hydroxy-2-methylpropoxy)-4-(4,4,5,5-tetramethyl-1,3,2-dioxaborolan-2-yl)pyrazolo[1,5-*a*]pyridine-3-carbonitrile and 3-(5-bromothiazol-2-yl)-*N*-phenyl-3,6-diazabicyclo[3.1.1]heptane-6-carboxamide as raw materials with reference to step 8 of embodiment **1.**

MS m/z (ESI): 530.1 [M+H]⁺.

### [Not according to the invention] Embodiment 52

### 6-(2-Hydroxyethoxy)-4-(5-(6-((6-methoxypyridin-3-yl)methyl)-3,6-diazabicyclo[3.1.1]heptan-3-yl)-1,3,4-thiadiazol-2-yl)pyrazolo[1,5-a]pyridine-3-carbonitrile

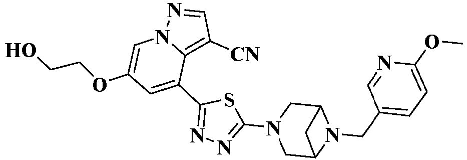

### Step 1: tert-butyl 3-(5-bromo-1,3,4-thiadiazol-2-yl)-3,6-diazabicyclo[3.1.1]heptane-6-carboxylate

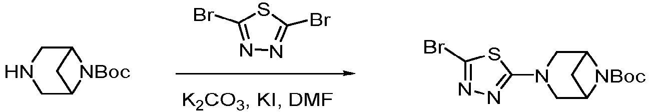

*Tert*-butyl 3-(5-bromo-1,3,4-thiadiazol-2-yl)-3,6-diazabicyclo[3.1.1]heptane-6-carboxylate (530 mg, white solid, 67%) was obtained by using 2,5-dibromo-1,3,4-thiadiazole as raw material with reference to step 1 of embodiment **49.**

MS m/z (ESI): 361.0 [M+H]⁺.

### Step 2: 2-(3,6-diazabicyclo[3.1.1]heptan-3-yl)-5-bromo-1,3,4-thiadiazole

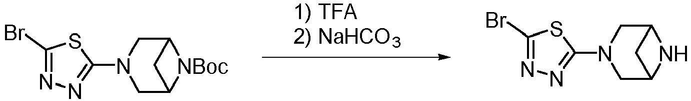

2-(3,6-Diazabicyclo[3.1.1]heptan-3-yl)-5-bromo-1,3,4-thiadiazole (350 mg, white solid, 99%) was obtained by using *tert-butyl* 3-(5-bromo-1,3,4-thiadiazol-2-yl)-3,6-diazabicyclo[3.1.1]heptane-6-carboxylate as raw material with reference to step 5 of embodiment **1.**

MS m/z (ESI): 260.9 [M+H]⁺.

### Step 3: 2-bromo-5-(6-((6-methoxypyridin-3-yl)methyl)-3,6-diazabicyclo[3.1.1]heptan-3-yl)-1,3,4-thiadiazole

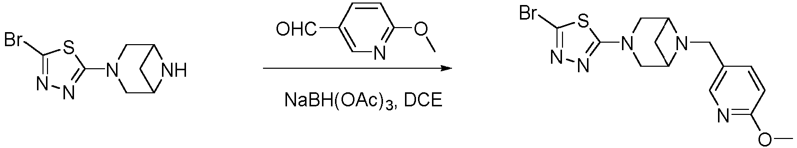

2-Bromo-5-(6-((6-methoxypyridin-3-yl)methyl)-3,6-diazabicyclo[3.1.1]heptan-3-yl)-1,3,4-thiadiazole (120 mg, white solid, 56%) was obtained by using 2-(3,6-diazabicyclo[3.1.1]heptan-3-yl)-5-bromo-1,3,4-thiadiazole as raw material with reference to step 6 of embodiment **1.**

MS m/z (ESI): 382.0 [M+H]⁺.

### Step 4: 6-(2-hydroxyethoxy)-4-(5-(6-((6-methoxypyridin-3-yl)methyl)-3,6-diazabicyclo[3.1.1]heptan-3-yl)-1,3,4-thiadiazol-2-yl)pyrazolo[1,5-a]pyridine-3-carbonitrile

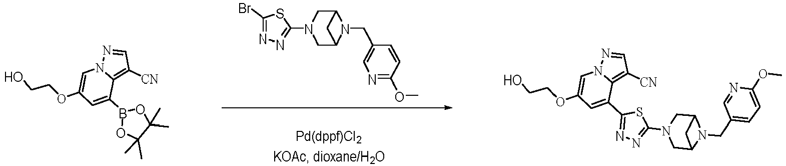

6-(2-Hydroxyethoxy)-4-(5-(6-((6-methoxypyridin-3-yl)methyl)-3,6-diazabicyclo[3.1.1]heptan-3-yl)-1,3,4-thiadiazol-2-yl)pyrazolo[1,5-*a*]pyridine-3-carbonitrile (25 mg, white solid, 38%) was obtained by using 6-(2-hydroxyethoxy)-4-(4,4,5,5-tetramethyl-1,3,2-dioxaborolan-2-yl)pyrazolo[1,5-*a*]pyridine-3-carbonitrile and 2-bromo-5-(6-((6-methoxypyridin-3-yl)methyl)-3,6-diazabicyclo[3.1.1]heptan-3-yl)-1,3,4-thiadiazole as raw materials with reference to step 8 of embodiment **1.**

MS m/z (ESI): 505.1 [M+H]⁺.

### [Not according to the invention] Embodiment 53

### 4-(5-(6-((5-Fluoro-6-methoxypyridin-3-yl)methyl)-3,6-diazabicyclo[3.1.1]heptan-3-yl)-1,3,4-thiadiazol-2-yl)-6-(2-hydroxyethoxy)pyrazolo[1,5-a]pyridine-3-carbonitrile

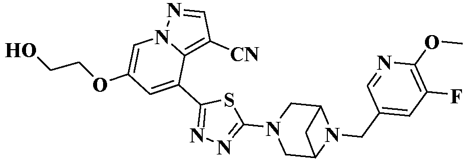

### Step 1: 2-bromo-5-(6-((5-fluoro-6-methoxypyridin-3-yl)methyl)-3,6-diazabicyclo[3.1.1]heptan-3-yl)-1,3,4-thiadiazole

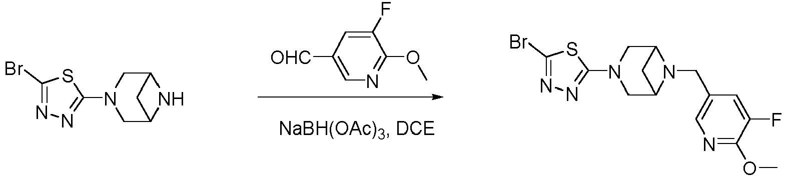

2-Bromo-5-(6-((5-fluoro-6-methoxypyridin-3-yl)methyl)-3,6-diazabicyclo[3.1.1]heptan-3-yl)-1,3,4-thiadiazole (150 mg, white solid, 59%) was obtained by using 2-(3,6-diazabicyclo[3.1.1]heptan-3-yl)-5-bromo-1,3,4-thiadiazole as raw material with reference to step 6 of embodiment **1.**

MS m/z (ESI): 400.0 [M+H]⁺.

### Step 2: 4-(5-(6-((5-fluoro-6-methoxypyridin-3-yl)methyl)-3,6-diazabicyclo[3.1.1]heptan-3-yl)-1,3,4-thiadiazol-2-yl)-6-(2-hydroxyethoxy)pyrazolo[1,5-a]pyridine-3-carbonitrile

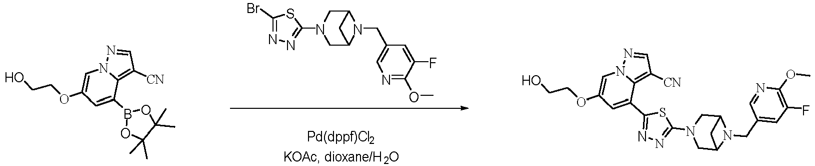

4-(5-(6-((5-Fluoro-6-methoxypyridin-3-yl)methyl)-3,6-diazabicyclo[3.1.1]heptan-3-yl)-1,3,4-thiadiazol-2-yl)-6-(2-hydroxyethoxy)pyrazolo[1,5-*a*]pyridine-3-carbonitrile (32 mg, white solid, 41%) was obtained by using 6-(2-hydroxyethoxy)-4-(4,4,5,5-tetramethyl-1,3,2-dioxaborolan-2-yl)pyrazolo[1,5-*a*]pyridine-3-carbonitrile and 2-bromo-5-(6-((5-fluoro-6-methoxypyridin-3-yl)methyl)-3,6-diazabicyclo[3.1.1]heptan-3-yl)-1,3,4-thiadiazole as raw materials with reference to step 8 of embodiment **1.**

MS m/z (ESI): 523.1 [M+H]⁺.

### [Not according to the invention] Embodiment 54

### 6-(2-Hydroxy-2-methylpropoxy)-4-(3-(6-((6-methoxypyridin-3-yl)methyl)-3,6-diazabicyclo[3.1.1]heptan-3-yl)-1H-pyrazol-1-yl)pyrazolo[1,5-a]pyridine-3-carbonitrile

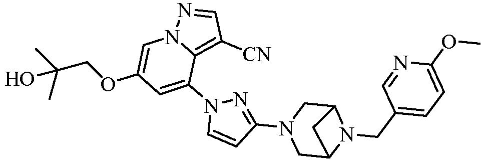

### Step 1: preparation of tert-butyl 3-(1-(4-methoxybenzyl)-1H-pyrazol-3-yl)-3,6-diazabicyclo[3.1.1]heptane-6-carboxylate

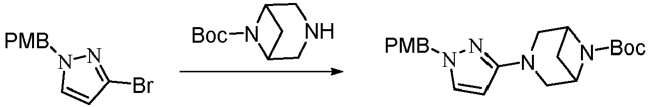

3-Bromo-1-(4-methoxybenzyl)-1*H*-pyrazole (5 g, 18.7 mmol) was dissolved in DMF (30 mL), *tert-*butyl 3,6-diazabicyclo[3.1.1]heptane-6-carboxylate (4.45 g, 22.5 mmol) and *tert-butanol* sodium (2.7 g, 28.1 mmol) were added thereto. Then Pd₂(dba)₃ (1.7 g, 1.87 mmol) and RuPhos (1.75 g, 3.74 mmol) were added thereto under the protection of nitrogen. The reaction was stirred at 90 °C overnight. Water (50 mL) was added to the reaction mixture, and then ethyl acetate (100 mL) was added thereto for extraction. The organic phase was dried and evaporated to dryness. The crude product was purified by column chromatography to obtain *tert-butyl* 3-(1-(4-methoxybenzyl)-1*H*-pyrazol-3-yl)-3,6-diazabicyclo[3.1.1]heptane-6-carboxylate (5 g, yield: 69%).

MS m/z (ESI): 385.2[M+H]⁺.

### Step 2: preparation of 3-(1-(4-methoxybenzyl)-1H-pyrazol-3-yl)-3,6-diazabicyclo[3.1.1]heptane

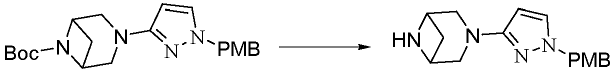

3-(1-(4-Methoxybenzyl)-1*H*-pyrazol-3-yl)-3,6-diazabicyclo[3.1.1]heptane was obtained by using *tert-butyl* 3-(1-(4-methoxybenzyl)-1*H*-pyrazol-3-yl)-3,6-diazabicyclo[3.1.1]heptane-6-carboxylate as raw material with reference to step 4 of embodiment 43.

MS m/z (ESI): 285.2[M+H]⁺.

### Step 3: 3-(1-(4-methoxybenzyl)-1H-pyrazol-3-yl)-6-((6-methoxypyridin-3-yl)methyl)-3,6-diazabicyclo[3.1.1]heptane

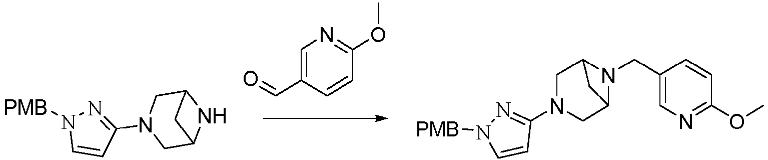

3-(1-(4-Methoxybenzyl)-1*H*-pyrazol-3-yl)-3,6-diazabicyclo[3.1.1]heptane (2 g, 7.0 mmol) was dissolved in DCE (30 mL), and 6-methoxynicotinaldehyde (1.9 g, 14.1 mmol) and sodium triacetoxyborohydride (4.5 g, 21.0 mmol) were added. The reaction mixture was stirred at room temperature overnight. Water (60 mL) was added thereto, and then the mixture was extracted with dichloromethane (50 mL). The organic phase was dried and evaporated to dryness. The crude product was purified by column chromatography to obtain 3-(1-(4-methoxybenzyl)-1*H*-pyrazol-3-yl)-6-((6-methoxypyridin-3-yl)methyl)-3,6-diazabicyclo[3.1.1]heptane (2.1 g, yield: 75%).

MS m/z (ESI): 406.2[M+H]⁺.

### Step 4: 6-((6-methoxypyridin-3-yl)methyl)-3-(1H-pyrazol-3-yl)-3,6-diazabicyclo[3.1.1]heptane

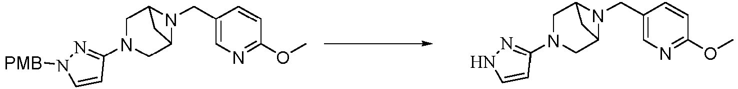

6-((6-Methoxypyridin-3-yl)methyl)-3-(1*H*-pyrazol-3-yl)-3,6-diazabicyclo[3.1.1]heptane (yield: 80%) was obtained by using 3-(1-(4-methoxybenzyl)-1*H*-pyrazol-3-yl)-6-((6-methoxypyridin-3-yl)methyl)-3,6-diazabicyclo[3.1.1]heptane as raw material with reference to step 4 of embodiment 42.

MS m/z (ESI): 286.2[M+H]⁺.

### Step 5: 6-(2-hydroxy-2-methylpropoxy)-4-(3-(6-((6-methoxypyridin-3-yl)methyl)-3,6-diazabicyclo[3.1.1]heptan-3-yl)-1H-pyrazol-1-yl)pyrazolo[1,5-a]pyridine-3-carbonitrile

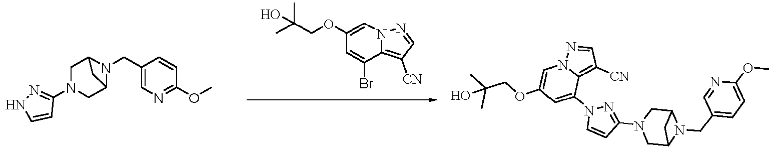

6-(2-Hydroxy-2-methylpropoxy)-4-(3-(6-((6-methoxypyridin-3-yl)methyl)-3,6-diazabicyclo[3.1.1]heptan-3-yl)-1*H*-pyrazol-1-yl)pyrazolo[1,5-*a*]pyridine-3-carbonitrile was obtained by using 6-((6-methoxypyridin-3-yl)methyl)-3-(1*H*-pyrazol-3-yl)-3,6-diazabicyclo[3.1.1]heptane and 4-bromo-6-(2-hydroxy-2-methylpropoxy)pyrazolo[1,5-*a*]pyridine-3-carbonitrile as raw material with reference to step 4 of embodiment 42.

MS m/z (ESI): 515.2[M+H]⁺.

### [Not according to the invention] Embodiment 55

### 4-(3-(6-((6-Ethoxy-5-fluoropyridin-3-yl)methyl)-3,6-diazabicyclo[3.1.1]heptan-3-yl)-1H-pyrazol-1-yl)-6-(2-hydroxy-2-methylpropoxy)pyrazolo[1,5-a]pyridine-3-carbonitrile

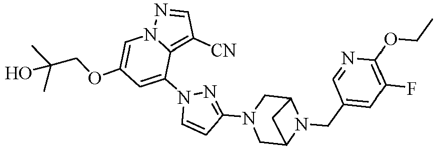

### Step 1: preparation of tert-butyl 3-(1-(4-methoxybenzyl)-1H-pyrazol-3-yl)-3,6-diazabicyclo[3.1.1]heptane-6-carboxylate

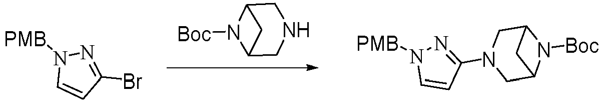

3-Bromo-1-(4-methoxybenzyl)-1*H*-pyrazole (5 g, 18.7 mmol) was dissolved in DMF (30 mL), and *tert*-butyl 3,6-diazabicyclo[3.1.1]heptane-6-carboxylate (4.45 g, 22.5 mmol) and *tert*-butanol sodium (2.7 g, 28.1 mmol) were added thereto. Pd₂(dba)₃ (1.7 g, 1.87 mmol) and RuPhos(1.75 g, 3.74 mmol) were added thereto under the protection of nitrogen. The reaction was stirred at 90 °C overnight. Water (50 mL) was added thereto, and then the mixture was extracted with ethyl acetate (100 mL). The organic phase was dried and evaporated to dryness. The crude product was purified by column chromatography to obtain *tert-butyl* 3-(1-(4-methoxybenzyl)-1*H*-pyrazol-3-yl)-3,6-diazabicyclo[3.1.1]heptane-6-carboxylate (5 g, yield: 69%).

MS m/z (ESI): 385.2[M+H]⁺.

### Step 2: preparation of 3-(1-(4-methoxybenzyl)-1H-pyrazol-3-yl)-3,6-diazabicyclo[3.1.1]heptane

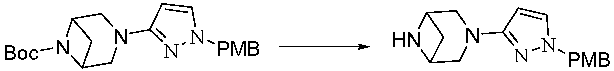

3-(1-(4-Methoxybenzyl)-1*H*-pyrazol-3-yl)-3,6-diazabicyclo[3.1.1]heptane was obtained by using *tert-butyl* 3-(1-(4-methoxybenzyl)-1*H*-pyrazol-3-yl)-3,6-diazabicyclo[3.1.1]heptane-6-carboxylate as raw material with reference to step 4 of embodiment 43 (3.5 g, yield: 95%).

MS m/z (ESI): 285.2[M+H]⁺.

### Step 3: preparation of 6-((6-ethoxy-5-fluoropyridin-3-yl)methyl)-3-(1-(4-methoxybenzyl)-1H-pyrazol-3-yl)-3,6-diazabicyclo[3.1.1]heptane

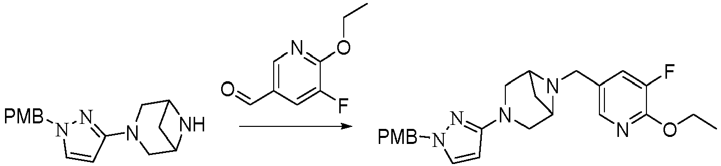

3-(1-(4-Methoxybenzyl)-1*H*-pyrazol-3-yl)-3,6-diazabicyclo[3.1.1]heptane (2 g, 7.0 mmol) was dissolved in DCE (30 mL), and 6-ethoxy-5-fluoronicotinaldehyde (2.4 g, 14.1 mmol) and sodium triacetoxyborohydride (4.5 g, 21.0 mmol) were added thereto. The reaction mixture was stirred at room temperature overnight. Water (60 mL) was added thereto and then the mixture was extracted with dichloromethane (50 mL). The organic phase was dried and evaporated to dryness. The crude product was purified by column chromatography to obtain 6-((6-ethoxy-5-fluoropyridin-3-yl)methyl)-3-(1-(4-methoxybenzyl)-1*H*-pyrazol-3-yl)-3,6-diazabicyclo[3.1.1]heptane (2.5 g, yield: 81%).

MS m/z (ESI): 438.2[M+H]⁺.

### Step 4: preparation of 6-((6-ethoxy-5-fluoropyridin-3-yl)methyl)-3-(1H-pyrazol-3-yl)-3,6-diazabicyclo[3.1.1]heptane

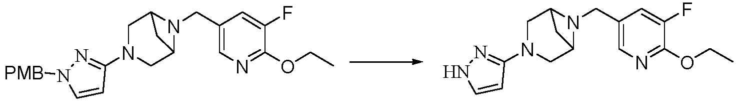

6-((6-Ethoxy-5-fluoropyridin-3-yl)methyl)-3-(1*H*-pyrazol-3-yl)-3,6-diazabicyclo[3.1.1]heptane was obtained by using 6-((6-ethoxy-5-fluoropyridin-3-yl)methyl)-3-(1-(4-methoxybenzyl)-1*H*-pyrazol-3-yl)-3,6-diazabicyclo[3.1.1]heptane as raw material with reference to step 4 of embodiment 42.

MS m/z (ESI): 318.2[M+H]⁺.

### Step 5: 4-(3-(6-((6-ethoxy-5-fluoropyridin-3-yl)methyl)-3,6-diazabicyclo[3.1.1]heptan-3-yl)-1H-pyrazol-1-yl)-6-(2-hydroxy-2-methylpropoxy)pyrazolo[1,5-a]pyridine-3-carbonitrile

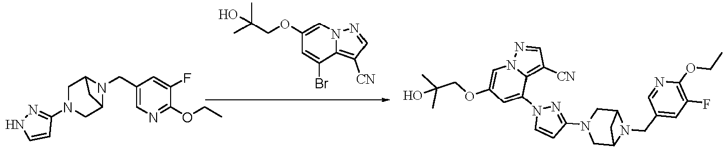

4-(3-(6-((6-Ethoxy-5-fluoropyridin-3-yl)methyl)-3,6-diazabicyclo[3.1.1]heptan-3-yl)-1*H*-pyrazol-1-yl)-6-(2-hydroxy-2-methylpropoxy)pyrazolo[1,5-*a*]pyridine-3-carbonitrile (80 mg, yield: 32%) was obtained by using 6-((6-ethoxy-5-fluoropyridin-3-yl)methyl)-3-(1*H*-pyrazol-3-yl)-3,6-diazabicyclo[3.1.1]heptane and 4-bromo-6-(2-hydroxy-2-methylpropoxy)pyrazolo[1,5-*a*]pyridine-3-carbonitrile as raw material with reference to step 4 of embodiment 42.

MS m/z (ESI): 547.2[M+H]⁺.

### [Not according to the invention] Embodiment 56

### 6-(2-Hydroxy-2-methylpropoxy)-4-(3-(4-((6-methoxypyridin-3-yl)oxo)piperidin-1-yl)-1H-pyrazol-1-yl)pyrazolo[1,5-a]pyridine-3-carbonitrile

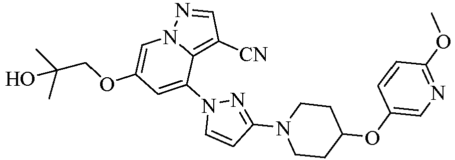

### Step 1: preparation of 2-methoxy-5-(piperidin-4-oxy)pyridine

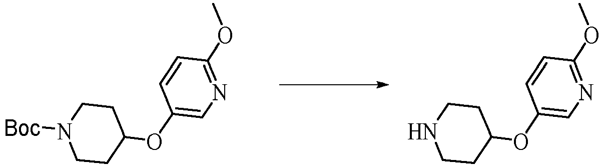

2-Methoxy-5-(piperidin-4-oxy)pyridine (2 g, yield: 90%) was obtained by using *tert*-butyl 4-((6-methoxypyridin-3-yl)oxy)piperidine-1-carboxylate as raw material with reference to step 4 of embodiment 43.

MS m/z (ESI): 209.2[M+H]⁺.

### Step 2: tert-butyl 3-(4-((6-methoxypyridin-3-yl)oxo)piperidin-1-yl)-1H-pyrazole-1-carboxylate

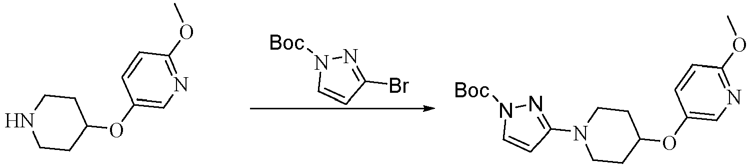

*Tert-butyl* 3-(4-((6-methoxypyridin-3-yl)oxo)piperidin-1-yl)-1*H*-pyrazole-1-carboxylate (yield: 68%) was obtained by using 2-methoxy-5-(piperidin-4-oxy)pyridine and *tert*-butyl 3-bromo-1*H*-pyrazole-1-carboxylate as raw material with reference to step 1 of embodiment 55.

MS m/z (ESI): 375.2[M+H]⁺.

### Step 3: preparation of 5-((1-(1H-pyrazol-3-yl)piperidin-4-yl)oxo)-2-methoxypyridine

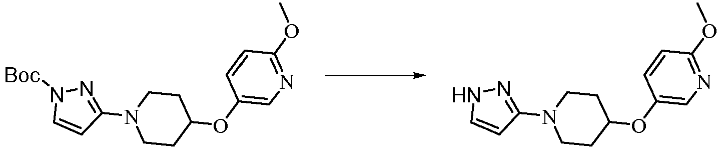

5-((1-(1*H*-pyrazol-3-yl)piperidin-4-yl)oxo)-2-methoxypyridine (yield: 88%) was obtained by using *tert*-butyl 3-(4-((6-methoxypyridin-3-yl)oxo)piperidin-1-yl)-1*H*-pyrazole-1-carboxylate as raw material with reference to step 4 of embodiment 43.

MS m/z (ESI): 275.2[M+H]⁺.

### Step 4: 6-(2-hydroxy-2-methylpropoxy)-4-(3-(4-((6-methoxypyridin-3-yl)oxo)piperidin-1-yl)-1H-pyrazol-1-yl)pyrazolo[1,5-a]pyridine-3-carbonitrile

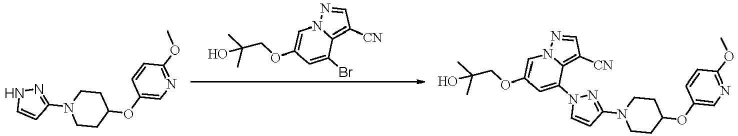

6-(2-Hydroxy-2-methylpropoxy)-4-(3-(4-((6-methoxypyridin-3-yl)oxo)piperidin-1-yl)-1*H-*pyrazol-1-yl)pyrazolo[1,5-*a*]pyridine-3-carbonitrile (75 mg, yield: 30%) was obtained by using 5-((1*-*(1*H-*pyrazol-3-yl)piperidin-4-yl)oxo)-2-methoxypyridine and 4-bromo-6-(2-hydroxy-2-methylpropoxy)pyrazolo[1,5-*a*]pyridine-3-carbonitrile as raw material with reference to step 4 of embodiment 42.

MS m/z (ESI): 504.2[M+H]⁺.

### [Not according to the invention] Embodiment 57

### 4-(4-Chloro-6-(6-((5-fluoro-6-methoxypyridin-3-yl)methyl)-3,6-diazabicyclo[3.1.1]heptan-3-yl)pyridin-3-yl)-6-(2-hydroxy-2-methylpropoxy)pyrazolo[1,5-a]pyridine-3-carbonitrile

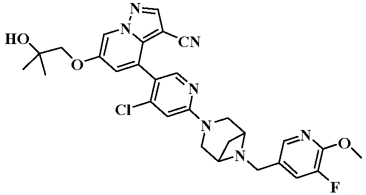

### Step 1: tert-butyl 3-(5-bromo-4-chloropyridin-2-yl)-3,6-diazabicyclo[3.1.1]heptane-6-carboxylate

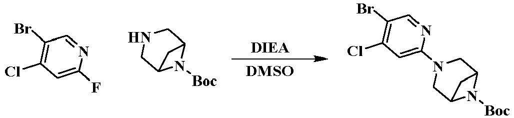

The product *tert-butyl* 3-(5-bromo-4-chloropyridin-2-yl)-3,6-diazabicyclo[3.1.1]heptane-6-carboxylate was obtained by using 5-bromo-4-chloro-2-fluoropyridine as raw material with reference to step 2 of embodiment 8.

MS m/z (ESI):388.0[M+H]⁺, 390.0[M+H+2]⁺.

### Step 2: 3-(5-bromo-4-chloropyridin-2-yl)-3,6-diazabicyclo[3.1.1]heptane

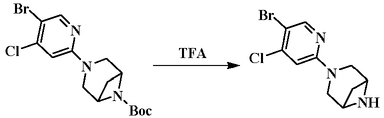

*Tert*-butyl 3-(5-bromo-4-chloropyridin-2-yl)-3,6-diazabicyclo[3.1.1]heptane-6-carboxylate (300 mg, 0.78 mmol) was dissolved in dichloromethane (5 mL), and trifluoroacetic acid (3 mL) was slowly added thereto dropwise, and the mixture was stirred at room temperature for 3 hours. The reaction mixture was concentrated, dissolved in ethyl acetate (10 mL), saturated sodium carbonate solution (5 mL) was added thereto, and the mixture was washed with saturated sodium chloride solution (5 mL x 2); then the organic phase was dried over anhydrous sodium sulfate and concentrated under reduced pressure to obtain 3-(5-bromo-4-chloropyridin-2-yl)-3,6-diazabicyclo[3.1.1]heptane (230 mg).

MS m/z (ESI): 288.0 [M+H]⁺, 290.0[M+2+H]⁺.

### Step 3: 3-(5-bromo-4-chloropyridin-2-yl)-6-((5-fluoro-6-methoxypyridin-3-yl)methyl)-3,6-diazabicyclo[3.1.1]heptane

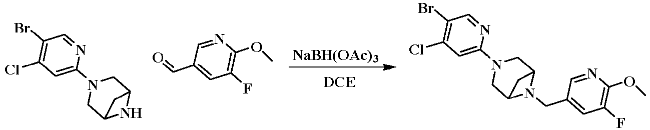

3-(5-Bromo-4-chloropyridin-2-yl)-3,6-diazabicyclo[3.1.1]heptane (230 mg, 0.78 mmol), 5-fluoro-6-methoxynicotinaldehyde (120 mg, 0.78 mmol) were dissolved in dichloroethane (5 mL), and sodium borohydride acetate (248 mg, 1.17 mmol) was added thereto under stirring, then the reaction mixture was stirred at room temperature for 12 hours. Saturated sodium carbonate solution (5 mL) thereto, then the reaction mixture was extracted with ethyl acetate (10 mL), washed with saturated sodium chloride solution (5 mL x 2); and the organic phase was dried over anhydrous sodium sulfate and purified by column chromatography under reduced pressure (dichloromethane/methanol: 30/1) to obtain 3-(5-bromo-4-chloropyridin-2-yl)-6-((5-fluoro-6-methoxypyridin-3-yl) methyl)-3,6-diazabicyclo[3.1.1]heptane (200 mg, white solid, 58.1%).

MS m/z (ESI): 427.0 [M+H]⁺, 429.0[M+2+H]⁺.

### Step 4: 3-(4-chloro-5-(4,4,5,5-tetramethyl-1,3,2-dioxaborolan-2-yl)pyridin-2-yl)-6-((5-fluoro-6-methoxypyridin-3-yl)methyl)-3,6-diazabicyclo[3.1.1]heptane

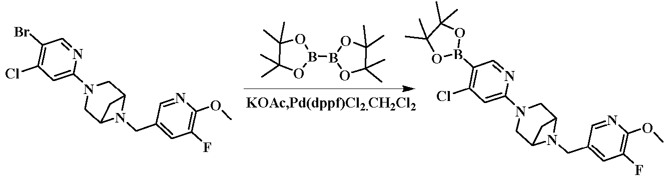

3-(5-Bromo-4-chloropyridin-2-yl)-6-((5-fluoro-6-methoxypyridin-3-yl)methyl)-3,6-diazabicyclo[3.1.1]heptane (200 mg, 0.47 mmol), bis(pinacolato)diboron (142 mg, 0.56 mmol), 1,1'-bis(diphenylphosphino)ferrocene-palladium(II)dichloride dichloromethane complex (19 mg, 0.023 mmol), potassium acetate (91 mg, 0.93 mmol), and dioxane (5 mL) were added sequentially in a 25 mL three-necked flask; and the reaction mixture was replaced with nitrogen five times. The reaction mixture was heated to 85 °C under the protection of nitrogen, and the mixture was stirred for 5 hours and then cooled to room temperature; the reaction mixture was concentrated, dissolved in ethyl acetate (10 mL) and washed with saturated saline (5 mLx3), and the organic phase was dried over anhydrous sodium sulfate, filtered and evaporated to dryness. The crude product was purified by prep-HPLC to obtain 3-(4-chloro-5-(4,4,5,5-tetramethyl-1,3,2-dioxaborolan-2-yl)pyridin-2-yl)-6-((5-fluoro-6-methoxypyridin-3-yl)methyl)-3,6-diazabicyclo[3.1.1]heptane (160 mg, yield: 72.2%).

MS m/z (ESI):475.2[M+H]⁺.

### Step 5: 4-(4-chloro-6-(6-((5-fluoro-6-methoxypyridin-3-yl)methyl)-3,6-diazabicyclo[3.1.1]heptan-3-yl)pyridin-3-yl)-6-(2-hydroxy-2-methylpropoxy)pyrazolo[1,5-a]pyridine-3-carbonitrile

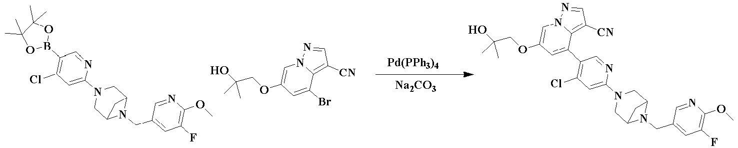

4-(4-Chloro-6-(6-((5-fluoro-6-methoxypyridin-3-yl)methyl)-3,6-diazabicyclo[3.1.1]heptan-3-yl)pyridin-3-yl)-6-(2-hydroxy-2-methylpropoxy)pyrazolo[1,5-*a*]pyridine-3-carbonitrile was obtained by using 3-(4-chloro-5-(4,4,5,5-tetramethyl-1,3,2-dioxaborolan-2-yl)pyridin-2-yl)-6-((5-fluoro-6-methoxypyridin-3-yl)methyl)-3,6-diazabicyclo[3.1.1]heptane as raw materials with reference to step 3 of embodiment 7.

MS m/z (ESI):578.2[M+H]⁺.

### [Not according to the invention] Embodiment 58

### 4-(4-Chloro-6-(4-(pyridin-2-oxy)piperidin-1-yl)pyridin-3-yl)-6-(2-hydroxy-2-methylpropoxy)pyrazolo[1,5-a]pyridine-3-carbonitrile

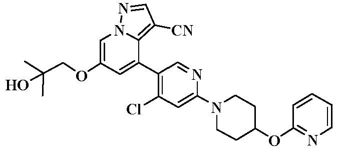

### Step 1: 5-bromo-4-chloro-2-(4-(pyridin-2-oxy)piperidin-1-yl)pyridine

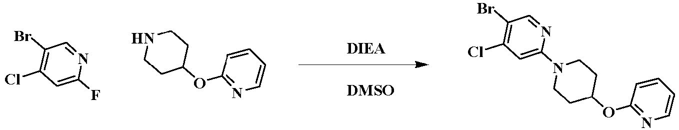

5-Bromo-4-chloro-2-(4-(pyridin-2-oxy)piperidin-1-yl)pyridine was obtained by using 5-bromo-4-chloro-2-fluoropyridine as raw material with reference to step 2 of embodiment 8.

MS m/z (ESI):368.0[M+H]⁺ , 370.0[M+H+2]⁺ .

### Step 2: 4-chloro-2-(4-(pyridin-2-oxy)piperidin-1-yl)-5-(4,4,5,5-tetramethyl-1,3,2-dioxaborolan-2-yl)pyridine

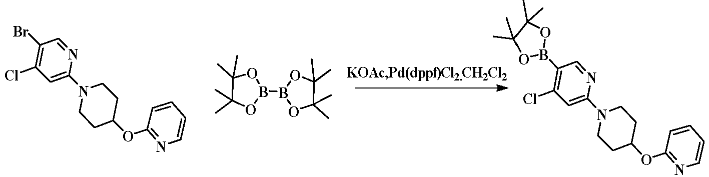

4-Chloro-2-(4-(pyridin-2-oxy)piperidin-1-yl)-5-(4,4,5,5-tetramethyl-1,3,2-dioxaborolan-2-yl)pyridine was obtained by using 5-bromo-4-chloro-2-(4-(pyridin-2-oxy)piperidin-1-yl)pyridine as raw material with reference to step 4 of embodiment 57.

MS m/z (ESI):416.2[M+H]⁺.

### Step 3: 4-(4-chloro-6-(4-(pyridin-2-oxy)piperidin-1-yl)pyridin-3-yl)-6-(2-hydroxy-2-methylpropoxy)pyrazolo[1,5-a]pyridine-3-carbonitrile

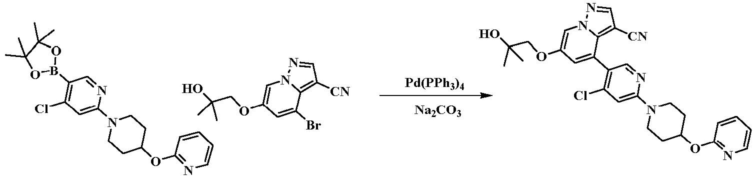

4-(4-Chloro-6-(4-(pyridin-2-oxy)piperidin-1-yl)pyridin-3-yl)-6-(2-hydroxy-2-methylpropoxy)pyrazolo[1,5-*a*]pyridine-3-carbonitrile was obtained by using 4-chloro-2-(4-(pyridin-2-oxy)piperidin-1-yl)-5-(4,4,5,5-tetramethyl-1,3,2-dioxaborolan-2-yl)pyridine as raw material with reference to step 3 of embodiment 7.

MS m/z (ESI):519.1[M+H]⁺.

### [Not according to the invention] Embodiment 59

### 4-(6-(4-Amino-4-((6-methoxypyridin-3-yl)methyl)piperidin-1-yl)pyridin-3-yl)-6-(2-hydroxy-2-methylpropoxy)pyrazolo[1,5-a]pyridine-3-carbonitrile

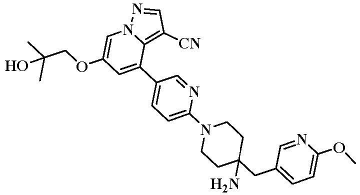

### Step 1: preparation of 1-(tert-butyl) 4-ethyl 4-((6-methoxypyridin-3-yl)methyl)piperidine-1,4-dicarboxylate

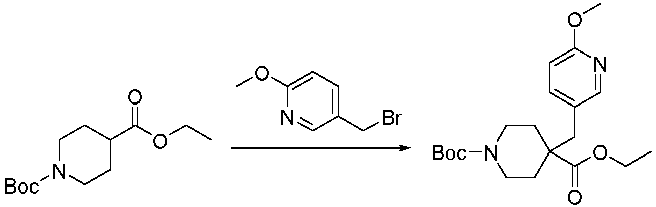

*1-(Tert-butyl)* 4-ethyl piperidine-1,4-dicarboxylate (10 g, 38.9 mmol) was dissolved in anhydrous tetrahydrofuran (100 mL), and the mixture was cooled to -76°C under the protection of nitrogen. LDA (29.2 mL, 58.4 mmol) was slowly added dropwise to the reaction mixture, and the temperature was controlled not to be higher than -70°C. After the addition was completed, the reaction was slowly warmed to 0°C, stirred for half an hour and cooled to -76 °C. 5-(Bromomethyl)-2-methoxypyridine (9.4 g, 46.6 mmol) was slowly added to the reaction mixture. After the addition was completed, the reaction mixture was slowly warmed to room temperature, and stirred for 4 hours. Saturated ammonium chloride aqueous solution was added to quench the reaction, and then the mixture was extracted with ethyl acetate. The organic phase was dried and evaporated to dryness. The crude product was purified by column chromatography to obtain *1-(tert-butyl)* 4-ethyl 4-((6-methoxypyridin-3-yl)methyl)piperidine-1,4-dicarboxylate (3.5 g, yield: 24%).

MS m/z (ESI): 379.2[M+H]⁺.

### Step 2: preparation of 1-(tert-butoxycarbonyl)-4-((6-methoxypyridin-3-yl)methyl)piperidine-4-carboxylic acid

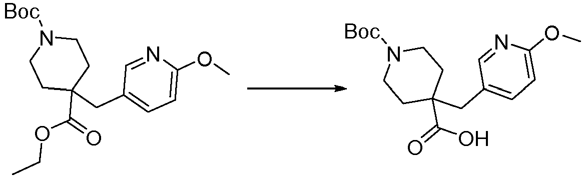

1-(*Tert*-butoxycarbonyl)-4-((6-methoxypyridin-3-yl)methyl)piperidine-4-carboxylic acid (2 g, yield: 80%) was obtained by using 51-(*tert*-butyl) 4-ethyl 4-((6-methoxypyridin-3-yl)methyl)piperidine-1,4-dicarboxylate as raw material with reference to step 2 of embodiment 40.

MS m/z (ESI): 351.2[M+H]⁺.

### Step 3: preparation of tert-butyl 4-((tert-butoxycarbonyl)amino)-4-((6-methoxypyridin-3-yl)methyl)piperidine-1-carboxylate

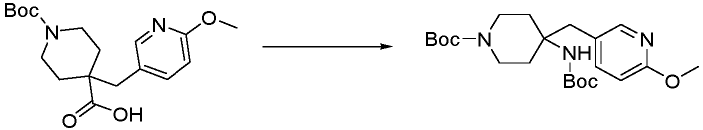

1-(*Tert*-butoxycarbonyl)-4-((6-methoxypyridin-3-yl)methyl)piperidine-4-carboxylic acid (2 g, 5.7 mmol) was dissolved in tert-butanol (50 mL), and DIEA (1.1 g, 8.6 mmol) and diphenyl azidophosphate (1.7 g, 6.3 mmol) were added thereto. The reaction mixture was stirred at 80 °C for 6 hours. The reaction mixture was evaporated to dryness. The crude product was purified by column chromatography to obtain *tert*-butyl 4-((*tert*-butoxycarbonyl)amino)-4-((6-methoxypyridin-3-yl)methyl)piperidine-1-carboxylate (1.6 g, yield: 67%).

MS m/z (ESI): 422.2[M+H]⁺.

### Step 3: preparation of 4-((6-methoxypyridin-3-yl)methyl)piperidin-4-amine

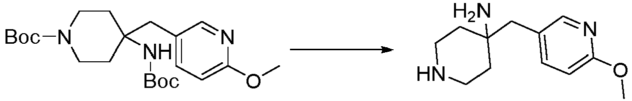

4-((6-Methoxypyridin-3-yl)methyl)piperidin-4-amine (0.8 g, yield: 70%) was obtained by using *tert*-butyl 4-((*tert*-butoxycarbonyl)amino)-4-((6-methoxypyridin-3-yl)methyl)piperidine-1-carboxylate as raw material with reference to step 4 of embodiment 43.

MS m/z (ESI): 222.2[M+H]⁺.

### Step 4: preparation of 4-(6-(4-amino-4-((6-methoxypyridin-3-yl)methyl)piperidin-1-yl)pyridin-3-yl)-6-(2-hydroxy-2-methylpropoxy)pyrazolo[1,5-a]pyridine-3-carbonitrile

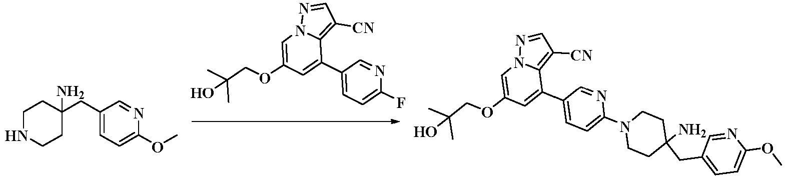

4-(6-(4-Amino-4-((6-methoxypyridin-3-yl)methyl)piperidin-1-yl)pyridin-3-yl)-6-(2-hydroxy-2-methylpropoxy)pyrazolo[1,5-*a*]pyridine-3-carbonitrile (55 mg, yield: 46%) was obtained by using 4-((6-methoxypyridin-3-yl)methyl)piperidin-4-amine and 4-(6-fluoropyridin-3-yl)-6-(2-hydroxy-2-methylpropoxy)pyrazolo[1,5-*a*]pyridine-3-carbonitrile as raw material with reference to step 6 of embodiment 42.

MS m/z (ESI): 528.2[M+H]⁺.

### [Not according to the invention] Embodiment 60

### 4-(6-(4-Amino-4-(pyridin-3-ylmethyl)piperidin-1-yl)pyridin-3-yl)-6-(2-hydroxy-2-methylpropoxy)pyrazolo[1,5-a]pyridine-3-carbonitrile

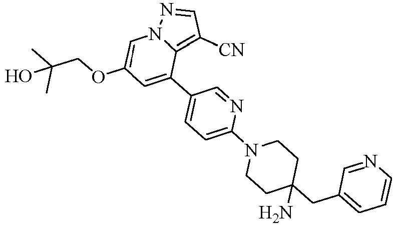

### Step 1: 1-(tert-butyl) 4-ethyl 4-(pyridin-3-ylmethyl)piperidine-1,4-dicarboxylate

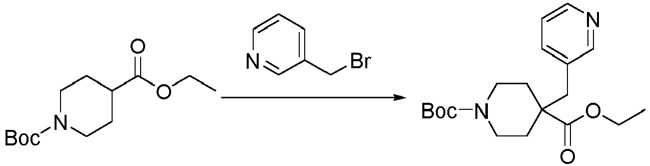

1-(*Tert-*butyl) 4-ethyl 4-(pyridin-3-ylmethyl)piperidine-1,4-dicarboxylate (3 g, yield: 68%) was obtained by using 1-(*tert*-butyl) 4-ethyl piperidine-1,4-dicarboxylate and 3-(bromomethyl)pyridine as raw material with reference to step 1 of embodiment 59.

MS m/z (ESI): 349.2[M+H]⁺.

### Step 2: preparation of 1-(tert-butoxycarbonyl)-4-(pyridin-3-ylmethyl)piperidine-4-carboxylic acid

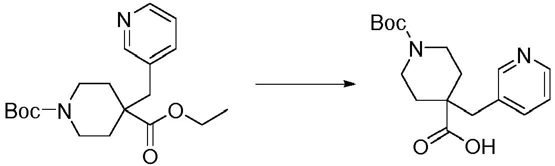

1-(*Tert*-butoxycarbonyl)-4-(pyridin-3-ylmethyl)piperidine-4-carboxylic acid (2.2 g, yield: 78%) was obtained by using 1-(*tert*-butyl) 4-ethyl 4-(pyridin-3-ylmethyl)piperidine-1,4-dicarboxylate as raw material with reference to step 2 of embodiment 40.

MS m/z (ESI): 321.2[M+H]⁺.

### Step 3: preparation of tert-butyl 4-((tert-butoxycarbonyl)amino)-4-(pyridin-3-ylmethyl)piperidine-1-carboxylate

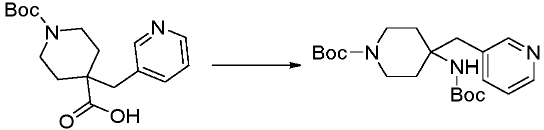

*Tert-butyl* 4-((*tert*-butoxycarbonyl)amino)-4-(pyridin-3-ylmethyl)piperidine-1-carboxylate (1.2 g, yield: 48%) was obtained by using 1-(*tert*-butoxycarbonyl)-4-(pyridin-3-ylmethyl)piperidine-4-carboxylic acid as raw material with reference to step 3 of embodiment 59.

MS m/z (ESI): 392.2[M+H]⁺.

### Step 4: preparation of 4-(pyridin-3-ylmethyl)piperidin-4-amine

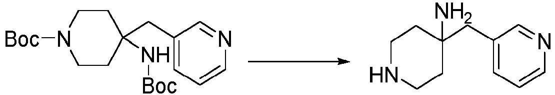

4-(Pyridin-3-ylmethyl)piperidin-4-amine (0.6 g, yield: 73%) was obtained by using *tert-butyl* 4-((*tert*-butoxycarbonyl)amino)-4-(pyridin-3-ylmethyl)piperidine-1-carboxylate as raw material with reference to step 4 of embodiment 43.

MS m/z (ESI): 192.2[M+H]⁺.

### Step 5: 4-(6-(4-amino-4-(pyridin-3-ylmethyl)piperidin-1-yl)pyridin-3-yl)-6-(2-hydroxy-2-methylpropoxy)pyrazolo[1,5-a]pyridine-3-carbonitrile

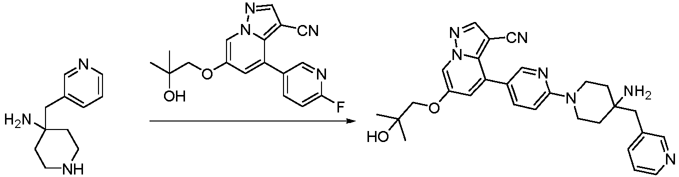

4-(6-(4-Amino-4-(pyridin-3-ylmethyl)piperidin-1-yl)pyridin-3-yl)-6-(2-hydroxy-2-methylpropoxy)pyrazolo[1,5-*a*]pyridine-3-carbonitrile (65 mg, yield: 44%) was obtained by using 4-(pyridin-3-ylmethyl)piperidin-4-amine and 4-(6-fluoropyridin-3-yl)-6-(2-hydroxy-2-methylpropoxy)pyrazolo[1,5-*a*]pyridine-3-carbonitrile as raw materials with reference to step 6 of embodiment 42.

MS m/z (ESI): 498.2[M+H]⁺.

### [Not according to the invention] Embodiment 61

### 4-(4-(4-Amino-1-((6-methoxypyridin-3-yl)methyl)piperidin-4-yl)phenyl)-6-(2-hydroxy-2-methylpropoxy)pyrazolo[1,5-a]pyridine-3-carbonitrile

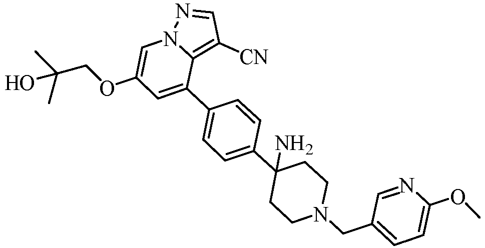

### Step 1: preparation of benzyl 4-((tert-butylsulfinyl<sulfinyl>)amino)-4-(4-chlorophenyl)piperidine-1-carboxylate

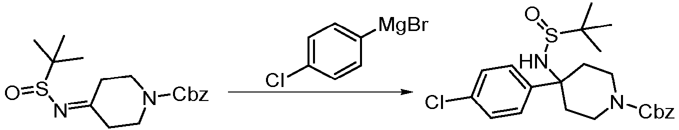

Benzyl 4-((*tert-*butyl sulfinyl<sulfinyl>)amino)-4-(4-chlorophenyl)piperidine-1-carboxylate (2 g, yield: 33%) was obtained by using benzyl 4-((*tert*-butylsulfinyl<sulfinyl>)imino)piperidine-1-carboxylate and (4-chlorophenyl)magnesium bromide as raw materials with reference to step 1 of embodiment 42.

MS m/z (ESI): 449.2[M+H]⁺.

### Step 2: preparation of benzyl 4-((tert-butylsulfinyl<sulfinyl>)amino)-4-(4-(3-cyano-6-(2-hydroxy-2-methylpropoxy)pyrazolo[1,5-a]pyridin-4-yl)phenyl)piperidine-1-carboxylate

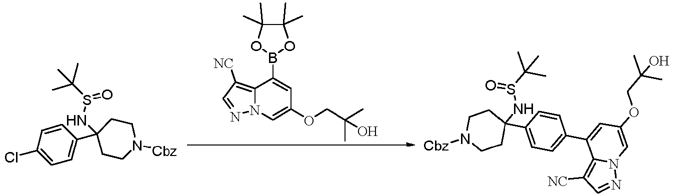

Benzyl 4-((*tert*-butylsulfinyl<sulfinyl>)amino)-4-(4-(3-cyano-6-(2-hydroxy-2-methylpropoxy)pyrazolo[1,5-*a*]pyridin-4-yl)phenyl)piperidine-1-carboxylate (1.2 g, yield: 66%) was obtained by using benzyl 4-((*tert*-butylsulfnyl<sulfinyl>)amino)-4-(4-chlorophenyl)piperidine-1 -carboxylate and 6-(2-hydroxy-2-methylpropoxy)-4-(4,4,5,5-tetramethyl-1,3,2-dioxaborolan-2-yl)pyrazolo[1,5-*a*]pyridine-3-carbonitrile as raw materials with reference to step 3 of embodiment 37.

MS m/z (ESI): 644.2[M+H]⁺.

### Step 3: preparation of N-(4-(4-(3-cyano-6-(2-hydroxy-2-methylpropoxy)pyrazolo[1,5-a]pyridin-4-yl)phenyl)piperidin-4-yl)-2-methylpropane-2-sulfinamide

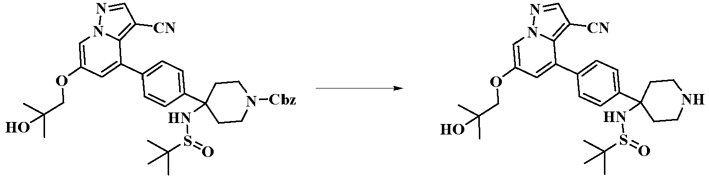

*N*-(4-(4-(3-cyano-6-(2-hydroxy-2-methylpropoxy)pyrazolo[1,5-*a*]pyridin-4-yl)phenyl)piperidin-4-yl)-2-methylpropane-2-sulfinamide was obtained by using benzyl 4-((*tert-*butylsulfinyl<sulfinyl>)amino)-4-(4-(3-cyano-6-(2-hydroxy-2-methylpropoxy)pyrazolo[1,5-*a*]pyridin-4-yl)phenyl)piperidine-1-carboxylate as raw material with reference to step 4 of embodiment 42.

MS m/z (ESI): 510.2[M+H]⁺.

### Step 4: preparation of N-(4-(4-(3-cyano-6-(2-hydroxy-2-methylpropoxy)pyrazolo[1,5-a]pyridin-4-yl)phenyl)-1-((6-methoxypyridin-3-yl)methyl)piperidin-4-yl)-2-methylpropane-2-sulfinamide

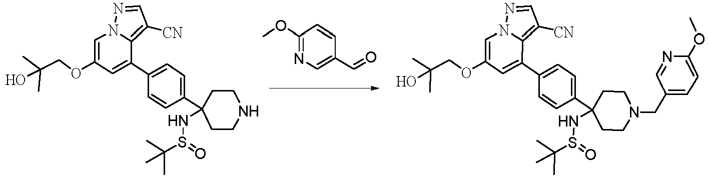

*N*-(4-(4-(3-cyano-6-(2-hydroxy-2-methylpropoxy)pyrazolo[1,5-*a*]pyridin-4-yl)phenyl)-1-((6-methoxypyridin-3-yl)methyl)piperidin-4-yl)-2-methylpropane-2-sulfinamide (0.3 g, yield: 56%) was obtained by using *N*-(4-(4-(3-cyano-6-(2-hydroxy-2-methylpropoxy)pyrazolo[1,5-*a*]pyridin-4-yl)phenyl)piperidin-4-yl)-2-methylpropane-2-sulfinamide and 6-methoxynicotinaldehyde as raw materials with reference to step 3 of embodiment 55.

MS m/z (ESI): 631.3[M+H]⁺.

### Step 5: 4-(4-(4-amino-1-((6-methoxypyridin-3-yl)methyl)piperidin-4-yl)phenyl)-6-(2-hydroxy-2-methylpropoxy)pyrazolo[1,5-a]pyridine-3-carbonitrile

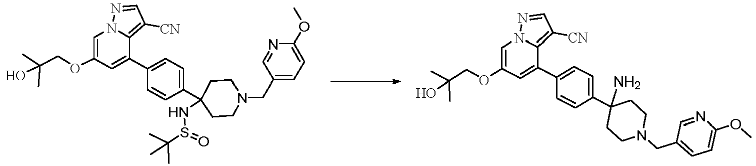

4-(4-(4-Amino-1-((6-methoxypyridin-3-yl)methyl)piperidin-4-yl)phenyl)-6-(2-hydroxy-2-methylpropoxy)pyrazolo[1,5-*a*]pyridine-3-carbonitrile (yield: 85%) was obtained by using *N-*(4-(4-(3-cyano-6-(2-hydroxy-2-methylpropoxy)pyrazolo[1,5-*a*]pyridin-4-yl)phenyl)-1-((6-methoxypyridin-3-yl)methyl)piperidin-4-yl)-2-methylpropane-2-sulfinamide as raw material with reference to step 2 of embodiment 42.

MS m/z (ESI): 527.3[M+H]⁺.

### [Not according to the invention] Embodiment 62

### 4-(6-(4-Amino-4-(indoline-1-carbonyl)piperidin-1-yl)pyridin-3-yl)-6-(2-hydroxy-2-methylpropoxy)pyrazolo[1,5-a]pyridine-3-carbonitrile

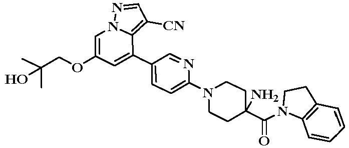

### Step 1: ethyl 4-((tert-butoxycarbonyl)amino)-1-(5-(3-cyano-6-(2-hydroxy-2-methylpropoxy)pyrazolo[1,5-a]pyridin-4-yl)pyridin-2-yl)piperidine-4-carboxylate

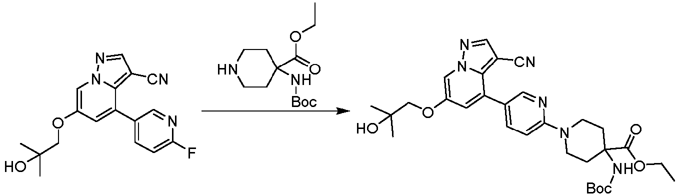

A mixture of 4-(6-fluoropyridin-3-yl)-6-(2-hydroxy-2-methylpropoxy)pyrazolo[1,5-*a*]pyridine-3-carbonitrile (3 g, 9.2 mmol), ethyl 4-((*tert-*butoxycarbonyl)amino)piperidine-4-carboxylate (3.0 g, 11.04 mmol), *N,N*-diisopropylethylamine (3.6 g, 27.6 mmol) and acetonitrile (30 mL) was stirred at 80 °C for 16 hours, and the mixture was stirred at 80 °C for 16 hours; after the reaction was completed, the mixture was mixed with silica gel and concentrated under reduced pressure to dryness, then separated by column chromatography (dichloromethane/methanol = 10:1) to obtain colorless oil ethyl 4-((*tert-*butoxycarbonyl)amino)-1-(5-(3-cyano-6-(2-hydroxy-2-methylpropoxy)pyrazolo[1,5-*a*]pyridin-4-yl)pyridin-2-yl)piperidine-4-carboxylate (800 mg, yield: 15%).

MS m/z (ESI): 579.3 [M+H]⁺.

### Step 2: 4-((tert-butoxycarbonyl)amino)-1-(5-(3-cyano-6-(2-hydroxy-2-methylpropoxy)pyrazolo[1,5-a]pyridin-4-yl)pyridin-2-yl)piperidine-4-carboxylic acid

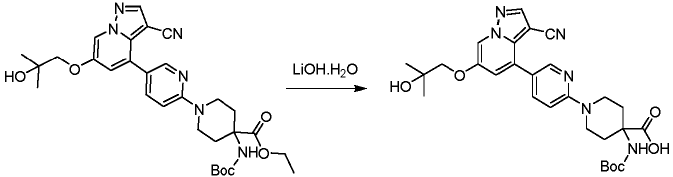

Lithium hydroxide monohydrate (116 mg, 2.76 mmol) was added to a solution of ethyl 4-((*tert-*butoxycarbonyl)amino)-1-(5-(3-cyano-6-(2-hydroxy-2-methylpropoxy)pyrazolo[1,5-*a*]pyridin-4-yl)pyridin-2-yl)piperidine-4-carboxylate (800 mg, 1.38 mmol) in methanol (10 mL) and water (10 mL); and the mixture was stirred at room temperature for 16 hours; after the reaction was completed, with the pH value was adjusted to 2 with dilute hydrochloric acid, a white solid precipitated and was filtered; and the filter cake was washed with water and dried to obtain white solid 4-((*tert*-butoxycarbonyl)amino)-1-(5-(3-cyano-6-(2-hydroxy-2-methylpropoxy)pyrazolo[1,5-*a*]pyridin-4-yl)pyridin-2-yl)piperidine-4-carboxylic acid (600 mg, yield: 79%).

MS m/z (ESI): 551.2 [M+H]⁺.

### Step 3: tert-butyl (1-(5-(3-cyano-6-(2-hydroxy-2-methylpropoxy)pyrazolo[1,5-a]pyridin-4-yl)pyridin-2-yl)-4-(indoline-1-carbonyl)piperidin-4-yl)carbamate

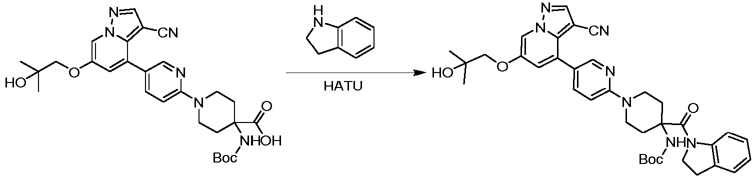

A mixture of 4-((*tert-*butoxycarbonyl)amino)-1-(5-(3-cyano-6-(2-hydroxy-2-methylpropoxy)pyrazolo[1,5-*a*]pyridin-4-yl)pyridin-2-yl)piperidine-4-carboxylic acid (100 mg, 0.18 mmol), 2-(7-azabenzotriazol-1-yl)-*N,N,N',N'*-tetramethyluronium hexafluorophosphate (82.91 mg, 0.22 mmol), triethylamine (36 mg, 0.36 mmol), dihydroindole (32 mg, 0.27 mmol) and dichloromethane (5 mL) was stirred at room temperature for 16 hours; after the reaction was completed, the mixture was quenched with water, extracted with ethyl acetate, the organic phase was washed with saturated saline, dried over anhydrous sodium sulfate, filtered, and the filtrate was concentrated under reduced pressure to dryness and separated by column chromatography (dichloromethane/methanol=10:1) to obtain colorless oil *tert-butyl* (1-(5-(3-cyano-6-(2-hydroxy-2-methylpropoxy)pyrazolo[1,5-*a*]pyridin-4-yl)pyridin-2-yl)-4-(indoline-1-carbonyl)piperidin-4-yl)carbamate (30 mg, yield: 26%).

MS m/z (ESI): 652.3 [M+H]⁺.

### Step 4: 4-(6-(4-amino-4-(indoline-1-carbonyl)piperidin-1-yl)pyridin-3-yl)-6-(2-hydroxy-2-methylpropoxy)pyrazolo[1,5-a]pyridine-3-carbonitrile

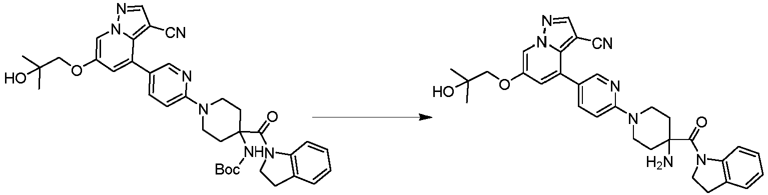

*Tert-butyl* (1-(5-(3-cyano-6-(2-hydroxy-2-methylpropoxy)pyrazolo[1,5-*a*]pyridin-4-yl)pyridin-2-yl)-4-(indoline)-1-carbonyl)piperidin-4-yl)carbamate (30 mg, 0.046 mmol) was dissolved in dichloromethane (3 mL), then trifluoroacetic acid (1 mL) was added thereto, and the mixture was stirred at room temperature for 1 hour; after the reaction was completed, the mixture was concentrated under reduced pressure to dryness and separated by preparative chromatography to obtain light yellow oil 4-(6-(4-amino-4-(indoline-1-carbonyl)piperidin-1-yl)pyridin-3-yl)-6-(2-hydroxy-2-methylpropoxy)[1,5-*a*]pyridine-3-carbonitrile (12 mg, yield: 47%).

¹H NMR (400 MHz, CDCl₃) δ 8.34 (s, 1H), 8.26 - 8.18 (m, 2H), 8.16 (s, 1H), 7.70 (d, *J* = 6.6 Hz, 1H), 7.18 (m, 3H), 7.07-7.03 (m, 1H), 6.83 (d, *J =* 8.7 Hz, 1H), 4.42-4.39 (m, 2H), 4.11-4.07 (m, 2H), 3.85-3.81(m, 4H), 3.13-3.09 (t, *J* = 7.7 Hz, 2H), 2.51-2.47(m, 2H), 1.92-1.89 (m, 4H), 1.40 (s, 6H).

MS m/z (ESI): 552.3 [M+H]⁺.

### [Not according to the invention] Embodiment 63

### 4-Amino-1-(5-(3-cyano-6-(2-hydroxy-2-methylpropoxy)pyrazolo[1,5-a]pyridin-4-yl)pyridin-2-yl)-N-(6-methoxypyridin-3-yl)piperidine-4-carboxamide

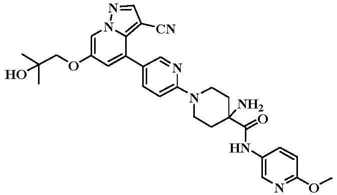

The synthesis of embodiment 63 was referred to embodiment 62.

MS m/z (ESI): 557.2 [M+H]⁺.

### [Not according to the invention] Embodiment 64

### 4-Amino-1-(5-(3-cyano-6-(2-hydroxy-2-methylpropoxy)pyrazolo[1,5-a]pyridin-4-yl)pyridin-2-yl)-N-methyl-N-phenylpiperidine-4-carboxamide

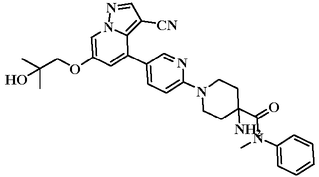

The synthesis of embodiment 64 was referred to embodiment 62.

MS m/z (ESI): 540.2 [M+H]⁺.

### [Not according to the invention] Embodiment 65

### 4-(6-(4-Amino-4-(2,3-dihydro-1H-pyrrolo[3,2-b]pyridine-1-carbonyl)piperidin-1-yl)pyridin-3-yl)-6-(2-hydroxy-2-methylpropoxy)pyrazolo[1,5-a]pyridine-3-carbonitrile

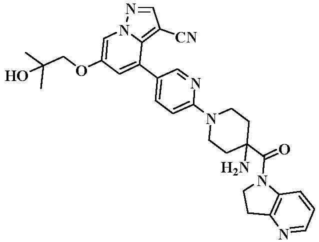

The synthesis of embodiment 65 was referred to embodiment 62.

MS m/z (ESI): 553.2 [M+H]⁺

### [Not according to the invention] Embodiment 66

### 4-(6-(3-((1H-pyrazol-1-yl)methyl)-3-aminoazetidin-1-yl)pyridin-3-yl)-6-(2-hydroxy-2-methylpropoxy)pyrazolo[1,5-a]pyridine-3-carbonitrile

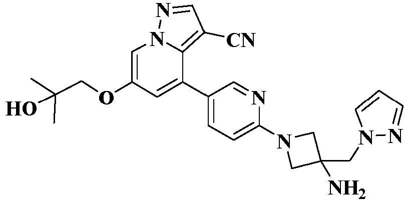

### Step 1: preparation of 1-(tert-butyl) 3-methyl 3-((tert-butoxycarbonyl)amino)azetidine-1,3-dicarboxylate

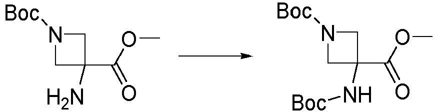

*1-(Tert-butyl)* 3-methyl 3-aminoazetidine-1,3-dicarboxylate (10 g, 43.4 mmol) was dissolved in dichloromethane (100 mL); and *di-tert-butyl* dicarbonate (10 g, 45.6 mmol) and DIEA (8.4 g, 65.1 mmol) were added thereto. The reaction mixture was stirred at room temperature overnight. Water was added thereto, and then the mixture was extracted with dichloromethane. The organic phase was dried and evaporated to dryness. The crude product was purified by column chromatography to obtain *1-(tert-butyl)* 3-methyl 3-((*tert*-butoxycarbonyl)amino)azetidine-1,3-dicarboxylate (13 g, yield: 91%).

MS m/z (ESI): 331.2[M+H]⁺.

### Step 2: Preparation of tert-butyl 3-((tert-butoxycarbonyl)amino)-3-(hydroxymethyl)azetidine-1-carboxylate

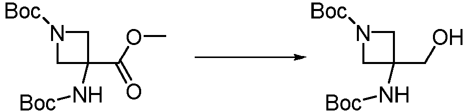

*1-(Tert-butyl)* 3-methyl 3-((*tert*-butoxycarbonyl)amino)azetidine-1,3-dicarboxylate (5 g, 15.1 mmol) was dissolved in anhydrous tetrahydrofuran (50 mL), and lithium tetrahydroaluminum (0.86 g, 22.7 mmol) was added thereto. The reaction mixture was stirred at 70 °C overnight. Water was added to quench the reaction, and then the mixture was extracted with ethyl acetate. The mixture was filtered. The filtrate was dried and evaporated to dryness. The crude product was purified by column chromatography to obtain *tert-*butyl 3-((*tert-*butoxycarbonyl)amino)-3-(hydroxymethyl)azetidine-1-carboxylate (2.3 g, yield: 50%).

MS m/z (ESI): 303.2[M+H]⁺.

### Step 3: preparation of tert-butyl 3-((tert-butoxycarbonyl)amino)-3-(((methylsulfonyl)oxo)methyl)azetidine-1-carboxylate

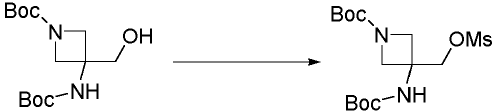

*Tert-butyl* 3-((*tert*-butoxycarbonyl)amino)-3-(hydroxymethyl)azetidine-1-carboxylate (2.3 g, 7.6 mmol) was dissolved in anhydrous dichloromethane (30 mL) and, DIEA (2 g, 15.2 mmol) was added thereto. MsCl (1.1 g, 9.1 mmol) was slowly added dropwise to the reaction mixture at 0°C. After the addition was completed, the reaction mixture was slowly warmed to room temperature, and stirred for 4 hours. Water was added to the reaction, and then the mixture was extracted with dichloromethane. The organic phase was evaporated to dryness to obtain *tert-*butyl 3-((*tert*-butoxycarbonyl)amino)-3-(((methylsulfonyl)oxo)methyl)azetidine-1-carboxylate (2.8 g, yield: 97%).

MS m/z (ESI): 381.2[M+H]⁺.

### Step 4: preparation of tert-butyl 3-((1H-pyrazol-1-yl)methyl)-3-((tert-butoxycarbonyl)amino)azetidine-1-carboxylate

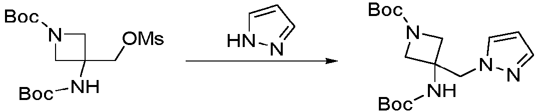

*Tert-butyl* 3-((1*H*-pyrazol-1-yl)methyl)-3-((*tert-*butoxycarbonyl)amino)azetidine-1-carboxylate was obtained by using *tert*-butyl 3-((*tert*-butoxycarbonyl)amino)-3-(((methylsulfonyl)oxy)methyl)azetidine-1-carboxylate and 1*H*-pyrazole as raw materials with reference to step 5 of embodiment 37.

MS m/z (ESI): 353.2[M+H]⁺.

### Step 5: preparation of 3-((1H-pyrazol-1-yl)methyl)azetidin-3-amine

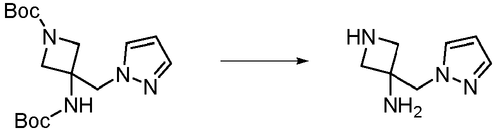

3-((1*H*-pyrazol-1-yl)methyl)azetidin-3-amine (0.7 g, yield: 90%) was obtained by using *tert-butyl* 3-((1*H*-pyrazol-1-yl)methyl)-3-((tert-butoxycarbonyl)amino)azetidine-1-carboxylate as raw material with reference to step 4 of embodiment 43.

MS m/z (ESI): 153.1[M+H]⁺.

### Step 5: preparation of 4-(6-(3-((1H-pyrazol-1-yl)methyl)-3-aminoazetidin-1-yl)pyridin-3-yl)-6-(2-hydroxy-2-methylpropoxy)pyrazolo[1,5-a]pyridine-3-carbonitrile

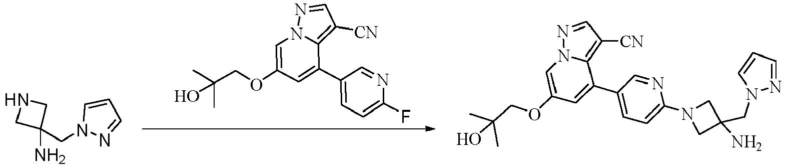

4-(6-(3-((1*H*-pyrazol-1-yl)methyl)-3-aminoazetidin-1-yl)pyridin-3-yl)-6-(2-hydroxy-2-methylpropoxy)pyrazolo[1,5-*a*]pyridine-3-carbonitrile (63 mg, yield: 69%) was obtained by using 3-((1*H-*pyrazol-1-yl)methyl)azetidin-3-amine and 4-(6-fluoropyridin-3-yl)-6-(2-hydroxy-2-methylpropoxy)pyrazolo[1,5-*a*]pyridine-3-carbonitrile as raw materials with reference to step 6 of embodiment 42.

MS m/z (ESI): 459.2[M+H]⁺.

### [Not according to the invention] Embodiment 67

### 4-(6-(3-Amino-3-((6-methoxypyridin-3-yl)methyl)azetidin-1-yl)pyridin-3-yl)-6-(2-hydroxy-2-methylpropoxy)pyrazolo[1,5-a]pyridine-3-carbonitrile

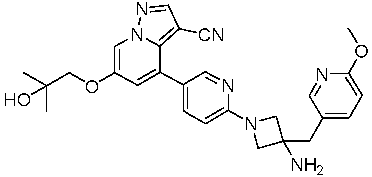

### Step 1: preparation of 1-(tert-butyl) 3-methyl 3-((6-methoxypyridin-3-yl)methyl)azetidine-1,3-dicarboxylate

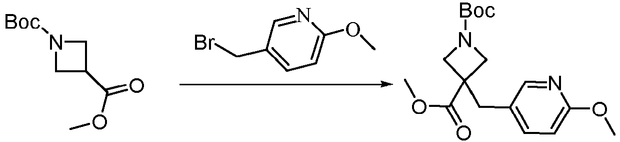

*1-(tert-butyl)* 3-methyl 3-((6-methoxypyridin-3-yl)methyl)azetidine-1,3-dicarboxylate (5 g, yield: 63%) was obtained by using 1-(*tert*-butyl) 3-methyl azetidine-1,3-dicarboxylate and 5-(bromomethyl)-2-methoxypyridine as raw material with reference to step 1 of embodiment 59.

MS m/z (ESI): 337.2[M+H]⁺.

### Step 2: preparation of 1-(tert-butoxycarbonyl)-3-((6-methoxypyridin-3-yl)methyl)azetidine-3-carboxylic acid

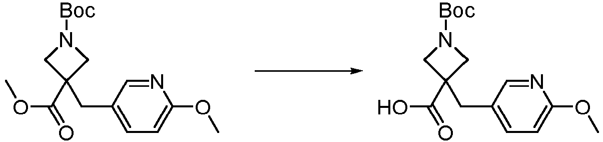

1-(*Tert*-butoxycarbonyl)-3-((6-methoxypyridin-3-yl)methyl)azetidine-3-carboxylic acid (2.5 g, yield: 76%) was obtained by using *1-(tert-butyl)* 3-methyl 3-((6-methoxypyridin-3-yl)methyl)azetidine-1,3-dicarboxylate as raw material with reference to step 2 of embodiment 40.

MS m/z (ESI): 323.2[M+H]⁺.

### Step 3: preparation of tert-butyl 3-((tert-butoxycarbonyl)amino)-3-((6-methoxypyridin-3-yl)methyl)azetidine-1-carboxylate

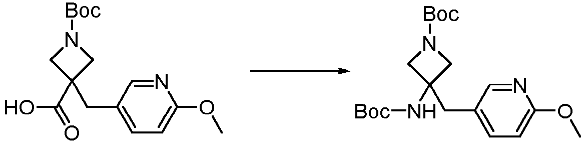

*Tert-butyl* 3-((*tert*-butoxycarbonyl)amino)-3-((6-methoxypyridin-3-yl)methyl)azetidine-1-carboxylate (1.5 g, yield: 67%) was obtained by using 1-(*tert*-butoxycarbonyl)-3-((6-methoxypyridin-3-yl)methyl)azetidine-3-carboxylic acid as raw material with reference to step 3 of embodiment 59.

MS m/z (ESI): 394.2[M+H]⁺.

### Step 4: preparation of 3-((6-methoxypyridin-3-yl)methyl)azetidin-3-amine

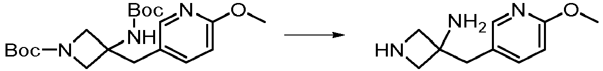

3-((6-Methoxypyridin-3-yl)methyl)azetidin-3-amine (0.7 g, yield: 91%) was obtained by using *tert-*butyl 3-((*tert*-butoxycarbonyl)amino)-3-((6-methoxypyridin-3-yl)methyl)azetidine-1-carboxylate as raw material with reference to step 4 of embodiment 43.

MS m/z (ESI): 194.1[M+H]⁺.

### Step 5: preparation of 4-(6-(3-amino-3-((6-methoxypyridin-3-yl)methyl)azetidin-1-yl)pyridin-3-yl)-6-(2-hydroxy-2-methylpropoxy)pyrazolo[1,5-a]pyridine-3-carbonitrile

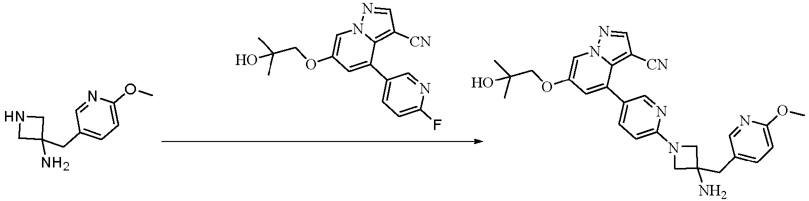

4-(6-(3-Amino-3-((6-methoxypyridin-3-yl)methyl)azetidin-1-yl)pyridin-3-yl)-6-(2-hydroxy-2-methylpropoxy)pyrazolo[1,5-*a*]pyridine-3-carbonitrile (53 mg, yield: 59%) was obtained by using 3-((6-methoxypyridin-3-yl)methyl)azetidin-3-amine and 4-(6-fluoropyridin-3-yl)-6-(2-hydroxy-2-methylpropoxy)pyrazolo[1,5-*a*]pyridine-3-carbonitrile as raw material with reference to step 6 of embodiment 42.

MS m/z (ESI): 500.2[M+H]⁺.

### [Not according to the invention] Embodiment 68

### 4-(4-(3-Amino-1-((6-methoxypyridin-3-yl)methyl)azetidin-3-yl)phenyl)-6-(2-hydroxy-2-methylpropoxy)pyrazolo[1,5-a]pyridine-3-carbonitrile

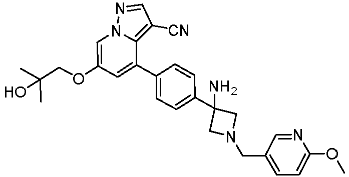

### Step 1: preparation of benzyl 3-((tert-butylsulfinyl<sulfinyl>)amino)-3-(4-chlorophenyl)azetidin-1-carboxylate

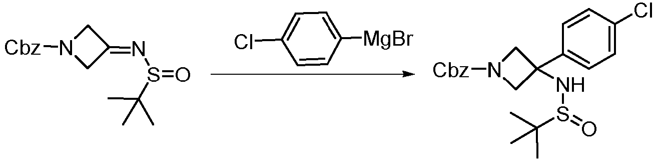

Benzyl 3-((*tert*-butylsulfmyl<sulfmyl>)amino)-3-(4-chlorophenyl)azetidin-1-carboxylate (2.2 g, yield: 35%) was obtained by using benzyl 3-((*tert*-butylsulfinyl<sulfinyl>)imino)azetidin-1-carboxylate and (4-chlorophenyl)magnesium bromide as raw materials with reference to step 1 of embodiment 42.

MS m/z (ESI): 421.2[M+H]⁺.

### Step 2: preparation of benzyl 3-((tert-butylsulfmyl<sulfmyl>)amino)-3-(4-(3-cyano-6-(2-hydroxy-2-methylpropoxy)pyrazolo[1,5-a]pyridin-4-yl)phenyl)azetidin-1-carboxylate

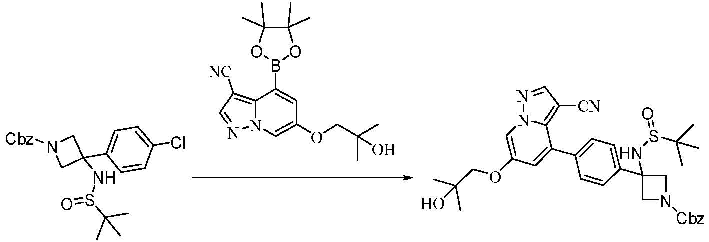

Benzyl 3-((*tert*-butylsulfinyl<sulfinyl>)amino)-3-(4-(3-cyano-6-(2-hydroxy-2-methylpropoxy)pyrazolo[1,5-*a*]pyridin-4-yl)phenyl)azetidine-1-carboxylate (1.3 g, yield: 68%) was obtained by using benzyl 3-((*tert*-butylsulfinyl<sulfinyl>)amino)-3-(4-chlorophenyl)azetidine-1-carboxylate and 6-(2-hydroxy-2-methylpropoxy)-4-(4,4,5,5-tetramethyl-1,3,2-dioxaborolan-2-yl)pyrazolo[1,5-*a*]pyridine-3-carbonitrile as raw materials with reference to step 3 of embodiment 37.

MS m/z (ESI): 616.2[M+H]⁺.

### Step 3: preparation of N-(3-(4-(3-cyano-6-(2-hydroxy-2-methylpropoxy)pyrazolo[1,5-a]pyridin-4-yl)phenyl)azetidin-3-yl)-2-methylpropane-2-sulfinamide

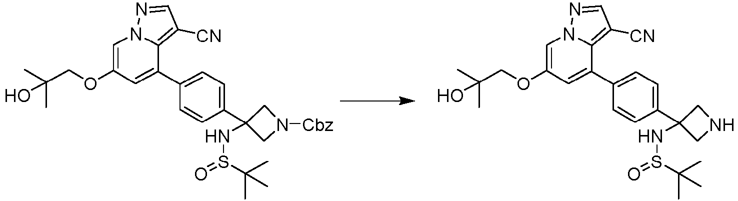

*N*-(3-(4-(3-cyano-6-(2-hydroxy-2-methylpropoxy)pyrazolo[1,5-*a*]pyridin-4-yl)phenyl)azetidin-3-yl)-2-methylpropane-2-sulfinamide (0.9 g, yield: 82%) was obtained by using benzyl 3-((*tert-*butylsulfmyl<sulfmyl>)amino)-3-(4-(3-cyano-6-(2-hydroxy-2-methylpropoxy)pyrazolo[1,5-a]pyridin-4-yl)phenyl)azetidine-1-carboxylate as raw materials with reference to step 4 of embodiment 42.

MS m/z (ESI): 482.2[M+H]⁺.

### Step 4: preparation of N-(3-(4-(3-cyano-6-(2-hydroxy-2-methylpropoxy)pyrazolo[1,5-a]pyridin-4-yl)phenyl)-1-((6-methoxypyridin-3-yl)methyl)azetidin-3-yl)-2-methylpropane-2-sulfinamide

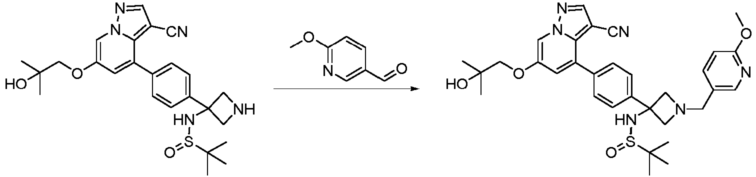

*N*-(3-(4-(3-cyano-6-(2-hydroxy-2-methylpropoxy)pyrazolo[1,5-*a*]pyridin-4-yl)phenyl)-1-((6-methoxypyridin-3-yl)methyl)azetidin-3-yl)-2-methylpropane-2-sulfinamide (0.4 g, yield: 62%) was obtained by using *N-*(3-(4-(3-cyano-6-(2-hydroxy-2-methylpropoxy)pyrazolo[1,5-*a*]pyridin-4-yl)phenyl)azetidin-3-yl)-2-methylpropane-2-sulfinamide and 6-methoxynicotinaldehyde as raw materials with reference to step 3 of embodiment 55.

MS m/z (ESI): 603.3[M+H]⁺.

### Step 5: 4-(4-(3-amino-1-((6-methoxypyridin-3-yl)methyl)azetidin-3-yl)phenyl)-6-(2-hydroxy-2-methylpropoxy)pyrazolo[1,5-a]pyridine-3-carbonitrile

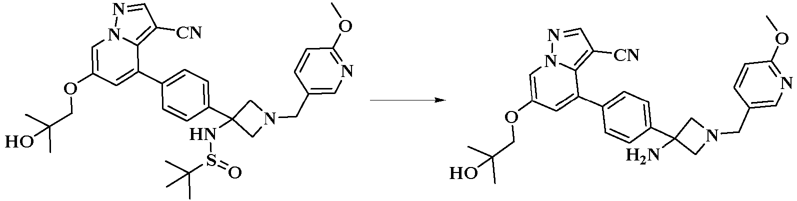

4-(4-(3-Amino-1-((6-methoxypyridin-3-yl)methyl)azetidin-3-yl)phenyl)-6-(2-hydroxy-2-methylpropoxy)pyrazolo[1,5-*a*]pyridine-3-carbonitrile (150 mg, yield: 84%) was obtained by using N-(3-(4-(3<cyano-6-(2-hydroxy-2-methylpropoxy)pyrazolo[1,5-a]pyridin-4-yl)phenyl)-1-((6-methoxypyridin-3-yl)methyl)azetidin-3-yl)-2-methylpropane-2-sulfinamide as raw material with reference to step 2 of embodiment 42.

MS m/z (ESI): 499.2[M+H]⁺.

### [Not according to the invention] Embodiment 69

### 4-(6-(3-(3-Amino-1H-pyrazol-1-yl)azetidin-1-yl)pyridin-3-yl)-6-(2-hydroxy-2-methylpropoxy)pyrazolo[1,5-a]pyridine-3-carbonitrile

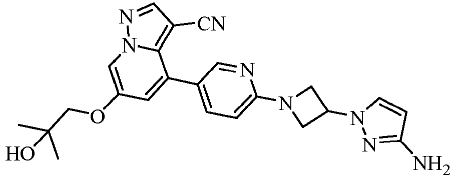

### Step 1: tert-butyl 3-(3-nitro-1H-pyrazol-1-yl)azetidine-1-carboxylate

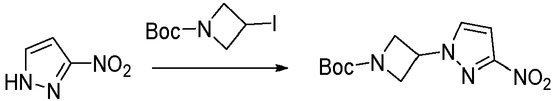

3-Nitro-1*H*-pyrazole (10 g, 88.4 mmol) was dissolved in anhydrous DMF (120 mL), and *tert-butyl* 3-iodoazetidine-1-carboxylate (30 g, 106.1 mmol) and potassium carbonate (24.4 g, 176.9 mmol) were added thereto. The reaction mixture was stirred at 60 °C overnight. Water was added thereto, and then the mixture was extracted with ethyl acetate. The organic phase was dried and evaporated to dryness. The crude product was purified by column chromatography to obtain tert-butyl 3-(3-nitro-1*H*-pyrazol-1-yl)azetidine-1-carboxylate (20 g, yield: 84%).

MS m/z (ESI): 269.1[M+H]⁺.

### Step 2: preparation of 1-(azetidin-3-yl)-3-nitro-1H-pyrazole

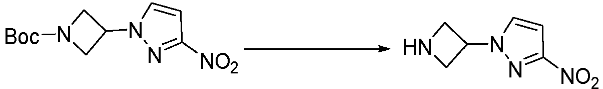

1-(Azetidin-3-yl)-3-nitro-1*H*-pyrazole (5 g, yield: 93%) was obtained by using *tert*-butyl 3-(3-nitro-1*H*-pyrazol-1-yl)azetidine-1-carboxylate as raw material with reference to step 4 of embodiment 43. MS m/z (ESI): 169.1[M+H]⁺.

### Step 3: preparation of 6-(2-hydroxy-2-methylpropoxy)-4-(6-(3-(3-nitro-1H-pyrazol-1-yl)azetidin-1-yl)pyridin-3-yl)pyrazolo[1,5-a]pyridine-3-carbonitrile

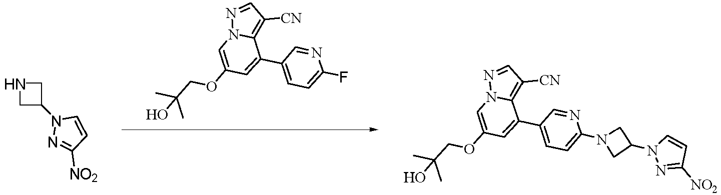

6-(2-Hydroxy-2-methylpropoxy)-4-(6-(3-(3-nitro-1*H*-pyrazol-1-yl)azetidin-1-yl)pyridin-3-yl)pyrazolo[1,5-*a*]pyridine-3-carbonitrile (500 mg, yield: 68%) was obtained by using 1-(azetidin-3-yl)-3-nitro-1*H*-pyrazole and 4-(6-fluoropyridin-3-yl)-6-(2-hydroxy-2-methylpropoxy)pyrazolo[1,5-*a*]pyridine-3-carbonitrile as raw materials with reference to step 6 of embodiment 42.

MS m/z (ESI): 475.2[M+H]⁺.

### Step 4: preparation of 4-(6-(3-(3-amino-1H-pyrazol-1-yl)azetidin-1-yl)pyridin-3-yl)-6-(2-hydroxy-2-methylpropoxy)pyrazolo[1,5-a]pyridine-3-carbonitrile

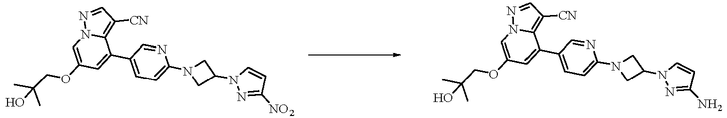

6-(2-Hydroxy-2-methylpropoxy)-4-(6-(3-(3-nitro-1*H*-pyrazol-1-yl)azetidin-1-yl)pyridin-3-yl)pyrazolo[1,5-*a*]pyridine-3-carbonitrile (200 mg, 0.42 mmol) was dissolved in tetrahydrofuran (20 mL); and iron powder (235 mg, 4.22 mmol), ammonium chloride (225 mg, 4.22 mmol) and water (10 mL) were added thereto. The reaction mixture was stirred at 80 °C for 1 hour. Then ethyl acetate was added to the reaction mixture and filtered. The filtrate was dried and evaporated to dryness. The crude product was purified by prep-HPLC to obtain 4-(6-(3-(3-amino-1*H*-pyrazol-1-yl)azetidin-1-yl)pyridin-3-yl)-6-(2-hydroxy-2-methylpropoxy)pyrazolo[1,5-*a*]pyridine-3-carbonitrile (75 mg, yield: 40%).

MS m/z (ESI): 445.2[M+H]⁺.

### [Not according to the invention] Embodiment 70

### N-(1-(1-(5-(3-cyano-6-(2-hydroxy-2-methylpropoxy)pyrazolo[1,5-a]pyridin-4-yl)pyridin-2-yl)azetidin-3-yl)-1H-pyrazol-3-yl)cyclopropanecarboxamide

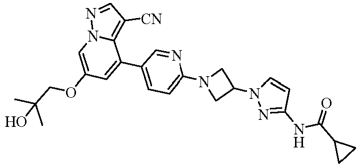

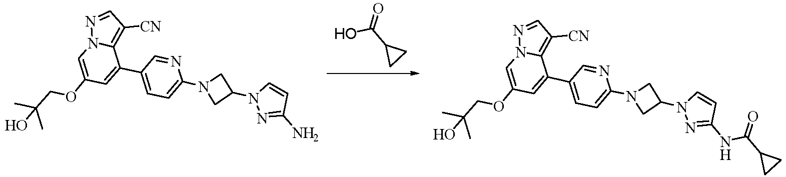

*N*-(1-(1-(5-(3-cyano-6-(2-hydroxy-2-methylpropoxy)pyrazolo[1,5-*a*]pyridin-4-yl)pyridin-2-yl)azetidin-3-yl)-1*H*-pyrazol-3-yl)cyclopropanecarboxamide (85 mg, 72%) was obtained by using 4-(6-(3-(3-amino-1*H*-pyrazol-1-yl)azetidin-1-yl)pyridin-3-yl)-6-(2-hydroxy-2-methylpropoxy)pyrazolo[1,5-*a*]pyridine-3-carbonitrile and cyclopropanecarboxylic acid as raw materials with reference to step 3 of embodiment 40.

MS m/z (ESI): 513.2[M+H]⁺.

### [Not according to the invention] Embodiment 71

### 1-(1-(5-(3-Cyano-6-(2-hydroxy-2-methylpropoxy)pyrazolo[1,5-a]pyridin-4-yl)pyridin-2-yl)azetidin-3-yl)-N-cyclopropyl-1H-pyrazole-3-carboxamide

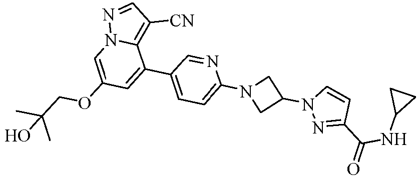

### Step 1: preparation of 1-diphenylmethylazetidin-3-yl methanesulfonate

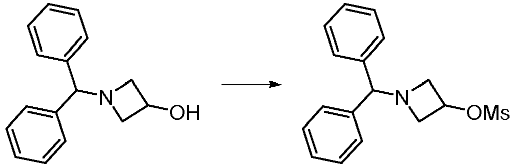

1-Diphenylmethylazetidin-3-yl methanesulfonate (10 g, yield: 95%) was obtained by using 1-diphenylmethylazetidin-3-ol as raw material with reference to step 3 of embodiment 66.

MS m/z (ESI): 318.2[M+H]⁺.

### Step 2: preparation of methyl 1-(1-diphenylmethylazetidin-3-yl)-1H-pyrazole-3-carboxylate

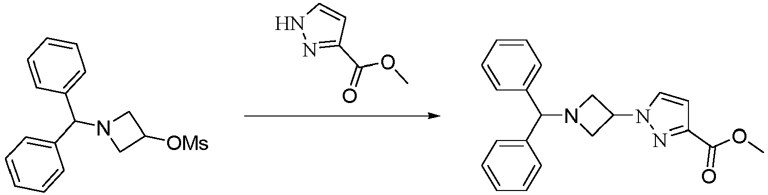

Methyl 1-(1-diphenylmethylazetidin-3-yl)-1*H*-pyrazole-3-carboxylate (3.5 g, yield: 40%) was obtained by using 1-diphenylmethylazetidin-3-yl methanesulfonate and methyl 1*H*-pyrazole-3-carboxylate as raw materials with reference to step 5 of embodiment 37.

MS m/z (ESI): 348.2[M+H]⁺.

### Step 3: preparation of 1-(1-diphenylmethylazetidin-3-yl)-1H-pyrazole-3-carboxylic acid

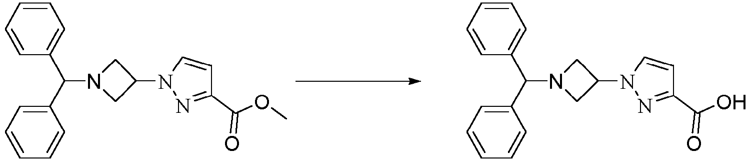

1-(1-Diphenylmethylazetidin-3-yl)-1*H*-pyrazole-3-carboxylic acid (2.0 g, yield: 75%) was obtained by using methyl 1-(1-diphenylmethylazetidin-3-yl)-1*H*-pyrazole-3-carboxylate as raw material with reference to step 2 of embodiment 40.

MS m/z (ESI): 334.2[M+H]⁺.

### Step 4: preparation of 1-(1-diphenylmethylazetidin-3-yl)-N-cyclopropyl-1H-pyrazole-3-carboxamide

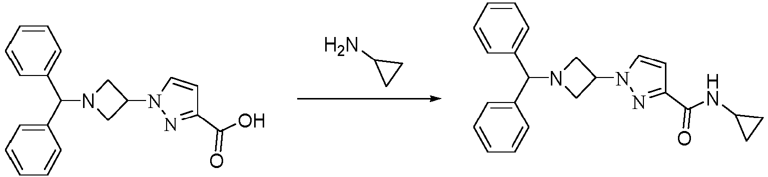

1-(1-Diphenylmethylazetidin-3-yl)-*N*-cyclopropyl-1*H*-pyrazole-3-carboxamide (500 mg, yield: 71%) was obtained by using 1-(1-diphenylmethylazetidin-3-yl)-1*H*-pyrazole-3-carboxylic acid as raw material with reference to step 3 of embodiment 40.

MS m/z (ESI): 373.2[M+H]⁺.

### Step 5: preparation of 1-(azetidin-3-yl)-N-cyclopropyl-1H-pyrazole-3-carboxamide

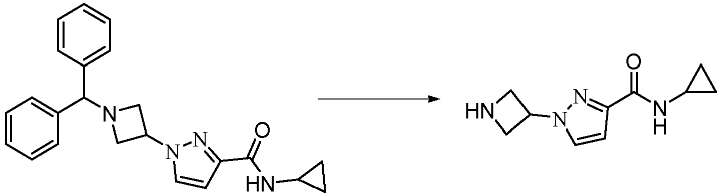

1-(azetidin-3-yl)-*N*-cyclopropyl-1*H*-pyrazole-3-carboxamide (250 mg, yield: 72%) was obtained by using 1-(1-diphenylmethylazetidin-3-yl)-*N*-cyclopropyl-1*H*-pyrazole-3-carboxamide as raw material with reference to step 4 of embodiment 42.

MS m/z (ESI): 207.2[M+H]⁺.

### Step 6: preparation of 1-(1-(5-(3-cyano-6-(2-hydroxy-2-methylpropoxy)pyrazolo[1,5-a]pyridin-4-yl)pyridin-2-yl)azetidin-3-yl)-N-cyclopropyl-1H-pyrazole-3-carboxamide

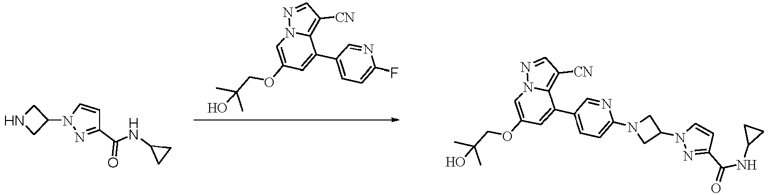

1-(1-(5-(3-Cyano-6-(2-hydroxy-2-methylpropoxy)pyrazolo[1,5-*a*]pyridin-4-yl)pyridin-2-yl)azetidin-3-yl)-*N*-cyclopropyl-1*H*-pyrazole-3-carboxamide was obtained by using 1-(azetidin-3-yl)-*N-*cyclopropyl-1*H*-pyrazole-3-carboxamide and 4-(6-fluoropyridin-3-yl)-6-(2-hydroxy-2-methylpropoxy)pyrazolo[1,5-a]pyridine-3-carbonitrile as raw materials with reference to step 6 of embodiment 42.

MS m/z (ESI): 513.2[M+H]⁺.

### [Not according to the invention] Embodiment 72

### 6-(2-Hydroxy-2-methylpropoxy)-4-(6-(5-((6-methoxypyridin-3-yl)oxy)hexahydrocyclopenta[c]pyrrol-2(1H)-yl)pyridin-3-yl)pyrazolo[1,5-a]pyridine-3-carbonitrile

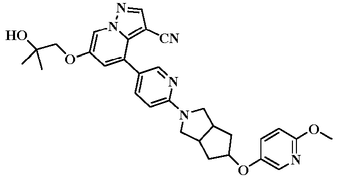

### Step 1: tert-butyl 5-((6-methoxypyridin-3-yl)oxy)hexahydrocyclopenta[c]pyrrole-2(1H)-carboxylate

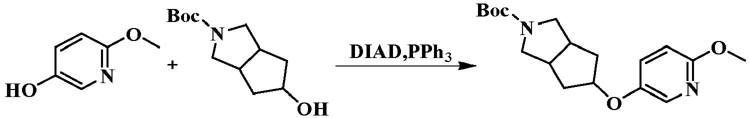

*Tert-butyl* 5-((6-methoxypyridin-3-yl)oxy)hexahydrocyclopenta[*c*]pyrrole-2(1*H*)-carboxylate was obtained by using 6-methoxypyridin-3-ol and *tert-butyl* 5-hydroxyhexahydrocyclopenta[c]pyrrole-2(1H)-carboxylate as raw material with reference to step 1 of embodiment 34.

MS m/z (ESI):335.2[M+H]⁺.

### Step 2: 5-((6-methoxypyridin-3-yl)oxy)octahydrocyclopenta[c]pyrrole

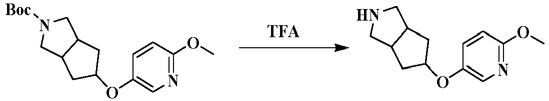

5-((6-Methoxypyridin-3-yl)oxo)octahydrocyclopenta[*c*]pyrrole was obtained by using *tert-butyl* 5-((6-methoxypyridin-3-yl)oxo)hexahydrocyclopenta[*c*]pyrrole-2(1*H*)-carboxylate as raw material with reference to step 2 of embodiment 57.

MS m/z (ESI):235.1[M+H]⁺.

### Step 3: 6-(2-hydroxy-2-methylpropoxy)-4-(6-(5-((6-methoxypyridin-3-yl)oxo)hexahydrocyclopenta[c]pyrrol-2(1H)-yl)pyridin-3-yl)pyrazolo[1,5-a]pyridine-3-carbonitrile

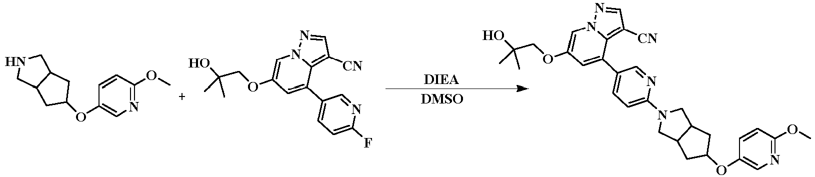

6-(2-Hydroxy-2-methylpropoxy)-4-(6-(5-((6-methoxypyridin-3-yl)oxo)hexahydrocyclopenta[*c*]pyrrol-2(1*H*)-yl)pyridin-3-yl)pyrazolo[1,5-*a*]pyridine-3-carbonitrile was obtained by using 5-((6-methoxypyridin-3-yl)oxo)octahydrocyclopenta[c]pyrrole as raw material with reference to step 2 of embodiment 8.

MS m/z (ESI)541.2[M+H]⁺ .

### [Not according to the invention] Embodiment 73

### 6-(2-Hydroxy-2-methylpropoxy)-4-(6-(5-(pyridin-3-ylmethoxy)hexahydrocyclopenta[c]pyrrol-2(1H)-yl)pyridin-3-yl)pyrazolo[1,5-a]pyridine-3-carbonitrile

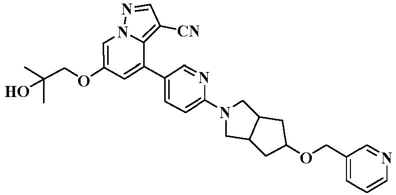

### Step 1: tert-butyl 5-(pyridin-3-ylmethoxy)hexahydrocyclopenta[c]pyrrole-2(1H)-carboxylate

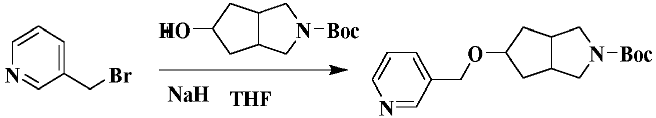

*Tert-butyl* 5-hydroxyhexahydrocyclopenta[c]pyrrole-2(1*H*)-carboxylate (660 mg, 2.9 mmol) was dissolved in tetrahydrofuran (10 mL), and sodium hydride (140 mg, 3.5 mmol, 60%) was added under stirring, then the reaction mixture was stirred at room temperature for 1 hour. Then, 3-(bromomethyl)pyridine (500 mg, 2.9 mmol) was added to the reaction mixture, and the stirring was continued for 12 hours. Water (5 mL) was added thereto, and the reaction mixture was extracted with ethyl acetate (10 mL) and washed with saturated sodium chloride solution (5 mL x 2); the organic phase was dried over anhydrous sodium sulfate, concentrated under reduced pressure and purified by column chromatography (dichloromethane/methanol: 30/1) to obtain *tert*-butyl 5-(pyridin-3-ylmethoxy)hexahydrocyclopenta[*c*]pyrrole-2(1*H*)-carboxylate (550 mg, white solid, 59.5%).

MS m/z (ESI): 319.2 [M+H]⁺.

### Step 2: 5-(pyridin-3-ylmethoxy)octahydrocyclopenta[c]pyrrole

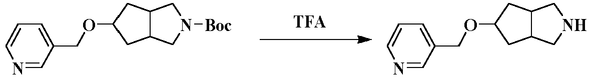

5-(Pyridin-3-ylmethoxy)octahydrocyclopenta[*c*]pyrrole was obtained by using *tert*-butyl 5-(pyridin-3-ylmethoxy)hexahydrocyclopenta[*c*]pyrrole-2(1*H*)-carboxylate as raw material with reference to step 2 of embodiment 57.

MS m/z (ESI)219.2[M+H]⁺.

### Step 3: 6-(2-hydroxy-2-methylpropoxy)-4-(6-(5-(pyridin-3-ylmethoxy)hexahydrocyclopenta[c]pyrrol-2(1H)-yl)pyridin-3-yl)pyrazolo[1,5-a]pyridine-3-carbonitrile

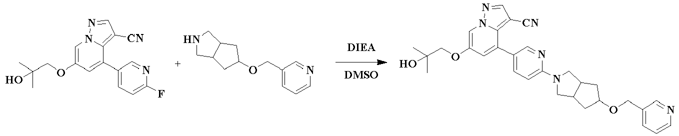

6-(2-Hydroxy-2-methylpropoxy)-4-(6-(5-(pyridin-3-ylmethoxy)hexahydrocyclopenta[c]pyrrol-2(1*H*)-yl)pyridin-3-yl)pyrazolo[1,5-*a*]pyridine-3-carbonitrile was obtained by using 5-(pyridin-3-ylmethoxy)octahydrocyclopenta[c]pyrrole as raw material with reference to step 2 of embodiment 8.

MS m/z (ESI)525.2[M+H]⁺ .

### [Not according to the invention] Embodiment 74

### 6-(2-Hydroxy-2-methylpropoxy)-4-(6-(5-(((6-methoxypyridin-3-yl)methyl)amino)-5-methylhexahydrocyclopenta[c]pyrrol-2(1H)-yl)pyridin-3-yl)pyrazolo[1,5-a]pyridine-3-carbonitrile

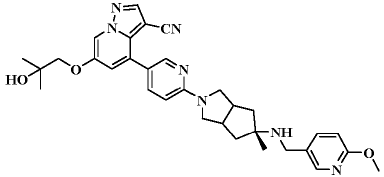

### Step 1: 2-(5-bromopyridin-2-yl)hexahydrocyclopenta[c]pyrrol-5(1H)-one

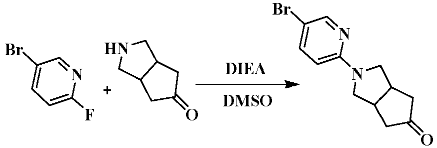

2-(5-Bromopyridin-2-yl)hexahydrocyclopenta[*c*]pyrrol-5(1*H*)-one was obtained by using 5-bromo-2-fluoropyridine and hexahydrocyclopenta[*c*]pyrrol-5(1*H*)-one as raw material with reference to step 2 of embodiment 8.

MS m/z (ESI)281.0[M+H]⁺.

### Step 2: 2-(5-bromopyridin-2-yl)-5-methyleneoctahydrocyclopenta[c]pyrrole

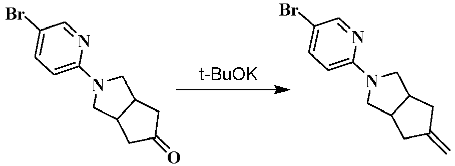

Methyltriphenylphosphine bromide (870 mg, 2.45 mmol) was dissolved in tetrahydrofuran (10 mL), the mixture was replaced with N₂, cooled to 0°C; and potassium *tert*-butoxide (330 mg, 2.93 mmol) was added thereto, the reaction was stirred at room temperature for 2 hours. The mixture was cooled to 0 °C, a solution of 2-(5-bromopyridin-2-yl)hexahydrocyclopenta[*c*]pyrrol-5(1*H*)-one (450 mg, 1.60 mmol) in tetrahydrofuran (10 mL) was added thereto. The reaction was stirred at room temperature for 2 hours, and then stirred at 50 °C overnight. The reaction was quenched with water (10 mL), the aqueous phase was extracted with ethyl acetate (10 mL); the organic phases were combined, dried over anhydrous sodium sulfate, filtered, concentrated, and the crude product was purified by column chromatography (petroleum ether/ethyl acetate: 1/1) to obtain 2-(5-bromopyridin-2-yl)-5-methyleneoctahydrocyclopenta[*c*]pyrrole (300 mg, yield: 67%).

MS m/z (ESI)279.0[M+H]⁺.

### Step 3: N-(2-(5-bromopyridin-2-yl)-5-methyloctahydrocyclopenta[c]pyrrol-5-yl)formamide

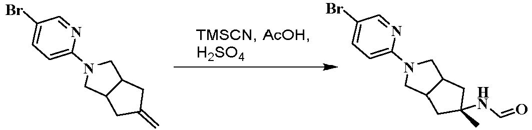

2-(5-Bromopyridin-2-yl)-5-methyleneoctahydrocyclopenta[*c*]pyrrole (300 mg, 1.07 mmol) was dissolved in dichloromethane (30 mL), acetic acid (180 mg, 3.0 mmol) was added thereto, and the mixture was cooled to 0 °C, trimethylcyanosilane (200 mg, 2.0 mmol) was added thereto dropwise, and the reaction was stirred at 0 °C for half an hour. Then a mixed solution of acetic acid (180 mg, 3.0 mmol) and concentrated sulfuric acid (400 mg, 4.0 mmol) was added slowly dropwise and the reaction was stirred overnight at 0 °C to room temperature. At 0 °C, 3 M sodium hydroxide aqueous solution was added until Ph>12. The mixture was separated, the aqueous phase was extracted with ethyl acetate (20 mL); and the organic phases were combined, dried over anhydrous sodium sulfate, filtered and concentrated to obtain *N*-(2-(5-bromopyridin-2-yl)-5-methyloctahydrocyclopenta[*c*]pyrrol-5-yl)formamide (230 mg, yield: 66.0%).

MS m/z (ESI): 324.0 [M+H]⁺.

### Step 4: 2-(5-bromopyridin-2-yl)-5-methyloctahydrocyclopenta[c]pyrrol-5-amine

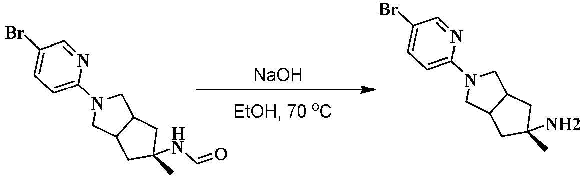

N-(2-(5-bromopyridin-2-yl)-5-methyloctahydrocyclopenta[c]pyrrol-5-yl)formamide (230 mg, 0.7 mmol) was dissolved in ethanol (5 mL); sodium hydroxide (80 mg, 2.1 mmol) and water (2 mL) were added thereto, and the reaction was stirred at 70 °C overnight. The mixture was evaporated to dryness to remove the solvent, water (5 mL) was added thereto; and the aqueous phase was extracted with ethyl acetate (5 mL x 2), the organic phase was washed with 1 M hydrochloric acid aqueous solution (5 mL), the aqueous phase was adjusted to pH > 12 with 3 M sodium hydroxide aqueous solution, then the aqueous phase was extracted with ethyl acetate (5 mL x 2); and the organic phases were combined, dried over anhydrous sodium sulfate, filtered and concentrated to obtain 2-(5-bromopyridin-2-yl)-5-methyl-octahydrocyclopenta[*c*]pyrrol-5-amine (150 mg, yield: 71%).

MS m/z (ESI): 296.0 [M+H]⁺.

### Step 5: 2-(5-bromopyridin-2-yl)-N-((6-methoxypyridin-3-yl)methyl)-5-methyloctahydrocyclopenta[c]pyrrol-5-amine

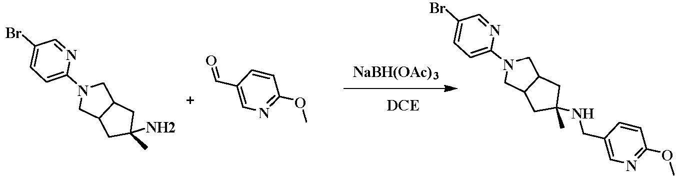

2-(5-Bromopyridin-2-yl)-*N*-((6-methoxypyridin-3-yl)methyl)-5-methyloctahydrocyclopenta[*c*]pyrrol-5-amine was obtained by using 2-(5-bromopyridin-2-yl)-5-methyloctahydrocyclopenta[*c*]pyrrol-5-amine as raw material with reference to step 3 of embodiment 57.

MS m/z (ESI): 417.1 [M+H]⁺.

### Step 6: N-((6-methoxypyridin-3-yl)methyl)-5-methyl-2-(5-(4,4,5,5-tetramethyl-1,3,2-dioxaborolan-2-yl)pyridin-2-yl)octahydrocyclopenta[c]pyrrol-5-amine

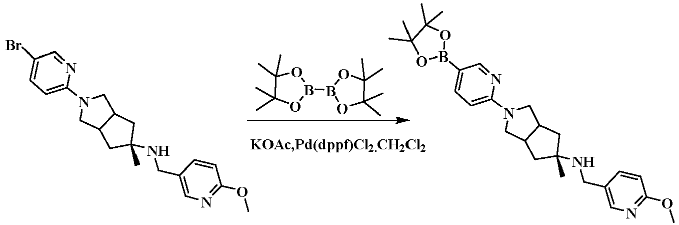

*N*-((6-methoxypyridin-3-yl)methyl)-5-methyl-2-(5-(4,4,5,5-tetramethyl-1,3,2-dioxaborolan-2-yl)pyridin-2-yl)octahydrocyclopenta[c]pyrrol-5-amine was obtained by using 2-(5-bromopyridin-2-yl)-*N*-((6-methoxypyridin-3-yl)methyl)-5-methyloctahydrocyclopenta[*c*]pyrrol-5-amine as raw material with reference to step 4 of embodiment 57.

MS m/z (ESI): 465.3 [M+H]⁺.

### Step 7: 6-(2-hydroxy-2-methylpropoxy)-4-(6-(5-(((6-methoxypyridin-3-yl)methyl)amino)-5-methylhexahydrocyclopenta[c]pyrrol-2(1H)-yl)pyridin-3-yl)pyrazolo[1,5-a]pyridine-3-carbonitrile

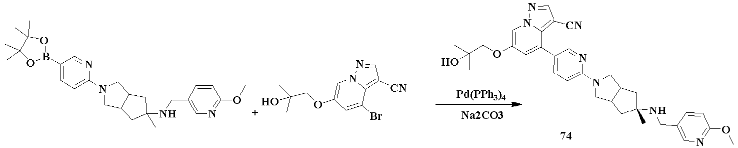

6-(2-Hydroxy-2-methylpropoxy)-4-(6-(5-(((6-methoxypyridin-3-yl)methyl)amino)-5-methylhexahydrocyclopenta[*c*]pyrrol-2(1*H*)-yl)pyridin-3-yl)pyrazolo[1,5-a]pyridine-3-carbonitrile was obtained by using *N*-((6-methoxypyridin-3-yl)methyl)-5-methyl-2-(5-(4,4,5,5-tetramethyl-1,3,2-dioxaborolan-2-yl)pyridin-2-yl)octahydrocyclopenta[c]pyrrol-5-amine as raw material with reference to step 3 of embodiment 7.

MS m/z (ESI):568.3[M+H]⁺.

### [Not according to the invention] Embodiment 75

### N-(2-(5-(3-cyano-6-(2-hydroxy-2-methylpropoxy)pyrazolo[1,5-a]pyridin-4-yl)pyridin-2-yl)-5-methyloctahydrocyclopenta[c]pyrrol-5-yl)-5-fluoro-2-methylbenzamide

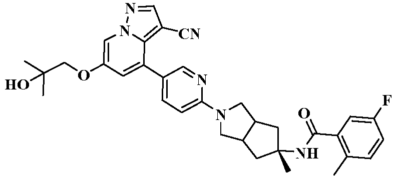

### Step 1: N-(2-(5-bromopyridin-2-yl)-5-methyloctahydrocyclopenta[c]pyrrol-5-yl)-5-fluoro-2-methylbenzamide

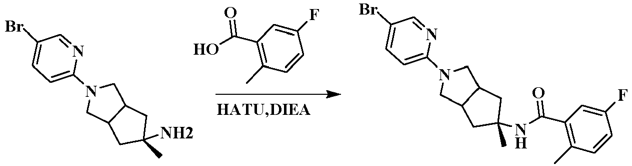

2-(5-Bromopyridin-2-yl)-5-methyloctahydrocyclopenta[*c*]pyrrol-5-amine (300 mg, 1.0 mmol), 5-fluoro-2-methylbenzoic acid (156 mg, 1.0 mmol), 2-(7-azabenzotriazol-1-yl)-*N*,*N*,*N*',*N*'-tetramethyluronium hexafluorophosphate (380 mg, 1.0 mmol), diisopropylethylamine (390 mg, 3 mmol), and dimethylformamide (5 mL) were added to a 25 mL three-necked flask sequentially. The reaction mixture was stirred at room temperature for 5 hours, then dissolved in ethyl acetate (10 mL) and washed with saturated saline (5 mLx3), and the organic phase was dried over anhydrous sodium sulfate, filtered and evaporated to dryness. The crude product was purified by column chromatography to obtain *N*-(2-(5-bromopyridin-2-yl)-5-methyloctahydrocyclopenta[*c*]pyrrol-5-yl)-5-fluoro-2-methylbenzamide (260 mg, yield: 59.3%).

MS m/z (ESI):432.1[M+H]⁺.

### Step 2: 5-fluoro-2-methyl-N-(5-methyl-2-(5-(4,4,5,5-tetramethyl-1,3,2-dioxaborolan-2-yl)pyridin-2-yl)octahydrocyclopenta[c]pyrrol-5-yl)benzamide

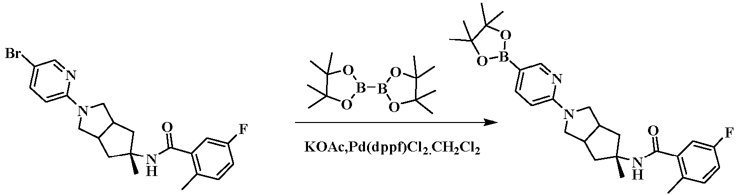

5-Fluoro-2-methyl-N-(5-methyl-2-(5-(4,4,5,5-tetramethyl-1,3,2-dioxaborolan-2-yl)pyridin-2-yl)octahydrocyclopenta[c]pyrrol-5-yl)benzamide was obtained by using *N*-(2-(5-bromopyridin-2-yl)-5-methyloctahydrocyclopenta[*c*]pyrrol-5-yl)-5-fluoro-2-methylbenzamide as raw material with reference to step 4 of embodiment 57.

MS m/z (ESI): 480.2 [M+H]⁺.

### Step 3: N-(2-(5-(3-cyano-6-(2-hydroxy-2-methylpropoxy)pyrazolo[1,5-a]pyridin-4-yl)pyridin-2-yl)-5-methyloctahydrocyclopenta[c]pyrrol-5-yl)-5-fluoro-2-methylbenzamide

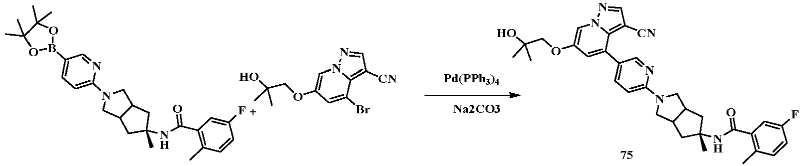

*N*-(2-(5-(3-cyano-6-(2-hydroxy-2-methylpropoxy)pyrazolo[1,5-*a*]pyridin-4-yl)pyridin-2-yl)-5-methyloctahydrocyclopenta[*c*]pyrrol-5-yl)-5-fluoro-2-methylbenzamide was obtained by using 5-fluoro-2-methyl-*N*-(5-methyl-2-(5-(4,4,5,5-tetramethyl-1,3,2-dioxaborolan-2-yl)pyridin-2-yl)octahydrocyclopenta[c]pyrrol-5-yl)benzamide as raw material with reference to step 3 of embodiment 7.

MS m/z (ESI):583.2[M+H]⁺.

### [Not according to the invention] Embodiment 76

### 2-Chloro-N-(2-(5-(3-cyano-6-(2-hydroxy-2-methylpropoxy)pyrazolo[1,5-a]pyridin-4-yl)pyridin-2-yl)-5-methyloctahydrocyclopenta[c]pyrrol-5-yl)benzamide

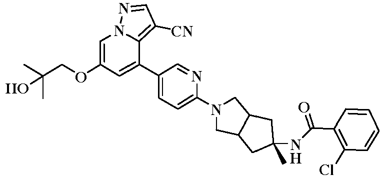

### Step 1: N-(2-(5-bromopyridin-2-yl)-5-methyloctahydrocyclopenta[c]pyrrol-5-yl)-2-chlorobenzamide

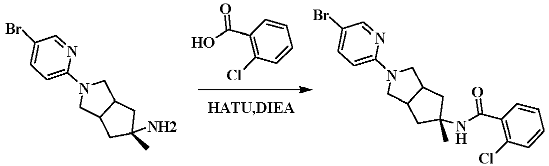

*N*-(2-(5-bromopyridin-2-yl)-5-methyloctahydrocyclopenta[*c*]pyrrol-5-yl)-2-chlorobenzamide was obtained by using 2-(5-bromopyridin-2-yl)-5-methyloctahydrocyclopenta[*c*]pyrrol-5-amine and 2-chlorobenzoic acid as raw materials with reference to step 1 of embodiment 75.

MS m/z (ESI):434.0[M+H]⁺.

### Step 2: 2-chloro-N-(5-methyl-2-(5-(4,4,5,5-tetramethyl-1,3,2-dioxaborolan-2-yl)pyridin-2-yl)octahydrocyclopenta[c]pyrrol-5-yl)benzamide

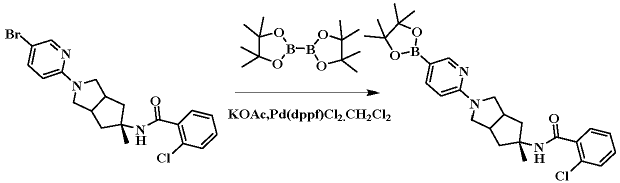

2-Chloro-*N*-(5-methyl-2-(5-(4,4,5,5-tetramethyl-1,3,2-dioxaborolan-2-yl)pyridin-2-yl)octahydrocyclopenta[c]pyrrol-5-yl)benzamide was obtained by using *N*-(2-(5-bromopyridin-2-yl)-5-methyloctahydrocyclopenta[*c*]pyrrol-5-yl)-2-chlorobenzamide as raw material with reference to step 4 of embodiment 57.

MS m/z (ESI): 482.2 [M+H]⁺.

### Step 3: 2-chloro-N-(2-(5-(3-cyano-6-(2-hydroxy-2-methylpropoxy)pyrazolo[1,5-a]pyridin-4-yl)pyridin-2-yl)-5-methyloctahydrocyclopenta[c]pyrrol-5-yl)benzamide

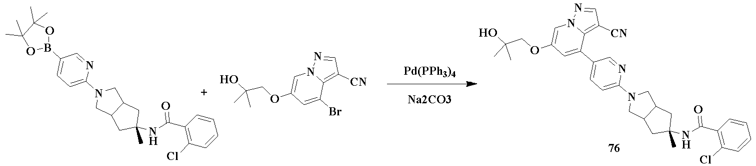

2-Chloro-N-(2-(5-(3-cyano-6-(2-hydroxy-2-methylpropoxy)pyrazolo[1,5-*a*]pyridin-4-yl)pyridin-2-yl)-5-methyloctahydrocyclopenta[*c*]pyrrol-5-yl)benzamide was obtained by using 2-chloro-*N*-(5-methyl-2-(5-(4,4,5,5-tetramethyl-1,3,2-dioxaborolan-2-yl)pyridin-2-yl)octahydrocyclopenta[*c*]pyrrol-5-yl)benzamide as raw material with reference to step 3 of embodiment 7.

MS m/z (ESI):585.2[M+H]⁺.

### [Not according to the invention] Embodiment 77

### N-(2-(5-(3-cyano-6-(2-hydroxy-2-methylpropoxy)pyrazolo[1,5-a]pyridin-4-yl)pyridin-2-yl)-5-methyloctahydrocyclopenta[c]pyrrol-5-yl)-3-fluoro-6-methylpicolinamide

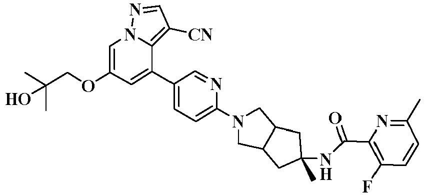

### Step 1: N-(2-(5-bromopyridin-2-yl)-5-methyloctahydrocyclopenta[c]pyrrol-5-yl)-3-fluoro-6-methylpicolinamide

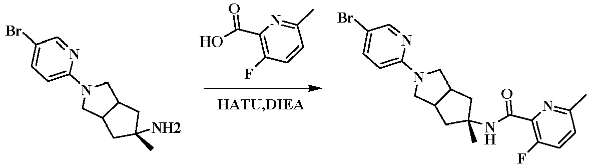

*N*-(2-(5-bromopyridin-2-yl)-5-methyloctahydrocyclopenta[*c*]pyrrol-5-yl)-3-fluoro-6-methylpicolinamide was obtained by using 2-(5-bromopyridin-2-yl)-5-methyloctahydrocyclopenta[*c*]pyrrol-5-amine and 3-fluoro-6-methylpicolinic acid as raw material with reference to step 1 of embodiment 75.

MS m/z (ESI):433.0[M+H]⁺.

### Step 2: 3-fluoro-6-methyl-N-(5-methyl-2-(5-(4,4,5,5-tetramethyl-1,3,2-dioxaborolan-2-yl)pyridin-2-yl)octahydrocyclopenta[c]pyrrol-5-yl)picolinamide

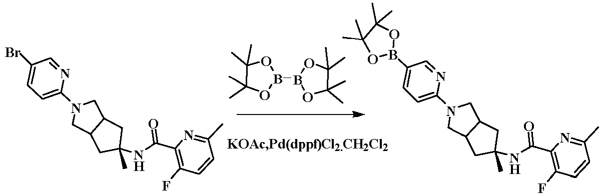

3-Fluoro-6-methyl-*N*-(5-methyl-2-(5-(4,4,5,5-tetramethyl-1,3,2-dioxaborolan-2-yl)pyridin-2-yl)octahydrocyclopenta[c]pyrrol-5-yl)picolinamide was obtained by using *N*-(2-(5-bromopyridin-2-yl)-5-methyloctahydrocyclopenta[*c*]pyrrol-5-yl)-3-fluoro-6-methylpicolinamide as raw material with reference to step 4 of embodiment 57.

MS m/z (ESI): 481.2 [M+H]⁺.

### Step 3: N-(2-(5-(3-cyano-6-(2-hydroxy-2-methylpropoxy)pyrazolo[1,5-a]pyridin-4-yl)pyridin-2-yl)-5-methyloctahydrocyclopenta[c]pyrrol-5-yl)-3-fluoro-6-methylpicolinamide

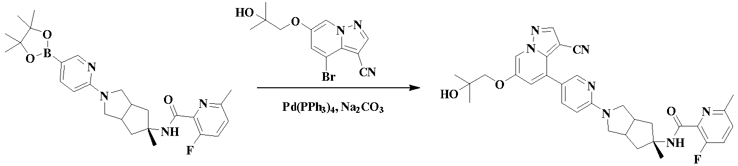

N-(2-(5-(3-cyano-6-(2-hydroxy-2-methylpropoxy)pyrazolo[1,5-a]pyridin-4-yl)pyridin-2-yl)-5-methyloctahydrocyclopenta[*c*]pyrrol-5-yl)-3-fluoro-6-methylpicolinamide was obtained by using 3-fluoro-6-methyl-N-(5-methyl-2-(5-(4,4,5,5-tetramethyl-1,3,2-dioxaborolan-2-yl)pyridin-2-yl)octahydrocyclopenta[*c*]pyrrol-5-yl)picolinamide as raw material with reference to step 3 of embodiment 7.

MS m/z (ESI):584.2[M+H]⁺.

### [Not according to the invention] Embodiment 78

### 6-(2-Hydroxy-2-methylpropoxy)-4-(6-((1R,5S)-3-((6-methoxypyridin-3-yl)oxo)-8-azabicyclo[3.2.1]octan-8-yl)pyridin-3-yl)pyrazolo[1,5-a]pyridine-3-carbonitrile

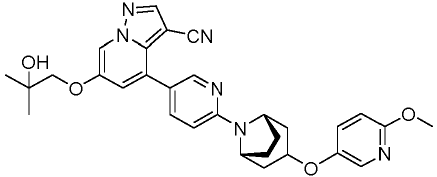

### Step 1: tert-butyl (1R,5S)-3-((6-methoxypyridin-3-yl)oxo)-8-azabicyclo[3.2.1]octane-8-carboxylate

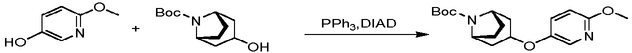

*Tert-butyl* (1*R*,5*S*)-3-((6-methoxypyridin-3-yl)oxo)-8-azabicyclo[3.2.1]octane-8-carboxylate was obtained by using 6-methoxypyridin-3-ol and *tert*-butyl (1*R*,5*S*)-3-hydroxy-8-azabicyclo[3.2.1]octane-8-carboxylate as raw materials with reference to step 1 of embodiment 34.

MS m/z (ESI):335.1 [M+H]⁺.

### Step 2: (1R,5S)-3-((6-methoxypyridin-3-yl)oxo)-8-azabicyclo[3.2.1]octane

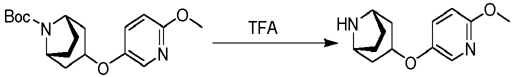

(1*R*,5*S*)-3-((6-methoxypyridin-3-yl)oxo)-8-azabicyclo[3.2.1]octane was obtained by using *tert-butyl* (1R,5*S*)-3-((6-methoxypyridin-3-yl)oxo)-8-azabicyclo[3.2.1]octane-8-carboxylate as raw material with reference to step 2 of embodiment 57.

MS m/z (ESI):235.1[M+H]⁺.

### Step 3: 6-(2-hydroxy-2-methylpropoxy)-4-(6-((1R,5S)-3-((6-methoxypyridin-3-yl)oxo)-8-azabicyclo[3.2.1]octan-8-yl)pyridin-3-yl)pyrazolo[1,5-a]pyridine-3-carbonitrile

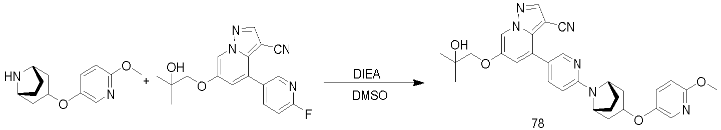

The product 6-(2-hydroxy-2-methylpropoxy)-4-(6-((1*R*,5*S*)-3-((6-methoxypyridin-3-yl)oxo)-8-azabicyclo[3.2.1]octan-8-yl)pyridin-3-yl)pyrazolo[1,5-*a*]pyridine-3-carbonitrile was obtained by using (1*R*,5*S*)-3-((6-methoxypyridin-3-yl)oxo)-8-azabicyclo[3.2.1]octane and 4-(6-fluoropyridin-3-yl)-6-(2-hydroxy-2-methylpropoxy)pyrazolo[1,5-*a*]pyridine-3-carbonitrile as raw materials with reference to step 2 of embodiment 8.

MS m/z (ESI):541.2[M+H]⁺.

### [Not according to the invention] Embodiment 79

### 4-(6-(1-((5-Fluoro-6-methoxypyridin-3-yl)methyl)-2,5-dihydro-1H-pyrrol-3-yl)pyridin-3-yl)-6-(2-hydroxyethoxy)pyrazolo[1,5-a]pyridine-3-carbonitrile

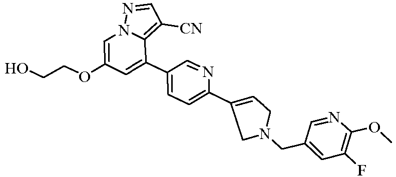

### Step 1: tert-butyl 3-hydroxy-3-(5-(4,4,5,5-tetramethyl-1,3,2-dioxaborolan-2-yl)pyridin-2-yl)pyrrolidine-1-carboxylate

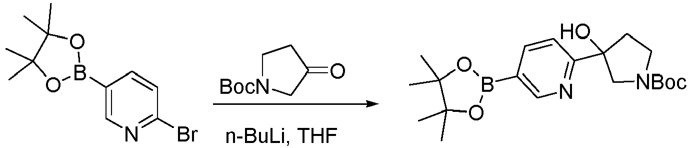

2-Bromo-5-(4,4,5,5-tetramethyl-1,3,2-dioxaborolan-2-yl)pyridine (500 mg, 1.7 mmol) was dissolved in 20 mL of THF, n-BuLi (1 mL, 2.6 mmol) was added thereto at -78 °C, the mixture was stirred at - 78 °C for half an hour; and *tert*-butyl 3-oxopyrrolidine-1 -carboxylate was added thereto at -78 °C, the mixture was stirred from -78 °C to room temperature for 2 hours, 10 mL of ammonium chloride aqueous solution was added, and the mixture was extracted with ethyl acetate (20 mL*3). The organic phase was washed with saturated saline, dried over anhydrous sodium sulfate. The residue was filtered, evaporated to dryness; and the crude product was separated by column chromatography (eluted with petroleum ether/ethyl acetate = 2/1) to obtain *tert-butyl* 3-hydroxy-3-(5-(4,4,5,5-tetramethyl-1,3,2-dioxaborolan-2-yl)pyridin-2-yl)pyrrolidine-1-carboxylate (379 mg, white solid, yield was 57%).

MS m/z (ESI): 391.2 [M+H]⁺.

### Step 2: tert-butyl 3-(5-(4,4,5,5-tetramethyl-1,3,2-dioxaborolan-2-yl)pyridin-2-yl)-2,5-dihydro-1H-pyrrole-1-carboxylate

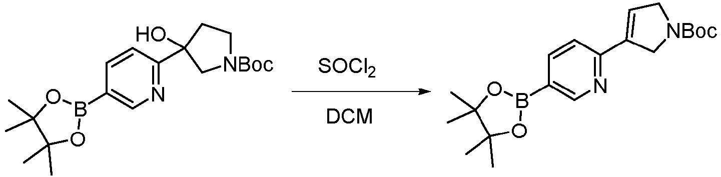

*Tert-butyl* 3-hydroxy-3-(5-(4,4,5,5-tetramethyl-1,3,2-dioxaborolan-2-yl)pyridin-2-yl)pyrrolidine-1-carboxylate (300 mg, 0.76 mmol) was dissolved in 20 mL of DCM, and SOCl₂ (1 mL) was added thereto at room temperature, and the mixture was stirred at room temperature for 2 hours, 10 mL of ammonium chloride aqueous solution was added thereto, and the mixture was extracted with ethyl acetate ( 20 mL*3). The organic phase was washed with saturated saline, dried over anhydrous sodium sulfate. The residue was filtered, evaporated to dryness; and the crude product was separated by column chromatography (eluted with petroleum ether/ethyl acetate = 3/1) to obtain *tert-butyl* 3-(5-(4,4,5,5-tetramethyl-1,3,2-dioxaborolan-2-yl)pyridin-2-yl)-2,5-dihydro-1*H*-pyrrole-1-carboxylate (197 mg, white solid, yield was 70%).

MS m/z (ESI): 373.2 [M+H]⁺.

### Step 3: tert-butyl 3-(5-(3-cyano-6-(2-hydroxyethoxy)pyrazolo[1,5-a]pyridin-4-yl)pyridin-2-yl)-2,5-dihydro-1H-pyrrole-1-carboxylate

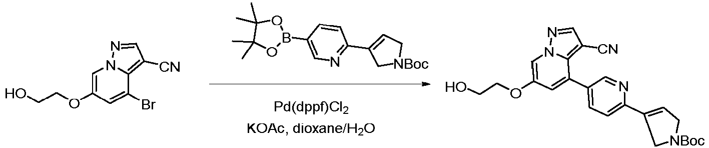

*Tert-butyl* 3-(5-(3-cyano-6-(2-hydroxyethoxy)pyrazolo[1,5-*a*]pyridin-4-yl)pyridin-2-yl)-2,5-dihydro-1*H*-pyrrole-1-carboxylate (180 mg, white solid, yield: 72%) was obtained by using 4-bromo-6-(2-hydroxyethoxy)pyrazolo[1,5-a]pyridine-3-carbonitrile and *tert-butyl* 3-(5-(4,4,5,5-tetramethyl-1,3,2-dioxaborolan-2-yl)pyridin-2-yl)-2,5-dihydro-1*H*-pyrrole-1-carboxylate as raw material with reference to step 8 of embodiment 1.

MS m/z (ESI): 448.1 [M+H]⁺.

### Step 4: 4-(6-(2,5-dihydro-1H-pyrrol-3-yl)pyridin-3-yl)-6-(2-hydroxyethoxy)pyrazolo[1,5-a]pyridine-3-carbonitrile

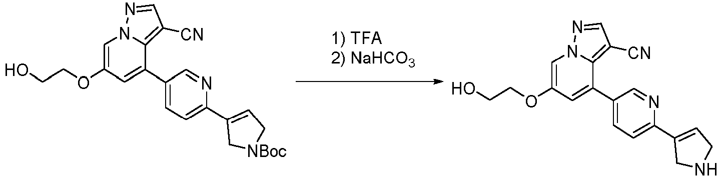

4-(6-(2,5-Dihydro-1*H*-pyrrol-3-yl)pyridin-3-yl)-6-(2-hydroxyethoxy)pyrazolo[1,5-*a*]pyridine-3-carbonitrile (120 mg, white solid, 99%) was obtained by using *tert-butyl* 3-(5-(3-cyano-6-(2-hydroxyethoxy)pyrazolo[1,5-*a*]pyridin-4-yl)pyridin-2-yl)-2,5-dihydro-1*H*-pyrrole-1-carboxylate as raw material with reference to the step 5 of embodiment **1.**

MS m/z (ESI): 348.1 [M+H]⁺.

### Step 5: 4-(6-(1-((5-fluoro-6-methoxypyridin-3-yl)methyl)-2,5-dihydro-1H-pyrrol-3-yl)pyridin-3-yl)-6-(2-hydroxyethoxy)pyrazolo[1,5-a]pyridine-3-carbonitrile

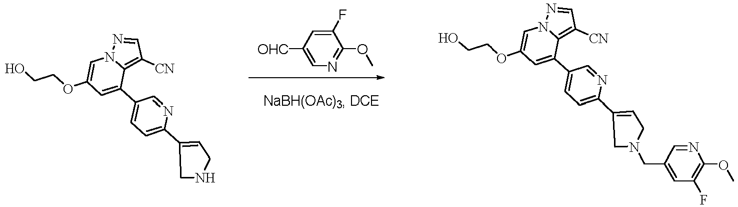

4-(6-(1-((5-Fluoro-6-methoxypyridin-3-yl)methyl)-2,5-dihydro-1*H*-pyrrol-3-yl)pyridin-3-yl)-6-(2-hydroxyethoxy)pyrazolo[1,5-a]pyridine-3-carbonitrile (34 mg, white solid, 62%) was obtained by using 4-(6-(2,5-dihydro-1*H*-pyrrol-3-yl)pyridin-3-yl)-6-(2-hydroxyethoxy)pyrazolo[1,5-a]pyridine-3-carbonitrile as raw material with reference to step 6 of embodiment **1.**

MS m/z (ESI): 487.1 [M+H]⁺.

### [Not according to the invention] Embodiment 80

### 6-(2-Hydroxy-2-methylpropoxy)-4-(6-(1-((6-methoxypyridin-3-yl)methyl)-2,5-dihydro-1H-pyrrol-3-yl)pyridin-3-yl)pyrazolo[1,5-a]pyridine-3-carbonitrile

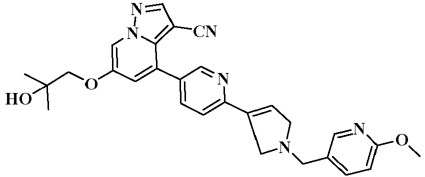

### Step 1: tert-butyl 3-(5-(3-cyano-6-(2-hydroxy-2-methylpropoxy)pyrazolo[1,5-a]pyridin-4-yl)pyridin-2-yl)-2,5-dihydro-1H-pyrrole-1-carboxylate

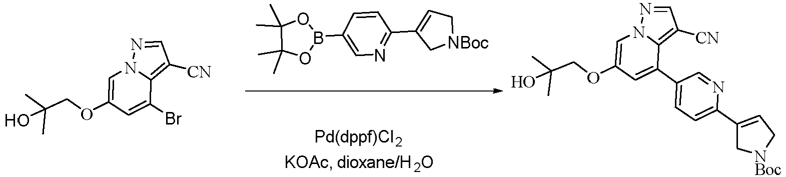

*Tert*-butyl 3-(5-(3-cyano-6-(2-hydroxy-2-methylpropoxy)pyrazolo[1,5-a]pyridin-4-yl)pyridin-2-yl)-2,5-dihydro-1*H*-pyrrole-1-carboxylate (165 mg, white solid, yield: 65%) was obtained by using 4-bromo-6-(2-hydroxy-2-methylpropoxy)pyrazolo[1,5-*a*]pyridine-3-carbonitrile and *tert-butyl* 3-(5-(4,4,5,5-tetramethyl-1,3,2-dioxaborolan-2-yl)pyridin-2-yl)-2,5-dihydro-1*H*-pyrrole-1-carboxylate as raw materials with reference to step 8 of embodiment 1.

MS m/z (ESI): 476.2 [M+H]⁺.

### Step 2: 4-(6-(2,5-dihydro-1H-pyrrol-3-yl)pyridin-3-yl)-6-(2-hydroxy-2-methylpropoxy)pyrazolo[1,5-a]pyridine-3-carbonitrile

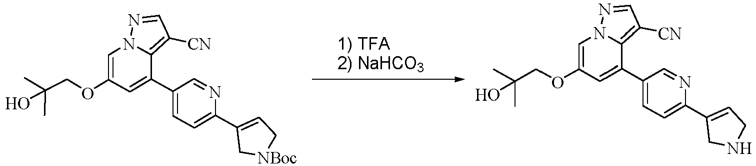

4-(6-(2,5-Dihydro-1*H*-pyrrol-3-yl)pyridin-3-yl)-6-(2-hydroxy-2-methylpropoxy)pyrazolo[1,5-a]pyridine-3-carbonitrile (82 mg, white solid, 67%) was obtained by using *tert-butyl* 3-(5-(3-cyano-6-(2-hydroxy-2-methylpropoxy)pyrazolo[1,5-a]pyridin-4-yl)pyridin-2-yl)-2,5-dihydro-1*H*-pyrrole-1-carboxylate as raw material with reference to the step 5 of embodiment **1.**

MS m/z (ESI): 376.1 [M+H]⁺.

### Step 3: 6-(2-hydroxy-2-methylpropoxy)-4-(6-(1-((6-methoxypyridin-3-yl)methyl)-2,5-dihydro-1H-pyrrol-3-yl)pyridin-3-yl)pyrazolo[1,5-a]pyridine-3-carbonitrile

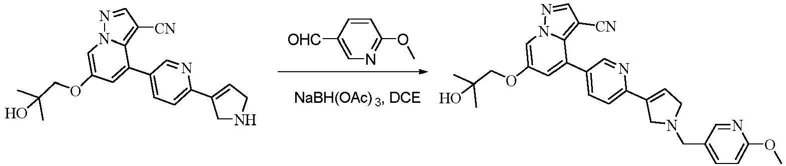

6-(2-Hydroxy-2-methylpropoxy)-4-(6-(1-((6-methoxypyridin-3-yl)methyl)-2,5-dihydro-1*H*-pyrrol-3-yl)pyridin-3-yl)pyrazolo[1,5-*a*]pyridine-3-carbonitrilee (23 mg, white solid, 42%) was obtained by using 4-(6-(2,5-dihydro-1*H*-pyrrol-3-yl)pyridin-3-yl)-6-(2-hydroxy-2-methylpropoxy)pyrazolo[1,5-a]pyridine-3-carbonitrile as raw material with reference to step 6 of embodiment **1.**

MS m/z (ESI): 497.2 [M+H]⁺.

### [Not according to the invention] Embodiment 81

### 6-(3-Hydroxy-3-methylbutyl)-4-(6-(6-((6-methoxypyridin-3-yl)methyl)-3,6-diazabicyclo[3.1.1]heptan-3-yl)pyridin-3-yl)-7-carbonyl-6,7-dihydropyrazolo[1,5a]pyrimidine-3-carbonitrile

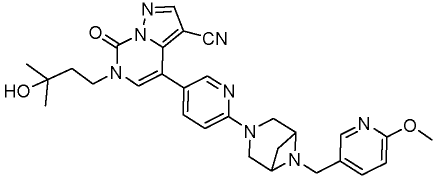

MS m/z (ESI): 541.2 [M+H]⁺.

### [Not according to the invention] Embodiment 82

### 4-(6-(6-((6-Methoxypyridin-3-yl)methyl)-3,6-diazabicyclo[3.1.1]heptan-3-yl)pyridin-3-yl)-6-(2-(methylsulfonyl)ethyl)pyrazolo[1,5-a]pyridine-3-carbonitrile

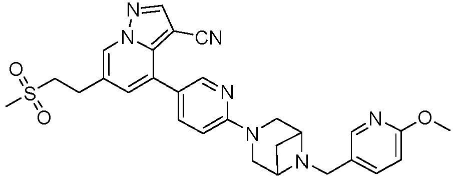

### Step 1: 3-bromo-5-(2-(methylsulfonyl)ethyl)pyridine

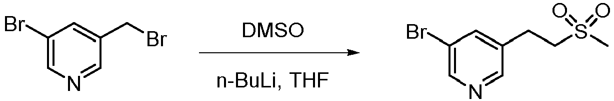

DMSO (780 mg, 10 mmol) was dissolved in 10 mL of THF, n-BuLi (4 mL, 10 mmol) was added thereto at -78 °C, and the mixture was stirred at -78 °C for 0.5 hours; 3-bromo-5-(bromomethyl)pyridine (500 mg, 2 mmol) was added at -78 °C, then the mixture was slowly warmed up to room temperature and stirred for 2 hours, 10 mL of ammonium chloride aqueous solution was added, and the mixture was extracted with ethyl acetate (20 mL*3). The organic phase was washed with saturated saline, dried over anhydrous sodium sulfate. The residue was filtered and evaporated to dryness, and the crude product was separated by column chromatography (eluted with dichloromethane/methanol = 10/1) to obtain 3-bromo-5-(2-(methylsulfonyl)ethyl)pyridine (252 mg, the yield was 48%).

MS m/z (ESI): 263.9 [M+H]⁺.

### Step 2: 4-bromo-6-(2-(methylsulfonyl)ethyl)pyrazolo[1,5-a]pyridine-3-carbonitrile

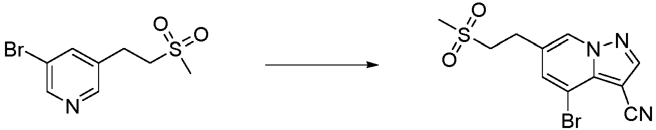

4-Bromo-6-(2-(methylsulfonyl)ethyl)pyrazolo[1,5-a]pyridine-3-carbonitrile (350 mg, white solid) was obtained by using 3-bromo-5-(2-(methylsulfonyl)ethyl)pyridine as raw material with reference to the embodiment **11.**

MS m/z (ESI): 327.9 [M+H]⁺.

### Step 3: 6-(2-(methylsulfonyl)ethyl)-4-(4,4,5,5-tetramethyl-1,3,2-dioxaborolan-2-yl)pyrazolo[1,5-a]pyridine-3-carbonitrile

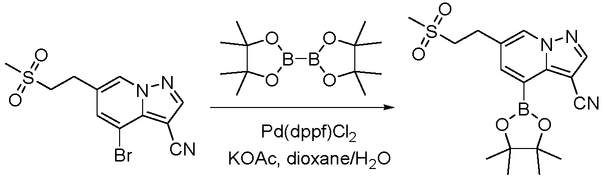

6-(2-(Methylsulfonyl)ethyl)-4-(4,4,5,5-tetramethyl-1,3,2-dioxaborolan-2-yl)pyrazolo[1,5-a]pyridine-3-carbonitrile (230 mg, white solid, 68%) was obtained by using 4-bromo-6-(2-(methylsulfonyl)ethyl)pyrazolo[1,5-a]pyridine-3-carbonitrile as raw material with reference to step 7 of embodiment **1.**

MS m/z (ESI): 376.1 [M+H]⁺.

### Step 4: 4-(6-(6-((6-methoxypyridin-3-yl)methyl)-3,6-diazabicyclo[3.1.1]heptan-3-yl)pyridin-3-yl)-6-(2-(methylsulfonyl)ethyl)pyrazolo[1,5-a]pyridine-3-carbonitrile

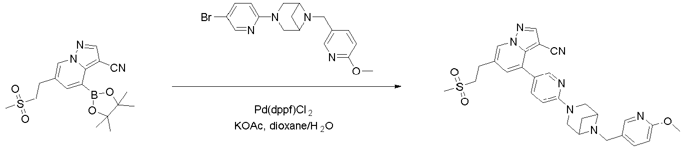

4-(6-(6-((6-Methoxypyridin-3-yl)methyl)-3,6-diazabicyclo[3.1.1]heptan-3-yl)pyridin-3-yl)-6-(2-(methylsulfonyl)ethyl)pyrazolo[1,5-*a*]pyridine-3-carbonitrile (30 mg, white solid, 48%) was obtained by using 6-(2-(methylsulfonyl)ethyl)-4-(4,4,5,5-tetramethyl-1,3,2-dioxaborolan-2-yl)pyrazolo[1,5-a]pyridine-3-carbonitrile as raw materials with reference to step 8 of embodiment **1.**

MS m/z (ESI): 544.2 [M+H]⁺.

### [Not according to the invention] Embodiment 83

### 6-(3-Hydroxy-3-methylbutyl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

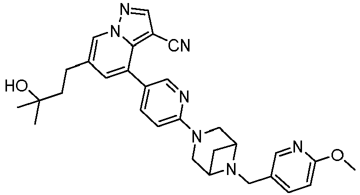

The product 6-(3-hydroxy-3-methylbutyl)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (30 mg, white solid) was obtained with reference to embodiment 82.

MS m/z (ESI): 524.2 [M+H]⁺.

### [Not according to the invention] Embodiment 84

### 6-(3-Hydroxy-3-methylbutyl)-4-(6-(4-(pyridin-2-oxy)piperidin-1-yl)pyridin-3-yl)pyrazolo[1,5-a]pyridine-3-carbonitrile

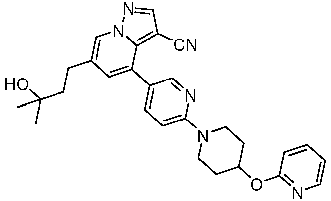

6-(3-Hydroxy-3-methylbutyl)-4-(6-(4-(pyridin-2-oxy)piperidin-1-yl)pyridin-3-yl)pyrazolo[1,5-a]pyridine-3-carbonitrile (38 mg, white solid) was obtained with reference to embodiment **82.**

MS m/z (ESI): 483.2 [M+H]⁺.

### [Not according to the invention] Embodiment 85

### 6-((1-Imino-1-hydroxy-1l6-thietan-3-yl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

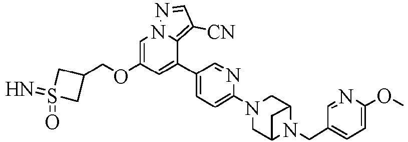

### Step 1: thietan-3-ylmethanol

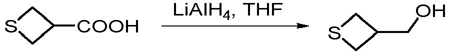

Thietane-3-carboxylic acid (500 mg, 4.2 mmol) was dissolved in 10 mL of THF, LiAlH₄(8.4 mL, 8.4 mmol) was added thereto at-78°C; and the mixture was stirred at-78°C for 2 hours, the temperature was slowly raised to room temperature and the mixture was stirred for 2 hours, then 10 mL of ammonium chloride aqueous solution was added, and the mixture was extracted with ethyl acetate (20mL*3). The organic phase was washed with saturated saline and dried over anhydrous sodium sulfate. The residue was filtered and evaporated to dryness to obtain crude product thietan-3-ylmethanol (314 mg, the yield was 72%).

### Step 2: 4-bromo-6-(thietan-3-ylmethoxy)pyrazolo[1,5-a]pyridine-3-carbonitrile

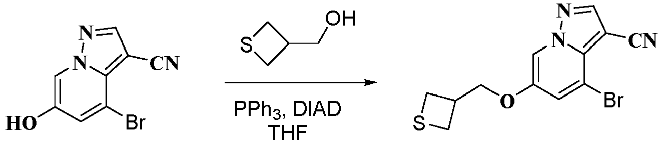

4-Bromo-6-(thietan-3-ylmethoxy)pyrazolo[1,5-*a*]pyridine-3-carbonitrile (210 mg, white solid, 67%) was obtained by using 4-bromo-6-hydroxypyrazolo[1,5-*a*]pyridine-3-carbonitrile and thietan-3-ylmethanol as raw materials with reference to step 2 of embodiment **1.**

MS m/z (ESI): 323.9 [M+H]⁺.

### Step 3: 4-bromo-6-((1-hydroxythietan-3-yl)methoxy)pyrazolo[1,5-a]pyridine-3-carbonitrile

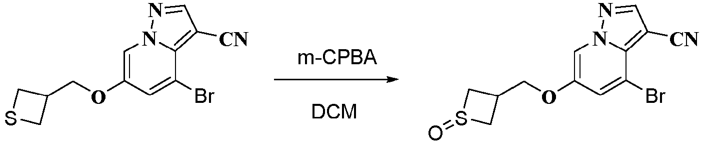

4-Bromo-6-((1-hydroxythietan-3-yl)methoxy)pyrazolo[1,5-*a*]pyridine-3-carbonitrile (180 mg, white solid, 85%) was obtained by using 4-bromo-6-(thietan-3-ylmethoxy)pyrazolo[1,5-a]pyridine-3-carbonitrile as raw material with reference to step 3 of embodiment **1.**

MS m/z (ESI): 339.9 [M+H]⁺.

### Step 4: 4-bromo-6-((1-imino-1-hydroxy-1l6-thietan-3-yl)methoxy)pyrazolo[1,5-a]pyridine-3-carbonitrile

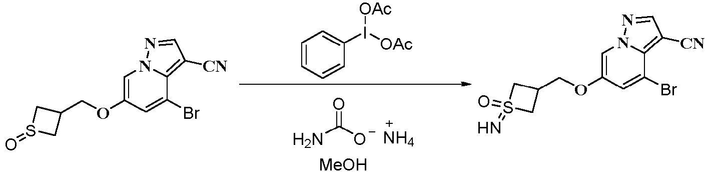

4-Bromo-6-((1-imino-1-hydroxy-1l6-thietan-3-yl)methoxy)pyrazolo[1,5-*a*]pyridine-3-carbonitrile (150 mg, white solid, 79%) was obtained by using 4-bromo-6-((1- hydroxythietan-3-yl)methoxy)pyrazolo[1,5-a]pyridine-3-carbonitrile as raw material with reference to embodiment **3.**

MS m/z (ESI): 354.9 [M+H]⁺.

### Step 5: 6-((1-imino-1-hydroxy-1l6-thietan-3-yl)methoxy)-4-(4,4,5,5-tetramethyl-1,3,2-dioxaborolan-2-yl)pyrazolo[1,5-a]pyridine-3-carbonitrile

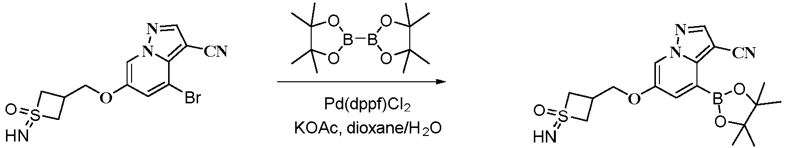

6-((1-Imino-1-hydroxy-1l6-thietan-3-yl)methoxy)-4-(4,4,5,5-tetramethyl-1,3,2-dioxaborolan-2-yl)pyrazolo[1,5-a]pyridine-3-carbonitrile (96 mg, white solid, 64%) was obtained by using 4-bromo-6-((1-imino-1-hydroxy-1l6-thietan-3-yl)methoxy)pyrazolo[1,5-*a*]pyridine-3-carbonitrile as raw material with reference to step 7 of embodiment **1.**

MS m/z (ESI): 403.1 [M+H]⁺.

### Step 6: 6-((1-imino-1-hydroxy-1l6-thietan-3-yl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

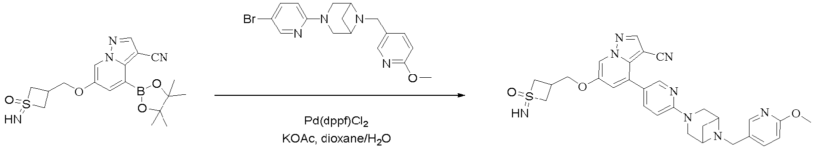

6-((1-Imino-1-hydroxy-116-thietan-3-yl)methoxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (30 mg, white solid, 34%) was obtained by using 6-((1-imino-1-hydroxy-1l6-thietan-3-yl)methoxy)-4-(4,4,5,5-tetramethyl-1,3,2-dioxaborolan-2-yl)pyrazolo[1,5-*a*]pyridine-3-carbonitrile as raw materials with reference to step 8 of embodiment 1.

MS m/z (ESI): 571.2 [M+H]⁺.

¹H NMR (400 MHz, MeOD) δ 8.53 (s, 1H), 8.38 (s, 1H), 8.36 (s, 1H), 8.19 (s, 1H), 7.87 (d, *J* = 8.8 Hz, 1H), 7.77 (d, *J* = 8.7 Hz, 1H), 7.32 (s, 1H), 6.90 (d, *J* = 8.7 Hz, 1H), 6.83 (d, *J* = 8.7 Hz, 1H), 4.43 - 4.22 (m, 4H), 4.21 - 4.05 (m, 4H), 4.06 - 3.94 (m, 5H), 3.91 (s, 3H), 3.67 - 3.59 (m, 1H), 3.22 - 3.11 (m, 1H), 2.25 - 1.88 (m, 2H).

### [Not according to the invention] Embodiment 86

### 6-((1-Imino-1-hydroxy-1l6-thietan-3-yl)methoxy)-4-(6-(4-((6-methoxypyridin-3-yl)oxo)piperidin-1-yl)pyridin-3-yl)pyrazolo[1,5-a]pyridine-3-carbonitrile

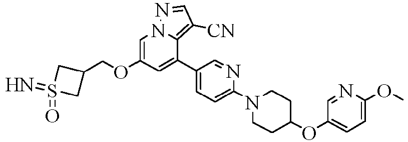

6-((1-Imino-1-hydroxy-1l6-thietan-3-yl)methoxy)-4-(6-(4-((6-methoxypyridin-3-yl)oxo)piperidin-1-yl)pyridin-3-yl)pyrazolo[1,5-*a*]pyridine-3-carbonitrile (34 mg, white solid, 36%) was obtained by using 6-((1-imino-1-hydroxy-1l6-thietan-3-yl)methoxy)-4-(4,4,5,5-tetramethyl-1,3,2-dioxaborolan-2-yl)pyrazolo[1,5-*a*]pyridine-3-carbonitrile as raw material with reference to step 8 of embodiment **1.**

MS m/z (ESI): 560.2 [M+H]⁺.

### [Not according to the invention] Embodiment 87

### 6-(2-(1-Imino-1-hydroxyl-1l6-thietan-3-yl)ethoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

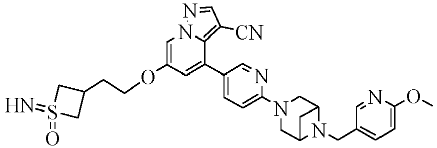

6-(2-(1-Imino-1-hydroxyl-1l6-thietan-3-yl)ethoxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (35 mg, white solid) was obtained by using 2-(thietan-3-yl)ethan-1-ol as raw material with reference to embodiment **85.**

MS m/z (ESI): 585.2 [M+H]⁺.

### [Not according to the invention] Embodiment 88

### 4-(6-(6-((6-Methoxypyridin-3-yl)methyl)-3,6-diazabicyclo[3.1.1]heptan-3-yl)pyridin-3-yl)-6-(2-(1-hydroxyltetrahydro-2H-thiopyran-4-yl)ethoxy)pyrazolo[1,5-a]pyridine-3-carbonitrile

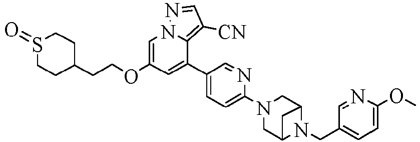

4-(6-(6-((6-Methoxypyridin-3-yl)methyl)-3,6-diazabicyclo[3.1.1]heptan-3-yl)pyridin-3-yl)-6-(2-(1-hydroxyltetrahydro-2*H*-thiopyran-4-yl)ethoxy)pyrazolo[1,5-*a*]pyridine-3-carbonitrile (29 mg, white solid) was obtained by using 2-(tetrahydro-2*H*-thiopyran-4-yl)ethan-1-ol as raw materials with reference to embodiment **85.**

MS m/z (ESI): 598.2 [M+H]⁺.

### [Not according to the invention] Embodiment 89

### 6-(2-(1-Imino-1-hydroxyhexahydro-1l6-thiopyran-4-yl)ethoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

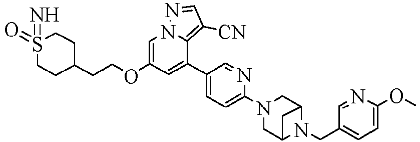

6-(2-(1-Imino-1-hydroxyhexahydro-1l6-thiopyran-4-yl)ethoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile (18mg, white solid) was obtained by using 4-(6-(6-((6-methoxypyridin-3-yl)methyl)-3,6-diazabicyclo[3.1.1]heptan-3-yl)pyridin-3-yl)-6-(2-(1-hydroxytetrahydro-2*H*-thiopyran-4-yl)ethoxy)pyrazolo[1,5-*a*]pyridine-3-carbonitrile as raw material with reference to embodiment **3.**

MS m/z (ESI): 613.2 [M+H]⁺.

### [Not according to the invention] Embodiment 90

### 6-((1-Imino-1-hydroxyhexahydro-1l6-thiopyran-4-yl)oxo)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

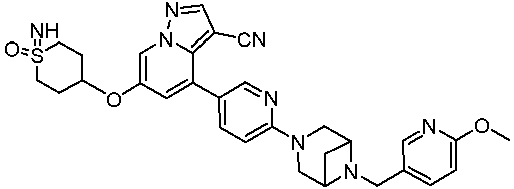

### Step 1: 4-bromo-6-((tetrahydro-2H-thiopyran-4-yl)oxo)pyrazolo[1,5-a]pyridine-3-carbonitrile

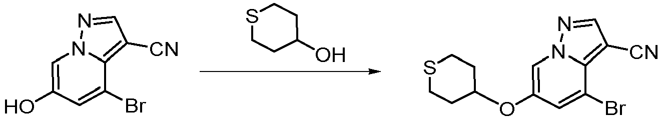

4-Bromo-6-hydroxypyrazolo[1,5-*a*]pyridine-3-carbonitrile (10 g, 42.0 mmol) was dissolved in tetrahydrofuran (100 mL), and tetrahydro-2*H*-thiopyran-4-ol (6 g, 50.4 mmol) and triphenylphosphine (22 g, 84.0 mmol) were added thereto. DEAD (14.6 g, 84.0 mmol) was slowly added dropwise to the reaction mixture. The reaction was stirred at room temperature overnight. Water was added to quench the reaction, and then the mixture was extracted with ethyl acetate. The organic phase was dried and evaporated to dryness. The crude product was purified by column chromatography to obtain 4-bromo-6-((tetrahydro-2*H*-thiopyran-4-yl)oxo)pyrazolo[1,5-*a*]pyridine-3-carbonitrile (7 g, yield: 49%).

MS m/z (ESI): 337.9[M+H]⁺.

### Step 2: preparation of 4-bromo-6-((1-imino-1-hydroxyhexahydro-1l6-thiopyran-4-yl)oxo)pyrazolo[1,5-a]pyridine-3-carbonitrile

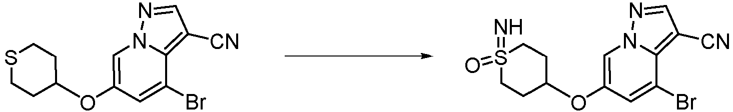

4-Bromo-6-((tetrahydro-2*H*-thiopyran-4-yl)oxo)pyrazolo[1,5-*a*]pyridine-3-carbonitrile (3 g, 8.9 mmol) was dissolved in methanol (40 mL), and ammonium carbonate (1.6 g, 16.9 mmol) and (diacetoxyiodo)benzene (5.7 g, 17.8 mmol) were added thereto. The reaction was stirred at room temperature overnight. The reaction mixture was evaporated to dryness. The crude product was purified by column chromatography to obtain 4-bromo-6-((1-imino-1-hydroxyhexahydro-1l6-thiopyran-4-yl)oxo)pyrazolo[1,5-a]pyridine-3-carbonitrile (350 mg, yield: 11%).

MS m/z (ESI): 368.9[M+H]⁺.

### Step 3: preparation of 6-((1-imino-1-hydroxyhexahydro-1l6-thiopyran-4-yl)oxo)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

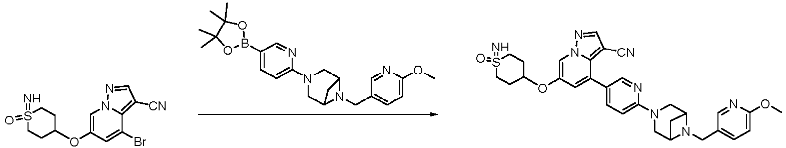

6-((1-Imino-1-hydroxyhexahydro-1l6-thiopyran-4-yl)oxo)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile (35 mg, yield: 36%) was obtained by using 4-bromo-6-((1-imino-1-hydroxyhexahydro-1l6-thiopyran-4-yl)oxo)pyrazolo[1,5-*a*]pyridine-3-carbonitrile and 6-((6-methoxypyridin-3-yl)methyl)-3-(5-(4,4,5,5-tetramethyl-1,3,2-dioxaborolan-2-yl)pyridin-2-yl)-3,6-diazabicyclo[3.1.1]heptane as raw materials with reference to step 3 of embodiment 37.

MS m/z (ESI): 585.2[M+H]⁺.

¹H NMR (400 MHz, DMSO-d6) δ 8.89 (dd, J = 7.6, 2.0 Hz, 1H), 8.62 (s, 1H), 8.42 (t, J = 2.4 Hz, 1H), 8.07 (s, 1H), 7.85 (d, J = 8.6 Hz, 1H), 7.72 - 7.60 (m, 1H), 7.51 - 7.40 (m, 1H), 6.78 (t, J = 9.4 Hz, 2H), 4.90 - 4.78 (m, 1H), 3.82 (s, 3H), 3.79 - 3.61 (m, 4H), 3.60 - 3.45 (m, 4H), 3.27 - 3.13 (m, 2H), 3.13 - 2.98 (m, 2H), 2.65 - 2.53 (m, 1H),2.31 - 2.14 (m, 3H), 2.13 - 1.92 (m, 1H), 1.59 (d, J = 8.4 Hz, 1H), 0.84 - 0.69 (m, 1H).

### [Not according to the invention] Embodiment 91

### 6-((1-Imino-1-hydroxylhexahydro-1l6-thiopyran-4-yl)oxo)-4-(6-(4-(pyridin-2-oxy)piperidin-1-yl)pyridin-3-yl)pyrazolo[1,5-a]pyridine-3-carbonitrile

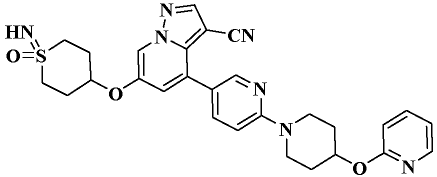

### Step 1: preparation of 6-((1-imino-1-hydroxylhexahydro-1l6-thiopyran-4-yl)oxo)-4-(6-(4-(pyridin-2-oxy)piperidin-1-yl)pyridin-3-yl)pyrazolo[1,5-a]pyridine-3-carbonitrile

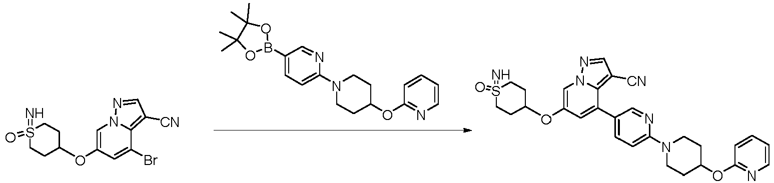

6-((1-Imino-1-hydroxylhexahydro-1l6-thiopyran-4-yl)oxo)-4-(6-(4-(pyridin-2-oxy)piperidin-1-yl)pyridin-3-yl)pyrazolo[1,5-*a*]pyridine-3-carbonitrile (45 mg, yield: 38%) was obtained by using 4-bromo-6-((1-imino-1-hydroxyhexahydro-1l6-thiopyran-4-yl)oxo)pyrazolo[1,5-*a*]pyridine-3-carbonitrile and 2-(4-(pyridin-2-oxy)piperidin-1-yl)-5-(4,4,5,5-tetramethyl-1,3,2-dioxaborolan-2-yl)pyridine as raw materials with reference to step 3 of embodiment 37.

MS m/z (ESI): 544.2[M+H]⁺.

### Embodiment 92

### 6-(3-Amino-3-methylbut-1-yn-1-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

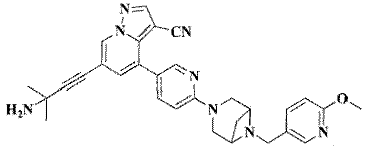

6-(3-Amino-3-methylbut-1-yn-1-yl)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 2-methylbut-3-yn-2-amine as raw material with reference to embodiment 31.

MS m/z (ESI): 519.2 [M+H]⁺.

¹H NMR (400 MHz, Methanol-d₄) δ 8.82 (d, J = 1.3 Hz, 1H), 8.46 (s, 1H), 8.34 (d, J = 2.4 Hz, 1H), 8.09 (d, J = 2.3 Hz, 1H), 7.84 (dd, J = 8.9, 2.5 Hz, 1H), 7.72 (dd, J = 8.5, 2.5 Hz, 1H), 7.41 (d, J = 1.4 Hz, 1H), 6.88 (d, J = 8.9 Hz, 1H), 6.78 (d, J = 8.5 Hz, 1H), 4.01 - 3.83 (m, 5H), 3.84 - 3.73 (m, 2H), 3.72 - 3.56 (m, 4H), 2.78 - 2.65 (m, 1H), 1.71 (d, J = 9.0 Hz, 1H), 1.52 (s, 6H).

### Embodiment 93

### 6-(3-Amino-3-methylbut-1-yn-1-yl)-4-(6-(4-((6-methoxypyridin-3-yl)oxo)piperidin-1-yl)pyridin-3-yl)pyrazolo[1,5-a]pyridine-3-carbonitrile

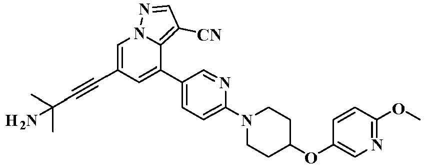

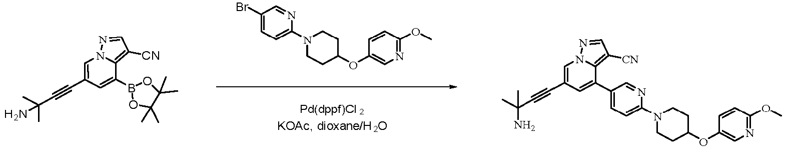

6-(3-Amino-3-methylbut-1-yn-1-yl)-4-(6-(4-((6-methoxypyridin-3-yl)oxo)piperidin-1-yl)pyridin-3-yl)pyrazolo[1,5-a]pyridine-3-carbonitrile was obtained by using 6-(3-amino-3-methylbut-1-yn-1-yl)-4-(4,4,5,5-tetramethyl-1,3,2-dioxaborolan-2-yl)pyrazolo[1,5-a]pyridine-3-carbonitrile and 5-bromo-2-(4-((6-methoxypyridin-3-yl)oxo)piperidin-1-yl)pyridine as raw materials with reference to the step 8 of embodiment **1.**

MS m/z (ESI): 508.2 [M+H]⁺.

### Embodiment 94

### 4-(6-(6-((5-Fluoro-6-methoxypyridin-3-yl)methyl)-3,6-diazabicyclo[3.1.1]heptan-3-yl)pyridin-3-yl)-6-(3-hydroxy-3-methylbut-1-yn-1-yl)pyrazolo[1,5-a]pyridine-3-carbonitrile

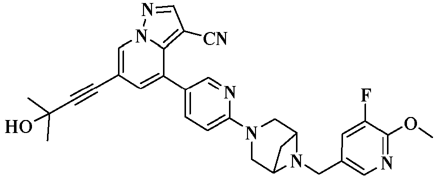

### Step 1: 6-bromo-3-cyanopyrazolo[1,5-a]pyridin-4-yl trifluoromethanesulfonate

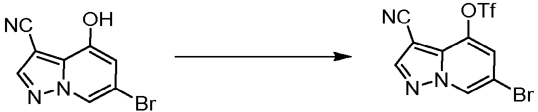

6-Bromo-4-hydroxypyrazolo[1,5-*a*]pyridine-3-carbonitrile (5 g, 21 mmol) and triethylamine (4.2 g, 42 mmol) were dissolved in dichloromethane (500 mL), and trifluoromethanesulfonic anhydride (8.9 g, 31.5 mmol) was added under ice bath; then the mixture was stirred at room temperature for 12 hours, 100 mL of water was added, and the mixture was extracted with ethyl acetate (80 mL*3). The organic phase was washed with saturated saline, dried over anhydrous sodium sulfate, filtered and evaporated to dryness, the crude product was separated by column chromatography (petroleum ether/ethyl acetate = 1/1) to obtain 6-bromo-3-cyanopyrazolo[1,5-*a*]pyridin-4-yl trifluoromethanesulfonate (5 g, white solid, yield was 64%).

¹H NMR (400 MHz, DMSO) δ 9.60 (d, *J* = 0.9 Hz, 1H), 8.85 (s, 1H), 8.23 (s, 1H).

### Step 2: 6-bromo-4-(6-fluoropyridin-3-yl)pyrazolo[1,5-a]pyridine-3-carbonitrile

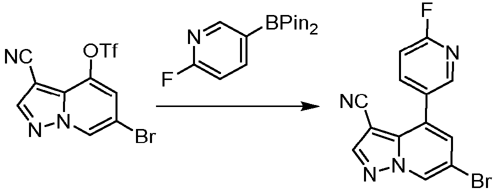

1,1'-Bis(diphenylphosphino)ferrocene-palladium(II)dichloride dichloromethane complex (494 mg, 0.68 mmol) was added to a mixed solution of 6-bromo-3-cyanopyrazolo[1,5-*a*]pyridin-4-yl trifluoromethanesulfonate (5 g, 13.51 mmol), 2-fluoro-5-(4,4,5,5-tetramethyl-1,3,2-dioxaborolan-2-yl)pyridine (3.3 g, 14.86 mmol), potassium carbonate (3.7 g, 27 mmol) and dioxane (100 mL), the mixture was replaced with nitrogen three times and then stirred at 70 °C under the protection of the protection of nitrogen for 16 hours; the reaction was cooled and filtered, the filtrate was concentrated under reduced pressure to dryness and separated by column chromatography (dichloromethane/methanol = 10:1) to obtain white solid 6-bromo-4-(6-fluoropyridin-3-yl)pyrazolo[1,5-a]pyridine-3-carbonitrile (3 g, yield: 70%).

¹H NMR (400 MHz, DMSO) δ 9.49 (d, *J* = 1.3 Hz, 1H), 8.73 (s, 1H), 8.51 (d, *J* = 2.0 Hz, 1H), 8.27 (td, *J* = 8.2, 2.5 Hz, 1H), 7.86 (d, *J* = 1.2 Hz, 1H), 7.40 (dd, *J =* 8.5, 2.5 Hz, 1H).

MS m/z (ESI): 317.0 [M+H]⁺.

### Step 3: tert-butyl 3-(5-(6-bromo-3-cyanopyrazolo[1,5-a]pyridin-4-yl)pyridin-2-yl)-3,6-diazabicyclo[3.1.1]heptane-6-carboxylate

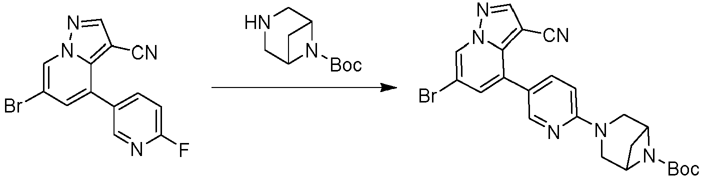

*Tert*-butyl 3-(5-(6-bromo-3-cyanopyrazolo[1,5-*a*]pyridin-4-yl)pyridin-2-yl)-3,6-diazabicyclo[3.1.1]heptane-6-carboxylate was obtained by using 6-bromo-4-(6-fluoropyridin-3-yl)pyrazolo[1,5-a]pyridine-3-carbonitrile as raw material with reference to step 7 of embodiment 11.

MS m/z (ESI):495.1 [M+H]⁺.

### Step 4: 4-(6-(3,6-diazabicyclo[3.1.1]heptan-3-yl)pyridin-3-yl)-6-bromopyrazolo[1,5-a]pyridine-3-carbonitrile

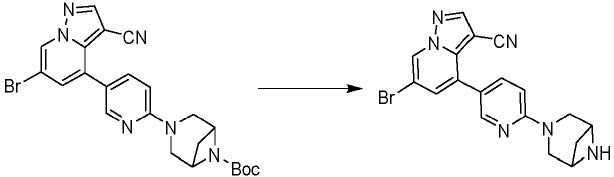

4-(6-(3,6-Diazabicyclo[3.1.1]heptan-3-yl)pyridin-3-yl)-6-bromopyrazolo[1,5-*a*]pyridine-3-carbonitrile was obtained by using *tert*-butyl 3-(5-(6-bromo-3-cyanopyrazolo[1,5-*a*]pyridin-4-yl)pyridin-2-yl)-3,6-diazabicyclo[3.1.1]heptane-6-carboxylate as raw material with reference to step 8 of embodiment 11.

MS m/z (ESI): 395.1 [M+H]⁺.

### Step 5: 6-bromo-4-(6-(6-((5-fluoro-6-methoxypyridin-3-yl)methyl)-3,6-diazabicyclo[3.1.1]heptan-3-yl)pyridin-3-yl)pyrazolo[1,5-a]pyridine-3-carbonitrile

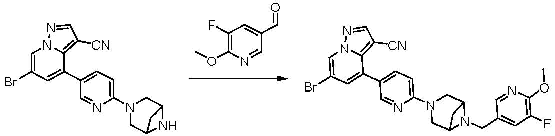

6-Bromo-4-(6-(6-((5-fluoro-6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 4-(6-(3,6-diazabicyclo[3.1.1]heptan-3-yl)pyridin-3-yl)-6-bromopyrazolo[1,5-*a*]pyridine-3-carbonitrile and 5-fluoro-6-methoxynicotinaldehyde as raw material with reference to step 9 of embodiment 11.

MS m/z (ESI):534.1 [M+H]⁺.

### Step 6: 4-(6-(6-((5-fluoro-6-methoxypyridin-3-yl)methyl)-3,6-diazabicyclo[3.1.1]heptan-3-yl)pyridin-3-yl)-6-(3-hydroxy-3-methylbut-1-yn-1-yl)pyrazolo[1,5-a]pyridine-3-carbonitrile

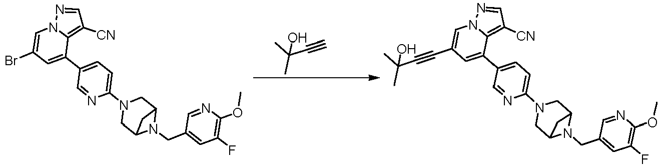

4-(6-(6-((5-Fluoro-6-methoxypyridin-3-yl)methyl)-3,6-diazabicyclo[3.1.1]heptan-3-yl)pyridin-3-yl)-6-(3-hydroxy-3-methylbut-1-yn-1-yl)pyrazolo[1,5-*a*]pyridine-3-carbonitrile was obtained by using 2-methylbut-3-yn-2-ol and 6-bromo-4-(6-(6-((5-fluoro-6-methoxypyridin-3-yl)methyl)-3,6-diazabicyclo[3.1.1]heptan-3-yl)pyridin-3-yl)pyrazolo[1,5-*a*]pyridine-3-carbonitrile as raw materials with reference to step 2 of embodiment 31.

MS m/z (ESI):538.2 [M+H]⁺.

¹H NMR (400 MHz, CDCl3) δ 8.61 (s, 1H), 8.40 (d, J = 2.2 Hz, 1H), 8.31 (s, 1H), 7.86 (s, 1H), 7.76 (dd, J = 8.8, 2.4 Hz, 1H), 7.47 (d, J = 11.1 Hz, 1H), 7.30 (s, 1H), 6.68 (d, J = 8.7 Hz, 1H), 4.01 (s, 3H), 3.80 (s, 4H), 3.59 (s, 4H), 2.78 - 2.53 (m, 2H), 1.65 (s, 6H).

### Embodiment 95

### 6-(3-Cyclopropyl-3-hydroxyprop-1-yn-1-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

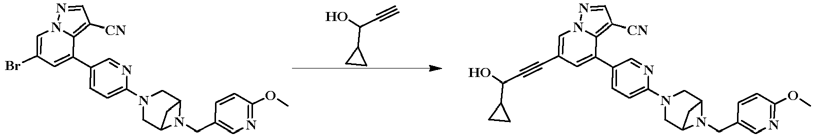

6-Bromo-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (50 mg, 0.1 mmol) was dissolved in *N*,*N*-dimethylformamide (2 mL), and 1- cyclopropylprop-2-yn-1-ol (47 mg, 0.5 mmol), triethylamine (50 mg, 0.5 mmol), Pd₂(PPh₃)₂Cl₂ (7 mg, 0.01 mmol), CuI (1 mg, 0.01 mmol) were added thereto, the reaction was carried out at 65°C overnight under the protection of nitrogen. 5 mL of ammonium chloride aqueous solution was added, and the mixture was extracted with ethyl acetate (3mL*3). The organic phase was washed with saturated saline, dried over anhydrous sodium sulfate. The residue was filtered and evaporated to dryness, and the crude product was separated by column chromatography (eluted with dichloromethane/methanol = 10/1) to obtain 6-(3-cyclopropyl-3-hydroxyprop-1-yn-1-yl)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (15 mg, white solid, yield was 29%).

MS m/z (ESI): 532.2 [M+H]⁺.

### Embodiment 96

### 6-((1-Cyanocyclopentyl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

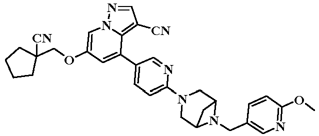

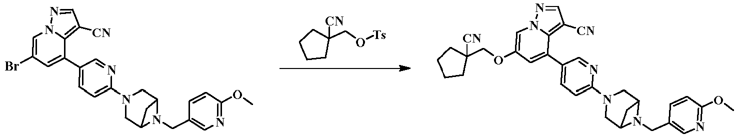

6-((1-Cyanocyclopentyl)methoxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 6-bromo-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile and (1-cyanocyclopentyl)methyl 4-methylbenzenesulfonate as raw materials with reference to step 2 of embodiment 106.

MS m/z (ESI): 561.2 [M+H]⁺.

### Embodiment 97

### 3-Chloro-N-(1-(5-(3-cyano-6-(3-hydroxy-3-methylbut-1-yn-1-yl)pyrazolo[1,5-a]pyridin-4-yl)pyridin-2-yl)-4-methylpiperidin-4-yl)picolinamide

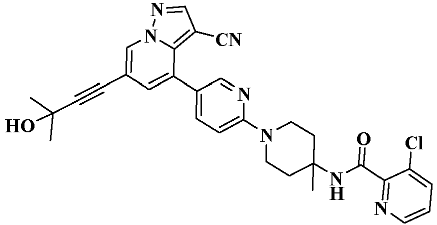

### Step 1: tert-butyl (1-(5-(6-bromo-3-cyanopyrazolo[1,5-a]pyridin-4-yl)pyridin-2-yl)-4-methylpiperidin-4-yl)carbamate

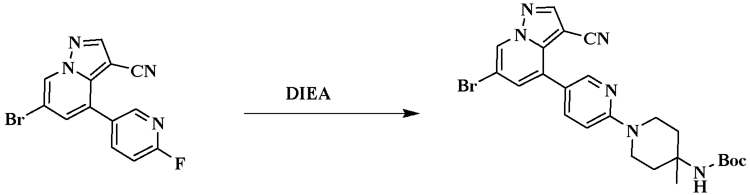

*Tert*-butyl (1-(5-(6-bromo-3-cyanopyrazolo[1,5-*a*]pyridin-4-yl)pyridin-2-yl)-4-methylpiperidin-4-yl)carbamate (white solid) was obtained by using 6-bromo-4-(6-fluoropyridin-3-yl)pyrazolo[1,5-a]pyridine-3-carbonitrile as raw material with reference to step 4 of embodiment 1.

MS m/z (ESI): 511.1 [M+H]⁺.

### Step 2: 4-(6-(4-amino-4-methylpiperidin-1-yl)pyridin-3-yl)-6-bromopyrazolo[1,5-a]pyridine-3-carbonitrile

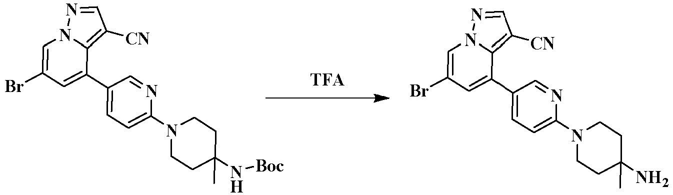

4-(6-(4-Amino-4-methylpiperidin-1-yl)pyridin-3-yl)-6-bromopyrazolo[1,5-*a*]pyridine-3-carbonitrile (white solid) was obtained by using *tert*-butyl (1-(5-(6-bromo-3-cyanopyrazolo[1,5-*a*]pyridin-4-yl)pyridin-2-yl)-4-methylpiperidin-4-yl)carbamate as raw material with reference to the step 5 of embodiment 1.

MS m/z (ESI): 411.1 [M+H]⁺.

### Step 3: N-(1-(5-(6-bromo-3-cyanopyrazolo[1,5-a]pyridin-4-yl)pyridin-2-yl)-4-methylpiperidin-4-yl)-3-chloropicolinamide

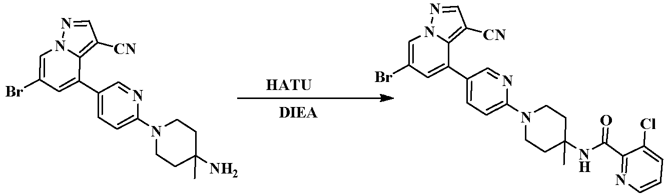

*N*-(1-(5-(6-bromo-3-cyanopyrazolo[1,5-*a*]pyridin-4-yl)pyridin-2-yl)-4-methylpiperidin-4-yl)-3-chloropicolinamide (white solid) was obtained by using 4-(6-(4-amino-4-methylpiperidin-1-yl)pyridin-3-yl)-6-bromopyrazolo[1,5-*a*]pyridine-3-carbonitrile as raw material with reference to step 1 of embodiment 96.

MS m/z (ESI): 550.0 [M+H]⁺.

### Step 4: 3-chloro-N-(1-(5-(3-cyano-6-(3-hydroxy-3-methylbut-1-yn-1-yl)pyrazolo[1,5-a]pyridin-4-yl)pyridin-2-yl)-4-methylpiperidin-4-yl)picolinamide

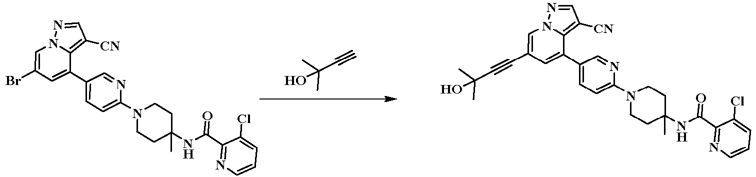

3-Chloro-N-(1-(5-(3-cyano-6-(3-hydroxy-3-methylbut-1-yn-1-yl)pyrazolo[1,5-*a*]pyridin-4-yl)pyridin-2-yl)-4-methylpiperidin-4-yl)picolinamide (white solid) was obtained by using *N*-(1-(5-(6-bromo-3-cyanopyrazolo[1,5-*a*]pyridin-4-yl)pyridin-2-yl)-4-methylpiperidin-4-yl)-3-chloropicolinamide as raw materials with reference to step 2 of embodiment 96.

MS m/z (ESI): 554.2 [M+H]⁺.

¹H NMR (400 MHz, Methanol-d4) δ 8.81 (d, J = 1.3 Hz, 1H), 8.50 (dd, J = 4.7, 1.3 Hz, 1H), 8.45 (s, 1H), 8.28 (d, J = 2.6 Hz, 1H), 7.96 (dd, J = 8.2, 1.4 Hz, 1H), 7.75 (dd, J = 8.8, 2.6 Hz, 1H), 7.48 (dd, J = 8.2, 4.7 Hz, 1H), 7.38 (d, J = 1.3 Hz, 1H), 6.97 (d, J = 8.9 Hz, 1H), 4.18 - 4.03 (m, 2H), 3.48 - 3.37 (m, 2H), 2.54 - 2.34 (m, 2H), 1.80 - 1.68 (m, 2H), 1.58 (s, 6H), 1.56 (s, 3H).

### Embodiment 98

### 6-((S)-3-hydroxybut-1-yn-1-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

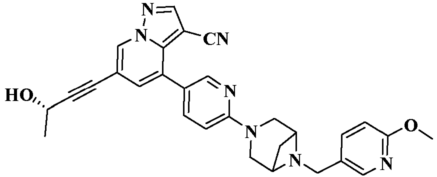

6-((S)-3-hydroxybut-1-yn-1-yl)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (white solid) was obtained by using 6-bromo-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile and (*S*)-but-3-yn-2-ol as raw materials with reference to step 2 of embodiment 96.

MS m/z (ESI): 506.2 [M+H]⁺.

¹H NMR (400 MHz, Methanol-d4) δ 8.85 (s, 1H), 8.47 (s, 1H), 8.35 (d, J = 2.5 Hz, 1H), 8.08 (d, J = 2.5 Hz, 1H), 7.84 (dd, J = 8.9, 2.6 Hz, 1H), 7.72 (dd, J = 8.5, 2.5 Hz, 1H), 7.42 (s, 1H), 6.88 (d, J = 8.9 Hz, 1H), 6.78 (d, J = 8.6 Hz, 1H), 4.72 (q, J = 6.6 Hz, 1H), 3.96 - 3.84 (m, 5H), 3.83 - 3.74 (m, 2H), 3.69 - 3.56 (m, 4H), 2.76 - 2.64 (m, 1H), 1.70 (d, J = 8.9 Hz, 1H), 1.51 (d, J = 6.6 Hz, 3H).

### Embodiment 99

### 6-((R)-3-hydroxybut-1-yn-1-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

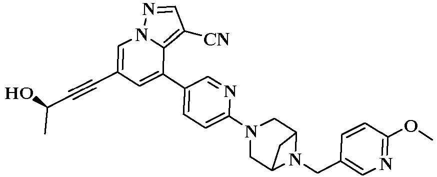

6-((*R*)-3-hydroxybut-1-yn-1-yl)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (white solid) was obtained by using 6-bromo-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile and (*S*)-but-3-yn-2-ol as raw materials with reference to step 2 of embodiment 96.

MS m/z (ESI): 506.2 [M+H]⁺.

¹H NMR (400 MHz, Methanol-d₄) δ 8.85 (d, J = 1.4 Hz, 1H), 8.47 (s, 1H), 8.35 (d, J = 2.5 Hz, 1H), 8.09 (s, 1H), 7.84 (dd, J = 8.8, 2.5 Hz, 1H), 7.72 (dd, J = 8.5, 2.5 Hz, 1H), 7.42 (d, J = 1.4 Hz, 1H), 6.88 (d, J = 8.9 Hz, 1H), 6.78 (d, J = 8.5 Hz, 1H), 4.75 - 4.70 (m, 1H), 3.92 - 3.85 (m, 5H), 3.84 - 3.75 (m, 2H), 3.68 - 3.58 (m, 4H), 2.74 - 2.67 (m, 1H), 1.71 (d, J = 9.0 Hz, 1H), 1.51 (d, J = 6.7 Hz, 3H).

### Embodiment 100

### N-(1-(5-(6-(3-amino-3-methylbut-1-yn-1-yl)-3-cyanopyrazolo[1,5-a]pyridin-4-yl)pyridin-2-yl)-4-methylpiperidin-4-yl)-3-chloropicolinamide

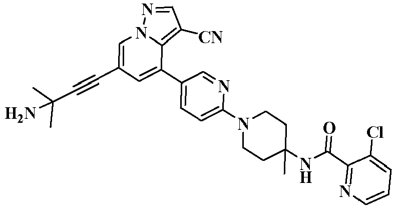

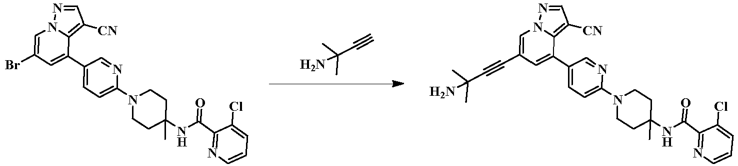

*N*-(1-(5-(6-(3-amino-3-methylbut-1-yn-1-yl)-3-cyanopyrazolo[1,5-*a*]pyridin-4-yl)pyridin-2-yl)-4-methylpiperidin-4-yl)-3-chloropicolinamide (white solid) was obtained by using N-(1-(5-(6-bromo-3-cyanopyrazolo[1,5-*a*]pyridin-4-yl)pyridin-2-yl)-4-methylpiperidin-4-yl)-3-chloropicolinamide and 2-methylbut-3-yn-2-amine as raw materials with reference to step 2 of embodiment 96.

MS m/z (ESI): 553.2 [M+H]⁺.

¹H NMR (400 MHz, Methanol-d₄) δ 8.80 (d, J = 1.4 Hz, 1H), 8.50 (d, J = 4.8 Hz, 1H), 8.45 (s, 1H), 8.28 (d, J = 2.5 Hz, 1H), 7.99 - 7.94 (m, 1H), 7.78 - 7.73 (m, 1H), 7.51 - 7.46 (m, 1H), 7.38 (s, 1H), 6.98 (d, J = 8.8 Hz, 1H), 4.15 - 4.05 (m, 2H), 3.50 - 3.45 (m, 2H), 2.44 - 2.38 (m, 2H), 1.78 - 1.72 (m, 2H), 1.56 (s, 3H), 1.52 (s, 6H).

### Embodiment 101

### 6-(3-Amino-3-methylbut-1-yn-1-yl)-4-(6-(6-((6-(methoxy-d3)pyridin-3-yl)methyl)-3,6-diazabicydo[3.1.1]heptan-3-yl)pyridin-3-yl)pyrazolo[1,5-a]pyridine-3-carbonitrile

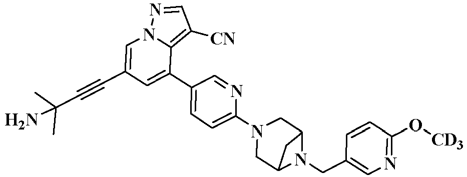

### Step 1: preparation of 6-bromo-4-(6-(6-((6-(methoxy-d3)pyridin-3-yl)methyl)-3,6-diazabicyclo[3.1.1]heptan-3-yl)pyridin-3-yl)pyrazolo[1,5-a]pyridine-3-carbonitrile

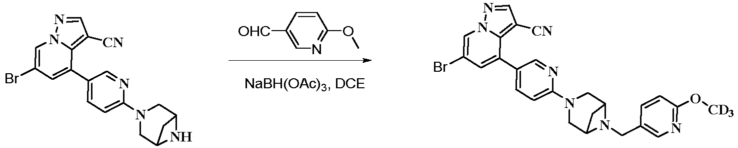

6-Bromo-4-(6-(6-((6-(methoxy-d3)pyridin-3-yl)methyl)-3,6-diazabicyclo[3.1.1]heptan-3-yl)pyridin-3-yl)pyrazolo[1,5-*a*]pyridine-3-carbonitrile (yellow solid) was obtained by using 4-(6-(3,6-diazabicyclo[3.1.1]heptan-3-yl)pyridin-3-yl)-6-bromopyrazolo[1,5-*a*]pyridine-3-carbonitrile as raw material with reference to step 6 of embodiment 1. MS m/z (ESI): 519.1 [M+H]⁺.

### Step 2: preparation of 6-(3-amino-3-methylbut-1-yn-1-yl)-4-(6-(6-((6-(methoxy-d3)pyridin-3-yl)methyl)-3,6-diazabicyclo[3.1.1]heptan-3-yl)pyridin-3-yl)pyrazolo[1,5-a]pyridine-3-carbonitrile

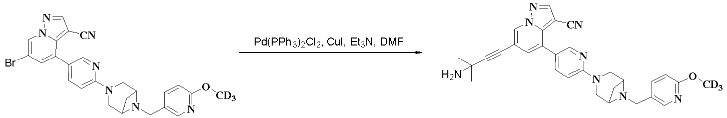

6-(3-Amino-3-methylbut-1-yn-1-yl)-4-(6-(6-((6-(methoxy-d3)pyridin-3-yl)methyl)-3,6-diazabicyclo[3.1.1]heptan-3-yl)pyridin-3-yl)pyrazolo[1,5-*a*]pyridine-3-carbonitrile (white solid) was obtained by using 6-bromo-4-(6-(6-((6-(methoxy-d3)pyridin-3-yl)methyl)-3,6-diazabicyclo[3.1.1]heptan-3-yl)pyridin-3-yl)pyrazolo[1,5-*a*]pyridine-3-carbonitrile as raw material with reference to step 2 of embodiment **96.**

MS m/z (ESI): 522.2 [M+H]⁺.

### Embodiment 102

### 6-(3-Amino-3-methylbut-1-yn-1-yl)-4-(6-(6-((5-fluoro-6-methoxypyridin-3-yl)methyl)-3,6-diazabicydo[3.1.1]heptan-3-yl)pyridin-3-yl)pyrazolo[1,5-a]pyridine-3-carbonitrile

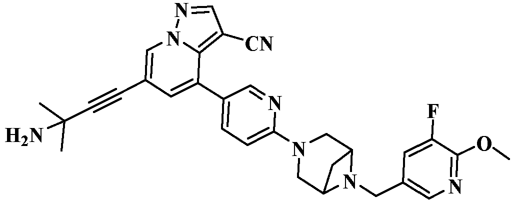

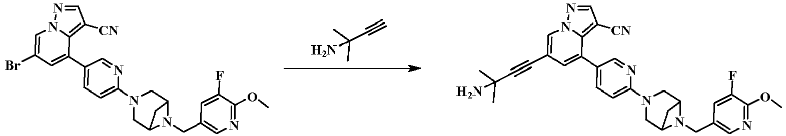

6-(3-Amino-3-methylbut-1-yn-1-yl)-4-(6-(6-((5-fluoro-6-methoxypyridin-3-yl)methyl)-3,6-diazabicyclo[3.1.1]heptan-3-yl)pyridin-3-yl)pyrazolo[1,5-*a*]pyridine-3-carbonitrile (white solid) was obtained by using 6-bromo-4-(6-(6-((5-fluoro-6-methoxypyridin-3-yl)methyl)-3,6-diazabicyclo[3.1.1]heptan-3-yl)pyridin-3-yl)pyrazolo[1,5-*a*]pyridine-3-carbonitrile and 2-methylbut-3-yn-2-amine as raw material with reference to step 2 of embodiment 96.

MS m/z (ESI): 537.2 [M+H]⁺.

### Embodiment 103

### N-(1-(5-(6-(3-amino-3-methylbut-1-yn-1-yl)-3-cyanopyrazolo[1,5-a]pyridin-4-yl)pyridin-2-yl)-4-methylpiperidin-4-yl)-5-fluoro-2-methylbenzamide

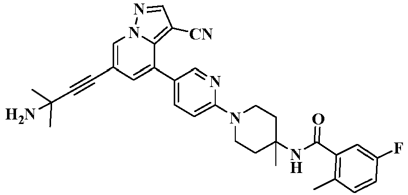

### Step 1: N-(1-(5-(6-bromo-3-cyanopyrazolo[1,5-a]pyridin-4-yl)pyridin-2-yl)-4-methylpiperidin-4-yl)-5-fluoro-2-methylbenzamide

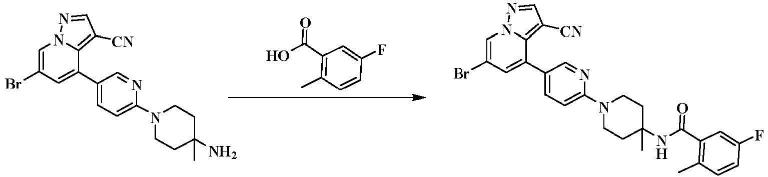

*N*-(1-(5-(6-bromo-3-cyanopyrazolo[1,5-*a*]pyridin-4-yl)pyridin-2-yl)-4-methylpiperidin-4-yl)-5-fluoro-2-methylbenzamide (white solid) was obtained by using 4-(6-(4-amino-4-methylpiperidin-1-yl)pyridin-3-yl)-6-bromopyrazolo[1,5-*a*]pyridine-3-carbonitrile and 5-fluoro-2-methylbenzoic acid as raw material with reference to step 2 of embodiment 96.

MS m/z (ESI): 547.1 [M+H]⁺.

### Step 2: N-(1-(5-(6-(3-amino-3-methylbut-1-yn-1-yl)-3-cyanopyrazolo[1,5-a]pyridin-4-yl)pyridin-2-yl)-4-methylpiperidin-4-yl)-5-fluoro-2-methylbenzamide

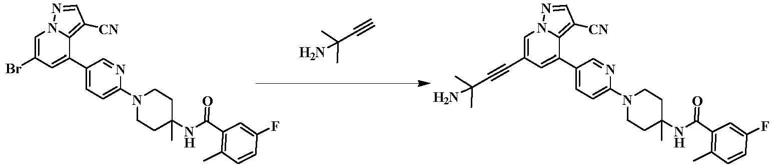

*N*-(1-(5-(6-(3-amino-3-methylbut-1-yn-1-yl)-3-cyanopyrazolo[1,5-*a*]pyridin-4-yl)pyridin-2-yl)-4-methylpiperidin-4-yl)-5-fluoro-2-methylbenzamide (white solid) was obtained by using N-(1-(5-(6-bromo-3-cyanopyrazolo[1,5-*a*]pyridin-4-yl)pyridin-2-yl)-4-methylpiperidin-4-yl)-5-fluoro-2-methylbenzamide and 2-methylbut-3-yn-2-amine as raw materials with reference to step 2 of embodiment 96.

MS m/z (ESI): 550.2 [M+H]⁺.

¹H NMR (400 MHz, Methanol-d₄) δ 8.80 (d, J = 1.3 Hz, 1H), 8.45 (s, 1H), 8.28 (d, J = 2.6 Hz, 1H), 7.76 (dd, J = 8.9, 2.6 Hz, 1H), 7.38 (d, J = 1.2 Hz, 1H), 7.28 - 7.21 (m, 1H), 7.11 - 7.02 (m, 2H), 6.98 (d, J = 8.9 Hz, 1H), 4.08 - 4.00 (m, 2H), 3.43 - 3.38 (m, 2H), 2.46 - 2.39 (m, 2H), 2.38 (s, 3H), 1.75 - 1.69 (m, 2H), 1.54 (s, 3H), 1.52 (s, 6H).

### Embodiment 104

### 6-(3-Hydroxy-3-methylbut-1-yn-1-yl)-4-(6-(6-((6-(methoxy-d3)pyridin-3-yl)methyl)-3,6-diazabicyclo[3.1.1]heptan-3-yl)pyridin-3-yl)pyrazolo[1,5-a]pyridine-3-carbonitrile

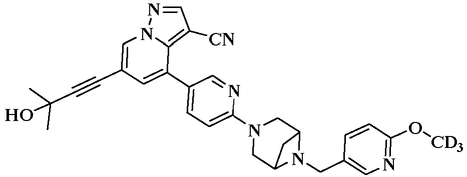

6-(3-Hydroxy-3-methylbut-1-yn-1-yl)-4-(6-(6-((6-(methoxy-d3)pyridin-3-yl)methyl)-3,6-diazabicyclo[3.1.1]heptan-3-yl)pyridin-3-yl)pyrazolo[1,5-*a*]pyridine-3-carbonitrile (white solid) was obtained by using 6-bromo-4-(6-(6-((6-(methoxy-d3)pyridin-3-yl)methyl)-3,6-diazabicyclo[3.1.1]heptan-3-yl)pyridin-3-yl)pyrazolo[1,5-*a*]pyridine-3-carbonitrile as raw material with reference to step 2 of embodiment **96.**

MS m/z (ESI): 523.3 [M+H]⁺.

### Embodiment 105

### 4-(6-(6-((6-Cyclopropoxypyridin-3-yl)methyl)-3,6-diazabicyclo[3.1.1]heptan-3-yl)pyridin-3-yl)-6-(3-hydroxy-3-methylbut-1-yn-1-yl)pyrazolo[1,5-a]pyridine-3-carbonitrile

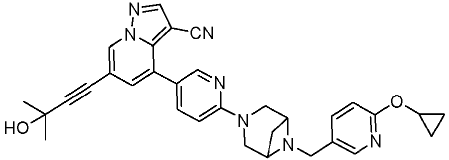

### Step 1: 5-(chloromethyl)-2-cyclopropoxypyridine

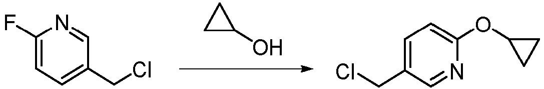

5-(Chloromethyl)-2-fluoropyridine (900 mg, 6.2 mmol) was dissolved in 30 mL of THF, and cyclopropanol (539 mg, 9.3 mmol) was added thereto. *Tert*-butyl potassium (1.04 g, 9.3 mmol) was slowly added to the reaction mixture in batches. The reaction was stirred at room temperature for 4 hours. Water was added to the reaction mixture to quench the reaction, and then the mixture was extracted with ethyl acetate. The organic phase was dried and evaporated to dryness. The crude product was purified by column chromatography to obtain 5-(chloromethyl)-2-cyclopropoxypyridine (0.6 g, yield: 52%).

MS m/z (ESI): 184.2 [M+H]⁺.

### Step 2: 6-Bromo-4-(6-(6-((6-cyclopropoxypyridin-3-yl)methyl)-3,6-diazabicyclo[3.1.1]heptan-3-yl)pyridin-3-yl)pyrazolo[1,5-a]pyridine-3-carbonitrile

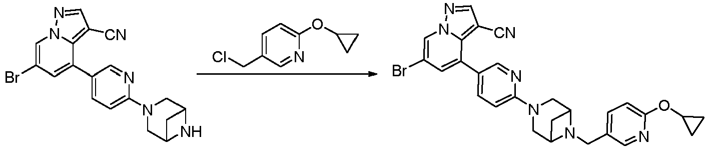

4-(6-(3,6-Diazabicyclo[3.1.1]heptan-3-yl)pyridin-3-yl)-6-bromopyrazolo[1,5-*a*]pyridine-3-carbonitrile (500 mg, 1.26 mmol) was dissolved in DMAc (15 mL), then 5-(chloromethyl)-2-cyclopropoxypyridine (279 mg, 1.52 mmol) and potassium tert-butoxide (284 mg, 2.53 mmol) were added thereto. The reaction mixture was stirred at 90 °C for 4 hours. Water was added to quench the reaction, and then the mixture was extracted with ethyl acetate. The organic phase was dried and evaporated to dryness. The crude product was purified by column chromatography to obtain 6-bromo-4-(6-(6-((6-cyclopropoxypyridin-3-yl)methyl)-3,6-diazabicyclo[3.1.1]heptan-3-yl)pyridin-3-yl)pyrazolo[1,5-*a*]pyridine-3-carbonitrile (300 mg, yield was 44%).

MS m/z (ESI): 542.2 [M+H]⁺.

### Step 3: 4-(6-(6-((6-cyclopropoxypyridin-3-yl)methyl)-3,6-diazabicyclo[3.1.1]heptan-3-yl)pyridin-3-yl)-6-(3-hydroxy-3-methylbut-1-yn-1-yl)pyrazolo[1,5-a]pyridine-3-carbonitrile

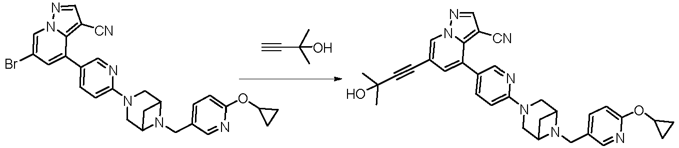

4-(6-(6-((6-Cyclopropoxypyridin-3-yl)methyl)-3,6-diazabicyclo[3.1.1]heptan-3-yl)pyridin-3-yl)-6-(3-hydroxy-3-methylbut-1-yn-1-yl)pyrazolo[1,5-*a*]pyridine-3-carbonitrile was obtained by using 2-methylbut-3-yn-2-ol and 6-bromo-4-(6-(6-((6-cyclopropoxypyridin-3-yl)methyl)-3,6-diazabicyclo[3.1.1]heptan-3-yl)pyridin-3-yl)pyrazolo[1,5-*a*]pyridine-3-carbonitrile as raw material with reference to embodiment 31.

MS m/z (ESI): 546.2 [M+H]⁺.

### Embodiment 106

### 6-((1-Cyanocyclopropyl)methoxy)-4-(6-(6-((6-methoxypyridin-3-yl)methyl)-3,6-diazabicydo[3.1.1]heptan-3-yl)pyridin-3-yl)pyrazolo[1,5-a]pyridine-3-carbonitrile

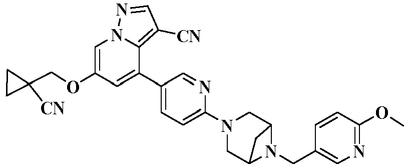

### Step 1: 6-hydroxy-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

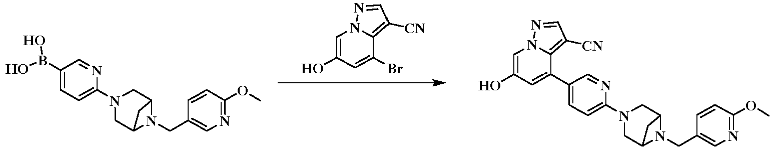

6-Hydroxy-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using (6-(6-((6-methoxypyridin-3-yl)methyl)-3,6-diazabicyclo[3.1.1]heptan-3-yl)pyridin-3-yl)boronic acid and 4-bromo-6-hydroxypyrazolo[1,5-*a*]pyridine-3-carbonitrile as raw material with reference to step 8 of embodiment 1 (500mg, white solid, yield was 80%).

MS m/z (ESI): 454.1 [M+H]⁺.

### Step 2: 6-((1-Cyanocyclopropyl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

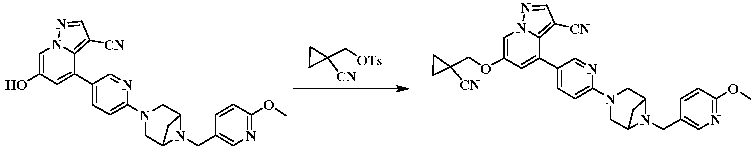

6-Hydroxy-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (50 mg, 0.11 mmol) was dissolved in 2 mL of DMF; (1-cyanocyclopropyl)methyl 4-methylbenzenesulfonate (28 mg, 0.11 mmol) and potassium carbonate (42 mg, 0.3 mmol) were added thereto, and the mixture was stirred overnight at 90 °C. 10 mL of water was added, and the mixture was extracted with ethyl acetate (2mL*3). The organic phase was washed with saturated saline, dried over anhydrous sodium sulfate. The residue was filtered, evaporated to dryness, and the crude product was purified by prep-HPLC to obtain 6-((1-cyanocyclopropyl)methoxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (23 mg, white solid, yield: 39%).

MS m/z (ESI): 533.2 [M+H]⁺.

¹H NMR (400 MHz, Methanol-d₄) δ 8.47 (d, J = 2.1 Hz, 1H), 8.39 - 8.33 (m, 2H), 8.09 (s, 1H), 7.86 (dd, J = 8.8, 2.5 Hz, 1H), 7.72 (dd, J = 8.5, 2.5 Hz, 1H), 7.35 (d, J = 2.1 Hz, 1H), 6.88 (d, J = 8.9 Hz, 1H), 6.78 (d, J = 8.5 Hz, 1H), 4.15 (s, 2H), 3.93 - 3.87 (m, 5H), 3.82 - 3.77 (m, 2H), 3.69 - 3.60 (m, 4H), 2.74 - 2.65 (m, 1H), 1.75 - 1.66 (m, 1H), 1.47 - 1.40 (m, 2H), 1.28 - 1.24 (m, 2H).

### Embodiment 107

### 6-((1-Cyanocyclopropyl)methoxy)-4-(6-(6-((6-(methoxy-d3)pyridin-3-yl)methyl)-3,6-diazabicydo[3.1.1]heptan-3-yl)pyridin-3-yl)pyrazolo[1,5-a]pyridine-3-carbonitrile

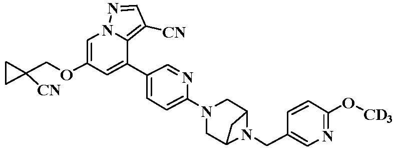

### Step 1: 3-(5-bromopyridin-2-yl)-6-((6-(methoxy-d3)pyridin-3-yl)methyl)-3,6-diazabicyclo[3.1.1]heptane

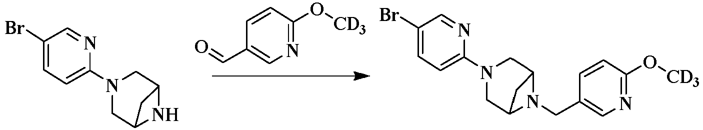

3-(5-Bromopyridin-2-yl)-6-((6-(methoxy-d3)pyridin-3-yl)methyl)-3,6-diazabicyclo[3.1.1]heptane was obtained by using 3-(5-bromopyridin-2-yl)-3,6-diazabicyclo[3.1.1]heptane and 6-(methoxy-d3)nicotinaldehyde as raw material with reference to step 6 of embodiment 1.

MS m/z (ESI): 378.0 [M+H]⁺.

### Step 2: 6-((6-(methoxy-d3)pyridin-3-yl)methyl)-3-(5-(4,4,5,5-tetramethyl-1,3,2-dioxaborolan-2-yl)pyridin-2-yl)-3,6-diazabicyclo[3.1.1]heptane

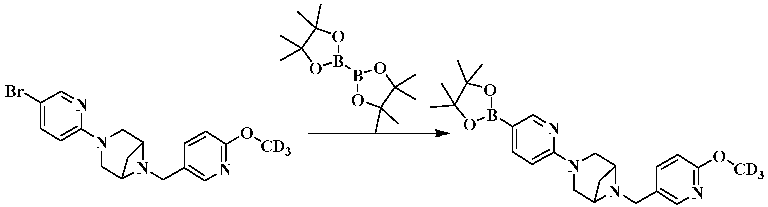

6-((6-(Methoxy-d3)pyridin-3-yl)methyl)-3-(5-(4,4,5,5-tetramethyl-1,3,2-dioxaborolan-2-yl)pyridin-2-yl)-3,6-diazabicyclo[3.1.1]heptane was obtained by using 3-(5-bromopyridin-2-yl)-6-((6-(methoxy-d3)pyridin-3-yl)methyl)-3,6-diazabicyclo[3.1.1]heptane as raw material with reference to step 7 of embodiment 1.

MS m/z (ESI): 426.2 [M+H]⁺.

### Step 3: 6-hydroxy-4-(6-(6-((6-(methoxy-d3)pyridin-3-yl)methyl)-3,6-diazabicyclo[3.1.1]heptan-3-yl)pyridin-3-yl)pyrazolo[1,5-a]pyridine-3-carbonitrile

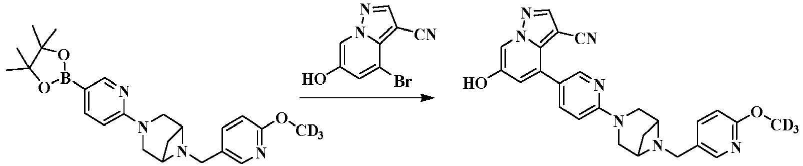

6-Hydroxy-4-(6-(6-((6-(methoxy-d3)pyridin-3-yl)methyl)-3,6-diazabicyclo[3.1.1]heptan-3-yl)pyridin-3-yl)pyrazolo[1,5-*a*]pyridine-3-carbonitrile was obtained by using 6-((6-(methoxy-d3)pyridin-3-yl)methyl)-3-(5-(4,4,5,5-tetramethyl-1,3,2-dioxaborolan-2-yl)pyridin-2-yl)-3,6-diazabicyclo[3.1.1]heptane and 4-bromo-6-hydroxypyrazolo[1,5-*a*]pyridine-3-carbonitrile as raw material with reference to step 8 of embodiment 1.

MS m/z (ESI): 457.2 [M+H]⁺.

### Step 4: 6-((1-cyanocyclopropyl)methoxy)-4-(6-(6-((6-(methoxy-d3)pyridin-3-yl)methyl)-3,6-diazabicyclo[3.1.1]heptan-3-yl)pyridin-3-yl)pyrazolo[1,5-a]pyridine-3-carbonitrile

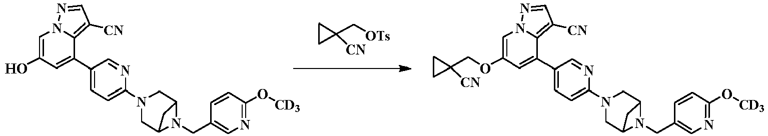

6-((1-Cyanocyclopropyl)methoxy)-4-(6-(6-((6-(methoxy-d3)pyridin-3-yl)methyl)-3,6-diazabicyclo[3.1.1]heptan-3-yl)pyridin-3-yl)pyrazolo[1,5-*a*]pyridine-3-carbonitrile was obtained by using 6-hydroxy-4-(6-(6-((6-(methoxy-d3)pyridin-3-yl)methyl)-3,6-diazabicyclo[3.1.1]heptan-3-yl)pyridin-3-yl)pyrazolo[1,5-*a*]pyridine-3-carbonitrile and (1-cyanocyclopropyl)methyl 4-methylbenzenesulfonate as raw material with reference to step 2 of embodiment 106.

MS m/z (ESI): 536.2 [M+H]⁺.

¹H NMR (400 MHz, Chloroform-d) δ 8.42 (d, J = 2.5 Hz, 1H), 8.23 (s, 1H), 8.13 (d, J= 2.2 Hz, 1H), 8.11 (d, J = 2.1 Hz, 1H), 7.91 - 7.74 (m, 2H), 7.19 (d, J = 2.1 Hz, 1H), 6.76 (d, J = 8.6 Hz, 1H), 6.71 (d, J = 8.8 Hz, 1H), 4.09 - 3.99 (m, 4H), 3.98 - 3.89 (m, 2H), 3.84 - 3.64 (m, 4H), 2.05 - 1.97 (m, 1H), 1.79 - 1.71 (m, 1H), 1.53 - 1.48 (m, 2H), 1.21 - 1.15 (m, 2H).

### Embodiment 108

### 6-((1-Hydroxycyclopropyl)ethynyl)-4-(6-(6-((6-methoxypyridin-3-yl)methyl)-3,6-diazabicydo[3.1.1]heptan-3-yl)pyridin-3-yl)pyrazolo[1,5-a]pyridine-3-carbonitrile

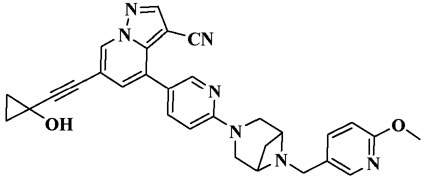

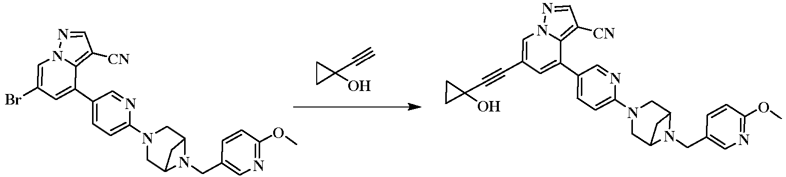

6-((1-Hydroxycyclopropyl)ethynyl)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (white solid) was obtained by using 6-bromo-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile and 1-ethynylcyclopropan-1-ol as raw material with reference to step 2 of embodiment 96.

MS m/z (ESI): 518.2 [M+H]⁺.

### Embodiment 109

### 6-(4-Fluoro-3-(fluoromethyl)-3-hydroxybut-1-yn-1-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

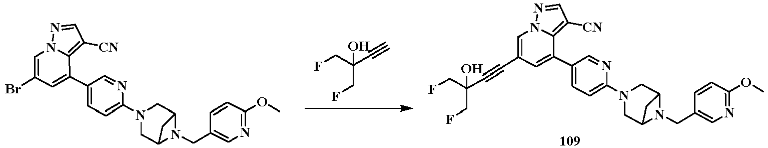

6-(4-Fluoro-3-(fluoromethyl)-3-hydroxybut-1-yn-1-yl)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (white solid) was obtained by using 6-bromo-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile and 1-fluoro-2-(fluoromethyl)but-3-yn-2-ol as raw material with reference to step 2 of embodiment 96.

MS m/z (ESI): 556.2 [M+H]⁺.

### Embodiment 110

### 4-(6-(6-((5-Fluoro-6-methoxypyridin-3-yl)methyl)-3,6-diazabicyclo[3.1.1]heptan-3-yl)pyridin-3-yl)-6-(3-(2-hydroxypropan-2-yl)azetidin-1-yl)pyrazolo[1,5-a]pyridine-3-carbonitrile

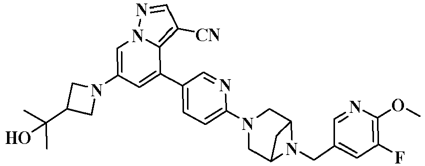

### Step 1: 4-(6-(6-((5-fluoro-6-methoxypyridin-3-yl)methyl)-3,6-diazabicyclo[3.1.1]heptan-3-yl)pyridin-3-yl)-6-(3-(2-hydroxypropan-2-yl)azetidin-1-yl)pyrazolo[1,5-a]pyridine-3-carbonitrile

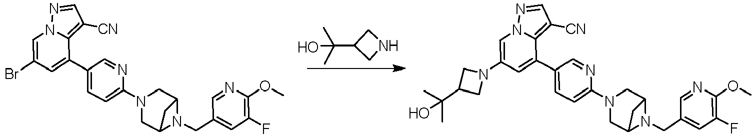

Tris(dibenzylideneacetone)dipalladium (5 mg, 0.005 mmol) and 4,5-bis(diphenylphosphino)-9,9-dimethylxanthene (3 mg, 0.005 mmol) was added to a mixed solution of 6-bromo-4-(6-(6-((5-fluoro-6-methoxypyridin-3-yl)methyl)-3,6-diazabicyclo[3.1.1]heptan-3-yl)pyridin-3-yl)pyrazolo[1,5-a]pyridine-3-carbonitrile (50 mg, 0.094 mmol), 2-(azetidin-3-yl)propan-2-ol (16 mg, 0.14 mmol ), cesium carbonate (122 mg, 0.376 mmol) and toluene (3 mL); and the mixture was replaced with nitrogen and stirred at 130 °C for 2 hours under microwave; and after the reaction was completed, the mixture was cooled and filtered; then the filtrate was concentrated under reduced pressure to dryness and separated by preparative chromatography to obtain white solid 4-(6-(6-((5-fluoro-6-methoxypyridin-3-yl)methyl)-3,6-diazabicyclo[3.1.1]heptan-3-yl)pyridin-3-yl)-6-(3-(2-hydroxypropan-2-yl)azetidin-1-yl)pyrazolo[1,5-*a*]pyridine-3-carbonitrile ( 15 mg, yield was 28%).

MS m/z (ESI):569.3 [M+H]⁺.

¹H NMR (400 MHz, CDCl₃) δ 8.39 (d, *J* = 2.1 Hz, 1H), 8.14 (s, 1H), 7.88 (s, 1H), 7.79 (dd, *J* = 8.8, 2.3 Hz, 1H), 7.72 (d, *J* = 1.6 Hz, 1H), 7.57 (s, 1H), 6.75 (d, *J* = 1.7 Hz, 1H), 6.69 (d, *J =* 8.7 Hz, 1H), 4.01 (s, 3H), 3.99 - 3.93 (m, 2H), 3.91 - 3.83 (m, 4H), 3.66 (s, 4H), 2.91 - 2.79 (m, 2H), 1.25 (s, 6H).

### Embodiment 111

### 3-Chloro-N-(1-(5-(3-cyano-6-(3-(2-hydroxypropan-2-yl)azetidin-1-yl)pyrazolo[1,5-a]pyridin-4-yl)pyridin-2-yl)-4-methylpiperidin-4-yl)picolinamide

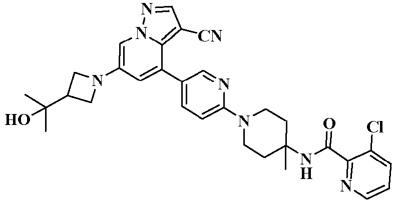

3-Chloro-*N*-(1-(5-(3-cyano-6-(3-(2-hydroxypropan-2-yl)azetidin-1-yl)pyrazolo[1,5-*a*]pyridin-4-yl)pyridin-2-yl)-4-methylpiperidin-4-yl)picolinamide was obtained by using 2-(azetidin-3-yl)propan-2-ol and *N*-(1-(5-(6-bromo-3-cyanopyrazolo[1,5-*a*]pyridin-4-yl)pyridin-2-yl)-4-methylpiperidin-4-yl)-3-chloromethylpyridinamide as raw material with reference to step 1 of embodiment 110.

MS m/z (ESI):585.2 [M+H]⁺.

¹H NMR (400 MHz, CDCl₃) δ 8.46 (d, *J* = 3.9 Hz, 1H), 8.34 (s, 1H), 8.13 (s, 1H), 7.94 (s, 1H), 7.83 (d, J= 8.1 Hz, 2H), 7.71 (s, 1H), 7.38 (dd, J = 8.1, 4.5 Hz, 1H), 6.77 (s, 1H), 4.14 (s, 2H), 3.96 (t, *J* = 7.6 Hz, 2H), 3.85 (t, *J* = 6.6 Hz, 2H), 3.48 (s, 2H), 2.48 (s, 2H), 1.89 - 1.80 (m, 2H), 1.25 (s, 3H), 1.24 (s, 6H).

### Embodiment 112

### 6-(6-Hydroxy-6-methyl-2-azaspiro[3.3]heptan-2-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

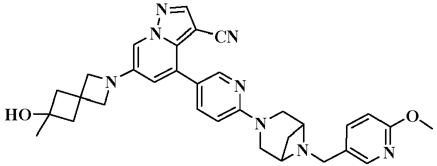

### Step 1: 6-bromo-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

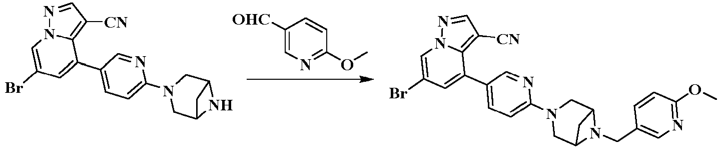

6-Bromo-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 4-(6-(3,6-diazabicyclo[3.1.1]heptan-3-yl)pyridin-3-yl)-6-bromopyrazolo[1,5-*a*]pyridine-3-carbonitrile and 6-methoxynicotinaldehyde as raw materials with reference to step 9 of embodiment 11.

MS m/z (ESI):516.1 [M+H]⁺.

### Step 2: 6-(6-hydroxy-6-methyl-2-azaspiro[3.3]heptan-2-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

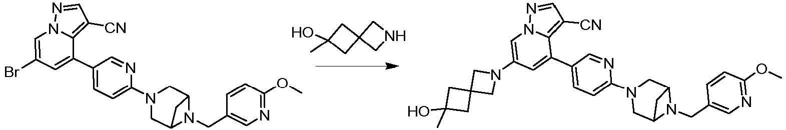

6-(6-Hydroxy-6-methyl-2-azaspiro[3.3]heptan-2-yl)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 6-methyl-2-azaspiro[3.3]heptan-6-ol as raw material with reference to step 1 of embodiment 110.

MS m/z (ESI):563.3 [M+H]⁺.

¹H NMR (400 MHz, CDCl₃) δ 8.38 (d, *J* = 2.1 Hz, 1H), 8.14 (d, J= 6.8 Hz, 2H), 7.89 (s, 1H), 7.80 (dd, *J* = 8.7, 2.3 Hz, 1H), 7.68 (d, *J* = 1.6 Hz, 1H), 6.76 (d, *J* = 8.5 Hz, 1H), 6.71 (t, *J* = 5.9 Hz, 2H),4.01- 3.93 (m, 10H), 3.74 (s, 4H), 2.96 (s, 1H), 2.39 (s, 4H), 1.40 (s, 3H).

### Embodiment 113

### 3-Chloro-N-(1-(5-(3-cyano-6-(6-hydroxy-6-methyl-2-azaspiro[3.3]heptan-2-yl)pyrazolo[1,5-a]pyridin-4-yl)pyridin-2-yl)-4-methylpiperidin-4-yl)picolinamide

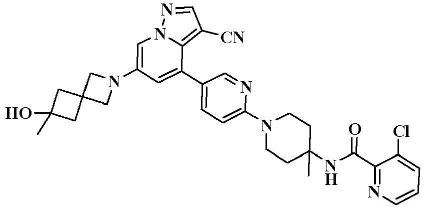

3-Chloro-N-(1-(5-(3-cyano-6-(6-hydroxy-6-methyl-2-azaspiro[3.3]heptan-2-yl)pyrazolo[1,5-*a*]pyridin-4-yl)pyridin-2-yl)-4-methylpiperidin-4-yl)picolinamide was obtained by using 6-methyl-2-azaspiro[3.3]heptan-6-ol as raw material with reference to step 1 of embodiment 110.

MS m/z (ESI): 597.2 [M+H]⁺.

¹H NMR (400 MHz, CDCl3) δ 8.47 (s, 1H), 8.32 (s, 1H), 8.13 (s, 1H), 7.94 (s, 1H), 7.83 (d, J = 8.6 Hz, 1H), 7.67 (s, 1H), 7.39 (d, J = 4.6 Hz, 1H), 6.89 (s, 1H), 6.71 (s, 1H), 4.15 (s, 2H), 3.94 (d, J = 5.8 Hz, 4H), 3.47 (s, 2H), 2.48 (s, 2H), 2.38 (s, 4H), 1.84 (s, 2H), 1.39 (s, 3H), 1.25 (s, 3H).

### Embodiment 114

### 6-(3-Hydroxy-3-methylazetidin-1-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

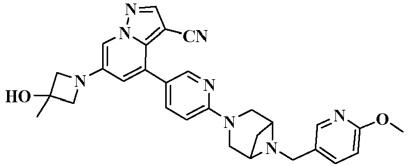

6-(3-Hydroxy-3-methylazetidin-1-yl)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 3-methylazetidin-3-ol as raw material with reference to step 1 of embodiment 110.

MS m/z (ESI):523.3 [M+H]⁺.

¹H NMR (400 MHz, CDCl₃) δ 8.40 (s, 1H), 8.15 (s, 3H), 7.82 (d, J= 7.4 Hz, 1H), 7.74 (s, 1H), 6.80 (d, J= 8.4 Hz, 1H), 6.75 (d, *J* = 1.7 Hz, 1H), 6.72 (d, *J* = 8.8 Hz, 1H), 4.21 (s, 2H), 4.01 (s, 2H), 3.93-3.92 (m, 7H), 3.84 (d, *J* = 7.3 Hz, 4H), 1.68 (s, 3H).

### [Not according to the invention] Embodiment 115

### 6-(3-Hydroxy-3-methylpyrrolidin-1-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

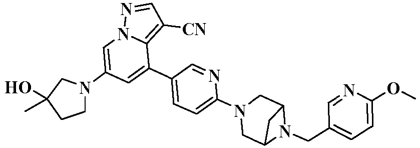

6-(3-Hydroxy-3-methylpyrrolidin-1-yl)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 3-methylpyrrolidin-3-ol as raw material with reference to step 1 of embodiment 110.

MS m/z (ESI):537.3 [M+H]⁺.

¹H NMR (400 MHz, CDCl₃) δ 8.44 (s, 2H), 8.16 (m, 2H), 7.88 (d, *J* = 6.7 Hz, 1H), 7.80 (s, 1H), 6.93 (s, 1H), 6.86 *(d, J=* 8.8 Hz, 1H), 6.76 (d, *J* = 8.6 Hz, 1H), 4.18-4.13 (m, 2H), 4.07-4.03(m, 2H), 3.93 (s, 3H), 3.65-3.61 (m, 2H), 3.46-3.41 (m, 2H), 2.24 - 2.12 (m, 3H), 2.0-1.96 (m, 3H), 1.31 (s, 3H).

### [Not according to the invention] Embodiment 116

### 6-(4-Hydroxy-4-methylpiperidin-1-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

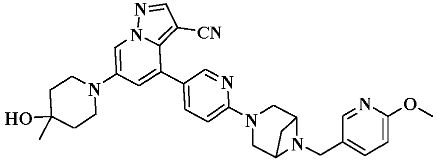

6-(4-Hydroxy-4-methylpiperidin-1-yl)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 4-methylpiperidin-4-ol as raw material with reference to step 1 of embodiment 110.

MS m/z (ESI): 551.3 [M+H]⁺.

¹H NMR (400 MHz, CDCl₃) δ 8.41 (s, 1H), 8.18 (s, 1H), 8.14 (s, 1H), 8.07 (s, 2H), 7.82 (d, J= 6.7 Hz, 1H), 7.22 (d, J=1.6 Hz, 1H), 6.79 (d, *J* = 8.6 Hz, 1H), 6.71 (d, *J* = 8.7 Hz, 1H), 3.99 (s, 2H), 3.93 (s, 3H), 3.85 (s, 4H), 3.36-3.32 (m, 2H), 3.23 - 3.15 (m, 2H), 1.85 - 1.74 (m, 6H), 1.35 (s, 3H).

### Embodiment 117

### 6-((1-Cyanocyclobutyl)methoxy)-4-(6-(6-((6-methoxypyridin-3-yl)methyl)-3,6-diazabicydo[3.1.1]heptan-3-yl)pyridin-3-yl)pyrazolo[1,5-a]pyridine-3-carbonitrile

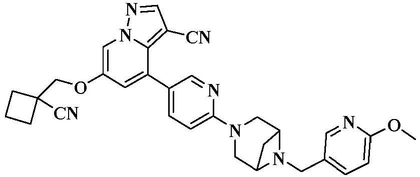

6-((1-Cyanocyclobutyl)methoxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (white solid) was obtained with reference to embodiment 106.

MS m/z (ESI): 547.2 [M+H]⁺.

### Embodiment 118

### 6-((3-Cyanooxetan-3-yl)methoxy)-4-(6-(6-((6-methoxypyridin-3-yl)methyl)-3,6-diazabicydo[3.1.1]heptan-3-yl)pyridin-3-yl)pyrazolo[1,5-a]pyridine-3-carbonitrile

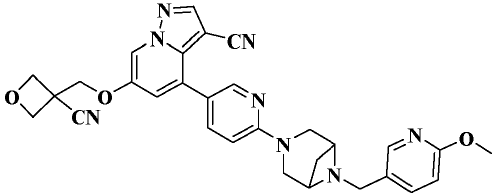

6-((3-Cyanooxetan-3-yl)methoxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (12 mg, white solid, 29%) was obtained with reference to embodiment 106.

MS m/z (ESI): 549.2 [M+H]⁺.

H NMR (400 MHz, DMSO) δ 8.83 (d, *J* = 2.0 Hz, 1H), 8.64 (s, 1H), 8.42 (d, *J* = 2.1 Hz, 1H), 8.06 (s, 1H), 7.86 *(d, J=* 8.8 Hz, 1H), 7.68 (d, *J* = 6.9 Hz, 1H), 7.40 (s, 1H), 6.81 - 6.76 (m, 2H), 4.93 (d, *J* = 6.5 Hz, 2H), 4.70 (s, 2H), 4.68 *(d, J=* 6.5 Hz, 2H), 3.82 (s, 3H), 3.67 (d, *J* = 5.3 Hz, 2H), 3.53-3.47 (m, 6H), 2.01 - 1.99 (m, 2H).

### [Not according to the invention] Embodiment 119

### 6-(((1-Hydroxycyclopropyl)methyl)(methyl)amino)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

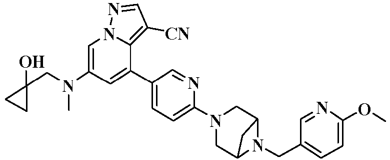

6-(((1-Hydroxycyclopropyl)methyl)(methyl)amino)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 1-((methylamino)methyl)cyclopropan-1-ol as raw material with reference to step 1 of embodiment 110.

MS m/z (ESI):537.3 [M+H]⁺.

### Embodiment 120

### 6-(((1-Cyanocyclopropyl)methyl)amino)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

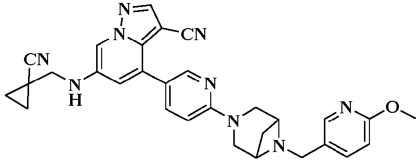

6-(((1-Cyanocyclopropyl)methyl)amino)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 1-(aminomethyl)cyclopropane-1-carbonitrile as raw material with reference to step 1 of embodiment 110.

MS m/z (ESI):532.3 [M+H]⁺.

### Embodiment 121

### 4-(6-(6-((6-Cyclopropoxypyridin-3-yl)methyl)-3,6-diazabicyclo[3.1.1]heptan-3-yl)pyridin-3-yl)-6-(3-(2-hydroxypropan-2-yl)azetidin-1-yl)pyrazolo[1,5-a]pyridine-3-carbonitrile

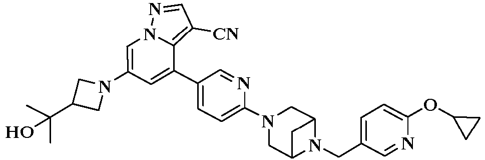

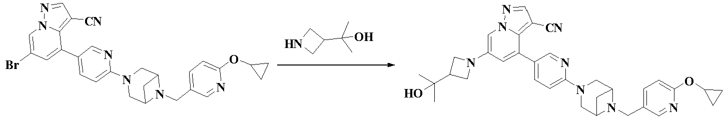

4-(6-(6-((6-Cyclopropoxypyridin-3-yl)methyl)-3,6-diazabicyclo[3.1.1]heptan-3-yl)pyridin-3-yl)-6-(3-(2-hydroxypropan-2-yl)azetidin-1-yl)pyrazolo[1,5-*a*]pyridine-3-carbonitrile was obtained by using 6-bromo-4-(6-(6-((6-cyclopropoxypyridin-3-yl)methyl)-3,6-diazabicyclo[3.1.1]heptan-3-yl)pyridin-3-yl)pyrazolo[1,5-*a*]pyridine-3-carbonitrile as raw materials with reference to step 2 of embodiment 110.

MS m/z (ESI): 577.2[M+H]⁺.

### Embodiment 122

### 6-(3-(2-Hydroxypropan-2-yl)azetidin-1-yl)-4-(6-(6-((6-(methoxy-d3)pyridin-3-yl)methyl)-3,6-diazabicyclo[3.1.1]heptan-3-yl)pyridin-3-yl)pyrazolo[1,5-a]pyridine-3-carbonitrile

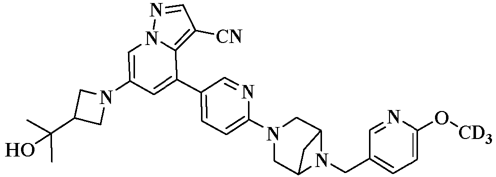

6-(3-(2-Hydroxypropan-2-yl)azetidin-1-yl)-4-(6-(6-((6-(methoxy-d3)pyridin-3-yl)methyl)-3,6-diazabicyclo[3.1.1]heptan-3-yl)pyridin-3-yl)pyrazolo[1,5-*a*]pyridine-3-carbonitrile (20 mg, yellow solid, 30%) was obtained by using 6-bromo-4-(6-(6-((6-(methoxy-d3)pyridin-3-yl)methyl)-3,6-diazabicyclo[3.1.1]heptan-3-yl)pyridin-3-yl)pyrazolo[1,5-*a*]pyridine-3-carbonitrile as raw material with reference to step 1 of embodiment **110.**

MS m/z (ESI): 554.3 [M+H]⁺.

¹H NMR (400 MHz, MeOD) δ 8.32 (d, J = 2.1 Hz, 1H), 8.24 (s, 1H), 8.08 (d, J = 2.0 Hz, 1H), 7.84 (d, J = 1.6 Hz, 1H), 7.82 (dd, J = 8.8, 2.4 Hz, 1H), 7.71 (dd, J = 8.6, 2.3 Hz, 1H), 6.92 (d, J = 1.7 Hz, 1H), 6.87 (d, J = 8.8 Hz, 1H), 6.78 (d, J = 8.6 Hz, 1H), 3.97 (t, J = 7.8 Hz, 2H), 3.91 - 3.85 (m, 4H), 3.79 (d, J = 5.7 Hz, 2H), 3.65 (s, 1H), 3.62 (s, 3H), 2.92 - 2.84 (m, 1H), 2.71 (s, 1H), 1.70 (d, J = 8.9 Hz, 1H), 1.21 (s, 6H).

### [Not according to the invention] Embodiment 123

### N-(3-cyano-4-(6-(6-((6-methoxypyridin-3-yl)methyl)-3,6-diazabicyclo[3.1.1]heptan-3-yl)pyridin-3-yl)pyrazolo[1,5-a]pyridin-6-yl)-2-hydroxy-2-methylpropanamide

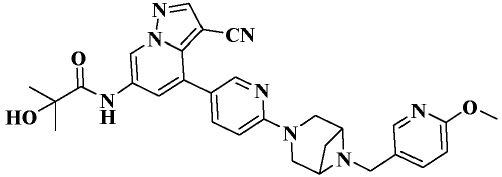

### Step 1: tert-butyl (3-cyano-4-(6-(6-((6-methoxypyridin-3-yl)methyl)-3,6-diazabicyclo[3.1.1]heptan-3-yl)pyridin-3-yl)pyrazolo[1,5-a]pyridin-6-yl)carbamate

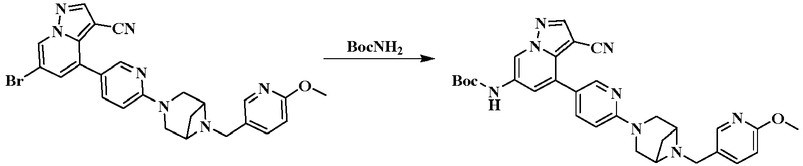

*Tert-butyl* (3-cyano-4-(6-(6-((6-methoxypyridin-3-yl)methyl)-3,6-diazabicyclo[3.1.1]heptan-3-yl)pyridin-3-yl)pyrazolo[1,5-*a*]pyridin-6-yl)carbamate was obtained by using *tert-butyl* carbamate as raw material with reference to step 1 of embodiment 110.

MS m/z (ESI):553.3 [M+H]⁺.

### Step 2: 6-amino-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

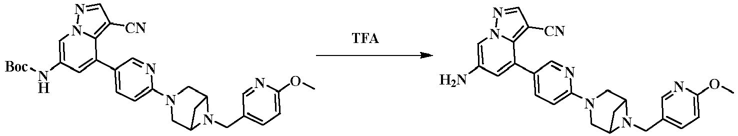

6-Amino-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using *tert-butyl* (3-cyano-4-(6-(6-((6-methoxypyridin-3-yl)methyl)-3,6-diazabicyclo[3.1.1]heptan-3-yl)pyridin-3-yl)pyrazolo[1,5-*a*]pyridin-6-yl)carbamate as raw material with reference to step 1 of embodiment 110.

MS m/z (ESI):453.3 [M+H]⁺.

### Step 3: N-(3-cyano-4-(6-(6-((6-methoxypyridin-3-yl)methyl)-3,6-diazabicyclo[3.1.1]heptan-3-yl)pyridin-3-yl)pyrazolo[1,5-a]pyridin-6-yl)-2-hydroxy-2-methylpropanamide

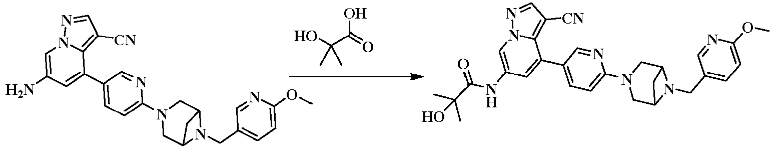

N-(3-cyano-4-(6-(6-((6-methoxypyridin-3-yl)methyl)-3,6-diazabicyclo[3.1.1]heptan-3-yl)pyridin-3-yl)pyrazolo[1,5-*a*]pyridin-6-yl)-2-hydroxy-2-methylpropanamide was obtained by using 2-hydroxy-2-methylpropanoic acid as raw material with reference to step 4 of embodiment 28.

MS m/z (ESI): 539.2 [M+H]⁺.

### [Not according to the invention] Embodiment 124

### 6-((1-Aminocyclopropyl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

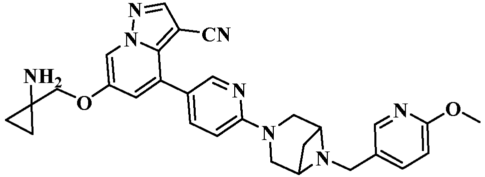

### Step 1: preparation of tert-butyl (1-(((3-cyano-4-(6-(6-((6-methoxypyridin-3-yl)methyl)-3,6-diazabicyclo[3.1.1]heptan-3-yl)pyridin-3-yl)pyrazolo[1,5-a]pyridin-6-yl)oxo)methyl)cyclopropyl)carbamate

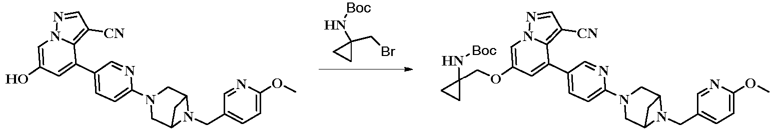

*tert-butyl* (1-(((3-cyano-4-(6-(6-((6-methoxypyridin-3-yl)methyl)-3,6-diazabicyclo[3.1.1]heptan-3-yl)pyridin-3-yl)pyrazolo[1,5-*a*]pyridin-6-yl)oxy)methyl)cyclopropyl)carbamate was obtained by using *tert-*butyl (1-(bromomethyl)cyclopropyl)carbamate and 6-hydroxy-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile as raw materials with reference to step 5 of embodiment 37.

MS m/z (ESI): 623.3 [M+H]⁺.

### Step 2: preparation of 6-((1-aminocyclopropyl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

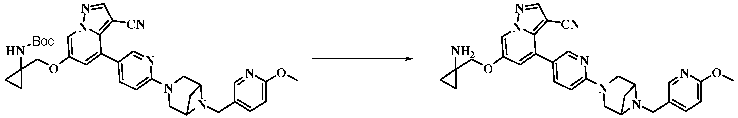

6-((1-Aminocyclopropyl)methoxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using *tert-butyl* (1-(((3-cyano-4-(6-(6-((6-methoxypyridin-3-yl)methyl)-3,6-diazabicyclo[3.1.1]heptan-3-yl)pyridin-3-yl)pyrazolo[1,5-*a*]pyridin-6-yl)oxo)methyl)cyclopropyl)carbamate as raw material with reference to step 4 of embodiment 43.

MS m/z (ESI): 523.3 [M+H]⁺.

### [Not according to the invention] Embodiment 125

### 6-((Cis-3-hydroxy-3-methylcyclobutyl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

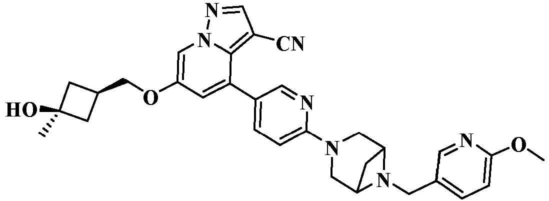

### Step 1: cis-3-(hydroxymethyl)-1-methylcyclobutan-1-ol

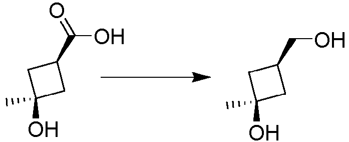

Borane-tetrahydrofuran solution (7.5 mL, 15 mmol, 2 M) was added dropwise to a solution of cis-3-hydroxy-3-methylcyclobutane-1-carboxylic acid (1 g, 7.68 mmol) in tetrahydrofuran (10 mL) at 0 °C, then the mixture was stirred at room temperature for 2 hours; and after the reaction was completed, the reaction mixture was quenched with methanol, then extracted with ethyl acetate; the organic phase was combined, washed with saturated saline, dried over anhydrous sodium sulfate, filtered and concentrated under reduced pressure to dryness to obtain colorless oil cis-3-(hydroxymethyl)-1-methylcyclobutan-1-ol (600 mg, crude product).

### Step 2: (cis-3-hydroxy-3-methylcyclobutyl)methyl 4-methylbenzenesulfonate

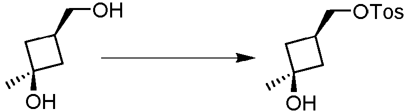

P-toluenesulfonyl chloride (987 mg ,5.17 mmol) was added to a mixed solution of cis-3-(hydroxymethyl)-1-methylcyclobutan-1-ol (600 mg, 5.17 mmol), triethylamine (1.04 g ,10.34 mmol) and dichloromethane (10 mL), and then the mixture was stirred at room temperature for 16 hours; after the reaction was completed, the reaction mixture was quenched with water, and then extracted with ethyl acetate; the organic phase was combined, washed with saturated saline, dried over anhydrous sodium sulfate, filtered, concentrated under reduced pressure to dryness, and then separated by column chromatography to obtain colorless oil (cis-3-hydroxy-3-methylcyclobutyl)methyl 4-methylbenzenesulfonate (800 mg, yield was 57%).

### Step 3: 6-((cis-3-hydroxy-3-methylcyclobutyl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

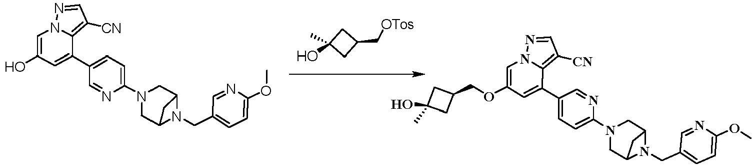

6-((Cis-3-hydroxy-3-methylcyclobutyl)methoxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 6-hydroxy-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile and (cis-3-hydroxy-3-methylcyclobutyl)methyl 4-methylbenzenesulfonate as raw materials with reference to step 2 of embodiment 106.

MS m/z (ESI):552.3[M+H]⁺.

¹H NMR (400 MHz, CDCl₃) δ 8.41 (d, J = 1.9 Hz, 1H), 8.21 (s, 1H), 8.13 (s, 2H), 7.87 - 7.73 (m, 2H), 7.13 (d, J = 1.6 Hz, 1H), 6.75 (d, J = 8.5 Hz, 1H), 6.70 (d, J = 8.8 Hz, 1H), 4.03 (d, J = 5.8 Hz, 2H), 3.92 (s, 7H), 3.70 (s, 4H), 2.89 (s, 1H), 2.41-2.36(m, 1H), 2.32-2.27(m, 2H), 2.05-2.00 (m, 3H), 1.45 (s, 3H).

### [Not according to the invention] Embodiment 126

### 6-((3-(Hydroxymethyl)bicyclo[1.1.1]pentan-1-yl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

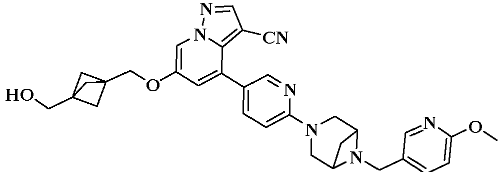

### Step 1: bicyclo[1.1.1]pentane-1,3-diyldimethanol

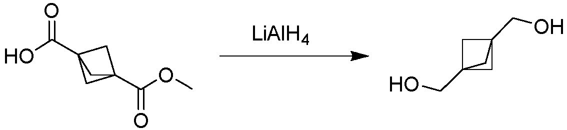

Lithium aluminum hydride (670 mg,17.63 mmol) was added to a solution of 3-(methyl ester <methoxycarbonyl>)bicyclo[1.1.1]pentane-1-carboxylic acid (1 g, 5.88 mmol) in tetrahydrofuran (10 mL) at 0 °C , then the mixture was warmed to room temperature and stirred for 16 hours; after the reaction was completed, the reaction mixture was quenched with sodium sulfate decahydrate, filtered, and the filtrate was concentrated under reduced pressure to dryness to obtain colorless oil bicyclo[1.1.1]pentane-1,3-diyldimethanol (640 mg,85%).

¹H NMR (400 MHz, DMSO) δ 4.39 (t, *J* = 5.5 Hz, 2H), 3.35 (d, *J* = 5.5 Hz, 4H), 1.45 (s, 6H).

### Step 2: (3-(hydroxymethyl)bicyclo[1.1.1]pentan-1-yl)methyl 4-methylbenzenesulfonate

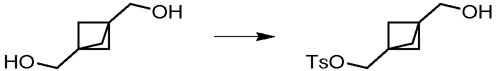

(3-(Hydroxymethyl)bicyclo[1.1.1]pentan-1-yl)methyl 4-methylbenzenesulfonate was obtained by using bicyclo[1.1.1]pentane-1,3-diyldimethanol as raw material with reference to step 2 of embodiment 125.

### Step 3: 6-((3-(hydroxymethyl)bicyclo[1.1.1]pentan-1-yl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

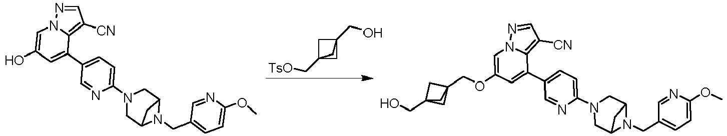

6-((3-(Hydroxymethyl)bicyclo[1.1.1]pentan-1-yl)methoxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 6-hydroxy-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile and (3-(hydroxymethyl)bicyclo[1.1.1]pentan-1-yl)methyl 4-methylbenzenesulfonate as raw materials with reference to step 2 of embodiment 106.

MS m/z (ESI):564.3[M+H]⁺.

### Embodiment 127

### (E)-6-(3-hydroxy-3-methylbut-1-en-1-yl)-4-(6-(6-((6-methoxy-d3)pyridin-3-yl)methyl)-3,6-diazabicyclo[3.1.1]heptan-3-yl)pyridin-3-yl)pyrazolo[1,5-a]pyridine-3-carbonitrile

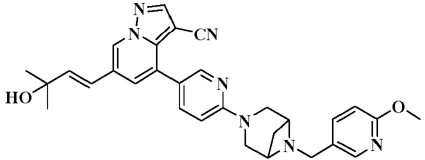

(E)-6-(3-hydroxy-3-methylbut-1-en-1-yl)-4-(6-(6-((6-methoxy-d3)pyridin-3-yl)methyl)-3,6-diazabicyclo[3.1.1]heptan-3-yl)pyridin-3-yl)pyrazolo[1,5-*a*]pyridine-3-carbonitrile (10 mg, white solid, 30%) was obtained by using 6-bromo-4-(6-(6-((6-(methoxy-d3)pyridin-3-yl)methyl)-3,6-diazabicyclo[3.1.1]heptan-3-yl)pyridin-3-yl)pyrazolo[1,5-*a*]pyridine-3-carbonitrile and (E)-2-methyl-4-(4,4,5,5-tetramethyl-1,3,2-dioxaborolan-2-yl)but-3-en-2-ol as raw materials with reference to step 8 of embodiment 1.

MS m/z (ESI): 522.3 [M+H]⁺.

¹H NMR (400 MHz, MeOD) δ 8.71 (s, 1H), 8.40 (s, 1H), 8.36 (d, J = 2.1 Hz, 1H), 8.09 (s, 1H), 7.86 (dd, J = 8.8, 2.3 Hz, 1H), 7.72 (dd, J = 8.5, 2.3 Hz, 1H), 7.64 (s, 1H), 6.89 (d, J = 8.9 Hz, 1H), 6.78 (d, J = 8.6 Hz, 1H), 6.65 (q, J = 16.1 Hz, 2H), 3.91 (s, 1H), 3.88 (s, 4H), 3.79 (d, J = 5.5 Hz, 2H), 3.67 - 3.62 (m, 4H), 2.71 (s, 1H), 1.71 (d, J = 9.0 Hz, 1H), 1.40 (s, 6H).

### [Not according to the invention] Embodiment 128

### 6-((3-Hydroxybicyclo[1.1.1]pentan-1-yl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

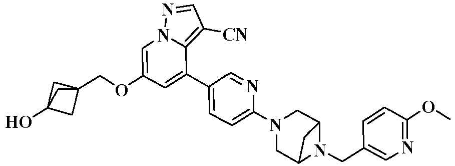

### Step 1: 6-((3-hydroxybicyclo[1.1.1]pentan-1-yl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

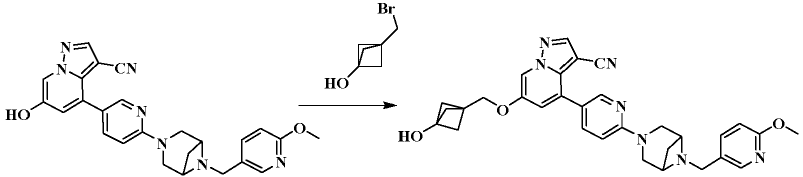

A mixture of 6-hydroxy-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (50 mg, 0.11 mmol), 3-(bromomethyl)bicyclo[1.1.1]pentan-1-ol (29 mg, 0.17 mmol), potassium carbonate (46 mg , 0.33 mmol) and acetonitrile (5 mL) was stirred at 70 °C for 2 hour, then the mixture was cooled to room temperature and filtered, and the filtrate was concentrated under reduced pressure to dryness and separated by preparative chromatography to obtain 6-((3-hydroxybicyclo[1.1.1]pentan-1-yl)methoxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile(10 mg, yield was 17%).

MS m/z (ESI): 550.2 [M+H]⁺.

¹H NMR (400 MHz, CDCl₃) δ 8.45 (s, 1H), 8.25 - 8.15 (m, 3H), 7.85 (d, J= 4.0 Hz, 1H), 7.17 (s, 1H), 6.84 (d, *J* = 9.2 Hz, 1H), 6.74 (d, *J* = 7.8 Hz, 2H), 4.09 (s, 2H), 4.02 (s, 2H), 3.94 (s, 3H), 3.24-3.19 (m, 2H), 2.93-2.88(m, 2H), 2.24 - 2.20 (m, 2H), 1.58 (s, 6H).

### Embodiment 129

### 6-(4-Hydroxy-4-methylpent-1-yn-1-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

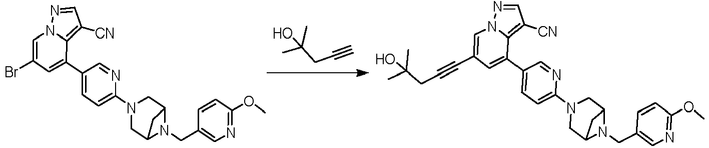

6-(4-Hydroxy-4-methylpent-1-yn-1-yl)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 2-methylpent-4-yn-2-ol as raw material with reference to step 2 of embodiment 31.

MS m/z (ESI):534.3 [M+H]⁺.

¹H NMR (400 MHz, CDCl₃) δ 8.62 (s, 1H), 8.41 (d, J= 2.1 Hz, 1H), 8.30 (s, 1H), 8.13 (s, 1H), 7.78 (dd, *J* = 8.8, 2.4 Hz, 2H), 7.31 (s, 1H), 6.75 (d, *J* = 8.6 Hz, 1H), 6.70 (d, *J* = 8.8 Hz, 1H), 3.98-3.93 (m, 7H), 3.72 (s, 4H), 2.66 (s, 2H), 1.41 (s, 6H).

### Embodiment 130

### 6-((1-Aminocyclopropyl)ethynyl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

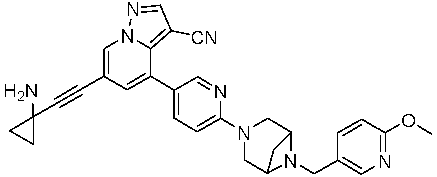

6-((1-Aminocyclopropyl)ethynyl)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 1-ethynylcyclopropane-1-amine as raw material with reference to step 2 of embodiment 31.

MS m/z (ESI): 517.2 [M+H]⁺.

### Embodiment 131

### 6-(3-Hydroxy-3-methylbut-1-yn-1-yl)-4-(6-((3S,5R)-4-((6-methoxypyridin-3-yl)methyl)-3,5-dimethylpiperazin-1-yl)pyridin-3-yl)pyrazolo[1,5-a]pyridine-3-carbonitrile

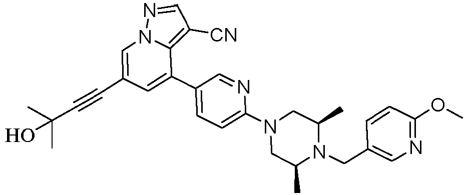

### Step 1: tert-butyl (3S,5R)-4-((6-methoxypyridin-3-yl)methyl)-3,5-dimethylpiperazine-1-carboxylate

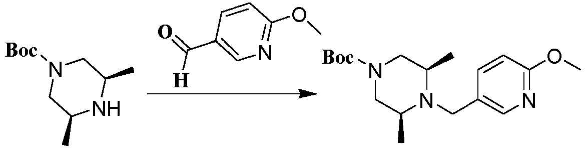

*Tert*-butyl (3*S*,5*R*)-4-((6-methoxypyridin-3-yl)methyl)-3,5-dimethylpiperazine-1-carboxylate was obtained by using *tert*-butyl (3*S*,5*R*)-3,5-dimethylpiperazine-l-carboxylate and 6-methoxynicotinaldehyde as raw materials with reference to step 9 of embodiment 11.

MS m/z (ESI):336.2[M+H]⁺.

### Step 2: (2S,6R)-1-((6-methoxypyridin-3-yl)methyl)-2,6-dimethylpiperazine

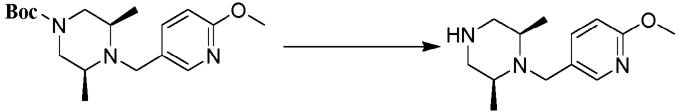

(2*S*,6*R*)-1-((6-methoxypyridin-3-yl)methyl)-2,6-dimethylpiperazine was obtain by using *tert-butyl* (3*S*,5*R*)-4-((6-methoxypyridin-3-yl)methyl)-3,5-dimethylpiperazine-1-carboxylate as raw material with reference to step 8 of embodiment 11.

MS m/z (ESI):236.2[M+H]⁺.

### Step 3: 6-bromo-4-(6-((3S,5R)-4-((6-methoxypyridin-3-yl)methyl)-3,5-dimethylpiperazin-1-yl)pyridin-3-yl)pyrazolo[1,5-a]pyridine-3-carbonitrile

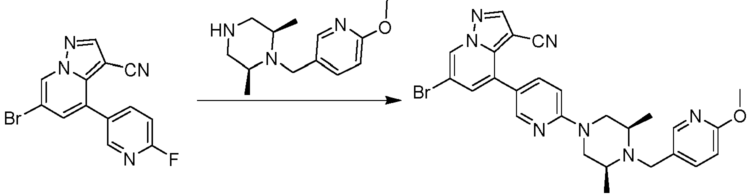

6-Bromo-4-(6-((3*S*,5*R*)-4-((6-methoxypyridin-3-yl)methyl)-3,5-dimethylpiperazin-1-yl)pyridin-3-yl)pyrazolo[1,5-*a*]pyridine-3-carbonitrile was obtained by using 6-bromo-4-(6-fluoropyridin-3-yl)pyrazolo[1,5-*a*]pyridine-3-carbonitrile and (2*R*,6*S*)-1-((6-methoxypyridin-3-yl)methyl)-2,6-dimethylpiperazine as raw materials with reference to step 7 of embodiment 11

MS m/z (ESI):532.1[M+H]⁺.

### Step 4: 6-(3-hydroxy-3-methylbut-1-yn-1-yl)-4-(6-((3S,5R)-4-((6-methoxypyridin-3-yl)methyl)-3,5-dimethylpiperazin-1-yl)pyridin-3-yl)pyrazolo[1,5-a]pyridine-3-carbonitrile

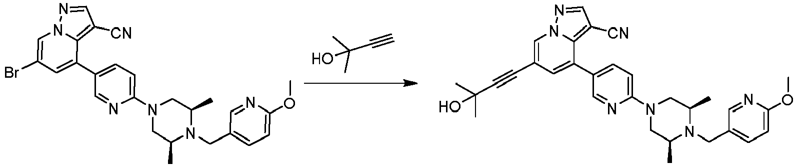

6-(3-Hydroxy-3-methylbut-1-yn-1-yl)-4-(6-((3*S*,5*R*)-4-((6-methoxypyridin-3-yl)methyl)-3,5-dimethylpiperazin-1-yl)pyridin-3-yl)pyrazolo[1,5-*a*]pyridine-3-carbonitrile (white solid) was obtained by using 6-bromo-4-(6-((3*S*,5*R*)-4-((6-methoxypyridin-3-yl)methyl)-3,5-dimethylpiperazin-1-yl)pyridin-3-yl)pyrazolo[1,5-*a*]pyridine-3-carbonitrile as raw material with reference to step 2 of embodiment 31.

MS m/z (ESI):536.3[M+H]⁺.

### Embodiment 132

### 6-(3-hydroxy-3-methylbut-1-yn-1-yl)-4-(6-(6-((6-methoxypyridin-3-yl)methyl-d2)-3,6-diazabicyclo[3.1.1]hcptan-3-yl)pyridin-3-yl)pyrazolo[1,5-a]pyridine-3-carbonitrile

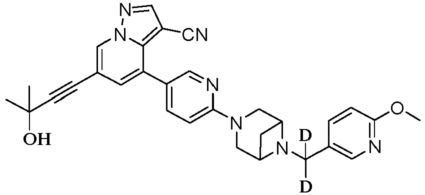

### Step 1: (6-methoxypyridin-3-yl)methan-d2-ol

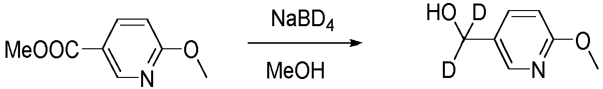

Methyl 6-methoxynicotinate (500 mg, 3 mmol) was dissolved in 10 mL of MeOH, and sodium boron deuteride (187 mg, 4.5 mmol) was added at 0 °C, and the mixture was stirred at room temperature for 3 hours. 10 mL of water was added, and the mixture was extracted with ethyl acetate (20mL*3). The organic phase was washed with saturated saline, dried over anhydrous sodium sulfate. The residue was filtered, evaporated to dryness; and the crude product was separated by column chromatography (eluted with petroleum ether/ethyl acetate = 10/1) to obtain (6-methoxypyridin-3-yl)methan-d2-ol (330 mg, yield was 78%).

MS m/z (ESI): 142.2 [M+H]⁺.

### Step 2: 5-(bromomethyl-d2)-2-methoxypyridine

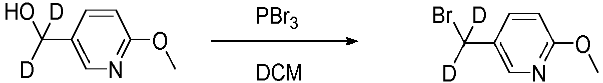

(6-Methoxypyridin-3-yl)methan-d2-ol (330 mg, 2.3 mmol) was dissolved in 10 mL of DCM, and Pbr3₃ (824 mg, 3 mmol) was added at 0°C under the protection of nitrogen, and the mixture was stirred at room temperature for 3 hours. 10 mL of water was added, and the mixture was extracted with ethyl acetate (20mL*3). The organic phase was washed with saturated saline, dried over anhydrous sodium sulfate. The residue was filtered, evaporated to dryness; and the crude product was separated by column chromatography (eluted with petroleum ether/ethyl acetate = 10/1) to obtain 5-(bromomethyl-d2)-2-methoxypyridine (375 mg, yield was 80%).

MS m/z (ESI): 204.0 [M+H]⁺.

### Step 3: 6-bromo-4-(6-(6-((6-methoxypyridin-3-yl)methyl-d2)-3,6-diazabicyclo[3.1.1]heptan-3-yl)pyridin-3-yl)pyrazolo[1,5-a]pyridine-3-carbonitrile

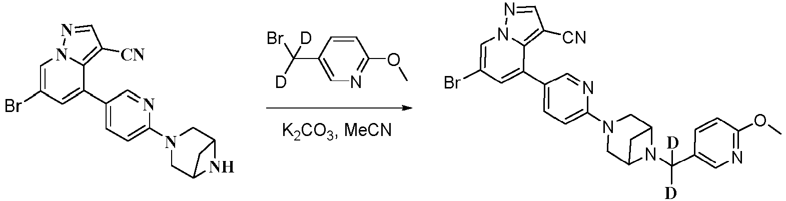

5-(Bromomethyl-d2)-2-methoxypyridine (370 mg, 1.8 mmol), 4-(6-(3,6-diazabicyclo[3.1.1]heptan-3-yl)pyridin-3-yl)-6-bromopyrazolo[1,5-*a*]pyridine-3-carbonitrile (605 mg, 1.5 mmol), potassium carbonate (621 mg, 4.5 mmol) were dissolved in 20 mL, and the reaction was carried out at 80 °C for 3 hours under the protection of nitrogen. 10 mL of water was added, and the mixture was extracted with ethyl acetate (20mL*3). The organic phase was washed with saturated saline, dried over anhydrous sodium sulfate. The residue was filtered and evaporated to dryness, and the crude product was separated by column chromatography (eluted with dichloromethane/methanol = 10/1) to obtain 6-bromo-4-(6-(6-((6-methoxypyridin-3-yl)methyl-d2)-3,6-diazabicyclo[3.1.1]heptan-3-yl)pyridin-3-yl)pyrazolo[1,5-*a*]pyridine-3-carbonitrile (426 mg, yield was 55%).

MS m/z (ESI): 518.1 [M+H]⁺.

### Step 4: 6-(3-hydroxy-3-methylbut-1-yn-1-yl)-4-(6-(6-((6-methoxypyridin-3-yl)methyl-d2)-3,6-diazabicyclo[3.1.1]heptan-3-yl)pyridin-3-yl)pyrazolo[1,5-a]pyridine-3-carbonitrile

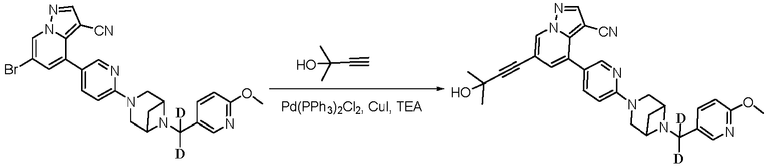

6-(3-Hydroxy-3-methylbut-1-yn-1-yl)-4-(6-(6-((6-methoxypyridin-3-yl)methyl-d2)-3,6-diazabicyclo[3.1.1]heptan-3-yl)pyridin-3-yl)pyrazolo[1,5-*a*]pyridine-3-carbonitrile (14 mg, white solid, yield was 26%) was obtained with reference to step 2 of embodiment 31.

MS m/z (ESI): 522.2 [M+H]⁺.

### [Not according to the invention] Embodiment 133

### 4-(6-(6-((6-Cyclopropoxypyridin-3-yl)methyl)-3,6-diazabicyclo[3.1.1]heptan-3-yl)pyridin-3-y1)-6-(2-hydroxy-2-methylpropoxy)pyrazolo[1,5-a]pyridine-3-carbonitrile

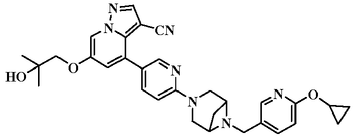

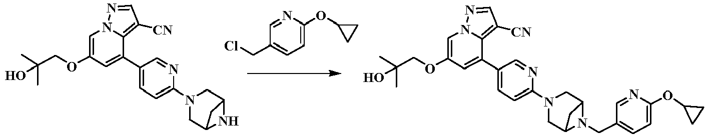

5-(Chloromethyl)-2-cyclopropoxypyridine (91 mg, 0.495 mmol) and 4-(6-(3,6-diazabicyclo[3.1.1]heptan-3-yl)pyridin-3-yl)-6-(2-hydroxy-2-methylpropoxy)pyrazolo[1,5-*a*]pyridine-3-carbonitrile (200 mg, 0.495 mmol) were dissolved in DMSO (15 mL), and cesium carbonate (322 mg, 0.989 mmol) was added thereto. The reaction was stirred at 90 °C for 2 hours. Water was added to the reaction mixture, and then the mixture was extracted with ethyl acetate. The organic phase was dried and evaporated to dryness. The crude product was purified by prep-HPLC to obtain 4-(6-(6-((6-cyclopropoxypyridin-3-yl)methyl)-3,6-diazabicyclo[3.1.1]heptan-3-yl)pyridin-3-yl)-6-(2-hydroxy-2-methylpropoxy)pyrazolo[1,5-*a*]pyridine-3-carbonitrile (33.6 mg, yield was 12%).

MS m/z (ESI): 552.2 [M+H]⁺.

¹H NMR (400 MHz, DMSO-d6) δ 8.68 (d, J = 2.0 Hz, 1H), 8.59 (s, 1H), 8.41 (d, J = 2.5 Hz, 1H), 8.10 (d, J = 2.4 Hz, 1H), 7.85 (dd, J = 8.8, 2.6 Hz, 1H), 7.70 (dd, J = 8.5, 2.4 Hz, 1H), 7.31 (d, J = 2.1 Hz, 1H), 6.80 (dd, J = 8.7, 3.9 Hz, 2H), 4.72 (s, 1H), 4.21 - 4.11 (m, 1H), 3.88 (s, 2H), 3.80 - 3.64 (m, 4H), 3.62 - 3.47 (m, 4H), 2.55 - 2.53 (m, 1H), 1.59 (d, J = 8.4 Hz, 1H), 1.23 (s, 6H), 0.79 - 0.71 (m, 2H), 0.68 - 0.59 (m, 2H).

### Embodiment 134

### 6-((1-Hydroxycyclopropyl)ethynyl)-4-(6-(6-((6-methoxypyridin-3-yl)methyl-d2)-3,6-diazabicyclo[3.1.1]heptan-3-yl)pyridin-3-yl)pyrazolo[1,5-a]pyridine-3-carbonitrile

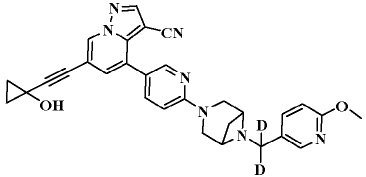

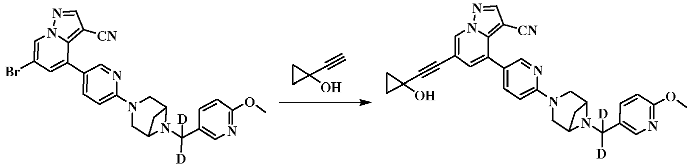

6-((1-Hydroxycyclopropyl)ethynyl)-4-(6-(6-((6-methoxypyridin-3-yl)methyl-d2)-3,6-diazabicyclo[3.1.1]heptan-3-yl)pyridin-3-yl)pyrazolo[1,5-*a*]pyridine-3-carbonitrile was obtained by using 6-bromo-4-(6-(6-((6-methoxypyridin-3-yl)methyl-d2)-3,6-diazabicyclo[3.1.1]heptan-3-yl)pyridin-3-yl)pyrazolo[1,5-*a*]pyridine-3-carbonitrile and 1-ethynylcyclopropan-1-ol as raw materials with reference to embodiment 95.

MS m/z (ESI): 520.2 [M+H]⁺.

### [Not according to the invention] Embodiment 135

### 6-((3-Hydroxybicyclo[1.1.1]pentan-1-yl)methoxy)-4-(6-(6-((6-(methoxy-d3)pyridin-3-yl)methyl)-3,6-diazabicyclo[3.1.1]heptan-3-yl)pyridin-3-yl)pyrazolo[1,5-a]pyridine-3-carbonitrile

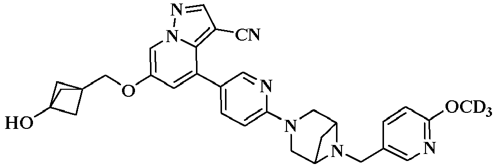

6-((3-Hydroxybicyclo[1.1.1]pentan-1-yl)methoxy)-4-(6-(6-((6-(methoxy-d3)pyridin-3-yl)methyl)-3,6-diazabicyclo[3.1.1]heptan-3-yl)pyridin-3-yl)pyrazolo[1,5-*a*]pyridine-3-carbonitrile was obtained by using 6-hydroxy-4-(6-(6-((6-(methoxy-d3)pyridin-3-yl)methyl)-3,6-diazabicyclo[3.1.1]heptan-3-yl)pyridin-3-yl)pyrazolo[1,5-*a*]pyridine-3-carbonitrile and 3-(bromomethyl)bicyclo[1.1.1]pentan-1-ol as raw materials with reference to step 1 of embodiment 131.

MS m/z (ESI):553.3 [M+H]⁺.

### Embodiment 136

### 6-((1-Cyanocyclopropyl)methoxy)-4-(6-(6-((6-methoxypyridin-3-yl)methyl-d2)-3,6-diazabicyclo[3.1.1]heptan-3-yl)pyridin-3-yl)pyrazolo[1,5-a]pyridine-3-carbonitrile

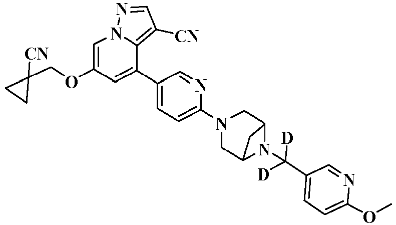

### Step 1: 3-(5-bromopyridin-2-yl)-6-((6-methoxypyridin-3-yl)methyl-d2)-3,6-diazabicyclo[3.1.1]heptane

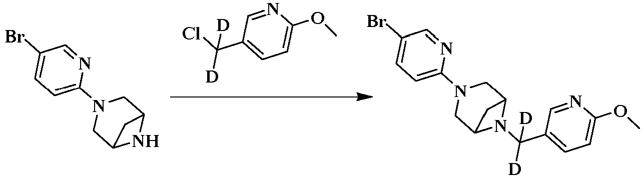

3-(5-Bromopyridin-2-yl)-6-((6-methoxypyridin-3-yl)methyl-d2)-3,6-diazabicyclo[3.1.1]heptane was obtained by using 3-(5-bromopyridin-2-yl)-3,6-diazabicyclo[3.1.1]heptane and 5-(chloromethyl-d2)-2-methoxypyridine as raw materials with reference to step 1 of embodiment 132.

MS m/z (ESI): 377.0 [M+H]⁺.

### Step 2: 6-hydroxy-4-(6-(6-((6-methoxypyridin-3-yl)methyl-d2)-3,6-diazabicyclo[3.1.1]heptan-3-yl)pyridin-3-yl)pyrazolo[1,5-a]pyridine-3-carbonitrile

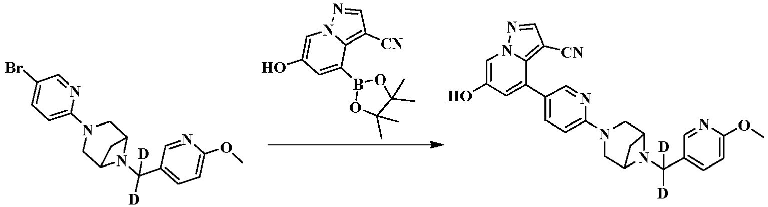

6-Hydroxy-4-(6-(6-((6-methoxypyridin-3-yl)methyl-d2)-3,6-diazabicyclo[3.1.1]heptan-3-yl)pyridin-3-yl)pyrazolo[1,5-*a*]pyridine-3-carbonitrile was obtained by using 3-(5-bromopyridin-2-yl)-6-((6-methoxypyridin-3-yl)methyl-d2)-3,6-diazabicyclo[3.1.1]heptane and 6-hydroxy-4-(4,4,5,5-tetramethyl-1,3,2-dioxaborolan-2-yl)pyrazolo[1,5-*a*]pyridine-3-carbonitrile as raw materials with reference to step 8 of embodiment 1.

MS m/z (ESI): 456.2 [M+H]⁺.

### Step 3: 6-((1-cyanocyclopropyl)methoxy)-4-(6-(6-((6-methoxypyridin-3-yl)methyl-d2)-3,6-diazabicyclo[3.1.1]heptan-3-yl)pyridin-3-yl)pyrazolo[1,5-a]pyridine-3-carbonitrile

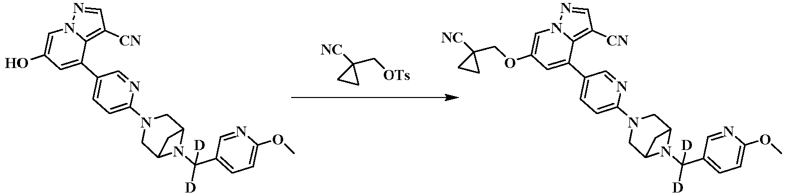

6-((1-Cyanocyclopropyl)methoxy)-4-(6-(6-((6-methoxypyridin-3-yl)methyl-d2)-3,6-diazabicyclo[3.1.1]heptan-3-yl)pyridin-3-yl)pyrazolo[1,5-*a*]pyridine-3-carbonitrile was obtained by using 6-hydroxy-4-(6-(6-((6-methoxypyridin-3-yl)methyl-d2)-3,6-diazabicyclo[3.1.1]heptan-3-yl)pyridin-3-yl)pyrazolo[1,5-*a*]pyridine-3-carbonitrile as raw material with reference to step 2 of embodiment 106.

MS m/z (ESI): 535.2 [M+H]⁺.

### [Not according to the invention] Embodiment 137

### 6-((3-Cyano-3-methylcyclobutyl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

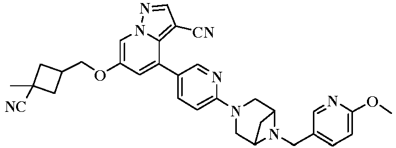

6-((3-Cyano-3-methylcyclobutyl)methoxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 6-hydroxy-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile and (3-cyano-3-methylcyclobutyl)methyl 4-methylbenzenesulfonate as raw materials with reference to step 2 of embodiment 106.

MS m/z (ESI): 561.2 [M+H]⁺.

### [Not according to the invention] Embodiment 138

### 6-((3-Cyanobicyclo[1.1.1]pentan-1-yl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

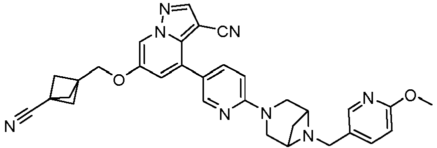

6-((3-Cyanobicyclo[1.1.1]pentan-1-yl)methoxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using (3-cyanobicyclo[1.1.1]pentan-1-yl)methyl 4-methylbenzenesulfonate as raw material with reference to step 2 of embodiment 106.

MS m/z (ESI): 559.3.[M+H]⁺.

### Embodiment 139

### N-(1-(5-(6-((1-aminocyclopropyl)ethynyl)-3-cyanopyrazolo[1,5-a]pyridin-4-yl)pyridin-2-yl)-4-methylpiperidin-4-yl)-3-chloropicolinamide

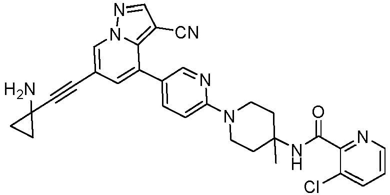

*N*-(1-(5-(6-((1-aminocyclopropyl)ethynyl)-3-cyanopyrazolo[1,5-*a*]pyridin-4-yl)pyridin-2-yl)-4-methylpiperidin-4-yl)-3-chloropicolinamide was obtained by using 1-ethynylcyclopropan-1-amine as raw material with reference to step 2 of embodiment 31.

MS m/z (ESI): 551.2[M+H]⁺.

### [Not according to the invention] Embodiment 140

### N-(1-(5-(6-((1-aminocyclopropyl)methoxy)-3-cyanopyrazolo[1,5-a]pyridin-4-yl)pyridin-2-yl)-4-methylpiperidin-4-yl)-3-chloropicolinamide

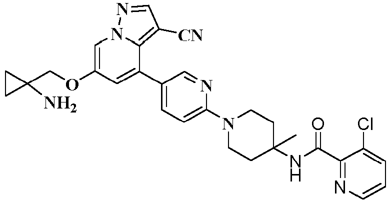

### Step 1: tert-butyl (1-(((4-(6-(4-(3-chloropicolinamido)-4-methylpiperidin-1-yl)pyridin-3-yl)-3-cyanopyrazolo[1,5-a]pyridin-6-yl)oxo)methyl)cyclopropyl)carbamate

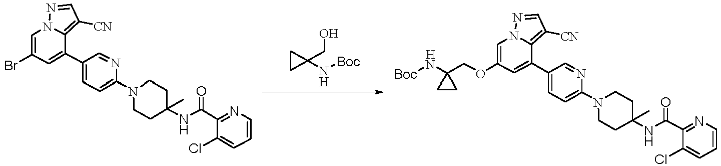

*N*-(1-(5-(6-bromo-3-cyanopyrazolo[1,5-*a*]pyridin-4-yl)pyridin-2-yl)-4-methylpiperidin-4-yl)-3-chloropicolinamide (100 mg, 0.182 mmol) was dissolved in NMP (2 mL), and *tert-butyl* (1-(hydroxy)cyclopropyl)carbamate (51 mg, 0.272 mmol) and cesium carbonate (177 mg, 0.545 mmol) were added thereto. The reaction was stirred at 160°C for 1 hour under microwave, and then directly prepared to obtain *tert-butyl* (1-(((4-(6-(4-(3-chloropicolinamido)-4-methylpiperidin-1-yl)pyridin-3-yl)-3-cyanopyrazolo[1,5-*a*]pyridin-6-yl)oxy)methyl)cyclopropyl)carbamate.

MS m/z (ESI): 657.2 [M+H]⁺.

### Step 2: N-(1-(5-(6-((1-aminocyclopropyl)methoxy)-3-cyanopyrazolo[1,5-a]pyridin-4-yl)pyridin-2-yl)-4-methylpiperidin-4-yl)-3-chloropicolinamide

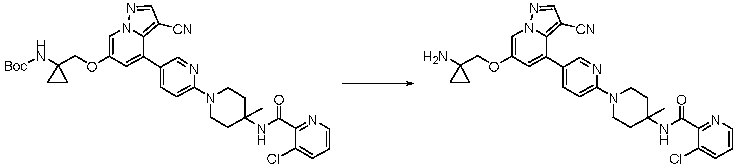

*N*-(1-(5-(6-((1-aminocyclopropyl)methoxy)-3-cyanopyrazolo[1,5-*a*]pyridin-4-yl)pyridin-2-yl)-4-methylpiperidin-4-yl)-3-chloropicolinamide was obtained by using *tert-butyl* (1-(((4-(6-(4-(3-chloropicolinamido)-4-methylpiperidin-1-yl)pyridin-3-yl)-3-cyanopyrazolo[1,5-*a*]pyridin-6-yl)oxo)methyl)cyclopropyl)carbamate as raw material with reference to step 5 of embodiment **1.**

MS m/z (ESI): 557.2 [M+H]⁺.

### [Not according to the invention] Embodiment 141

### 6-((1-Imino-1-hydroxyhexahydro-1l6-thiopyran-4-yl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

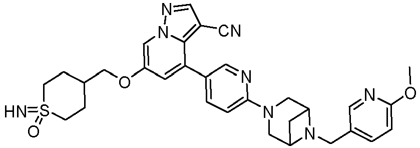

6-((1-Imino-1-hydroxyhexahydro-1l6-thiopyran-4-yl)methoxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using (tetrahydro-2H-thiopyran-4-yl)methanol as raw material with reference to embodiment 90.

¹H NMR (400 MHz, DMSO-d6) δ 8.70 (d, J = 2.1 Hz, 1H), 8.60 (s, 1H), 8.41 (d, J = 2.5 Hz, 1H), 8.07 (d, J = 2.4 Hz, 1H), 7.84 (dd, J = 8.8, 2.5 Hz, 1H), 7.68 (dd, J = 8.5, 2.4 Hz, 1H), 7.32 (d, J = 2.1 Hz, 1H), 6.78 (t, J = 8.5 Hz, 2H), 4.16 (t, J = 6.5 Hz, 1H), 4.09 - 3.98 (m, 1H), 3.89 (d, J = 17.8 Hz, 1H), 3.82 (s, 3H), 3.77 - 3.64 (m, 4H), 3.60 - 3.45 (m, 4H), 3.32 - 3.26 (m, 1H), 3.20 - 3.09 (m, 1H), 3.08 - 2.97 (m, 1H), 2.79 (q, J = 11.3 Hz, 1H), 2.65 - 2.53 (m, 1H), 2.38 - 2.25 (m, 1H), 2.19 - 2.05 (m, 1H), 2.02 - 1.93 (m, 1H), 1.92 - 1.73 (m, 1H), 1.58 (d, J = 8.4 Hz, 1H).

MS m/z (ESI): 599.2 [M+H]⁺.

### Embodiment 142

### 6-((1-Cyanocyclopropyl)methoxy)-4-(6-(6-((5-fluoro-6-methoxypyridin-3-yl)methyl)-3,6-diazabicyclo[3.1.1]hcptan-3-yl)pyridin-3-yl)pyrazolo[1,5-a]pyridine-3-carbonitrile

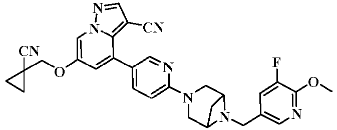

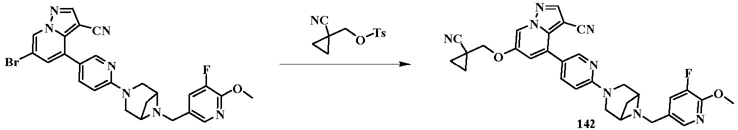

6-((1-Cyanocyclopropyl)methoxy)-4-(6-(6-((5-fluoro-6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 6-bromo-4-(6-(6-((5-fluoro-6-methoxypyridin-3-yl)methyl)-3,6-diazabicyclo[3.1.1]heptan-3-yl)pyridin-3-yl)pyrazolo[1,5-*a*]pyridine-3-carbonitrile and (1-cyanocyclopropyl)methyl 4-methylbenzenesulfonate as raw material with reference to step 2 of embodiment 106.

MS m/z (ESI): 551.2 [M+H]⁺.

¹H NMR (400 MHz, DMSO-d₆) δ 8.67 (d, J = 2.1 Hz, 1H), 8.62 (s, 1H), 8.42 (d, J = 2.5 Hz, 1H), 7.92 (d, J = 1.8 Hz, 1H), 7.86 (dd, J = 8.8, 2.6 Hz, 1H), 7.64 (dd, J = 11.6, 1.9 Hz, 1H), 7.39 (d, J = 2.1 Hz, 1H), 6.79 (d, J = 8.8 Hz, 1H), 4.21 (s, 2H), 3.92 (s, 3H), 3.84 - 3.65 (m, 4H), 3.63 - 3.48 (m, 4H), 2.61 - 2.53 (m, 1H), 1.59 (d, J = 8.4 Hz, 1H), 1.51 - 1.33 (m, 2H), 1.31 - 1.16 (m, 2H).

### [Not according to the invention] Embodiment 143

### 4-(6-(6-((5-Fluoro-6-methoxypyridin-3-yl)methyl)-3,6-diazabicyclo[3.1.1]heptan-3-yl)pyridin-3-yl)-6-((3-hydroxybicyclo[1.1.1]pentan-1-yl)methoxy)pyrazolo[1,5-a]pyridine-3-carbonitrile

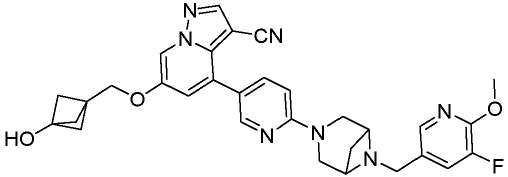

4-(6-(6-((5-Fluoro-6-methoxypyridin-3-yl)methyl)-3,6-diazabicyclo[3.1.1]heptan-3-yl)pyridin-3-yl)-6-((3-hydroxybicyclo[1.1.1]pentan-1-yl)methoxy)pyrazolo[1,5-*a*]pyridine-3-carbonitrile was obtained by using 3-(bromomethyl)bicyclo[1.1.1]pentan-1-ol and 4-(6-(6-((5-fluoro-6-methoxypyridin-3-yl)methyl)-3,6-diazabicyclo[3.1.1]heptan-3-yl)pyridin-3-yl)-6-hydroxypyrazolo[1,5-*a*]pyridine-3-carbonitrile as raw material with reference to step 1 of embodiment 128.

MS m/z (ESI): 568.2 [M+H]⁺.

### Embodiment 144

### 6-((1-Cyano-3,3-difluorocyclobutyl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

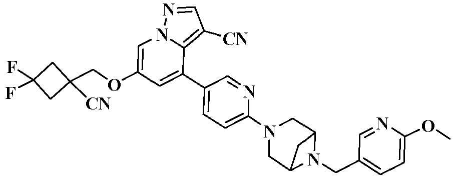

### Step 1: 1-(chloromethyl)-3,3-difluorocyclobutane-1-carbonitrile

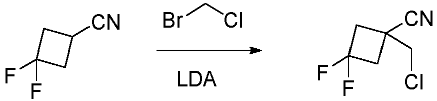

3,3-Difluorocyclobutane-1-carbonitrile (200 mg, 1.7 mmol) was dissolved in 10 mL of THF, LDA (1 mL, 2 M) was added at -78°C, and the mixture was stirred at -78°C for 1 hour. Bromochloromethane (441 mg, 3.4 mmol) was added thereto at -78 °C, and the mixture was stirred at -78 °C to room temperature for 3 hours. Water (20 mL) was added to the reaction mixture, and then the mixture was extracted with ethyl acetate (20 mL*3). The organic phase was dried and evaporated to dryness to obtain crude product 1-(chloromethyl)-3,3-difluorocyclobutane-1-carbonitrile (280 mg, yield: 99%).

### Step 2: 6-((1-Cyano-3,3-difluorocyclobutyl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

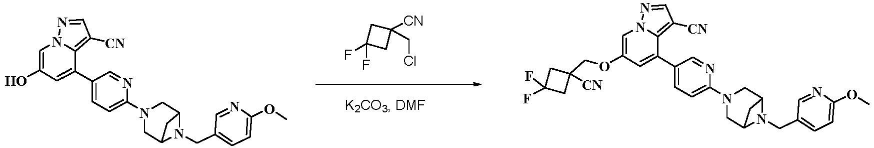

6-Hydroxy-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (50 mg, 0.13 mmol) was dissolved in 5 mL of DMF; and 1-(chloromethyl)-3,3-difluorocyclobutane-1-carbonitrile ( 280 mg), potassium carbonate (55 mg, 0.4 mmol) were added thereto, and the mixture was stirred at 80 °C for 5 hours. The residue was evaporated to dryness, and the crude product was purified by prep-HPLC to obtain product 6-((1-cyano-3,3-difluorocyclobutyl)methoxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (3.4 mg, yield. 4.5%).

MS m/z (ESI): 583.2[M+H]⁺.

¹H NMR (400 MHz, CDCl₃) δ 8.45 (d, *J* = 2.4 Hz, 1H), 8.26 (s, 1H), 8.23 (d, *J* = 1.3 Hz, 1H), 8.18 (s, 1H), 8.01 (s, 1H), 7.86 (d, *J=* 4.5 Hz, 1H), 7.22 - 7.19 (m, 1H), 6.90 - 6.83 (m, 1H), 6.77 (s, 1H), 4.28 (s, 2H), 3.94 (s, 3H), 3.34 - 3.22 (m, 3H), 3.07 - 2.97 (m, 3H), 2.96 - 2.88 (m, 2H), 2.25 - 2.19 (m, 2H), 2.06 - 1.93 (m, 4H).

### Embodiment 145

### 6-((3-Cyanooxetan-3-yl)methoxy)-4-(6-(6-((5-fluoro-6-methoxypyridin-3-yl)methyl)-3,6-diazabicydo[3.1.1]heptan-3-yl)pyridin-3-yl)pyrazolo[1,5-a]pyridine-3-carbonitrile

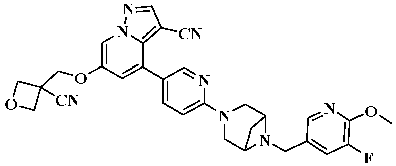

6-((3-Cyanooxetan-3-yl)methoxy)-4-(6-(6-((5-fluoro-6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 4-(6-(6-((5-fluoro-6-methoxypyridin-3-yl)methyl)-3,6-diazabicyclo[3.1.1]heptan-3-yl)pyridin-3-yl)-6-hydroxypyrazolo[1,5-*a*]pyridine-3-carbonitrile as raw material with reference to embodiment 106.

MS m/z (ESI): 567.2 [M+H]⁺.

### Embodiment 146

### 3-Chloro-N-(1-(5-(3-cyano-6-((1-cyanocyclopropyl)methoxy)pyrazolo[1,5-a]pyridin-4-yl)pyridin-2-yl)-4-methylpiperidin-4-yl)picolinamide

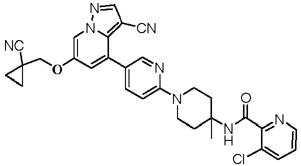

### Step 1: 3-chloro-N-(1-(5-(3-cyano-6-hydroxypyrazolo[1,5-a]pyridin-4-yl)pyridin-2-yl)-4-methylpiperidin-4-yl)picolinamide

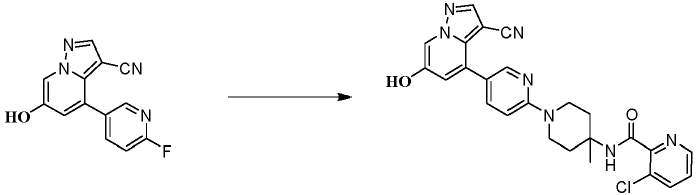

3-Chloro-*N*-(1-(5-(3-cyano-6-hydroxypyrazolo[1,5-*a*]pyridin-4-yl)pyridin-2-yl)-4-methylpiperidin-4-yl)picolinamide was obtained by using 4-(6-fluoropyridin-3-yl)-6-hydroxypyrazolo[1,5-*a*]pyridine-3-carbonitrile as raw material with reference to step 1 to step 3 of embodiment 97.

MS m/z (ESI):488.1 [M+H]⁺.

### Step 2: 3-chloro-N-(1-(5-(3-cyano-6-((1-cyanocyclopropyl)methoxy)pyrazolo[1,5-a]pyridin-4-yl)pyridin-2-yl)-4-methylpiperidin-4-yl)picolinamide

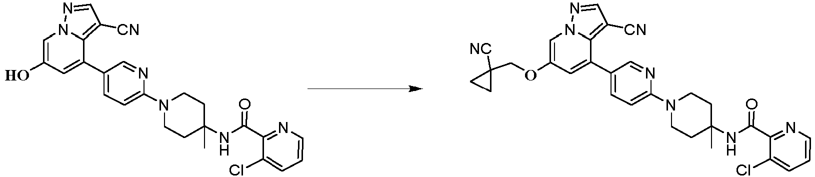

3-Chloro-*N*-(1-(5-(3-cyano-6-((1-cyanocyclopropyl)methoxy)pyrazolo[1,5-*a*]pyridin-4-yl)pyridin-2-yl)-4-methylpiperidin-4-yl)picolinamide was obtained by using 3-chloro-N-(1-(5-(3-cyano-6-hydroxypyrazolo[1,5-*a*]pyridin-4-yl)pyridin-2-yl)-4-methylpiperidin-4-yl)picolinamide as raw material with reference to step 2 of embodiment 106.

MS m/z (ESI): 567.1 [M+H]⁺.

¹H NMR (400 MHz, DMSO-d₆) δ 8.66 (d, J = 2.3 Hz, 1H), 8.60 (s, 1H), 8.53 (d, J = 4.7 Hz, 1H), 8.34 (d, J = 2.5 Hz, 1H), 8.28 (s, 1H), 8.03 - 7.98 (m, 1H), 7.82 - 7.74 (m, 1H), 7.52 - 7.48 (m, 1H), 7.39 (d, J = 1.9 Hz, 1H), 6.99 (d, J = 8.7 Hz, 1H), 4.20 (s, 2H), 4.10 - 4.01 (m, 2H), 3.40 - 3.34 (m, 2H), 2.34 - 2.30 (m, 2H), 1.63 - 1.54 (m, 2H), 1.45 (s, 3H), 1.44 - 1.39 (m, 2H), 1.22 - 1.16 (m, 2H).

### Embodiment 147

### N-(1-(5-(3-cyano-6-((1-cyanocyclopropyl)methoxy)pyrazolo[1,5-a]pyridin-4-yl)pyridin-2-yl)-4-methylpiperidin-4-yl)-3-fluoropicolinamide

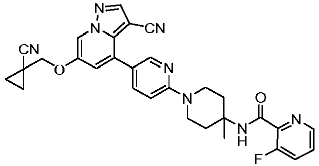

### Step 1: N-(1-(5-(3-cyano-6-hydroxypyrazolo[1,5-a]pyridin-4-yl)pyridin-2-yl)-4-methylpiperidin-4-yl)-3-fluoropicolinamide

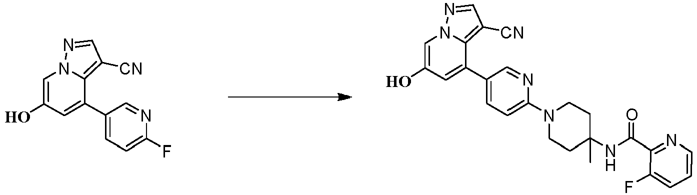

*N*-(1-(5-(3-cyano-6-hydroxypyrazolo[1,5-*a*]pyridin-4-yl)pyridin-2-yl)-4-methylpiperidin-4-yl)-3-fluoropicolinamide was obtained by using 4-(6-fluoropyridin-3-yl)-6-hydroxypyrazolo[1,5-*a*]pyridine-3-carbonitrile as raw material with reference to step 1 to step 3 of embodiment 97.

MS m/z (ESI):472.1 [M+H]⁺.

### Step 2: N-(1-(5-(3-cyano-6-((1-cyanocyclopropyl)methoxy)pyrazolo[1,5-a]pyridin-4-yl)pyridin-2-yl)-4-methylpiperidin-4-yl)-3-fluoropicolinamide

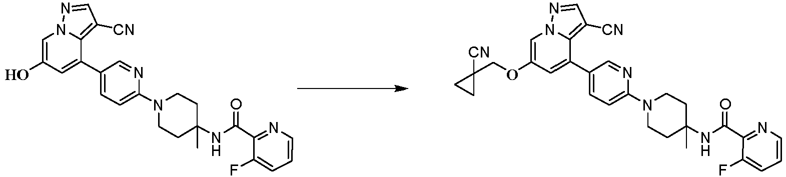

*N*-(1-(5-(3-cyano-6-((1-cyanocyclopropyl)methoxy)pyrazolo[1,5-*a*]pyridin-4-yl)pyridin-2-yl)-4-methylpiperidin-4-yl)-3-fluoropicolinamide was obtained by using N-(1-(5-(3-cyano-6-hydroxypyrazolo[1,5-*a*]pyridin-4-yl)pyridin-2-yl)-4-methylpiperidin-4-yl)-3-fluoropicolinamide as raw material with reference to step 2 of embodiment 106.

MS m/z (ESI): 551.2 [M+H]⁺.

¹H NMR (400 MHz, DMSO-d₆) δ 8.66 (d, J = 2.1 Hz, 1H), 8.60 (s, 1H), 8.51 - 8.43 (m, 1H), 8.35 (d, J = 2.5 Hz, 1H), 8.21 (s, 1H), 7.89 - 7.81 (m, 1H), 7.78 (dd, J = 8.8, 2.6 Hz, 1H), 7.68 - 7.58 (m, 1H), 7.39 (d, J = 2.1 Hz, 1H), 6.99 (d, J = 8.9 Hz, 1H), 4.20 (s, 2H), 4.10 - 3.98 (m, 2H), 3.31 - 3.24 (m, 2H), 2.40 - 2.27 (m, 2H), 1.69 - 1.56 (m, 2H), 1.46 (s, 3H), 1.45 - 1.40 (m, 2H), 1.24 - 1.16 (m, 2H).

### Embodiment 148

### 6-((4-Cyanotetrahydro-2H-pyran-4-yl)methoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

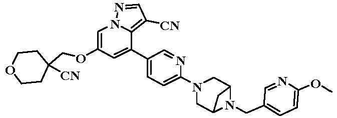

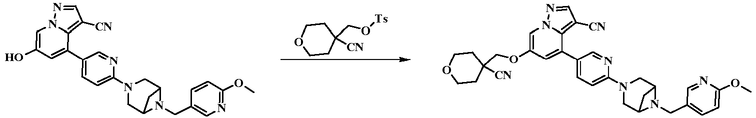

6-((4-Cyanotetrahydro-2H-pyran-4-yl)methoxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 6-hydroxy-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile and (4-cyanotetrahydro-2H-pyran-4-yl)methyl 4-methylbenzenesulfonate as raw materials with reference to step 2 of embodiment 106.

MS m/z (ESI): 577.2 [M+H]⁺.

¹H NMR (400 MHz, Chloroform-d) δ 8.42 (d, J = 2.5 Hz, 1H), 8.24 (s, 1H), 8.17 (d, J= 2.1 Hz, 1H), 8.13 (d, J = 2.4 Hz, 1H), 7.90-7.78 (m, 2H), 7.18 (d, J = 2.1 Hz, 1H), 6.76 (d, J = 8.5 Hz, 1H), 6.71 (d, J = 8.8 Hz, 1H), 4.14 - 4.02 (m, 4H), 3.99-3.88 (m, 6H), 3.86 - 3.61 (m, 6H), 2.12-2.05 (m, 2H), 1.90 - 1.78 (m, 3H), 1.78 - 1.64 (m, 2H).

### Embodiment 149

### 6-((4-Cyanotetrahydro-2H-pyran-4-yl)methoxy)-4-(6-(6-((S-fluoro-6-methoxypyridin-3-yl)methyl)-3,6-diazabicyclo[3.1.1]heptan-3-yl)pyridin-3-yl)pyrazolo[1,5-a]pyridine-3-carbonitrile

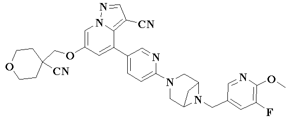

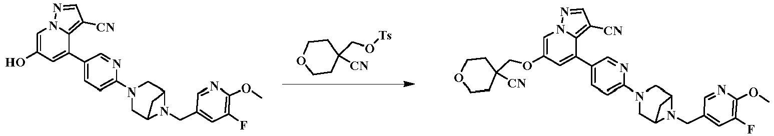

6-((4-Cyanotetrahydro-2H-pyran-4-yl)methoxy)-4-(6-(6-((5-fluoro-6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 4-(6-(6-((5-fluoro-6-methoxypyridin-3-yl)methyl)-3,6-diazabicyclo[3.1.1]heptan-3-yl)pyridin-3-yl)-6-hydroxypyrazolo[1,5-*a*]pyridine-3-carbonitrile and (4-cyanotetrahydro-2H-pyran-4-yl)methyl 4-methylbenzenesulfonate as raw materials with reference to step 2 of embodiment 106.

MS m/z (ESI): 595.2 [M+H]⁺.

¹H NMR 1H NMR (400 MHz, Chloroform-d) δ 8.42 (d, J = 2.5 Hz, 1H), 8.24 (s, 1H), 8.17 (d, J = 2.1 Hz, 1H), 7.88 (s, 1H), 7.79 (dd, J = 8.8, 2.6 Hz, 1H), 7.55 (d, J = 19.9 Hz, 1H), 7.17 (d, J = 2.1 Hz, 1H), 6.70 (d, J = 8.8 Hz, 1H), 4.09 (d, J = 4.0 Hz, 1H), 4.06 (s, 3H), 4.01 (s, 3H), 3.91 - 3.78 (m, 6H), 3.70 - 3.62 (m, 3H), 2.90 - 2.73 (m, 1H), 2.11 - 2.05 (m, 2H), 1.89 - 1.77 (m, 3H), 1.73 - 1.69 (m, 1H).

### Embodiment 150

### 3-Chloro-N-(1-(5-(3-cyano-6-((3-cyanooxetan-3-yl)methoxy)pyrazolo[1,5-a]pyridin-4-yl)pyridin-2-yl)-4-methylpiperidin-4-yl)picolinamide

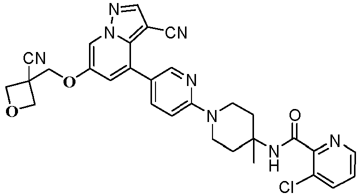

3-Chloro-*N*-(1-(5-(3-cyano-6-((3-cyanooxetan-3-yl)methoxy)pyrazolo[1,5-*a*]pyridin-4-yl)pyridin-2-yl)-4-methylpiperidin-4-yl)picolinamide was obtained by using 3-chloro-*N*-(1-(5-(3-cyano-6-hydroxypyrazolo[1,5-*a*]pyridin-4-yl)pyridin-2-yl)-4-methylpiperidin-4-yl)methylpyridinamide as raw material with reference to embodiment 106.

MS m/z (ESI): 583.2 [M+H]⁺.

### Embodiment 151

### 4-(6-(6-((5-Fluoro-6-methoxypyridin-3-yl)methyl)-3,6-diazabicyclo[3.1.1]heptan-3-yl)pyridin-3-yl)-6-(3-hydroxy-4-methoxy-3-methylbut-1-yn-1-yl)pyrazolo[1,5-a]pyridine-3-carbonitrile

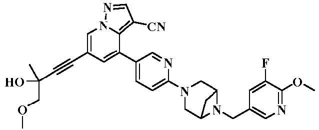

4-(6-(6-((5-Fluoro-6-methoxypyridin-3-yl)methyl)-3,6-diazabicyclo[3.1.1]heptan-3-yl)pyridin-3-yl)-6-(3-hydroxy-4-methoxy-3-methylbut-1-yn-1-yl)pyrazolo[1,5-*a*]pyridine-3-carbonitrile was obtained by using 6-bromo-4-(6-(6-((5-fluoro-6-methoxypyridin-3-yl)methyl)-3,6-diazabicyclo[3.1.1]heptan-3-yl)pyridine-3-yl)pyrazolo[1,5-*a*]pyridine- 3-carbonitrile and 1-methoxy-2-methylbut-3-yn-2-ol as raw materials with reference to step 2 of embodiment 31.

MS m/z (ESI):568.2 [M+H]⁺.

¹H NMR (400 MHz, CDCl₃) δ 8.64 (s, 1H), 8.40 (d, *J* = 2.1 Hz, 1H), 8.31 (s, 1H), 7.90 (s, 1H), 7.78 (dd, *J* = 8.8, 2.4 Hz, 1H), 7.67 (dd, *J* = 11.8, 7.1 Hz, 1H), 7.32 (s, 1H), 6.70 (d, *J* = 8.7 Hz, 1H), 4.02 (s, 3H), 3.89 (s, 3H), 3.72 (s, 5H), 3.60 (d, *J* = 9.1 Hz, 1H), 3.52 (s, 3H), 3.45 (d, *J* = 9.1 Hz, 1H), 2.97 (m, 2H), 1.58 (s, 3H).

### Embodiment 152A

### 6-((2-Hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

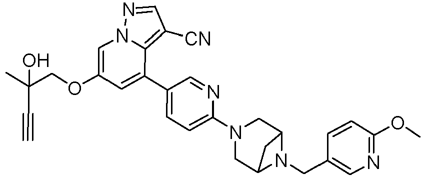

### Step 1: 4-(6-(6-((6-methoxypyridin-3-yl)methyl)-3,6-diazabicyclo[3.1.1]heptan-3-yl)pyridin-3-yl)-6-(2-oxopropoxy)pyrazolo[1,5-a]pyridine-3-carbonitrile

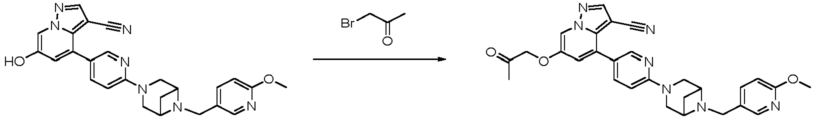

6-Hydroxy-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (200 mg, 0.441 mmol) was dissolved in DMF (20 mL), and bromoacetone (121 mg, 0.882 mmol ), cesium carbonate (431 mg, 1.32 mmol) and sodium iodide (66 mg, 0.441 mmol) were added thereto, respectively. The reaction was stirred at room temperature overnight. Water was added to the reaction mixture, and then the mixture was extracted with ethyl acetate. The organic phase was dried and evaporated to dryness. The crude product was purified by column chromatography to obtain 4-(6-(6-((6-methoxypyridin-3-yl)methyl)-3,6-diazabicyclo[3.1.1]heptan-3-yl)pyridin-3-yl)-6-(2-oxopropoxy)pyrazolo[1,5-*a*]pyridine-3-carbonitrile (160 mg, yield: 71%).

MS m/z (ESI): 510.1 [M+H]⁺.

### Step 2: 6-((2-hydroxy-2-methylbut-3-yn-1-yl)oxy)-4-(6-(6-((6-methoxypyridin-3-yl)methyl) -3, 6-diazabicyclo[3.1.1]heptan-3-yl)pyridin-3-yl)pyrazolo[1,5-a]pyridine-3-carbonitrile

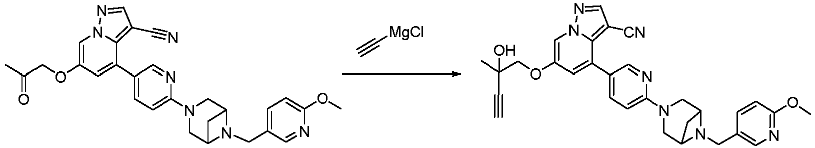

4-(6-(6-((6-Methoxypyridin-3-yl)methyl)-3,6-diazabicyclo[3.1.1]heptan-3-yl)pyridin-3-yl)-6-(2-oxopropoxy)pyrazolo[1,5-*a*]pyridine-3-carbonitrile (160 mg, 0.314 mmol) was dissolved in anhydrous tetrahydrofuran (15 mL), and then ethynylmagnesium chloride ( 6.28 mL, 3.14 mmol, 0.5 M) was slowly added thereto. After the addition was completed, the reaction mixture was stirred for 3 hours. Ammonium chloride aqueous solution was added to quench the reaction, and then the mixture was extracted with ethyl acetate. The organic phase was dried and evaporated to dryness. The crude product was purified by column chromatography to obtain 6-((2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile(48 mg, yield was 28%).

MS m/z (ESI): 536.1 [M+H]⁺.

¹H NMR (400 MHz, DMSO-d6) δ 8.74 (d, J = 2.1 Hz, 1H), 8.60 (s, 1H), 8.42 (d, J = 2.5 Hz, 1H), 8.07 (d, J = 2.4 Hz, 1H), 7.85 (dd, J = 8.8, 2.6 Hz, 1H), 7.68 (dd, J = 8.5, 2.4 Hz, 1H), 7.33 (d, J = 2.1 Hz, 1H), 6.78 (dd, J = 10.8, 8.6 Hz, 2H), 5.84 (s, 1H), 4.08 (s, 2H), 3.82 (s, 3H), 3.78 - 3.70 (m, 2H), 3.70 - 3.65 (m, 2H), 3.61 - 3.52 (m, 2H), 3.50 (s, 2H), 3.40 (s, 1H), 2.57 - 2.53 (m, 1H), 1.59 (d, J = 8.5 Hz, 1H), 1.49 (s, 3H).

### Embodiment 152

### 6-(((R)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

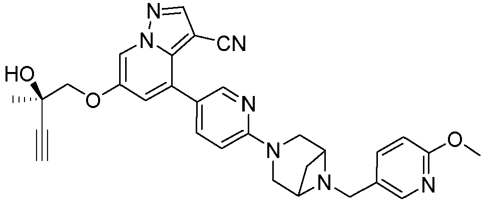

### Step 1: 4-(6-(6-((6-methoxypyridin-3-yl)methyl)-3,6-diazabicyclo[3.1.1]heptan-3-yl)pyridin-3-yl)-6-(2-oxopropoxy)pyrazolo[1,5-a]pyridine-3-carbonitrile

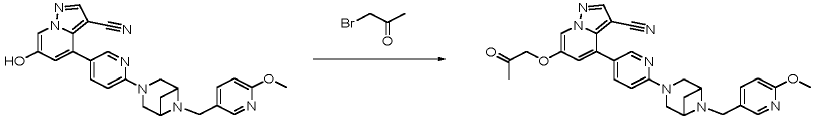

6-Hydroxy-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (200 mg, 0.441 mmol) was dissolved in DMF (20 mL), and bromoacetone (121 mg, 0.882 mmol ), cesium carbonate (431 mg, 1.32 mmol) and sodium iodide (66 mg, 0.441 mmol) were added thereto, respectively. The reaction was stirred at room temperature overnight. Water was added to the reaction mixture, and then the mixture was extracted with ethyl acetate. The organic phase was dried and evaporated to dryness. The crude product was purified by column chromatography to obtain 4-(6-(6-((6-methoxypyridin-3-yl)methyl)-3,6-diazabicyclo[3.1.1]heptan-3-yl)pyridin-3-yl)-6-(2-oxopropoxy)pyrazolo[1,5-*a*]pyridine-3-carbonitrile (160 mg, yield: 71%).

MS m/z (ESI): 510.1 [M+H]⁺.

### Step 2: 6-((2-hydroxy-2-methylbut-3-yn-1-yl)oxy)-4-(6-(6-((6-methoxypyridin-3-yl)methyl) -3, 6-diazabicyclo[3.1.1]heptan-3-yl)pyridin-3-yl)pyrazolo[1,5-a]pyridine-3-carbonitrile

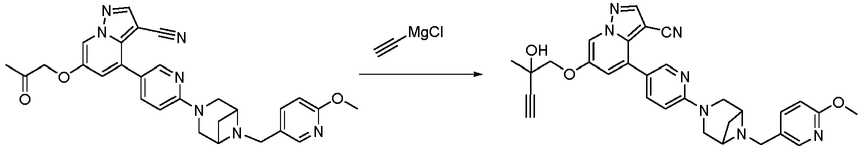

4-(6-(6-((6-Methoxypyridin-3-yl)methyl)-3,6-diazabicyclo[3.1.1]heptan-3-yl)pyridin-3-yl)-6-(2-oxopropoxy)pyrazolo[1,5-*a*]pyridine-3-carbonitrile (160 mg, 0.314 mmol) was dissolved in anhydrous tetrahydrofuran (15 mL), and then ethynylmagnesium chloride ( 6.28 mL, 3.14 mmol, 0.5 M) was slowly added thereto. After the addition was completed, the reaction mixture was stirred for 3 hours. Ammonium chloride aqueous solution was added to quench the reaction, and then the mixture was extracted with ethyl acetate. The organic phase was dried and evaporated to dryness. The crude product was purified by column chromatography to obtain 6-((2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile(48 mg, yield was 28%).

MS m/z (ESI): 536.1 [M+H]⁺.

### Step 3: 6-(((R)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

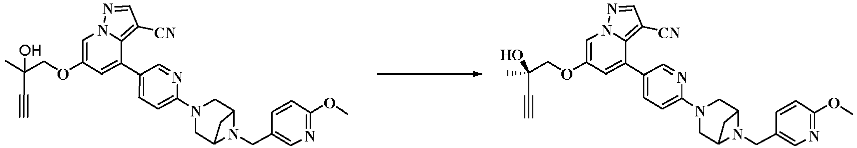

6-(((R)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile(16 mg) was obtained by chiral resolution from 6-((-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (48.2 mg, 0.09 mmol).

### Chiral resolution conditions:

**Table 1**

| | |
|---|---|
| Instrument | CHIRALPAK IBN |
| Column type | 5.0 cm I.D.× 25 cm L, 10 µm |
| Mobile phase | Hexane/EtOH/DCM=60/30/10(V/V/V) |
| Flow rate | 60 mL/min |
| Detection wavelength | UV 254 nm |
| Column temperature | 35°C |

t*_{R}*=9.002 min
MS m/z (ESI): 536.1 [M+H]⁺.
¹H NMR (400 MHz, DMSO-*d*₆) δ 8.74 (d, *J* = 2.1 Hz, 1H), 8.60 (s, 1H), 8.42 (d, *J* = 2.5 Hz, 1H), 8.07 (d, *J =* 2.4 Hz, 1H), 7.85 (dd, *J =* 8.8, 2.6 Hz, 1H), 7.68 (dd, *J =* 8.5, 2.4 Hz, 1H), 7.33 (d, *J =* 2.1 Hz, 1H), 6.78 (m, 2H), 5.84 (s, 1H), 4.08 (s, 2H), 3.82 (s, 3H), 3.78 - 3.63 (m, 4H), 3.61 - 3.46 (m, 4H), 3.40 (s, 1H), 2.57 - 2.53 (m, 1H), 1.59 (d, *J =* 8.5 Hz, 1H), 1.49 (s, 3H).

### Embodiment 153

### 6-(((S)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

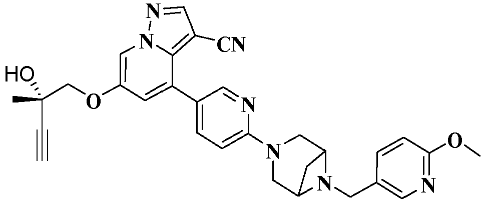

### Step 1: 6-(((S)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

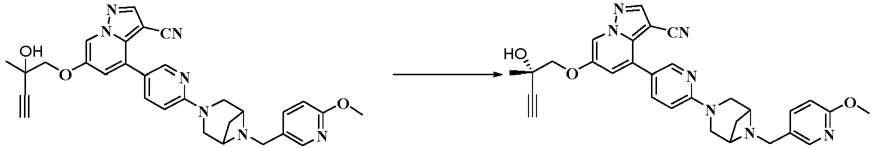

6-(((*S*)-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile(18 mg) was obtained by chiral resolution from 6-((-2-hydroxy-2-methylbut-3-yn-1-yl)oxy)-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile (48.2 mg, 0.09 mmol).

### Chiral resolution conditions:

**Table 2**

| | |
|---|---|
| Instrument | CHIRALPAK IBN |
| Column type | 5.0 cm I.D. × 25 cm L, 10 µm |
| Mobile phase | Hexane/EtOH/DCM=60/30/10(V/V/V) |
| Flow rate | 60 ml/min |
| Detection wavelength | UV 254 nm |
| Column temperature | 35°C |

t*_{R}*=7.431 min
MS m/z (ESI): 536.1 [M+H]⁺.
¹H NMR (400 MHz, DMSO-*d*₆) δ 8.74 (d, *J* = 2.1 Hz, 1H), 8.60 (s, 1H), 8.42 (d, *J* = 2.5 Hz, 1H), 8.07 (d, *J =* 2.4 Hz, 1H), 7.85 (dd, *J =* 8.8, 2.6 Hz, 1H), 7.68 (dd, *J =* 8.5, 2.4 Hz, 1H), 7.33 (d, *J =* 2.1 Hz, 1H), 6.78 (m, 2H), 5.84 (s, 1H), 4.08 (s, 2H), 3.82 (s, 3H), 3.78 - 3.63 (m, 4H), 3.61 - 3.46 (m, 4H), 3.40 (s, 1H), 2.57 - 2.53 (m, 1H), 1.59 (d, *J =* 8.5 Hz, 1H), 1.49 (s, 3H).

### Embodiment 154

### 4-(6-(6-((5-Fluoro-6-methoxypyridin-3-yl)methyl)-3,6-diazabicyclo[3.1.1]heptan-3-yl)pyridin-3-yl)-6-(((S)-2-hydroxy-2-methylbut-3-yn-1-yl)oxy)pyrazolo[1,5-a]pyridine-3-carbonitrile

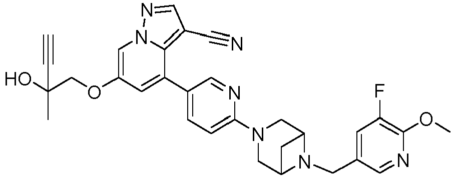

### Step 1: 4-(6-(6-((5-fluoro-6-methoxypyridin-3-yl)methyl)-3,6-diazabicyclo[3.1.1]heptan-3-yl)pyridin-3-yl)-6-(2-oxopropoxy)pyrazolo[1,5-a]pyridine-3-carbonitrile

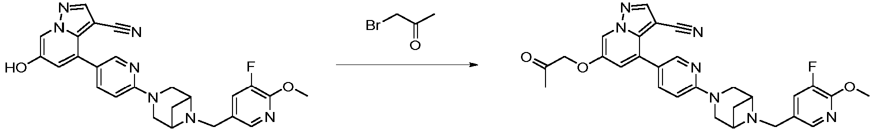

4-(6-(6-((5-Fluoro-6-methoxypyridin-3-yl)methyl)-3,6-diazabicyclo[3.1.1]heptan-3-yl)pyridin-3-yl)-6-(2-oxopropoxy)pyrazolo[1,5-*a*]pyridine-3-carbonitrile (190 mg, yield was 84%) was obtained by using 4-(6-(6-((5-fluoro-6-methoxypyridin-3-yl)methyl)-3,6-diazabicyclo[3.1.1]heptan-3-yl)pyridin-3-yl)-6-hydroxypyrazolo[1,5-*a*]pyridine-3-carbonitrile as raw material with reference to step 1 of embodiment **152.**

MS m/z (ESI): 528.1 [M+H]⁺.

### Step 2: 4-(6-(6-((5-fluoro-6-methoxypyridin-3-yl)methyl)-3,6-diazabicyclo[3.1.1]heptan-3-yl)pyridin-3-yl)-6-(((S)-2-hydroxy-2-methylbut-3-yn-1-yl)oxy)pyrazolo[1,5-a]pyridine-3-carbonitrile

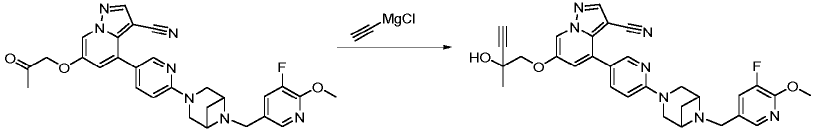

4-(6-(6-((5-Fluoro-6-methoxypyridin-3-yl)methyl)-3,6-diazabicyclo[3.1.1]heptan-3-yl)pyridin-3-yl)-6-(((*S*)-2-hydroxy-2-methylbut-3-yn-1-yl)oxy)pyrazolo[1,5-*a*]pyridine-3-carbonitrile (40 mg, yield was 22%) was obtained by using 4-(6-(6-((5-fluoro-6-methoxypyridin-3-yl)methyl)-3,6-diazabicyclo[3.1.1]heptan-3-yl)pyridin-3-yl)-6-(2-oxopropoxy)pyrazolo[1,5-*a*]pyridine-3-carbonitrile as raw material with reference to step 2 of embodiment **152.**

MS m/z (ESI): 554.3 [M+H]⁺.

¹H NMR (400 MHz, DMSO-*d*₆) δ 8.74 (d, *J* = 2.1 Hz, 1H), 8.60 (s, 1H), 8.42 (d, *J* = 2.5 Hz, 1H), 7.92 (d, *J* = 1.9 Hz, 1H), 7.85 (dd, *J* = 8.8, 2.6 Hz, 1H), 7.64 (dd, *J* = 11.5, 1.9 Hz, 1H), 7.33 (d, *J* = 2.1 Hz, 1H), 6.79 (d, *J =* 8.8 Hz, 1H), 5.84 (s, 1H), 4.08 (s, 2H), 3.92 (s, 3H), 3.81 - 3.65 (m, 4H), 3.64 - 3.48 (m, 4H), 3.40 (s, 1H), 2.59 - 2.53 (m, 1H), 1.59 (d, *J =* 8.4 Hz, 1H), 1.49 (s, 3H).

### [Not according to the invention] Embodiment 155

### 3-(((3-Cyano-4-(6-(6-((6-methoxypyridin-3-yl)methyl)-3,6-diazabicyclo[3.1.1]heptan-3-yl)pyridin-3-yl)pyrazolo[1,5-a]pyridin-6-yl)oxo)methyl)bicyclo[1.1.1]pentane-1-carboxamide

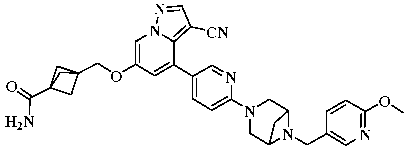

3-(((3-Cyano-4-(6-(6-((6-methoxypyridin-3-yl)methyl)-3,6-diazabicyclo[3.1.1]heptan-3-yl)pyridin-3-yl)pyrazolo[1,5-*a*]pyridin-6-yl)oxy)methyl)bicyclo[1.1.1]pentane-1-carboxamide was obtained by using (3-carbamoylbicyclo[1.1.1]pentan-1-yl)methyl 4-methylbenzenesulfonate and 6-hydroxy-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile as raw materials with reference to step 2 of embodiment 106.

MS m/z (ESI):577.3 [M+H]⁺.

¹H NMR (400 MHz, CDCl₃) δ 9.70 - 9.62 (m, 1H), 8.41 (d, *J* = 2.1 Hz, 1H), 8.21 (s, 1H), 8.16 - 8.08 (m, 2H), 7.93 - 7.77 (m, 2H), 7.13 *(d, J=* 1.9 Hz, 1H), 6.73 (dd, *J =* 22.6, 8.7 Hz, 2H), 5.52 (s, 1H), 5.39 (s, 1H), 4.07 (s, 2H), 3.93 (s, 5H), 3.72 (s, 4H), 2.96 (s, 1H), 2.88 (s, 1H), 2.15 (s, 6H).

### [Not according to the invention] Embodiment 156

### 3-(((3-Cyano-4-(6-(6-((6-methoxypyridin-3-yl)methyl)-3,6-diazabicyclo[3.1.1]heptan-3-yl)pyridin-3-yl)pyrazolo[1,5-a]pyridin-6-yl)oxo)methyl)-N-methylbicyclo[1.1.1]pentane-1-carboxamide

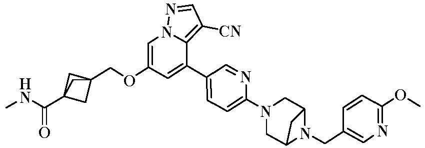

3-(((3-Cyano-4-(6-(6-((6-methoxypyridin-3-yl)methyl)-3,6-diazabicyclo[3.1.1]heptan-3-yl)pyridin-3-yl)pyrazolo[1,5-*a*]pyridin-6-yl)oxy)methyl)-*N*-methylbicyclo[1.1.1]pentane-1-carboxamide was obtained by using (3-(methylcarbamoyl)bicyclo[1.1.1]pentan-1-yl)methyl 4-methylbenzenesulfonate and 6-hydroxy-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile as raw materials with reference to step 2 of embodiment 106.

MS m/z (ESI):591.3 [M+H]⁺.

¹H NMR (400 MHz, CDCl₃) δ 8.40 (d, *J* = 2.2 Hz, 1H), 8.21 (s, 1H), 8.14 - 8.05 (m, 2H), 7.78 (dd, *J= 8.8, 2.4* Hz, 1H), 7.67 (d, *J* = 7.5 Hz, 1H), 7.12 (d, *J=* 1.9 Hz, 1H), 6.70 (dd, *J* = 15.4, 8.7 Hz, 2H), 5.58 (d, *J* = 4.5 Hz, 1H), 4.05 (s, 2H), 3.92 (s, 3H), 3.83 (dd, *J* = 17.6, 8.8 Hz, 4H), 3.60 (s, 4H), 2.83 (d, *J* = 4.9 Hz, 3H), 2.72 *(d, J=* 5.8 Hz, 1H), 2.11 (s, 6H), 1.67 *(d, J=* 8.7 Hz, 1H).

### Embodiment 157

### 4-(6-(6-((5-Fluoro-6-methoxypyridin-3-yl)methyl)-3,6-diazabicyclo[3.1.1]heptan-3-yl)pyridin-3-yl)-6-((R)-3-hydroxybut-1-yn-1-yl)pyrazolo[1,5-a]pyridine-3-carbonitrile

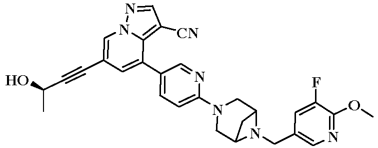

4-(6-(6-((5-Fluoro-6-methoxypyridin-3-yl)methyl)-3,6-diazabicyclo[3.1.1]heptan-3-yl)pyridin-3-yl)-6-((R)-3-hydroxybut-1-yn-1-yl)pyrazolo[1,5-*a*]pyridine-3-carbonitrile was obtained by using from (R)-but-3-yn-2-ol and 6-bromo-4-(6-(6-((5-fluoro-6-methoxypyridin-3-yl)methyl)-3,6-diazabicyclo[3.1.1]heptan-3-yl)pyrazolo[1,5-*a*]pyridine-3-carbonitrile as raw materials with reference to step 2 of embodiment 31.

MS m/z (ESI):524.2 [M+H]⁺.

¹H NMR (400 MHz, CDCl₃) δ 8.62 (d, *J* = 1.0 Hz, 1H), 8.40 *(d, J=* 2.2 Hz, 1H), 8.31 (s, 1H), 7.89 (s, 1H), 7.78 (dd, *J* = 8.8, 2.4 Hz, 1H), 7.62 (s, 1H), 7.30 (d, *J* = 1.2 Hz, 1H), 6.70 (d, *J* = 8.8 Hz, 1H), 4.80 (q, *J* = 6.6 Hz, 1H), 4.01 (s, 3H), 3.97 - 3.82 (m, 3H), 3.71 (s, 4H), 2.87 (s, 2H), 1.74 (d, *J* = 8.2 Hz, 1H), 1.59 (d, *J* = 6.6 Hz, 3H).

### [Not according to the invention] Embodiment 158

### 6-((3-Cyanobicyclo[1.1.1]pentan-1-yl)methoxy)-4-(6-(6-((5-fluoro-6-methoxypyridin-3-yl)methyl)-3,6-diazabicyclo[3.1.1]heptan-3-yl)pyridin-3-yl)pyrazolo[1,5-a]pyridine-3-carbonitrile

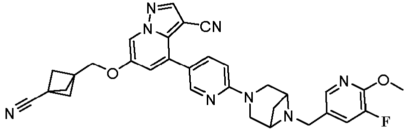

6-((3-Cyanobicyclo[1.1.1]pentan-1-yl)methoxy)-4-(6-(6-((5-fluoro-6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using (3-cyanobicyclo[1.1.1]pentan-1-yl)methyl 4-methylbenzenesulfonate and 4-(6-(6-((5-fluoro-6-methoxypyridin-3-yl)methyl)-3,6-diazabicyclo[3.1.1]heptan-3-yl)pyridin-3-yl)-6-hydroxypyrazolo[1,5-*a*]pyridine-3-carbonitrile as raw materials with reference to step 2 of embodiment 106.

MS m/z (ESI):577.2 [M+H]⁺.

¹H NMR (400 MHz, CDCl₃) δ 8.40 (d, *J* = 1.9 Hz, 1H), 8.22 (s, 1H), 8.07 (s, 1H), 7.88 (s, 1H), 7.79 (dd, *J* = 8.7, 2.0 Hz, 1H), 7.60 (s, 1H), 7.11 (s, 1H), 6.70 (d, *J* = 8.8 Hz, 1H), 4.01 (s, 5H), 3.88 (m, 4H), 3.68 (s, 4H), 2.85 (s, 1H), 2.37 (s, 6H), 1.76 - 1.69 (m, 1H).

### [Not according to the invention] Embodiment 159

### 6-((3-Cyano-3-methylcyclobutyl)methoxy)-4-(6-(6-((5-fluoro-6-methoxypyridin-3-yl)methyl)-3,6-diazabicyclo[3.1.1]heptan-3-yl)pyridin-3-yl)pyrazolo[1,5-a]pyridine-3-carbonitrile

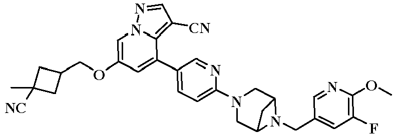

6-((3-Cyano-3-methylcyclobutyl)methoxy)-4-(6-(6-((5-fluoro-6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using (3-cyano-3-methylcyclobutyl)methyl 4-methylbenzenesulfonate and 4-(6-(6-((5-fluoro-6-methoxypyridin-3-yl)methyl)-3,6-diazabicyclo[3.1.1]heptan-3-yl)pyridin-3-yl)-6-hydroxypyrazolo[1,5-*a*]pyridine-3-carbonitrile as raw materials with reference to step 2 of embodiment 106.

MS m/z (ESI): 579.3 [M+H]⁺.

### [Not according to the invention] Embodiment 160

### 4-(6-(6-((5-Fluoro-6-methoxypyridin-3-yl)methyl)-3,6-diazabicyclo[3.1.1]heptan-3-yl)pyridin-3-yl)-6-((3-(hydroxymethyl)bicyclo[1.1.1]pentan-1-yl)methoxy)pyrazolo[1,5-a]pyridine-3-carbonitrile

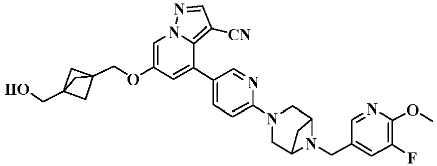

4-(6-(6-((5-Fluoro-6-methoxypyridin-3-yl)methyl)-3,6-diazabicyclo[3.1.1]heptan-3-yl)pyridin-3-yl)-6-((3-(hydroxymethyl)bicyclo[1.1.1]pentan-1-yl)methoxy)pyrazolo[1,5-*a*]pyridine-3-carbonitrile was obtained by using (3-(hydroxymethyl)bicyclo[1.1.1]pentan-1-yl)methyl 4-methylbenzenesulfonate and 4-(6-(6-((5-fluoro-6-methoxypyridin-3-yl)methyl)-3,6-diazabicyclo[3.1.1]heptan-3-yl)pyridin-3-yl)-6-hydroxypyrazolo[1,5-*a*]pyridine-3-carbonitrile as raw materials with reference to step 2 of embodiment 106.

MS m/z (ESI): 582.3 [M+H]⁺.

¹H NMR (400 MHz, CDCl₃) δ 8.41 (d, *J* = 2.1 Hz, 1H), 8.21 (s, 1H), 8.11 (d, *J* = 1.9 Hz, 1H), 7.88 (s, 1H), 7.79 (dd, *J* = 8.8, 2.4 Hz, 1H), 7.54 (d, *J* = 10.8 Hz, 1H), 7.14 (d, *J* = 1.9 Hz, 1H), 6.69 (d, *J* = 8.8 Hz, 1H), 4.05 (s, 2H), 4.01 (s, 3H), 3.85 (d, *J* = 11.3 Hz, 4H), 3.66 (s, 6H), 2.79 (s, 1H), 1.82 (s, 6H), 1.70 (d,*J* = 8.8 Hz, 1H).

### [Not according to the invention] Embodiment 161

### 4-(6-(6-((5-Fluoro-6-methoxypyridin-3-yl)methyl)-3,6-diazabicyclo[3.1.1]heptan-3-yl)pyridin-3-yl)-6-((cis-3-hydroxy-3-methylcyclobutyl)methoxy)pyrazolo[1,5-a]pyridine-3-carbonitrile

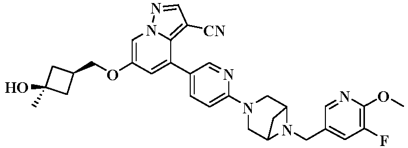

4-(6-(6-((5-Fluoro-6-methoxypyridin-3-yl)methyl)-3,6-diazabicyclo[3.1.1]heptan-3-yl)pyridin-3-yl)-6-((cis-3-hydroxy-3-methylcyclobutyl)methoxy)pyrazolo[1,5-*a*]pyridine-3-carbonitrile was obtained by using (cis-3-hydroxy-3-methylcyclobutyl)methyl 4-methylbenzenesulfonate and 4-(6-(6-((5-fluoro-6-methoxypyridin-3-yl)methyl)-3,6-diazabicyclo[3.1.1]heptan-3-yl)pyridin-3-yl)-6-hydroxypyrazolo[1,5-*a*]pyridine-3-carbonitrile as raw materials with reference to step 2 of embodiment 106.

MS m/z (ESI):570.3 [M+H]⁺.

¹H NMR (400 MHz, CDCl₃) δ 8.42 (d, *J* = 2.1 Hz, 1H), 8.21 (s, 1H), 8.13 (d, *J* = 1.9 Hz, 1H), 7.92 (s, 1H), 7.81 (dd, *J* = 8.7, 2.2 Hz, 1H), 7.75 (s, 1H), 7.14 (d, *J* = 1.9 Hz, 1H), 6.71 (d, *J* = 8.8 Hz, 1H), 4.14 - 3.99 (m, 7H), 3.93 (s, 2H), 3.77 (s, 4H), 3.00 (s, 1H), 2.38 (dd, *J =* 15.0, 7.6 Hz, 1H), 2.29 (dd, *J* = 12.5, 7.4 Hz, 2H), 2.02 (t, *J* = 10.2 Hz, 2H), 1.78 (s, 2H), 1.45 (s, 3H).

### Embodiment 162

### 3-Chloro-N-(1-(5-(3-cyano-6-(3-hydroxy-3-methylazetidin-1-yl)pyrazolo[1,5-a]pyridin-4-yl)pyridin-2-yl)-4-methylpiperidin-4-yl)picolinamide

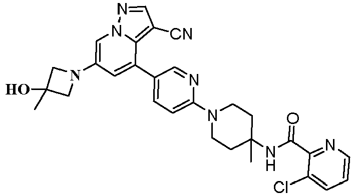

3-Chloro-*N*-(1-(5-(3-cyano-6-(3-hydroxy-3-methylazetidin-1-yl)pyrazolo[1,5-*a*]pyridin-4-yl)pyridin-2-yl)-4-methylpiperidin-4-yl)picolinamide was obtained by using 3-methylazetidin-3-ol and *N*-(1-(5-(6-bromo-3-cyanopyrazolo[1,5-*a*]pyridin-4-yl)pyridin-2-yl)-4-methylpiperidin-4-yl)-3-chloromethylpyridinamide as raw materials with reference to step 1 of embodiment 110.

MS m/z (ESI):557.2 [M+H]⁺.

¹H NMR (400 MHz, CDCl₃) δ 8.46 (d, *J* = 3.7 Hz, 1H), 8.33 (s, 1H), 8.14 (s, 1H), 7.94 (s, 1H), 7.83 (d, *J* = 8.6 Hz, 1H), 7.72 (s, 1H), 7.38 (dd, *J* = 8.1, 4.5 Hz, 1H), 6.92 (d, *J* = 9.6 Hz, 1H), 6.75 (s, 1H), 4.15 (s, 2H), 3.91 *(d, J =* 7.3 Hz, 2H), 3.82 (d, *J =* 7.2 Hz, 2H), 3.49 (s, 2H), 2.49 (s, 2H), 2.24 - 2.18 (m, 1H), 1.88 - 1.80 (m, 2H), 1.68 - 1.65 (m, 3H), 1.25 (s, 3H).

### Embodiment 163

### 3-Chloro-N-(1-(5-(3-cyano-6-(3-cyano-3-methylazetidin-1-yl)pyrazolo[1,5-a]pyridin-4-yl)pyridin-2-yl)-4-methylpiperidin-4-yl)picolinamide

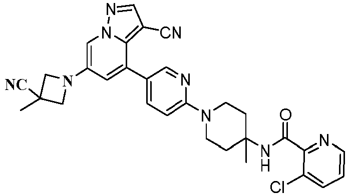

3-Chloro-*N*-(1-(5-(3-cyano-6-(3-cyano-3-methylazetidin-1-yl)pyrazolo[1,5-*a*]pyridin-4-yl)pyridin-2-yl)-4-methylpiperidin-4-yl)picolinamide was obtained by using 3-methylazetidin-3-carbonitrile and *N*-(1-(5-(6-bromo-3-cyanopyrazolo[1,5-*a*]pyridin-4-yl)pyridin-2-yl)-4-methylpiperidin-4-yl)-3-chloromethylpyridinamide as raw materials with reference to step 1 of embodiment 110.

MS m/z (ESI):566.2 [M+H]⁺.

¹H NMR (400 MHz, CDCl₃) δ 8.46 (dd, *J* = 4.5, 1.3 Hz, 1H), 8.31 (d, *J* = 2.3 Hz, 1H), 8.16 (s, 1H), 7.91 (s, 1H), 7.83 (dd, *J* = 8.1, 1.3 Hz, 1H), 7.71 (d, *J* = 1.9 Hz, 1H), 7.37 (dd, *J* = 8.1, 4.5 Hz, 1H), 6.82 (d,*J* = 8.9 Hz, 1H), 6.67 (d, *J* = 1.9 Hz, 1H), 4.28 (d, *J* = 7.0 Hz, 2H), 4.09 (d, *J* = 13.6 Hz, 2H), 3.88 (d, *J* = 7.0 Hz, 2H), 3.39 (t, *J* = 11.1 Hz, 2H), 2.43 *(d, J=* 13.8 Hz, 2H), 1.84 *(d, J=* 4.3 Hz, 1H), 1.82 (s, 3H), 1.78 (d, *J* = 4.1 Hz, 1H), 1.60 (s, 3H).

### Embodiment 164

### 4-(6-(6-((5-Fluoro-6-methoxypyridin-3-yl)methyl)-3,6-diazabicyclo[3.1.1]heptan-3-yl)pyridin-3-yl)-6-(3-hydroxy-3-methylazetidin-1-yl)pyrazolo[1,5-a]pyridine-3-carbonitrile

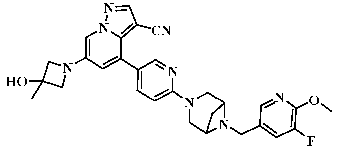

4-(6-(6-((5-Fluoro-6-methoxypyridin-3-yl)methyl)-3,6-diazabicyclo[3.1.1]heptan-3-yl)pyridin-3-yl)-6-(3-hydroxy-3-methylazetidin-1-yl)pyrazolo[1,5-*a*]pyridine-3-carbonitrile was obtained by using 3-methylazetidin-3-ol and 6-bromo-4-(6-(6-((5-fluoro-6-methoxypyridin-3-yl)methyl)-3,6-diazabicyclo[3.1.1]heptan-3-yl)pyridin-3-yl)pyrazolo[1,5-*a*]pyridine-3-carbonitrile as raw materials with reference to step 1 of embodiment 110.

MS m/z (ESI): 541.2 [M+H]⁺.

¹H NMR (400 MHz, CDCl₃) δ 8.40 (s, 1H), 8.15 (s, 1H), 7.95 (s, 2H), 7.82 (d, *J* = 8.2 Hz, 1H), 7.74 (d, *J* = 1.6 Hz, 1H), 6.74 (dd, *J =* 11.8, 5.2 Hz, 2H), 4.25 (s, 2H), 4.02 (s, 3H), 3.88 (dd, *J* = 36.0, 7.4 Hz, 7H), 3.22 (s, 1H), 2.25 - 2.18 (m, 1H), 2.01 (s, 1H), 1.85 (s, 2H), 1.68 (s, 3H).

### Embodiment 165

### 1-Cyano-N-(3-cyano-4-(6-(6-((6-methoxypyridin-3-yl)methyl)-3,6-diazabicyclo[3.1.1]heptan-3-yl)pyridinpyridin-3-yl)pyrazolo[1,5-a]pyridin-6-yl)cyclopropane-1-carboxamide

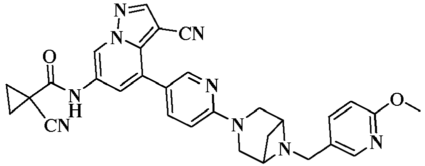

### Step 1: 1-cyano-N-(3-cyano-4-(6-(6-((6-methoxypyridin-3-yl)methyl)-3,6-diazabicyclo[3.1.1]heptan-3-yl)pyridinpyridin-3-yl)pyrazolo[1,5-a]pyridin-6-yl)cyclopropane-1-carboxamide

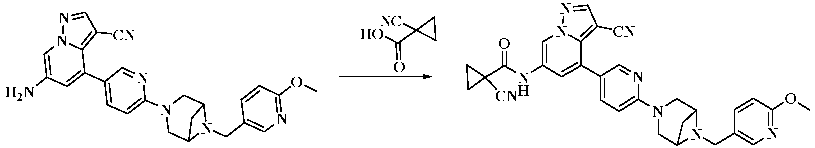

1-Cyano-*N*-(3-cyano-4-(6-(6-((6-methoxypyridin-3-yl)methyl)-3,6-diazabicyclo[3.1.1]heptan-3-yl)pyridin-3-yl)pyrazolo[1,5-*a*]pyridin-6-yl)cyclopropane-1-carboxamide (off-white solid) was obtained by using 6-amino-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile and 1-cyanocyclopropane-1-carboxylic acid as raw materials with reference to step 3 of embodiment 40.

MS m/z (ESI): 546.1 [M+H]⁺.

¹H NMR (400 MHz, DMSO-d₆) δ 10.43 (br., s, 1H), 9.26 (d, *J* = 1.7 Hz, 1H), 8.65 (s, 1H), 8.40 (d, *J* = 2.6 Hz, 1H), 8.07 (d, *J* = 2.3 Hz, 1H), 7.83 (dd, *J* = 8.8, 2.6 Hz, 1H), 7.74 - 7.64 (m, 2H), 6.82 (d, *J* = 8.8 Hz, 1H), 6.77 (d, *J* = 8.5 Hz, 1H), 3.82 (s, 3H), 3.80 - 3.65 (m, 4H), 3.61 - 3.49 (m, 4H), 1.79 - 1.70 (m, 3H), 1.59 (d, *J* = 8.4 Hz, 1H), 1.26 - 1.13 (m, 2H).

### Embodiment 166

### 6-(3-Hydroxy-4-methoxy-3-methylbut-1-yn-1-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

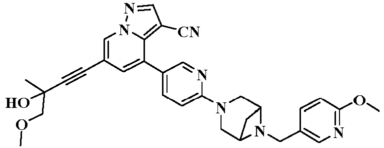

6-(3-Hydroxy-4-methoxy-3-methylbut-1-yn-1-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile (white solid) was obtained from 6-bromo-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile and 1-methoxy-2-methylbut-3-yn-2-ol.

MS m/z (ESI):550.3 [M+H]⁺.

¹H NMR (400 MHz, DMSO) δ 9.09 (s, 1H), 8.76 (s, 1H), 8.42 (s, 1H), 8.09 (s, 1H), 7.86 (s, 1H), 7.70 (s, 1H), 7.40 (s, 1H), 6.80 (s, 1H), 5.72 (s, 1H), 3.79 (m, 14H), 3.15 - 2.98 (m, 4H), 1.47 (s, 3H).

### Embodiment 167

### 6-(3-Cyano-3-methylazetidin-1-yl)-4-(6-(6-((5-fluoro-6-methoxypyridin-3-yl)methyl)-3,6-diazabicyclo[3.1.1]heptan-3-yl)pyridin-3-yl)pyrazolo[1,5-a]pyridine-3-carbonitrile

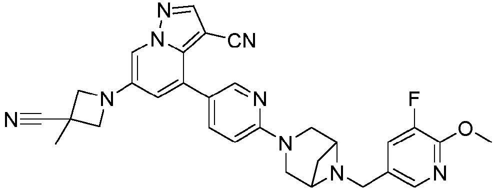

6-(3-Cyano-3-methylazetidin-1-yl)-4-(6-(6-((5-fluoro-6-methoxypyridin-3-yl)methyl)-3,6-diazabicyclo[3.1.1]heptan-3-yl)pyridin-3-yl)pyrazolo[1,5-*a*]pyridine-3-carbonitrile was obtained by using 6-bromo-4-(6-(6-(5-fluoro-6-methoxypyridin-3-yl)methyl)-3,6-diazabicyclo[3.1.1]heptan-3-yl)pyridin-3-yl)pyrazolo[1,5-*a*]pyridine-3-carbonitrile and 3-methylazetidin-3-carbonitrile as raw materials with reference to step 1 of embodiment 110.

MS m/z (ESI):550.2 [M+H]⁺.

¹H NMR (400 MHz, CDCl₃) δ 8.38 (d, *J =* 2.2 Hz, 1H), 8.18 (s, 1H), 7.87 (s, 1H), 7.79 (dd, *J* = 8.8, 2.4 Hz, 1H), 7.73 (d, *J* = 1.7 Hz, 1H), 7.54 (d, *J* = 10.5 Hz, 1H), 6.70 (dd, *J =* 5.2, 3.4 Hz, 2H), 4.29 (d, *J =* 7.0 Hz, 2H), 4.01 (s, 3H), 3.91-3.85 (m, 6H), 3.67-3.64 (m, 4H), 2.80 (s, 1H), 1.83 (s, 3H), 1.70 (d, *J=* 8.7 Hz, 1H).

### Embodiment 168

### 6-(3-Cyano-3-methylazetidin-1-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

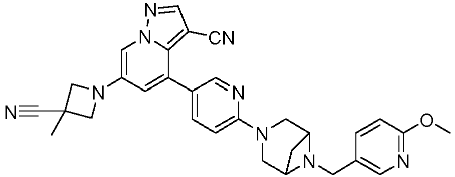

### Step 1: preparation of 6-(3-cyano-3-methylazetidin-1-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

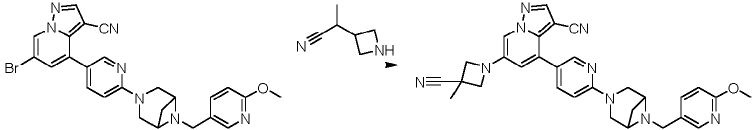

A mixture of 6-bromo-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile (100 mg, 0.19 mmol), tris(dibenzylideneacetone)dipalladium (9 mg, 0.0095 mmol), 2-dicyclohexylphospho-2',4',6'-triisopropylbiphenyl (5 mg, 0.011 mmol), cesium carbonate (123 mg, 0.38 mmol), 3-methylazetidin-3-carbonitrile (28 mg, 0.29 mmol) and toluene (5 mL) was replaced with nitrogen, stirred at 130 °C under microwave conditions for 2 hours. After the reaction was cooled to room temperature, the reaction mixture was concentrated, dissolved with ethyl acetate and washed with saturated saline, the organic phase was dried over anhydrous sodium sulfate, filtered, evaporated to dryness, and then purified by preparative chromatography to obtain 6-(3-cyano-3-methylazetidin-1-yl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile (45 mg, white solid, yield: 45%).

MS m/z (ESI):532.3 [M+H]⁺.

¹H NMR (400 MHz, DMSO) δ 8.53 (s, 1H), 8.40 (d, *J* = 2.1 Hz, 1H), 8.21 (d, *J* = 1.5 Hz, 1H), 8.07 (s, 1H), 7.83 (d, *J* = 8.7 Hz, 1H), 7.68 (d, *J* = 8.5 Hz, 1H), 7.03 (d, *J* = 1.7 Hz, 1H), 6.78 (t, *J* = 9.3 Hz, 2H), 4.27 (d, *J* = 7.7 Hz, 2H), 3.91 (d, *J* = 7.7 Hz, 2H), 3.82 (s, 3H), 3.76-3.67 (m, 4H), 3.56-3.50 (m, 4H), 2.04 - 1.93 (m, 1H), 1.67 (s, 3H), 1.59 (d, *J =* 7.9 Hz, 1H).

### Embodiment 169

### 6-((4-Hydroxytetrahydro-2H-pyran-4-yl)ethynyl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

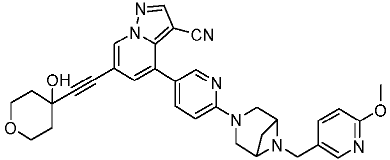

6-((4-Hydroxytetrahydro-2H-pyran-4-yl)ethynyl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile was obtained by using 6-bromo-4-(6-(6-((6-methoxypyridin-3-yl)methyl)-3,6-diazabicyclo[3.1.1]heptan-3-yl)pyridin-3-yl)pyrazolo[1,5-*a*]pyridine-3-carbonitrile and 4-ethynyltetrahydro-2H-pyran-4-ol as raw materials with reference to step 2 of embodiment 31.

MS m/z (ESI):562.3 [M+H]⁺.

¹H NMR (400 MHz, CDCl₃) δ 8.66 (s, 1H), 8.42 (d, *J* =2.0 Hz, 1H), 8.32 (s, 1H), 8.14 (s, 1H), 8.02 (s, 1H), 7.80 (d, J= 8.6 Hz, 1H), 7.31 (s, 1H), 6.79 *(d, J=* 8.6 Hz, 1H), 6.72 *(d, J=* 8.9 Hz, 1H), 4.12 (d,*J* = 7.1 Hz, 2H), 4.04 - 3.90 (m, 7H), 3.88 - 3.68 (m, 6H), 2.32 (s, 1H), 2.13 - 2.03 (m, 3H), 1.96-1.90 (m, 2H).

### Embodiment 170

### 4-(6-(6-((5-Fluoro-6-methoxypyridin-3-yl)methyl)-3,6-diazabicyclo[3.1.1]heptan-3-yl)pyridin-3-yl)-6-((4-hydroxytetrahydro-2H-pyran-4-yl)ethynyl)pyrazolo[1,5-a]pyridine-3-carbonitrile

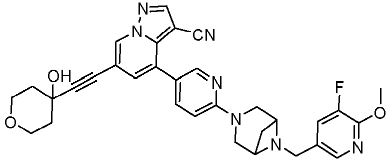

4-(6-(6-((5-Fluoro-6-methoxypyridin-3-yl)methyl)-3,6-diazabicyclo[3.1.1]heptan-3-yl)pyridin-3-yl)-6-((4-hydroxytetrahydro-2H-pyran-4-yl)ethynyl)pyrazolo[1,5-a]pyridine-3-carbonitrile was obtained by using 6-bromo-4-(6-(6-((5-fluoro-6-methoxypyridin-3-yl)methyl)-3,6-diazabicyclo[3.1.1]heptan-3-yl)pyridin-3-yl)pyrazolo[1,5-a]pyridine-3-carbonitrile and 4-ethynyltetrahydro-2H-pyran-4-ol as raw materials with reference to step 2 of embodiment 31.

MS m/z (ESI):580.2 [M+H]⁺.

¹H NMR (400 MHz, CDCl₃) δ 8.66 (s, 1H), 8.42 (s, 1H), 8.32 (s, 1H), 7.97 - 7.75 (m, 3H), 7.31 (s, 1H), 6.72 (d, *J* = 8.6 Hz, 1H), 4.20 - 4.08 (m, 2H), 4.05 - 3.93 (m, 7H), 3.84-3.70 (m, 6H), 2.13 - 2.03 (m, 3H), 1.97 - 1.88 (m, 3H).

### Embodiment 171

### 6-((3-Hydroxyoxetan-3-yl)ethynyl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

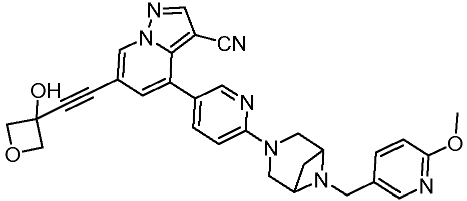

6-((3-Hydroxyoxetan-3-yl)ethynyl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile (white solid)) was obtained with reference to step 2 of embodiment **31.**

MS m/z (ESI): 534.2 [M+H]⁺.

¹H NMR (400 MHz, CDCl₃) δ 8.66 (s, 1H), 8.40 (s, 1H), 8.33 (s, 1H), 8.12 (s, 1H), 7.77 (d, *J* = 8.7 Hz, 2H), 7.29 (d, *J* = 10.6 Hz, 1H), 6.72 (dd, *J* = 16.3, 8.5 Hz, 2H), 4.95 (d, *J* = 6.7 Hz, 2H), 4.83 (d, *J =* 6.7 Hz, 2H), 3.92 (s, 3H), 3.89 (s, 4H), 3.67 (s, 4H), 2.82 (s, 1H), 1.72 (d, *J* = 8.2 Hz, 2H).

### Embodiment 172

### 4-(6-(6-((5-Fluoro-6-methoxypyridin-3-yl)methyl)-3,6-diazabicyclo[3.1.1]heptan-3-yl)pyridin-3-yl)-6-((3-hydroxyoxetan-3-yl)ethynyl)pyrazolo[1,5-a]pyridine-3-carbonitrile

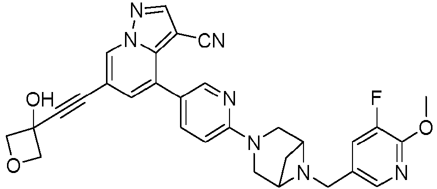

4-(6-(6-((5-Fluoro-6-methoxypyridin-3-yl)methyl)-3,6-diazabicyclo[3.1.1]heptan-3-yl)pyridin-3-yl)-6-((3-hydroxyoxetan-3-yl)ethynyl)pyrazolo[1,5-*a*]pyridine-3-carbonitrile (white solid) was obtained with reference to step 2 of embodiment **31.**

MS m/z (ESI): 552.2 [M+H]⁺.

¹H NMR (400 MHz, CDCl₃) δ 8.66 (s, 1H), 8.41 (s, 1H), 8.33 (s, 1H), 7.89 (s, 1H), 7.77 (d, *J* = 8.7 Hz, 1H), 7.54 (s, 1H), 7.30 (s, 1H), 6.70 (d, *J =* 8.7 Hz, 1H), 4.95 *(d, J=* 6.7 Hz, 2H), 4.83 (d, *J* = 6.8 Hz, 2H), 4.01 (s, 3H), 3.88 (s, 4H), 3.66 (s, 4H), 2.81 (s, 1H), 1.71 (d, *J* = 6.8 Hz, 2H).

### Embodiment 173

### 6-((1-Hydroxycyclobutyl)ethynyl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile

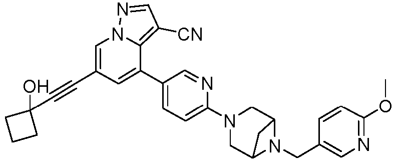

6-((1-Hydroxycyclobutyl)ethynyl)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile was obtained by using 6-bromo-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile and 1-ethynylcyclobutan-1-ol as raw materials with reference to embodiment 31.

MS m/z (ESI): 532.2 [M+H]⁺.

¹H NMR (400 MHz, DMSO-d₆) δ 9.14 (d, J = 1.3 Hz, 1H), 8.75 (s, 1H), 8.42 (d, J = 2.5 Hz, 1H), 8.07 (d, J = 2.3 Hz, 1H), 7.86 (dd, J = 8.8, 2.5 Hz, 1H), 7.68 (dd, J = 8.5, 2.4 Hz, 1H), 7.46 (d, J = 1.4 Hz, 1H), 6.83 - 6.73 (m, 2H), 6.00 (s, 1H), 3.82 (s, 3H), 3.79 - 3.67 (m, 4H), 3.60 - 3.50 (m, 4H), 2.58 - 2.56 (m, 1H), 2.47 - 2.39 (m, 2H), 2.29 - 2.19 (m, 2H), 1.85 - 1.78 (m, 2H), 1.62 - 1.57 (m, 1H).

### Embodiment 174

### 4-(6-(6-((5-Fluoro-6-methoxypyridin-3-yl)methyl)-3,6-diazabicyclo[3.1.1]heptan-3-yl)pyridin-3-yl)-6-((1-hydroxycyclobutyl)ethynyl)pyrazolo[1,5-a]pyridine-3-carbonitrile

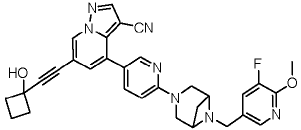

4-(6-(6-((5-Fluoro-6-methoxypyridin-3-yl)methyl)-3,6-diazabicyclo[3.1.1]heptan-3-yl)pyridin-3-yl)-6-((1-hydroxycyclobutyl)ethynyl)pyrazolo[1,5-*a*]pyridine-3-carbonitrile was obtained by using 6-bromo-4-(6-(6-((5-fluoro-6-methoxypyridin-3-yl)methyl)-3,6-diazabicyclo[3.1.1]heptan-3-yl)pyridin-3-yl)pyrazolo[1,5-a]pyridine-3-carbonitrile and 1-ethynylcyclobutan-1-ol as raw materials with reference to step 2 of embodiment 31.

MS m/z (ESI): 550.2 [M+H]⁺.

¹H NMR (400 MHz, DMSO-d₆) δ 9.14 (s, 1H), 8.75 (s, 1H), 8.42 (s, 1H), 7.91 (s, 1H), 7.86 (dd, J = 8.7, 2.6 Hz, 1H), 7.64 (d, J = 11.4 Hz, 1H), 7.45 (d, J = 1.3 Hz, 1H), 6.80 (d, J = 8.8 Hz, 1H), 5.99 (s, 1H), 3.92 (s, 3H), 3.76 - 3.68 (m, 4H), 3.60 - 3.51 (m, 4H), 2.58 - 2.56 (m, 1H), 2.45 - 2.35 (m, 2H), 2.27 - 2.23 (m, 2H), 1.83 - 1.78 (m, 2H), 1.59 (d, J = 8.2 Hz, 1H).

### Embodiment 175

### 3-Chloro-N-(1-(5-(3-cyano-6-((3-hydroxyoxetan-3-yl)ethynyl)pyrazolo[1,5-a]pyridin-4-yl)pyridin-2-yl)-4-methylpiperidin-4-yl)picolinamide

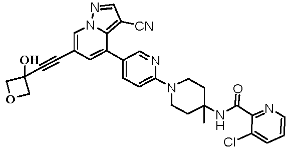

3-Chloro-*N*-(1-(5-(3-cyano-6-((3-hydroxyoxetan-3-yl)ethynyl)pyrazolo[1,5-*a*]pyridin-4-yl)pyridin-2-yl)-4-methylpiperidin-4-yl)picolinamide was obtained by using 3-ethynyloxetancyclo-3-ol and *N*-(1-(5-(6-bromo-3-cyanopyrazolo[1,5-*a*]pyridin-4-yl)pyridin-2-yl)-4-methylpiperidin-4-yl)-3-chloromethylpyridinamide as raw materials with reference to step 2 of embodiment 31.

MS m/z (ESI):568.2 [M+H]⁺.

¹H NMR (400 MHz, CDCl₃) δ 8.57 (s, 1H), 8.39 (d, *J* = 3.9 Hz, 1H), 8.24 (s, 2H), 7.84 (s, 1H), 7.76 (d, *J* = 8.1 Hz, 1H), 7.67 - 7.60 (m, 1H), 7.31 (dd, *J =* 7.8, 4.6 Hz, 1H), 7.23 (s, 1H), 6.77 *(d, J=* 9.0 Hz, 1H), 4.87 (d, *J* = 6.6 Hz, 2H), 4.64 (d, *J* = 6.7 Hz, 2H), 3.99 (d, *J* = 13.6 Hz, 2H), 3.34 (t, *J* = 11.0 Hz, 2H), 2.36 (d, *J= 14.0* Hz, 2H), 1.73 (d, *J* = 3.8 Hz, 2H), 1.53 (s, 3H).

### [Not according to the invention] Embodiment 176

### 1-((8-(6-(6-((6-Methoxypyridin-3-yl)methyl)-3,6-diazabicyclo[3.1.1]heptan-3-yl)pyridin-3-yl)-[1,2,4]triazolo[1,5-a]pyridin-6-yl)oxo)-2-methylpropan-2-ol

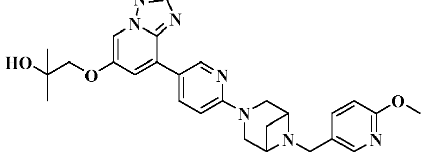

1-((8-(6-(6-((6-Methoxypyridin-3-yl)methyl)-3,6-diazabicyclo[3.1.1]heptan-3-yl)pyridin-3-yl)-[1,2,4]triazolo[1,5-*a*]pyridin-6-yl)oxy)-2-methylpropan-2-ol was obtained by using 8-(6-(6-((6-methoxypyridin-3-yl)methyl)-3,6-diazabicyclo[3.1.1]heptan-3-yl)pyridin-3-yl)-[1,2,4]triazolo[1,5-*a*]pyridin-6-ol and 1-chloro-2-methyl-2-propanol as raw materials with reference to step 1 of embodiment 152.

MS m/z (ESI): 502.2[M+H]⁺.

### Biological test evaluation

The present disclosure is further described below in conjunction with test embodiments to explain the disclosure, but these embodiments are not meant to limit the scope of the disclosure.

### I. Testing enzymology experiments

### Test embodiment 1: determination of the inhibitory effect of compounds of the present disclosure on the activity of RET wild-type and mutant kinases

### 1. Experimental purpose:

The purpose of this test embodiment is to measure the inhibitory ability of the compounds on the activity of RET wild-type and mutant kinases.

### 2.1 Experimental instruments:

Centrifuge (Eppendorf 5810R);
Microplate reader (BioTek Synergy H1);
Pipette (Eppendorf & Rainin).

### 2.2 Experimental reagents:

RET enzyme was purchased from Carna Company, and the article number was 08-159;
RET M918T enzyme was purchased from Carna Company, and the article number was 08-508;
KIF5B-RET was purchased from SignalChem Company, the article number was R02-19FG-05;
CCDC6-RET was purchased from SignalChem Company, the article number was R02-19BG-05;
RET V804M enzyme was purchased from Thermofisher Company, and the article number was PV6223;
RET V804L enzyme was purchased from Thermofisher Company, and the article number was PV4397;
HTRF KinEASE-TK kit was purchased from Cisbio Company, and the article number was 62TK0PEC;
ATP was purchased from Thermofisher Company, and the article number was PV3227;
384-well plate was purchased from PerkinElmer Company, and the article number was 6007290.

### 2.3 Test compound:

The compound of the embodiment of the present disclosure was self-made.

### 3. Experimental methods:

In this experiment, the homogeneous time-resolved fluorescence (HTRF) method was used for the detection of compounds on RET kinase activity. The experiments were carried out in 384-well plates, different concentrations of compound solutions were prepared using experimental buffers (25 mM HEPES, 10 mM MgCl2, 0.01% TritonX-100) and was added to 384-well plates, then diluted RET, RET M918T, CCDC6-RET, KIF5B-RET, RET V804M or RET V804L kinase solutions (0.01-2 nM) and substrate TK-substrate biotin (500 nM-1 µM) and Km (0.19-200 µM) concentrations of ATP solution were added thereto respectively, the total reaction system was 10 µL, the mixture was mixed well by centrifugation at 1000 rpm for 1 min; and the reaction was carried out at room temperature for 45 min, 10 µL of Sa-XL665 and TK-ab-Cryptate prepared with the assay solution were added, then the mixture was mixed well by centrifugation at 1000 rpm for 1 minute, and readings at 665 nm and 620 nm were recorded using a BioTek Synergy H1 instrument after 1 hour of reaction at room temperature.

### 4. Experimental data processing methods:

The IC₅₀ values were obtained by taking readings with a BioTek Synergy H1 instrument, the readings at 665 nm and 620 nm were recorded, and the ratio (665 nm/620 nm) was calculated, and then the inhibition rate was calculated, and the concentrations and the inhibition rates were fitted to a nonlinear regression curve using Graphpad Prism software.

### 5. Experimental results:

According to the above test methods, the test data obtained of the compounds of specific embodiments against multiple mutant kinases of RET were shown in Table 3.

**Table 3: The IC₅₀ values of the compounds for inhibiting the activity of multiple mutant kinases of RET**

| Embodimen t number | RET M918T IC₅₀ (nM) | CCDC6-RET IC₅₀ (nM) | KIF5B-RET IC₅₀ (nM) | RET V804M IC₅₀ (nM) | RET V804L IC₅₀ (nM) |
|---|---|---|---|---|---|
| 23 | 1.06 | NA | NA | NA | NA |
| 31 | 0.67 | NA | NA | NA | NA |
| 92 | 0.38 | NA | NA | NA | NA |
| 94 | 0.66 | NA | 0.97 | 1.8 | 0.74 |
| 97 | 0.79 | NA | NA | NA | NA |
| 106 | 0.47 | NA | NA | NA | NA |
| 152 | 0.21 | 0.85 | 0.53 | 0.6 | 0.24 |
| 153 | 0.17 | 1.74 | 0.36 | 3.0 | 0.23 |
| 168 | 0.41 | 0.98 | 0.42 | 0.6 | 0.49 |
| 175 | 0.37 | 1.72 | 0.50 | 1.1 | 0.28 |

| | | | | | |
|---|---|---|---|---|---|
| Note: "NA" in the table means no test was performed. | | | | | |

According to the above test methods, the test data obtained of the compounds of specific embodiments against wild-type mutant kinases of RET were shown in Table 2.

### 6. Experimental conclusion:

The compounds of embodiments of the present disclosure show good inhibitory activity against multiple mutant kinases of RET and significant activity in the drug-resistant mutations RET M918T, KIF5B-RET and RET V804L.

### Test embodiment 2: determination of the inhibitory effect of the compounds of the present disclosure on the activity of KDR kinase

### 1. Experimental purpose:

The purpose of this test embodiment was to measure the inhibitory ability of compounds on the activity of KDR kinases.

### 2.1 Experimental instruments:

Centrifuge (Eppendorf 5810R);
Microplate reader (BioTek Synergy H1);
Pipette (Eppendorf & Rainin)

### 2.2 Experimental reagents:

KDR kinase was purchased from Carna Company, and the article number was 08-191;
HTRF KinEASE-TK kit was purchased from Cisbio Company, and the article number was 62TK0PEC;
ATP was purchased from Thermofisher Company, and the article number was PV3227;
384-well plate was purchased from PerkinElmer Company, and the article number was 6007290.

### 2.3 Experimental compound:

The compound of the embodiment of the present disclosure was self-made.

### 3. Experimental methods:

In this experiment, the homogeneous time-resolved fluorescence (HTRF) method was used for the detection of compounds on KDR kinase activity. The experiments were carried out in 384-well plates, different concentrations of compound solutions were prepared using experimental buffers (25 mM HEPES, 10 mM MgCl2, 0.01% TritonX-100) and were added to 384-well plates, then diluted KDR kinase solutions (0.05 nM) and substrate TK-substrate biotin (500 nM-1 µM) and Km (0.19-200 µM) concentrations of ATP solution were added, the total reaction system was 10 µL, the mixture was mixed well by centrifugation at 1000 rpm for 1 min; and after the reaction was carried out at room temperature for 45 min, 10 µL of Sa-XL665 and TK-ab-Cryptate prepared with the assay solution were added, then the mixture was mixed well by centrifugation at 1000 rpm for 1 minute, and readings at 665 nm and 620 nm were recorded using a BioTek Synergy H1 instrument after 1 hour of reaction at room temperature.

### 4. Experimental data processing methods:

The IC₅₀ values were obtained by taking readings with a BioTek Synergy H1 instrument, the readings at 665nm and 620nm were recorded, and the ratio (665nm/ 620nm) was calculated, and then the inhibition rate was calculated, and the concentrations and the inhibition rates were fitted to a nonlinear regression curve using Graphpad Prism software.

### 5. Experimental results:

The test data of specific embodiments obtained through the above test methods were shown in Table 4:

**Table 4: Relative IC50 values of compounds inhibiting the activity of RET kinases and KDR kinases**

| Embodimen t number | RET IC₅₀ (nM) | KDR IC₅₀ (nM) | Ratio(KDR/RET) |
|---|---|---|---|
| 1 | 0.47 | 28 | 59 |
| 6 | 0.79 | 27.7 | 35 |
| 23 | 0.69 | 11 | 16 |
| 31 | 0.48 | 16 | 34 |
| 32 | 1.22 | 1.6 | 1.3 |
| 34 | 0.76 | 70.0 | 92 |
| 62 | 1.3 | 17 | 14 |
| 75 | 3.6 | 124 | 35 |
| 85 | 1.3 | 69 | 55 |
| 92 | 0.39 | 8.2 | 21 |
| 94 | 0.31 | 23 | 73 |
| 97 | 0.38 | 46 | 122 |
| 98 | 0.41 | 9.4 | 23 |
| 99 | 0.32 | 24 | 75 |
| 106 | 0.32 | 88 | 280 |
| 110 | 0.75 | 23 | 30 |
| 111 | 1.2 | 61 | 49 |
| 112 | 0.49 | 1.8 | 4 |
| 113 | 1.1 | 8.9 | 8 |
| 114 | 0.4 | 5.6 | 14 |
| 115 | 0.7 | 4.7 | 7 |
| 116 | 0.49 | 6.2 | 13 |
| 117 | 0.83 | 213.3 | 257 |
| 118 | 0.71 | 60.4 | 85 |
| 122 | 0.4 | 11 | 27 |
| 123 | 1.56 | 104.8 | 67 |
| 127 | 0.26 | 6.5 | 25 |
| 128 | 0.40 | 176 | 438 |
| 129 | 0.42 | 13 | 30 |
| 138 | 0.8 | 21.6 | 27 |
| 140 | 1.54 | 204.4 | 133 |
| 141 | 0.34 | 9 | 26 |
| 142 | 0.31 | 37.5 | 121 |
| 144 | 1.1 | 209.3 | 187 |
| 146 | 0.85 | 185.3 | 218 |
| 147 | 1.1 | 150.0 | 136 |
| 148 | 0.58 | 183.9 | 317 |
| 149 | 0.58 | 257.1 | 445 |
| 151 | 0.35 | 11.2 | 32 |
| 152A | 0.48 | 35.24 | 73 |
| 152 | 0.34 | 21.7 | 63 |
| 153 | 0.38 | 44.34 | 118 |
| 154 | 0.33 | 14.5 | 44 |
| 155 | 0.25 | 3.0 | 12 |
| 156 | 0.25 | 4.0 | 16 |
| 157 | 0.44 | 18.9 | 43 |
| 161 | 0.37 | 7.4 | 20 |
| 162 | 1.0 | 24 | 24 |
| 163 | 0.72 | 15.8 | 22 |
| 165 | 1.8 | 246.6 | 137 |
| 168 | 0.37 | 6.90 | 19 |
| 175 | 0.25 | 47.0 | 185 |

### 6. Experimental conclusion:

The above data show that the compounds of the embodiments shown in the present disclosure have strong inhibitory effect on RET kinase activity and poor inhibitory effect on KDR kinase activity. Comparing the two groups of data, it can be seen that the series of compounds of the present disclosure have high selectivity in inhibiting KDR/RET kinase activity.

### II. Testing cytological experiments

### Test embodiment 1: determination of the inhibitory effect of compounds of the present disclosure on the proliferation activity of TT cells

### 1. Experimental purpose:

The purpose of this test embodiment was to measure the inhibitory effect of the compounds on the proliferative activity of TT cells.

### 2.1 Experimental instruments:

Microplate reader (BioTek Synergy H1);
Pipette (Eppendorf & Rainin).

### 2.2 Experimental reagents:

TT cells were purchased from the cell bank of the Chinese Academy of Sciences.
Cell Titer-Glo cells were purchased from Promega Company, and the article number was G7573.

### 2.3 Test compound:

The compound of the embodiment of the present disclosure was self-made.

### 3. Experimental methods:

When TT cells were cultured to the appropriate fusion level, TT cells were collected, and the cells were adjusted to the appropriate cell concentration using complete medium, and the cell suspension was spread in a 96-well plate, 90 µL per well, and placed in a 37°C, 5% CO₂ incubator overnight; and compound solutions of different concentrations were prepared using DMSO and culture medium; and a solvent control was set, the compound solution was added to a 96-well plate, 10 µL per well, at 37°C in a 5% CO₂ incubator for 72 hours; CellTiter- Glo solution was added thereto and the mixture was mixed well by shaking, incubated for 10 min in the dark, and read by BioTek Synergy H1 microplate reader.

### 4. Experimental data processing methods:

The luminescence signal values were used to calculate the inhibition rate, the concentration and the inhibition rate were fitted to a nonlinear regression curve using Graphpad Prism software.

### 5. Experimental results:

**Table 5**

| Embodiment number | TT (MTC, RET C634W) IC₅₀ (nM) |
|---|---|
| 1 | 11.53 |
| 23 | 11.00 |
| 31 | 17.00 |
| 34 | 29.30 |
| 92 | 13.13 |
| 94 | 18.99 |
| 97 | 9.44 |
| 98 | 18.11 |
| 99 | 20.71 |
| 100 | 14.62 |
| 103 | 23.63 |
| 104 | 14.97 |
| 106 | 18.97 |
| 110 | 6.59 |
| 112 | 2.07 |
| 122 | 9.45 |
| 125 | 12.22 |
| 126 | 9.17 |
| 127 | 5.87 |
| 128 | 13.30 |
| 129 | 19.60 |
| 138 | 24.67 |
| 138 | 25.11 |
| 140 | 29.61 |
| 141 | 9.00 |
| 142 | 28.53 |
| 146 | 27.45 |
| 149 | 28.40 |
| 152 | 14.11 |
| 153 | 27.78 |
| 154 | 12.65 |
| *155* | 5.18 |
| 156 | 6.84 |
| 160 | 8.65 |
| 161 | 13.86 |
| 162 | 15.01 |
| 164 | 5.95 |
| 166 | 24.39 |
| 167 | 3.48 |
| 168 | 4.10 |
| 169 | 13.92 |
| 170 | 10.51 |
| 171 | 17.30 |
| 172 | 13.77 |
| 173 | 17.77 |
| 175 | 18.51 |

### 6. Experimental conclusion:

The above data show that the compounds of embodiments of the present disclosure have good inhibitory effect on the proliferation of TT cells.

### Test embodiment 2: determination of the inhibitory effect of compounds of the present disclosure on the proliferation activity of Ba/F3 KIFSB-RET cells

### 1. Experimental purpose:

The inhibitory effect of the compounds on the proliferative activity of Ba/F3 KIF5B-RET cells was measured.

### 2. Instruments and reagents:

### 2.1 Experimental instruments:

Microplate reader (BioTek Synergy H1);
Pipette (Eppendorf & Rainin).

### 2.2 Experimental reagents:

Ba/F3 KIF5B-RET was provided by Kyinno biotechnology Co., Ltd, and the cell number was CVCL_UE86, which can be found on the cell information website https://web.expasy.org/cellosaurus/, the stably transformed cell line does not need to rely on IL-3 for growth;
Cell Titer-Glo cells were purchased from Promega Company, and the article number was G7573.

### 2.3 Test compound:

The compound of the embodiment of the present disclosure was self-made.

### 3. Experimental methods:

When Ba/F3 KIF5B-RET cells were cultured to the appropriate cell density, cells were collected, and the cells were adjusted to the appropriate cell concentration using complete medium, and the cell suspension was spread in a 96-well plate, 90 µL per well, and placed in a 37°C, 5% CO₂ incubator for overnight; compound solutions of different concentrations were prepared using DMSO and culture medium; a solvent control was set, and the compound solution was added to a 96-well plate with 10 µL per well at 37°C in a 5% CO₂ incubator for 72 to 144 hours; CellTiter- Glo solution was added thereto and mixed well by shaking, incubated for 10 min in the dark, and read by BioTek Synergy H1 microplate reader.

### 4. Experimental data processing methods:

The luminescence signal values were used to calculate the inhibition rate, the concentrations and the inhibition rates were fitted to a nonlinear regression curve using Graphpad Prism software.

### 5. Experimental results:

**Table 6:**

| Embodiment number | Ba/F3 KIF5B-RET IC₅₀ (nM) |
|---|---|
| 1 | 24.62 |
| 23 | 13.00 |
| 31 | 12.00 |
| 34 | 28.88 |
| 94 | 22.62 |
| 95 | 27.83 |
| 99 | 16.94 |
| 100 | 15.42 |
| 103 | 18.55 |
| 104 | 13.54 |
| 106 | 15.90 |
| 125 | 15.30 |
| 126 | 7.06 |
| 138 | 30.64 |
| 142 | 19.61 |
| 146 | 32.10 |
| 148 | 33.97 |
| 149 | 17.96 |
| 151 | 16.77 |
| 152 | 11.05 |
| 153 | 22.56 |
| 154 | 7.03 |
| 155 | 14.84 |
| 156 | 5.33 |
| 157 | 26.30 |
| 160 | 5.59 |
| 161 | 8.59 |
| 162 | 22.46 |
| 164 | 12.04 |
| 166 | 34.73 |
| 167 | 6.04 |
| 168 | 4.79 |
| 169 | 15.62 |
| 170 | 13.66 |
| 171 | 23.93 |
| 172 | 16.79 |
| 173 | 21.93 |
| 175 | 15.49 |

### 6. Experimental conclusion:

The above data show that the compounds of embodiments of the present disclosure have good inhibitory effect on the proliferation of Ba/F3 KIF5B-RET cells.

### Test Example 4. The inhibitory effect of the compound of the present disclosure on the phosphorylation of ERK, a downstream signal factor of TT cells

### 1. Experimental purpose:

The inhibitory effect of the compound on the phosphorylation level of ERK, a downstream signal factor of TT cells, was detected.

### 2. Experimental instruments and reagents:

### 2.1 Experimental instruments:

Imager (Biorad ChemiDoc^{™}MP);
Pipette (Eppendorf & Rainin).

### 2.2 Experimental reagents:

pERK antibody was purchased from Cell Signaling Technology Company, the article number was 4370S;
Total ERK antibody was purchased from Cell Signaling Technology Company, the article number was 4696S;
The internal reference GAPDH was purchased from Cell Signaling Technology Company, the article number was 5174S;
The fluorescent secondary antibodies were purchased from LI-COR Company, the article numbers were P/N 925-68071 and P/N 926-32210.

### 2.3 Test compound:

The compound of the embodiment of the present disclosure was self-made.

### 3. Experimental methods:

In this experiment, the inhibitory effect of compounds on the phosphorylation level of ERK, a downstream signaling factor in TT cells, was measured by Western Blot method. When TT cells were cultured to the appropriate fusion degree, the cells were collected and adjusted to a suitable cell concentration using complete medium, the cell suspension was spread in a 24-well plate, 1 mL per well, and placed in a 37°C, 5% CO₂ incubator overnight, and compound dilutions of different concentrations (3.7 nM, 11.1 nM, 33.3 nM, 100 nM, 300 nM) were added at 37°C and acted for 2 hours; the cell supernatant was aspirated, washed one time with PBS, and the proteins were collected with lysate; after protein denaturation, Western blot experiments were performed: performing protein electrophoresis at 120V for approximately 75 minutes, followed by 45 minutes of transfer to PVDF membranes at 10V with a semi-dry transfer instrument, enclosing at 5% BSA for 1 hour at room temperature, and then PVDF membranes were cut into strips of appropriate size and incubated with prepared antibody dilutions overnight at 4°C and washed 6 times with TBST, followed by imaging with goat anti-mouse secondary antibody and sheep anti-rabbit secondary antibody for 1 hour at room temperature, and the membrane was washed 6 times with TBST for imaging in the Biorad ChemiDoc^{™} MP Imaging System.

### 4. Experimental data processing methods:

The inhibitory effect of compounds on ERK phosphorylation levels in TT cells at different concentrations was determined by detecting protein bands.

### 5. Experimental results:

Embodiment 152 and embodiment 153 can significantly inhibit the phosphorylation level of ERK in TT cells with a dose-dependent effect. After the compound was incubated with TT cells for 2 hours at 37°C, embodiment 152 almost completely inhibited ERK phosphorylation at 300 nM, 100 nM, 33.3 nM, and 11.1 nM; and at 3.7 nM, it could inhibit about half of the ERK phosphorylation level. Whereas, embodiment 153 can completely inhibit the phosphorylation of ERK at 300 nM and 100 nM, the degree of inhibition was reduced at 33.3 nM, at 11.1 nM, it could inhibit half of the ERK phosphorylation level, and the inhibition level was weaker at 3.7 nM.

### 6. Experimental conclusion:

According to the above scheme, the compounds shown in the present disclosure show dose-dependent inhibitory effect on the phosphorylation of ERK, the signal factor downstream of TT cells.

### III. Determination of Balb/C mouse pharmacokinetics

### 1. Research purpose:

The pharmacokinetic behavior of the following compound embodiments, administered orally at a dose of 5 mg/kg in plasma in mice, was studied using Balb/C mice as test animals.

### 2. Test scheme:

### 2.1 Test drugs:

The compound of the embodiment of the present disclosure was self-made.

### 2.2 Test animals:

Balb/C Mouse 6/embodiment, male, Shanghai Jiesijie Laboratory Animal Co., Ltd, Animal Production License No.(SCXK (Shanghai) 2013-0006 N0.311620400001794).

### 2.3 Administration:

Balb/C mice, males; p.o. after overnight fasting, respectively, at a dose of 5 mg/kg, administered in a volume of 10 mL/kg.

### 2.4 Sample preparation:

0.5%CMC-Na(1%Tween80) was dissolved by ultrasound, and prepared into clear solution or uniform suspension.

### 2.5 Sample collection:

Before and after administration, 0.1 mL of blood was collected from mice at 0, 0.5, 1, 2, 4, 6, 8 and 24 hours by orbital collection, placed in EDTA-K₂ tubes, centrifuged at 6000 rpm at 4 °C for 6 min to separate plasma, and stored at -80 °C.

### 2.6 Sample treatment:

40 µL of plasma sample was precipitated by adding 160 µL of acetonitrile, mixed and centrifuged at 3500 × g for 5-20 min.
100 µL of the supernatant solution after treatment was taken to analyze the concentration of the test compound by LC/MS/MS.

### 2.7 Liquid phase analysis

Liquid phase conditions: Shimadzu LC-20AD pump
Mass spectrometry conditions: AB Sciex API 4000 mass spectrometer
Chromatographic column: phenomenex Gemiu 5 µM C18 50 × 4.6 mm
Mobile phase: Liquid A was 0.1% formic acid aqueous solution, liquid B was acetonitrile
Flow rate: 0.8 mL/min
Elution time: 0-4.0 minutes, the eluent was as follows:

**Table 7**

| Time/minute | Liquid A | Liquid B |
|---|---|---|
| 0.01 | 90% | 10% |
| 0.5 | 90% | 10% |
| 0.8 | 5% | 95% |
| 2.4 | 5% | 95% |
| 2.5 | 90% | 10% |
| 4.0 | Stop | |

### 3. Experimental results and analysis

The main pharmacokinetic parameters were calculated using WmNonlin 6.1 and the results of the mouse pharmacokinetic experiments were shown in Table 8 below.

**Table 8 Results of pharmacogenetic experiments in mice**

| Emb odim ent Num ber | Pharmacokinetic experiment (5 mg/kg) | | | | | |
|---|---|---|---|---|---|---|
| | Peak time | Blood concentrati on | Curve area | Curve area | Half-life | Average residence time |
| | tₘₐₓ(ng/mL) | Cₘₐₓ(ng/mL) | AUC₀₋ₜ(ng/mL×h) | AUC_{0-∞}(ng/mL×h) | t_{1/2}(h) | MRT(h) |
| 23 | 0.5 | 3283.3 | 13821.5 | 13830.0 | 2.18 | 3.76 |
| 31 | 0.5 | 3706.7 | 21369.7 | 21511.8 | 3.37 | 4.91 |
| 34 | 0.5 | 1920.0 | 16076.7 | 16389.2 | 4.2 | 5.84 |
| 94 | 0.5 | 2786.7 | 18167.5 | 18230.7 | 2.9 | 4.61 |
| 106 | 0.5 | 3120.0 | 18865.2 | 18904.9 | 2.66 | 4.71 |
| 117 | 0.5 | 1490.0 | 6208.1 | 6220.8 | 2.19 | 3.80 |
| 125 | 0.5 | 1616.7 | 13953.1 | 14019.7 | 3.45 | 5.22 |
| 142 | 2.0 | 1943.3 | 21624.6 | 22084.0 | 4.26 | 6.51 |
| 146 | 0.5 | 9340.0 | 24470.9 | 24473.6 | 1.7 | 2.85 |
| 148 | 0.5 | 1326.7 | 7343.3 | 7369.3 | 2.34 | 4.27 |
| 149 | 0.5 | 3083.0 | 27955.0 | 28150.0 | 3.26 | 5.56 |
| 152A | 0.5 | 2816.7 | 13773.0 | 13789.2 | 2.4 | 4.07 |
| 152 | 1.0 | 2936.7 | 10978.5 | 10981.2 | 1.3 | 2.90 |
| 153 | 1.0 | 3920.0 | 14458.5 | 14460.7 | 1.2 | 2.70 |
| 154 | 2.0 | 2400.0 | 20159.0 | 20251.0 | 3.05 | 5.24 |
| 157 | 0.5 | 1703.3 | 7208.0 | 7218.5 | 1.73 | 3.47 |
| 168 | 0.5 | 4680.0 | 51613.2 | 51946.2 | 3.2 | 6.0 |

### 4. Experimental conclusion:

It can be seen from the results of mouse pharmacokinetic experiments in the table that the compounds of the embodiments of the present disclosure show good metabolic properties, and both the exposure AUC and the maximum blood drug concentration Cₘₐₓ show good performance.

### IV. Tumor suppression experiment on Ba/F3 KIFSB-RET transplanted tumor model

### Experimental purpose:

To evaluate the anti-tumor activity of the tested compounds against the subcutaneously transplanted tumor of Ba/F3 KIF5B-RET cells in nude mice.

### 2. Experimental instruments and reagents:

### 2.1 Instrument:

Ultra Clean Bench (BSC-1300II A2, Shanghai Boxun Industrial Co., Ltd. Medical Equipment Factory);
CO₂ incubator (311,Thermo);
Centrifuge (Centrifuge 5720R, Eppendorf);
Automatic cell counter (Countess II, life);
Pipette (10-20 µL, Eppendorf);
Microscope (TS100, Nikon);
Vernier caliper (500-196, Sanfeng, Japan);
Cell culture flask (T25/T75/T225, Corning).

### 2.2 Reagents:

RPMI1640 (22400-089, Gibco);
Fetal bovine serum (FBS)(10099-141, Gibco);
Phosphate buffer (PBS)(10010-023, Gibco).

### 2.3 Test compound:

The compound of the embodiment of the present disclosure was self-made.

### 3. Experimental operation:

Ba/F3 KIF5B-RET cells were removed from the cell bank, resuscitated and added to RPMI1640 medium (RPMI1640+10% FBS+1% Glu+1% P/S), then cultured in a CO₂ incubator (incubator temperature was 37°C, CO₂ concentration was 5%), and when the cell number expanded to the required number for *in vivo* inoculation, Ba/F3 KIF5B-RET cells were collected. The cells were counted with an automatic cell counter, resuspended with PBS according to the count results, made into a cell suspension (density was 2×107/mL), and placed in an ice box for use.

Female BALB/c nude mice aged 6-8 weeks were used, weighing about 18-22g. Mice were reared in SPF animal house, and were reared in a single cage with 5 mice in each cage. All cages, bedding and water were sterilized at high temperature before use, and all animals can eat and drink freely. Nude mice were labeled with disposable universal ear tags for mice and rats before the start of the experiment, and the skin of the inoculation site was disinfected with 75% medical alcohol before inoculation. 0.1 mL (containing 2*106 cells) of Ba/F3 KIF5B-RET cells were inoculated subcutaneously on the right back of each mouse. When the tumor volume reached 60-200 mm³, it began to be administered in groups, with 5 rats in each group. Each test compound was orally administered twice a day for 14 days. The tumor volume was measured twice a week, the weight of mice was weighed, and the tumor TGI(%) was calculated.

### 4. Data processing:

The tumor volume (mm³) was calculated as V=0.5*D*d*d, where D and d were the major and minor diameters of the tumor, respectively.

### Calculation of TGI (%):

When there was no tumor regression, TGI(%) = [(1-(mean tumor volume at the end of the administration in a treatment group - mean tumor volume at the start of administration in the treatment group))/(mean tumor volume at the end of treatment in the solvent control group - mean tumor volume at the start of treatment in the solvent control group)] × 100%.When there was tumor regression, TGI(%) = [1-(mean tumor volume at the end of dosing in a treatment group - mean tumor volume at the beginning of dosing in the treatment group)/mean tumor volume at the beginning of dosing in the treatment group] × 100%.

### 5. Experimental results:

**Table 9 Pharmacodynamic parameters**

| **Grouping** | **Tumor volume (mm³, Mean** ± **SD)** | | **TGI(%)** |
|---|---|---|---|
| | **Day 0** | **Day 14** | **Day 14** |
| 23 | 100.80 ± 31.28 | 232.46 ± 133.65 | 90.82 |
| 10 mg/kg | (101.24 ± 35.15) | (1,536.07 ± 263.57) | |
| 94 | 132.34 ± 19.40 | 118.64 ± 84.34 | 110.35 |
| 30 mg/kg | (133.86 ± 28.86) | (1,366.89 ± 291.81) | |
| 146 | 133.41 ± 18.82 | 98.26 ± 33.71 | 126.35 |
| 30 mg/kg | (133.86 ± 28.86) | (1,366.89 ± 291.81) | |
| 152A | 100.24 ± 27.85 | 247.52 ± 85.65 | 89.74 |
| 10 mg/kg | (101.24 ± 35.15) | (1,536.07 ± 263.57) | |
| 152 | 126.13 ± 17.24 | 219.86 ± 64.31 | 94.66 |
| 10 mg/kg | (126.52 ± 16.11) | 1,882.69 ± 968.48) | |
| 153 | 126.93 ± 16.98 | 330.85 ± 147.00 | 88.39 |
| 10 mg/kg | (126.52 ± 16.11) | (1,882.69 ± 968.48) | |
| 154 | 100.40 ± 29.77 | 276.69 ± 106.21 | 87.71 |
| 10 mg/kg | (101.24 ± 35.15) | (1,536.07 ± 263.57) | |
| 168 | 101.59 ± 36.86 | 40.84 ± 53.92 | 159.80 |
| 10 mg/kg | (101.24 ± 35.15) | (1,536.07 ± 263.57) | |

| | | | |
|---|---|---|---|
| Note: The data in parentheses indicate that the tumor volume of the embodiment corresponding to the Vehicle QD x 3w group (i.e., control group) at the corresponding time | | | |

### 6. Experimental conclusion:

The above data show that after 14 days of continuous oral administration, the compounds of embodiments of the present disclosure can significantly inhibit the growth of Ba/F3 KIF5B-RET nude mouse transplant tumors.

### V. Pharmacodynamic experiment on subcutaneous xenotransplantation tumor model of human bone marrow-like thyroid cancer cell TT

### 1. Experimental purpose:

BALB/c nude mice were used as the test animals, and the TT xenograft tumor model of human myeloid thyroid cancer cells was used for *in* vivo pharmacodynamic experiments to evaluate the antitumor effects of the test compounds.

### 2. Experimental instruments and reagents:

### 2.1 Instrument:

CO₂ incubator (311, Thermo);
High-speed refrigerated centrifuge (Multifuge X3R, Thermo);
Automatic cell counter (Mini-006-0484, cellometer);
Biosafety cabinet (1300 SERIES A2, Thermo);
Electronic balance (JJ300Y, Changshu Shuangjie);
Vernier caliper (0-150mm/0.01mm, Mitutoyo, Japan).

### 2.2 Reagents:

F-12K (21127-022, Gibco);
Fetal bovine serum (FBS)(10099-141, Gibco);
Penicillin dual antibodies (PS) (SV30010, Hyclone).

### 3 Test animals:

BALB/c nude mice, female, 6-8 weeks old, weighing 18-22 g, provided by Shanghai Lingchang Biotechnology Co., Ltd.

### 4 Test compound:

The compound of the embodiment of the present disclosure was self-made.

### 5. Experimental operation:

### 5.1 Cell culture:

TT cells were cultured *in vitro* in a monolayer under the following conditions: F-12K medium with 20% heat-inactivated fetal bovine serum and 1% penicillin-streptomycin double antibodies at 37°C and 5% CO₂. Trypsin-EDTA was used twice a week digestion for digestion treatment passaging. When cells were in an exponential growth period, cells were collected, counted and inoculated.

### 5.2 Tumor inoculation:

TT cells were collected when they were full in a logarithmic growth period, ensuring over 90% of vitality. 0.2 mL cell suspension containing about 1 x 10⁷ of TT cells (cells suspended in basic F-12K medium and added with 50% Matrigel) was subcutaneously inoculated into the right back tumor of each mouse when the average volume of the tumor reached about 150-200 mm³.

### 5.3 Experimental grouping and administration:

1. The day of grouping was day 0 (D0). The interval of BID administration was 6-8 h/18-16 h. The first dose was given in the afternoon of day D0 and the last dose was given in the morning of day D21.
2. Dosing volume: 10 L/g according to the body weight of nude mice.

Animal husbandry: animals were kept in the experimental environment for 7 days after arrival to start the experiment. Animals were housed in SPF animal rooms in IVC (Independent Ventilation System) cages (5 per cage).

Animal grouping: Before administration, the animals were weighed and the tumor volume was measured. The mice were randomly grouped according to tumor volume (randomized grouping design) with 5 animals per group.

Observation: animals were monitored daily for health status and death, routine checks include observation of the effects of medication on daily behavioral performance such as behavioral activity, food and water intake, changes in body weight (measured twice a week or every other day), physical signs or other abnormalities. The number of animal deaths and side effects within the group were recorded based on the number of animals in each group.

Experimental index: The diameter of tumor was measured by vernier caliper twice a week. The tumor volume was calculated by the formula: V = a×b²/2, a and b denote the long and short diameter of the tumor, respectively. The tumor suppressive efficacy of the compounds was evaluated by TGI (%). TGI (%) =(1-(TV_{Treatment/Dx} =TV_{Treatment/D1})/(TV_{Contro/Dx}-TV_{Control/D1}))×100%. Tumor suppressive efficacy was measured by TGI=(1-(TW_{control}-TWₜᵣₑₐₜₘₑₙₜ)/TW_{control}×100%. TGI ≧ 58% indicates that the tested substance can effectively inhibit tumor growth, and TGI ≧ 90% indicates that the tested substance can effectively inhibit tumor growth.

### 5.4 Data analysis:

T test was used for comparison between two groups. One-way ANOVA was used for comparison among three or more groups. If there were significant differences in F values, multiple comparisons should be made after ANOVA analysis. GraphPadPrism 5.0 was used for all data analysis.

### 6. Experimental results:

**Table 10 Pharmacodynamic parameters**

| Grouping | Tumor volume (mm³, mean±SEM) | | Tumor inhibition rate TGI |
|---|---|---|---|
| | d0 | d22 | d22 |
| Vehicle | 206.10 ± 22.69 | 1,276.23 ± 161.33 | - |
| 94 | 199.73 ± 24.07 | 203.56 ± 31.74 | 99.64% |
| 30 mg/kg | | | |
| 168 | 205.23 ± 16.57 | 144.14 ± 8.95 | 129.77% |
| 10 mg/kg | | | |

### 7. Experimental conclusion:

The above data show that after 21 days of continuous oral administration, the compounds of embodiments of the present disclosure can significantly inhibit the growth of TT nude mouse transplanted tumors of thyroid cancer cells.

Although the above describes specific embodiments of the present disclosure, it should be understood by those skilled in the art that these are merely illustrative embodiments and that a variety of changes or modifications can be made to these embodiments without departing from the principles and substance of the present disclosure. Therefore, the scope of protection of the present disclosure is defined by the appended claims.

## Claims

1. A compound represented by general formula (XI) or (XI-A), a stereoisomer thereof or a pharmaceutically acceptable salt thereof: 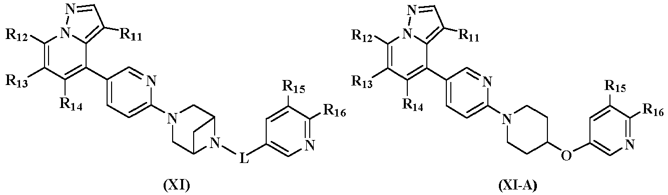 wherein:
L is selected from bond, -(CH₂)ₙ₁CRₐₐR_{1b}-, -(CH₂)ₙ₁NRₐₐC(O)(CH₂)ₙ₂-, - (CH₂)ₙ₁C(O)(CH₂)ₙ₂(CRₐₐR_{bb})ₘ-, -(CH₂)ₙ₁C(O)(CRₐₐR_{bb})ₘ(CH₂)ₙ₂-, -(CH₂)ₙ₁C(O)NR_{cc}(CRₐₐR_{bb})ₙ₂-, - (CH₂)ₙ₁(O)(CH₂)ₙ₂- or -(CH₂)ₙ₁NRₐₐ(CH₂)ₙ₂-;
R₁₁ is selected from hydrogen, deuterium, alkyl, deuterated alkyl, haloalkyl, alkoxy, haloalkoxy, halogen, amino, nitro, hydroxyl, cyano, alkenyl, alkynyl, cycloalkyl, heterocyclyl, aryl, heteroaryl, - (CH₂)ₙ₁Rₐₐ, -(CH₂)ₙ₁ORₐₐ, -(CH₂)ₙ₁S(CH₂)ₙ₂Rₐₐ, -(CH₂)ₙ₁C(O)Rₐₐ, -(CH₂)ₙ₁C(O)ORₐₐ, -(CH₂)ₙ₁S(O)ₘRₐₐ, - (CH₂)ₙ₁S(O)(=NRₐₐ)(CH₂)ₙ₂R_{bb}, -(CH₂)ₙ₁NRₐₐR_{bb}, -(CH₂)ₙ₁P(O)RₐₐR_{bb}, -(CH₂)ₙ₁C(O)NRₐₐR_{bb}, - (CH₂)ₐ₁NRₐₐC(O)R_{bb} or -(CH₂)ₙ₁NRₐₐS(O)ₘR_{bb};
R₁₂ is hydrogen, deuterium, alkyl, deuterated alkyl, haloalkyl, alkoxy, haloalkoxy, halogen, amino, nitro, hydroxyl, cyano, alkenyl, alkynyl, cycloalkyl, heterocyclyl, aryl or heteroaryl;
R₁₃ is selected from alkenyl, alkynyl, -(C≡C)ₙ₁(CRₐₐR_{bb})ₘR_{cc}, -(C≡C)ₙ₁(CRₐₐR_{bb})ₘR_{cc}, - (CH₂)ₙ₁O(CH₂)ₙ₂(CRₐₐR_{bb})ₘR_{cc}, wherein the alkenyl, or alkynyl is optionally further substituted by one or more substituents selected from hydrogen, deuterium, unsubstituted alkyl, unsubstituted deuterated alkyl, unsubstituted haloalkyl, unsubstituted hydroxyalkyl, unsubstituted cyanoalkyl, unsubstituted alkoxy, unsubstituted haloalkoxy, halogen, unsubstituted amino, nitro, hydroxyl, cyano, oxo, thio, imino, unsubstituted alkenyl, unsubstituted alkynyl, unsubstituted cycloalkyl, unsubstituted heterocyclyl, unsubstituted aryl, unsubstituted heteroaryl, -(CH₂)ₙ₁R_{dd}, -(CH₂)ₙ₁OR_{dd}, -(CH₂)ₙ₁(CH₂)ₙ₂R_{dd}, - (CH₂)ₙ₁C(O)R_{dd}, -(CH₂)ₙ₁C(O)OR_{dd}, -(CH₂)ₙ₁S(O)ₘR_{dd}, -(CH₂)ₙ₁S(O)(=NR_{dd})(CH₂)ₙ₂Rₑₑ, -(CH₂)ₙ₁NR_{dd}Rₑₑ, - (CH₂)ₙ₁P(O)R_{dd}Rₑₑ, -(CH₂)ₙ₁C(O)NR_{dd}Rₑₑ, -(CH₂)ₙ₁NR_{dd}C(O)Rₑₑ or -(CH₂)ₙ₁NR_{dd}S(O)ₘRₑₑ;
R₁₄ is hydrogen, deuterium, alkyl, deuterated alkyl, haloalkyl, alkoxy, haloalkoxy, halogen, amino, nitro, hydroxyl, cyano, alkenyl, alkynyl, cycloalkyl, heterocyclyl, aryl or heteroaryl;
R₁₅ and R₁₆ are each independently selected from hydrogen, deuterium, alkyl, deuterated alkyl, haloalkyl, alkoxy, haloalkoxy, halogen, amino, nitro, hydroxyl, cyano, alkenyl, alkynyl, cycloalkyl, heterocyclyl, aryl, heteroaryl or -(CH₂)ₙ₁ORₐₐ;
Rₐₐ, R_{bb}, R_{dd} and Rₑₑ are each independently selected from hydrogen, deuterium, alkyl, deuterated alkyl, haloalkyl, alkoxy, haloalkoxy, halogen, cyano, nitro, hydroxyl, amino, alkenyl, alkynyl, cycloalkyl, heterocyclyl, aryl or heteroaryl, wherein the alkyl, deuterated alkyl, haloalkyl, alkoxy, haloalkoxy, alkenyl, alkynyl, cycloalkyl, heterocyclyl, aryl and heteroaryl are optionally further substituted by one or more substituents selected from hydrogen, deuterium, unsubstituted alkyl, unsubstituted deuterated alkyl, unsubstituted haloalkyl, unsubstituted alkoxy, unsubstituted haloalkoxy, halogen, cyano, nitro, hydroxyl, amino, oxo, imino, unsubstituted alkenyl, unsubstituted alkynyl, unsubstituted cycloalkyl, unsubstituted heterocyclyl, unsubstituted aryl, or unsubstituted heteroaryl;
R_{cc} is each independently selected from cyano or alkynyl; or,
any two of Rₐₐ, R_{bb}, R_{dd} and Rₑₑ are connected to form a cycloalkyl, heterocyclyl, aryl or heteroaryl, wherein the cycloalkyl, heterocyclyl, aryl and heteroaryl are optionally further substituted by one or more substituents selected from hydrogen, deuterium, unsubstituted alkyl, unsubstituted deuterated alkyl, unsubstituted haloalkyl, unsubstituted alkoxy, unsubstituted haloalkoxy, halogen, cyano, nitro, hydroxyl, amino, oxo, imine, unsubstituted alkenyl, unsubstituted alkynyl, unsubstituted cycloalkyl, unsubstituted heterocyclyl, unsubstituted aryl, or unsubstituted heteroaryl;
m is 0, 1 or 2;
n1 is 0, 1, or 2; and
n2 is 0, 1, or 2.

2. The compound as defined in claim 1, the stereoisomer thereof or the pharmaceutically acceptable salt thereof, being a compound of formula (XIII): 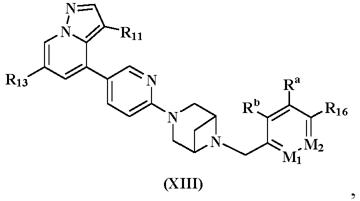 wherein:
R₁₁ is selected from hydrogen, deuterium, halogen, amino, nitro, hydroxyl, cyano, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₂₋₆ alkenyl or C₂₋₆ alkynyl;
R₁₃ is selected from C₂₋₆ alkenyl, C₂₋₆ alkynyl, -O(CH₂)ₙ₁(CRₐₐR_{bb})ₘR_{cc};
Rₐₐ, and R_{bb} are each independently selected from hydrogen, deuterium, halogen, amino, nitro, hydroxyl, cyano, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₂₋₆ alkenyl or C₂₋₆ alkynyl;
R_{cc} are selected from cyano, or C₂₋₆ alkynyl;
M₁ is -CH-;
M₂ is -N-;
R₁₆ is selected from hydrogen, deuterium, halogen, amino, nitro, hydroxyl, cyano, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₂₋₆ alkenyl or C₂₋₆ alkynyl;
R^{a} is independently selected from hydrogen, deuterium, halogen, amino, nitro, hydroxyl, cyano, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₂₋₆ alkenyl or C₂₋₆ alkynyl;
R^{b} is hydrogen;
n1 is an integer of 1, or 2;
m is an integer of 1, or 2;
preferably,
R₁₁ is selected from hydrogen, deuterium, halogen, amino, nitro, hydroxyl, cyano or C₁₋₃ alkyl;
R₁₃ is selected from C₂₋₄ alkenyl, C₂₋₄ alkynyl, or -OCH₂CRₐₐR_{bb}R_{cc};
Rₐₐ, and R_{bb} are each independently selected from hydrogen, deuterium, halogen, amino, nitro, hydroxyl, cyano, C₁₋₃ alkyl, C₁₋₃ deuterated alkyl, C₁₋₃ haloalkyl, C₁₋₃ alkoxy, C₁₋₃ deuterated alkoxy, C₁₋₃ haloalkoxy, C₂₋₄ alkenyl or C₂₋₄ alkynyl;
R_{cc} are cyano or C₂₋₄ alkynyl;
M₁ is -CH-, M₂ is -N-;
R₁₆ is selected from hydrogen, deuterium, halogen, amino, nitro, hydroxyl, cyano, C₁₋₃ alkyl, C₁₋₃ deuterated alkyl, C₁₋₃ haloalkyl, C₁₋₃ alkoxy, C₁₋₃ deuterated alkoxy or C₁₋₃ haloalkoxy;
R^{a} is independently selected from hydrogen, deuterium or halogen;
R^{b} is hydrogen.

3. A compound represented by general formula (IX-B), a stereoisomer thereof or a pharmaceutically acceptable salt thereof: 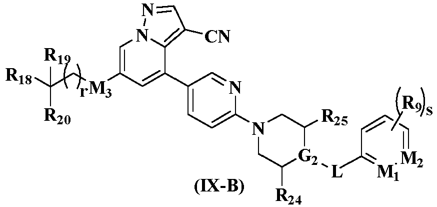 wherein:
M₃ is selected from bond, -O-, -S-, -NH- or -NCH₃-;
R₁₈ and R₁₉ are each independently selected from hydrogen, deuterium, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, halogen, amino, nitro, hydroxyl, cyano, C₂₋₆ alkenyl, C₂₋₆ alkynyl, C₃₋₈ cycloalkyl, 3-12 membered heterocyclyl, C₆₋₁₀ aryl or 5-12 membered heteroaryl, preferably hydrogen or methyl;
or, R₁₈ and R₁₉ together with the carbon atoms they are attached to form a C₃₋₈ cycloalkyl or a 3-12 membered heterocyclyl, preferably C₃₋₆ cycloalkyl or 3-7 membered heterocyclyl comprising 1-2 oxygen atoms, nitrogen atoms or sulfur atoms, more preferably cyclopropyl, cyclobutyl, cyclopentyl, oxetanyl, azetidinyl, bicyclo[1,1,1]pentane or 1- imino-1-oxothiopyran, wherein the C₃₋₈ cycloalkyl or 3-12 membered heterocyclyl is optionally further substituted by one or more substituents selected from hydrogen, C₁₋₆ alkyl, hydroxyl, cyano or C₁₋₆ hydroxyalkyl;
R₂₀ is selected from cyano, or C₂₋₆ alkynyl; preferably cyano;
R₂₄ and R₂₅ are each independently selected from hydrogen, deuterium, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, halogen, amino, nitro, hydroxyl, cyano, C₂₋₆ alkenyl, C₂₋₆ alkynyl, C₃₋₈ cycloalkyl, 3-12 membered heterocyclyl, C₆₋₁₀ aryl, 5-12 membered heteroaryl or -(CH₂)ₙ₁ORₐₐ, preferably hydrogen or methyl;
or, R₂₄ and R₂₅ together with the carbon atoms they are attached to and G₂ form a C₃₋₈ cycloalkyl or 3-12 membered heterocyclyl, preferably azetidinyl;
L is selected from bond, -(CH₂)ₙ₁CRₐₐR_{bb}-, -(CH₂)ₙ₁NRₐₐC(O)(CH₂)ₙ₂-, - (CH₂)ₙ₁C(O)(CH₂)ₙ₂(CRₐₐR_{bb})ₘ-, -(CH₂)ₙ₁C(O)(CRₐₐR_{bb})ₘ(CH₂)ₙ₂-, -(CH₂)ₙ₁C(O)NR_{cc}(CRₐₐR_{bb})ₙ₂-, - (CH₂)ₙ₁(O)(CH₂)ₙ₂- or -(CH₂)ₙ₁NRₐₐ(CH₂)ₙ₂-, preferably -CH₂-, -CD₂-, -O- or -NHC(O)-;
G₂ is selected from N or CRₐₐ, preferably N, CH or CCH₃;
M₁ is selected from N or CRₐₐ, preferably N, CH or CCH₃;
M₂ is selected from N or CRₐₐ, preferably N or CH;
R₉ is selected from hydrogen, deuterium, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, halogen, amino, nitro, hydroxyl, cyano, C₂₋₆ alkenyl, C₂₋₆ alkynyl, C₃₋₈ cycloalkyl, 3-12 membered heterocyclyl, C₆₋₁₀ aryl, 5-12 membered heteroaryl or -(CH₂)ₙ₁ORₐₐ; preferably hydrogen, halogen, C₁₋₆ alkoxy, C₁₋₆ deuterated alkoxy, or -ORₐₐ; most preferably hydrogen, fluorine, chlorine, methyl, methoxy, deuterated methoxy or cyclopropoxy;
Rₐₐ, R_{bb} and R_{cc} are each independently selected from hydrogen, deuterium, cyano, amino, C₁₋₆ alkyl, C₁₋₆ alkoxy, C₁₋₆ hydroxyalkyl, hydroxyl, C₃₋₈ cycloalkyl, 3-12 membered heterocyclyl or C₆₋₁₄ aryl, wherein the C₁₋₆ alkyl, C₁₋₆ alkoxy, C₁₋₆ hydroxyalkyl, hydroxyl, C₃₋₈ cycloalkyl, 3-12 membered heterocylcyl and C₆₋₁₄ aryl are optionally further substituted by one or more substituents selected from hydrogen, halogen, cyano, hydroxyl, oxo, imino, C₁₋₆ alkyl or C₁₋₆ hydroxyalkyl;
or, any two of Rₐₐ, R_{bb} and R_{cc} are optionally connected to form a C₃₋₈ cycloalkyl or a 3-12 membered heterocyclyl, wherein the C₃₋₈ cycloalkyl and 3-12 membered heterocyclyl are optionally further substituted by one or more substituents selected from hydrogen, amino, halogen, cyano, hydroxyl, oxo, imino, C₁₋₆ alkyl or C₁₋₆ hydroxyalkyl;
s is 0, 1 , 2 or 3;
m is 0, 1 or 2;
n1 is 0, 1, or 2; and
n2 is 0, 1, or 2;
r is 0, 1 or 2;
or, 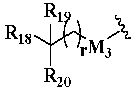 is selected from 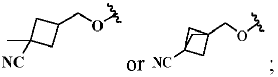 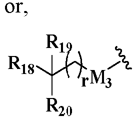 is selected from 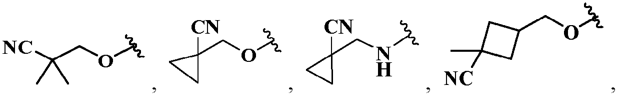 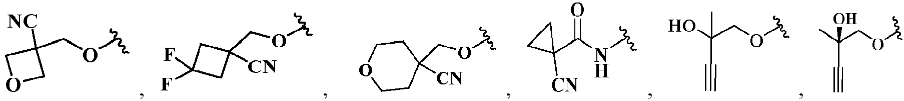
or

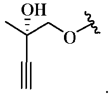

4. The compound as defined in claim 3, the stereoisomer thereof or the pharmaceutically acceptable salt thereof, being a compound of formula (X): 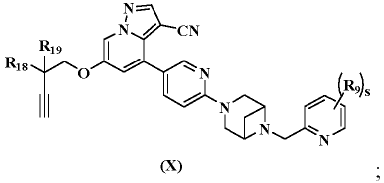
wherein, R₁₈ and R₁₉ are each independently selected from hydrogen, deuterium, C₁₋₃ alkyl, C₁₋₃ deuterated alkyl, C₁₋₃ haloalkyl, C₁₋₃ alkoxy, C₁₋₃ deuterated alkoxy, C₁₋₃ haloalkoxy, halogen, amino, nitro, hydroxyl, cyano, C₂₋₄ alkenyl, C₂₋₄ alkynyl, C₃₋₆ cycloalkyl, 3-6 membered heterocyclyl, C₆₋₁₀ aryl or 5-6 membered heteroaryl, preferably hydroxyl or methyl;
R₉ is selected from hydrogen, deuterium, C₁₋₃ alkyl, C₁₋₃ deuterated alkyl, C₁₋₃ haloalkyl, C₁₋₃ alkoxy, C₁₋₃ deuterated alkoxy, C₁₋₃ haloalkoxy, halogen, amino, nitro, hydroxyl, cyano, C₂₋₄ alkenyl or C₂₋₄ alkynyl;
s is 0, 1, 2 or 3.

5. A compound represented by general formula (IX-C), a stereoisomer thereof or a pharmaceutically acceptable salt thereof: 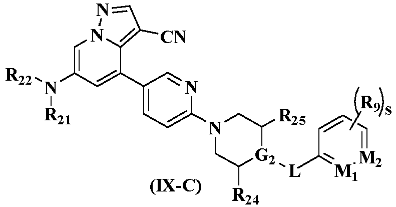 wherein:
R₂₁ and R₂₂ are each independently selected from hydrogen, deuterium, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, halogen, amino, nitro, hydroxyl, cyano, C₂₋₆ alkenyl, C₂₋₆ alkynyl, C₃₋₈ cycloalkyl, 3-12 membered heterocyclyl, C₆₋₁₀ aryl, 5-12 membered heteroaryl, -(CH₂)ₙ₁C(O)Rₐₐ or -(CH₂)ₙ₁Rₐₐ; or
R₂₁ and R₂₂ together with the nitrogen atoms they are attached to form a azetidinyl, wherein the azetidinyl is optionally further substituted by one or more substituents selected from hydrogen, amino, halogen, cyano, hydroxyl, oxo, C₁₋₆ alkyl or C₁₋₆ hydroxyalkyl;
R₂₄ and R₂₅ are each independently selected from hydrogen, deuterium, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, halogen, amino, nitro, hydroxyl, cyano, C₂₋₆ alkenyl, C₂₋₆ alkynyl, C₃₋₈ cycloalkyl, 3-12 membered heterocyclyl, C₆₋₁₀ aryl, 5-12 membered heteroaryl or -(CH₂)ₙ₁ORₐₐ, preferably hydrogen or methyl;
or, R₂₄ and R₂₅ together with the carbon atoms they are attached to and G₂ form a C₃₋₈ cycloalkyl or 3-12 membered heterocyclyl, preferably azetidinyl;
L is selected from bond, -(CH₂)ₙ₁CRₐₐR_{bb}-, -(CH₂)ₙ₁NRₐₐC(O)(CH₂)ₙ₂-, - (CH₂)ₙ₁C(O)(CH₂)ₙ₂(CRₐₐR_{bb})ₘ-, -(CH₂)ₙ₁C(O)(CRₐₐR_{bb})ₘ(CH₂)ₙ₂-, -(CH₂)ₙ₁C(O)NR_{cc}(CRₐₐR_{bb})ₙ₂-, - (CH₂)ₙ₁(O)(CH₂)ₙ₂- or -(CH₂)ₙ₁NRₐₐ(CH₂)ₙ₂-, preferably -CH₂-, -CD₂-, -O- or -NHC(O)-;
G₂ is selected from N or CRₐₐ, preferably N, CH or CCH₃;
M₁ is selected from N or CRₐₐ, preferably N, CH or CCH₃;
M₂ is selected from N or CRₐₐ, preferably N or CH;
R₉ is selected from hydrogen, deuterium, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, halogen, amino, nitro, hydroxyl, cyano, C₂₋₆ alkenyl, C₂₋₆ alkynyl, C₃₋₈ cycloalkyl, 3-12 membered heterocyclyl, C₆₋₁₀ aryl, 5-12 membered heteroaryl or -(CH₂)ₙ₁ORₐₐ; preferably hydrogen, halogen, C₁₋₆ alkoxy, C₁₋₆ deuterated alkoxy, or -ORₐₐ; most preferably hydrogen, fluorine, chlorine, methyl, methoxy, deuterated methoxy or cyclopropoxy;
Rₐₐ, R_{bb} and R_{cc} are each independently selected from hydrogen, deuterium, cyano, amino, C₁₋₆ alkyl, C₁₋₆ alkoxy, C₁₋₆ hydroxyalkyl, hydroxyl, C₃₋₈ cycloalkyl, 3-12 membered heterocyclyl or C₆₋₁₄ aryl, wherein the C₁₋₆ alkyl, C₁₋₆ alkoxy, C₁₋₆ hydroxyalkyl, hydroxyl, C₃₋₈ cycloalkyl, 3-12 membered heterocylcyl and C₆₋₁₄ aryl are optionally further substituted by one or more substituents selected from hydrogen, halogen, cyano, hydroxyl, oxo, imino, C₁₋₆ alkyl or C₁₋₆ hydroxyalkyl;
or, any two of Rₐₐ, R_{bb} and R_{cc} are optionally connected to form a C₃₋₈ cycloalkyl or a 3-12 membered heterocyclyl, wherein the C₃₋₈ cycloalkyl and 3-12 membered heterocyclyl are optionally further substituted by one or more substituents selected from hydrogen, amino, halogen, cyano, hydroxyl, oxo, imino, C₁₋₆ alkyl or C₁₋₆ hydroxyalkyl;
s is 0, 1 , 2 or 3;
m is 0, 1 or 2;
n1 is 0, 1, or 2; and
n2 is 0, 1, or 2.

6. The compound as defined in claim 5, the stereoisomer thereof or the pharmaceutically acceptable salt thereof,
R₂₁ and R₂₂ are each independently selected from hydrogen, deuterium, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, halogen, amino, nitro, hydroxyl, cyano, C₂₋₆ alkenyl, C₂₋₆ alkynyl, C₃₋₈ cycloalkyl, 3-12 membered heterocyclyl, C₆₋₁₀ aryl, 5-12 membered heteroaryl, -(CH₂)ₙ₁C(O)Rₐₐ or -(CH₂)ₙ₁Rₐₐ; or
R₂₁ and R₂₂ together with the nitrogen atoms they are attached to form a azetidinyl, wherein the azetidinyl, is optionally further substituted by one or more substituents selected from hydrogen, amino, halogen, cyano, hydroxyl, oxo, C₁₋₆ alkyl or C₁₋₆ hydroxyalkyl;
R₂₄ and R₂₅ are each independently selected from hydrogen or methyl;
or, R₂₄ and R₂₅ together with the carbon atoms they are attached to and G₂ form a azetidinyl;
L is selected from -CH₂-, -CD₂-, -O- or -NHC(O)-;
G₂ is selected from N, CH or CCH₃;
M₁ is selected from N, CH or CCH₃;
M₂ is selected from N or CH;
R₉ is selected from hydrogen, fluorine, chlorine, methyl, methoxy, deuterated methoxy or cyclopropoxy;
Rₐₐ is each independently selected from hydrogen, deuterium, cyano, amino, C₁₋₆ alkyl, C₁₋₆ alkoxy, C₁₋₆ hydroxyalkyl, hydroxyl, C₃₋₈ cycloalkyl, 3-12 membered heterocyclyl or C₆₋₁₄ aryl, wherein the C₁₋₆ alkyl, C₁₋₆ alkoxy, C₁₋₆ hydroxyalkyl, hydroxyl, C₃₋₈ cycloalkyl, 3-12 membered heterocylcyl and C₆₋₁₄ aryl are optionally further substituted by one or more substituents selected from hydrogen, halogen, cyano, hydroxyl, oxo, imino, C₁₋₆ alkyl or C₁₋₆ hydroxyalkyl;
s is 0, 1 , 2 2 or 3; and
n1 is 0, 1, or 2.

7. A compound represented by general formula (IX-C), a stereoisomer thereof or a pharmaceutically acceptable salt thereof: 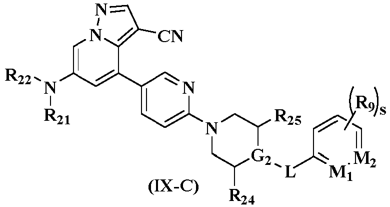 wherein:
R₂₁ and R₂₂ are each independently selected from hydrogen, deuterium, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, halogen, amino, nitro, hydroxyl, cyano, C₂₋₆ alkenyl, C₂₋₆ alkynyl, C₃₋₈ cycloalkyl, 3-12 membered heterocyclyl, C₆₋₁₀ aryl, 5-12 membered heteroaryl, -(CH₂)ₙ₁C(O)Rₐₐ or -(CH₂)ₙ₁Rₐₐ; or
R₂₁ and R₂₂ together with the nitrogen atoms they are attached to form azetidinyl, or 2-azaspiro[3.3]heptane, wherein the azetidinyl or 2-azaspiro[3.3]heptane are optionally further substituted by one or more substituents selected from hydrogen, C₁₋₆ alkyl, hydroxyl or hydroxyalkyl;
R₂₄ and R₂₅ are each independently selected from hydrogen, deuterium, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, halogen, amino, nitro, hydroxyl, cyano, C₂₋₆ alkenyl, C₂₋₆ alkynyl, C₃₋₈ cycloalkyl, 3-12 membered heterocyclyl, C₆₋₁₀ aryl, 5-12 membered heteroaryl or -(CH₂)ₙ₁ORₐₐ, preferably hydrogen or methyl;
or, R₂₄ and R₂₅ together with the carbon atoms they are attached to and G₂ form a C₃₋₈ cycloalkyl or 3-12 membered heterocyclyl, preferably azetidinyl;
L is selected from bond, -(CH₂)ₙ₁CRₐₐR_{bb}-, -(CH₂)ₙ₁NRₐₐC(O)(CH₂)ₙ₂-, - (CH₂)ₙ₁C(O)(CH₂)ₙ₂(CRₐₐR_{bb})ₘ-, -(CH₂)ₙ₁C(O)(CRₐₐR_{bb})ₘ(CH₂)ₙ₂-, -(CH₂)ₙ₁C(O)NR_{cc}(CRₐₐR_{bb})ₙ₂-, - (CH₂)ₙ₁(O)(CH₂)ₙ₂- or -(CH₂)ₙ₁NRₐₐ(CH₂)ₙ₂-, preferably -CH₂-, -CD₂-, -O- or -NHC(O)-;
G₂ is selected from N or CRₐₐ, preferably N, CH or CCH₃;
M₁ is selected from N or CRₐₐ, preferably N, CH or CCH₃;
M₂ is selected from N or CRₐₐ, preferably N or CH;
R₉ is selected from hydrogen, deuterium, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, halogen, amino, nitro, hydroxyl, cyano, C₂₋₆ alkenyl, C₂₋₆ alkynyl, C₃₋₈ cycloalkyl, 3-12 membered heterocyclyl, C₆₋₁₀ aryl, 5-12 membered heteroaryl or -(CH₂)ₙ₁ORₐₐ; preferably hydrogen, halogen, C₁₋₆ alkoxy, C₁₋₆ deuterated alkoxy, or -ORₐₐ; most preferably hydrogen, fluorine, chlorine, methyl, methoxy, deuterated methoxy or cyclopropoxy;
Rₐₐ, R_{bb} and R_{cc} are each independently selected from hydrogen, deuterium, cyano, amino, C₁₋₆ alkyl, C₁₋₆ alkoxy, C₁₋₆ hydroxyalkyl, hydroxyl, C₃₋₈ cycloalkyl, 3-12 membered heterocyclyl or C₆₋₁₄ aryl, wherein the C₁₋₆ alkyl, C₁₋₆ alkoxy, C₁₋₆ hydroxyalkyl, hydroxyl, C₃₋₈ cycloalkyl, 3-12 membered heterocylcyl and C₆₋₁₄ aryl are optionally further substituted by one or more substituents selected from hydrogen, halogen, cyano, hydroxyl, oxo, imino, C₁₋₆ alkyl or C₁₋₆ hydroxyalkyl;
or, any two of Rₐₐ, R_{bb} and R_{cc} are optionally connected to form a C₃₋₈ cycloalkyl or a 3-12 membered heterocyclyl, wherein the C₃₋₈ cycloalkyl and 3-12 membered heterocyclyl are optionally further substituted by one or more substituents selected from hydrogen, amino, halogen, cyano, hydroxyl, oxo, imino, C₁₋₆ alkyl or C₁₋₆ hydroxyalkyl;
s is 0, 1 , 2 or 3;
m is 0, 1 or 2;
n1 is 0, 1, or 2; and
n2 is 0, 1, or 2;
or, R₂₁ and R₂₂ together with the carbon atoms they are attached to form a azetidinyl, or 2-azaspiro[3.3]heptane, wherein the azetidinyl, or 2-azaspiro[3.3]heptane is optionally further substituted by one or more substituents selected from of hydrogen, C₁₋₃ alkyl, cyano, hydroxyl or hydroxyalkyl.

8. A compound represented by general formula (IX-C), a stereoisomer thereof or a pharmaceutically acceptable salt thereof: 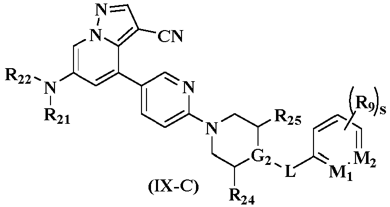 wherein: 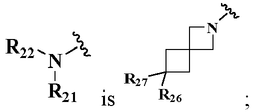
R₂₆ and R₂₇ are each independently selected from hydrogen, deuterium, halogen, amino, nitro, hydroxyl, cyano, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₁₋₆ hydroxyalkyl, C₂₋₆ alkenyl, C₂₋₆ alkynyl, C₃₋₈ cycloalkyl, 3-12 membered heterocyclyl, C₆₋₁₀ aryl or 5-12 membered heteroaryl; preferably hydrogen, hydroxyl, cyano, C₁₋₃ alkyl or C₁₋₃ hydroxyalkyl; more preferably hydrogen, hydroxyl, cyano methyl or hydroxyisopropyl;
R₂₄ and R₂₅ are each independently selected from hydrogen, deuterium, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, halogen, amino, nitro, hydroxyl, cyano, C₂₋₆ alkenyl, C₂₋₆ alkynyl, C₃₋₈ cycloalkyl, 3-12 membered heterocyclyl, C₆₋₁₀ aryl, 5-12 membered heteroaryl or -(CH₂)ₙ₁ORₐₐ, preferably hydrogen or methyl;
or, R₂₄ and R₂₅ together with the carbon atoms they are attached to and G₂ form a C₃₋₈ cycloalkyl or 3-12 membered heterocyclyl, preferably azetidinyl;
L is selected from bond, -(CH₂)ₙ₁CRₐₐR_{bb}-, -(CH₂)ₙ₁NRₐₐC(O)(CH₂)ₙ₂-, - (CH₂)ₙ₁C(O)(CH₂)ₙ₂(CRₐₐR_{bb})ₘ-, -(CH₂)ₙ₁C(O)(CRₐₐR_{bb})ₘ(CH₂)ₙ₂-, -(CH₂)ₙ₁C(O)NR_{cc}(CRₐₐR_{bb})ₙ₂-, - (CH₂)ₙ₁(O)(CH₂)ₙ₂- or -(CH₂)ₙ₁NRₐₐ(CH₂)ₙ₂-, preferably -CH₂-, -CD₂-, -O- or -NHC(O)-;
G₂ is selected from N or CRₐₐ, preferably N, CH or CCH₃;
M₁ is selected from N or CRₐₐ, preferably N, CH or CCH₃;
M₂ is selected from N or CRₐₐ, preferably N or CH;
R₉ is selected from hydrogen, deuterium, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, halogen, amino, nitro, hydroxyl, cyano, C₂₋₆ alkenyl, C₂₋₆ alkynyl, C₃₋₈ cycloalkyl, 3-12 membered heterocyclyl, C₆₋₁₀ aryl, 5-12 membered heteroaryl or -(CH₂)ₙ₁ORₐₐ; preferably hydrogen, halogen, C₁₋₆ alkoxy, C₁₋₆ deuterated alkoxy, or -ORₐₐ; most preferably hydrogen, fluorine, chlorine, methyl, methoxy, deuterated methoxy or cyclopropoxy;
Rₐₐ, R_{bb} and R_{cc} are each independently selected from hydrogen, deuterium, cyano, amino, C₁₋₆ alkyl, C₁₋₆ alkoxy, C₁₋₆ hydroxyalkyl, hydroxyl, C₃₋₈ cycloalkyl, 3-12 membered heterocyclyl or C₆₋₁₄ aryl, wherein the C₁₋₆ alkyl, C₁₋₆ alkoxy, C₁₋₆ hydroxyalkyl, hydroxyl, C₃₋₈ cycloalkyl, 3-12 membered heterocylcyl and C₆₋₁₄ aryl are optionally further substituted by one or more substituents selected from hydrogen, halogen, cyano, hydroxyl, oxo, imino, C₁₋₆ alkyl or C₁₋₆ hydroxyalkyl;
or, any two of Rₐₐ, R_{bb} and R_{cc} are optionally connected to form a C₃₋₈ cycloalkyl or a 3-12 membered heterocyclyl, wherein the C₃₋₈ cycloalkyl and 3-12 membered heterocyclyl are optionally further substituted by one or more substituents selected from hydrogen, amino, halogen, cyano, hydroxyl, oxo, imino, C₁₋₆ alkyl or C₁₋₆ hydroxyalkyl;
s is 0, 1 , 2 or 3;
m is 0, 1 or 2;
n1 is 0, 1, or 2; and
n2 is 0, 1, or 2.

9. A compound represented by general (IX-A), a stereoisomer thereof or a pharmaceutically acceptable salt thereof: 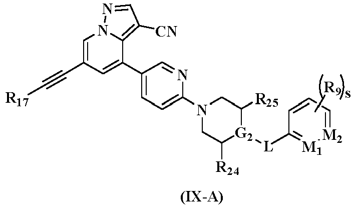 wherein:
L is selected from bond, -(CH₂)ₙ₁CRₐₐR_{bb}-, -(CH₂)ₙ₁NRₐₐC(O)(CH₂)ₙ₂-, - (CH₂)ₙ₁C(O)(CH₂)ₙ₂(CRₐₐR_{bb})ₘ-, -(CH₂)ₙ₁C(O)(CRₐₐR_{bb})ₘ(CH₂)ₙ₂-, -(CH₂)ₙ₁C(O)NR_{cc}(CRₐₐR_{bb})ₙ₂-, - (CH₂)ₙ₁(O)(CH₂)ₙ₂- or -(CH₂)ₙ₁NRₐₐ(CH₂)ₙ₂-, preferably -CH₂-, -CD₂-, -O- or -NHC(O)-;
G₂ is selected from N or CRₐₐ, preferably N, CH or CCH₃;
M₁ is selected from N or CRₐₐ, preferably N, CH or CCH₃;
M₂ is selected from N or CRₐₐ, preferably N or CH;
R₉ is selected from hydrogen, deuterium, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, halogen, amino, nitro, hydroxyl, cyano, C₂₋₆ alkenyl, C₂₋₆ alkynyl, C₃₋₈ cycloalkyl, 3-12 membered heterocyclyl, C₆₋₁₀ aryl, 5-12 membered heteroaryl or -(CH₂)ₙ₁ORₐₐ; preferably hydrogen, halogen, C₁₋₆ alkoxy, C₁₋₆ deuterated alkoxy, or -ORₐₐ; most preferably hydrogen, fluorine, chlorine, methyl, methoxy, deuterated methoxy or cyclopropoxy;
R₁₇ is selected from C₁₋₆ alkyl, C₁₋₆ haloalkyl, C₁₋₆ alkoxy, C₂₋₆ alkynyl, 3-12 membered heterocyclyl, 5-12 membered heteroaryl or -(CH₂)ₙ₁(CRₐₐR_{bb}) R_{cc}, wherein the C₁₋₆ alkyl, C₁₋₆ haloalkyl, C₁₋₆ alkoxy, C₂₋₆ alkynyl, 3-12 membered heterocyclyl and 5-12 membered heteroaryl are optionally further substituted by one or more substituents selected from hydrogen, C₁₋₆ haloalkyl, hydroxyl, cyano, oxo, thio, amino, imino, C₁₋₆ alkoxy, C₁₋₆ hydroxyalkyl, C₁₋₆ cyanoalkyl, C₃₋₈ cycloalkyl or 3-12 membered heterocyclyl;
R₂₄ and R₂₅ are each independently selected from hydrogen, deuterium, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, halogen, amino, nitro, hydroxyl, cyano, C₂₋₆ alkenyl, C₂₋₆ alkynyl, C₃₋₈ cycloalkyl, 3-12 membered heterocyclyl, C₆₋₁₀ aryl, 5-12 membered heteroaryl or -(CH₂)ₙ₁ORₐₐ, preferably hydrogen or methyl;
or, R₂₄ and R₂₅ together with the carbon atoms they are attached to and G2 form a C₃₋₈ cycloalkyl or 3-12 membered heterocyclyl, preferably azetidinyl;
Rₐₐ, R_{bb} and R_{cc} are each independently selected from hydrogen, deuterium, cyano, amino, C₁₋₆ alkyl, C₁₋₆ alkoxy, C₁₋₆ hydroxyalkyl, hydroxyl, C₃₋₈ cycloalkyl, 3-12 membered heterocyclyl or C₆₋₁₄ aryl, wherein the C₁₋₆ alkyl, C₁₋₆ alkoxy, C₁₋₆ hydroxyalkyl, hydroxyl, C₃₋₈ cycloalkyl, 3-12 membered heterocylcyl and C₆₋₁₄ aryl are optionally further substituted by one or more substituents selected from hydrogen, halogen, cyano, hydroxyl, oxo, imino, C₁₋₆ alkyl or C₁₋₆ hydroxyalkyl;
or, any two of Rₐₐ, R_{bb} and R_{cc} are optionally connected to form a C₃₋₈ cycloalkyl or a 3-12 membered heterocyclyl, wherein the C₃₋₈ cycloalkyl and 3-12 membered heterocyclyl are optionally further substituted by one or more substituents selected from hydrogen, amino, halogen, cyano, hydroxyl, oxo, imino, C₁₋₆ alkyl or C₁₋₆ hydroxyalkyl;
n1 is 0, 1 or 2;
n2 is 0, 1 or 2;
m is 0, 1 or 2; and
s is 0, 1, 2 or 3.

10. The compound as defined in claim 1, the stereoisomer thereof or the pharmaceutically acceptable salt thereof, wherein,
L is selected from bond, -(CH₂)ₙ₁CRₐₐR_{bb}-, -(CH₂)ₙ₁NRₐₐC(O)(CH₂)ₙ₂-, - (CH₂)ₙ₁C(O)(CRₐₐR_{bb})ₘ(CH₂)ₙ₂-, -(CH₂)ₙ₁C(O)(CH₂)ₘ(CRₐₐR_{bb})ₙ₂-, -(CH₂)ₙ₁C(O)NR_{cc}(CRₐₐR_{bb})ₙ₂-, - (CH₂)ₙ₁(O)(CH₂)ₙ₂- or -(CH₂)ₙ₁NRₐₐ(CH₂)ₙ₂-;
R₁₁ is selected form cyano, -(CH₂)ₙ₁P(O)RₐₐR_{bb} or -(CH₂)ₙ₁C(O)NRₐₐR_{bb};
R₁₂ is selected from hydrogen, C₁₋₆ alkyl or halogen;
R₁₃ is selected from C₂₋₆ alkynyl, -(C≡C)ₙ₁(CRₐₐR_{bb})ₘR_{cc}, -(C≡C)ₙ₁(CRₐₐR_{bb})ₘR_{cc}, or - (CH₂)ₙ₁O(CH₂)ₙ₂(CRₐₐR_{bb})ₘR_{cc};
R₁₄ is selected from hydrogen or halogen;
R₁₅ is selected from hydrogen or halogen;
R₁₆ is selected from hydrogen, alkoxy or -ORₐₐ;
Rₐₐ, and R_{bb} are each independently selected from hydrogen, deuterium, cyano, amino, C₁₋₆ alkyl, C₁₋₆ alkoxy, hydroxyl, C₃₋₈ cycloalkyl, 3-12 membered heterocyclyl or C₆₋₁₄ aryl, wherein the C₁₋₆ alkyl, C₁₋₆ alkoxy, C₃₋₈ cycloalkyl, 3-12 membered heterocylcyl and C₆₋₁₄ aryl are optionally further substituted by one or more substituents selected from hydrogen, halogen, cyano, hydroxyl, oxo, imino,or C₁₋₆ alkyl;
R_{cc} is selected from cyano;
or, any two of Rₐₐ and R_{bb} are optionally connected to form a C₃₋₈ cycloalkyl or a 3-12 membered heterocyclyl, wherein the C₃₋₈ cycloalkyl and 3-12 membered heterocyclyl are optionally further substituted by one or more substituents selected from hydrogen, amino, halogen, cyano, hydroxyl, oxo, imine, or C₁₋₆ alkyl.

11. A compound, a stereoisomer thereof or a pharmaceutically acceptable salt thereof, wherein, the specific structure of the compound is as follows:

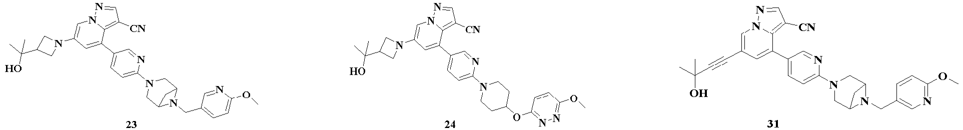

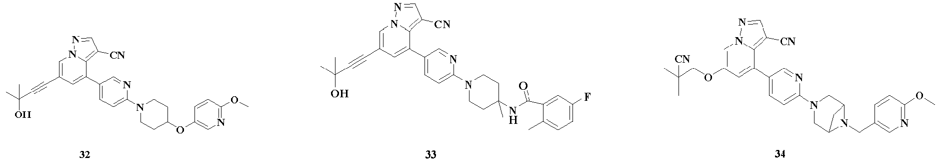

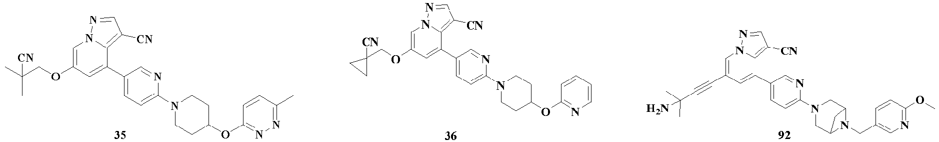

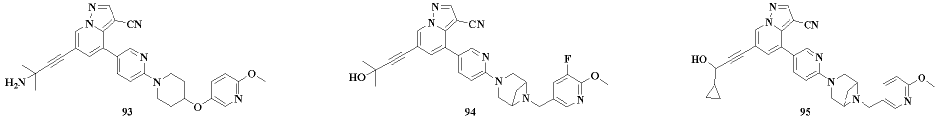

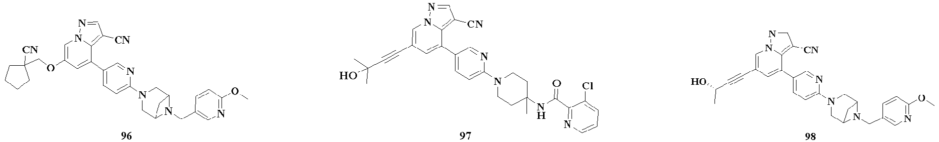

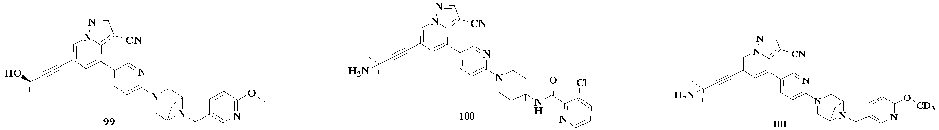

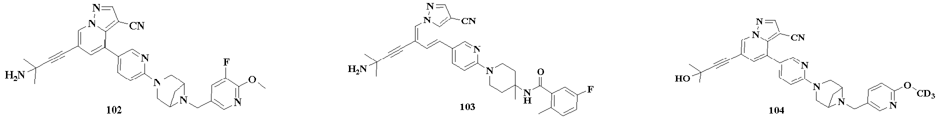

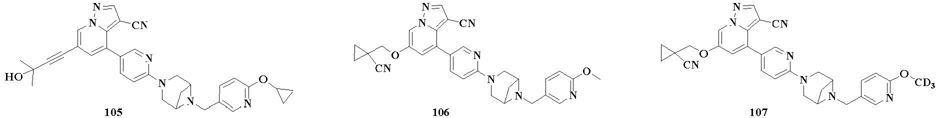

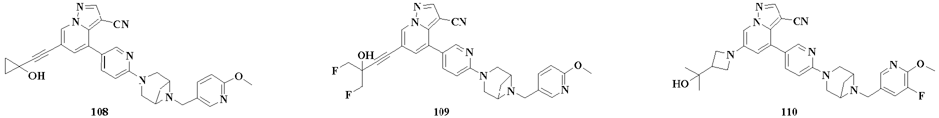

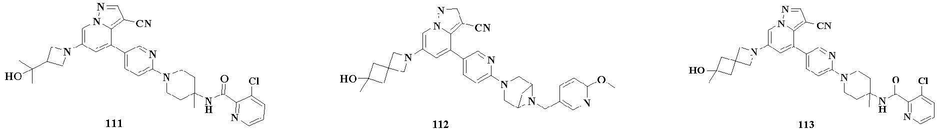

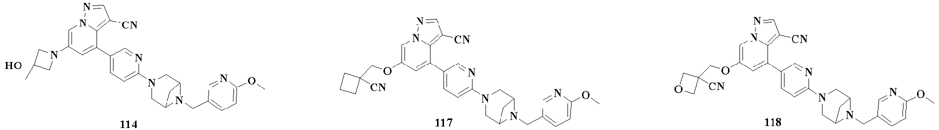

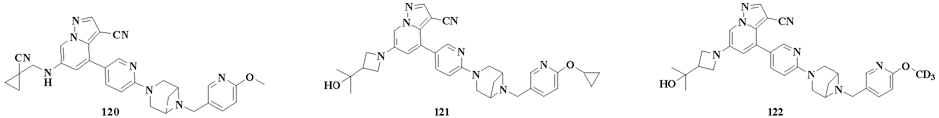

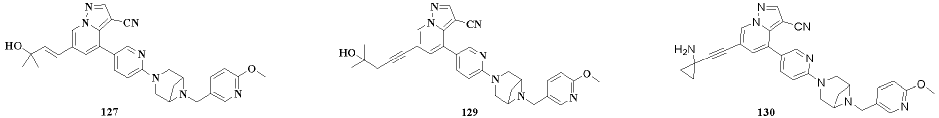

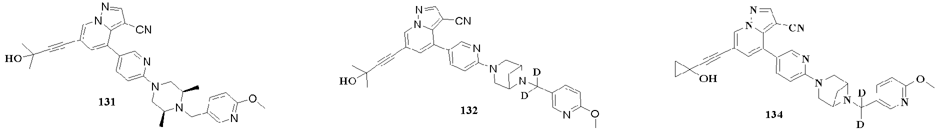

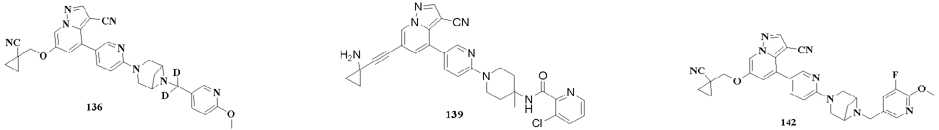

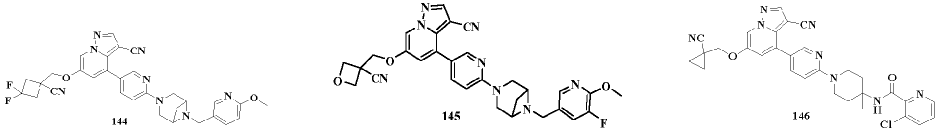

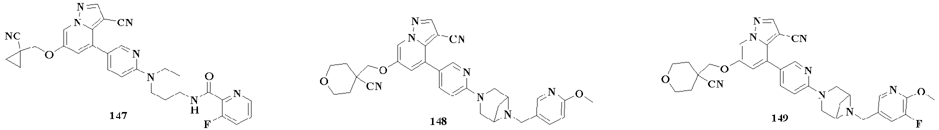

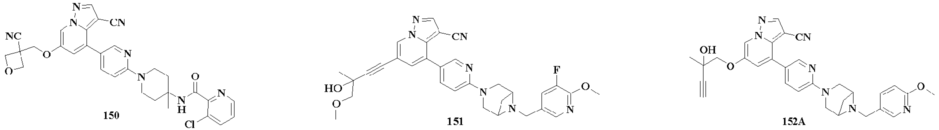

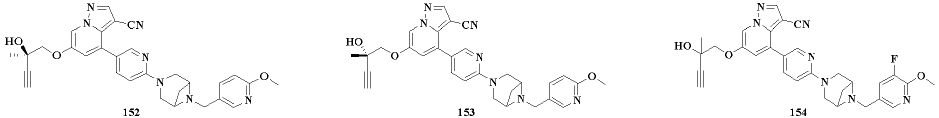

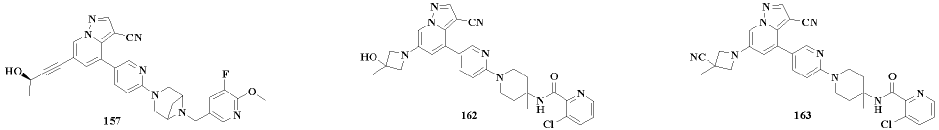

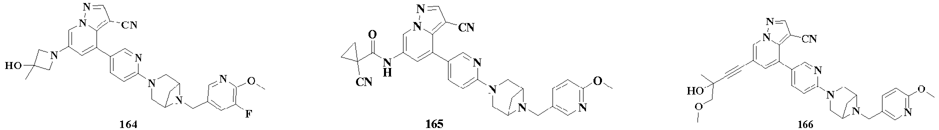

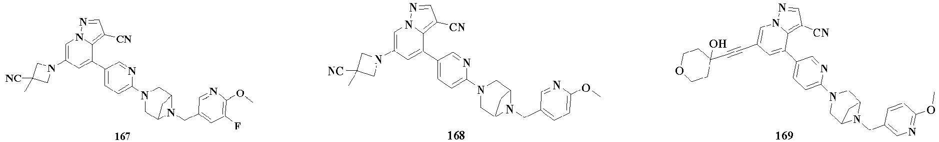

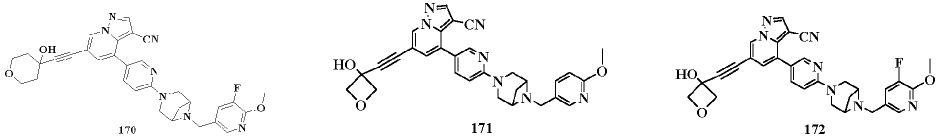

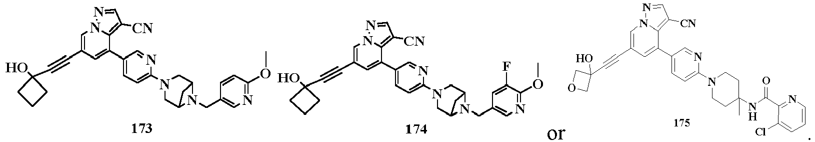

12. A method for preparing the compound represented by general formula (IX-A) as defined in claim 9, the stereoisomer thereof or the pharmaceutically acceptable salt thereof, wherein, comprising the following steps: 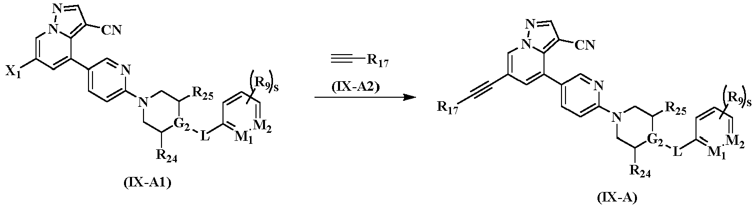
a coupling reaction is carried out with general formula (IX-A1) and general formula (IX-A2) to obtain the compound represented by general formula (IX-A) or the stereoisomer thereof and the pharmaceutically acceptable salt thereof;
wherein:
X₁ is selected from halogen; preferably fluorine, chlorine, bromine or iodine; more preferably bromine.

13. A method for preparing the compound represented by general formula (IX-B) as defined in claim 3, the stereoisomer thereof or the pharmaceutically acceptable salt thereof, wherein, comprising the following steps: 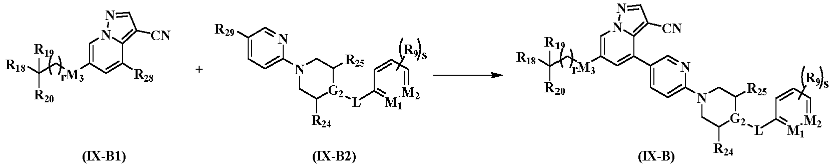
a reaction is carried out with general formula (IX-B1) and general formula (IX-B2) to obtain the compound represented by general formula (IX-B) or the stereoisomer thereof and the pharmaceutically acceptable salt thereof;
wherein:
R₂₈ is selected from halogen, boric acid or borate ester; preferably fluorine, chlorine, bromine, iodine, - B(OH)₂ or 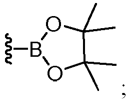
R₂₉ is selected from halogen, boric acid or borate ester; preferably fluorine, chlorine, bromine, iodine, - B(OH)₂ or 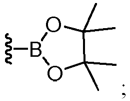
when R₂₈ is halogen, R₂₉ is selected from boric acid or borate ester;
when R₂₈ is selected from boric acid or borate ester, R₂₉ is halogen;
or,
wherein, comprising the following steps: 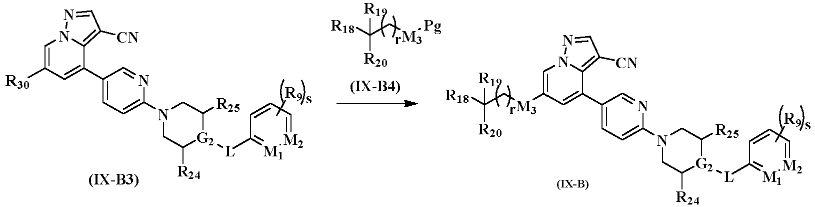
a reaction is carried out with general formula (IX-B3) and general formula (IX-B4) to obtain the compound represented by general formula (IX-B) or the stereoisomer thereof and the pharmaceutically acceptable salt thereof;
wherein:
R₃₀ is selected from halogen, hydroxyl; preferably fluorine, chlorine, bromine, iodine, or hydroxyl; more preferably bromine or hydroxyl;
Pg is selected from hydrogen, halogen or hydroxyl protecting group, and the halogen is preferably fluorine, chlorine, bromine or iodine;
when Pg is a hydroxyl protecting group, it is selected from methyl, *tert-*butyl*,* triphenyl, methyl sulfide methyl ether, 2-methoxyethoxymethyl ether, methoxymethyl ether, *p*-methoxybenzyl ether, pivaloyl, benzyl ether, methoxymethyl, trimethylsilyl, tetrahydrofuranyl, *tert*-butyldisilyl, acetyl, benzoyl or *p-*toluenesulfonyl; preferably*p*-toluenesulfonyl;
when M₃ is -O-, Pg is selected from hydrogen or hydroxyl protecting group.

14. A method for preparing the compound represented by general formula (IX-C) as defined in any one of claims 5 to 8, the stereoisomer thereof or the pharmaceutically acceptable salt thereof, wherein, comprising the following steps: 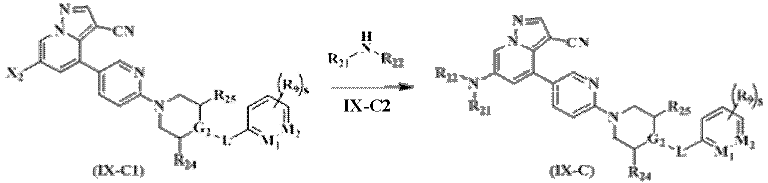
a reaction is carried out with general formula (IX-C1) and general formula (IX-C2) to obtain the compound represented by general formula (IX-C) or the stereoisomer thereof and the pharmaceutically acceptable salt thereof;
wherein:
X₂ is selected from halogen; preferably fluorine, chlorine, bromine or iodine; more preferably bromine.

15. A pharmaceutical composition comprising a therapeutically effective amount of the compound as defined in any one of claims 1-11, and the stereoisomer thereof or the pharmaceutically acceptable salt thereof, and one or more pharmaceutically acceptable carriers.

16. The compound represented as defined in any one of claims 1-11, the stereoisomer thereof or the pharmaceutically acceptable salt thereof, or the pharmaceutical composition as defined in claim 15 for use as medicaments for the treatment and/or prevention of non-small cell lung cancer, fibrosarcoma, pancreatic tumor, medullary thyroid carcinoma, thyroid papillary tumor, soft tissue sarcoma, highly solid tumor, breast tumor and colon tumor and other related diseases.

## Patentansprüche

1. Verbindung, dargestellt durch die allgemeine Formel (XI) oder (XI-A), ein Stereoisomer davon oder ein pharmazeutisch akzeptables Salz davon: 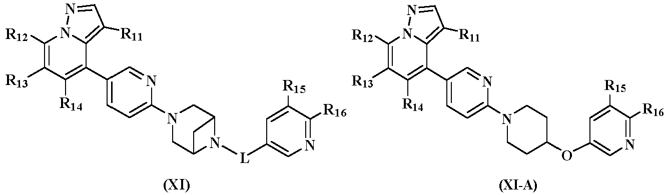 worin:
L ausgewählt ist aus einer Bindung, -(CH₂)ₙ₁CRₐₐR_{bb}-, - (CH₂)ₙ₁NRₐₐC(O)(CH₂)ₙ₂-, - (CH₂)ₙ₁C(O)(CH₂)ₙ₂(CRₐₐR_{bb})ₘ-, - (CH₂)ₙ₁C(O)(CRₐₐR_{bb})ₘ(CH₂)ₙ₂-, - (CH₂)ₙ₁C(O)NR_{cc}(CRₐₐR_{bb})ₙ₂-, - (CH₂)ₙ₁(O)(CH₂)ₙ₂- oder -(CH₂)ₙ₁NRₐₐ(CH₂)ₙ₂-;
R₁₁ ausgewählt ist aus Wasserstoff, Deuterium, Alkyl, deuteriertem Alkyl, Haloalkyl, Alkoxy, Haloalkoxy, Halogen, Amino, Nitro, Hydroxyl, Cyano, Alkenyl, Alkinyl, Cycloalkyl, Heterocyclyl, Aryl, Heteroaryl, -(CH₂)ₙ₁Rₐₐ, - (CH₂)ₙ₁ORₐₐ, - (CH₂)ₙ₁S(CH₂)ₙ₂Rₐₐ, - (CH₂)ₙ₁C(O) Rₐₐ, -(CH₂)ₙ₁C(O)ORₐₐ, -(CH₂)ₙ₁S(O)ₘRₐₐ, - (CH₂)ₙ₁S(O)(=NRₐₐ)(CH₂)ₙ₂R_{bb}, -(CH₂)ₙ₁NRₐₐR_{bb}, - (CH₂)ₙ₁P(O) RₐₐR_{bb}, -(CH₂)ₙ₁C(O)NRₐₐR_{bb}, -(CH₂)ₘNRₐₐC(O)R_{bb} oder -(CH₂)ₙ₁NRₐₐS(O)ₘR_{bb};
R₁₂ ist Wasserstoff, Deuterium, Alkyl, deuteriertes Alkyl, Haloalkyl, Alkoxy, Haloalkoxy, Halogen, Amino, Nitro, Hydroxyl, Cyano, Alkenyl, Alkinyl, Cycloalkyl, Heterocyclyl, Aryl oder Heteroaryl;
R₁₃ ausgewählt ist aus Alkenyl, Alkinyl, - (C≡C)ₙ₁(CRₐₐR_{bb})ₘR_{cc}, -(C=C)ₙ₁(CRₐₐR_{bb})ₘR_{cc}, - (CH₂)ₙ₁O(CH₂)ₙ₂(CRₐₐR_{bb})ₘR_{cc}, wobei das Alkenyl oder Alkinyl gegebenenfalls weiter substituiert ist durch einen oder mehrere Substituenten, ausgewählt aus Wasserstoff, Deuterium, unsubstituiertem Alkyl, unsubstituiertem deuteriertem Alkyl, unsubstituiertem Haloalkyl, unsubstituiertem Hydroxyalkyl, unsubstituiertem Cyanoalkyl, unsubstituiertes Alkoxy, unsubstituiertes Haloalkoxy, Halogen, unsubstituiertes Amino, Nitro, Hydroxyl, Cyano, Oxo, Thio, Imino, unsubstituiertes Alkenyl, unsubstituiertes Alkinyl, unsubstituiertes Cycloalkyl, unsubstituiertes Heterocyclyl, unsubstituiertes Aryl, unsubstituiertes Heteroaryl, - (CH₂)ₙ₁R_{dd}, -(CH₂)ₙ₁OR_{dd}, -(CH₂)ₙ₁S(CH₂)ₙ₂R_{dd}, -(CH₂)ₙ₁C(O)R_{dd}, -(CH₂)ₙ₁C(O)OR_{dd}, -(CH₂)n₁S(O)ₘR_{dd}, - (CH₂)ₙ₁S(O)(=NR_{dd})(CH₂)ₙ₂Rₑₑ, -(CH₂)ₙ₁NR_{dd}Rₑₑ, - (CH₂)ₙ₁P(O)R_{dd}Rₑₑ, -(CH₂)ₙ₁C(O)NR_{dd}Rₑₑ, -(CH₂)ₙ₁NR_{dd}C(O)Rₑₑ oder -(CH₂)ₙ₁NR_{dd}S(O)ₘRₑₑ;
R₁₄ Wasserstoff, Deuterium, Alkyl, deuteriertes Alkyl, Haloalkyl, Alkoxy, Haloalkoxy, Halogen, Amino, Nitro, Hydroxyl, Cyano, Alkenyl, Alkinyl, Cycloalkyl, Heterocyclyl, Aryl oder Heteroaryl ist;
R₁₅ und R₁₆ sind jeweils unabhängig voneinander ausgewählt aus Wasserstoff, Deuterium, Alkyl, deuteriertem Alkyl, Haloalkyl, Alkoxy, Haloalkoxy, Halogen, Amino, Nitro, Hydroxyl, Cyano, Alkenyl, Alkinyl, Cycloalkyl, Heterocyclyl, Aryl, Heteroaryl oder -(CH₂)ₙ₁ORₐₐ;
Rₐₐ, R_{bb}, R_{dd} und Rₑₑ jeweils unabhängig voneinander ausgewählt sind aus Wasserstoff, Deuterium, Alkyl, deuteriertem Alkyl, Haloalkyl, Alkoxy, Haloalkoxy, Halogen, Cyano, Nitro, Hydroxyl, Amino, Alkenyl, Alkinyl, Cycloalkyl, Heterocyclyl, Aryl oder Heteroaryl, wobei das Alkyl, deuterierte Alkyl, Haloalkyl, Alkoxy, Haloalkoxy, Alkenyl, Alkinyl, Cycloalkyl, Heterocyclyl, Aryl und Heteroaryl gegebenenfalls weiter substituiert sind durch einen oder mehrere Substituenten, ausgewählt aus Wasserstoff, Deuterium, unsubstituiertem Alkyl, unsubstituiertem deuteriertem Alkyl, unsubstituiertem Haloalkyl, unsubstituiertem Alkoxy, unsubstituiertem Haloalkoxy, Halogen, Cyano, Nitro, Hydroxyl, Amino, Oxo, Imino, unsubstituiertem Alkenyl, unsubstituiertem Alkinyl, unsubstituiertem Cycloalkyl, unsubstituiertem Heterocyclyl, unsubstituiertem Aryl oder unsubstituiertem Heteroaryl;
R_{cc} jeweils unabhängig voneinander aus Cyano oder Alkinyl ausgewählt ist; oder,
zwei beliebige von Rₐₐ, R_{bb}, R_{dd} und Rₑₑ verbunden sind, um ein Cycloalkyl, Heterocyclyl, Aryl oder Heteroaryl zu bilden, wobei das Cycloalkyl, Heterocyclyl, Aryl und Heteroaryl gegebenenfalls ferner durch einen oder mehrere Substituenten substituiert sind, ausgewählt aus Wasserstoff, Deuterium, unsubstituiertem Alkyl, unsubstituiertem deuteriertem Alkyl, unsubstituiertem Haloalkyl, unsubstituiertem Alkoxy, unsubstituiertem Haloalkoxy, Halogen, Cyano, Nitro, Hydroxyl, Amino, Oxo, Imin, unsubstituiertem Alkenyl, unsubstituiertem Alkinyl, unsubstituiertem Cycloalkyl, unsubstituiertem Heterocyclyl, unsubstituiertem Aryl oder unsubstituiertem Heteroaryl;
m 0, 1 oder 2 ist;
n1 0, 1 oder 2 ist; und
n2 0, 1 oder 2 ist.

2. Verbindung gemäß Anspruch 1, das Stereoisomer davon oder das pharmazeutisch akzeptable Salz davon, das eine Verbindung der Formel (XIII) ist: 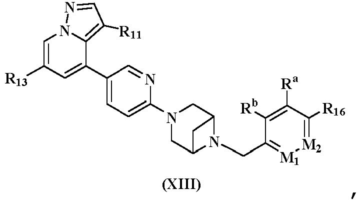 worin:
R₁₁ ausgewählt ist aus Wasserstoff, Deuterium, Halogen, Amino, Nitro, Hydroxyl, Cyano, C₁₋₆-Alkyl, deuteriertem C₁₋₆-Alkyl, C₁₋₆-Haloalkyl, C₁₋₆-Alkoxy, deuteriertem C₁₋₆-Alkoxy, C₁₋₆-Haloalkoxy, C₂₋₆-Alkenyl oder C₂₋₆-Alkinyl;
R₁₃ ausgewählt ist aus C₂₋₆-Alkenyl, C₂₋₆-Alkinyl, - O(CH₂)ₙ₁(CRₐₐR_{bb})ₘR_{cc};
Rₐₐ und R_{bb} jeweils unabhängig ausgewählt sind aus Wasserstoff, Deuterium, Halogen, Amino, Nitro, Hydroxyl, Cyano, C₁₋₆-Alkyl, deuteriertem C₁₋₆-Alkyl, C₁₋₆-Haloalkyl, C₁₋₆-Alkoxy, deuteriertem C₁₋₆-Alkoxy, C₁₋₆-Haloalkoxy, C₂₋₆-Alkenyl oder C₂₋₆-Alkinyl;
R_{cc} ausgewählt sind aus Cyano oder C₂₋₆-Alkinyl;
M₁ ist -CH-;
M₂ ist -N-;
R₁₆ ausgewählt ist aus Wasserstoff, Deuterium, Halogen, Amino, Nitro, Hydroxyl, Cyano, C₁₋₆-Alkyl, deuteriertem C₁₋₆-Alkyl, C₁₋₆-Haloalkyl, C₁₋₆-Alkoxy, deuteriertem C₁₋₆-Alkoxy, C₁₋₆-Haloalkoxy, C₂₋₆-Alkenyl oder C₂₋₆-Alkinyl;
R^{a} unabhängig ausgewählt ist aus Wasserstoff, Deuterium, Halogen, Amino, Nitro, Hydroxyl, Cyano, C₁₋₆-Alkyl, deuteriertem C₁₋₆-Alkyl, C₁₋₆-Haloalkyl, C₁₋₆-Alkoxy, deuteriertem C₁₋₆-Alkoxy, C₁₋₆-Haloalkoxy, C₂₋₆-Alkenyl oder C₂₋₆-Alkinyl;
R^{b} Wasserstoff ist;
n1 eine ganze Zahl von 1 oder 2 ist;
m eine ganze Zahl von 1 oder 2 ist; vorzugsweise,
R₁₁ ausgewählt ist aus Wasserstoff, Deuterium, Halogen, Amino, Nitro, Hydroxyl, Cyano oder C₁₋₃-Alkyl;
R₁₃ ausgewählt ist aus C₂₋₄-Alkenyl, C₂₋₄-Alkinyl oder -OCH₂CRₐₐR_{bb}R_{cc};
Rₐₐ und R_{bb} jeweils unabhängig voneinander ausgewählt sind aus Wasserstoff, Deuterium, Halogen, Amino, Nitro, Hydroxyl, Cyano, C₁₋₃-Alkyl, C₁₋₃-deuteriertem Alkyl, C₁₋₃-Haloalkyl, C₁₋₃-Alkoxy, C₁₋₃-deuteriertem Alkoxy, C₁₋₃-Haloalkoxy, C₂₋₄-Alkenyl oder C₂₋₄-Alkinyl;
R_{cc} sind Cyano oder C₂₋₄-Alkinyl;
M₁ ist -CH-, M₂ ist -N-;
R₁₆ ausgewählt ist aus Wasserstoff, Deuterium, Halogen, Amino, Nitro, Hydroxyl, Cyano, C₁₋₃-Alkyl, C₁₋₃-deuteriertem Alkyl, C₁₋₃-Haloalkyl, C₁₋₃-Alkoxy, C₁₋₃-deuteriertem Alkoxy oder C₁₋₃-Haloalkoxy;
R^{a} unabhängig ausgewählt ist aus Wasserstoff, Deuterium oder Halogen;
R^{b} Wasserstoff ist.

3. Verbindung, dargestellt durch die allgemeine Formel (IX-B), ein Stereoisomer davon oder ein pharmazeutisch akzeptables Salz davon: 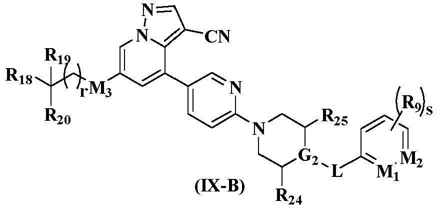 worin:
M₃ ausgewählt ist aus einer Bindung, -O-, -S-, -NH- oder -NCH₃-;
R₁₈ und R₁₉ jeweils unabhängig voneinander ausgewählt sind aus Wasserstoff, Deuterium, C₁₋₆-Alkyl, deuteriertem C₁₋₆-Alkyl, C₁₋₆-Haloalkyl, C₁₋₆-Alkoxy, deuteriertem C₁₋₆-Alkoxy, C₁₋₆-Haloalkoxy, Halogen, Amino, Nitro, Hydroxyl, Cyano, C₂₋₆-Alkenyl, C₂₋₆-Alkinyl, C₃₋₈-Cycloalkyl, 3-12-gliedrigem Heterocyclyl, C₆₋₁₀-Aryl oder 5-12-gliedrigem Heteroaryl, vorzugsweise Wasserstoff oder Methyl;
oder R₁₈ und R₁₉ zusammen mit den Kohlenstoffatomen, an die sie gebunden sind, bilden ein C₃₋₈-Cycloalkyl oder ein 3-12-gliedriges Heterocyclyl, vorzugsweise ein C₃₋₆-Cycloalkyl oder ein 3-7-gliedriges Heterocyclyl umfassend 1-2 Sauerstoffatome, Stickstoffatome oder Schwefelatome, besonders bevorzugt Cyclopropyl, Cyclobutyl, Cyclopentyl, Oxetanyl, Azetidinyl, Bicyclo[1,1,1]pentan oder 1-Imino-1-oxothiopyran, wobei das C₃₋₈-Cycloalkyl oder 3-12-gliedrige Heterocyclyl gegebenenfalls weiter durch einen oder mehrere Substituenten, ausgewählt aus Wasserstoff, C₁₋₆-Alkyl, Hydroxyl, Cyano oder C₁₋₆-Hydroxyalkyl, substituiert ist;
R₂₀ ist ausgewählt aus Cyano oder C₂₋₆-Alkinyl; vorzugsweise Cyano;
R₂₄ und R₂₅ jeweils unabhängig voneinander ausgewählt sind aus Wasserstoff, Deuterium, C₁₋₆-Alkyl, deuteriertem C₁₋₆-Alkyl, C₁₋₆-Haloalkyl, C₁₋₆-Alkoxy, deuteriertem C₁₋₆-Alkoxy, C₁₋₆-Haloalkoxy, Halogen, Amino, Nitro, Hydroxyl, Cyano, C₂₋₆-Alkenyl, C₂₋₆-Alkinyl, C₃₋₈-Cycloalkyl, 3-12-gliedrigem Heterocyclyl, C₆₋₁₀-Aryl, 5-12-gliedrigem Heteroaryl oder -(CH₂)ₙ₁ORₐₐ, vorzugsweise Wasserstoff oder Methyl;
oder R₂₄ und R₂₅ bilden zusammen mit den Kohlenstoffatomen, an die sie gebunden sind, und G₂ ein C₃₋₈-Cycloalkyl oder einen 3-12-gliedrigen Heterocyclyl, vorzugsweise Azetidinyl;
L ausgewählt ist aus Bindung, -(CH₂)ₙ₁CRₐₐR_{bb}-, - (CH₂)ₙ₁NRₐₐC(O)(CH₂)ₙ₂-, -(CH₂)ₙ₁C(O)(CH₂)ₙ₂(CRₐₐR_{bb})ₘ-, - (CH₂)ₙ₁C(O)(CRₐₐR_{bb})ₘ(CH₂)ₙ₂-, -(CH₂)ₙ₁C(O)NR_{cc}(CRₐₐR_{bb})ₙ₂-, - (CH₂)ₙ₁(O)(CH₂)ₙ₂- oder -(CH₂)ₙ₁NRₐₐ(CH₂)ₙ₂-, vorzugsweise - CH₂-, -CD₂-, -O- oder -NHC(O)-;
G₂ ausgewählt ist aus N oder CRₐₐ, vorzugsweise N, CH oder CCH₃;
M₁ ausgewählt ist aus N oder CRₐₐ, vorzugsweise N, CH oder CCH₃;
M₂ ausgewählt ist aus N oder CRₐₐ, vorzugsweise N oder CH;
R₉ ausgewählt ist aus Wasserstoff, Deuterium, C₁₋₆-Alkyl, deuteriertem C₁₋₆-Alkyl, C₁₋₆-Haloalkyl, C₁₋₆-Alkoxy, deuteriertem C₁₋₆-Alkoxy, C₁₋₆-Haloalkoxy, Halogen, Amino, Nitro, Hydroxyl, Cyano, C₂₋₆-Alkenyl, C₂₋₆-Alkinyl, C₃₋₈-Cycloalkyl, 3-12-gliedrigem Heterocyclyl, C₆₋₁₀-Aryl, 5-12-gliedrigem Heteroaryl oder - (CH₂)ₙ₁ORₐₐ; vorzugsweise Wasserstoff, Halogen, C₁₋₆-Alkoxy, deuteriertes C₁₋₆-Alkoxy oder -ORₐₐ; besonders bevorzugt Wasserstoff, Fluor, Chlor, Methyl, Methoxy, deuteriertes Methoxy oder Cyclopropoxy;
Rₐₐ, R_{bb} und R_{cc} jeweils unabhängig voneinander ausgewählt sind aus Wasserstoff, Deuterium, Cyano, Amino, C₁₋₆-Alkyl, C₁₋₆-Alkoxy, C₁₋₆-Hydroxyalkyl, Hydroxyl, C₃₋₈-Cycloalkyl, 3-12-gliedrigem Heterocyclyl oder C₆₋₁₄-Aryl, wobei das C₁₋₆-Alkyl, C₁₋₆-Alkoxy, C₁₋₆-Hydroxyalkyl, Hydroxyl, C₃₋₈-Cycloalkyl, 3-12-gliedriges Heterocylcyl und C₆₋₁₄-Aryl gegebenenfalls weiter substituiert sind durch einen oder mehrere Substituenten, ausgewählt aus Wasserstoff, Halogen, Cyano, Hydroxyl, Oxo, Imino, C₁₋₆-Alkyl oder C₁₋₆-Hydroxyalkyl;
oder zwei beliebige von Rₐₐ, R_{bb} und R_{cc} gegebenenfalls verbunden sind, um ein C₃₋₈-Cycloalkyl oder ein 3-12-gliedriges Heterocyclyl zu bilden, wobei das C₃₋₈-Cycloalkyl und das 3-12-gliedrige Heterocyclyl gegebenenfalls weiter durch einen oder mehrere Substituenten, ausgewählt aus Wasserstoff, Amino, Halogen, Cyano, Hydroxyl, Oxo, Imino, C₁₋₆-Alkyl oder C₁₋₆-Hydroxyalkyl, substituiert sind;
s ist 0, 1, 2 oder 3;
m 0, 1 oder 2 ist;
n1 ist 0, 1 oder 2; und
n2 ist 0, 1 oder 2;
r 0, 1 oder 2 ist;
oder 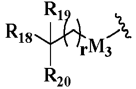 ausgewählt ist aus 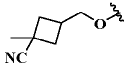 oder 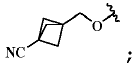 oder 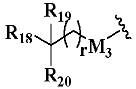 ausgewählt ist aus

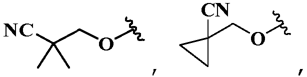

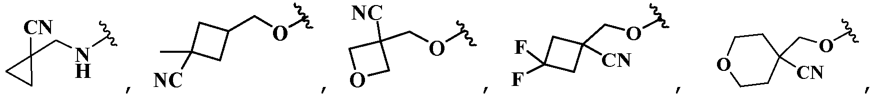

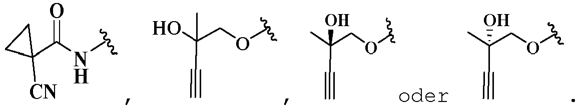

4. Verbindung gemäß Anspruch 3, das Stereoisomer davon oder das pharmazeutisch akzeptable Salz davon, das eine Verbindung der Formel (X) ist: 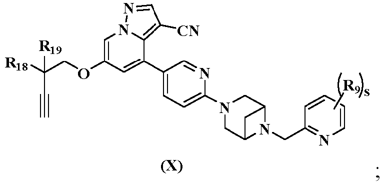
worin R₁₈ und R₁₉ jeweils unabhängig voneinander ausgewählt sind aus Wasserstoff, Deuterium, C₁₋₃-Alkyl, deuteriertem C₁₋₃-Alkyl, C₁₋₃-Haloalkyl, C₁₋₃-Alkoxy, deuteriertem C₁₋₃-Alkoxy, C₁₋₃-Haloalkoxy, Halogen, Amino, Nitro, Hydroxyl, Cyano, C₂₋₄-Alkenyl, C₂₋₄-Alkinyl, C₃₋₆-Cycloalkyl, 3-6-gliedrigem Heterocyclyl, C₆₋₁₀-Aryl oder 5-6-gliedrigem Heteroaryl, vorzugsweise Hydroxyl oder Methyl;
R₉ ausgewählt ist aus Wasserstoff, Deuterium, C₁₋₃-Alkyl, deuteriertem C₁₋₃-Alkyl, C₁₋₃-Haloalkyl, C₁₋₃-Alkoxy, deuteriertem C₁₋₃-Alkoxy, C₁₋₃-Haloalkoxy, Halogen, Amino, Nitro, Hydroxyl, Cyano, C₂₋₄-Alkenyl oder C₂₋₄-Alkinyl;
s 0, 1, 2 oder 3 ist.

5. Verbindung, dargestellt durch die allgemeine Formel (IX-C), ein Stereoisomer davon oder ein pharmazeutisch akzeptables Salz davon: 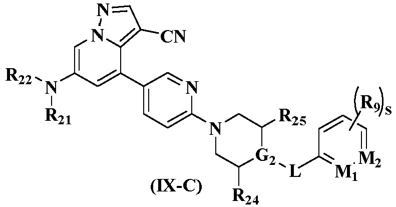 worin:
R₂₁ und R₂₂ jeweils unabhängig voneinander aus Wasserstoff, Deuterium, C₁₋₆-Alkyl, deuteriertem C₁₋₆-Alkyl, C₁₋₆-Haloalkyl, C₁₋₆-Alkoxy, deuteriertem C₁₋₆-Alkoxy, C₁₋₆-Haloalkoxy, Halogen, Amino, Nitro, Hydroxyl, Cyano, C₂₋₆-Alkenyl, C₂₋₆-Alkinyl, C₃₋₈-Cycloalkyl, 3-12-gliedrigem Heterocyclyl, C₆₋₁₀-Aryl, 5-12-gliedrigem Heteroaryl, - (CH₂)ₙ₁C(O)Rₐₐ oder -(CH₂)ₙ₁Rₐₐ ausgewählt sind; oder
R₂₁ und R₂₂ zusammen mit den Stickstoffatomen, an die sie gebunden sind, ein Azetidinyl bilden, wobei das Azetidinyl gegebenenfalls weiter durch einen oder mehrere Substituenten, ausgewählt aus Wasserstoff, Amino, Halogen, Cyano, Hydroxyl, Oxo, C₁₋₆-Alkyl oder C₁₋₆-Hydroxyalkyl, substituiert ist;
R₂₄ und R₂₅ jeweils unabhängig voneinander ausgewählt sind aus Wasserstoff, Deuterium, C₁₋₆-Alkyl, deuteriertem C₁₋₆-Alkyl, C₁₋₆-Halogenalkyl, C₁₋₆-Alkoxy, deuteriertem C₁₋₆-Alkoxy, C₁₋₆-Halogenalkoxy, Halogen, Amino, Nitro, Hydroxyl, Cyano, C₂₋₆-Alkenyl, C₂₋₆-Alkinyl, C₃₋₈-Cycloalkyl, 3-12-gliedrigem Heterocyclyl, C₆₋₁₀-Aryl, 5-12-gliedrigem Heteroaryl oder -(CH₂)ₙ₁ORₐₐ, vorzugsweise Wasserstoff oder Methyl;
oder R₂₄ und R₂₅ bilden zusammen mit den Kohlenstoffatomen, an die sie gebunden sind, und G₂ ein C₃₋₈-Cycloalkyl oder einen 3-12-gliedrigen Heterocyclyl, vorzugsweise Azetidinyl;
L ausgewählt ist aus Bindung, -(CH₂)ₙ₁CRₐₐR_{bb}-, - (CH₂)ₙ₁NRₐₐC(O)(CH₂)ₙ₂-, - (CH₂)ₙ₁C(O)(CH₂)ₙ₂(CRₐₐR_{bb})ₘ-, - (CH₂)ₙ₁C(O)(CRₐₐR_{bb})ₘ(CH₂)ₙ₂-, -(CH₂)ₙ₁C(O)NR_{cc}(CRₐₐR_{bb})ₙ₂-, - (CH₂)ₙ₁(O)(CH₂)ₙ₂- oder -(CH₂)ₙ₁NRₐₐ(CH₂)ₙ₂-, vorzugsweise - CH₂-, -CD₂-, -O- oder -NHC(O)-;
G₂ ausgewählt ist aus N oder CRₐₐ, vorzugsweise N, CH oder CCH₃;
M₁ ausgewählt ist aus N oder CRₐₐ, vorzugsweise N, CH oder CCH₃;
M₂ ausgewählt ist aus N oder CRₐₐ, vorzugsweise N oder CH;
R₉ ausgewählt ist aus Wasserstoff, Deuterium, C₁₋₆-Alkyl, deuteriertem C₁₋₆-Alkyl, C₁₋₆-Halogenalkyl, C₁₋₆-Alkoxy, deuteriertem C₁₋₆-Alkoxy, C₁₋₆-Halogenalkoxy, Halogen, Amino, Nitro, Hydroxyl, Cyano, C₂₋₆-Alkenyl, C₂₋₆-Alkinyl, C₃₋₈-Cycloalkyl, 3-12-gliedrigem Heterocyclyl, C₆₋₁₀-Aryl, 5-12-gliedrigem Heteroaryl oder -(CH₂)ₙ₁ORₐₐ; vorzugsweise Wasserstoff, Halogen, C₁₋₆-Alkoxy, deuteriertes C₁₋₆-Alkoxy oder -ORₐₐ; besonders bevorzugt Wasserstoff, Fluor, Chlor, Methyl, Methoxy, deuteriertes Methoxy oder Cyclopropoxy;
Rₐₐ, R_{bb} und R_{cc} jeweils unabhängig voneinander ausgewählt sind aus Wasserstoff, Deuterium, Cyano, Amino, C₁₋₆-Alkyl, C₁₋₆-Alkoxy, C₁₋₆-Hydroxyalkyl, Hydroxyl, C₃₋₈-Cycloalkyl, 3-12-gliedrigem Heterocyclyl oder C₆₋₁₄-Aryl, wobei das C₁₋₆-Alkyl, C₁₋₆-Alkoxy, C₁₋₆-Hydroxyalkyl, Hydroxyl, C₃₋₈-Cycloalkyl, 3-12-gliedriges Heterocylcyl und C₆₋₁₄-Aryl gegebenenfalls weiter substituiert sind durch einen oder mehrere Substituenten, ausgewählt aus Wasserstoff, Halogen, Cyano, Hydroxyl, Oxo, Imino, C₁₋₆-Alkyl oder C₁₋₆-Hydroxyalkyl;
oder zwei beliebige von Rₐₐ, R_{bb} und R_{cc} gegebenenfalls verbunden sind, um ein C₃₋₈-Cycloalkyl oder ein 3-12-gliedriges Heterocyclyl zu bilden, wobei das C₃₋₈-Cycloalkyl und das 3-12-gliedrige Heterocyclyl gegebenenfalls weiter durch einen oder mehrere Substituenten, ausgewählt aus Wasserstoff, Amino, Halogen, Cyano, Hydroxyl, Oxo, Imino, C₁₋₆-Alkyl oder C₁₋₆-Hydroxyalkyl, substituiert sind;
s ist 0, 1, 2 oder 3;
m 0, 1 oder 2 ist;
n1 ist 0, 1 oder 2; und
n2 ist 0, 1 oder 2.

6. Verbindung gemäß Anspruch 5, das Stereoisomer davon oder das pharmazeutisch akzeptable Salz davon,
R₂₁ und R₂₂ jeweils unabhängig voneinander ausgewählt sind aus Wasserstoff, Deuterium, C₁₋₆-Alkyl, deuteriertem C₁₋₆-Alkyl, C₁₋₆-Haloalkyl, C₁₋₆-Alkoxy, deuteriertem C₁₋₆-Alkoxy, C₁₋₆-Haloalkoxy, Halogen, Amino, Nitro, Hydroxyl, Cyano, C₂₋₆-Alkenyl, C₂₋₆-Alkinyl, C₃₋₈-Cycloalkyl, 3-12-gliedrigem Heterocyclyl, C₆₋₁₀-Aryl, 5-12-gliedrigem Heteroaryl, -(CH₂)ₙ₁C(O)Rₐₐ oder -(CH₂)ₙ₁Rₐₐ; oder
R₂₁ und R₂₂ zusammen mit den Stickstoffatomen, an die sie gebunden sind, ein Azetidinyl bilden, wobei das Azetidinyl gegebenenfalls weiter durch einen oder mehrere Substituenten, ausgewählt aus Wasserstoff, Amino, Halogen, Cyano, Hydroxyl, Oxo, C₁₋₆-Alkyl oder C₁₋₆-Hydroxyalkyl, substituiert ist;
R₂₄ und R₂₅ jeweils unabhängig voneinander aus Wasserstoff oder Methyl ausgewählt sind;
oder R₂₄ und R₂₅ zusammen mit den Kohlenstoffatomen, an die sie gebunden sind, und G₂ ein Azetidinyl bilden;
L ausgewählt ist aus -CH₂-, -CD₂-, -O- oder - NHC(O)-;
G₂ ausgewählt ist aus N, CH oder CCH₃;
M₁ ausgewählt ist aus N, CH oder CCH₃;
M₂ ausgewählt ist aus N oder CH;
R₉ ausgewählt ist aus Wasserstoff, Fluor, Chlor, Methyl, Methoxy, deuteriertem Methoxy oder Cyclopropoxy; Raa jeweils unabhängig voneinander ausgewählt ist aus Wasserstoff, Deuterium, Cyano, Amino, C₁₋₆-Alkyl, C₁₋₆-Alkoxy, C₁₋₆-Hydroxyalkyl, Hydroxyl, C₃₋₈-Cycloalkyl, 3-12-gliedrigem Heterocyclyl oder C₆₋₁₄-Aryl, wobei C₁₋₆-Alkyl, C₁₋₆-Alkoxy, C₁₋₆-Hydroxyalkyl, Hydroxyl, C₃₋₈-Cycloalkyl, 3-12-gliedriges Heterocyclyl und C₆₋₁₄-Aryl gegebenenfalls weiter substituiert sind durch einen oder mehrere Substituenten, ausgewählt aus Wasserstoff, Halogen, Cyano, Hydroxyl, Oxo, Imino, C₁₋₆-Alkyl oder C₁₋₆-Hydroxyalkyl;
s 0, 1, 2 oder 3 ist; und
n1 0, 1 oder 2 ist.

7. Verbindung, dargestellt durch die allgemeine Formel (IX-C), ein Stereoisomer davon oder ein pharmazeutisch akzeptables Salz davon: 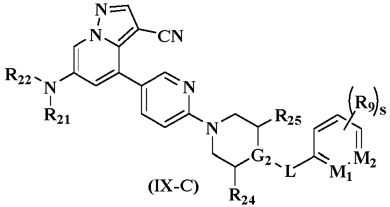 worin:
R₂₁ und R₂₂ jeweils unabhängig voneinander ausgewählt sind aus Wasserstoff, Deuterium, C₁₋₆-Alkyl, deuteriertem C₁₋₆-Alkyl, C₁₋₆-Haloalkyl, C₁₋₆-Alkoxy, deuteriertem C₁₋₆-Alkoxy, C₁₋₆-Haloalkoxy, Halogen, Amino, Nitro, Hydroxyl, Cyano, C₂₋₆-Alkenyl, C₂₋₆-Alkinyl, C₃₋₈-Cycloalkyl, 3-12-gliedrigem Heterocyclyl, C₆₋₁₀-Aryl, 5-12-gliedrigem Heteroaryl, -(CH₂)ₙ₁C(O)Rₐₐ oder -(CH₂)ₙ₁Rₐₐ; oder
R₂₁ und R₂₂ zusammen mit den Stickstoffatomen, an die sie gebunden sind, Azetidinyl oder 2-Azaspiro[3.3]heptan bilden, wobei das Azetidinyl oder 2-Azaspiro[3.3]heptan gegebenenfalls durch einen oder mehrere Substituenten, ausgewählt aus Wasserstoff, C₁₋₆-Alkyl, Hydroxyl oder Hydroxyalkyl, weiter substituiert ist;
R₂₄ und R₂₅ jeweils unabhängig voneinander ausgewählt sind aus Wasserstoff, Deuterium, C₁₋₆-Alkyl, deuteriertem C₁₋₆-Alkyl, C₁₋₆-Haloalkyl, C₁₋₆-Alkoxy, deuteriertem C₁₋₆-Alkoxy, C₁₋₆-Haloalkoxy, Halogen, Amino, Nitro, Hydroxyl, Cyano, C₂₋₆-Alkenyl, C₂₋₆-Alkinyl, C₃₋₈-Cycloalkyl, 3-12-gliedrigem Heterocyclyl, C₆₋₁₀-Aryl, 5-12-gliedrigem Heteroaryl oder -(CH₂)ₙ₁ORₐₐ, vorzugsweise Wasserstoff oder Methyl;
oder R₂₄ und R₂₅ bilden zusammen mit den Kohlenstoffatomen, an die sie gebunden sind, und G₂ ein C₃₋₈-Cycloalkyl oder einen 3-12-gliedrigen Heterocyclyl, vorzugsweise Azetidinyl;
L ausgewählt ist aus Bindung, -(CH₂)ₙ₁CRₐₐR_{bb}-, - (CH₂)ₙ₁NRₐₐC(O)(CH₂)ₙ₂-, -(CH₂)ₙ₁C(O)(CH₂)ₙ₂(CRₐₐR_{bb})ₘ-, - (CH₂)ₙ₁C(O)(CRₐₐR_{bb})ₘ(CH₂)ₙ₂-, -(CH₂)ₙ₁C(O)NR_{cc}(CRₐₐR_{bb})ₙ₂-, - (CH₂)ₙ₁(O)(CH₂)ₙ₂- oder -(CH₂)ₙ₁NRₐₐ(CH₂)ₙ₂-, vorzugsweise - CH₂-, -CD₂-, -O- oder -NHC(O)-;
G₂ ausgewählt ist aus N oder CRₐₐ, vorzugsweise N, CH oder CCH₃;
M₁ ausgewählt ist aus N oder CRₐₐ, vorzugsweise N, CH oder CCH₃;
M₂ ausgewählt ist aus N oder CRₐₐ, vorzugsweise N oder CH;
R₉ ausgewählt ist aus Wasserstoff, Deuterium, C₁₋₆-Alkyl, deuteriertem C₁₋₆-Alkyl, C₁₋₆-Halogenalkyl, C₁₋₆-Alkoxy, deuteriertem C₁₋₆-Alkoxy, C₁₋₆-Halogenalkoxy, Halogen, Amino, Nitro, Hydroxyl, Cyano, C₂₋₆-Alkenyl, C₂₋₆-Alkinyl, C₃₋₈-Cycloalkyl, 3-12-gliedrigem Heterocyclyl, C₆₋₁₀-Aryl, 5-12-gliedrigem Heteroaryl oder -(CH₂)ₙ₁ORₐₐ; vorzugsweise Wasserstoff, Halogen, C₁₋₆-Alkoxy, deuteriertes C₁₋₆-Alkoxy oder -ORₐₐ; besonders bevorzugt Wasserstoff, Fluor, Chlor, Methyl, Methoxy, deuteriertes Methoxy oder Cyclopropoxy;
Rₐₐ, R_{bb} und R_{cc} jeweils unabhängig voneinander ausgewählt sind aus Wasserstoff, Deuterium, Cyano, Amino, C₁₋₆-Alkyl, C₁₋₆-Alkoxy, C₁₋₆-Hydroxyalkyl, Hydroxyl, C₃₋₈-Cycloalkyl, 3-12-gliedrigem Heterocyclyl oder C₆₋₁₄-Aryl, wobei das C₁₋₆-Alkyl, C₁₋₆-Alkoxy, C₁₋₆-Hydroxyalkyl, Hydroxyl, C₃₋₈-Cycloalkyl, 3-12-gliedriges Heterocylcyl und C₆₋₁₄-Aryl gegebenenfalls weiter substituiert sind durch einen oder mehrere Substituenten, ausgewählt aus Wasserstoff, Halogen, Cyano, Hydroxyl, Oxo, Imino, C₁₋₆-Alkyl oder C₁₋₆-Hydroxyalkyl;
oder zwei beliebige von Rₐₐ, R_{bb} und R_{cc} gegebenenfalls verbunden sind, um ein C₃₋₈-Cycloalkyl oder ein 3-12-gliedriges Heterocyclyl zu bilden, wobei das C₃₋₈-Cycloalkyl und das 3-12-gliedrige Heterocyclyl gegebenenfalls weiter durch einen oder mehrere Substituenten, ausgewählt aus Wasserstoff, Amino, Halogen, Cyano, Hydroxyl, Oxo, Imino, C₁₋₆-Alkyl oder C₁₋₆-Hydroxyalkyl, substituiert sind;
s ist 0, 1, 2 oder 3;
m 0, 1 oder 2 ist;
n1 ist 0, 1 oder 2; und
n2 ist 0, 1 oder 2;
oder R₂₁ und R₂₂ zusammen mit den Kohlenstoffatomen, an die sie gebunden sind, ein Azetidinyl oder 2-Azaspiro[3.3]heptan bilden, wobei das Azetidinyl oder 2-Azaspiro[3.3]heptan gegebenenfalls weiter durch einen oder mehrere Substituenten, ausgewählt aus Wasserstoff, C₁₋₃-Alkyl, Cyano, Hydroxyl oder Hydroxyalkyl, substituiert ist.

8. Verbindung der allgemeinen Formel (IX-C), ein Stereoisomer davon oder ein pharmazeutisch akzeptables Salz davon: 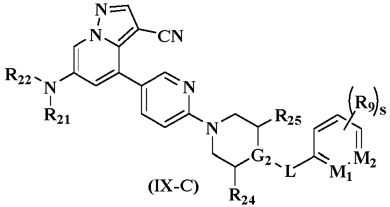 worin: 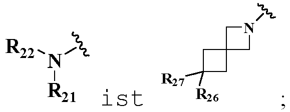
R₂₆ und R₂₇ sind jeweils unabhängig voneinander ausgewählt aus Wasserstoff, Deuterium, Halogen, Amino, Nitro, Hydroxyl, Cyano, C₁₋₆-Alkyl, deuteriertem C₁₋₆-Alkyl, C₁₋₆-Haloalkyl, C₁₋₆-Alkoxy, deuteriertem C₁₋₆-Alkoxy, C₁₋₆-Haloalkoxy, C₁₋₆-Hydroxyalkyl, C₂₋₆-Alkenyl, C₂₋₆-Alkinyl, C₃₋₈-Cycloalkyl, 3-12-gliedrigem Heterocyclyl, C₆₋₁₀-Aryl oder 5-12-gliedrigem Heteroaryl; vorzugsweise Wasserstoff, Hydroxyl, Cyano, C₁₋₃-Alkyl oder C₁₋₃-Hydroxyalkyl; besonders bevorzugt Wasserstoff, Hydroxyl, Cyanomethyl oder Hydroxyisopropyl;
R₂₄ und R₂₅ jeweils unabhängig voneinander ausgewählt sind aus Wasserstoff, Deuterium, C₁₋₆-Alkyl, deuteriertem C₁₋₆-Alkyl, C₁₋₆-Haloalkyl, C₁₋₆-Alkoxy, deuteriertem C₁₋₆-Alkoxy, C₁₋₆-Haloalkoxy, Halogen, Amino, Nitro, Hydroxyl, Cyano, C₂₋₆-Alkenyl, C₂₋₆-Alkinyl, C₃₋₈-Cycloalkyl, 3-12-gliedrigem Heterocyclyl, C₆₋₁₀-Aryl, 5-12-gliedrigem Heteroaryl oder -(CH₂)ₙ₁ORₐₐ, vorzugsweise Wasserstoff oder Methyl;
oder R₂₄ und R₂₅ bilden zusammen mit den Kohlenstoffatomen, an die sie gebunden sind, und G₂ ein C₃₋₈-Cycloalkyl oder einen 3-12-gliedrigen Heterocyclyl, vorzugsweise Azetidinyl;
L ausgewählt ist aus Bindung, -(CH₂)ₙ₁CRₐₐR_{bb}-, - (CH₂)ₙ₁NRₐₐC(O)(CH₂)ₙ₂-, -(CH₂)ₙ₁C(O)(CH₂)ₙ₂(CRₐₐR_{bb})ₘ-, - (CH₂)ₙ₁C(O)(CRₐₐR_{bb})ₘ(CH₂)ₙ₂-, -(CH₂)ₙ₁C(O)NR_{cc}(CRₐₐR_{bb})ₙ₂-, - (CH₂)ₙ₁(O)(CH₂)ₙ₂- oder -(CH₂)ₙ₁NRₐₐ(CH₂)ₙ₂-, vorzugsweise - CH₂-, -CD₂-, -O- oder -NHC(O)-;
G₂ ausgewählt ist aus N oder CRₐₐ, vorzugsweise N, CH oder CCH₃;
M₁ ausgewählt ist aus N oder CRₐₐ, vorzugsweise N, CH oder CCH3;
M₂ ausgewählt ist aus N oder CRₐₐ, vorzugsweise N oder CH;
R₉ ausgewählt ist aus Wasserstoff, Deuterium, C₁₋₆-Alkyl, deuteriertem C₁₋₆-Alkyl, C₁₋₆-Haloalkyl, C₁₋₆-Alkoxy, deuteriertem C₁₋₆-Alkoxy, C₁₋₆-Haloalkoxy, Halogen, Amino, Nitro, Hydroxyl, Cyano, C₂₋₆-Alkenyl, C₂₋₆-Alkinyl, C₃₋₈-Cycloalkyl, 3-12-gliedrigem Heterocyclyl, C₆₋₁₀-Aryl, 5-12-gliedrigem Heteroaryl oder -(CH₂)ₙ₁ORₐₐ; vorzugsweise Wasserstoff, Halogen, C₁₋₆-Alkoxy, deuteriertes C₁₋₆-Alkoxy oder -ORₐₐ; besonders bevorzugt Wasserstoff, Fluor, Chlor, Methyl, Methoxy, deuteriertes Methoxy oder Cyclopropoxy;
Rₐₐ, R_{bb} und R_{cc} jeweils unabhängig voneinander ausgewählt sind aus Wasserstoff, Deuterium, Cyano, Amino, C₁₋₆-Alkyl, C₁₋₆-Alkoxy, C₁₋₆-Hydroxyalkyl, Hydroxyl, C₃₋₈-Cycloalkyl, 3-12-gliedrigem Heterocyclyl oder C₆₋₁₄-Aryl, wobei das C₁₋₆-Alkyl, C₁₋₆-Alkoxy, C₁₋₆-Hydroxyalkyl, Hydroxyl, C₃₋₈-Cycloalkyl, 3-12-gliedriges Heterocylcyl und C₆₋₁₄-Aryl gegebenenfalls weiter substituiert sind durch einen oder mehrere Substituenten, ausgewählt aus Wasserstoff, Halogen, Cyano, Hydroxyl, Oxo, Imino, C₁₋₆-Alkyl oder C₁₋₆-Hydroxyalkyl;
oder zwei beliebige von Rₐₐ, R_{bb} und R_{cc} gegebenenfalls verbunden sind, um ein C₃₋₈-Cycloalkyl oder ein 3-12-gliedriges Heterocyclyl zu bilden, wobei das C₃₋₈-Cycloalkyl und das 3-12-gliedrige Heterocyclyl gegebenenfalls weiter durch einen oder mehrere Substituenten substituiert sind, die aus Wasserstoff, Amino, Halogen, Cyano, Hydroxyl, Oxo, Imino, C₁₋₆-Alkyl oder C₁₋₆-Hydroxyalkyl ausgewählt sind;
s ist 0, 1, 2 oder 3;
m 0, 1 oder 2 ist;
n1 ist 0, 1 oder 2; und
n2 ist 0, 1 oder 2.

9. Verbindung, dargestellt durch die allgemeine Formel (IX-A), ein Stereoisomer davon oder ein pharmazeutisch akzeptables Salz davon: 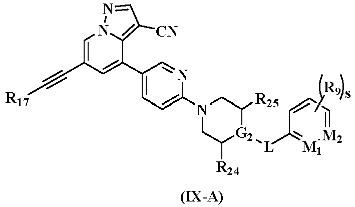 worin:
L ausgewählt ist aus Bindung, -(CH₂)ₙ₁CRₐₐR_{bb}-, - (CH₂)ₙ₁NRₐₐC(O)(CH₂)ₙ₂-, -(CH₂)ₙ₁C(O)(CH₂)ₙ₂(CRₐₐR_{bb})ₘ-,-(CH₂)ₙ₁C(O)(CRₐₐR_{bb})ₘ(CH₂)ₙ₂-, -(CH₂)ₙ₁C(O)NR_{cc}(CRₐₐR_{bb})ₙ₂-, - (CH₂)ₙ₁(O)(CH₂)ₙ₂- oder -(CH₂)ₙ₁NRₐₐ(CH₂)ₙ₂-, vorzugsweise - CH₂-, -CD₂-, -O- oder -NHC(O)-;
G₂ ausgewählt ist aus N oder CRₐₐ, vorzugsweise N, CH oder CCH₃;
M₁ ausgewählt ist aus N oder CRₐₐ, vorzugsweise N, CH oder CCH₃;
M₂ ausgewählt ist aus N oder CRₐₐ, vorzugsweise N oder CH;
R₉ ausgewählt ist aus Wasserstoff, Deuterium, C₁₋₆-Alkyl, deuteriertem C₁₋₆-Alkyl, C₁₋₆-Haloalkyl, C₁₋₆-Alkoxy, deuteriertem C₁₋₆-Alkoxy, C₁₋₆-Haloalkoxy, Halogen, Amino, Nitro, Hydroxyl, Cyano, C₂₋₆-Alkenyl, C₂₋₆-Alkinyl, C₃₋₈-Cycloalkyl, 3-12-gliedrigem Heterocyclyl, C₆₋₁₀-Aryl, 5-12-gliedrigem Heteroaryl oder - (CH₂)ₙ₁ORₐₐ; vorzugsweise Wasserstoff, Halogen, C₁₋₆-Alkoxy, deuteriertes C₁₋₆-Alkoxy oder -ORₐₐ; besonders bevorzugt Wasserstoff, Fluor, Chlor, Methyl, Methoxy, deuteriertes Methoxy oder Cyclopropoxy;
R₁₇ ausgewählt ist aus C₁₋₆-Alkyl, C₁₋₆-Haloalkyl, C₁₋₆-Alkoxy, C₂₋₆-Alkinyl, 3-12-gliedrigem Heterocyclyl, 5-12-gliedrigem Heteroaryl oder - (CH₂)ₙ₁(CRₐₐR_{bb})R_{cc}, wobei das C₁₋₆-Alkyl, C₁₋₆-Haloalkyl, C₁₋₆-Alkoxy, C₂₋₆-Alkinyl, 3-12-gliedriges Heterocyclyl und 5-12-gliedriges Heteroaryl gegebenenfalls weiter substituiert sind durch einen oder mehrere Substituenten, ausgewählt aus Wasserstoff, C₁₋₆-Haloalkyl, Hydroxyl, Cyano, Oxo, Thio, Amino, Imino, C₁₋₆-Alkoxy, C₁₋₆-Hydroxyalkyl, C₁₋₆-Cyanoalkyl, C₃₋₈-Cycloalkyl oder 3-12-gliedrigem Heterocyclyl;
R₂₄ und R₂₅ jeweils unabhängig voneinander ausgewählt sind aus Wasserstoff, Deuterium, C₁₋₆-Alkyl, deuteriertem C₁₋₆-Alkyl, C₁₋₆-Haloalkyl, C₁₋₆-Alkoxy, deuteriertem C₁₋₆-Alkoxy, C₁₋₆-Haloalkoxy, Halogen, Amino, Nitro, Hydroxyl, Cyano, C₂₋₆-Alkenyl, C₂₋₆-Alkinyl, C₃₋₈-Cycloalkyl, 3-12-gliedrigem Heterocyclyl, C₆₋₁₀-Aryl, 5-12-gliedrigem Heteroaryl oder -(CH₂)ₙ₁ORₐₐ, vorzugsweise Wasserstoff oder Methyl;
oder R₂₄ und R₂₅ bilden zusammen mit den Kohlenstoffatomen, an die sie gebunden sind, und G₂ ein C₃₋₈-Cycloalkyl oder einen 3-12-gliedrigen Heterocyclyl, vorzugsweise Azetidinyl;
Rₐₐ, R_{bb} und R_{cc} jeweils unabhängig voneinander ausgewählt sind aus Wasserstoff, Deuterium, Cyano, Amino, C₁₋₆-Alkyl, C₁₋₆-Alkoxy, C₁₋₆-Hydroxyalkyl, Hydroxyl, C₃₋₈-Cycloalkyl, 3-12-gliedrigem Heterocyclyl oder C₆₋₁₄-Aryl, wobei das C₁₋₆-Alkyl, C₁₋₆-Alkoxy, C₁₋₆-Hydroxyalkyl, Hydroxyl, C₃₋₈-Cycloalkyl, 3-12-gliedriges Heterocyclyl und C₆₋₁₄-Aryl gegebenenfalls weiter substituiert sind durch einen oder mehrere Substituenten, ausgewählt aus Wasserstoff, Halogen, Cyano, Hydroxyl, Oxo, Imino, C₁₋₆-Alkyl oder C₁₋₆-Hydroxyalkyl;
oder zwei beliebige von Rₐₐ, R_{bb} und R_{cc} gegebenenfalls verbunden sind, um ein C₃₋₈-Cycloalkyl oder ein 3-12-gliedriges Heterocyclyl zu bilden, wobei das C₃₋₈-Cycloalkyl und das 3-12-gliedrige Heterocyclyl gegebenenfalls weiter durch einen oder mehrere Substituenten, ausgewählt aus Wasserstoff, Amino, Halogen, Cyano, Hydroxyl, Oxo, Imino, C₁₋₆-Alkyl oder C₁₋₆-Hydroxyalkyl, substituiert sind;
n1 ist 0, 1 oder 2;
n2 ist 0, 1 oder 2;
m ist 0, 1 oder 2; und
s ist 0, 1, 2 oder 3.

10. Die in Anspruch 1 definierte Verbindung, das Stereoisomer davon oder das pharmazeutisch akzeptable Salz davon, wobei,
L ausgewählt ist aus Bindung, -(CH₂)ₙ₁CRₐₐR_{bb}-, - (CH₂)ₙ₁NRₐₐC(O)(CH₂)ₙ₂-, -(CH₂)ₙ₁C(O)(CRₐₐR_{bb})ₘ(CH₂)ₙ₂-, - (CH₂)ₙ₁C(O)(CH₂)ₘ(CRₐₐR_{bb})ₙ₂-, -(CH₂)ₙ₁C(O)NR_{cc}(CRₐₐR_{bb})ₙ₂-, - (CH₂)ₙ₁(O)(CH₂)ₙ₂- oder -(CH₂)ₙ₁NRₐₐ(CH₂)ₙ₂-;
R₁₁ ausgewählt ist aus Cyano, -(CH₂)ₙ₁P(O)RₐₐR_{bb} oder -(CH₂)ₙ₁C(O)NRₐₐR_{bb};
R₁₂ ausgewählt ist aus Wasserstoff, C₁₋₆-Alkyl oder Halogen;
R₁₃ ausgewählt ist aus C₂₋₆-Alkinyl, - (C≡C)ₙ₁(CRₐₐR_{bb})ₘR_{cc}, -(C=C)ₙ₁(CRₐₐR_{bb})ₘR_{cc}, oder - (CH₂)ₙ₁O(CH₂)ₙ₂(CRₐₐR_{bb})mR_{cc};
R₁₄ ausgewählt ist aus Wasserstoff oder Halogen;
R₁₅ ausgewählt ist aus Wasserstoff oder Halogen;
R₁₆ aus Wasserstoff, Alkoxy oder -ORₐₐ ausgewählt ist;
Rₐₐ und R_{bb} jeweils unabhängig voneinander ausgewählt sind aus Wasserstoff, Deuterium, Cyano, Amino, C₁₋₆-Alkyl, C₁₋₆-Alkoxy, Hydroxyl, C₃₋₈-Cycloalkyl, 3-12-gliedrigem Heterocyclyl oder C₆₋₁₄-Aryl, wobei das C₁₋₆-Alkyl, C₁₋₆-Alkoxy, C₃₋₈-Cycloalkyl, 3-12-gliedrige Heterocyclyl und C₆₋₁₄-Aryl gegebenenfalls weiter substituiert sind durch einen oder mehrere Substituenten, ausgewählt aus Wasserstoff, Halogen, Cyano, Hydroxyl, Oxo, Imino oder C₁₋₆-Alkyl;
R_{cc} ausgewählt ist aus Cyano;
oder beliebige zwei von Rₐₐ und R_{bb} gegebenenfalls verbunden sind, um ein C₃₋₈-Cycloalkyl oder ein 3-12-gliedriges Heterocyclyl zu bilden, wobei das C₃₋₈-Cycloalkyl und das 3-12-gliedrige Heterocyclyl gegebenenfalls durch einen oder mehrere Substituenten, ausgewählt aus Wasserstoff, Amino, Halogen, Cyano, Hydroxyl, Oxo, Imin oder C₁₋₆-Alkyl, weiter substituiert sind.

11. Verbindung, ein Stereoisomer davon oder ein pharmazeutisch akzeptables Salz davon, wobei die spezifische Struktur der Verbindung wie folgt ist:

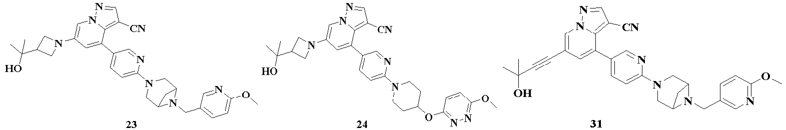

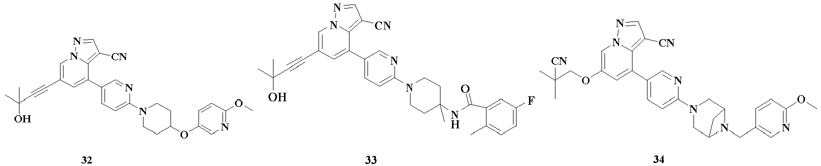

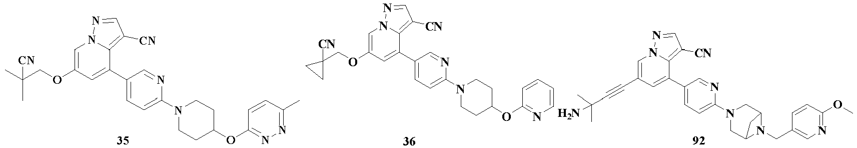

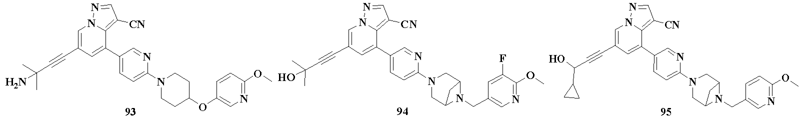

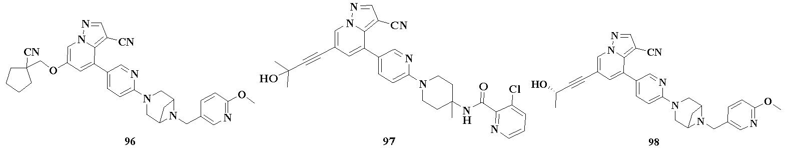

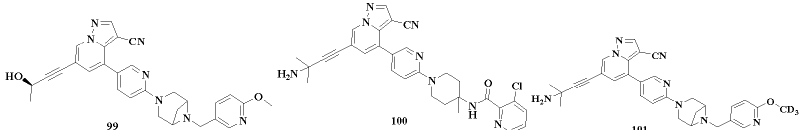

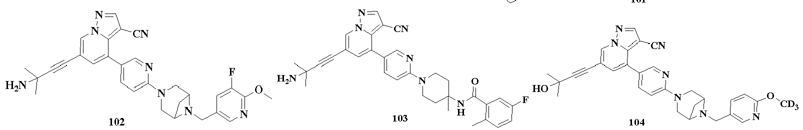

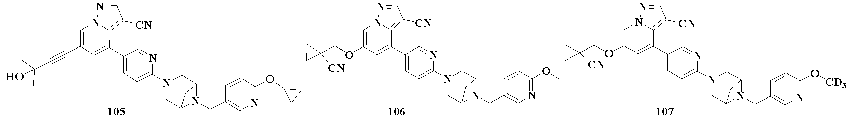

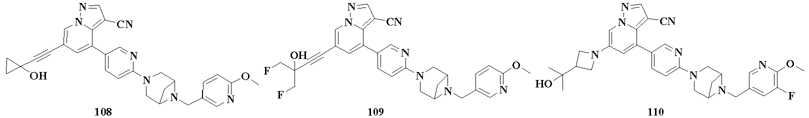

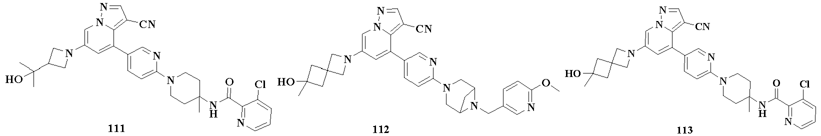

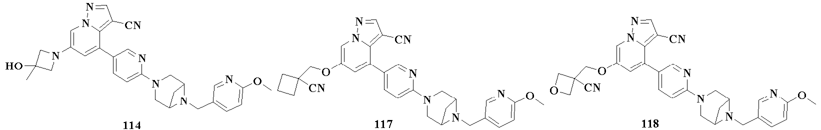

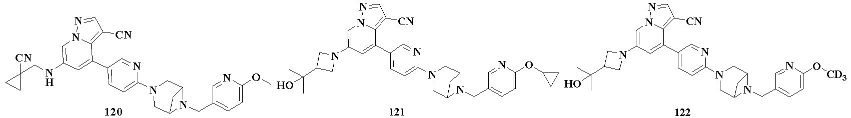

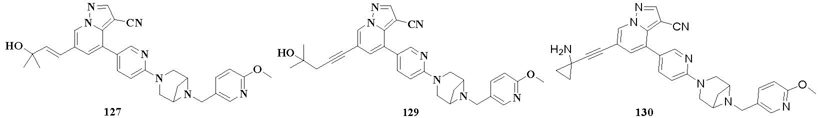

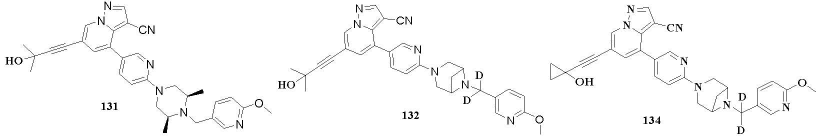

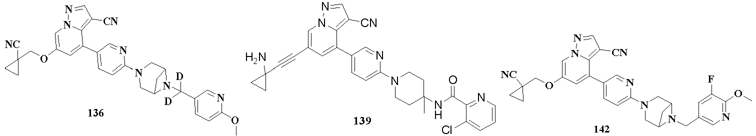

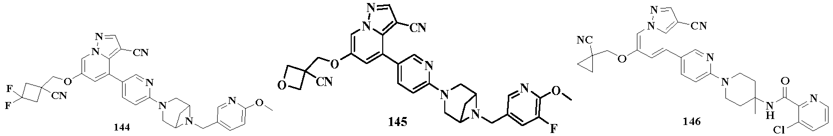

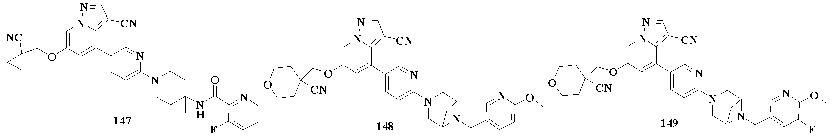

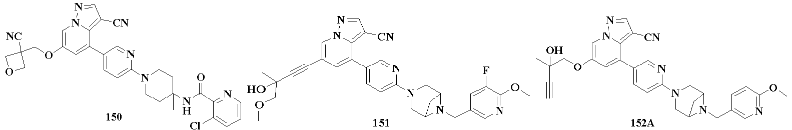

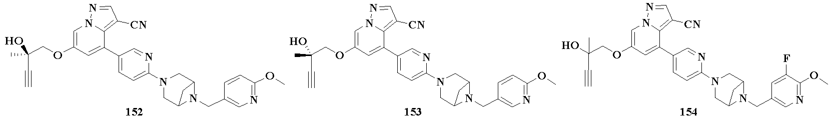

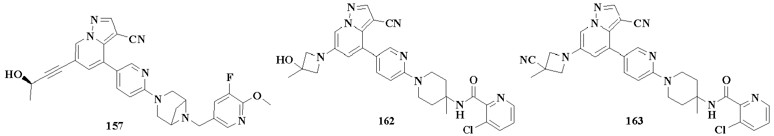

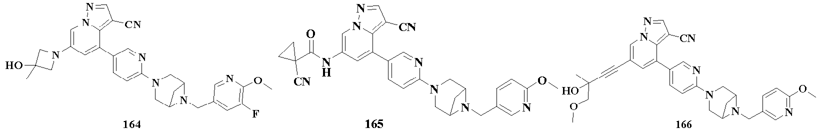

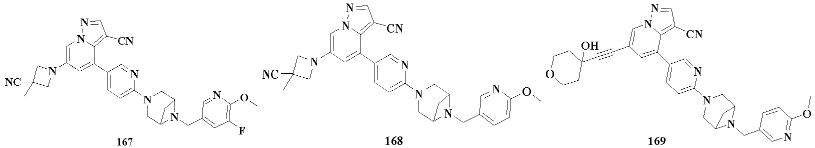

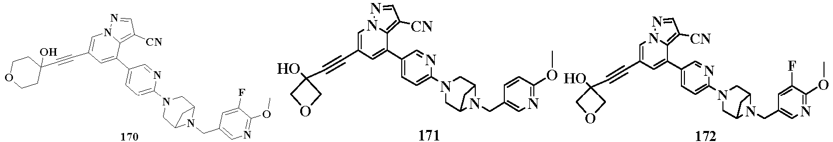

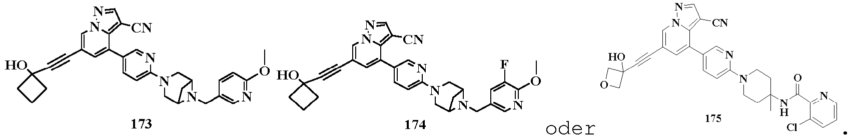

12. Verfahren zur Herstellung der Verbindung der allgemeinen Formel (IX-A), wie in Anspruch 9 definiert, des Stereoisomers davon oder des pharmazeutisch akzeptablen Salzes davon, wobei das Verfahren die folgenden Schritte umfasst: 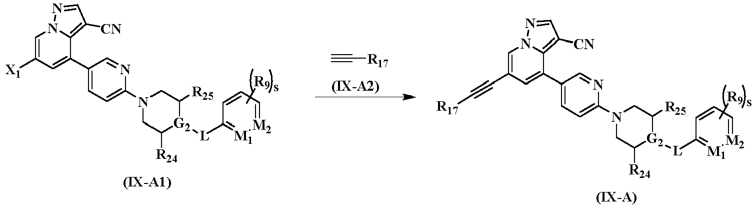
eine Kupplungsreaktion wird mit der allgemeinen Formel (IX-A1) und der allgemeinen Formel (IX-A2) durchgeführt, um die durch die allgemeine Formel (IX-A) dargestellte Verbindung oder das Stereoisomer davon und das pharmazeutisch akzeptable Salz davon zu erhalten;
worin:
X₁ ausgewählt ist aus Halogen; vorzugsweise Fluor, Chlor, Brom oder Jod; besonders bevorzugt Brom.

13. Verfahren zur Herstellung der Verbindung, dargestellt durch die allgemeine Formel (IX-B), wie in Anspruch 3 definiert, des Stereoisomers davon oder des pharmazeutisch akzeptablen Salzes davon, wobei das Verfahren die folgenden Schritte umfasst: 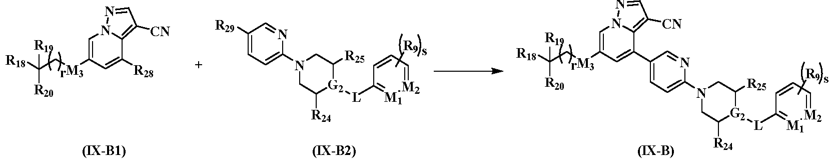
eine Reaktion wird mit der allgemeinen Formel (IX-B1) und der allgemeinen Formel (IX-B2) durchgeführt, um die durch die allgemeine Formel (IX-B) dargestellte Verbindung oder das Stereoisomer davon und das pharmazeutisch akzeptable Salz davon zu erhalten;
worin:
R₂₈ ausgewählt ist aus Halogen, Borsäure oder Boratester; vorzugsweise Fluor, Chlor, Brom, Jod, - B(OH)₂ oder 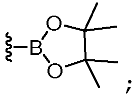
R₂₉ ausgewählt ist aus Halogen, Borsäure oder Boratester; vorzugsweise Fluor, Chlor, Brom, Iod, - B(OH)₂ oder 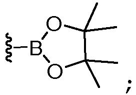
wenn R₂₈ Halogen ist, ist R₂₉ ausgewählt aus Borsäure oder Boratester;
wenn R₂₈ ausgewählt ist aus Borsäure oder Boratester, ist R₂₉ Halogen;
oder,
wobei es die folgenden Schritte umfasst: 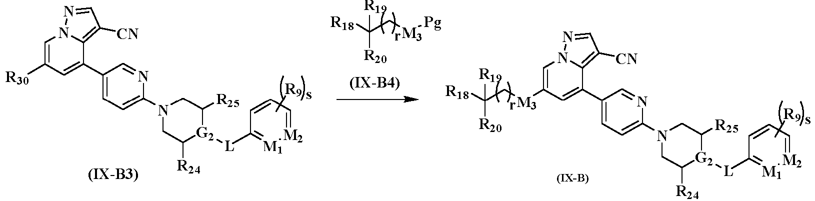
eine Reaktion mit der allgemeinen Formel (IX-B3) und der allgemeinen Formel (IX-B4) durchgeführt wird, um die durch die allgemeine Formel (IX-B) dargestellte Verbindung oder das Stereoisomer davon und das pharmazeutisch akzeptable Salz davon zu erhalten; worin:
R₃₀ ausgewählt ist aus Halogen, Hydroxyl; vorzugsweise Fluor, Chlor, Brom, Jod oder Hydroxyl; besonders bevorzugt Brom oder Hydroxyl;
Pg ausgewählt ist aus Wasserstoff, Halogen oder einer Hydroxyl-Schutzgruppe, und das Halogen vorzugsweise Fluor, Chlor, Brom oder Iod ist;
wenn Pg eine Hydroxylschutzgruppe ist, ist sie ausgewählt aus Methyl, tert-Butyl, Triphenyl, Methylsulfidmethylether, 2-Methoxyethoxymethylether, Methoxymethylether, p-Methoxybenzylether, Pivaloyl, Benzylether, Methoxymethyl, Trimethylsilyl, Tetrahydrofuranyl, tert-Butyldisilyl, Acetyl, Benzoyl oder p-Toluolsulfonyl; vorzugsweise p-Toluolsulfonyl;
wenn M₃ -O- ist, ist Pg ausgewählt aus Wasserstoff oder einer Hydroxyl-Schutzgruppe.

14. Verfahren zur Herstellung der Verbindung, dargestellt durch die allgemeine Formel (IX-C), wie in einem der Ansprüche 5 bis 8 definiert, des Stereoisomers davon oder des pharmazeutisch akzeptablen Salzes davon, wobei das Verfahren die folgenden Schritte umfasst: 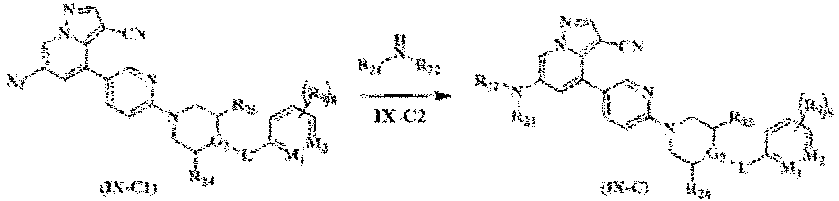
eine Reaktion wird mit der allgemeinen Formel (IX-C1) und der allgemeinen Formel (IX-C2) durchgeführt, um die durch die allgemeine Formel (IX-C) dargestellte Verbindung oder das Stereoisomer davon und das pharmazeutisch akzeptable Salz davon zu erhalten;
worin:
X₂ ausgewählt ist aus Halogen; vorzugsweise Fluor, Chlor, Brom oder Jod; besonders bevorzugt Brom.

15. Pharmazeutische Zusammensetzung, umfassend eine therapeutisch wirksame Menge der in einem der Ansprüche 1 bis 11 definierten Verbindung und des Stereoisomers davon oder des pharmazeutisch akzeptablen Salzes davon und einen oder mehrere pharmazeutisch annehmbare Träger.

16. Die Verbindung, die wie in einem der Ansprüche 1-11 definiert ist, das Stereoisomer davon oder das pharmazeutisch akteptable Salz davon oder die pharmazeutische Zusammensetzung, wie in Anspruch 15 definiert, zur Verwendung als Arzneimittel zur Behandlung und/oder Vorbeugung von nicht-kleinzelligem Lungenkrebs, Fibrosarkom, Pankreastumor, medullärem Schilddrüsenkarzinom, papillärem Schilddrüsentumor, Weichteilsarkom, hochgradig solidem Tumor, Brusttumor und Kolontumor und anderen verwandten Krankheiten.

## Revendications

1. Composé représenté par la formule générale (XI) ou (XI-A), stéréoisomère de celui-ci ou sel pharmaceutiquement acceptable de celui-ci : 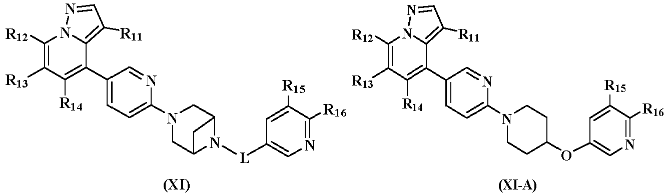 dans lequel :
L est sélectionné parmi une liaison, -(CH₂)ₙ₁CRₐₐR_{bb}-, -(CH₂)ₙ₁NRₐₐC(O)(CH₂)ₙ₂-, (CH₂)ₙ₁C(O)(CH₂)ₙ₂(CRₐₐR_{bb})ₘ-, -(CH₂)ₙ₁C(O)(CRₐₐR_{bb})ₘ(CH₂)ₙ₂-, -(CH₂)ₙ₁C(O)NR_{cc}(CRₐₐR_{bb})ₙ₂-, -(CH₂)ₙ₁(O)(CH₂)ₙ₂- ou -(CH₂)ₙ₁NRₐₐ(CH₂)ₙ₂- ;
R₁₁ est sélectionné parmi l'hydrogène, le deutérium, un alkyle, un alkyle deutéré, un haloalkyle, un alcoxy, un haloalcoxy, un halogène, un amino, un nitro, un hydroxyle, un cyano, un alcényle, un alcynyle, un cycloalkyle, un hétérocyclyle, un aryle, un hétéroaryle, -(CH₂)ₙ₁Rₐₐ, -(CH₂)ₙ₁ORₐₐ, -(CH₂)ₙ₁S(CH₂)ₙ₂Rₐₐ, -(CH₂)ₙ₁C(O)Rₐₐ, -(CH₂)ₙ₁C(O)ORₐₐ, -(CH₂)ₙ₁S(O)ₘRₐₐ, -(CH₂)ₙ₁S(O)(=NRₐₐ)(CH₂)ₙ₂R_{bb}, -(CH₂)ₙ₁NRₐₐR_{bb}, -(CH₂)ₙ₁P(O)RₐₐR_{bb}, -(CH₂)ₙ₁C(O)NRₐₐR_{bb}, -(CH₂)ₙ₁NRₐₐC(O)R_{bb} ou -(CH₂)ₙ₁NRₐₐS(O)ₘR_{bb} ;
R₁₂ est de l'hydrogène, du deutérium, un alkyle, un alkyle deutéré, un haloalkyle, un alcoxy, un haloalcoxy, un halogène, un amino, un nitro, un hydroxyle, un cyano, un alcényle, un alcynyle, un cycloalkyle, un hétérocyclyle, un aryle ou un hétéroaryle ;
R₁₃ est sélectionné parmi un alcényle, un alcynyle, -(C=C)ₙ₁(CRₐₐR_{bb})ₘR_{cc}, -(C=C)ₙ₁(CRₐₐR_{bb})ₘR_{cc}, -(CH₂)ₙ₁O(CH₂)ₙ₂(CRₐₐR_{bb})ₘR_{cc}, dans lequel l'alcényle, ou l'alcynyle est en outre facultativement substitué par un ou plusieurs substituants sélectionnés parmi l'hydrogène, le deutérium, un alkyle non substitué, un alkyle deutéré non substitué, un haloalkyle non substitué, un hydroxyalkyle non substitué, un cyanoalkyle non substitué, un alcoxy non substitué, un haloalcoxy non substitué, un halogène, un amino non substitué, un nitro, un hydroxyle, un cyano, un oxo, un thio, un imino, un alcényle non substitué, un alcynyle non substitué, un cycloalkyle non substitué, un hétérocyclyle non substitué, un aryle non substitué, un hétéroaryle **non** substitué, -(CH₂)ₙ₁R_{dd}, -(CH₂)ₙ₁OR_{dd}, -(CH₂)ₙ₁S(CH₂)ₙ₂R_{dd}, -(CH₂)ₙ₁C(O)R_{dd}, -(CH₂)ₙ₁C(O)OR_{dd}, -(CH₂)ₙ₁S(O)ₘR_{dd}, -(CH₂)ₙ₁S(O)(=NR_{dd})(CH₂)ₙ₂Rₑₑ, -(CH₂)ₙ₁NR_{dd}Rₑₑ, -(CH₂)ₙ₁P(O)R_{dd}Rₑₑ, -(CH₂)ₙ₁C(O)NR_{dd}Rₑₑ, -(CH₂)ₙ₁NR_{dd}C(O)Rₑₑ ou -(CH₂)ₙ₁NR_{dd}S(O)ₘRₑₑ ;
R₁₄ est de l'hydrogène, du deutérium, un alkyle, un alkyle deutéré, un haloalkyle, un alcoxy, un haloalcoxy, un halogène, un amino, un nitro, un hydroxyle, un cyano, un alcényle, un alcynyle, un cycloalkyle, un hétérocyclyle, un aryle ou un hétéroaryle ;
R₁₅ et R₁₆ sont chacun indépendamment sélectionnés parmi l'hydrogène, le deutérium, un alkyle, un alkyle deutéré, un haloalkyle, un alcoxy, un haloalcoxy, un halogène, un amino, un nitro, un hydroxyle, un cyano, un alcényle, un alcynyle, un cycloalkyle, un hétérocyclyle, un aryle, un hétéroaryle ou -(CH₂)ₙ₁ORₐₐ ;
Rₐₐ, R_{bb}, R_{dd} et Rₑₑ sont chacun indépendamment sélectionnés parmi l'hydrogène, le deutérium, un alkyle, un alkyle deutéré, un haloalkyle, un alcoxy, un haloalcoxy, un halogène, un cyano, un nitro, un hydroxyle, un amino, un alcényle, un alcynyle, un cycloalkyle, un hétérocyclyle, un aryle ou un hétéroaryle, dans lequel l'alkyle, l'alkyle deutéré, l'haloalkyle, l'alcoxy, l'haloalcoxy, l'alcényle, l'alcynyle, le cycloalkyle, le hétérocyclyle, l'aryle et l'hétéroaryle sont en outre facultativement substitués par un ou plusieurs substituants sélectionnés parmi l'hydrogène, le deutérium, un alkyle non substitué, un alkyle deutéré non substitué, un haloalkyle non substitué, un alcoxy non substitué, un haloalcoxy non substitué, un halogène, un cyano, un nitro, un hydroxyle, un amino, un oxo, un imino, un alcényle non substitué, un alcynyle non substitué, un cycloalkyle non substitué, un hétérocyclyle non substitué, un aryle non substitué, ou un hétéroaryle non substitué ;
R_{cc} est chacun indépendamment sélectionné parmi un cyano ou un alcynyle ; ou
deux quelconques parmi Rₐₐ, R_{bb}, R_{dd} et Rₑₑ sont reliés pour former un cycloalkyle, un hétérocyclyle, un aryle ou un hétéroaryle, dans lequel le cycloalkyle, l'hétérocyclyle, l'aryle et l'hétéroaryle sont en outre facultativement substitués par un ou plusieurs substituants sélectionnés parmi l'hydrogène, le deutérium, un alkyle non substitué, un alkyle deutéré non substitué, un haloalkyle non substitué, un alcoxy non substitué, un haloalcoxy non substitué, un halogène, un cyano, un nitro, un hydroxyle, un amino, un oxo, une imine, un alcényle non substitué, un alcynyle non substitué, un cycloalkyle non substitué, un hétérocyclyle non substitué, un aryle non substitué, ou un hétéroaryle non substitué ;
m est 0, 1 ou 2 ;
n1 est 0, 1, ou 2 ; et
n2 est 0, 1, ou 2.

2. Composé tel que défini dans la revendication 1, stéréoisomère de celui-ci ou sel pharmaceutiquement acceptable de celui-ci, qui est un composé de formule (XIII) : 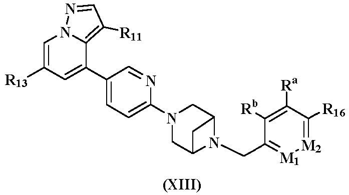 dans lequel :
R₁₁ est sélectionné parmi l'hydrogène, le deutérium, un halogène, un amino, un nitro, un hydroxyle, un cyano, un C₁₋₆ alkyle, un C₁₋₆ alkyle deutéré, un C₁₋₆ haloalkyle, un C₁₋₆ alcoxy, un C₁₋₆ alcoxy deutéré, un C₁₋₆ haloalcoxy, un C₂₋₆ alcényle ou un C₂₋₆ alcynyle ;
R₁₃ est sélectionné parmi C₂₋₆ alcényle, un C₂₋₆ alcynyle, -O(CH₂)ₙ₁(CRₐₐR_{bb})ₘR_{cc} ;
Rₐₐ, et R_{bb} sont chacun indépendamment sélectionnés parmi l'hydrogène, le deutérium, un halogène, un amino, un nitro, un hydroxyle, un cyano, un C₁₋₆ alkyle, un C₁₋₆ alkyle deutéré, un C₁₋₆ haloalkyle, un C₁₋₆ alcoxy, un C₁₋₆ alcoxy deutéré, un C₁₋₆ haloalcoxy, un C₂₋₆ alcényle ou un C₂₋₆ alcynyle ;
R_{cc} sont sélectionnés parmi un cyano, ou un C₂₋₆ alcynyle ;
M₁ est -CH- ;
M₂ est -N- ;
R₁₆ est sélectionné parmi l'hydrogène, le deutérium, un halogène, un amino, un nitro, un hydroxyle, un cyano, un C₁₋₆ alkyle, un C₁₋₆ alkyle deutéré, un C₁₋₆ haloalkyle, un C₁₋₆ alcoxy, un C₁₋₆ alcoxy deutéré, un C₁₋₆ haloalcoxy, un C₂₋₆ alcényle ou un C₂₋₆ alcynyle ;
R^{a} est indépendamment sélectionné parmi l'hydrogène, le deutérium, un halogène, un amino, un nitro, un hydroxyle, un cyano, un C₁₋₆ alkyle, un C₁₋₆ alkyle deutéré, un C₁₋₆ haloalkyle, un C₁₋₆ alcoxy, un C₁₋₆ alcoxy deutéré, un C₁₋₆ haloalcoxy, un C₂₋₆ alcényle ou un C₂₋₆ alcynyle ;
R^{b} est de l'hydrogène ;
n1 est un nombre entier de 1, ou 2 ;
m est un nombre entier de 1, ou 2 ;
préférablement,
R₁₁ est sélectionné parmi l'hydrogène, le deutérium, un halogène, un amino, un nitro, un hydroxyle, un cyano ou un C₁₋₃ alkyle ;
R₁₃ est sélectionné parmi un C₂₋₄ alcényle, un C₂₋₄ alcynyle, ou -OCH₂CRₐₐR_{bb}R_{cc} ;
Rₐₐ, et R_{bb} sont chacun indépendamment sélectionnés parmi l'hydrogène, le deutérium, un halogène, un amino, un nitro, un hydroxyle, un cyano, un C₁₋₃ alkyle, un C₁₋₃ alkyle deutéré, un C₁₋₃ haloalkyle, un C₁₋₃ alcoxy, un C₁₋₃ alcoxy deutéré, un C₁₋₃ haloalcoxy, un C₂₋₄ alcényle ou un C₂₋₄ alcynyle ;
R_{cc} sont cyano ou un C₂₋₄ alcynyle ;
M₁ est -CH-, M₂ est -N- ;
R₁₆ est sélectionné parmi l'hydrogène, le deutérium, un halogène, un amino, un nitro, un hydroxyle, un cyano, un C₁₋₃ alkyle, un C₁₋₃ alkyle deutéré, un C₁₋₃ haloalkyle, un C₁₋₃ alcoxy, un C₁₋₃ alcoxy deutéré ou un C₁₋₃ haloalcoxy ;
R^{a} est indépendamment sélectionné parmi l'hydrogène, le deutérium ou un halogène ;
R^{b} est de l'hydrogène.

3. Composé représenté par la formule générale (IX-B), stéréoisomère de celui-ci ou sel pharmaceutiquement acceptable de celui-ci : 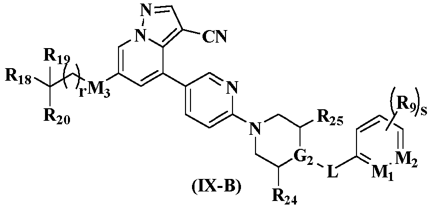 dans lequel :
M₃ est sélectionné parmi une liaison, -O-, -S-, -NH- or -NCH₃- ;
R₁₈ et R₁₉ sont chacun indépendamment sélectionnés parmi l'hydrogène, le deutérium, un C₁₋₆ alkyle, un C₁₋₆ alkyle deutéré, un C₁₋₆ haloalkyle, un C₁₋₆ alcoxy, un C₁₋₆ alcoxy deutéré, un C₁₋₆ haloalcoxy, un halogène, un amino, un nitro, un hydroxyle, un cyano, un C₂₋₆ alcényle, un C₂₋₆ alcynyle, un C₃₋₈ cycloalkyle, un hétérocyclyle à 3-12 chaînons, un C₆₋₁₀ aryle ou un hétéroaryle à 5-12 chaînons, préférablement de l'hydrogène ou du méthyle ;
ou, R₁₈ et R₁₉ avec les atomes de carbone auxquels ils sont attachés forment un C₃₋₈ cycloalkyle ou un hétérocyclyle à 3-12 chaînons, préférablement un C₃₋₆ cycloalkyle ou un hétérocyclyle à 3-7 chaînons comprenant 1-2 atomes d'oxygène, atomes d'azote ou atomes de soufre, plus préférablement du cyclopropyle, du cyclobutyle, du cyclopentyle, de l'oxétanyle, de l'azetidinyle, du bicyclo[1,1,1]pentane ou 1-imino-1-oxothiopyrane, dans lequel le C₃₋₈ cycloalkyle ou l'hétérocyclyle à 3-12 chaînons est en outre facultativement substitué par un ou plusieurs substituants sélectionnés parmi l'hydrogène, un C₁₋₆ alkyle, un hydroxyle, un cyano ou un C₁₋₆ hydroxyalkyle ;
R₂₀ est sélectionné parmi un cyano, ou un C₂₋₆ alcynyle ; préférablement un cyano ;
R₂₄ et R₂₅ sont chacun indépendamment sélectionnés parmi l'hydrogène, le deutérium, un C₁₋₆ alkyle, un C₁₋₆ alkyle deutéré, un C₁₋₆ haloalkyle, un C₁₋₆ alcoxy, un C₁₋₆ alcoxy deutéré, un C₁₋₆ haloalcoxy, un halogène, un amino, un nitro, un hydroxyle, un cyano, un C₂₋₆ alcényle, un C₂₋₆ alcynyle, un C₃₋₈ cycloalkyle, un hétérocyclyle à 3-12 chaînons, un C₆₋₁₀ aryle, un hétéroaryle à 5-12 chaînons ou -(CH₂)ₙ₁ORₐₐ, préférablement de l'hydrogène ou du méthyle ;
ou, R₂₄ et R₂₅ avec les atomes de carbone auxquels ils ont attachés et G₂ forment un C₃₋₈ cycloalkyle ou un hétérocyclyle à 3-12 chaînons, préférablement un azétidinyle ;
L est sélectionné parmi une liaison, -(CH₂)ₙ₁CRₐₐR_{bb}-, -(CH₂)ₙ₁NRₐₐC(O)(CH₂)ₙ₂-, (CH₂)ₙ₁C(O)(CH₂)ₙ₂(CRₐₐR_{bb})ₘ-, -(CH₂)ₙ₁C(O)(CRₐₐR_{bb})ₘ(CH₂)ₙ₂-, -(CH₂)ₙ₁C(O)NR_{cc}(CRₐₐR_{bb})ₙ₂-, -(CH₂)ₙ₁(O)(CH₂)ₙ₂- ou -(CH₂)ₙ₁NRₐₐ(CH₂)ₙ₂-, préférablement-CH₂-, -CD₂-, -O- ou -NHC(O)- ;
G₂ est sélectionné parmi N ou CRₐₐ, préférablement N, CH ou CCH₃ ;
M₁ est sélectionné parmi N ou CRₐₐ, préférablement N, CH ou CCH₃ ;
M₂ est sélectionné parmi N ou CRaa, préférablement N ou CH ;
R₉ est sélectionné parmi l'hydrogène, le deutérium, un C₁₋₆ alkyle, un C₁₋₆ alkyle deutéré, un C₁₋₆ haloalkyle, un C₁₋₆ alcoxy, un C₁₋₆ alcoxy deutéré, un C₁₋₆ haloalcoxy, un halogène, un amino, un nitro, un hydroxyle, un cyano, un C₂₋₆ alcényle, un C₂₋₆ alcynyle, un C₃₋₈ cycloalkyle, un hétérocyclyle à 3-12 chaînons, un C₆₋₁₀ aryle, un hétéroaryle à 5-12 chaînons ou -(CH₂)ₙ₁ORₐₐ; préférablement de l'hydrogène, un halogène, un C₁₋₆ alcoxy, un C₁₋₆ alcoxy deutéré, ou -ORₐₐ ; le plus préférentiellement de l'hydrogène, du fluor, du chlore, du méthyle, un méthoxy, un méthoxy deutéré ou un cyclopropoxy ;
Rₐₐ, R_{bb} et R_{cc} sont chacun indépendamment sélectionnés parmi l'hydrogène, le deutérium, un cyano, un amino, un C₁₋₆ alkyle, un C₁₋₆ alcoxy, un C₁₋₆ hydroxyalkyle, un hydroxyle, un C₃₋₈ cycloalkyle, un hétérocyclyle à 3-12 chaînons ou un C₆₋₁₄ aryle, dans lequel le C₁₋₆ alkyle, le C₁₋₆ alcoxy, le C₁₋₆ hydroxyalkyle, l'hydroxyle, le C₃₋₈ cycloalkyle, l'hétérocyclyle à 3-12 chaînons et le C₆₋₁₄ aryle sont en outre facultativement substitués par un ou plusieurs substituants sélectionnés parmi l'hydrogène, un halogène, un cyano, un hydroxyle, un oxo, un imino, un C₁₋₆ alkyle ou un C₁₋₆ hydroxyalkyle ;
ou, deux quelconques parmi Rₐₐ, R_{bb} et R_{cc} sont facultativement reliés pour former un C₃₋₈ cycloalkyle ou un hétérocyclyle à 3-12 chaînons, dans lequel le C₃₋₈ cycloalkyle et l'hétérocyclyle à 3-12 chaînons sont en outre facultativement substitués par un ou plusieurs substituants sélectionnés parmi l'hydrogène, un amino, un halogène, un cyano, un hydroxyle, un oxo, un imino, un C₁₋₆ alkyle ou un C₁₋₆ hydroxyalkyle ;
s est 0, 1, 2 ou 3 ;
m est 0, 1 ou 2 ;
n1 est 0, 1, ou 2 ; et
n2 est 0, 1, ou 2 ;
r est 0, 1 ou 2 ;
ou, 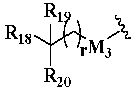 est sélectionné parmi 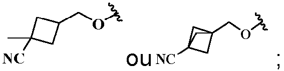 ou, 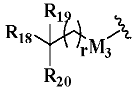 est sélectionné parmi

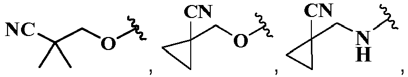

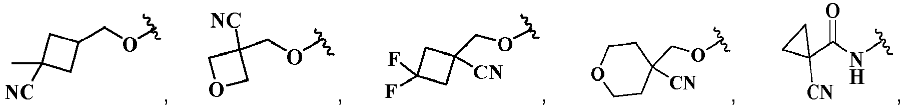

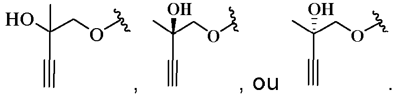

4. Composé tel que défini dans la revendication 3, stéréoisomère de celui-ci ou sel pharmaceutiquement acceptable de celui-ci, qui est un composé de formule (X) : 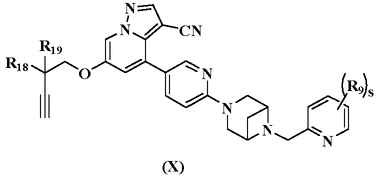
dans lequel, R₁₈ et R₁₉ sont chacun indépendamment sélectionnés parmi l'hydrogène, le deutérium, un C₁₋₃ alkyle, un C₁₋₃ alkyle deutéré, un C₁₋₃ haloalkyle, un C₁₋₃ alcoxy, un C₁₋₃ alcoxy deutéré, un C₁₋₃ haloalcoxy, un halogène, un amino, un nitro, un hydroxyle, un cyano, un C₂₋₄ alcényle, un C₂₋₄ alcynyle, un C₃₋₆ cycloalkyle, un hétérocyclyle à 3-6 chaînons, un C₆₋₁₀ aryle ou un hétéroaryle à 5-6 chaînons, préférablement de l'hydroxyle ou du méthyle ;
R₉ est sélectionné parmi l'hydrogène, le deutérium, un C₁₋₃ alkyle, un C₁₋₃ alkyle deutéré, un C₁₋₃ haloalkyle, un C₁₋₃ alcoxy, un C₁₋₃ alcoxy deutéré, un C₁₋₃ haloalcoxy, un halogène, un amino, un nitro, un hydroxyle, un cyano, un C₂₋₄ alcényle ou un C₂₋₄ alcynyle ;
s est 0, 1, 2 ou 3.

5. Composé représenté par la formule générale (IX-C), stéréoisomère de celui-ci ou sel pharmaceutiquement acceptable de celui-ci : 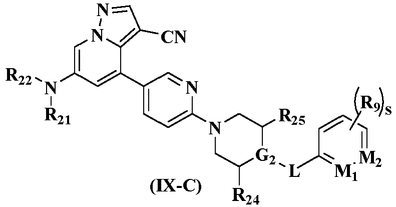 dans lequel :
R₂₁ et R₂₂ sont chacun indépendamment sélectionnés parmi l'hydrogène, le deutérium, un C₁₋₆ alkyle, un C₁₋₆ alkyle deutéré, un C₁₋₆ haloalkyle, un C₁₋₆ alcoxy, un C₁₋₆ alcoxy deutéré, un C₁₋₆ haloalcoxy, un halogène, un amino, un nitro, un hydroxyle, un cyano, un C₂₋₆ alcényle, un C₂₋₆ alcynyle, un C₃₋₈ cycloalkyle, un hétéroaryle à 3-12 chaînons, un C₆₋₁₀ aryle, un hétéroaryle à 5-12 chaînons, -(CH₂)ₙ₁C(O)Rₐₐ ou -(CH₂)ₙ₁Rₐₐ ; ou
R₂₁ et R₂₂ avec les atomes d'azote auxquels ils sont attachés forment un azétidinyle, dans lequel l'azétidinyle est en outre facultativement substitué par un ou plusieurs substituants sélectionnés parmi l'hydrogène, un amino, un halogène, un cyano, un hydroxyle, un oxo, un C₁₋₆ alkyle ou un C₁₋₆ hydroxyalkyle ;
R₂₄ et R₂₅ sont chacun indépendamment sélectionnés parmi l'hydrogène, le deutérium, un C₁₋₆ alkyle, un C₁₋₆ alkyle deutéré, un C₁₋₆ haloalkyle, un C₁₋₆ alcoxy, un C₁₋₆ alcoxy deutéré, un C₁₋₆ haloalcoxy, un halogène, un amino, un nitro, un hydroxyle, un cyano, un C₂₋₆ alcényle, un C₂₋₆ alcynyle, un C₃₋₈ cycloalkyle, un hétérocyclyle à 3-12 chaînons, un C₆₋₁₀ aryle, un hétéroaryle à 5-12 chaînons ou -(CH₂)ₙ₁ORₐₐ, préférablement de l'hydrogène ou du méthyle ;
ou, R₂₄ et R₂₅ avec les atomes de carbone auxquels ils sont attachés et G₂ forment un C₃₋₈ cycloalkyle ou un hétérocyclyle à 3-12 chaînons, préférablement de l'azétidinyle ;
L est sélectionné parmi une liaison, -(CH₂)ₙ₁CRₐₐR_{bb}-, -(CH₂)ₙ₁NRₐₐC(O)(CH₂)ₙ₂-, (CH₂)ₙ₁C(O)(CH₂)ₙ₂(CRₐₐR_{bb})ₘ-, -(CH₂)ₙ₁C(O)(CRₐₐR_{bb})ₘ(CH₂)ₙ₂-, -(CH₂)ₙ₁C(O)NR_{cc}(CRₐₐR_{bb})ₙ₂-, -(CH₂)ₙ₁(O)(CH₂)ₙ₂- ou -(CH₂)ₙ₁NRₐₐ(CH₂)ₙ₂-, préférablement -CH₂-, -CD₂-, -O- ou -NHC(O)- ;
G₂ est sélectionné parmi N ou CRₐₐ, préférablement N, CH ou CCH₃ ;
M₁ est sélectionné parmi N ou CRₐₐ, préférablement N, CH ou CCH₃ ;
M₂ est sélectionné parmi N ou CRₐₐ, préférablement N ou CH ;
R₉ est sélectionné parmi l'hydrogène, le deutérium, un C₁₋₆ alkyle, un C₁₋₆ alkyle deutéré, un C₁₋₆ haloalkyle, un C₁₋₆ alcoxy, un C₁₋₆ alcoxy deutéré, un C₁₋₆ haloalcoxy, un halogène, un amino, un nitro, un hydroxyle, un cyano, un C₂₋₆ alcényle, un C₂₋₆ alcynyle, un C₃₋₈ cycloalkyle, un hétérocyclyle à 3-12 chaînons, un C₆₋₁₀ aryle, un hétéroaryle à 5-12 chaînons ou -(CH₂)ₙ₁ORₐₐ ; préférablement de l'hydrogène, un halogène, un C₁₋₆ alcoxy, un C₁₋₆ alcoxy deutéré, ou -ORₐₐ ; le plus préférentiellement de l'hydrogène, du fluor, du chlore, du méthyle, un méthoxy, un méthoxy deutéré ou un cyclopropoxy ;
Rₐₐ, R_{bb} et R_{cc} sont chacun indépendamment sélectionnés parmi l'hydrogène, le deutérium, un cyano, un amino, un C₁₋₆ alkyle, un C₁₋₆ alcoxy, un C₁₋₆ hydroxyalkyle, un hydroxyle, un C₃₋₈ cycloalkyle, un hétérocyclyle à 3-12 chaînons ou un C₆₋₁₄ aryle, dans lequel le C₁₋₆ alkyle, le C₁₋₆ alcoxy, le C₁₋₆ hydroxyalkyle, l'hydroxyle, le C₃₋₈ cycloalkyle, l'hétérocyclyle à 3-12 chaînons et le C₆₋₁₄ aryle sont en outre facultativement substitués par un ou plusieurs substituants sélectionnés parmi l'hydrogène, un halogène, un cyano, un hydroxyle, un oxo, un imino, un C₁₋₆ alkyle ou un C₁₋₆ hydroxyalkyle ;
ou, deux quelconques parmi Rₐₐ, R_{bb} et R_{cc} sont facultativement reliés pour former un C₃₋₈ cycloalkyle ou un hétérocyclyle à 3-12 chaînons, dans lequel le C₃₋₈ cycloalkyle et l'hétérocyclyle à 3-12 chaînons sont en outre facultativement substitués par un ou plusieurs substituants sélectionnés parmi l'hydrogène, un amino, un halogène, un cyano, un hydroxyle, un oxo, un imino, un C₁₋₆ alkyle ou un C₁₋₆ hydroxyalkyle ;
s est 0, 1, 2 ou 3 ;
m est 0, 1 ou 2 ;
n1 est 0, 1, ou 2 ; et
n2 est 0, 1, ou 2.

6. Composé tel que défini dans la revendication 5, stéréoisomère de celui-ci ou sel pharmaceutiquement acceptable de celui-ci,
R₂₁ et R₂₂ sont chacun indépendamment sélectionnés parmi l'hydrogène, le deutérium, un C₁₋₆ alkyle, un C₁₋₆ alkyle deutéré, un C₁₋₆ haloalkyle, un C₁₋₆ alcoxy, un C₁₋₆ alcoxy deutéré, un C₁₋₆ haloalcoxy, un halogène, un amino, un nitro, un hydroxyle, un cyano, un C₂₋₆ alcényle, un C₂₋₆ alcynyle, un C₃₋₈ cycloalkyle, un hétérocyclyle à 3-12 chaînons, un C₆₋₁₀ aryle, un hétéroaryle à 5-12 chaînons, -(CH₂)ₙ₁C(O)Rₐₐ ou -(CH₂)ₙ₁Rₐₐ ; ou
R₂₁ et R₂₂ avec les atomes d'azote auxquels ils sont attachés forment un azétidinyle, dans lequel l'azétidinyle est en outre facultativement substitué par un ou plusieurs substituants sélectionnés parmi l'hydrogène, un amino, un halogène, un cyano, un hydroxyle, un oxo, un C₁₋₆ alkyle ou un C₁₋₆ hydroxyalkyle ;
R₂₄ et R₂₅ sont chacun indépendamment sélectionnés parmi l'hydrogène ou le méthyle ;
ou, R₂₄ et R₂₅ avec les atomes de carbone auxquels ils sont attachés et G₂ forment un azétidinyle ;
L est sélectionné parmi -CH₂-, -CD₂-, -O- ou -NHC(O)- ;
G₂ est sélectionné parmi N, CH ou CCH₃ ;
M₁ est sélectionné parmi N, CH ou CCH₃ ;
M₂ est sélectionné parmi N ou CH ;
R₉ est sélectionné parmi l'hydrogène, le fluor, le chlore, le méthyle, un méthoxy, un méthoxy deutéré ou un cyclopropoxy ;
Rₐₐ est chacun indépendamment sélectionné parmi l'hydrogène, le deutérium, un cyano, un amino, un C₁₋₆ alkyle, un C₁₋₆ alcoxy, un C₁₋₆ hydroxyalkyle, un hydroxyle, un C₃₋₈ cycloalkyle, un hétérocyclyle à 3-12 chaînons ou un C₆₋₁₄ aryle, dans lequel le C₁₋₆ alkyle, le C₁₋₆ alcoxy, le C₁₋₆ hydroxyalkyle, l'hydroxyle, le C₃₋₈ cycloalkyle, l'hétérocyclyle à 3-12 chaînons et le C₆₁₄ aryle sont en outre facultativement substitués par un ou plusieurs substituants sélectionnés parmi l'hydrogène, un halogène, un cyano, un hydroxyle, un oxo, un imino, un C₁₋₆ alkyle ou un C₁₋₆ hydroxyalkyle ;
s est 0, 1, 2 ou 3 ; et
n1 est 0, 1, ou 2.

7. Composé représenté par la formule générale (IX-C), stéréoisomère de celui-ci ou sel pharmaceutiquement acceptable de celui-ci : 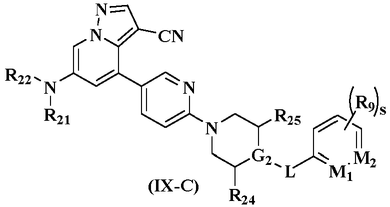 dans lequel :
R₂₁ et R₂₂ sont chacun indépendamment sélectionnés parmi l'hydrogène, le deutérium, un C₁₋₆ alkyle, un C1-6 alkyle deutéré, un C₁₋₆ haloalkyle, un C₁₋₆ alcoxy, un C₁₋₆ alcoxy deutéré, un C₁₋₆ haloalcoxy, un halogène, un amino, un nitro, un hydroxyle, un cyano, un C₂₋₆ alcényle, un C₂₋₆ alcynyle, un C₃₋₈ cycloalkyle, un hétérocyclyle à 3-12 chaînons, un C₆₋₁₀ aryle, un hétéroaryle à 5-12 chaînons, (CH₂)ₙ₁C(O)Rₐₐ ou -(CH₂)ₙ₁Rₐₐ ; ou
R₂₁ et R₂₂ avec les atomes d'azote auxquels ils sont attachés forment un azétidinyle, ou du 2-azaspiro[3.3]heptane, dans lequel l'azétidinyle ou 2-azaspiro[3.3]heptane sont en outre facultativement substitués par un ou plusieurs substituants sélectionnés parmi l'hydrogène, un C₁₋₆ alkyle, un hydroxyle ou un hydroxyalkyle ;
R₂₄ et R₂₅ sont chacun indépendamment sélectionnés parmi l'hydrogène, le deutérium, un C₁₋₆ alkyle, un C₁₋₆ alkyle deutéré, un C₁₋₆ haloalkyle, un C₁₋₆ alcoxy, un C₁₋₆ alcoxy deutéré, un C₁₋₆ haloalcoxy, un halogène, un amino, un nitro, un hydroxyle, un cyano, un C₂₋₆ alcényle, un C₂₋₆ alcynyle, un C₃₋₈ cycloalkyle, un hétérocyclyle à 3-12 chaînons, un C₆₋₁₀ aryle, un hétéroaryle à 5-12 chaînons ou -(CH₂)ₙ₁ORₐₐ, préférablement de l'hydrogène ou du méthyle ;
ou, R₂₄ et R₂₅ avec les atomes de carbone auxquels ils sont attachés et G₂ forment un C₃₋₈ cycloalkyle ou un hétérocyclyle à 3-12 chaînons, préférablement un azétidinyle ;
L est sélectionné parmi une liaison, -(CH₂)ₙ₁CRₐₐR_{bb}-, -(CH₂)ₙ₁NRₐₐC(O)(CH₂)ₙ₂-, (CH₂)ₙ₁C(O)(CH₂)ₙ₂(CRₐₐR_{bb})ₘ-, -(CH₂)ₙ₁C(O)(CRₐₐR_{bb})ₘ(CH₂)ₙ₂-, -(CH₂)ₙ₁C(O)NR_{cc}(CRₐₐR_{bb})ₙ₂-, -(CH₂)ₙ₁(O)(CH₂)ₙ₂- ou -(CH₂)ₙ₁NRₐₐ(CH₂)ₙ₂-, préférablement -CH₂-, -CD₂-, -O- ou -NHC(O)- ;
G₂ est sélectionné parmi N ou CRₐₐ, préférablement N, CH ou CCH₃ ;
M₁ est sélectionné parmi N ou CRₐₐ, préférablement N, CH ou CCH₃ ;
M₂ est sélectionné parmi N ou CRₐₐ, préférablement N ou CH ;
R₉ est sélectionné parmi l'hydrogène, le deutérium, un C₁₋₆ alkyle, un C₁₋₆ alkyle deutéré, un C₁₋₆ haloalkyle, un C₁₋₆ alcoxy, un C₁₋₆ alcoxy deutéré, un C₁₋₆ haloalcoxy, un halogène, un amino, un nitro, un hydroxyle, un cyano, un C₂₋₆ alcényle, un C₂₋₆ alcynyle, un C₃₋₈ cycloalkyle, un hétérocyclyle à 3-12 chaînons, un C₆₋₁₀ aryle, un hétéroaryle à 5-12 chaînons ou -(CH2)ₙ₁ORₐₐ ; préférablement de l'hydrogène, un halogène, un C₁₋₆ alcoxy, un C₁₋₆ alcoxy deutéré, ou -ORₐₐ ; le plus préférentiellement de l'hydrogène, du fluor, du chlore, du méthyle, un méthoxy, un méthoxy deutéré ou un cyclopropoxy ;
Rₐₐ, R_{bb} et R_{cc} sont chacun indépendamment sélectionnés parmi l'hydrogène, le deutérium, un cyano, un amino, un C₁₋₆ alkyle, un C₁₋₆ alcoxy, un C₁₋₆ hydroxyalkyle, un hydroxyle, un C₃₋₈ cycloalkyle, un hétérocyclyle à 3-12 chaînons ou un C₆₋₁₄ aryle, dans lequel le C₁₋₆ alkyle, le C₁₋₆ alcoxy, le C₁₋₆ hydroxyalkyle, l'hydroxyle, le C₃₋₈ cycloalkyle, l'hétérocyclyle à 3-12 chaînons et le C₆₋₁₄ aryle sont en outre facultativement substitués par un ou plusieurs substituants sélectionnés parmi l'hydrogène, un halogène, un cyano, un hydroxyle, un oxo, un imino, un C₁₋₆ alkyle ou un C₁₋₆ hydroxyalkyle ;
ou, deux quelconques parmi Rₐₐ, R_{bb} et R_{cc} sont facultativement reliés pour former un C₃₋₈ cycloalkyle ou un hétérocyclyle à 3-12 chaînons, dans lequel le C₃₋₈ cycloalkyle et l'hétérocyclyle à 3-12 chaînons sont en outre facultativement substitués par un ou plusieurs substituants sélectionnés parmi l'hydrogène, un amino, un halogène, un cyano, un hydroxyle, un oxo, un imino, un C₁₋₆ alkyle ou un C₁₋₆ hydroxyalkyle ;
s est 0, 1, 2 ou 3 ;
m est 0, 1 ou 2 ;
n1 est 0, 1, ou 2 ; et
n2 est 0, 1, ou 2 ;
ou, R₂₁ et R₂₂ avec les atomes de carbone auxquels ils sont attachés forment un azétidinyle, ou du 2-azaspiro[3.3]heptane, dans lequel l'azétidinyle, ou le 2-azaspiro[3.3]heptane est en outre facultativement substitué par un ou plusieurs substituants sélectionnés parmi l'hydrogène, un C₁₋₃ alkyle, un cyano, un hydroxyle ou un hydroxyalkyle.

8. Composé représenté par la formule générale (IX-C), stéréoisomère de celui-ci ou sel pharmaceutiquement acceptable de celui-ci : 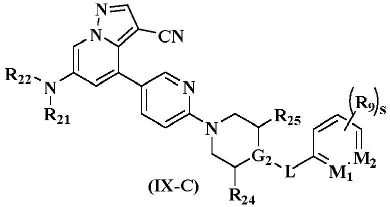 dans lequel : 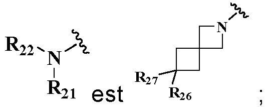
R₂₆ et R₂₇ sont chacun indépendamment sélectionnés parmi l'hydrogène, le deutérium, un halogène, un amino, un nitro, un hydroxyle, un cyano, un C₁₋₆ alkyle, un C₁₋₆ alkyle deutéré, un C₁₋₆ haloalkyle, un C₁₋₆ alcoxy, un C₁₋₆ alcoxy deutéré, un C₁₋₆ haloalcoxy, un C₁₋₆ hydroxyalkyle, un C₂₋₆ alcényle, un C₂₋₆ alcynyle, un C₃₋₈ cycloalkyle, un hétérocyclyle à 3-12 chaînons, un C₆₋₁₀ aryle ou un hétéroaryle à 5-12 chaînons ; préférablement de l'hydrogène, un hydroxyle, un cyano, un C₁₋₃ alkyle ou un C₁₋₃ hydroxyalkyle ; plus préférablement de l'hydrogène, un hydroxyle, un cyano méthyle ou de l'hydroxyisopropyle ;
R₂₄ et R₂₅ sont chacun indépendamment sélectionnés parmi l'hydrogène, le deutérium, un C₁₋₆ alkyle, un C₁₋₆ alkyle deutéré, un C₁₋₆ haloalkyle, un C₁₋₆ alcoxy, un C₁₋₆ alcoxy deutéré, un C₁₋₆ haloalcoxy, un halogène, un amino, un nitro, un hydroxyle, un cyano, un C₂₋₆ alcényle, un C₂₋₆ alcynyle, un C₃₋₈ cycloalkyle, un hétérocyclyle à 3-12 chaînons, un C₆₋₁₀ aryle, un hétéroaryle à 5-12 chaînons ou -(CH2)ₙ₁ORₐₐ, préférablement de l'hydrogène ou du méthyle ;
ou, R₂₄ et R₂₅ avec les atomes de carbone auxquels ils sont attachés et G₂ forment un C₃₋₈ cycloalkyle ou un hétérocyclyle à 3-12 chaînons, préférablement de l'azétidinyle ;
L est sélectionné parmi une liaison, -(CH₂)ₙ₁CRₐₐR_{bb}-, -(CH₂)ₙ₁NRₐₐC(O)(CH₂)ₙ₂-, (CH₂)ₙ₁C(O)(CH₂)ₙ₂(CRₐₐR_{bb})ₘ-, -(CH₂)ₙ₁C(O)(CRₐₐR_{bb})ₘ(CH₂)ₙ₂-, -(CH₂)ₙ₁C(O)NR_{cc}(CRₐₐR_{bb})ₙ₂-, -(CH₂)ₙ₁(O)(CH₂)ₙ₂- ou -(CH₂)ₙ₁NRₐₐ(CH₂)ₙ₂-, préférablement -CH₂-, -CD₂-, -O- ou -NHC(O)- ;
G₂ est sélectionné parmi N ou CRₐₐ, préférablement N, CH ou CCH₃ ;
M₁ est sélectionné parmi N ou CRₐₐ, préférablement N, CH ou CCH₃ ;
M₂ est sélectionné parmi N ou CRaa, préférablement N ou CH ;
R₉ est sélectionné parmi l'hydrogène, le deutérium, un C₁₋₆ alkyle, un C₁₋₆ alkyle deutéré, un C₁₋₆ haloalkyle, un C₁₋₆ alcoxy, un C₁₋₆ alcoxy deutéré, un C₁₋₆ haloalcoxy, un halogène, un amino, un nitro, un hydroxyle, un cyano, un C₂₋₆ alcényle, un C₂₋₆ alcynyle, un C₃₋₈ cycloalkyle, un hétérocyclyle à 3-12 chaînons, un C₆₋₁₀ aryle, un hétéroaryle à 5-12 chaînons ou -(CH₂)ₙ₁ORₐₐ ; préférablement de l'hydrogène, un halogène, un C₁₋₆ alcoxy, un C₁₋₆ alcoxy deutéré, ou -ORₐₐ ; le plus préférentiellement de l'hydrogène, du fluor, du chlore, du méthyle, un méthoxy, un méthoxy deutéré ou un cyclopropoxy ;
Rₐₐ, R_{bb} et R_{cc} sont chacun indépendamment sélectionnés parmi l'hydrogène, le deutérium, un cyano, un amino, un C₁₋₆ alkyle, un C₁₋₆ alcoxy, un C₁₋₆ hydroxyalkyle, un hydroxyle, un C₃₋₈ cycloalkyle, un hétérocyclyle à 3-12 chaînons ou un C₆₋₁₄ aryle, dans lequel le C₁₋₆ alkyle, le C₁₋₆ alcoxy, le C₁₋₆ hydroxyalkyle, l'hydroxyle, le C₃₋₈ cycloalkyle, l'hétérocyclyle à 3-12 chaînons et le C₆₋₁₄ aryle sont en outre facultativement substitués par un ou plusieurs substituants sélectionnés parmi l'hydrogène, un halogène, un cyano, un hydroxyle, un oxo, un imino, un C₁₋₆ alkyle ou un C₁₋₆ hydroxyalkyle ;
ou, deux quelconques parmi Rₐₐ, R_{bb} et R_{cc} sont facultativement reliés pour former un C₃₋₈ cycloalkyle ou un hétérocyclyle à 3-12 chaînons, dans lequel le C₃₋₈ cycloalkyle et l'hétérocyclyle à 3-12 chaînons sont en outre facultativement substitués par un ou plusieurs substituants sélectionnés parmi l'hydrogène, un amino, un halogène, un cyano, un hydroxyle, un oxo, un imino, un C₁₋₆ alkyle ou un C₁₋₆ hydroxyalkyle ;
s est 0, 1, 2 ou 3 ;
m est 0, 1 ou 2 ;
n1 est 0, 1, ou 2 ; et
n2 est 0, 1, ou 2.

9. Composé représenté par la formule générale (IX-A), stéréoisomère de celui-ci ou sel pharmaceutiquement acceptable de celui-ci : 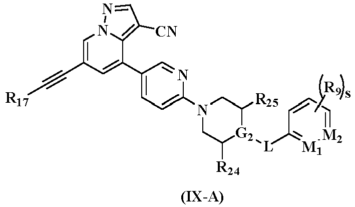 dans lequel :
L est sélectionné parmi une liaison, -(CH₂)ₙ₁CRₐₐR_{bb}-, -(CH₂)ₙ₁NRₐₐC(O)(CH₂)ₙ₂-, (CH₂)ₙ₁C(O)(CH₂)ₙ₂(CRₐₐR_{bb})ₘ-, -(CH₂)ₙ₁C(O)(CRₐₐR_{bb})ₘ(CH₂)ₙ₂-, -(CH₂)ₙ₁C(O)NR_{cc}(CRₐₐR_{bb})ₙ₂-, -(CH₂)ₙ₁(O)(CH₂)ₙ₂- ou -(CH₂)ₙ₁NRₐₐ(CH₂)ₙ₂-, préférablement -CH₂-, -CD₂-, -O- ou -NHC(O)- ;
G₂ est sélectionné parmi N ou CRₐₐ, préférablement N, CH ou CCH₃ ;
M₁ est sélectionné parmi N ou CRₐₐ, préférablement N, CH ou CCH₃ ;
M₂ est sélectionné parmi N ou CRₐₐ, préférablement N ou CH ;
R₉ est sélectionné parmi l'hydrogène, le deutérium, un C₁₋₆ alkyle, un C₁₋₆ alkyle deutéré, un C₁₋₆ haloalkyle, un C₁₋₆ alcoxy, un C₁₋₆ alcoxy deutéré, un C₁₋₆ haloalcoxy, un halogène, un amino, un nitro, un hydroxyle, un cyano, un C₂₋₆ alcényle, un C₂₋₆ alcynyle, un C₃₋₈ cycloalkyle, un hétérocyclyle à 3-12 chaînons, un C₆₋₁₀ aryle, un hétéroaryle à 5-12 chaînons ou -(CH₂)ₙ₁ORₐₐ ; préférablement de l'hydrogène, un halogène, un C₁₋₆ alcoxy, un C₁₋₆ alcoxy deutéré, ou -ORₐₐ ; le plus préférentiellement de l'hydrogène, du fluor, du chlore, du méthyle, un méthoxy, un méthoxy deutéré ou un cyclopropoxy ;
R₁₇ est sélectionné parmi un C₁₋₆ alkyle, un C₁₋₆ haloalkyle, un C₁₋₆ alcoxy, un C₂₋₆ alcynyle, un hétérocyclyle à 3-12 chaînons, un hétéroaryle à 5-12 chaînons ou -(CH₂)ₙ₁(CRₐₐR_{bb}) R_{cc}, dans lequel le C₁₋₆ alkyle, le C₁₋₆ haloalkyle, le C₁₋₆ alcoxy, le C₂₋₆ alcynyle, l'hétérocyclyle à 3-12 chaînons et l'hétéroaryle à 5-12 chaînons sont en outre facultativement substitués par un ou plusieurs substituants sélectionnés parmi l'hydrogène, un C₁₋₆ haloalkyle, un hydroxyle, un cyano, un oxo, un thio, un amino, un imino, un C₁₋₆ alcoxy, un C₁₋₆ hydroxyalkyle, un C₁₋₆ cyanoalkyle, un C₃₋₈ cycloalkyle ou un hétérocyclyle à 3-12 chaînons ;
R₂₄ et R₂₅ sont chacun indépendamment sélectionnés parmi l'hydrogène, le deutérium, un C₁₋₆ alkyle, un C₁₋₆ alkyle deutéré, un C₁₋₆ haloalkyle, un C₁₋₆ alcoxy, un C₁₋₆ alcoxy deutéré, un C₁₋₆ haloalcoxy, un halogène, un amino, un nitro, un hydroxyle, un cyano, un C₂₋₆ alcényle, un C₂₋₆ alcynyle, un C₃₋₈ cycloalkyle, un hétérocyclyle à 3-12 chaînons, un C₆₋₁₀ aryle, un hétéroaryle à 5-12 chaînons ou -(CH₂)ₙ₁ORₐₐ, préférablement de l'hydrogène ou du méthyle ;
ou, R₂₄ et R₂₅ avec les atomes de carbone auxquels ils sont attachés et G₂ forment un C₃₋₈ cycloalkyle ou un hétérocyclyle à 3-12 chaînons, préférablement de l'azétidinyle ;
Rₐₐ, R_{bb} et R_{cc} sont chacun indépendamment sélectionnés parmi l'hydrogène, le deutérium, un cyano, un amino, un C₁₋₆ alkyle, un C₁₋₆ alcoxy, un C₁₋₆ hydroxyalkyle, un hydroxyle, un C₃₋₈ cycloalkyle, un hétérocyclyle à 3-12 chaînons ou un C₆₋₁₄ aryle, dans lequel le C₁₋₆ alkyle, le C₁₋₆ alcoxy, le C₁₋₆ hydroxyalkyle, l'hydroxyle, le C₃₋₈ cycloalkyle, l'hétérocyclyle à 3-12 chaînons et le C₆₁₄ aryle sont en outre facultativement substitués par un ou plusieurs substituants sélectionnés parmi l'hydrogène, un halogène, un cyano, un hydroxyle, un oxo, un imino, un C₁-alkyle ou un C₁₋₆ hydroxyalkyle ;
ou, deux quelconques parmi Rₐₐ, R_{bb} et R_{cc} sont facultativement reliés pour former un C₃₋₈ cycloalkyle ou un hétérocyclyle à 3-12 chaînons, dans lequel le C₃₋₈ cycloalkyle et l'hétérocyclyle à 3-12 chaînons sont en outre facultativement substitués par un ou plusieurs substituants sélectionnés parmi l'hydrogène, un amino, un halogène, un cyano, un hydroxyle, un oxo, un imino, un C₁₋₆ alkyle ou un C₁₋₆ hydroxyalkyle ;
n1 est 0, 1 ou 2 ;
n2 est 0, 1 ou 2 ;
m est 0, 1 ou 2 ; et
s est 0, 1, 2 ou 3.

10. Composé tel que défini dans la revendication 1, stéréoisomère de celui-ci ou sel pharmaceutiquement acceptable de celui-ci, dans lequel,
L est sélectionné parmi une liaison, -(CH₂)ₙ₁CRₐₐR_{bb}-, -(CH₂)ₙ₁NRₐₐC(O)(CH₂)ₙ₂-, (CH₂)ₙ₁C(O)(CRₐₐR_{bb})ₘ(CH₂)ₙ₂-, -(CH2)ₙ₁C(O)(CH₂)ₘ(CRₐₐR_{bb})ₙ₂-, -(CH₂)ₙ₁C(O)NR_{cc}(CRₐₐR_{bb})n₂-, -(CH₂)ₙ₁(O)(CH₂)ₙ₂- ou -(CH₂)ₙ₁NRₐₐ(CH₂)ₙ₂- ;
R₁₁ est sélectionné parmi un cyano, -(CH₂)ₙ₁P(O)RₐₐR_{bb} ou -(CH₂)ₙ₁C(O)NRₐₐR_{bb} ;
R₁₂ est sélectionné parmi l'hydrogène, un C₁₋₆ alkyle ou un halogène ;
R₁₃ est sélectionné parmi un C₂₋₆ alcynyle, -(C≡C)ₙ₁(CRₐₐR_{bb})ₘR_{cc}, -(C=C)ₙ₁(CRₐₐR_{bb})ₘR_{cc}, ou -(CH₂)ₙ₁O(CH₂)ₙ₂(CRₐₐR_{bb})ₘR_{cc} ;
R₁₄ est sélectionné parmi l'hydrogène ou un halogène ;
R₁₅ est sélectionné parmi l'hydrogène ou un halogène ;
R₁₆ est sélectionné parmi l'hydrogène, un alcoxy ou -ORₐₐ ;
Rₐₐ, et R_{bb} sont chacun indépendamment sélectionnés parmi l'hydrogène, le deutérium, un cyano, un amino, un C₁₋₆ alkyle, un C₁₋₆ alcoxy, un hydroxyle, un C₃₋₈ cycloalkyle, un hétérocyclyle à 3-12 chaînons ou un C₆₋₁₄ aryle, dans lequel le C₁₋₆ alkyle, le C₁₋₆ alcoxy, le C₃₋₈ cycloalkyle, l'hétérocyclyle à 3-12 chaînons et le C₆₋₁₄ aryle sont en outre facultativement substitués par un ou plusieurs substituants sélectionnés parmi l'hydrogène, un halogène, un cyano, un hydroxyle, un oxo, un imino, ou C₁₋₆ alkyle ;
R_{cc} est sélectionné parmi un cyano ;
ou, deux quelconques parmi Rₐₐ et R_{bb} sont facultativement reliés pour former un C₃₋₈ cycloalkyle ou un hétérocyclyle à 3-12 chaînons, dans lequel le C₃₋₈ cycloalkyle et l'hétérocyclyle à 3-12 chaînons sont en outre facultativement substitués par un ou plusieurs substituants sélectionnés parmi l'hydrogène, un amino, un halogène, un cyano, un hydroxyle, un oxo, une imine, ou un C₁₋₆ alkyle.

11. Composé, stéréoisomère de celui-ci ou sel pharmaceutiquement acceptable de celui-ci, dans lequel, la structure spécifique du composé est la suivante :

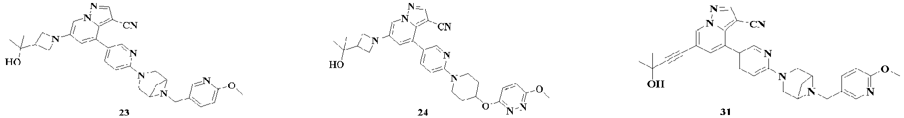

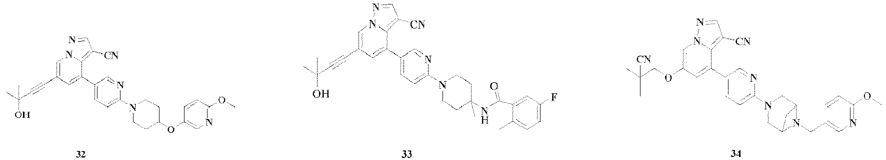

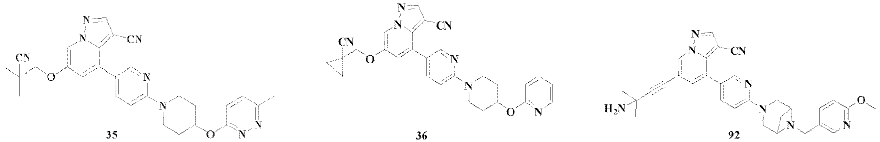

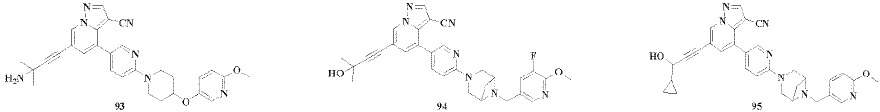

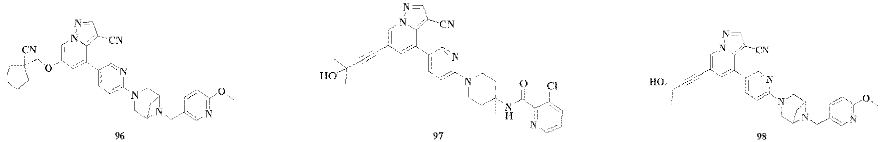

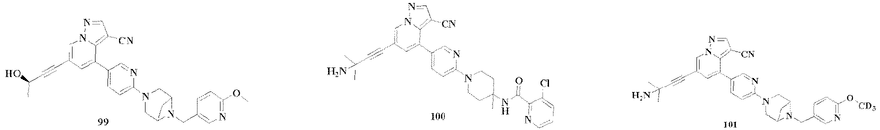

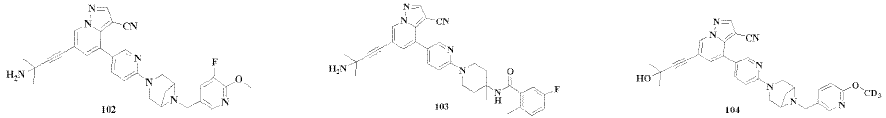

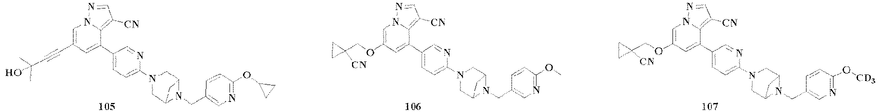

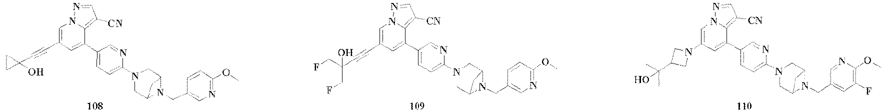

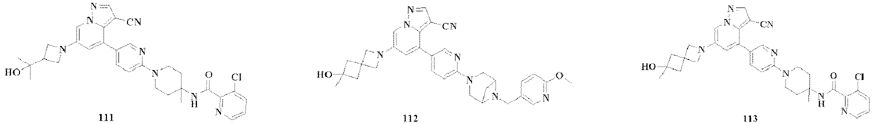

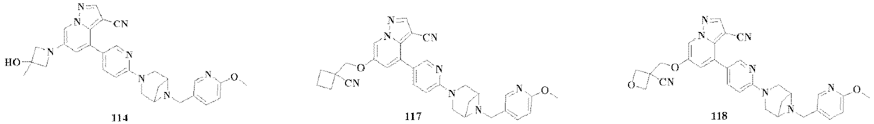

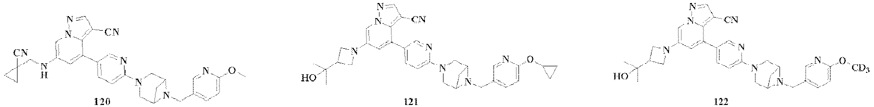

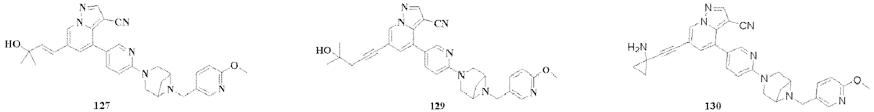

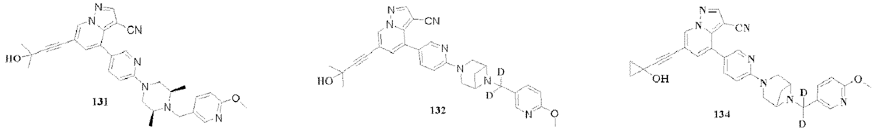

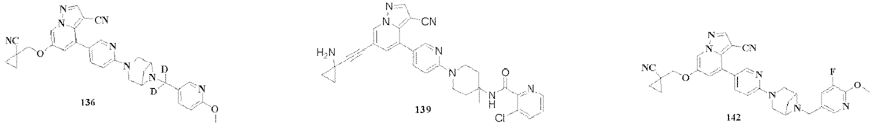

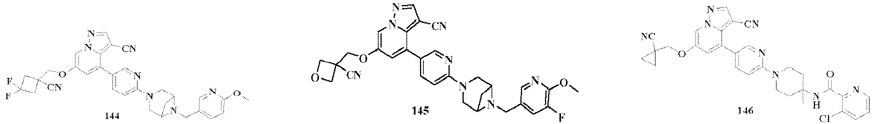

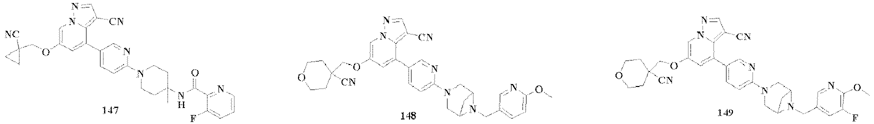

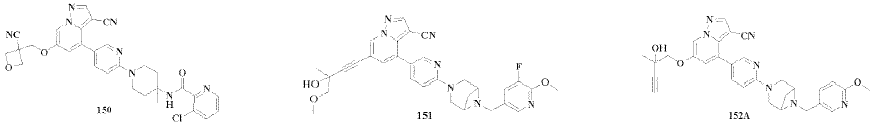

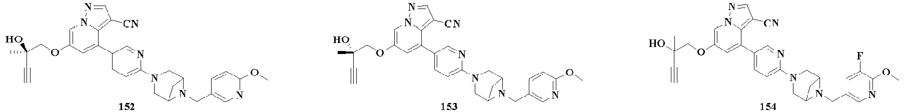

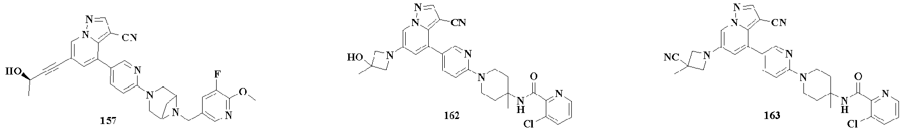

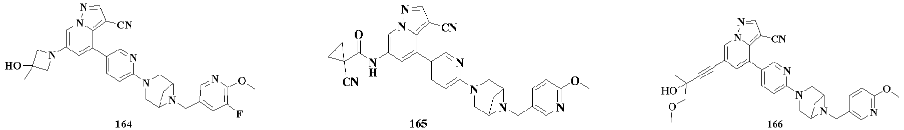

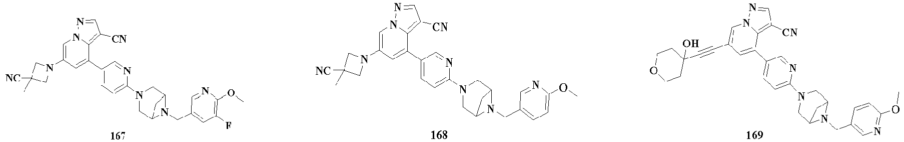

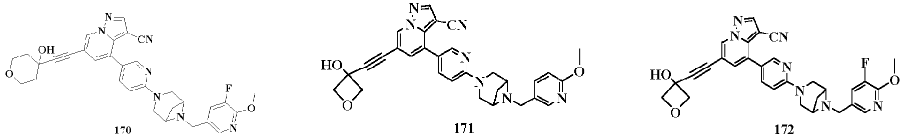

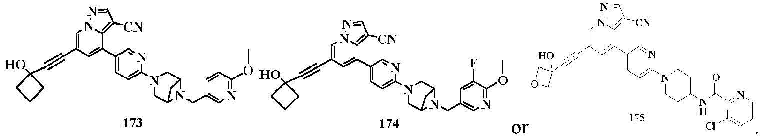

12. Procédé de préparation du composé représenté par la formule générale (IX-A) tel que défini dans la revendication 9, du stéréoisomère de celui-ci ou du sel pharmaceutiquement acceptable de celui-ci, dans lequel, comprenant les étapes suivantes : 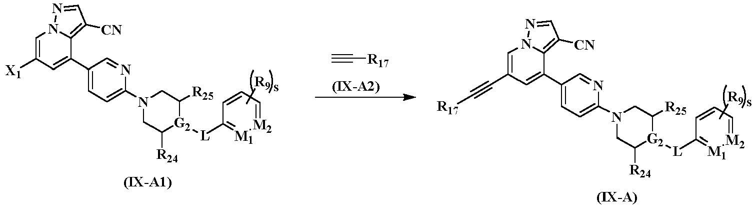
une réaction de couplage est mise en œuvre avec la formule générale (IX-A1) et la formule générale (IX-A2) pour obtenir le composé représenté par la formule générale (IX-A) ou le stéréoisomère de celui-ci et le sel pharmaceutiquement acceptable de celui-ci ;
dans lequel :
X₁ est sélectionné parmi un halogène ; préférablement le fluor, le chlore, le brome ou l'iode ; plus préférablement le brome.

13. Procédé de préparation du composé représenté par la formule générale (IX-B) tel que défini dans la revendication 3, du stéréoisomère de celui-ci ou du sel pharmaceutiquement acceptable de celui-ci, dans lequel, comprenant les étapes suivantes : 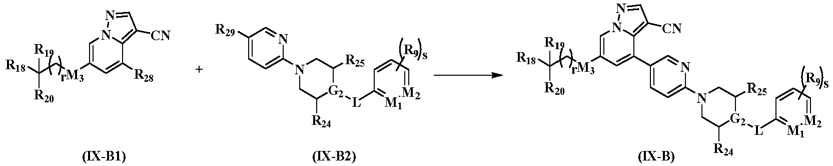
une réaction de couplage est mise en œuvre avec la formule générale (IX-B1) et la formule générale (IX-B2) pour obtenir le composé représenté par la formule générale (IX-B) ou le stéréoisomère de celui-ci et le sel pharmaceutiquement acceptable de celui-ci ;
dans lequel :
R₂₈ est sélectionné parmi un halogène, l'acide borique ou l'ester de borate ; préférablement le fluor, le chlore, le brome, l'iode, -B(OH)₂ ou 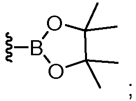
R₂₉ est sélectionné parmi un halogène, l'acide borique ou l'ester de borate ; préférablement le fluor, le chlore, le brome, l'iode, -B(OH)₂ ou 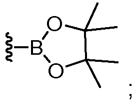
quand R₂₈ est un halogène, R₂₉ est sélectionné parmi l'acide borique ou l'ester de borate ;
quand R₂₈ est sélectionné parmi l'acide borique ou l'ester de borate, R₂₉ est un halogène ;
ou,
dans lequel, comprenant les étapes suivantes : 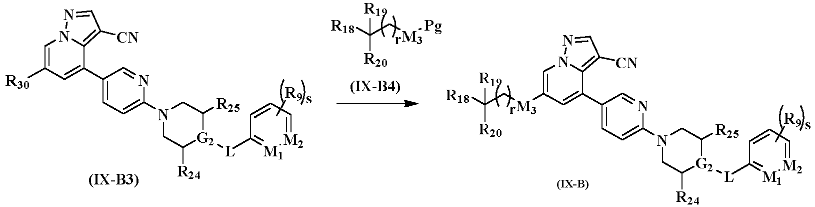
une réaction est mise en œuvre avec la formule générale (IX-B3) et la formule générale (IX-B4) pour obtenir le composé représenté par la formule générale (IX-B) ou le stéréoisomère de celui-ci et le sel pharmaceutiquement acceptable de celui-ci ;
dans lequel :
R₃₀ est sélectionné parmi un halogène, un hydroxyle ; préférablement le fluor, le chlore, le brome, l'iode, ou un hydroxyle ; plus préférablement le brome ou un hydroxyle ;
Pg est sélectionné parmi l'hydrogène, un halogène ou un groupe de protection hydroxyle, et l'halogène est préférablement du fluor, du chlore, du brome ou de l'iode ;
quand Pg est un groupe de protection hydroxyle, il est sélectionné parmi méthyle, tert-butyle, triphényle, sulfure de méthyle, éther de méthyle, éther de 2-méthoxyéthoxyméthyle, éther de méthoxyméthyle, éther de p-méthoxybenzyle, pivaloyle, éther de benzyle, méthoxyméthyle, triméthylsilyle, tétrahydrofuranyle, *tert*-butyldisilyle, acétyle, benzoyle ou *p*-toluènesulfonyle ; préférablement *p*-toluènesulfonyle :
quand M₃ est -O-, Pg est sélectionné parmi l'hydrogène ou un groupe de protection hydroxyle.

14. Procédé de préparation du composé représenté par la formule générale (IX-C) tel que défini dans l'une quelconque des revendications 5 à 8, du stéréoisomère de celui-ci ou du sel pharmaceutiquement acceptable de celui-ci, dans lequel, comprenant les étapes suivantes : 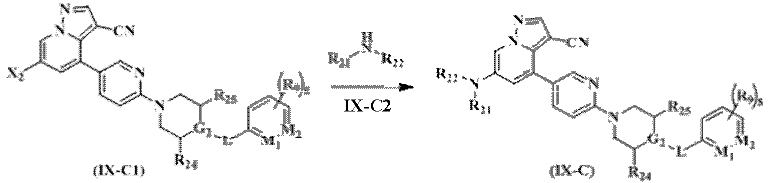
une réaction est mise en œuvre avec la formule générale (IX-C1) et la formule générale (IX-C2) pour obtenir le composé représenté par la formule générale (IX-C) ou le stéréoisomère de celui-ci et le sel pharmaceutiquement acceptable de celui-ci ;
dans lequel :
X₂ est sélectionné parmi un halogène ; préférablement le fluor, le chlore, le brome ou l'iode ; plus préférablement le brome.

15. Composition pharmaceutique comprenant une quantité thérapeutiquement efficace du composé tel que défini dans l'une quelconque des revendications 1 à 11, et du stéréoisomère de celui-ci ou du sel pharmaceutiquement acceptable de celui-ci, et un ou plusieurs vecteurs pharmaceutiquement acceptables.

16. Composé représenté tel que défini dans l'une quelconque des revendications 1 à 11, stéréoisomère de celui-ci ou sel pharmaceutiquement acceptable de celui-ci, ou composition pharmaceutique telle que définie dans la revendication 15 pour utilisation en tant que médicaments pour le traitement et/ou la prévention d'un cancer du poumon non à petites cellules, d'un fibrosarcome, d'une tumeur pancréatique, d'un carcinome médullaire de la thyroïde, d'un papillome de la thyroïde, d'un sarcome des tissus mous, d'une tumeur hautement solide, d'une tumeur du sein et d'une tumeur du côlon et d'autres maladies associées.
